# EUROPEAN PATENT APPLICATION

(11) **EP 2 105 502 A1**
(43) Date of publication of application: **30.09.2009**
(21) Application number: 09008582.0
(22) Date of filing: 30.11.2001
(51) Int. Cl.: C12N 15/12, C07K 14/47, C07K 16/30, G01N 33/574, C12Q 1/68, C12N 5/08, A61K 38/17, A61K 31/711, A61K 39/395, A61K 35/14

(54) **Compositions and methods for the therapy and diagnosis of lung cancer**

(30) Priority: 12.12.2000 US 735705; 07.05.2001 US 850716; 28.06.2001 US 897778
(62) Divisional of application: 01990067.9
(71) Applicant: CORIXA CORPORATION, Seattle, WA 98104 (US)
(72) Inventor: Wang, Tongtong, Medina, WA 98039 (US); Wang, Aijun, Issaquah, WA 98029 (US); Skeiky, Yasir A.W., Bellevue, WA 98006 (US); Li, Samuel X., Redmond, WA 98052 (US); Kalos, Michael D., Seattle, WA 98103 (US); Henderson, Robert A., Edmonds, WA 98026 (US); McNeill, Patricia D., Federal Way, WA 98003 (US); Fanger, Neil, Seattle, WA 98103 (US); Retter, Marc W., Carnation, WA 98014 (US); Durham, Margarita, Seattle, WA 98199 (US); Fanger, Gary R., Mill Creek, WA 98012 (US); Vedvick, Thomas S., Federal Way, WA 98003 (US); Carter, Darrick, Seattle, WA 98102 (US); Watanabe, Yoshihiro, Mercer Island, WA 98040 (US); Peckham, David W., Seattle, WA 98104 (US); Cai, Feng, Bellevue, WA 98004-3686 (US); Foy, Teresa M., Federal Way, WA 98003 (US)
(74) Representative: Walker, Ross Thomson

(57) **Abstract**

Compositions and methods for the therapy and diagnosis of cancer, particularly lung cancer, are disclosed. Illustrative compositions comprise one or more lung tumor polypeptides, immunogenic portions thereof, polynucleotides that encode such polypeptides, antigen presenting cell that expresses such polypeptides, and T cells that are specific for cells expressing such polypeptides. The disclosed compositions are useful, for example, in the diagnosis, prevention and/or treatment of diseases, particularly lung cancer.

SEQUENCE LISTING

<110> Corixa Corporation
      Wang, Tongtong
      Wang, Aijun
      Skeiky, Yasir A.W.
      Li, Samual X.
      Kalos, Michael D.
      Henderson, Robert A.
      McNeill, Patricia D.
      Fanger, Neil
      Retter, Marc W.
      Durham, Margarita
      Fanger, Gary R.
      Vedvick, Thomas S.
      Carter, Darrick
      Watanabe, Yoshihiro
      Peckman, David W.
      Cai, Feng
      Foy, Teresa M.

<120> COMPOSITIONS AND METHODS FOR THE THERAPY
  AND DIAGNOSIS OF LUNG CANCER

<130> 210121.45503PC

<140> PCT
<141> 2001-11-30

<160> 469

<170> FastSEQ for Windows Version 4.0

<210> 1
<211> 315
<212> DNA
<213> Homo sapiens

<220>
<221> misc_feature
<222> 236, 241
<223> n = A,T,C or G

<400> 1
gcagagacag actggtggtt gaacctggag gtgccaaaaa agccagctgc gggcccagga 60
cagctgccgt gagactcccg atgtcacagg cagtctgtgt ggttacagcg cccctcagtg 120
ttcatctcca gcagagacaa cggaggaggc tcccaccagg acggttctca ttatttatat 180
gttaatatgt ttgtaaactc atgtacagtt ttttttgggg gggaagcaat gggaanggta 240
naaattacaa atagaatcat ttgctgtaat ccttaaatgg caaacggtca ggccacgtga 300
aaaaaaaaaa aaaaa                                                  315

<210> 2
<211> 380
<212> DNA

<213> Homo sapiens

<400> 2
atttaggctt aagattttgt ttacccttgt tactaaggag caaattagta ttaaagtata 60
atatatataa acaaatacaa aaagttttga gtggttcagc ttttttattt tttttaatgg 120
cataactttt aacaacactg ctctgtaatg ggttgaactg tggtactcag actgagataa 180
ctgaaatgag tggatgtata gtgttattgc ataattatcc cactatgaag caaagggact 240
ggataaattc ccagtctaga ttattagcct ttgttaacca tcaagcacct agaagaagaa 300
ttattggaaa ttttgtcctc tgtaactggc actttggggt gtgacttatc ttttgccttt 360
gtaaaaaaaa aaaaaaaaaa 380

<210> 3
<211> 346
<212> DNA
<213> Homo sapiens

<220>
<221> misc_feature
<222> 316, 317, 318, 322, 323, 326, 329, 330, 331, 336, 337, 339,
340, 342, 343
<223> n = A,T,C or G

<400> 3
ttgtaagtat acaattttag aaaggattaa atgttattga tcattttact gaatactgca 60
catcctcacc atacaccatc cactttccaa taacatttaa tcctttctaa aattgtaagt 120
atacaattgt actttctttg gattttcata acaaatatac catagactgt taattttatt 180
gaagtttcct taatggaatg agtcattttt gtcttgtgct tttgaggtta cctttgcttt 240
gacttccaac aatttgatca tatagtgttg agctgtggaa atctttaagt ttattctata 300
gcaataattt ctattnnnag annccnggnn naaaannann annaaa 346

<210> 4
<211> 372
<212> DNA
<213> Homo sapiens

<220>
<221> misc_feature
<222> 297, 306, 332
<223> n = A,T,C or G

<400> 4
actagtctca ttactccaga attatgctct tgtacctgtg tggctgggtt tcttagtcgt 60
tggtttggtt tggttttttg aactggtatg tagggtggtt cacagttcta atgtaagcac 120
tctcttctcc aagttgtgct ttgtggggac aatcattctt tgaacattag agaggaaggc 180
agttcaagct gttgaaaaga ctattgctta tttttgtttt taaagaccta cttgacgtca 240
tgtggacagt gcacgtgcct tacgctacat cttgttttct aggaagaagg ggatgcnggg 300
aaggantggg tgctttgtga tggataaaac gnctaaataa cacaccttta cattttgaaa 360
aaaacaaaac aa 372

<210> 5
<211> 698
<212> DNA
<213> Homo sapiens

<220>
<221> misc_feature
<222> 8, 345, 422, 430, 433, 436, 438, 472, 481, 486, 515, 521,
536, 549, 553, 556, 557, 559, 568, 593, 597, 605, 611, 613,

616, 618, 620, 628, 630, 632, 634, 635, 639, 643, 647, 648,
649, 652, 654, 658, 664, 690
<223> n = A,T,C or G

<400> 5
actagtanga tagaaacact gtgtcccgag agtaaggaga gaagctacta ttgattagag 60
cctaacccag gttaactgca agaagaggcg ggatactttc agctttccat gtaactgtat 120
gcataaagcc aatgtagtcc agtttctaag atcatgttcc aagctaactg aatcccactt 180
caatacacac tcatgaactc ctgatggaac aataacaggc ccaagcctgt ggtatgatgt 240
gcacacttgc tagactcaga aaaaatacta ctctcataaa tgggtgggag tattttgggt 300
gacaacctac tttgcttggc tgagtgaagg aatgatattc atatnttcat ttattccatg 360
gacatttagt tagtgctttt tatataccag gcatgatgct gagtgacact cttgtgtata 420
tntccaaatn ttngtncngt cgctgcacat atctgaaatc ctatattaag antttcccaa 480
natgangtcc ctggtttttc cacgccactt gatcngtcaa ngatctcacc tctgtntgtc 540
ctaaaaccnt ctnctnnang gttagacngg acctctcttc tcccttcccg aanaatnaag 600
tgtgngaaga nanccncncn cccccctncn tncnncctng ccngctnnnc cncntgtngg 660
gggngccgcc cccgcggggg gacccccccn ttttcccc 698

<210> 6
<211> 740
<212> DNA
<213> Homo sapiens

<220>
<221> misc_feature
<222> 82, 406, 426, 434, 462, 536, 551, 558, 563, 567, 582, 584,
592, 638, 651, 660, 664, 673, 675, 697, 706, 711, 715, 716,
717, 723, 724, 725, 733
<223> n = A,T,C or G

<400> 6
actagtcaaa aatgctaaaa taatttggga gaaaatattt tttaagtagt gttatagttt 60
catgtttatc ttttattatg tnttgtgaag ttgtgtcttt tcactaatta cctatactat 120
gccaatattt ccttatatct atccataaca tttatactac atttgtaaga gaatatgcac 180
gtgaaactta acactttata aggtaaaaat gaggtttcca agatttaata atctgatcaa 240
gttcttgtta tttccaaata gaatggactt ggtctgttaa ggggctaagg gagaagaaga 300
agataaggtt aaaagttgtt aatgaccaaa cattctaaaa gaaatgcaaa aaaaaattta 360
ttttcaagcc ttcgaactat ttaaggaaag caaaatcatt tcctanatgc atatcatttg 420
tgagantttc tcantaatat cctgaatcat tcatttcagc tnaggcttca tgttgactcg 480
atatgtcatc tagggaaagt ctatttcatg gtccaaacct gttgccatag ttggtnaggc 540
tttcctttaa ntgtgaanta ttnacangaa attttctctt tnanagttct tnatagggtt 600
aggggtgtgg gaaaagcttc taacaatctg tagtgttncg tgttatctgt ncagaaccan 660
aatnacggat cgnangaagg actgggtcta tttacangaa cgaatnatct ngttnnntgt 720
gtnnncaact ccngggagcc 740

<210> 7
<211> 670
<212> DNA
<213> Homo sapiens

<220>
<221> misc_feature
<222> 265, 268, 457, 470, 485, 546, 553, 566, 590, 596, 613, 624,
639, 653, 659, 661
<223> n = A,T,C or G

<400> 7
gctggggagc tcggcatggc ggtccccgct gcagccatgg ggccctcggc gttgggccag 60

agcggccccg gctcgatggc cccgtggtgc tcagtgagca gcggcccgtc gcgctacgtg 120
cttgggatgc aggagctgtt ccggggccac agcaagaccg cgagttcctg gcgcacagcg 180
ccaaggtgca ctcggtggcc tggagttgcg acgggcgtcg cctacctcgg ggtcttcgac 240
aagacgccac gtcttcttgc tgganaanga ccgttggtca aagaaaacaa ttatcgggga 300
catggggata gtgtggacca ctttgttggc atccaagtaa tcctgaccta tttgttacgg 360
cgtctggaga taaaaccatt cgcatctggg atgtgaggac tacaaaatgc attgccactg 420
tgaacactaa aggggagaac attaatatct gctggantcc tgatgggcan accattgctg 480
tagcnacaag gatgatgtgg tgactttatt gatgccaaga aaccccgttc caaagcaaaa 540
aaacanttcc aanttcgaag tcaccnaaat ctcctggaac aatgaacatn aatatnttct 600
tcctgacaat ggnccttggg tgtntcacat cctcagctnc cccaaaactg aancctgtnc 660
natccacccc 670

<210> 8
<211> 689
<212> DNA
<213> Homo sapiens

<220>
<221> misc_feature
<222> 253, 335, 410, 428, 448, 458, 466, 479, 480, 482, 483, 485,
488, 491, 492, 495, 499, 500, 502, 503, 512, 516, 524, 525,
526, 527, 530, 540, 546, 550, 581, 593, 594, 601, 606, 609,
610, 620, 621, 622, 628, 641, 646, 656, 673
<223> n = A,T,C or G

<400> 8
actagtatct aggaatgaac agtaaaagag gagcagttgg ctacttgatt acaacagagt 60
aaatgaagta ctggatttgg gaaaacctgg ttttattaga acatatggaa tgaaagccta 120
cacctagcat tgcctactta gccccctgaa ttaacagagc ccaattgaga caaacccctg 180
gcaacaggaa attcaaggga gaaaaagtaa gcaacttggg ctaggatgag ctgactccct 240
tagagcaaag ganagacagc ccccattacc aaataccatt tttgcctggg gcttgtgcag 300
ctggcagtgt tcctgcccca gcatggcacc ttatngtttt gatagcaact tcgttgaatt 360
ttcaccaact tattacttga aattataata tagcctgtcc gtttgctgtn tccaggctgt 420
gatatatntt cctagtggtt tgactttnaa aataaatnag gtttantttt ctccccccnn 480
cnntnctncc nntcnctcnn cnntcccccc cnctcngtcc tccnnnnttn gggggggccn 540
cccccncggn ggacccccct ttggtccctt agtggaggtt natggcccct ggnnttatcc 600
nggccntann tttccccgtn nnaaatgntt ccccctccca ntcccnccac ctcaanccgg 660
aagcctaagt ttntaccctg ggggtcccc 689

<210> 9
<211> 674
<212> DNA
<213> Homo sapiens

<220>
<221> misc_feature
<222> 602, 632, 639, 668
<223> n = A,T,C or G

<400> 9
gtccactctc ctttgagtgt actgtcttac tgtgcactct gtttttcaac tttctagata 60
taaaaaatgc ttgttctata gtggagtaag agctcacaca cccaaggcag caagataact 120
gaaaaaagcg aggctttttt gccaccttgg taaaggccag ttcactgcta tagaactgct 180
ataagcctga agggaagtag ctatgagact ttccattttt cttagttctc ccaataggct 240
ccttcatgga aaaaggcttc ctgtaataat tttcacctaa tgaattagca gtgtgattat 300
ttctgaaata agagacaaat tgggccgcag agtcttcctg tgatttaaaa taaacaaccc 360
aaagttttgt ttggtcttca ccaaaggaca tactctaggg ggtatgttgt tgaagacatt 420
caaaaacatt agctgttctg tctttcaatt tcaagttatt ttggagactg cctccatgtg 480

agttaattac tttgctctgg aactagcatt attgtcatta tcatcacatt ctgtcatcat 540
catctgaata atattgtgga tttccccctc tgcttgcatc ttcttttgac tcctctggga 600
anaaatgtca aaaaaaaagg tcgatctact cngcaaggnc catctaatca ctgcgctgga 660
aggacccnct gccc 674

<210> 10
<211> 346
<212> DNA
<213> Homo sapiens

<220>
<221> misc_feature
<222> 320, 321, 322, 325, 326, 328, 329, 330, 332, 333, 334, 335,
342
<223> n = A,T,C or G

<400> 10
actagtctgc tgatagaaag cactatacat cctattgttt ctttctttcc aaaatcagcc 60
ttctgtctgt aacaaaaatg tactttatag agatggagga aaaggtctaa tactacatag 120
ccttaagtgt ttctgtcatt gttcaagtgt attttctgta acagaaacat atttggaatg 180
tttttctttt ccccttataa attgtaattc ctgaaatact gctgctttaa aaagtcccac 240
tgtcagatta tattatctaa caattgaata ttgtaaatat acttgtctta cctctcaata 300
aaagggtact tttctattan nnagnngnnn gnnnnataaa anaaaa 346

<210> 11
<211> 602
<212> DNA
<213> Homo sapiens

<400> 11
actagtaaaa agcagcattg ccaaataatc cctaattttc cactaaaaat ataatgaaat 60
gatgttaagc tttttgaaaa gtttaggtta aacctactgt tgttagatta atgtatttgt 120
tgcttccctt tatctggaat gtggcattag cttttttatt ttaaccctct ttaattctta 180
ttcaattcca tgacttaagg ttggagagct aaacactggg atttttggat aacagactga 240
cagttttgca taattataat cggcattgta catagaaagg atatggctac cttttgttaa 300
atctgcactt tctaaatatc aaaaaaggga aatgaagtta taaatcaatt tttgtataat 360
ctgtttgaaa catgagtttt atttgcttaa tattagggct ttgccccttt tctgtaagtc 420
tcttgggatc ctgtgtagaa ctgttctcat taaacaccaa acagttaagt ccattctctg 480
gtactagcta caaattcggt ttcatattct acttaacaat ttaaataaac tgaaatattt 540
ctagatggtc tacttctgtt catataaaaa caaaacttga tttccaaaaa aaaaaaaaaa 600
aa 602

<210> 12
<211> 685
<212> DNA
<213> Homo sapiens

<220>
<221> misc_feature
<222> 170, 279, 318, 321, 322, 422, 450, 453, 459, 467, 468, 470,
473, 475, 482, 485, 486, 491, 498, 503, 506, 509, 522, 526,
527, 528, 538, 542, 544, 551, 567, 568, 569, 574, 576, 582,
587, 588, 589, 590, 592, 593, 598, 599, 603, 605, 608
<223> n = A,T,C or G

<221> misc_feature
<222> 633, 634, 635, 644, 646, 648, 651, 655, 660, 662, 663, 672,
674, 675, 682, 683

<223> n = A,T,C or G

<400> 12
actagtcctg tgaaagtaca actgaaggca gaaagtgtta ggattttgca tctaatgttc 60
attatcatgg tattgatgga cctaagaaaa taaaaattag actaagcccc caaataagct 120
gcatgcattt gtaacatgat tagtagattt gaatatatag atgtagtatn ttgggtatct 180
aggtgtttta tcattatgta aaggaattaa agtaaaggac tttgtagttg tttttattaa 240
atatgcatat agtagagtgc aaaaatatag caaaaatana aactaaaggt agaaaagcat 300
tttagatatg ccttaatnta nnaactgtgc caggtggccc tcggaataga tgccaggcag 360
agaccagtgc ctgggtggtg cctccccttg tctgcccccc tgaagaactt ccctcacgtg 420
angtagtgcc ctcgtaggtg tcacgtggan tantggganc aggccgnncn gtnanaagaa 480
ancanngtga nagtttcncc gtngangcng aactgtccct gngccnnnac gctcccanaa 540
cntntccaat ngacaatcga gtttccnnnc tccngnaacc tngccgnnnn cnngcccnnc 600
cantntgnta accccgcgcc cggatcgctc tcnnntcgtt ctcncncnaa ngggntttcn 660
cnnccgccgt cncnnccccg cnncc 685

<210> 13
<211> 694
<212> DNA
<213> Homo sapiens

<220>
<221> misc_feature
<222> 503, 546, 599, 611, 636, 641, 643, 645, 656, 658, 662, 676,
679, 687
<223> n = A,T,C or G

<400> 13
cactagtcac tcattagcgt tttcaatagg gctcttaagt ccagtagatt acgggtagtc 60
agttgacgaa gatctggttt acaagaacta attaaatgtt tcattgcatt tttgtaagaa 120
cagaataatt ttataaaatg tttgtagttt ataattgccg aaaataattt aaagacactt 180
tttctctgtg tgtgcaaatg tgtgtttgtg atccattttt tttttttttt taggacacct 240
gtttactagc tagctttaca atatgccaaa aaaggatttc tccctgaccc catccgtggt 300
tcaccctctt ttccccccat gctttttgcc ctagtttata acaaaggaat gatgatgatt 360
taaaaagtag ttctgtatct tcagtatctt ggtcttccag aaccctctgg ttgggaaggg 420
gatcattttt tactggtcat ttccctttgg agtgtactac tttaacagat ggaaagaact 480
cattggccat ggaaacagcc gangtgttgg gagccagcag tgcatggcac cgtccggcat 540
ctggcntgat tggtctggct gccgtcattg tcagcacagt gccatgggac atggggaana 600
ctgactgcac ngccaatggt tttcatgaag aatacngcat ncncngtgat cacgtnancc 660
angacgctat gggggncana gggccanttg cttc 694

<210> 14
<211> 679
<212> DNA
<213> Homo sapiens

<220>
<221> misc_feature
<222> 29, 68, 83, 87, 94, 104, 117, 142, 145, 151, 187, 201, 211,
226, 229, 239, 241, 245, 252, 255, 259, 303, 309, 359, 387,
400, 441, 446, 461, 492, 504, 505, 512, 525, 527, 533, 574,
592, 609, 610, 618, 620, 626, 627, 633, 639, 645, 654
<223> n = A,T,C or G

<400> 14
cagccgcctg catctgtatc cagcgccang tcccgccagt cccagctgcg cgcgcccccc 60
agtcccgnac ccgttcggcc cangctnagt tagncctcac catnccggtc aaaggangca 120
ccaagtgcat caaatacctg cngtncggat ntaaattcat cttctggctt gccgggattg 180

ctgtccntgc cattggacta nggctccgat ncgactctca gaccanganc atcttcganc 240
naganactaa tnatnattnt tccagcttct acacaggagt ctatattctg atcggatccg 300
gcnccctcnt gatgctggtg ggcttcctga gctgctgcgg ggctgtgcaa gagtcccant 360
gcatgctggg actgttcttc ggcttcntct tggtgatatn cgccattgaa atacctgcgg 420
ccatctgggg atattccact ncgatnatgt gattaaggaa ntccacggag ttttacaagg 480
acacgtacaa cnacctgaaa accnnggatg anccccaccg ggaancnctg aangccatcc 540
actatgcgtt gaactgcaat ggtttggctg gggnccttga acaatttaat cncatacatc 600
tggccccann aaaggacntn ctcganncct tcnccgtgna attcngttct gatnccatca 660
cagaagtctc gaacaatcc 679

<210> 15
<211> 695
<212> DNA
<213> Homo sapiens

<220>
<221> misc_feature
<222> 105, 172, 176, 179, 189, 203, 212, 219, 221, 229, 231, 238,
242, 261, 266, 270, 278, 285, 286, 298, 311, 324, 337, 350,
363, 384, 391, 395, 405, 411, 424, 427, 443, 448, 453, 455,
458, 463, 467, 470, 479, 482, 484, 493, 499, 505, 518
<223> n = A,T,C or G

<221> misc_feature
<222> 520, 523, 531, 540, 584, 595, 597, 609, 611, 626, 628, 651,
652, 657, 661, 665, 669, 672, 681, 683, 691, 693
<223> n = A,T,C or G

<400> 15
actagtggat aaaggccagg gatgctgctc aacctcctac catgtacagg gacgtctccc 60
cattacaact acccaatccg aagtgtcaac tgtgtcagga ctaanaaacc ctggttttga 120
ttaaaaaagg gcctgaaaaa aggggagcca caaatctgtc tgcttcctca cnttantcnt 180
tggcaaatna gcattctgtc tcnttggctg cngcctcanc ncaaaaaanc ngaactcnat 240
cnggcccagg aatacatctc ncaatnaacn aaattganca aggcnntggg aaatgccnga 300
tgggattatc ntccgcttgt tgancttcta agtttcnttc ccttcattcn accctgccag 360
ccnagttctg ttagaaaaat gccngaattc naacnccggt tttcntactc ngaatttaga 420
tctncanaaa cttcctggcc acnattcnaa ttnanggnca cgnacanatn ccttccatna 480
ancncacccc acntttgana gccangacaa tgactgcntn aantgaaggc ntgaaggaan 540
aactttgaaa ggaaaaaaaa ctttgtttcc ggccccttcc aacncttctg tgttnancac 600
tgccttctng naaccctgga agcccngnga cagtgttaca tgttgttcta nnaaacngac 660
ncttnaatnt cnatcttccc nanaacgatt ncncc 695

<210> 16
<211> 669
<212> DNA
<213> Homo sapiens

<220>
<221> misc_feature
<222> 299, 354, 483, 555, 571, 573, 577, 642, 651, 662, 667
<223> n = A,T,C or G

<400> 16
cgccgaagca gcagcgcagg ttgtccccgt ttcccctccc ccttcccttc tccggttgcc 60
ttcccgggcc ccttacactc cacagtcccg gtcccgccat gtcccagaaa caagaagaag 120
agaaccctgc ggaggagacc ggcgaggaga agcaggacac gcaggagaaa gaaggtattc 180
tgcctgagag agctgaagag gcaaagctaa aggccaaata cccaagccta ggacaaaagc 240
ctggaggctc cgacttcctc atgaagagac tccagaaagg gcaaaagtac tttgactcng 300

gagactacaa catggccaaa gccaacatga agaataagca gctgccaagt gcangaccag 360
acaagaacct ggtgactggt gatcacatcc ccaccccaca ggatctgccc agagaaagtc 420
ctcgctcgtc accagcaagc ttgcgggtgg ccaagttgaa tgatgctgcc ggggctctgc 480
canatctgag acgcttccct ccctgcccca cccgggtcct gtgctggctc ctgcccttcc 540
tgcttttgca gccangggtc aggaagtggc ncnggtngtg gctggaaagc aaaacccttt 600
cctgttggtg tcccacccat ggagcccctg gggcgagccc angaacttga ncctttttgt 660
tntcttncc 669

<210> 17
<211> 697
<212> DNA
<213> Homo sapiens

<220>
<221> misc_feature
<222> 33, 48, 50, 55, 59, 60, 76, 77, 78, 90, 113, 118, 130, 135,
141, 143, 150, 156, 166, 167, 170, 172, 180, 181, 190, 192,
194, 199, 201, 209, 212, 224, 225, 226, 230, 233, 234, 236,
242, 244, 251, 253, 256, 268, 297, 305, 308, 311, 314
<223> n = A,T,C or G

<221> misc_feature
<222> 315, 317, 322, 324, 327, 333, 337, 343, 362, 364, 367, 368,
373, 384, 388, 394, 406, 411, 413, 423, 429, 438, 449, 450,
473, 476, 479, 489, 491, 494, 499, 505, 507, 508, 522, 523,
527, 530, 533, 535, 538, 539, 545, 548, 550, 552, 555
<223> n = A,T,C or G

<221> misc_feature
<222> 562, 563, 566, 568, 572, 577, 578, 580, 581, 591, 594, 622,
628, 632, 638, 642, 644, 653, 658, 662, 663, 665, 669, 675,
680, 686, 689
<223> n = A,T,C or G

<400> 17
gcaagatatg gacaactaag tgagaaggta atnctctact gctctagntn ctccnggcnn 60
gacgcgctga ggagannnac gctggcccan ctgccggcca cacacgggga tcntggtnat 120
gcctgcccan gggancccca ncnctcggan cccatntcac acccgnnccn tncgcccacn 180
ncctggctcn cncngcccng nccagctcnc gnccccctcc gccnnnctcn ttnncntctc 240
cncnccctcc ncnacnacct cctacccncg gctccctccc cagccccccc ccgcaancct 300
ccacnacncc ntcnncncga ancnccnctc gcnctcngcc ccngccccct gccccccgcc 360
cncnacnncg cgntcccccg cgcncgcngc ctcnccccct cccacnacag ncncacccgc 420
agncacgcnc tccgcccnct gacgccccnn cccgccgcgc tcaccttcat ggnccnacng 480
ccccgctcnc nccnctgcnc gccgncnngg cgccccgccc cnnccgngtn ccncncgnng 540
ccccngcngn angcngtgcg cnncangncc gngccgnncn ncaccctccg nccnccgccc 600
cgcccgctgg gggctcccgc cncgcggntc antccccncc cntncgccca ctntccgntc 660
cnncnctcnc gctcngcgcn cgcccnccnc ccccccc 697

<210> 18
<211> 670
<212> DNA
<213> Homo sapiens

<220>
<221> misc_feature
<222> 234, 292, 329, 437, 458, 478, 487, 524, 542, 549, 550, 557,
576, 597, 603, 604, 646, 665
<223> n = A,T,C or G

<400> 18
ctcgtgtgaa gggtgcagta cctaagccgg agcggggtag aggcgggccg gcaccccctt 60
ctgacctcca gtgccgccgg cctcaagatc agacatggcc cagaacttga acgacttggc 120
gggacggctg cccgccgggc cccggggcat gggcacggcc ctgaagctgt tgctgggggc 180
cggcgccgtg gcctacggtg tgcgcgaatc tgtgttcacc gtggaaggcg ggcncagagc 240
catcttcttc aatcggatcg gtggagtgca caggacacta tcctgggccg anggccttca 300
cttcaggatc cttggttcca gtaccccanc atctatgaca ttcgggccag acctcgaaaa 360
aatctcctcc ctacaggctc caaagaccta cagatggtga atatctccct gcgagtgttg 420
tctcgaccaa tgctcangaa cttcctaaca tgttccancg cctaagggct ggactacnaa 480
gaacgantgt tgccgtccat tgtcacgaag tgctcaagaa tttnggtggc caagttcaat 540
gncctcacnn ctgatcnccc agcggggcca agttanccct ggttgatccc cgggganctg 600
acnnaaaagg gccaaggact tcccctcatc ctggataatg tggccntcac aaagctcaac 660
tttanccacc 670

<210> 19
<211> 606
<212> DNA
<213> Homo sapiens

<220>
<221> misc_feature
<222> 506
<223> n = A,T,C or G

<400> 19
actagtgcca acctcagctc ccaggccagt tctctgaatg tcgaggagtt ccaggatctc 60
tggcctcagt tgtccttggt tattgatggg ggacaaattg gggatggcca gagccccgag 120
tgtcgccttg gctcaactgt ggttgatttg tctgtgcccg gaaagtttgg catcattcgt 180
ccaggctgtg ccctggaaag tactacagcc atcctccaac agaagtacgg actgctcccc 240
tcacatgcgt cctacctgtg aaactctggg aagcaggaag gcccaagacc tggtgctgga 300
tactatgtgt ctgtccactg acgactgtca aggcctcatt tgcagaggcc accggagcta 360
gggcactagc ctgactttta aggcagtgtg tctttctgag cactgtagac caagcccttg 420
gagctgctgg tttagccttg cacctgggga aaggatgtat ttatttgtat tttcatatat 480
cagccaaaag ctgaatggaa aagttnagaa cattcctagg tggccttatt ctaataagtt 540
tcttctgtct gttttgtttt tcaattgaaa agttattaaa taacagattt agaatctagt 600
gagacc 606

<210> 20
<211> 449
<212> DNA
<213> Homo sapiens

<400> 20
actagtaaac aacagcagca gaaacatcag tatcagcagc gtcgccagca ggagaatatg 60
cagcgccaga gccgaggaga acccccgctc cctgaggagg acctgtccaa actcttcaaa 120
ccaccacagc cgcctgccag gatggactcg ctgctcattg caggccagat aaacacttac 180
tgccagaaca tcaaggagtt cactgcccaa aacttaggca agctcttcat ggcccaggct 240
cttcaagaat acaacaacta agaaaaggaa gtttccagaa aagaagttaa catgaactct 300
tgaagtcaca ccagggcaac tcttggaaga aatatatttg catattgaaa agcacagagg 360
atttctttag tgtcattgcc gattttggct ataacagtgt ctttctagcc ataataaaat 420
aaaacaaaat cttgactgct tgctcaaaa 449

<210> 21
<211> 409
<212> DNA
<213> Homo sapiens

<400> 21
tatcaatcaa ctggtgaata attaaacaat gtgtggtgtg atcatacaaa gggtaccact 60
caatgataaa aggaacaagc tgcctatatg tggaacaaca tggatgcatt tcagaaactt 120
tatgttgagt gaaagaacaa acacggagaa catactatgt ggttctcttt atgtaacatt 180
acagaaataa aaacagaggc aaccaccttt gaggcagtat ggagtgagat agactggaaa 240
aaggaaggaa ggaaactcta cgctgatgga aatgtctgtg tcttcattgg gtggtagtta 300
tgtggggata tacatttgtc aaaatttatt gaactatata ctaaagaact ctgcatttta 360
ttgggatgta aataatacct caattaaaaa gacaaaaaaa aaaaaaaaa 409

<210> 22
<211> 649
<212> DNA
<213> Homo sapiens

<220>
<221> misc_feature
<222> 263, 353, 610, 635, 646
<223> n = A,T,C or G

<400> 22
acaattttca ttatcttaag cacattgtac atttctacag aacctgtgat tattctcgca 60
tgataaggat ggtacttgca tatggtgaat tactactgtt gacagtttcc gcagaaatcc 120
tatttcagtg gaccaacatt gtggcatggc agcaaatgcc aacattttgt ggaatagcag 180
caaatctaca agagaccctg gttggttttt cgttttgttt tctttgtttt ttcccccttc 240
tcctgaatca gcagggatgg aangagggta gggaagttat gaattactcc ttccagtagt 300
agctctgaag tgtcacattt aatatcagtt ttttttaaac atgattctag ttnaatgtag 360
aagagagaag aaagaggaag tgttcacttt tttaatacac tgatttagaa atttgatgtc 420
ttatatcagt agttctgagg tattgatagc ttgctttatt tctgccttta cgttgacagt 480
gttgaagcag ggtgaataac taggggcata tatatttttt ttttttgtaa gctgtttcat 540
gatgttttct ttggaatttc cggataagtt caggaaaaca tctgcatgtt gttatctagt 600
ctgaagttcn tatccatctc attacaacaa aaacncccag aacggnttg 649

<210> 23
<211> 669
<212> DNA
<213> Homo sapiens

<220>
<221> misc_feature
<222> 642, 661
<223> n = A,T,C or G

<400> 23
actagtgccg tactggctga aatccctgca ggaccaggaa gagaaccagt tcagactttg 60
tactctcagt caccagctct ggaattagat aaattccttg aagatgtcag gaatgggatc 120
tatcctctga cagcctttgg gctgcctcgg ccccagcagc cacagcagga ggaggtgaca 180
tcacctgtcg tgcccccctc tgtcaagact ccgacacctg aaccagctga ggtggagact 240
cgcaaggtgg tgctgatgca gtgcaacatt gagtcggtgg aggagggagt caaacaccac 300
ctgacacttc tgctgaagtt ggaggacaaa ctgaaccggc acctgagctg tgacctgatg 360
ccaaatgaga atatccccga gttggcggct gagctggtgc agctgggctt cattagtgag 420
gctgaccaga gccggttgac ttctctgcta gaagagactt gaacaagttc aattttgcca 480
ggaacagtac cctcaactca gccgctgtca ccgtctcctc ttagagctca ctcgggccag 540
gccctgatct gcgctgtggc tgtcctggac gtgctgcacc ctctgtcctt ccccccagtc 600
agtattacct gtgaagccct tccctccttt attattcagg anggctgggg gggctccttg 660
nttctaacc 669

<210> 24
<211> 442

<212> DNA
<213> Homo sapiens

<400> 24
actagtacca tcttgacaga ggatacatgc tcccaaaacg tttgttacca cacttaaaaa 60
tcactgccat cattaagcat cagtttcaaa attatagcca ttcatgattt actttttcca 120
gatgactatc attattctag tcctttgaat ttgtaagggg aaaaaaaaca aaaacaaaaa 180
cttacgatgc acttttctcc agcacatcag atttcaaatt gaaaattaaa gacatgctat 240
ggtaatgcac ttgctagtac tacacacttt ggtacaacaa aaaacagagg caagaaacaa 300
cggaaagaga aaagccttcc tttgttggcc cttaaactga gtcaagatct gaaatgtaga 360
gatgatctct gacgatacct gtatgttctt attgtgtaaa taaaattgct ggtatgaaat 420
gacctaaaaa aaaaaaaaga aa 442

<210> 25
<211> 656
<212> DNA
<213> Homo sapiens

<220>
<221> misc_feature
<222> 330, 342, 418, 548, 579, 608
<223> n = A,T,C or G

<400> 25
   tgcaagtacc acacactgtt tgaattttgc acaaaaagtg actgtaggat caggtgatag 60
   ccccggaatg tacagtgtct tggtgcacca agatgccttc taaaggctga cataccttgg 120
   accctaatgg ggcagagagt atagccctag cccagtggtg acatgaccac tccctttggg 180
   aggcctgagg tagaggggag tggtatgtgt tttctcagtg gaagcagcac atgagtgggt 240
   gacaggatgt tagataaagg ctctagttag ggtgtcattg tcatttgaga gactgacaca 300
   ctcctagcag ctggtaaagg ggtgctggan gccatggagg anctctagaa acattagcat 360
   gggctgatct gattacttcc tggcatcccg ctcactttta tgggaagtct tattagangg 420
   atgggacagt tttccatatc cttgctgtgg agctctggaa cactctctaa atttccctct 480
   attaaaaatc actgccctaa ctacacttcc tccttgaagg aatagaaatg gaactttctc 540
   tgacatantt cttggcatgg ggagccagcc acaaatgana atctgaacgt gtccaggttt 600
   ctcctganac tcatctacat agaattggtt aaaccctccc ttggaataag gaaaaa 656

<210> 26
<211> 434
<212> DNA
<213> Homo sapiens

<220>
<221> misc_feature
<222> 395
<223> n = A,T,C or G

<400> 26
actagttcag actgccacgc caaccccaga aaatacccca catgccagaa aagtgaagtc 60
ctaggtgttt ccatctatgt ttcaatctgt ccatctacca ggcctcgcga taaaaacaaa 120
acaaaaaaac gctgccaggt tttagaagca gttctggtct caaaaccatc aggatcctgc 180
caccagggtt cttttgaaat agtaccacat gtaaaaggga atttggcttt cacttcatct 240
aataactgaa ttgtcaggct ttgattgata attgtagaaa taagtagcct tctgttgtgg 300
gaataagtta taatcagtat tcatctcttt gttttttgtc actcttttct ctctaattgt 360
gtcatttgta ctgtttgaaa aatatttctt ctatnaaatt aaactaacct gccttaaaaa 420
aaaaaaaaaa aaaa 434

<210> 27
<211> 654

<212> DNA
<213> Homo sapiens

<220>
<221> misc_feature
<222> 505, 533, 563, 592, 613, 635, 638
<223> n = A,T,C or G

<400> 27
actagtccaa cacagtcaga aacattgttt tgaatcctct gtaaaccaag gcattaatct 60
taataaacca ggatccattt aggtaccact tgatataaaa aggatatcca taatgaatat 120
tttatactgc atcctttaca ttagccacta aatacgttat tgcttgatga agacctttca 180
cagaatccta tggattgcag catttcactt ggctacttca tacccatgcc ttaaagaggg 240
gcagtttctc aaaagcagaa acatgccgcc agttctcaag ttttcctcct aactccattt 300
gaatgtaagg gcagctggcc cccaatgtgg ggaggtccga acattttctg aattcccatt 360
ttcttgttcg cggctaaatg acagtttctg tcattactta gattccgatc tttcccaaag 420
gtgttgattt acaaagaggc cagctaatag cagaaatcat gaccctgaaa gagagatgaa 480
attcaagctg tgagccaggc agganctcag tatggcaaag gtcttgagaa tcngccattt 540
ggtacaaaaa aaattttaaa gcntttatgt tataccatgg aaccatagaa anggcaaggg 600
aattgttaag aanaatttta agtgtccaga cccanaanga aaaaaaaaaa aaaa 654

<210> 28
<211> 670
<212> DNA
<213> Homo sapiens

<220>
<221> misc_feature
<222> 101, 226, 274, 330, 385, 392, 397, 402, 452, 473, 476, 532,
534, 538, 550, 583, 595, 604, 613, 622, 643, 669
<223> n = A,T,C or G

<400> 28
cgtgtgcaca tactgggagg atttccacag ctgcacggtc acagccctta cggattgcca 60
ggaaggggcg aaagatatgt gggataaact gagaaaagaa nccaaaaacc tcaacatcca 120
aggcagctta ttcgaactct gcggcagcgg caacggggcg gcggggtccc tgctcccggc 180
gttcccggtg ctcctggtgt ctctctcggc agctttagcg acctgncttt ccttctgagc 240
gtggggccag ctccccccgc ggcgcccacc cacnctcact ccatgctccc ggaaatcgag 300
aggaagatca ttagttcttt ggggacgttn gtgattctct gtgatgctga aaaacactca 360
tatagggaat gtgggaaatc ctganctctt tnttatntcg tntgatttct tgtgttttat 420
ttgccaaaat gttaccaatc agtgaccaac cnagcacagc caaaaatcgg acntcngctt 480
tagtccgtct tcacacacag aataagaaaa cggcaaaccc accccacttt tnantttnat 540
tattactaan ttttttctgt tgggcaaaag aatctcagga acngccctgg ggccnccgta 600
ctanagttaa ccnagctagt tncatgaaaa atgatgggct ccncctcaat gggaaagcca 660
agaaaaagnc 670

<210> 29
<211> 551
<212> DNA
<213> Homo sapiens

<220>
<221> misc_feature
<222> 336, 474, 504, 511, 522, 523, 524, 540, 547
<223> n = A,T,C or G

<400> 29
actagtcctc cacagcctgt gaatccccct agacctttca agcatagtga gcggagaaga 60

agatctcagc gtttagccac cttacccatg cctgatgatt ctgtagaaaa ggtttcttct 120
ccctctccag ccactgatgg gaaagtattc tccatcagtt ctcaaaatca gcaagaatct 180
tcagtaccag aggtgcctga tgttgcacat ttgccacttg agaagctggg accctgtctc 240
cctcttgact taagtcgtgg ttcagaagtt acagcaccgg tagcctcaga ttcctcttac 300
cgtaatgaat gtcccagggc agaaaaagag gatacncaga tgcttccaaa tccttcttcc 360
aaagcaatag ctgatgggaa gaggagctcc agcagcagca ggaatatcga aaacagaaaa 420
aaaagtgaaa ttgggaagac aaaagctcaa cagcatttgg taaggagaaa aganaagatg 480
aggaaggaag agagaagaga gacnaagatc nctacggacc gnnncggaag aagaagaagn 540
aaaaaanaaa a 551

<210> 30
<211> 684
<212> DNA
<213> Homo sapiens

<220>
<221> misc_feature
<222> 545, 570, 606, 657, 684
<223> n = A,T,C or G

<400> 30
actagttcta tctggaaaaa gcccgggttg gaagaagctg tggagagtgc gtgtgcaatg 60
cgagactcat ttcttggaag catccctggc aaaaatgcag ctgagtacaa ggttatcact 120
gtgatagaac ctggactgct ttttgagata atagagatgc tgcagtctga agagacttcc 180
agcacctctc agttgaatga attaatgatg gcttctgagt caactttact ggctcaggaa 240
ccacgagaga tgactgcaga tgtaatcgag cttaaaggga aattcctcat caacttagaa 300
ggtggtgata ttcgtgaaga gtcttcctat aaagtaattg tcatgccgac tacgaaagaa 360
aaatgccccc gttgttggaa gtatacagcg ggagtcttca gatacactgt gtcctcgatg 420
tgcagaagtt gtcagtggga aaatagtatt aacagctcac tcgagcaaga accctcctga 480
cagtactggg ctagaagttt ggatggatta tttacaatat aggaaagaaa gccaagaatt 540
aggtnatgag tggatgagta aatggtggan gatggggaat tcaaatcaga attatggaag 600
aagttnttcc tgttactata gaaaggaatt atgtttattt acatgcagaa aatatanatg 660
tgtggtgtgt accgtggatg gaan 684

<210> 31
<211> 654
<212> DNA
<213> Homo sapiens

<220>
<221> misc_feature
<222> 326, 582, 651
<223> n = A,T,C or G

<400> 31
gcgcagaaaa ggaaccaata tttcagaaac aagcttaata ggaacagctg cctgtacatc 60
aacatcttct cagaatgacc cagaagttat catcgtggga gctggcgtgc ttggctctgc 120
tttggcagct gtgctttcca gagatggaag aaaggtgaca gtcattgaga gagacttaaa 180
agagcctgac agaatagttg gagaattcct gcagccgggt ggttatcatg ttctcaaaga 240
ccttggtctt ggagatacag tggaaggtct tgatgcccag gttgtaaatg gttacatgat 300
tcatgatcag ggaaagcaaa tcagangttc agattcctta ccctctgtca gaaaacaatc 360
aagtgcagag tggaagagct ttccatcacg gaagattcat catgagtctc cggaaagcag 420
ctatggcaga gcccaatgca aagtttattg aaggtgttgt gttacagtta ttagaggaag 480
atgatgttgt gatgggagtt cagtacaagg ataaagagac tgggagatat caaggaactc 540
catgctccac tgactgttgt tgcagatggg ctttctccca anttcaggaa aagcctggtc 600
tcaataaagt ttctgtatca ctcatttggt tggcttctta tgaagaatgc nccc 654

<210> 32

<211> 673
<212> DNA
<213> Homo sapiens

<220>
<221> misc_feature
<222> 376, 545, 627
<223> n = A,T,C or G

<400> 32
actagtgaag aaaaagaaat tctgatacgg gacaaaaatg ctcttcaaaa catcattctt 60
tatcacctga caccaggagt tttcattgga aaaggatttg aacctggtgt tactaacatt 120
ttaaagacca cacaaggaag caaaatcttt ctgaaagaag taaatgatac acttctggtg 180
aatgaattga aatcaaaaga atctgacatc atgacaacaa atggtgtaat tcatgttgta 240
gataaactcc tctatccagc agacacacct gttggaaatg atcaactgct ggaaatactt 300
aataaattaa tcaaatacat ccaaattaag tttgttcgtg gtagcacctt caaagaaatc 360
cccgtgactg tctatnagcc aattattaaa aaatacacca aaatcattga tgggagtgcc 420
tgtgggaaat aactgaaaaa gagaccgaga agaacgaatc attacaggtc ctgaaataaa 480
atacctagga tttctactgg aggtggagaa acagaagaac tctgaagaaa ttgttacaag 540
aagangtccc aaggtcacca aattcattga aggtggtgat ggtctttatt tgaagatgaa 600
gaaattaaaa gacgcttcag ggagacnccc catgaaggaa ttgccagcca caaaaaaatt 660
cagggattag aaa 673

<210> 33
<211> 673
<212> DNA
<213> Homo sapiens

<220>
<221> misc_feature
<222> 325, 419, 452, 532, 538, 542, 571, 600, 616, 651, 653, 672
<223> n = A,T,C or G

<400> 33
actagttatt tactttcctc cgcttcagaa ggtttttcag actgagagcc taagcatact 60
ggatctgttg tttcttttgg gtctcacctc atcagtgtgc atagtggcag aaattataaa 120
gaaggttgaa aggagcaggg aaaagatcca gaagcatgtt agttcgacat catcatcttt 180
tcttgaagta tgatgcatat tgcattattt tatttgcaaa ctaggaattg cagtctgagg 240
atcatttaga agggcaagtt caagaggata tgaagatttg agaacttttt aactattcat 300
tgactaaaaa tgaacattaa tgttnaagac ttaagacttt aacctgctgg cagtcccaaa 360
tgaaattatg caactttgat atcatattcc ttgatttaaa ttgggctttt gtgattgant 420
gaaactttat aaagcatatg gtcagttatt tnattaaaaa ggcaaaacct gaaccacctt 480
ctgcacttaa agaagtctaa cagtacaaat acctatctat cttagatgga tntatttntt 540
tntattttta aatattgtac tatttatggt nggtggggct ttcttactaa tacacaaatn 600
aatttatcat ttcaanggca ttctatttgg gtttagaagt tgattccaag nantgcatat 660
ttcgctactg tnt 673

<210> 34
<211> 684
<212> DNA
<213> Homo sapiens

<220>
<221> misc_feature
<222> 414, 472, 480, 490, 503, 507, 508, 513, 523, 574, 575, 598,
659, 662, 675
<223> n = A,T,C or G

<400> 34
actagtttat tcaagaaaag aacttactga ttcctctgtt cctaaagcaa gagtggcagg 60
tgatcagggc tggtgtagca tccggttcct ttagtgcagc taactgcatt tgtcactgat 120
gaccaaggag gaaatcacta agacatttga gaagcagtgg tatgaacgtt cttggacaag 180
ccacagttct gagccttaac cctgtagttt gcacacaaga acgagctcca cctccccttc 240
ttcaggagga atctgtgcgg atagattggc tggacttttc aatggttctg ggttgcaagt 300
gggcactgtt atggctgggt atggagcgga cagccccagg aatcagagcc tcagcccggc 360
tgcctggttg gaaggtacag gtgttcagca ccttcggaaa aagggcataa agtngtgggg 420
gacaattctc agtccaagaa gaatgcattg accattgctg gctatttgct tncctagtan 480
gaattggatn catttttgac cangatnntt ctnctatgct ttnttgcaat gaaatcaaat 540
cccgcattat ctacaagtgg tatgaagtcc tgcnnccccc agagaggctg ttcaggcnat 600
gtcttccaag ggcagggtgg gttacaccat tttacctccc ctctcccccc agattatgna 660
cncagaagga atttntttcc tccc 684

<210> 35
<211> 614
<212> DNA
<213> Homo sapiens

<220>
<221> misc_feature
<222> 17, 20, 152, 223, 267, 287, 304, 306, 316, 319, 321, 355,
365, 382, 391, 407, 419, 428, 434, 464, 467, 477, 480, 495,
499, 505, 515, 516, 522, 524, 527, 542, 547, 549, 567, 572,
576, 578
<223> n = A,T,C or G

<400> 35
actagtccaa cgcgttngcn aatattcccc tggtagccta cttccttacc cccgaatatt 60
ggtaagatcg agcaatggct tcaggacatg ggttctcttc tcctgtgatc attcaagtgc 120
tcactgcatg aagactggct tgtctcagtg tntcaacctc accagggctg tctcttggtc 180
cacacctcgc tccctgttag tgccgtatga cagcccccat canatgacct tggccaagtc 240
acggtttctc tgtggtcaat gttggtnggc tgattggtgg aaagtanggt ggaccaaagg 300
aagncncgtg agcagncanc nccagttctg caccagcagc gcctccgtcc tactngggtg 360
ttccngtttc tcctggccct gngtgggcta nggcctgatt cgggaanatg cctttgcang 420
gaaggganga taantgggat ctaccaattg attctggcaa aacnatntct aagattnttn 480
tgctttatgt ggganacana tctanctctc atttnntgct gnanatnaca ccctactcgt 540
gntcgancnc gtcttcgatt ttcgganaca cnccantnaa tactggcgtt ctgttgttaa 600
aaaaaaaaaa aaaa 614

<210> 36
<211> 686
<212> DNA
<213> Homo sapiens

<220>
<221> misc_feature
<222> 222, 224, 237, 264, 285, 548, 551, 628, 643, 645, 665, 674
<223> n = A,T,C or G

<400> 36
gtggctggcc cggttctccg cttctcccca tcccctactt tcctccctcc ctccctttcc 60
ctccctcgtc gactgttgct tgctggtcgc agactccctg acccctccct cacccctccc 120
taacctcggt gccaccggat tgcccttctt ttcctgttgc ccagcccagc cctagtgtca 180
gggcgggggc ctggagcagc ccgaggcact gcagcagaag ananaaaaga cacgacnaac 240
ctcagctcgc cagtccggtc gctngcttcc cgccgcatgg caatnagaca gacgccgctc 300
acctgctctg ggcacacgcg acccgtggtt gatttggcct tcagtggcat cacccttatg 360
ggtatttctt aatcagcgct tgcaaagatg gttaacctat gctacgccag ggagatacag 420

gagactggat tggaacattt ttggggtcta aaggtctgtt tggggtgcaa cactgaataa 480
ggatgccacc aaagcagcta cagcagctgc agatttcaca gcccaagtgt gggatgctgt 540
ctcagganat naattgataa cctggctcat aacacattgt caagaatgtg gatttcccca 600
ggatattatt atttgtttac cggggganag gataactgtt tcncntattt taattgaaca 660
aactnaaaca aaanctaagg aaatcc 686

<210> 37
<211> 681
<212> DNA
<213> Homo sapiens

<220>
<221> misc_feature
<222> 7, 10, 11, 19, 25, 32, 46, 53, 77, 93, 101, 103, 109, 115,
123, 128, 139, 157, 175, 180, 192, 193, 194, 212, 218, 226,
227, 233, 240, 241, 259, 260, 267, 289, 296, 297, 298, 312,
313, 314, 320, 325, 330, 337, 345, 346, 352, 353, 356
<223> n = A,T,C or G

<221> misc_feature
<222> 382, 385, 400, 427, 481, 484, 485, 491, 505, 515, 533, 542,
544, 554, 557, 560, 561, 564, 575, 583, 589, 595, 607, 619,
628, 634, 641, 645, 658, 670
<223> n = A,T,C or G

<400> 37
gagacanacn naacgtcang agaanaaaag angcatggaa cacaanccag gcncgatggc 60
caccttccca ccagcancca gcgcccccca gcngccccca ngnccggang accangactc 120
cancctgnat caatctganc tctattcctg gcccatncct acctcggagg tggangccgn 180
aaaggtcgca cnnncagaga agctgctgcc ancaccancc gccccnnccc tgncgggctn 240
nataggaaac tggtgaccnn gctgcanaat tcatacagga gcacgcgang ggcacnnnct 300
cacactgagt tnnngatgan gcctnaccan ggacctnccc cagcnnattg annacnggac 360
tgcggaggaa ggaagacccc gnacnggatc ctggccggcn tgccaccccc ccacccctag 420
gattatnccc cttgactgag tctctgaggg gctacccgaa cccgcctcca ttccctacca 480
natnntgctc natcgggact gacangctgg ggatnggagg ggctatcccc cancatcccc 540
tnanaccaac agcnacngan natnggggct ccccngggtc ggngcaacnc tcctncaccc 600
cggcgcnggc cttcggtgnt gtcctccntc aacnaattcc naaanggcgg gccccccngt 660
ggactcctcn ttgttccctc c 681

<210> 38
<211> 687
<212> DNA
<213> Homo sapiens

<220>
<221> misc_feature
<222> 3, 30, 132, 151, 203, 226, 228, 233, 252, 264, 279, 306,
308, 320, 340, 347, 380, 407, 429, 437, 440, 445, 448, 491,
559, 567, 586, 589, 593, 596, 603, 605, 606, 609, 626, 639,
655, 674, 682
<223> n = A,T,C or G

<400> 38
canaaaaaaa aaaacatggc cgaaaccagn aagctgcgcg atggcgccac ggcccctctt 60
ctcccggcct gtgtccggaa ggtttccctc cgaggcgccc cggctcccgc aagcggagga 120
gagggcggga cntgccgggg ccggagctca naggccctgg ggccgctctg ctctcccgcc 180
atcgcaaggg cggcgctaac ctnaggcctc cccgcaaagg tccccnangc ggnggcggcg 240
gggggctgtg anaaccgcaa aaanaacgct gggcgcgcng cgaacccgtc cacccccgcg 300

aaggananac ttccacagan gcagcgtttc cacagcccan agccacnttt ctagggtgat 360
gcaccccagt aagttcctgn cggggaagct caccgctgtc aaaaaanctc ttcgctccac 420
cggcgcacna aggggangan ggcangangc tgccgcccgc acaggtcatc tgatcacgtc 480
gcccgcccta ntctgctttt gtgaatctcc actttgttca accccacccg ccgttctctc 540
ctccttgcgc cttcctctna ccttaanaac cagcttcctc tacccnatng tanttnctct 600
gcncnngtng aaattaattc ggtccnccgg aacctcttnc ctgtggcaac tgctnaaaga 660
aactgctgtt ctgnttactg cngtccc 687

<210> 39
<211> 695
<212> DNA
<213> Homo sapiens

<220>
<221> misc_feature
<222> 300, 401, 423, 429, 431, 437, 443, 448, 454, 466, 492, 515,
523, 524, 536, 538, 541, 552, 561, 566, 581, 583, 619, 635,
636, 641, 649, 661, 694
<223> n = A,T,C or G

<400> 39
actagtctgg cctacaatag tgtgattcat gtaggacttc tttcatcaat tcaaaacccc 60
tagaaaaacg tatacagatt atataagtag ggataagatt tctaacattt ctgggctctc 120
tgacccctgc gctagactgt ggaaagggag tattattata gtatacaaca ctgctgttgc 180
cttattagtt ataacatgat aggtgctgaa ttgtgattca caatttaaaa acactgtaat 240
ccaaactttt ttttttaact gtagatcatg catgtgaatg ttaatgttaa tttgttcaan 300
gttgttatgg gtagaaaaaa ccacatgcct taaaatttta aaaagcaggg cccaaactta 360
ttagtttaaa attaggggta tgtttccagt ttgttattaa ntggttatag ctctgtttag 420
aanaaatcna ngaacangat ttngaaantt aagntgacat tatttnccag tgacttgtta 480
atttgaaatc anacacggca ccttccgttt tggtnctatt ggnntttgaa tccaancngg 540
ntccaaatct tnttggaaac ngtccnttta acttttttac nanatcttat ttttttattt 600
tggaatggcc ctatttaang ttaaaagggg ggggnnccac naccattcnt gaataaaact 660
naatatatat ccttggtccc ccaaaattta aggng 695

<210> 40
<211> 674
<212> DNA
<213> Homo sapiens

<220>
<221> misc_feature
<222> 403, 428, 432, 507, 530, 543, 580, 583, 591, 604, 608, 621,
624, 626, 639, 672
<223> n = A,T,C or G

<400> 40
actagtagtc agttgggagt ggttgctata ccttgacttc atttatatga atttccactt 60
tattaaataa tagaaaagaa aatcccggtg cttgcagtag agttatagga cattctatgc 120
ttacagaaaa tatagccatg attgaaatca aatagtaaag gctgttctgg ctttttatct 180
tcttagctca tcttaaataa gtagtacact tgggatgcag tgcgtctgaa gtgctaatca 240
gttgtaacaa tagcacaaat cgaacttagg atgtgtttct tctcttctgt gtttcgattt 300
tgatcaattc tttaattttg ggaacctata atacagtttt cctattcttg gagataaaaa 360
ttaaatggat cactgatatt taagtcattc tgcttctcat ctnaatattc catattctgt 420
attagganaa antacctccc agcacagccc cctctcaaac cccacccaaa accaagcatt 480
tggaatgagt ctcctttatt tccgaantgt ggatggtata acccatatcn ctccaatttc 540
tgnttgggtt gggtattaat ttgaactgtg catgaaaagn ggnaatcttt nctttgggtc 600
aaantttncc ggttaatttg nctngncaaa tccaatttnc tttaagggtg tctttataaa 660
atttgctatt cngg 674

<210> 41
<211> 657
<212> DNA
<213> Homo sapiens

<220>
<221> misc_feature
<222> 243, 247, 251, 261, 267, 272, 298, 312, 315, 421, 432, 434,
501, 524, 569, 594, 607, 650
<223> n = A,T,C or G

<400> 41
   gaaacatgca agtaccacac actgtttgaa ttttgcacaa aaagtgactg tagggatcag 60
   gtgatagccc cggaatgtac agtgtcttgg tgcaccaaga tgccttctaa aggctgacat 120
   accttgggac cctaatgggg cagagagtat agccctagcc cagtggtgac atgaccactc 180
   cctttgggag gctgaagtta aagggaatgg tatgtgtttt ctcatggaag cagcacatga 240
   atnggtnaca ngatgttaaa ntaaggntct antttgggtg tcttgtcatt tgaaaaantg 300
   acacactcct ancanctggt aaaggggtgc tggaagccat ggaagaactc taaaaacatt 360
   agcatgggct gatctgatta cttcctggca tcccgctcac ttttatggga agtcttatta 420
   naaggatggg ananttttcc atatccttgc tgttggaact ctggaacact ctctaaattt 480
   ccctctatta aaaatcactg nccttactac acttcctcct tganggaata gaaatggacc 540
   tttctctgac ttagttcttg gcatgggang cagcccaaat taaaatctga cttntccggt 600
   ttctccngaa ctcacctact tgaattggta aaacctcctt tggaattagn aaaaacc 657

<210> 42
<211> 389
<212> DNA
<213> Homo sapiens

<220>
<221> misc_feature
<222> 179, 317, 320
<223> n = A,T,C or G

<400> 42
actagtgctg aggaatgtaa acaagtttgc tgggccttgc gagacttcac caggttgttt 60
cgatagctca cactcctgca ctgtgcctgt cacccaggaa tgtctttttt aattagaaga 120
caggaagaaa acaaaaacca gactgtgtcc cacaatcaga aacctccgtt gtggcagang 180
ggccttcacc gccaccaggg tgtcccgcca gacagggaga gactccagcc ttctgaggcc 240
atcctgaaga attcctgttt gggggttgtg aaggaaaatc acccggattt aaaaagatgc 300
tgttgcctgc ccgcgtngtn gggaagggac tggtttcctg gtgaatttct taaaagaaaa 360
atattttaag ttaagaaaaa aaaaaaaaa 389

<210> 43
<211> 279
<212> DNA
<213> Homo sapiens

<400> 43
   actagtgaca agctcctggt cttgagatgt cttctcgtta aggagatggg ccttttggag 60
   gtaaaggata aaatgaatga gttctgtcat gattcactat tctagaactt gcatgacctt 120
   tactgtgtta gctctttgaa tgttcttgaa attttagact ttctttgtaa acaaataata 180
   tgtccttatc attgtataaa agctgttatg tgcaacagtg tggagatcct tgtctgattt 240
   aataaaatac ttaaacactg aaaaaaaaaa aaaaaaaaa 279

<210> 44
<211> 449

<212> DNA
<213> Homo sapiens

<220>
<221> misc_feature
<222> 245, 256, 264, 266, 273, 281, 323, 325, 337, 393
<223> n = A,T,C or G

<400> 44
actagtagca tcttttctac aacgttaaaa ttgcagaagt agcttatcat taaaaaacaa 60
caacaacaac aataacaata aatcctaagt gtaaatcagt tattctaccc cctaccaagg 120
atatcagcct gttttttccc ttttttctcc tgggaataat tgtgggcttc ttcccaaatt 180
tctacagcct ctttcctctt ctcatgcttg agcttccctg tttgcacgca tgcgttgtgc 240
aagantgggc tgtttngctt ggantncggt ccnagtggaa ncatgctttc ccttgttact 300
gttggaagaa actcaaacct tcnancccta ggtgttncca ttttgtcaag tcatcactgt 360
atttttgtac tggcattaac aaaaaaagaa atnaaatatt gttccattaa actttaataa 420
aactttaaaa gggaaaaaaa aaaaaaaaa 449

<210> 45
<211> 559
<212> DNA
<213> Homo sapiens

<220>
<221> misc_feature
<222> 263
<223> n = A,T,C or G

<400> 45
actagtgtgg gggaatcacg gacacttaaa gtcaatctgc gaaataattc ttttattaca 60
cactcactga agtttttgag tcccagagag ccattctatg tcaaacattc caagtactct 120
ttgagagccc agcattacat caacatgccc gtgcagttca aaccgaagtc cgcaggcaaa 180
tttgaagctt tgcttgtcat tcaaacagat gaaggcaaga gtattgctat tcgactaatt 240
ggtgaagctc ttggaaaaaa ttnactagaa tactttttgt gttaagttaa ttacataagt 300
tgtattttgt taactttatc tttctacact acaattatgc ttttgtatat atattttgta 360
tgatggatat ctataattgt agattttgtt tttacaagct aatactgaag actcgactga 420
aatattatgt atctagccca tagtattgta cttaactttt acagggtgaa aaaaaaattc 480
tgtgtttgca ttgattatga tattctgaat aaatatggga atatatttta atgtgggtaa 540
aaaaaaaaaa aaaaaggaa 559

<210> 46
<211> 731
<212> DNA
<213> Homo sapiens

<220>
<221> misc_feature
<222> 270, 467, 477, 502, 635, 660, 671, 688, 695, 697, 725
<223> n = A,T,C or G

<400> 46
actagttcta gtaccatggc tgtcatagat gcaaccatta tattccattt agtttcttcc 60
tcaggttccc taacaattgt ttgaaactga atatatatgt ttatgtatgt gtgtgtgttc 120
actgtcatgt atatggtgta tatgggatgt gtgcagtttt cagttatata tatattcata 180
tatacatatg catatatatg tataatatac atatatacat gcatacactt gtataatata 240
catatatata cacatatatg cacacatatn atcactgagt tccaaagtga gtctttattt 300
ggggcaattg tattctctcc ctctgtctgc tcactgggcc tttgcaagac atagcaattg 360
cttgatttcc tttggataag agtcttatct tcggcactct tgactctagc cttaacttta 420

gatttctatt ccagaatacc tctcatatct atcttaaaac ctaaganggg taaagangtc 480
ataagattgt agtatgaaag antttgctta gttaaattat atctcaggaa actcattcat 540
ctacaaatta aattgtaaaa tgatggtttg ttgtatctga aaaaatgttt agaacaagaa 600
atgtaactgg gtacctgtta tatcaaagaa cctcnattta ttaagtctcc tcatagccan 660
atccttatat ngccctctct gacctgantt aatananact tgaataatga atagttaatt 720
taggnttggg c 731

<210> 47
<211> 640
<212> DNA
<213> Homo sapiens

<220>
<221> misc_feature
<222> 5, 28, 106, 153, 158, 173, 176, 182, 189, 205, 210, 214,
225, 226, 229, 237, 260, 263, 269, 277, 281, 282, 322, 337,
338, 354, 365, 428, 441, 443, 456, 467, 476, 484, 503, 508,
554, 567, 575, 579, 588, 601, 606, 609, 611, 621, 636
<223> n = A,T,C or G

<400> 47
tgcgngccgg tttggccctt ctttgtanga cactttcatc cgccctgaaa tcttcccgat 60
cgttaataac tcctcaggtc cctgcctgca cagggttttt tcttantttg ttgcctaaca 120
gtacaccaaa tgtgacatcc tttcaccaat atngattnct tcataccaca tcntcnatgg 180
anacgactnc aacaattttt tgatnacccn aaanactggg ggctnnaana agtacantct 240
ggagcagcat ggacctgtcn gcnactaang gaacaanagt nntgaacatt tacacaacct 300
ttggtatgtc ttactgaaag anagaaacat gcttctnncc ctagaccacg aggncaaccg 360
caganattgc caatgccaag tccgagcggt tagatcaggt aatacattcc atggatgcat 420
tacatacntt gtccccgaaa nanaagatgc cctaanggct tcttcanact ggtccngaaa 480
acanctacac ctggtgcttg ganaacanac tctttggaag atcatctggc acaagttccc 540
cccagtgggt tttnccttgg cacctanctt accanatcna ttcggaancc attctttgcc 600
ntggcnttnt nttgggacca ntcttctcac aactgnaccc 640

<210> 48
<211> 257
<212> DNA
<213> Homo sapiens

<400> 48
actagtatat gaaaatgtaa atatcacttg tgtactcaaa caaaagttgg tcttaagctt 60
ccaccttgag cagccttgga aacctaacct gcctctttta gcataatcac attttctaaa 120
tgattttctt tgttcctgaa aaagtgattt gtattagttt tacatttgtt ttttggaaga 180
ttatatttgt atatgtatca tcataaaata tttaaataaa aagtatcttt agagtgaaaa 240
aaaaaaaaaa aaaaaaa 257

<210> 49
<211> 652
<212> DNA
<213> Homo sapiens

<220>
<221> misc_feature
<222> 410, 428, 496, 571, 647
<223> n = A,T,C or G

<400> 49
actagttcag atgagtggct gctgaagggg cccccttgtc attttcatta taacccaatt 60
tccacttatt tgaactctta agtcataaat gtataatgac ttatgaatta gcacagttaa 120

gttgacacta gaaactgccc atttctgtat tacactatca aataggaaac attggaaaga 180
tggggaaaaa aatcttattt taaaatggct tagaaagttt tcagattact ttgaaaattc 240
taaacttctt tctgtttcca aaacttgaaa atatgtagat ggactcatgc attaagactg 300
ttttcaaagc tttcctcaca tttttaaagt gtgattttcc ttttaatata catatttatt 360
ttctttaaag cagctatatc ccaacccatg actttggaga tatacctatn aaaccaatat 420
aacagcangg ttattgaagc agctttctca aatgttgctt cagatgtgca agttgcaaat 480
tttattgtat ttgtanaata caatttttgt tttaaactgt atttcaatct atttctccaa 540
gatgcttttc atatagagtg aaatatccca ngataactgc ttctgtgtcg tcgcatttga 600
cgcataactg cacaaatgaa cagtgtatac ctcttggttg tgcattnacc cc 652

<210> 50
<211> 650
<212> DNA
<213> Homo sapiens

<220>
<221> misc_feature
<222> 237, 270, 311, 443, 454, 488, 520, 535, 539, 556, 567, 594,
603, 634
<223> n = A,T,C or G

<400> 50
ttgcgctttg atttttttag ggcttgtgcc ctgtttcact tatagggtct agaatgcttg 60
tgttgagtaa aaaggagatg cccaatattc aaagctgcta aatgttctct ttgccataaa 120
gactccgtgt aactgtgtga acacttggga tttttctcct ctgtcccgag gtcgtcgtct 180
gctttctttt ttgggttctt tctagaagat tgagaaatgc atatgacagg ctgagancac 240
ctccccaaac acacaagctc tcagccacan gcagcttctc cacagcccca gcttcgcaca 300
ggctcctgga nggctgcctg ggggaggcag acatgggagt gccaaggtgg ccagatggtt 360
ccaggactac aatgtcttta tttttaactg tttgccactg ctgccctcac ccctgcccgg 420
ctctggagta ccgtctgccc canacaagtg ggantgaaat gggggtgggg gggaacactg 480
attcccantt agggggtgcc taactgaaca gtagggatan aaggtgtgaa cctgngaant 540
gcttttataa attatnttcc ttgttanatt tattttttaa tttaatctct gttnaactgc 600
ccngggaaaa ggggaaaaaa aaaaaaaaat tctntttaaa cacatgaaca 650

<210> 51
<211> 545
<212> DNA
<213> Homo sapiens

<220>
<221> misc_feature
<222> 66, 159, 195, 205, 214, 243, 278, 298, 306, 337, 366, 375,
382, 405, 446, 477, 492, 495, 503, 507, 508, 521, 537
<223> n = A,T,C or G

<400> 51
tggcgtgcaa ccagggtagc tgaagtttgg gtctgggact ggagattggc cattaggcct 60
cctganattc cagctccctt ccaccaagcc cagtcttgct acgtggcaca gggcaaacct 120
gactcccttt gggcctcagt ttcccctccc cttcatgana tgaaaagaat actacttttt 180
cttgttggtc taacnttgct ggacncaaag tgtngtcatt attgttgtat tgggtgatgt 240
gtncaaaact gcagaagctc actgcctatg agaggaanta agagagatag tggatganag 300
ggacanaagg agtcattatt tggtatagat ccacccntcc caacctttct ctcctcagtc 360
cctgcncctc atgtntctgg tntggtgagt cctttgtgcc accanccatc atgctttgca 420
ttgctgccat cctgggaagg gggtgnatcg tctcacaact tgttgtcatc gtttganatg 480
catgctttct tnatnaaaca aanaaannaa tgtttgacag ngtttaaaat aaaaaanaaa 540
caaaa 545

<210> 52

<211> 678
<212> DNA
<213> Homo sapiens

<220>
<221> misc_feature
<222> 98, 119, 121, 131, 136, 139, 140, 142, 143, 163, 168, 172,
176, 184, 189, 190, 191, 200, 201, 205, 207, 221, 223, 229,
230, 237, 240, 241, 255, 264, 266, 267, 276, 280, 288, 289,
291, 297, 301, 306, 308, 314, 315, 326, 332, 335, 337
<223> n = A,T,C or G

<221> misc_feature
<222> 339, 341, 343, 344, 345, 347, 350, 355, 356, 358, 362, 363,
372, 379, 395, 397, 398, 400, 403, 412, 414, 421, 423, 431,
435, 438, 439, 450, 457, 463, 467, 471, 474, 480, 483, 484,
487, 490, 491, 492, 493, 499, 500, 504, 508, 518, 536
<223> n = A,T,C or G

<221> misc_feature
<222> 538, 549, 551, 552, 554, 556, 557, 562, 563, 567, 571, 572,
576, 579, 590, 592, 595, 598, 606, 609, 613, 620, 622, 624,
626, 631, 634, 638, 641, 647, 654, 660, 661, 674
<223> n = A,T,C or G

<400> 52
actagtagaa gaactttgcc gcttttgtgc ctctcacagg cgcctaaagt cattgccatg 60
ggaggaagac gatttggggg gggagggggg gggggcangg tccgtggggc tttccctant 120
ntatctccat ntccantgnn cnntgtcgcc tcttccctcg tcncattnga anttantccc 180
tggnccccnn nccctctccn ncctncncct cccccctccg ncncctccnn ctttttntan 240
ncttccccat ctccntcccc cctnanngtc ccaacnccgn cagcaatnnc ncacttnctc 300
nctccncncc tccnnccgtt cttctnttct cnacntntnc ncnnntnccn tgccnntnaa 360
annctctccc cnctgcaanc gattctctcc ctccncnnan ctntccactc cntncttctc 420
ncncgctcct nttcntcnnc ccacctctcn ccttcgnccc cantacnctc nccncccctn 480
cgnntcnttn nnntcctcnn accncccncc tcccttcncc cctcttctcc ccggtntntc 540
tctctcccnc nncncnncct cnncccntcc nngcgnccnt ttccgccccn cnccnccntt 600
ccttcntcnc cantccatcn cntntnccat nctncctncc nctcacnccc gctncccccn 660
ntctctttca cacngtcc 678

<210> 53
<211> 502
<212> DNA
<213> Homo sapiens

<220>
<221> misc_feature
<222> 139, 146, 215, 217, 257, 263, 289, 386, 420, 452, 457, 461,
466, 482, 486
<223> n = A,T,C or G

<400> 53
tgaagatcct ggtgtcgcca tgggccgccg ccccgcccgt tgttaccggt attgtaagaa 60
caagccgtac ccaaagtctc gcttctgccg aggtgtccct gatgccaaaa ttcgcatttt 120
tgacctgggg cggaaaaang caaaantgga tgagtctccg ctttgtggcc acatggtgtc 180
agatcaatat gagcagctgt cctctgaagc cctgnangct gcccgaattt gtgccaataa 240
gtacatggta aaaagtngtg gcnaagatgc ttccatatcc gggtgcggnt ccaccccttc 300
cacgtcatcc gcatcaacaa gatgttgtcc tgtgctgggg ctgacaggct cccaacaggc 360
atgcgaagtg cctttggaaa acccanggca ctgtggccag ggttcacatt gggccaattn 420

atcatgttca tccgcaccaa ctgcagaaca angaacntgt naattnaagc cctgcccagg 480
gncaanttca aatttcccgg cc 502

<210> 54
<211> 494
<212> DNA
<213> Homo sapiens

<220>
<221> misc_feature
<222> 431, 442, 445
<223> n = A,T,C or G

<400> 54
actagtccaa gaaaaatatg cttaatgtat attacaaagg ctttgtatat gttaacctgt 60
tttaatgcca aaagtttgct ttgtccacaa tttccttaag acctcttcag aaagggattt 120
gtttgcctta atgaatactg ttgggaaaaa acacagtata atgagtgaaa agggcagaag 180
caagaaattt ctacatctta gcgactccaa gaagaatgag tatccacatt tagatggcac 240
attatgagga ctttaatctt tccttaaaca caataatgtt ttcttttttc ttttattcac 300
atgatttcta agtatatttt tcatgcagga cagtttttca accttgatgt acagtgactg 360
tgttaaattt ttctttcagt ggcaacctct ataatcttta aaatatggtg agcatcttgt 420
ctgttttgaa ngggatatga cnatnaatct atcagatggg aaatcctgtt tccaagttag 480
aaaaaaaaaa aaaa 494

<210> 55
<211> 606
<212> DNA
<213> Homo sapiens

<220>
<221> misc_feature
<222> 375, 395, 511, 542, 559, 569, 578, 581
<223> n = A,T,C or G

<400> 55
actagtaaaa agcagcattg ccaaataatc cctaattttc cactaaaaat ataatgaaat 60
gatgttaagc tttttgaaaa gtttaggtta aacctactgt tgttagatta atgtatttgt 120
tgcttccctt tatctggaat gtggcattag cttttttatt ttaaccctct ttaattctta 180
ttcaattcca tgacttaagg ttggagagct aaacactggg atttttggat aacagactga 240
cagttttgca taattataat cggcattgta catagaaagg atatggctac cttttgttaa 300
atctgcactt tctaaatatc aaaaaaggga aatgaagtat aaatcaattt ttgtataatc 360
tgtttgaaac atgantttta tttgcttaat attanggctt tgcccttttc tgttagtctc 420
ttgggatcct gtgtaaaact gttctcatta aacaccaaac agttaagtcc attctctggt 480
actagctaca aattccgttt catattctac ntaacaattt aaattaactg aaatatttct 540
anatggtcta cttctgtcnt ataaaaacna aacttgantt nccaaaaaaa aaaaaaaaaa 600
aaaaaa 606

<210> 56
<211> 183
<212> DNA
<213> Homo sapiens

<400> 56
actagtatat ttaaacttac aggcttattt gtaatgtaaa ccaccatttt aatgtactgt 60
aattaacatg gttataatac gtacaatcct tccctcatcc catcacacaa ctttttttgt 120
gtgtgataaa ctgattttgg tttgcaataa aaccttgaaa aataaaaaaa aaaaaaaaaa 180
aaa 183

<210> 57
<211> 622
<212> DNA
<213> Homo sapiens

<220>
<221> misc_feature
<222> 358, 368, 412, 414, 425, 430, 453, 455, 469, 475, 495, 499,
529, 540, 564, 575, 590
<223> n = A,T,C or G

<400> 57
actagtcact actgtcttct ccttgtagct aatcaatcaa tattcttccc ttgcctgtgg 60
gcagtggaga gtgctgctgg gtgtacgctg cacctgccca ctgagttggg gaaagaggat 120
aatcagtgag cactgttctg ctcagagctc ctgatctacc ccacccccta ggatccagga 180
ctgggtcaaa gctgcatgaa accaggccct ggcagcaacc tgggaatggc tggaggtggg 240
agagaacctg acttctcttt ccctctccct cctccaacat tactggaact ctatcctgtt 300
agggatcttc tgagcttgtt tccctgctgg gtgggacaga agacaaagga gaagggangg 360
tctacaanaa gcagcccttc tttgtcctct ggggttaatg agcttgacct ananttcatg 420
gaganaccan aagcctctga tttttaattt ccntnaaatg tttgaagtnt atatntacat 480
atatatattt ctttnaatnt ttgagtcttt gatatgtctt aaaatccant ccctctgccn 540
gaaacctgaa ttaaaaccat gaanaaaaat gtttncctta aagatgttan taattaattg 600
aaacttgaaa aaaaaaaaaa aa 622

<210> 58
<211> 433
<212> DNA
<213> Homo sapiens

<400> 58
gaacaaattc tgattggtta tgtaccgtca aaagacttga agaaatttca tgattttgca 60
gtgtggaagc gttgaaaatt gaaagttact gcttttccac ttgctcatat agtaaaggga 120
tcctttcagc tgccagtgtt gaataatgta tcatccagag tgatgttatc tgtgacagtc 180
accagcttta agctgaacca ttttatgaat accaaataaa tagacctctt gtactgaaaa 240
catatttgtg actttaatcg tgctgcttgg atagaaatat ttttactggt tcttctgaat 300
tgacagtaaa cctgtccatt atgaatggcc tactgttcta ttatttgttt tgacttgaat 360
ttatccacca aagacttcat ttgtgtatca tcaataaagt tgtatgtttc aactgaaaaa 420
aaaaaaaaaa aaa 433

<210> 59
<211> 649
<212> DNA
<213> Homo sapiens

<220>
<221> misc_feature
<222> 22, 190, 217, 430, 433, 484, 544, 550, 577, 583, 594
<223> n = A,T,C or G

<400> 59
   actagttatt atctgacttt cnggttataa tcattctaat gagtgtgaag tagcctctgg 60
   tgtcatttgg atttgcattt ctctgatgag tgatgctatc aagcaccttt gctggtgctg 120
   ttggccatat gtgtatgttc cctggagaag tgtctgtgct gagccttggc ccacttttta 180
   attaggcgtn tgtcttttta ttactgagtt gtaaganttc tttatatatt ctggattcta 240
   gacccttatc agatacatgg tttgcaaata ttttctccca ttctgtgggt tgtgttttca 300
   ctttatcgat aatgtcctta gacatataat aaatttgtat tttaaaagtg acttgatttg 360
   ggctgtgcaa ggtgggctca cgcttgtaat cccagcactt tgggagactg aggtgggtgg 420
   atcatatgan gangctagga gttcgaggtc agcctggcca gcatagcgaa aacttgtctc 480
   tacnaaaaat acaaaaatta gtcaggcatg gtggtgcacg tctgtaatac cagcttctca 540
   ggangctgan gcacaaggat cacttgaacc ccagaangaa gangttgcag tganctgaag 600
   atcatgccag ggcaacaaaa atgagaactt gtttaaaaaa aaaaaaaaa 649

<210> 60
<211> 423
<212> DNA
<213> Homo sapiens

<220>
<221> misc_feature
<222> 209, 222, 277, 389, 398
<223> n = A,T,C or G

<400> 60
actagttcag gccttccagt tcactgacaa acatggggaa gtgtgcccag ctggctggaa 60
acctggcagt gataccatca agcctgatgt ccaaaagagc aaagaatatt tctccaagca 120
gaagtgagcg ctgggctgtt ttagtgccag gctgcggtgg gcagccatga gaacaaaacc 180
tcttctgtat tttttttttc cattagtana acacaagact cngattcagc cgaattgtgg 240
tgtcttacaa ggcagggctt tcctacaggg ggtgganaaa acagcctttc ttcctttggt 300
aggaatggcc tgagttggcg ttgtgggcag gctactggtt tgtatgatgt attagtagag 360
caacccatta atcttttgta gtttgtatna aacttganct gagaccttaa acaaaaaaaa 420
aaa 423

<210> 61
<211> 423
<212> DNA
<213> Homo sapiens

<220>
<221> misc_feature
<222> 195, 285, 295, 329, 335, 340, 347, 367, 382, 383, 391, 396,
418
<223> n = A,T,C or G

<400> 61
cgggactgga atgtaaagtg aagttcggag ctctgagcac gggctcttcc cgccgggtcc 60
tccctcccca gaccccagag ggagaggccc accccgccca gccccgcccc agcccctgct 120
caggtctgag tatggctggg agtcgggggc cacaggcctc tagctgtgct gctcaagaag 180
actggatcag ggtanctaca agtggccggg ccttgccttt gggattctac cctgttccta 240
atttggtgtt ggggtgcggg gtccctggcc cccttttcca cactncctcc ctccngacag 300
caacctccct tggggcaatt gggcctggnt ctccncccgn tgttgcnacc ctttgttggt 360
ttaaggnctt taaaaatgtt annttttccc ntgccngggt taaaaaagga aaaaactnaa 420
aaa 423

<210> 62
<211> 683
<212> DNA
<213> Homo sapiens

<220>
<221> misc_feature
<222> 218, 291, 305, 411, 416, 441, 443, 453, 522, 523, 536, 542,
547, 566, 588, 592, 595, 603, 621, 628, 630, 632, 644, 645,
648, 655, 660, 672, 674, 676, 677, 683
<223> n = A,T,C or G

<400> 62

gctggagagg ggtacggact ttcttggagt tgtcccaggt tggaatgaga ctgaactcaa 60
gaagagaccc taagagactg gggaatggtt cctgccttca ggaaagtgaa agacgcttag 120
gctgtcaaca cttaaaggaa gtccccttga agcccagagt ggacagacta gacccattga 180
tggggccact ggccatggtc cgtggacaag acattccngt gggccatggc acaccggggg 240
ggatcaaaat gtgtacttgt ggggtctcgc cccttgccaa aaccaaacca ntcccactcc 300
tgtcnttgga ctttcttccc attccctcct ccccaaatgc acttcccctc ctccctctgc 360
ccctcctgtg tttttggaat tctgtttccc tcaaaattgt taatttttta nttttngacc 420
atgaacttat gtttggggtc nangttcccc ttnccaatgc atactaatat attaatggtt 480
atttattttt gaaatatttt ttaatgaact tggaaaaaat tnntggaatt tccttncttc 540
cnttttnttt gggggggtg ggggngtggg ttaaaatttt tttggaancc cnatnggaaa 600
ttnttacttg gggcccccct naaaaaantn anttccaatt cttnnatngc ccctnttccn 660
ctaaaaaaaa ananannaaa aan 683

<210> 63
<211> 731
<212> DNA
<213> Homo sapiens

<220>
<221> misc_feature
<222> 237, 249, 263, 288, 312, 317, 323, 326, 337, 352, 362, 370,
377, 400, 411, 414, 434, 436, 446, 457, 473, 486, 497, 498,
502, 512, 531, 546, 554, 563, 565, 566, 588, 597, 608, 611,
613, 615, 627, 632, 640, 641, 644, 654, 660, 663, 665
<223> n = A,T,C or G

<221> misc_feature
<222> 671, 678, 692, 697, 698, 699, 704, 705, 712, 714, 717, 718,
719, 723, 725, 730, 731
<223> n = A,T,C or G

<400> 63
   actagtcata aagggtgtgc gcgtcttcga cgtggcggtc ttggcgccac tgctgcgaga 60
   cccggccctg gacctcaagg tcatccactt ggtgcgtgat ccccgcgcgg tggcgagttc 120
   acggatccgc tcgcgccacg gcctcatccg tgagagccta caggtggtgc gcagccgaga 180
   ccgcgagctc accgcatgcc cttcttggag gccgcgggcc acaagcttgg cgcccanaaa 240
   gaaggcgtng ggggcccgca aantaccacg ctctgggcgc tatggaangt cctcttgcaa 300
   taatattggt tnaaaanctg canaanagcc cctgcanccc cctgaactgg gntgcagggc 360
   cncttacctn gtttggntgc ggttacaaag aacctgtttn ggaaaaccct nccnaaaacc 420
   ttccgggaaa attntncaaa tttttnttgg ggaattnttg ggtaaacccc ccnaaaatgg 480
   gaaacntttt tgccctnnaa antaaaccat tnggttccgg gggccccccc ncaaaaccct 540
   tttttntttt tttntgcccc cantnncccc ccggggcccc tttttttngg ggaaaanccc 600
   cccccctncc nanantttta aaagggnggg anaatttttn nttncccccc gggncccccn 660
   ggngntaaaa nggtttcncc cccccgaggg gnggggnnnc ctcnnaaacc cntntcnnna 720
   ccncnttttn n 731

<210> 64
<211> 313
<212> DNA
<213> Homo sapiens

<220>
<221> misc_feature
<222> 240
<223> n = A,T,C or G

<400> 64
actagttgtg caaaccacga ctgaagaaag acgaaaagtg ggaaataact tgcaacgtct 60

gttagagatg gttgctacac atgttgggtc tgtagagaaa catcttgagg agcagattgc 120
taaagttgat agagaatatg aagaatgcat gtcagaagat ctctcggaaa atattaaaga 180
gattagagat aagtatgaga agaaagctac tctaattaag tcttctgaag aatgaagatn 240
aaatgttgat catgtatata tatccatagt gaataaaatt gtctcagtaa agttgtaaaa 300
aaaaaaaaaa aaa 313

<210> 65
<211> 420
<212> DNA
<213> Homo sapiens

<220>
<221> misc_feature
<222> 400, 402, 403, 404, 405, 406, 409, 411, 412, 414, 415, 416
<223> n = A,T,C or G

<400> 65
actagttccc tggcaggcaa gggcttccaa ctgaggcagt gcatgtgtgg cagagagagg 60
caggaagctg gcagtggcag cttctgtgtc tagggagggg tgtggctccc tccttccctg 120
tctgggaggt tggagggaag aatctaggcc ttagcttgcc ctcctgccac ccttcccctt 180
gtagatactg ccttaacact ccctcctctc tcagctgtgg ctgccaccca agccaggttt 240
ctccgtgctc actaatttat ttccaggaaa ggtgtgtgga agacatgagc cgtgtataat 300
atttgtttta acattttcat tgcaagtatt gaccatcatc cttggttgtg tatcgttgta 360
acacaaatta atgatattaa aaagcatcca aacaaagccn annnnnaana nnannngaaa 420

<210> 66
<211> 676
<212> DNA
<213> Homo sapiens

<220>
<221> misc_feature
<222> 328, 454, 505, 555, 586, 612, 636, 641
<223> n = A,T,C or G

<400> 66
actagtttcc tatgatcatt aaactcattc tcagggttaa gaaaggaatg taaatttctg 60
cctcaatttg tacttcatca ataagttttt gaagagtgca gatttttagt caggtcttaa 120
aaataaactc acaaatctgg atgcatttct aaattctgca aatgtttcct ggggtgactt 180
aacaaggaat aatcccacaa tatacctagc tacctaatac atggagctgg ggctcaaccc 240
actgttttta aggatttgcg cttacttgtg gctgaggaaa aataagtagt tccgagggaa 300
gtagttttta aatgtgagct tatagatngg aaacagaata tcaacttaat tatggaaatt 360
gttagaaacc tgttctcttg ttatctgaat cttgattgca attactattg tactggatag 420
actccagccc attgcaaagt ctcagatatc ttanctgtgt agttgaattc cttggaaatt 480
ctttttaaga aaaaattgga gtttnaaaga aataaacccc tttgttaaat gaagcttggc 540
tttttggtga aaaanaatca tcccgcaggg cttattgttt aaaaanggaa ttttaagcct 600
ccctggaaaa anttgttaat taaatgggga aaatgntggg naaaaattat ccgttagggt 660
ttaaagggaa aactta 676

<210> 67
<211> 620
<212> DNA
<213> Homo sapiens

<220>
<221> misc_feature
<222> 419, 493, 519, 568, 605, 610

<223> n = A,T,C or G

<400> 67
caccattaaa gctgcttacc aagaacttcc ccagcatttt gacttccttg tttgatagct 60
gaattgtgag caggtgatag aagagccttt ctagttgaac atacagataa tttgctgaat 120
acattccatt taatgaaggg gttacatctg ttacgaagct actaagaagg agcaagagca 180
taggggaaaa aaatctgatc agaacgcatc aaactcacat gtgccccctc tactacaaac 240
agattgtagt gctgtggtgg tttattccgt tgtgcagaac ttgcaagctg agtcactaaa 300
cccaaagaga ggaaattata ggttagttaa acattgtaat cccaggaact aagtttaatt 360
cacttttgaa gtgttttgtt ttttattttt ggtttgtctg atttactttg ggggaaaang 420
ctaaaaaaaa agggatatca atctctaatt cagtgcccac taaaagttgt ccctaaaaag 480
tctttactgg aanttatggg actttttaag ctccaggtnt tttggtcctc caaattaacc 540
ttgcatgggc cccttaaaat tgttgaangg cattcctgcc tctaagtttg gggaaaattc 600
ccccnttttn aaaatttgga 620

<210> 68
<211> 551
<212> DNA
<213> Homo sapiens

<220>
<221> misc_feature
<222> 286, 464, 480, 501, 502, 518, 528, 533, 536, 537, 538, 539,
540, 541, 543, 544, 545, 547, 548, 549
<223> n = A,T,C or G

<400> 68
actagtagct ggtacataat cactgaggag ctatttctta acatgctttt atagaccatg 60
ctaatgctag accagtattt aagggctaat ctcacacctc cttagctgta agagtctggc 120
ttagaacaga cctctctgtg caataacttg tggccactgg aaatccctgg gccggcattt 180
gtattggggt tgcaatgact cccaagggcc aaaagagtta aaggcacgac tgggatttct 240
tctgagactg tggtgaaact ccttccaagg ctgagggggt cagtangtgc tctgggaggg 300
actcggcacc actttgatat tcaacaagcc acttgaagcc caattataaa attgttattt 360
tacagctgat ggaactcaat ttgaaccttc aaaactttgt tagtttatcc tattatattg 420
ttaaacctaa ttacatttgt ctagcattgg atttggttcc tgtngcatat gtttttttcn 480
cctatgtgct cccctccccc nnatcttaat ttaaaccnca attttgcnat tcnccnnnnn 540
nannnannna a 551

<210> 69
<211> 396
<212> DNA
<213> Homo sapiens

<220>
<221> misc_feature
<222> 235, 310, 323, 381
<223> n = A,T,C or G

<400> 69
cagaaatgga aagcagagtt ttcatttctg tttataaacg tctccaaaca aaaatggaaa 60
gcagagtttt cattaaatcc ttttaccttt tttttttctt ggtaatcccc tcaaataaca 120
gtatgtggga tattgaatgt taaagggata tttttttcta ttatttttat aattgtacaa 180
aattaagcaa atgttaaaag ttttatatgc tttattaatg ttttcaaaag gtatnataca 240
tgtgatacat tttttaagct tcagttgctt gtcttctggt actttctgtt atggcttttt 300
ggggagccan aaaccaatct acnatctctt tttgtttgcc aggacatgca ataaaattta 360
aaaaataaat aaaaactatt nagaaattga aaaaaa 396

<210> 70

<211> 536
<212> DNA
<213> Homo sapiens

<220>
<221> misc_feature
<222> 388, 446, 455
<223> n = A,T,C or G

<400> 70
actagtgcaa aagcaaatat aaacatcgaa aaggcgttcc tcacgttagc tgaagatatc 60
cttcgaaaga cccctgtaaa agagcccaac agtgaaaatg tagatatcag cagtggagga 120
ggcgtgacag gctggaagag caaatgctgc tgagcattct cctgttccat cagttgccat 180
ccactacccc gttttctctt cttgctgcaa aataaaccac tctgtccatt tttaactcta 240
aacagatatt tttgtttctc atcttaacta tccaagccac ctattttatt tgttctttca 300
tctgtgactg cttgctgact ttatcataat tttcttcaaa caaaaaaatg tatagaaaaa 360
tcatgtctgt gacttcattt ttaaatgnta cttgctcagc tcaactgcat ttcagttgtt 420
ttatagtcca gttcttatca acattnaaac ctatngcaat catttcaaat ctattctgca 480
aattgtataa gaataaaagt tagaatttaa caattaaaaa aaaaaaaaaa aaaaaa 536

<210> 71
<211> 865
<212> DNA
<213> Homo sapiens

<220>
<221> misc_feature
<222> 22, 35, 39, 56, 131, 138, 146, 183, 194, 197, 238, 269, 277,
282, 297, 316, 331, 336, 340, 341, 346, 349, 370, 376, 381,
382, 392, 396, 397, 401, 433, 444, 445, 454, 455, 469, 472,
477, 480, 482, 489, 497, 499, 511, 522, 526, 527
<223> n = A,T,C or G

<221> misc_feature
<222> 545, 553, 556, 567, 574, 580, 610, 613, 634, 638, 639, 663,
672, 689, 693, 694, 701, 704, 713, 723, 729, 732, 743, 744,
749, 761, 765, 767, 769, 772, 774, 780, 783, 788, 792, 803,
810, 824, 840, 848
<223> n = A,T,C or G

<400> 71
gacaaagcgt taggagaaga anagaggcag ggaanactnc ccaggcacga tggccncctt 60
cccaccagca accagcgccc cccaccagcc cccaggcccg gacgacgaag actccatcct 120
ggattaatct nacctctntc gcctgnccca ttcctacctc ggaggtggag gccggaaagg 180
tcncaccaag aganaanctg ctgccaacac caaccgcccc agccctggcg ggcacganag 240
gaaactggtg accaatctgc agaattctna gaggaanaag cnaggggccc cgcgctnaga 300
cagagctgga tatgangcca gaccatggac nctacncccn ncaatncana cgggactgcg 360
gaagatggan gacccncgac nngatcaggc cngctnncca nccccccacc cctatgaatt 420
attcccgctg aangaatctc tgannggctt ccannaaagc gcctccccnc cnaacgnaan 480
tncaacatng ggattanang ctgggaactg naaggggcaa ancctnnaat atccccagaa 540
acaanctctc ccnaanaaac tggggcncct catnggtggn accaactatt aactaaaccg 600
cacgccaagn aantataaaa gggggggccc tccncggnng accccctttt gtcccttaat 660
ganggttatc cnccttgcgt accatggtnc ccnnttctgt ntgnatgttt ccnctcccct 720
ccncctatnt cnagccgaac tcnnatttnc ccgggggtgc natcnantng tncncctttn 780
ttngttgncc cngccctttc cgncggaacn cgtttccccg ttantaacgg cacccggggn 840
aagggtgntt ggccccctcc ctccc 865

<210> 72

<211> 560
<212> DNA
<213> Homo sapiens

<220>
<221> misc_feature
<222> 83, 173, 183, 186, 209, 211, 215, 255, 321, 322, 323, 335,
344, 357, 361, 368, 394, 412, 415, 442, 455, 469, 472, 475,
487, 513, 522, 528, 531, 534, 546
<223> n = A,T,C or G

<400> 72
   cctggacttg tcttggttcc agaacctgac gacccggcga cggcgacgtc tcttttgact 60
   aaaagacagt gtccagtgct ccngcctagg agtctacggg gaccgcctcc cgcgccgcca 120
   ccatgcccaa cttctctggc aactggaaaa tcatccgatc ggaaaacttc gangaattgc 180
   tcnaantgct gggggtgaat gtgatgctna ngaanattgc tgtggctgca gcgtccaagc 240
   cagcagtgga gatcnaacag gagggagaca ctttctacat caaaacctcc accaccgtgc 300
   gcaccacaaa gattaacttc nnngttgggg aggantttga ggancaaact gtggatngga 360
   ngcctgtnaa aacctggtga aatgggagaa tganaataaa atggtctgtg ancanaaact 420
   cctgaaagga gaaggccccc anaactcctg gaccngaaaa actgacccnc cnatngggga 480
   actgatnctt gaaccctgaa cgggcgggat ganccttttt tnttgccncc naangggttc 540
   tttccntttc cccaaaaaaa 560

<210> 73
<211> 379
<212> DNA
<213> Homo sapiens

<220>
<221> misc_feature
<222> 8, 17, 18, 21, 26, 29, 30, 32, 53, 56, 67, 71, 81, 102, 104,
111, 112, 114, 119, 122, 124, 125, 134, 144, 146, 189, 190,
214, 215, 219, 220, 235, 237, 246, 280, 288, 302, 310, 313,
319, 322, 343, 353, 354
<223> n = A,T,C or G

<400> 73
ctggggancc ggcggtnngc nccatntcnn gncgcgaagg tggcaataaa aanccnctga 60
aaccgcncaa naaacatgcc naagatatgg acgaggaaga tngngctttc nngnacaanc 120
gnanngagga acanaacaaa ctcnangagc tctcaagcta atgccgcggg gaaggggccc 180
ttggccacnn gtggaattaa gaaatctggc aaanngtann tgttccttgt gcctnangag 240
ataagngacc ctttatttca tctgtattta aacctctctn ttccctgnca taacttcttt 300
tnccacgtan agntggaant anttgttgtc ttggactgtt gtncatttta gannaaactt 360
ttgttcaaaa aaaaaataa 379

<210> 74
<211> 437
<212> DNA
<213> Homo sapiens

<220>
<221> misc_feature
<222> 145, 355
<223> n = A,T,C or G

<400> 74
actagttcag actgccacgc caaccccaga aaatacccca catgccagaa aagtgaagtc 60
ctaggtgttt ccatctatgt ttcaatctgt ccatctacca ggcctcgcga taaaaacaaa 120

acaaaaaaac gctgccaggt tttanaagca gttctggtct caaaaccatc aggatcctgc 180
caccagggtt cttttgaaat agtaccacat gtaaaaggga atttggcttt cacttcatct 240
aatcactgaa ttgtcaggct ttgattgata attgtagaaa taagtagcct tctgttgtgg 300
gaataagtta taatcagtat tcatctcttt gtttttgtc actcttttct ctctnattgt 360
gtcatttgta ctgtttgaaa aatatttctt ctataaaatt aaactaacct gccttaaaaa 420
aaaaaaaaaa aaaaaaa 437

<210> 75
<211> 579
<212> DNA
<213> Homo sapiens

<220>
<221> misc_feature
<222> 440, 513, 539, 551
<223> n = A,T,C or G

<400> 75
ctccgtcgcc gccaagatga tgtgcggggc gccctccgcc acgcagccgg ccaccgccga 60
gacccagcac atcgccgacc aggtgaggtc ccagcttgaa gagaaagaaa acaagaagtt 120
ccctgtgttt aaggccgtgt cattcaagag ccaggtggtc gcggggacaa actacttcat 180
caaggtgcac gtcggcgacg aggacttcgt acacctgcga gtgttccaat ctctccctca 240
tgaaaacaag cccttgacct tatctaacta ccagaccaac aaagccaagc atgatgagct 300
gacctatttc tgatcctgac tttggacaag gcccttcagc cagaagactg acaaagtcat 360
cctccgtcta ccagagcgtg cacttgtgat cctaaaataa gcttcatctc cgggctgtgc 420
ccttggggtg gaaggggcan gatctgcact gcttttgcat ttctcttcct aaatttcatt 480
gtgttgattc tttccttcca ataggtgatc ttnattactt tcagaatatt ttccaaatna 540
gatatatttt naaaatcctt aaaaaaaaaa aaaaaaaaa 579

<210> 76
<211> 666
<212> DNA
<213> Homo sapiens

<220>
<221> misc_feature
<222> 411, 470, 476, 491, 506, 527, 560, 570, 632, 636, 643, 650,
654, 658
<223> n = A,T,C or G

<400> 76
gtttatccta tctctccaac cagattgtca gctccttgag ggcaagagcc acagtatatt 60
tccctgtttc ttccacagtg cctaataata ctgtggaact aggttttaat aattttttaa 120
ttgatgttgt tatgggcagg atggcaacca gaccattgtc tcagagcagg tgctggctct 180
ttcctggcta ctccatgttg gctagcctct ggtaacctct tacttattat cttcaggaca 240
ctcactacag ggaccaggga tgatgcaaca tccttgtctt tttatgacag gatgtttgct 300
cagcttctcc aacaataaaa agcacgtggt aaaacacttg cggatattct ggactgtttt 360
taaaaaatat acagtttacc gaaaatcata ttatcttaca atgaaaagga ntttatagat 420
cagccagtga acaacctttt cccaccatac aaaaattcct tttcccgaan gaaaanggct 480
ttctcaataa ncctcacttt cttaanatct tacaagatag ccccganatc ttatcgaaac 540
tcattttagg caaatatgan ttttattgtn cgttacttgt ttcaaaattt ggtattgtga 600
atatcaatta ccacccccat ctcccatgaa anaaanggga aanggtgaan ttcntaancg 660
cttaaa 666

<210> 77
<211> 396
<212> DNA
<213> Homo sapiens

<220>
<221> misc_feature
<222> 31, 54, 125, 128, 136, 163, 168, 198
<223> n = A,T,C or G

<400> 77
ctgcagcccg ggggatccac taatctacca nggttatttg gcagctaatt ctanatttgg 60
atcattgccc aaagttgcac ttgctggtct cttgggattt ggccttggaa aggtatcata 120
catanganta tgccanaata aattccattt ttttgaaaat canctccntg gggctggttt 180
tggtccacag cataacangc actgcctcct tacctgtgag gaatgcaaaa taaagcatgg 240
attaagtgag aagggagact ctcagccttc agcttcctaa attctgtgtc tgtgactttc 300
gaagtttttt aaacctctga atttgtacac atttaaaatt tcaagtgtac tttaaaataa 360
aatacttcta atgggaacaa aaaaaaaaaa aaaaaa 396

<210> 78
<211> 793
<212> DNA
<213> Homo sapiens

<220>
<221> misc_feature
<222> 309, 492, 563, 657, 660, 703, 708, 710, 711, 732, 740, 748,
758, 762, 765, 787
<223> n = A,T,C or G

<400> 78
gcatcctagc cgccgactca cacaaggcag gtgggtgagg aaatccagag ttgccatgga 60
gaaaattcca gtgtcagcat tcttgctcct tgtggccctc tcctacactc tggccagaga 120
taccacagtc aaacctggag ccaaaaagga cacaaaggac tctcgaccca aactgcccca 180
gaccctctcc agaggttggg gtgaccaact catctggact cagacatatg aagaagctct 240
atataaatcc aagacaagca acaaaccctt gatgattatt catcacttgg atgagtgccc 300
acacagtcna gctttaaaga aagtgtttgc tgaaaataaa gaaatccaga aattggcaga 360
gcagtttgtc ctcctcaatc tggtttatga aacaactgac aaacaccttt ctcctgatgg 420
ccagtatgtc ccaggattat gtttgttgac ccatctctga cagttgaagc cgatatcctg 480
ggaagatatt cnaaccgtct ctatgcttac aaactgcaga tacgctctgt tgcttgacac 540
atgaaaaagc tctcaagttg ctnaaaatga attgtaagaa aaaaaatctc cagccttctg 600
tctgtcggct tgaaaattga aaccagaaaa atgtgaaaaa tggctattgt ggaacanatn 660
gacacctgat taggttttgg ttatgttcac cactattttt aanaaaanan nttttaaaat 720
ttggttcaat tntctttttn aaacaatntg tttctacntt gnganctgat ttctaaaaaa 780
aataatnttt ggc 793

<210> 79
<211> 456
<212> DNA
<213> Homo sapiens

<220>
<221> misc_feature
<222> 89, 195, 255, 263, 266, 286, 353, 384, 423, 425, 436, 441
<223> n = A,T,C or G

<400> 79
actagtatgg ggtgggaggc cccacccttc tcccctaggc gctgttcttg ctccaaaggg 60
ctccgtggag agggactggc agagctgang ccacctgggg ctggggatcc cactcttctt 120
gcagctgttg agcgcaccta accactggtc atgcccccac ccctgctctc cgcacccgct 180
tcctcccgac cccangacca ggctacttct cccctcctct tgcctccctc ctgcccctgc 240
tgcctctgat cgtangaatt gangantgtc ccgccttgtg gctganaatg gacagtggca 300

ggggctggaa atgggtgtgt gtgtgtgtgt gtgtgtgtgt gtgtgtgtgt gcnccccccc 360
tgcaagaccg agattgaggg aaancatgtc tgctgggtgt gaccatgttt cctctccata 420
aantnccccct gtgacnctca naaaaaaaaa aaaaaa 456

<210> 80
<211> 284
<212> DNA
<213> Homo sapiens

<220>
<221> misc_feature
<222> 283
<223> n = A,T,C or G

<400> 80
ctttgtacct ctagaaaaga taggtattgt gtcatgaaac ttgagtttaa attttatata 60
taaaactaaa agtaatgctc actttagcaa cacatactaa aattggaacc atactgagaa 120
gaatagcatg acctccgtgc aaacaggaca agcaaatttg tgatgtgttg attaaaaaga 180
aataaataaa tgtgtatatg tgtaacttgt atgtttatgt ggaatacaga ttgggaaata 240
aaatgtattt cttactgtga aaaaaaaaaa aaaaaaaaaa aana 284

<210> 81
<211> 671
<212> DNA
<213> Homo sapiens

<220>
<221> misc_feature
<222> 388, 505, 600, 603, 615, 642, 644, 660
<223> n = A,T,C or G

<400> 81
gccaccaaca ttccaagcta ccctgggtac ctttgtgcag tagaagctag tgagcatgtg 60
agcaagcggt gtgcacacgg agactcatcg ttataattta ctatctgcca agagtagaaa 120
gaaaggctgg ggatatttgg gttggcttgg ttttgatttt ttgcttgttt gtttgttttg 180
tactaaaaca gtattatctt ttgaatatcg tagggacata agtatataca tgttatccaa 240
tcaagatggc tagaatggtg cctttctgag tgtctaaaac ttgacacccc tggtaaatct 300
ttcaacacac ttccactgcc tgcgtaatga agttttgatt catttttaac cactggaatt 360
tttcaatgcc gtcattttca gttagatnat tttgcacttt gagattaaaa tgccatgtct 420
atttgattag tcttattttt ttatttttac aggcttatca gtctcactgt tggctgtcat 480
tgtgacaaag tcaaataaac ccccnaggac aacacacagt atgggatcac atattgtttg 540
acattaagct ttggccaaaa aatgttgcat gtgttttacc tcgacttgct aaatcaatan 600
canaaaggct ggctnataat gttggtggtg aaataattaa tnantaacca aaaaaaaaan 660
aaaaaaaaaa a 671

<210> 82
<211> 217
<212> DNA
<213> Homo sapiens

<220>
<221> misc_feature
<222> 35
<223> n = A,T,C or G

<400> 82
   ctgcagatgt ttcttgaatg ctttgtcaaa ttaanaaagt taaagtgcaa taatgtttga 60
   agacaataag tggtggtgta tcttgtttct aataagataa acttttttgt ctttgcttta 120
   tcttattagg gagttgtatg tcagtgtata aaacatactg tgtggtataa caggcttaat 180
   aaattcttta aaaggaaaaa aaaaaaaaaa aaaaaaa 217

<210> 83
<211> 460
<212> DNA
<213> Homo sapiens

<220>
<221> misc_feature
<222> 104, 118, 172, 401, 422, 423, 444, 449
<223> n = A,T,C or G

<400> 83
cgcgagtggg agcaccagga tctcgggctc ggaacgagac tgcacggatt gttttaagaa 60
aatggcagac aaaccagaca tgggggaaat cgccagcttc gatnaggcca agctgaanaa 120
aacggagacg caggagaaga acaccctgcc gaccaaagag accattgagc angagaagcg 180
gagtgaaatt tcctaagatc ctggaggatt tcctaccccc gtcctcttcg agaccccagt 240
cgtgatgtgg aggaagagcc acctgcaaga tggacacgag ccacaagctg cactgtgaac 300
ctgggcactc cgcgccgatg ccaccggcct gtgggtctct gaagggaccc cccccaatcg 360
gactgccaaa ttctccggtt tgccccggga tattatacaa nattatttgt atgaataatg 420
annataaaac acacctcgtg gcancaaana aaaaaaaaaa 460

<210> 84
<211> 323
<212> DNA
<213> Homo sapiens

<220>
<221> misc_feature
<222> 70, 138, 178, 197, 228, 242, 244, 287, 311
<223> n = A,T,C or G

<400> 84
tggtggatct tggctctgtg gagctgctgg gacgggatct aaaagactat tctggaagct 60
gtggtccaan gcattttgct ggcttaacgg gtcccggaac aaaggacacc agctctctaa 120
aattgaagtt tacccganat aacaatcttt tgggcagaga tgcctatttt aacaaacncc 180
gtccctgcgc aacaacnaac aatctctggg aaataccggc catgaacntg ctgtctcaat 240
cnancatctc tctagctgac cgatcatatc gtcccagatt actacanatc ataataattg 300
atttcctgta naaaaaaaaa aaa 323

<210> 85
<211> 771
<212> DNA
<213> Homo sapiens

<220>
<221> misc_feature
<222> 63, 426, 471, 497, 521, 554, 583, 586, 606, 609, 615, 652,
686, 691, 694, 695, 706, 713, 730, 732, 743, 751
<223> n = A,T,C or G

<400> 85
aaactgggta ctcaacactg agcagatctg ttctttgagc taaaaaccat gtgctgtacc 60
aanagtttgc tcctggctgc tttgatgtca gtgctgctac tccacctctg cggcgaatca 120
gaagcaagca actttgactg ctgtcttgga tacacagacc gtattcttca tcctaaattt 180
attgtgggct tcacacggca gctggccaat gaaggctgtg acatcaatgc tatcatcttt 240
cacacaaaga aaaagttgtc tgtgtgcgca aatccaaaac agacttgggt gaaatatatt 300

gtgcgtctcc tcagtaaaaa agtcaagaac atgtaaaaac tgtggctttt ctggaatgga 360
attggacata gcccaagaac agaaagaact tgctggggtt ggaggtttca cttgcacatc 420
atgganggtt tagtgcttat cttatttgtg cctcctggac ttgtccaatt natgaagtta 480
atcatattgc atcatanttt gctttgttta acatcacatt naaattaaac tgtattttat 540
gttatttata gctntaggtt ttctgtgttt aactttttat acnaantttc ctaaactatt 600
ttggtntant gcaanttaaa aattatattt ggggggggaa taaatattgg antttctgca 660
gccacaagct ttttttaaaa aaccantaca nccnngttaa atggtnggtc ccnaatggtt 720
tttgcttttn antagaaaat ttnttagaac natttgaaaa aaaaaaaaaa a 771

<210> 86
<211> 628
<212> DNA
<213> Homo sapiens

<220>
<221> misc_feature
<222> 162, 249, 266, 348, 407, 427, 488, 518, 545, 566, 569, 597,
598, 611, 617, 621, 624
<223> n = A,T,C or G

<400> 86
actagtttgc tttacatttt tgaaaagtat tatttttgtc caagtgctta tcaactaaac 60
cttgtgttag gtaagaatgg aatttattaa gtgaatcagt gtgacccttc ttgtcataag 120
attatcttaa agctgaagcc aaaatatgct tcaaaagaaa angactttat tgttcattgt 180
agttcataca ttcaaagcat ctgaactgta gtttctatag caagccaatt acatccataa 240
gtggagaang aaatagatta atgtcnaagt atgattggtg gagggagcaa ggttgaagat 300
aatctggggt tgaaattttc tagttttcat tctgtacatt tttagttnga catcagattt 360
gaaatattaa tgtttacctt tcaatgtgtg gtatcagctg gactcantaa cacccctttc 420
ttccctnggg gatggggaat ggattattgg aaaatggaaa gaaaaaagta cttaaagcct 480
tcctttcnca gtttctggct cctaccctac tgatttancc agaataagaa aacattttat 540
catcntctgc tttattccca ttaatnaant tttgatgaat aaatctgctt ttatgcnnac 600
ccaaggaatt nagtggnttc ntcnttgt 628

<210> 87
<211> 518
<212> DNA
<213> Homo sapiens

<220>
<221> misc_feature
<222> 384, 421, 486
<223> n = A,T,C or G

<400> 87
tttttatttt tttttagaga gtagttcagc ttttatttat aaatttattg cctgttttat 60
tataacaaca ttatactgtt tatggtttaa tacatatggt tcaaaatgta taatacatca 120
agtagtacag ttttaaaatt ttatgcttaa aacaagtttt gtgtaaaaaa tgcagataca 180
ttttacatgg caaatcaatt tttaagtcat cctaaaaatt gatttttttt tgaaatttaa 240
aaacacattt aatttcaatt tctctcttat ataaccttta ttactatagc atggtttcca 300
ctacagttta acaatgcagc aaaattccca tttcacggta aattgggttt taagcggcaa 360
ggttaaaatg ctttgaggat cctnaatacc ctttgaactt caaatgaagg ttatggttgt 420
naatttaacc ctcatgccat aagcagaagc acaagtttag ctgcattttg ctctaaactg 480
taaaancgag ccccccgttg aaaaagcaaa agggaccc 518

<210> 88
<211> 1844
<212> DNA
<213> Homo sapiens

<400> 88
gagacagtga atcctagtat caaaggattt ttggcctcag aaaaagttgt tgattatttt 60
tattttattt tatttttcga gactccgtct caaaaaaaaa aaaaaaaaaa agaatcacaa 120
ggtatttgct aaagcatttt gagctgcttg gaaaaaggga agtagttgca gtagagtttc 180
ttccatcttc ttggtgctgg gaagccatat atgtgtcttt tactcaagct aaggggtata 240
agcttatgtg ttgaatttgc tacatctata tttcacatat tctcacaata agagaatttt 300
gaaatagaaa tatcatagaa catttaagaa agtttagtat aaataatatt ttgtgtgttt 360
taatcccttt gaagggatct atccaaagaa aatattttac actgagctcc ttcctacacg 420
tctcagtaac agatcctgtg ttagtctttg aaaatagctc attttttaaa tgtcagtgag 480
tagatgtagc atacatatga tgtataatga cgtgtattat gttaacaatg tctgcagatt 540
ttgtaggaat acaaaacatg gcctttttta taagcaaaac gggccaatga ctagaataac 600
acatagggca atctgtgaat atgtattata agcagcattc cagaaaagta gttggtgaaa 660
taattttcaa gtcaaaaagg gatatggaaa gggaattatg agtaacctct attttttaag 720
ccttgctttt aaattaaacg ctacagccat ttaagccttg aggataataa agcttgagag 780
taataatgtt aggttagcaa aggtttagat gtatcacttc atgcatgcta ccatgatagt 840
aatgcagctc ttcgagtcat ttctggtcat tcaagatatt cacccttttg cccatagaaa 900
gcaccctacc tcacctgctt actgacattg tcttagctga tcacaagatc attatcagcc 960
tccattattc cttactgtat ataaaataca gagttttata ttttcctttc ttcgtttttc 1020
accatattca aaacctaaat ttgtttttgc agatggaatg caaagtaatc aagtgttcgt 1080
gctttcacct agaagggtgt ggtcctgaag gaaagaggtc cctaaatatc ccccaccctg 1140
ggtgctcctc cttccctggt accctgacta ccagaagtca ggtgctagag cagctggaga 1200
agtgcagcag cctgtgcttc cacagatggg ggtgctgctg caacaaggct ttcaatgtgc 1260
ccatcttagg gggagaagct agatcctgtg cagcagcctg gtaagtcctg aggaggttcc 1320
attgctcttc ctgctgctgt cctttgcttc tcaacggggc tcgctctaca gtctagagca 1380
catgcagcta acttgtgcct ctgcttatgc atgagggtta aattaacaac cataaccttc 1440
atttgaagtt caaaggtgta ttcaggatcc tcaaagcatt ttaaccttgc cgcttaaaac 1500
ccaatttacc gtgaaatggg aattttgctg cattgttaaa ctgtagtgga aaccatgcta 1560
tagtaataaa ggttatataa gagagaaatt gaaattaaat gtgtttttaa atttcaaaaa 1620
aaaatcaatc tttaggatga cttaaaaatt gatttgccat gtaaaatgta tctgcatttt 1680
ttacacaaaa cttgttttaa gcataaaatt ttaaaactgt actacttgat gtattataca 1740
ttttgaacca tatgtattaa accataaaca gtataatgtt gttataataa aacaggcaat 1800
aaatttataa ataaaagctg aaaaaaaaaa aaaaaaaaaa aaaa 1844

<210> 89
<211> 523
<212> DNA
<213> Homo sapiens

<220>
<221> misc_feature
<222> 288, 352, 369, 398, 475, 511, 513
<223> n = A,T,C or G

<400> 89
tttttttttt tttttttagt caatccacat ttattgatca cttattatgt accaggcact 60
gggataaaga tgactgttag tcactcacag taaggaagaa aactagcaaa taagacgatt 120
acaatatgat gtagaaaatg ctaagccaga gatatagaaa ggtcctattg ggtccttctg 180
tcaccttgtc tttccacatc cctacccttc acaggccttc cctccagctt cctgcccccg 240
ctccccactg cagatcccct gggattttgc ctagagctaa acgagganat gggccccctg 300
gccctggcat gacttgaacc caaccacaga ctgggaaagg gagcctttcg anagtggatc 360
actttgatna gaaaacacat agggaattga agagaaantc cccaaatggc cacccgtgct 420
ggtgctcaag aaaagtttgc agaatggata aatgaaggat caagggaatt aatanatgaa 480
taattgaatg gtggctcaat aagaatgact ncnttgaatg acc 523

<210> 90
<211> 604
<212> DNA

<213> Homo sapiens

<220>
<221> misc_feature
<222> 563
<223> n = A,T,C or G

<400> 90
ccagtgtggt ggaatgcaaa gattaccccg gaagctttcg agaagctggg attccctgca 60
gcaaaggaaa tagccaatat gtgtcgtttc tatgaaatga agccagaccg agatgtcaat 120
ctcacccacc aactaaatcc caaagtcaaa agcttcagcc agtttatctc agagaaccag 180
gggagccttc aagggcatgt agaaaatcag ctgttcagat aggcctctgc accacacagc 240
ctctttcctc tctgatcctt ttcctcttta cggcacaaca ttcatgtttg acagaacatg 300
ctggaatgca attgtttgca acaccgaagg atttcctgcg gtcgcctctt cagtaggaag 360
cactgcattg gtgataggac acggtaattt gattcacatt taacttgcta gttagtgata 420
aggggtggta cacctgtttg gtaaaatgag aagcctcgga aacttgggag cttctctcct 480
accactaatg gggagggcag attattactg ggatttctcc tggggtgaat taatttcaag 540
ccctaattgc tgaaattccc ctnggcaggc tccagttttc tcaactgcat tgcaaaattc 600
cccc 604

<210> 91
<211> 858
<212> DNA
<213> Homo sapiens

<220>
<221> misc_feature
<222> 570, 591, 655, 664, 667, 683, 711, 759, 760, 765, 777, 787,
792, 794, 801, 804, 809, 817, 820
<223> n = A,T,C or G

<400> 91
tttttttttt ttttttttta tgattattat tttttttatt gatctttaca tcctcagtgt 60
tggcagagtt tctgatgctt aataaacatt tgttctgatc agataagtgg aaaaaattgt 120
catttcctta ttcaagccat gcttttctgt gatattctga tcctagttga acatacagaa 180
ataaatgtct aaaacagcac ctcgattctc gtctataaca ggactaagtt cactgtgatc 240
ttaaataagc ttggctaaaa tgggacatga gtggaggtag tcacacttca gcgaagaaag 300
agaatctcct gtataatctc accaggagat tcaacgaatt ccaccacact ggactagtgg 360
atcccccggg ctgcaggaat tcgatatcaa gcttatcgat accgtcgacc tcgagggggg 420
gcccggtacc caattcgccc tatagtgagt cgtattacgc gcgctcactg gccgtcgttt 480
tacaacgtcg tgactgggaa aaccctggcg ttacccaact taatcgcctt gcagcacatc 540
cccctttcgc cagctggcgt aatagcgaan agcccgcacc gatcgccctt ncaacagttg 600
cgcagcctga atggcgaatg ggacgcgccc tgtagcggcg cattaaagcg cggcngggtg 660
tggnggntcc cccacgtgac cgntacactt ggcagcgcct tacgccggtc nttcgctttc 720
ttcccttcct ttctcgcacc gttcgccggg tttccccgnn agctnttaat cgggggnctc 780
cctttanggg tncnaattaa nggnttacng gaccttngan cccaaaaact ttgattaggg 840
ggaaggtccc cgaagggg 858

<210> 92
<211> 585
<212> DNA
<213> Homo sapiens

<220>
<221> misc_feature
<222> 317, 319, 320, 321, 325, 327, 328, 330, 331, 332, 460, 462,
483, 485, 487, 523, 538, 566, 584
<223> n = A,T,C or G

<400> 92
gttgaatctc ctggtgagat tatacaggag attctctttc ttcgctgaag tgtgactacc 60
tccactcatg tcccatttta gccaagctta tttaagatca cagtgaactt agtcctgtta 120
tagacgagaa tcgaggtgct gttttagaca tttatttctg tatgttcaac taggatcaga 180
atatcacaga aaagcatggc ttgaataagg aaatgacaat tttttccact tatctgatca 240
gaacaaatgt ttattaagca tcagaaactc tgccaacact gaggatgtaa agatcaataa 300
aaaaaataat aatcatnann naaanannan nngaagggcg gccgccaccg cggtggagct 360
ccagcttttg ttccctttag tgagggttaa ttgcgcgctt ggcgttaatc atggtcatag 420
ctgtttcctg tgtgaaattg ttatccgctc cacaattccn cncaacatac gagccgggaa 480
gcntnangtg taaaagcctg gggtgcctaa attgagtgag ctnactcaca ttaattgngt 540
tgcgctccac ttgcccgctt ttccantccg ggaaacctgt tcgnc 585

<210> 93
<211> 567
<212> DNA
<213> Homo sapiens

<220>
<221> misc_feature
<222> 82, 158, 230, 232, 253, 266, 267, 268, 269, 270, 271, 272,
273, 274, 275, 276, 277, 278, 279, 280, 281, 282, 283, 284,
285, 286, 287, 295, 303, 307, 314, 349, 352, 354, 356, 366,
369, 379, 382, 386, 393, 404, 427, 428, 446, 450, 452
<223> n = A,T,C or G

<221> misc_feature
<222> 453, 454, 459, 462, 480, 481, 483, 488, 493, 501, 509, 511,
512, 518, 520, 525, 526, 532, 541, 557
<223> n = A,T,C or G

<400> 93
cggcagtgtt gctgtctgcg tgtccacctt ggaatctggc tgaactggct gggaggacca 60
agactgcggc tggggtgggc anggaaggga accgggggct gctgtgaagg atcttggaac 120
ttccctgtac ccaccttccc cttgcttcat gtttgtanag gaaccttgtg ccggccaagc 180
ccagtttcct tgtgtgatac actaatgtat ttgctttttt tgggaaatan anaaaaatca 240
attaaattgc tantgtttct ttgaannnnn nnnnnnnnnn nnnnnnnggg ggggncgccc 300
ccncggngga aacnccccct tttgttccct ttaattgaaa ggttaattng cncncntggc 360
gttaanccnt gggccaaanc tngttncccg tgntgaaatt gttnatcccc tcccaaattc 420
ccccccnncc ttccaaaccc ggaaancctn annntgttna ancccggggg gttgcctaan 480
ngnaattnaa ccnaaccccc ntttaaatng nntttgcncn ccacnngccc cnctttccca 540
nttcggggaa aaccctntcc gtgccca 567

<210> 94
<211> 620
<212> DNA
<213> Homo sapiens

<220>
<221> misc_feature
<222> 169, 171, 222, 472, 528, 559, 599
<223> n = A,T,C or G

<400> 94
actagtcaaa aatgctaaaa taatttggga gaaaatattt tttaagtagt gttatagttt 60
catgtttatc ttttattatg ttttgtgaag ttgtgtcttt tcactaatta cctatactat 120
gccaatattt ccttatatct atccataaca tttatactac atttgtaana naatatgcac 180
gtgaaactta acactttata aggtaaaaat gaggtttcca anatttaata atctgatcaa 240

gttcttgtta tttccaaata gaatggactt ggtctgttaa gggctaagga gaagaggaag 300
ataaggttaa aagttgttaa tgaccaaaca ttctaaaaga aatgcaaaaa aaaagtttat 360
tttcaagcct tcgaactatt taaggaaagc aaaatcattt cctaaatgca tatcatttgt 420
gagaatttct cattaatatc ctgaatcatt catttcacta aggctcatgt tnactccgat 480
atgtctctaa gaaagtacta tttcatggtc caaacctggt tgccatantt gggtaaaggc 540
tttcccttaa gtgtgaaant atttaaaatg aaattttcct ctttttaaaa attctttana 600
agggttaagg gtgttgggga 620

<210> 95
<211> 470
<212> DNA
<213> Homo sapiens

<220>
<221> misc_feature
<222> 61, 67, 79, 89, 106, 213, 271, 281, 330, 354, 387, 432, 448
<223> n = A,T,C or G

<400> 95
ctcgaccttc tctgcacagc ggatgaaccc tgagcagctg aagaccagaa aagccactat 60
nactttntgc ttaattcang agcttacang attcttcaaa gagtgngtcc agcatccttt 120
gaaacatgag ttcttaccag cagaagcaga cctttacccc accacctcag cttcaacagc 180
agcaggtgaa acaacccatc cagcctccac ctnaggaaat atttgttccc acaaccaagg 240
agccatgcca ctcaaaggtt ccacaacctg naaacacaaa nattccagag ccaggctgta 300
ccaaggtccc tgagccaggg ctgtaccaan gtccctgagc caggttgtac caangtccct 360
gagccaggat gtaccaaggt ccctgancca ggttgtccaa ggtccctgag ccaggctaca 420
ccaagggcct gngccaggca gcatcaangt ccctgaccaa ggcttatcaa 470

<210> 96
<211> 660
<212> DNA
<213> Homo sapiens

<220>
<221> misc_feature
<222> 299, 311, 360, 426, 538, 540, 542, 553, 563, 565, 592, 603,
604, 618, 633, 647, 649, 651, 653
<223> n = A,T,C or G

<400> 96
tttttttttt tttttttttt ggaattaaaa gcaatttaat gagggcagag caggaaacat 60
gcatttcttt tcattcgaat cttcagatga accctgagca gccgaagacc agaaaagcca 120
tgaagacttt ctgcttaatt caggggctta caggattctt cagagtgtgt gtgaacaaaa 180
gctttatagt acgtattttt aggatacaaa taagagagag actatggctt ggggtgagaa 240
tgtactgatt acaaggtcta cagacaatta agacacagaa acagatggga agagggtgnc 300
cagcatctgg nggttggctt ctcaagggct tgtctgtgca ccaaattact tctgcttggn 360
cttctgctga gctgggcctg gagtgaccgt tgaaggacat ggctctggta cctttgtgta 420
gcctgncaca ggaactttgg tgtatccttg ctcaggaact ttgatggcac ctggctcagg 480
aaacttgatg aagccttggt caagggacct tgatgcttgc tggctcaggg accttggngn 540
ancctgggct canggacctt tgncncaacc ttggcttcaa gggacccttg gnacatcctg 600
gcnnagggac ccttgggncc aaccctgggc ttnagggacc ctttggntnc nanccttggc 660

<210> 97
<211> 441
<212> DNA
<213> Homo sapiens

<220>
<221> misc_feature
<222> 12, 308
<223> n = A,T,C or G

<400> 97
gggaccatac anagtattcc tctcttcaca ccaggaccag ccactgttgc agcatgagtt 60
cccagcagca gaagcagccc tgcatcccac cccctcagct tcagcagcag caggtgaaac 120
agccttgcca gcctccacct caggaaccat gcatccccaa aaccaaggag ccctgccacc 180
ccaaggtgcc tgagccctgc caccccaaag tgcctgagcc ctgccagccc aaggttccag 240
agccatgcca ccccaaggtg cctgagccct gcccttcaat agtcactcca gcaccagccc 300
agcagaanac caagcagaag taatgtggtc cacagccatg cccttgagga gccggccacc 360
agatgctgaa tcccctatcc cattctgtgt atgagtccca tttgccttgc aattagcatt 420
ctgtctcccc caaaaaaaaa a 441

<210> 98
<211> 600
<212> DNA
<213> Homo sapiens

<220>
<221> misc_feature
<222> 295, 349, 489, 496, 583
<223> n = A,T,C or G

<400> 98
gtattcctct cttcacacca ggaccagcca ctgttgcagc atgagttccc agcagcagaa 60
gcagccctgc atcccacccc ctcagcttca gcagcagcag gtgaaacagc cttgccagcc 120
tccacctcag gaaccatgca tccccaaaac caaggagccc tgccacccca aggtgcctga 180
gccctgccac cccaaagtgc ctgagccctg ccagcccaag gttccagagc catgccaccc 240
caaggtgcct gagccctgcc cttcaatagt cactccagca ccagcccagc agaanaccaa 300
gcagaagtaa tgtggtccac agccatgccc ttgaggagcc ggccaccana tgctgaatcc 360
cctatcccat tctgtgtatg agtcccattt gccttgcaat tagcattctg tctcccccaa 420
aaaagaatgt gctatgaagc tttctttcct acacactctg agtctctgaa tgaagctgaa 480
ggtcttaant acaganctag ttttcagctg ctcagaattc tctgaagaaa agatttaaga 540
tgaaaggcaa atgattcagc tccttattac cccattaaat tcnctttcaa ttccaaaaaa 600

<210> 99
<211> 667
<212> DNA
<213> Homo sapiens

<220>
<221> misc_feature
<222> 345, 562, 635
<223> n = A,T,C or G

<400> 99
actagtgact gagttcctgg caaagaaatt tgacctggac cagttgataa ctcatgtttt 60
accatttaaa aaaatcagtg aaggatttga gctgctcaat tcaggacaaa gcattcgaac 120
ggtcctgacg ttttgagatc caaagtggca ggaggtctgt gttgtcatgg tgaactggag 180
tttctcttgt gagagttccc tcatctgaaa tcatgtatct gtctcacaaa tacaagcata 240
agtagaagat ttgttgaaga catagaaccc ttataaagaa ttattaacct ttataaacat 300
ttaaagtctt gtgagcacct gggaattagt ataataacaa tgttnatatt tttgatttac 360
attttgtaag gctataattg tatcttttaa gaaaacatac cttggatttc tatgttgaaa 420
tggagatttt taagagtttt aaccagctgc tgcagatata ttactcaaaa cagatatagc 480
gtataaagat atagtaaatg catctcctag agtaatattc acttaacaca ttggaaacta 540

ttatttttta gatttgaata tnaatgttat tttttaaaca cttgttatga gttacttggg 600
attacatttt gaaatcagtt cattccatga tgcanattac tgggattaga ttaagaaaga 660
cggaaaa 667

<210> 100
<211> 583
<212> DNA
<213> Homo sapiens

<220>
<221> misc_feature
<222> 404, 506, 514, 527, 528, 538, 548, 556, 568, 569
<223> n = A,T,C or G

<400> 100
gttttgtttg taagatgatc acagtcatgt tacactgatc taaaggacat atatataacc 60
ctttaaaaaa aaaatcactg cctcattctt atttcaagat gaatttctat acagactaga 120
tgtttttctg aagatcaatt agacattttg aaaatgattt aaagtgtttt ccttaatgtt 180
ctctgaaaac aagtttcttt tgtagtttta accaaaaaag tgcccttttt gtcactggat 240
tctcctagca ttcatgattt ttttttcata caatgaaatt aaaattgcta aaatcatgga 300
ctggctttct ggttggattt caggtaagat gtgtttaagg ccagagcttt tctcagtatt 360
tgattttttt ccccaatatt tgatttttta aaaatataca catnggtgct gcatttatat 420
ctgctggttt aaaattctgt catatttcac ttctagcctt ttagttatgg caaatcatat 480
tttactttta cttaaagcat ttggtnattt ggantatctg gttctannct aaaaaaanta 540
attctatnaa ttgaantttt ggtactcnnc catatttgga tcc 583

<210> 101
<211> 592
<212> DNA
<213> Homo sapiens

<220>
<221> misc_feature
<222> 218, 497, 502, 533, 544, 546, 548, 550, 555
<223> n = A,T,C or G

<400> 101
gtggagacgt acaaagagca gccgctcaag acacctggga agaaaaagaa aggcaagccc 60
gggaaacgca aggagcagga aaagaaaaaa cggcgaactc gctctgcctg gttagactct 120
ggagtgactg ggagtgggct agaaggggac cacctgtctg acacctccac aacgtcgctg 180
gagctcgatt cacggaggca ttgaaatttt cagcaganac cttccaagga catattgcag 240
gattctgtaa tagtgaacat atggaaagta ttagaaatat ttattgtctg taaatactgt 300
aaatgcattg gaataaaact gtctccccca ttgctctatg aaactgcaca ttggtcattg 360
tgaatatttt tttttttgcc aaggctaatc caattattat tatcacattt accataattt 420
attttgtcca ttgatgtatt tattttgtaa atgtatcttg gtgctgctga atttctatat 480
tttttgtaca taatgcnttt anatatacct atcaagtttg ttgataaatg acncaatgaa 540
gtgncncnan ttggnggttg aatttaatga atgcctaatt ttattatccc aa 592

<210> 102
<211> 587
<212> DNA
<213> Homo sapiens

<220>
<221> misc_feature
<222> 91, 131, 256, 263, 332, 392, 400, 403, 461, 496, 497, 499,
510, 511, 518, 519, 539, 554, 560, 576
<223> n = A,T,C or G

<400> 102
cgtcctaagc acttagacta catcagggaa gaacacagac cacatccctg tcctcatgcg 60
gcttatgttt tctggaagaa agtggagacc nagtccttgg ctttagggct ccccggctgg 120
gggctgtgca ntccggtcag ggcgggaagg gaaatgcacc gctgcatgtg aacttacagc 180
ccaggcggat gccccttccc ttagcactac ctggcctcct gcatcccctc gcctcatgtt 240
cctcccacct tcaaanaatg aanaacccca tgggcccagc cccttgccct ggggaaccaa 300
ggcagccttc caaaactcag gggctgaagc anactattag ggcaggggct gactttgggt 360
gacactgccc attccctctc agggcagctc angtcacccn ggnctcttga acccagcctg 420
ttcctttgaa aaagggcaaa actgaaaagg gcttttccta naaaaagaaa aaccaggaaa 480
ctttgccagg gcttcnntnt taccaaaacn ncttctcnng gatttttaat tccccattng 540
gcctccactt accnggggcn atgccccaaa attaanaatt tcccatc 587

<210> 103
<211> 496
<212> DNA
<213> Homo sapiens

<220>
<221> misc_feature
<222> 2, 17, 66, 74, 82, 119, 164, 166, 172, 200, 203, 228, 232,
271, 273, 415, 423, 445, 446, 473
<223> n = A,T,C or G

<400> 103
anaggactgg ccctacntgc tctctctcgt cctacctatc aatgcccaac atggcagaac 60
ctgcanccct tggncactgc anatggaaac ctctcagtgt cttgacatca ccctacccnt 120
gcggtgggtc tccaccacaa ccactttgac tctgtggtcc ctgnanggtg gnttctcctg 180
actggcagga tggaccttan ccnacatatc cctctgttcc ctctgctnag anaaagaatt 240
cccttaacat gatataatcc acccatgcaa ntngctactg gcccagctac catttaccat 300
ttgcctacag aatttcattc agtctacact ttggcattct ctctggcgat agagtgtggc 360
tgggctgacc gcaaaaggtg ccttacacac tggcccccac cctcaaccgt tgacncatca 420
gangcttgcc tcctccttct gattnncccc catgttggat atcagggtgc tcnagggatt 480
ggaaaagaaa caaaac 496

<210> 104
<211> 575
<212> DNA
<213> Homo sapiens

<220>
<221> misc_feature
<222> 18, 19, 45, 68, 77, 132, 155, 174, 219, 226, 238, 259, 263,
271, 273, 306, 323, 339, 363, 368, 370, 378, 381, 382, 436,
440, 449, 450, 456, 481, 485, 496, 503, 510, 512, 515, 528,
542, 552
<223> n = A,T,C or G

<400> 104
gcacctgctc tcaatccnnc tctcaccatg atcctccgcc tgcanaaact cctctgccaa 60
ctatggangt ggtttcnggg gtggctcttg ccaactggga agaagccgtg gtgtctctac 120
ctgttcaact cngtttgtgt ctgggggatc aactnggggc tatggaagcg gctnaactgt 180
tgttttggtg gaagggctgg taattggctt tgggaagtng cttatngaag ttggcctngg 240
gaagttgcta ttgaaagtng ccntggaagt ngntttggtg gggggttttg ctggtggcct 300
ttgttnaatt tgggtgcttt gtnaatggcg gccccctcnc ctgggcaatg aaaaaaatca 360
ccnatgcngn aaacctcnac nnaacagcct gggcttccct cacctcgaaa aaagttgctc 420
cccccccaaa aaaggncaan cccctcaann tggaangttg aaaaaatcct cgaatgggga 480
ncccnaaaac aaaaancccc ccntttcccn gnaanggggg aaataccncc cccccactta 540

cnaaaaccct tntaaaaaac cccccgggaa aaaaa 575

<210> 105
<211> 619
<212> DNA
<213> Homo sapiens

<220>
<221> misc_feature
<222> 260, 527, 560, 564, 566, 585, 599
<223> n = A,T,C or G

<400> 105
cactagtagg atagaaacac tgtgtcccga gagtaaggag agaagctact attgattaga 60
gcctaaccca ggttaactgc aagaagaggc gggatacttt cagctttcca tgtaactgta 120
tgcataaagc caatgtagtc cagtttctaa gatcatgttc caagctaact gaatcccact 180
tcaatacaca ctcatgaact cctgatggaa caataacagg cccaagcctg tggtatgatg 240
tgcacacttg ctagactcan aaaaaatact actctcataa atgggtggga gtattttggt 300
gacaacctac tttgcttggc tgagtgaagg aatgatattc atatattcat ttattccatg 360
gacatttagt tagtgctttt tatataccag gcatgatgct gagtgacact cttgtgtata 420
tttccaaatt tttgtacagt cgctgcacat atttgaaatc atatattaag acttccaaaa 480
aatgaagtcc ctggtttttc atggcaactt gatcagtaaa ggattcncct ctgtttggta 540
cttaaaacat ctactatatn gttnanatga aattcctttt ccccncctcc cgaaaaaana 600
aagtggtggg gaaaaaaaa 619

<210> 106
<211> 506
<212> DNA
<213> Homo sapiens

<220>
<221> misc_feature
<222> 8, 21, 31, 32, 58, 75, 89, 96, 99, 103, 122, 126, 147, 150,
158, 195, 210, 212, 219, 226, 246, 248, 249, 255, 258, 261,
263, 265, 275, 304, 317, 321, 331, 337, 340, 358, 371, 377,
380, 396, 450, 491
<223> n = A,T,C or G

<400> 106
cattggtnct ttcatttgct ntggaagtgt nnatctctaa cagtggacaa agttcccngt 60
gccttaaact ctgtnacact tttgggaant gaaaanttng tantatgata ggttattctg 120
angtanagat gttctggata ccattanatn tgcccccngt gtcagaggct catattgtgt 180
tatgtaaatg gtatntcatt cgctactatn antcaattng aaatanggtc tttgggttat 240
gaatantnng cagcncanct nanangctgt ctgtngtatt cattgtggtc atagcacctc 300
acancattgt aacctcnatc nagtgagaca nactagnaan ttcctagtga tggctcanga 360
ttccaaatgg nctcatntcn aatgtttaaa agttanttaa gtgtaagaaa tacagactgg 420
atgttccacc aactagtacc tgtaatgacn ggcctgtccc aacacatctc ccttttccat 480
gactgtggta ncccgcatcg gaaaaa 506

<210> 107
<211> 452
<212> DNA
<213> Homo sapiens

<220>
<221> misc_feature
<222> 289, 317, 378
<223> n = A,T,C or G

<400> 107
gttgagtctg tactaaacag taagatatct caatgaacca taaattcaac tttgtaaaaa 60
tcttttgaag catagataat attgtttggt aaatgtttct tttgtttggt aaatgtttct 120
tttaaagacc ctcctattct ataaaactct gcatgtagag gcttgtttac ctttctctct 180
ctaaggttta caataggagt ggtgatttga aaaatataaa attatgagat tggttttcct 240
gtggcataaa ttgcatcact gtatcatttt cttttttaac cggtaagant ttcagtttgt 300
tggaaagtaa ctgtganaac ccagtttccc gtccatctcc cttagggact acccatagaa 360
catgaaaagg tccccacnga agcaagaaga taagtctttc atggctgctg gttgcttaaa 420
ccactttaaa accaaaaaat tccccttgga aa 452

<210> 108
<211> 502
<212> DNA
<213> Homo sapiens

<220>
<221> misc_feature
<222> 22, 31, 126, 168, 183, 205, 219, 231, 236, 259, 283, 295,
296, 298, 301, 340, 354, 378, 383, 409, 433, 446, 455, 466,
488
<223> n = A,T,C or G

<400> 108
atcttcttcc cttaattagt tnttatttat ntattaaatt ttattgcatg tcctggcaaa 60
caaaaagaga ttgtagattg gcttctggct ccccaaaagc ccataacaga aagtaccaca 120
agaccncaac tgaagcttaa aaaatctatc acatgtataa tacctttnga agaacattaa 180
tanagcatat aaaactttta acatntgctt aatgttgtnc aattataaaa ntaatngaaa 240
aaaatgtccc tttaacatnc aatatcccac atagtgttat ttnaggggat taccnngnaa 300
naaaaaaagg gtagaaggga tttaatgaaa actctgcttn ccatttctgt ttanaaacgt 360
ctccagaaca aaaacttntc aantctttca gctaaccgca tttgagctna ggccactcaa 420
aaactccatt agncccactt tctaanggtc tctanagctt actaancctt ttgacccctt 480
accctggnta ctcctgccct ca 502

<210> 109
<211> 1308
<212> DNA
<213> Homo sapiens

<400> 109
acccgaggtc tcgctaaaat catcatggat tcacttggcg ccgtcagcac tcgacttggg 60
tttgatcttt tcaaagagct gaagaaaaca aatgatggca acatcttctt ttcccctgtg 120
ggcatcttga ctgcaattgg catggtcctc ctggggaccc gaggagccac cgcttcccag 180
ttggaggagg tgtttcactc tgaaaaagag acgaagagct caagaataaa ggctgaagaa 240
aaagaggtga ttgagaacac agaagcagta catcaacaat tccaaaagtt tttgactgaa 300
ataagcaaac tcactaatga ttatgaactg aacataacca acaggctgtt tggagaaaaa 360
acatacctct tccttcaaaa atacttagat tatgttgaaa aatattatca tgcatctctg 420
gaacctgttg attttgtaaa tgcagccgat gaaagtcgaa agaagattaa ttcctgggtt 480
gaaagcaaaa caaatgaaaa aatcaaggac ttgttcccag atggctctat tagtagctct 540
accaagctgg tgctggtgaa catggtttat tttaaagggc aatgggacag ggagtttaag 600
aaagaaaata ctaaggaaga gaaattttgg atgaataaga gcacaagtaa atctgtacag 660
atgatgacac agagccattc ctttagcttc actttcctgg aggacttgca ggccaaaatt 720
ctagggattc catataaaaa caacgaccta agcatgtttg tgcttctgcc caacgacatc 780
gatggcctgg agaagataat agataaaata agtcctgaga aattggtaga gtggactagt 840
ccagggcata tggaagaaag aaaggtgaat ctgcacttgc cccggtttga ggtggaggac 900
agttacgatc tagaggcggt cctggctgcc atggggatgg gcgatgcctt cagtgagcac 960
aaagccgact actcgggaat gtcgtcaggc tccgggttgt acgcccagaa gttcctgcac 1020
agttcctttg tggcagtaac tgaggaaggc accgaggctg cagctgccac tggcataggc 1080

tttactgtca catccgcccc aggtcatgaa aatgttcact gcaatcatcc cttcctgttc 1140
ttcatcaggc acaatgaatc caacagcatc ctcttcttcg gcagattttc ttctccttaa 1200
gatgatcgtt gccatggcat tgctgctttt agcaaaaaac aactaccagt gttactcata 1260
tgattatgaa aatcgtccat tcttttaaat ggtggctcac ttgcattt 1308

<210> 110
<211> 391
<212> PRT
<213> Homo sapiens

<400> 110


<210> 111
<211> 1419
<212> DNA
<213> Homo sapiens

<400> 111
ggagaactat aaattaagga tcccagctac ttaattgact tatgcttcct agttcgttgc 60
ccagccacca ccgtctctcc aaaaacccga ggtctcgcta aaatcatcat ggattcactt 120
ggcgccgtca gcactcgact tgggtttgat cttttcaaag agctgaagaa aacaaatgat 180
ggcaacatct tcttttcccc tgtgggcatc ttgactgcaa ttggcatggt cctcctgggg 240
acccgaggag ccaccgcttc ccagttggag gaggtgtttc actctgaaaa agagacgaag 300
agctcaagaa taaaggctga agaaaaagag gtggtaagaa taaaggctga aggaaaagag 360
attgagaaca cagaagcagt acatcaacaa ttccaaaagt ttttgactga aataagcaaa 420
ctcactaatg attatgaact gaacataacc aacaggctgt ttggagaaaa aacatacctc 480
ttccttcaaa aatacttaga ttatgttgaa aaatattatc atgcatctct ggaacctgtt 540
gattttgtaa atgcagccga tgaaagtcga aagaagatta attcctgggt tgaaagcaaa 600
acaaatgaaa aaatcaagga cttgttccca gatggctcta ttagtagctc taccaagctg 660
gtgctggtga acatggttta ttttaaaggg caatgggaca gggagtttaa gaaagaaaat 720
actaaggaag agaaattttg gatgaataag agcacaagta aatctgtaca gatgatgaca 780
cagagccatt cctttagctt cactttcctg gaggacttgc aggccaaaat tctagggatt 840
ccatataaaa acaacgacct aagcatgttt gtgcttctgc ccaacgacat cgatggcctg 900
gagaagataa tagataaaat aagtcctgag aaattggtag agtggactag tccagggcat 960
atggaagaaa gaaaggtgaa tctgcacttg ccccggtttg aggtggagga cagttacgat 1020
ctagaggcgg tcctggctgc catggggatg ggcgatgcct tcagtgagca caaagccgac 1080
tactcgggaa tgtcgtcagg ctccgggttg tacgcccaga agttcctgca cagttccttt 1140
gtggcagtaa ctgaggaagg caccgaggct gcagctgcca ctggcatagg ctttactgtc 1200
acatccgccc caggtcatga aaatgttcac tgcaatcatc ccttcctgtt cttcatcagg 1260
cacaatgaat ccaacagcat cctcttcttc ggcagatttt cttctcctta agatgatcgt 1320
tgccatggca ttgctgcttt tagcaaaaaa caactaccag tgttactcat atgattatga 1380
aaatcgtcca ttcttttaaa tggtggctca cttgcattt                        1419

<210> 112
<211> 400
<212> PRT
<213> Homo sapiens

<400> 112

<210> 113
<211> 957
<212> DNA
<213> Homo sapiens

<400> 113
ctcgaccttc tctgcacagc ggatgaaccc tgagcagctg aagaccagaa aagccactat 60
gactttctgc ttaattcagg agcttacagg attcttcaaa gagtgtgtcc agcatccttt 120
gaaacatgag ttcttaccag cagaagcaga cctttacccc accacctcag cttcaacagc 180
agcaggtgaa acaacccagc cagcctccac ctcaggaaat atttgttccc acaaccaagg 240
agccatgcca ctcaaaggtt ccacaacctg gaaacacaaa gattccagag ccaggctgta 300
ccaaggtccc tgagccaggc tgtaccaagg tccctgagcc aggttgtacc aaggtccctg 360
agccaggatg taccaaggtc cctgagccag gttgtaccaa ggtccctgag ccaggctaca 420
ccaaggtccc tgagccaggc agcatcaagg tccctgacca aggcttcatc aagtttcctg 480
agccaggtgc catcaaagtt cctgagcaag gatacaccaa agttcctgtg ccaggctaca 540
caaaggtacc agagccatgt ccttcaacgg tcactccagg cccagctcag cagaagacca 600
agcagaagta atttggtgca cagacaagcc cttgagaagc caaccaccag atgctggaca 660
ccctcttccc atctgtttct gtgtcttaat tgtctgtaga ccttgtaatc agtacattct 720
caccccaagc catagtctct ctcttatttg tatcctaaaa atacggtact ataaagcttt 780
tgttcacaca cactctgaag aatcctgtaa gcccctgaat taagcagaaa gtcttcatgg 840
cttttctggt cttcggctgc tcagggttca tctgaagatt cgaatgaaaa gaaatgcatg 900
tttcctgctc tgccctcatt aaattgcttt taattccaaa aaaaaaaaaa aaaaaaa 957

<210> 114
<211> 161
<212> PRT
<213> Homo sapiens

<400> 114
Met Ser Ser Tyr Gln Gln Lys Gln Thr Phe Thr Pro Pro Pro Gln Leu
1               5                   10                  15
Gln Gln Gln Gln Val Lys Gln Pro Ser Gln Pro Pro Pro Gln Glu Ile
            20                  25                  30
Phe Val Pro Thr Thr Lys Glu Pro Cys His Ser Lys Val Pro Gln Pro
        35                  40                  45
Gly Asn Thr Lys Ile Pro Glu Pro Gly Cys Thr Lys Val Pro Glu Pro
    50                  55                  60
Gly Cys Thr Lys Val Pro Glu Pro Gly Cys Thr Lys Val Pro Glu Pro
65                  70                  75                  80
Gly Cys Thr Lys Val Pro Glu Pro Gly Cys Thr Lys Val Pro Glu Pro
                85                  90                  95
Gly Tyr Thr Lys Val Pro Glu Pro Gly Ser Ile Lys Val Pro Asp Gln
            100                 105                 110
Gly Phe Ile Lys Phe Pro Glu Pro Gly Ala Ile Lys Val Pro Glu Gln
        115                 120                 125
Gly Tyr Thr Lys Val Pro Val Pro Gly Tyr Thr Lys Val Pro Glu Pro
    130                 135                 140
Cys Pro Ser Thr Val Thr Pro Gly Pro Ala Gln Gln Lys Thr Lys Gln
145                 150                 155                 160
Lys

<210> 115
<211> 506
<212> DNA
<213> Homo sapiens

<220>
<221> misc_feature
<222> 8, 21, 31, 32, 58, 75, 89, 96, 99, 103, 122, 126, 147, 150,
158, 195, 210, 212, 219, 226, 246, 248, 249, 255, 258, 261,
263, 265, 275, 304, 317, 321, 331, 337, 340, 358, 371, 377,
380, 396, 450, 491
<223> n = A,T,C or G

<400> 115
cattggtnct ttcatttgct ntggaagtgt nnatctctaa cagtggacaa agttcccngt 60
gccttaaact ctgtnacact tttgggaant gaaaanttng tantatgata ggttattctg 120
angtanagat gttctggata ccattanatn tgcccccngt gtcagaggct catattgtgt 180
tatgtaaatg gtatntcatt cgctactatn antcaattng aaatanggtc tttgggttat 240
gaatantnng cagcncanct nanangctgt ctgtngtatt cattgtggtc atagcacctc 300
acancattgt aacctcnatc nagtgagaca nactagnaan ttcctagtga tggctcanga 360
ttccaaatgg nctcatntcn aatgtttaaa agttanttaa gtgtaagaaa tacagactgg 420
atgttccacc aactagtacc tgtaatgacn ggcctgtccc aacacatctc ccttttccat 480
gactgtggta ncccgcatcg gaaaaa                                      506

<210> 116
<211> 3079
<212> DNA

<213> Homo sapiens

<400> 116
ggatccccgg gtttcctaaa ccccccacag agtcctgccc aggccaaaga gcaaggaaaa 60
ggtcaaaggg cagaaaaaat gctgagttag gaggagctat ggaaggataa acctggcctt 120
aaagaggtca aagtggttta taggggggcgc tgagggcttc ccacattctc tggcctaaac 180
cttgcaggca gatctgccca gtgggctctg ggatagctgt gccttcccta acaaaaaaat 240
tgtgcacaaa aggatgaaac tctattttcc ctctagcaca taaccaagaa tataaggcta 300
cagattgcct ttcccagagg gaaaaccctg cagcaacctg ctgcctggaa aagtgtaaga 360
gcagatcact ggggaatcgt ttgccccccg ctgatggaca gcttccccaa gctccaaggg 420
caggtgctca gcatgtaccg tactgggatg gttgtcaata ctcctggtcc tgtaagagtc 480
ccaggacact gccatgccaa tgccccctca gttcctggca tcctttttgg gctgctcaca 540
gccccagcct ctatggtgaa gacatacttg ctagcagcgt caccaacttg ttgccaagag 600
atcagtgctc gaaggcaagg ttatttctaa ctgagcagag cctgccagga agaaagcgtt 660
tgcaccccac accactgtgc aggtgtgacc ggtgagctca cagctgcccc ccaggcatgc 720
ccagcccact taatcatcac agctcgacag ctctctcgcc cagcccagtt ctggaaggga 780
taaaaagggg catcaccgtt cctgggtaac agagccacct tctgcgtcct gctgagctct 840
gttctctcca gcacctccca acccactagt gcctggttct cttgctccac caggaacaag 900
ccaccatgtc tcgccagtca agtgtgtctt ccggagcggg gggcagtcgt agcttcagca 960
ccgcctctgc catcaccccg tctgtctccc gcaccagctt cacctccgtg tcccggtccg 1020
ggggtggcgg tggtggtggc ttcggcaggg tcagccttgc gggtgcttgt ggagtgggtg 1080
gctatggcag ccggagcctc tacaacctgg ggggctccaa gaggatatcc atcagcacta 1140
gtggtggcag cttcaggaac cggtttggtg ctggtgctgg aggcggctat ggctttggag 1200
gtggtgccgg tagtggattt ggtttcggcg gtggagctgg tggtggcttt gggctcggtg 1260
gcggagctgg ctttggaggt ggcttcggtg gccctggctt tcctgtctgc cctcctggag 1320
gtatccaaga ggtcactgtc aaccagagtc tcctgactcc cctcaacctg caaatcgacc 1380
ccagcatcca gagggtgagg accgaggagc gcgagcagat caagaccctc aacaataagt 1440
ttgcctcctt catcgacaag gtgcggttcc tggagcagca gaacaaggtt ctggaaacaa 1500
agtggaccct gctgcaggag cagggcacca agactgtgag gcagaacctg gagccgttgt 1560
tcgagcagta catcaacaac ctcaggaggc agctggacag catcgtgggg gaacggggcc 1620
gcctggactc agagctgaga aacatgcagg acctggtgga agacttcaag aacaagtatg 1680
aggatgaaat caacaagcgt accactgctg agaatgagtt tgtgatgctg aagaaggatg 1740
tagatgctgc ctacatgaac aaggtggagc tggaggccaa ggttgatgca ctgatggatg 1800
agattaactt catgaagatg ttctttgatg cggagctgtc ccagatgcag acgcatgtct 1860
ctgacacctc agtggtcctc tccatggaca acaaccgcaa cctggacctg gatagcatca 1920
tcgctgaggt caaggcccag tatgaggaga ttgccaaccg cagccggaca gaagccgagt 1980
cctggtatca gaccaagtat gaggagctgc agcagacagc tggccggcat ggcgatgacc 2040
tccgcaacac caagcatgag atctctgaga tgaaccggat gatccagagg ctgagagccg 2100
agattgacaa tgtcaagaaa cagtgcgcca atctgcagaa cgccattgcg gatgccgagc 2160
agcgtgggga gctggccctc aaggatgcca ggaacaagct ggccgagctg gaggaggccc 2220
tgcagaaggc caagcaggac atggcccggc tgctgcgtga gtaccaggag ctcatgaaca 2280
ccaagctggc cctggacgtg gagatcgcca cttaccgcaa gctgctggag ggcgaggaat 2340
gcagactcag tggagaagga gttggaccag tcaacatctc tgttgtcaca agcagtgttt 2400
cctctggata tggcagtggc agtggctatg gcggtggcct cggtggaggt cttggcggcg 2460
gcctcggtgg aggtcttgcc ggaggtagca gtggaagcta ctactccagc agcagtgggg 2520
gtgtcggcct aggtggtggg ctcagtgtgg ggggctctgg cttcagtgca agcagtagcc 2580
gagggctggg ggtgggcttt ggcagtggcg gggtagcag ctccagcgtc aaatttgtct 2640
ccaccacctc ctcctcccgg aagagcttca agagctaaga acctgctgca agtcactgcc 2700
ttccaagtgc agcaacccag cccatggaga ttgcctcttc taggcagttg ctcaagccat 2760
gttttatcct tttctggaga gtagtctaga ccaagccaat tgcagaacca cattctttgg 2820
ttcccaggag agccccattc ccagcccctg gtctcccgtg ccgcagttct atattctgct 2880
tcaaatcagc cttcaggttt cccacagcat ggcccctgct gacacgagaa cccaaagttt 2940
tcccaaatct aaatcatcaa aacagaatcc ccaccccaat cccaaatttt gttttggttc 3000
taactacctc cagaatgtgt tcaataaaat gttttataat ataagctggt gtgcagaatt 3060
gtttttttt tctacccaa 3079

<210> 117
<211> 6921

<212> DNA
<213> Homo sapiens

<400> 117
gaattctgac tgtccactca aaacttctat tccgatcaaa gctatctgtg actacagaca 60
aattgagata accatttaca aagacgatga atgtgttttg gcgaataact ctcatcgtgc 120
taaatggaag gtcattagtc ctactgggaa tgaggctatg gtcccatctg tgtgcttcac 180
cgttcctcca ccaaacaaag aagcggtgga ccttgccaac agaattgagc aacagtatca 240
gaatgtcctg actctttggc atgagtctca cataaacatg aagagtgtag tatcctggca 300
ttatctcatc aatgaaattg atagaattcg agctagcaat gtggcttcaa taaagacaat 360
gctacctggt gaacatcagc aagttctaag taatctacaa tctcgttttg aagattttct 420
ggaagatagc caggaatccc aagtcttttc aggctcagat ataacacaac tggaaaagga 480
ggttaatgta tgtaagcagt attatcaaga acttcttaaa tctgcagaaa gagaggagca 540
agaggaatca gtttataatc tctacatctc tgaagttcga aacattagac ttcggttaga 600
gaactgtgaa gatcggctga ttagacagat tcgaactccc ctggaaagag atgatttgca 660
tgaaagtgtg ttcagaatca cagaacagga gaaactaaag aaagagctgg aacgacttaa 720
agatgatttg ggaacaatca caaataagtg tgaggagttt ttcagtcaag cagcagcctc 780
ttcatcagtc cctaccctac gatcagagct taatgtggtc cttcagaaca tgaaccaagt 840
ctattctatg tcttccactt acatagataa gttgaaaact gttaacttgg tgttaaaaaa 900
cactcaagct gcagaagccc tcgtaaaact ctatgaaact aaactgtgtg aagaagaagc 960
agttatagct gacaagaata atattgagaa tctaataagt actttaaagc aatggagatc 1020
tgaagtagat gaaaagagac aggtattcca tgccttagag gatgagttgc agaaagctaa 1080
agccatcagt gatgaaatgt ttaaaacgta taaagaacgg gaccttgatt ttgactggca 1140
caaagaaaaa gcagatcaat tagttgaaag gtggcaaaat gttcatgtgc agattgacaa 1200
caggttacgg gacttagagg gcattggcaa atcactgaag tactacagag acacttacca 1260
tcctttagat gattggatcc agcaggttga aactactcag agaaagattc aggaaaatca 1320
gcctgaaaat agtaaaaccc tagccacaca gttgaatcaa cagaagatgc tggtgtccga 1380
aatagaaatg aaacagagca aaatggacga gtgtcaaaaa tatgcagaac agtactcagc 1440
tacagtgaag gactatgaat tacaaacaat gacctaccgg gccatggtag attcacaaca 1500
aaaatctcca gtgaaacgcc gaagaatgca gagttcagca gatctcatta ttcaagagtt 1560
catggaccta aggactcgat atactgccct ggtcactctc atgacacaat atattaaatt 1620
tgctggtgat tcattgaaga ggctggaaga ggaggagatt aaaaggtgta aggagacttc 1680
tgaacatggg gcatattcag atctgcttca gcgtcagaag gcaacagtgc ttgagaatag 1740
caaacttaca ggaaagataa gtgagttgga aagaatggta gctgaactaa agaaacaaaa 1800
gtcccgagta gaggaagaac ttccgaaggt cagggaggct gcagaaaatg aattgagaaa 1860
gcagcagaga aatgtagaag atatctctct gcagaagata agggctgaaa gtgaagccaa 1920
gcagtaccgc agggaacttg aaaccattgt gagagagaag gaagccgctg aaagagaact 1980
ggagcgggtg aggcagctca ccatagaggc cgaggctaaa agagctgccg tggaagagaa 2040
cctcctgaat tttcgcaatc agttggagga aaacaccttt accagacgaa cactggaaga 2100
tcatcttaaa agaaaagatt taagtctcaa tgatttggag caacaaaaaa ataaattaat 2160
ggaagaatta agaagaaaga gagacaatga ggaagaactc ttgaagctga taaagcagat 2220
ggaaaaagac cttgcatttc agaaacaggt agcagagaaa cagttgaaag aaaagcagaa 2280
aattgaattg gaagcaagaa gaaaaataac tgaaattcag tatacatgta gagaaaatgc 2340
attgccagtg tgtccgatca cacaggctac atcatgcagg gcagtaacgg gtctccagca 2400
agaacatgac aagcagaaag cagaagaact caaacagcag gtagatgaac taacagctgc 2460
caatagaaag gctgaacaag acatgagaga gctgacatat gaacttaatg ccctccagct 2520
tgaaaaaacg tcatctgagg aaaaggctcg tttgctaaaa gataaactag atgaaacaaa 2580
taatacactc agatgcctta agttggagct ggaaaggaag gatcaggcgg agaaagggta 2640
ttctcaacaa ctcagagagc ttggtaggca attgaatcaa accacaggta aagctgaaga 2700
agccatgcaa gaagctagtg atctcaagaa aataaagcgc aattatcagt tagaattaga 2760
atctcttaat catgaaaaag ggaaactaca aagagaagta gacagaatca caagggcaca 2820
tgctgtagct gagaagaata ttcagcattt aaattcacaa attcattctt ttcgagatga 2880
gaaagaatta gaaagactac aaatctgcca gagaaaatca gatcatctaa aagaacaatt 2940
tgagaaaagc catgagcagt tgcttcaaaa tatcaaagct gaaaaagaaa ataatgataa 3000
aatccaaagg ctcaatgaag aattggagaa aagtaatgag tgtgcagaga tgctaaaaca 3060
aaaagtagag gagcttacta ggcagaataa tgaaaccaaa ttaatgatgc agagaattca 3120
ggcagaatca gagaatatag ttttagagaa acaaactatc cagcaaagat gtgaagcact 3180
gaaaattcag gcagatggtt ttaaagatca gctacgcagc acaaatgaac acttgcataa 3240

acagacaaaa acagagcagg attttcaaag aaaaattaaa tgcctagaag aagacctggc 3300
gaaaagtcaa aatttggtaa gtgaatttaa gcaaaagtgt gaccaacaga acattatcat 3360
ccagaatacc aagaaagaag ttagaaatct gaatgcggaa ctgaatgctt ccaaagaaga 3420
gaagcgacgc ggggagcaga aagttcagct acaacaagct caggtgcaag agttaaataa 3480
caggttgaaa aaagtacaag acgaattaca cttaaagacc atagaggagc agatgaccca 3540
cagaaagatg gttctgtttc aggaagaatc tggtaaattc aaacaatcag cagaggagtt 3600
tcggaagaag atggaaaaat taatggagtc caaagtcatc actgaaaatg atatttcagg 3660
cattaggctt gactttgtgt ctcttcaaca agaaaactct agagcccaag aaaatgctaa 3720
gctttgtgaa acaaacatta aagaacttga aagacagctt caacagtatc gtgaacaaat 3780
gcagcaaggg cagcacatgg aagcaaatca ttaccaaaaa tgtcagaaac ttgaggatga 3840
gctgatagcc cagaagcgtg aggttgaaaa cctgaagcaa aaaatggacc aacagatcaa 3900
agagcatgaa catcaattag ttttgctcca gtgtgaaatt caaaaaaaga gcacagccaa 3960
agactgtacc ttcaaaccag attttgagat gacagtgaag gagtgccagc actctggaga 4020
gctgtcctct agaaacactg gacaccttca cccaacaccc agatcccctc tgttgagatg 4080
gactcaagaa ccacagccat tggaagagaa gtggcagcat cgggttgttg aacagatacc 4140
caaagaagtc caattccagc caccaggggc tccactcgag aaagagaaaa gccagcagtg 4200
ttactctgag tacttttctc agacaagcac cgagttacag ataacttttg atgagacaaa 4260
ccccattaca agactgtctg aaattgagaa gataagagac caagccctga acaattctag 4320
accacctgtt aggtatcaag ataacgcatg tgaaatggaa ctggtgaagg ttttgacacc 4380
cttagagata gctaagaaca agcagtatga tatgcataca gaagtcacaa cattaaaaca 4440
agaaaagaac ccagttccca gtgctgaaga atggatgctt gaagggtgca gagcatctgg 4500
tggactcaag aaaggggatt tccttaagaa gggcttagaa ccagagacct tccagaactt 4560
tgatggtgat catgcatgtt cagtcaggga tgatgaattt aaattccaag ggcttaggca 4620
cactgtgact gccaggcagt tggtggaagc taagcttctg gacatgagaa caattgagca 4680
gctgcgactc ggtcttaaga ctgttgaaga agttcagaaa actcttaaca agtttctgac 4740
gaaagccacc tcaattgcag ggctttacct agaatctaca aaagaaaaga tttcatttgc 4800
ctcagcggcc gagagaatca taatagacaa aatggtggct ttggcatttt tagaagctca 4860
ggctgcaaca ggttttataa ttgatcccat ttcaggtcag acatattctg ttgaagatgc 4920
agttcttaaa ggagttgttg accccgaatt cagaattagg cttcttgagg cagagaaggc 4980
agctgtggga tattcttatt cttctaagac attgtcagtg tttcaagcta tggaaaatag 5040
aatgcttgac agacaaaaag gtaaacatat cttggaagcc cagattgcca gtgggggtgt 5100
cattgaccct gtgagaggca ttcgtgttcc tccagaaatt gctctgcagc aggggttgtt 5160
gaataatgcc atcttacagt ttttacatga gccatccagc aacacaagag ttttccctaa 5220
tcccaataac aagcaagctc tgtattactc agaattactg cgaatgtgtg tatttgatgt 5280
agagtcccaa tgctttctgt ttccatttgg ggagaggaac atttccaatc tcaatgtcaa 5340
gaaaacacat agaatttctg tagtagatac taaaacagga tcagaattga ccgtgtatga 5400
ggctttccag agaaacctga ttgagaaaag tatatatctt gaactttcag ggcagcaata 5460
tcagtggaag gaagctatgt tttttgaatc ctatgggcat tcttctcata tgctgactga 5520
tactaaaaca ggattacact tcaatattaa tgaggctata gagcagggaa caattgacaa 5580
agccttggtc aaaaagtatc aggaaggcct catcacactt acagaacttg ctgattcttt 5640
gctgagccgg ttagtcccca agaaagattt gcacagtcct gttgcagggt attggctgac 5700
tgctagtggg gaaaggatct ctgtactaaa agcctcccgt agaaatttgg ttgatcggat 5760
tactgccctc cgatgccttg aagcccaagt cagtacaggg ggcataattg atcctcttac 5820
tggcaaaaag taccgggtgg ccgaagcttt gcatagaggc ctggttgatg aggggtttgc 5880
ccagcagctg cgacagtgtg aattagtaat cacagggatt ggccatccca tcactaacaa 5940
aatgatgtca gtggtggaag ctgtgaatgc aaatattata aataaggaaa tgggaatccg 6000
atgtttggaa tttcagtact tgacaggagg gttgatagag ccacaggttc actctcggtt 6060
atcaatagaa gaggctctcc aagtaggtat tatagatgtc ctcattgcca caaaactcaa 6120
agatcaaaag tcatatgtca gaaatataat atgccctcag acaaaaagaa agttgacata 6180
taaagaagcc ttagaaaaag ctgattttga tttccacaca ggacttaaac tgttagaagt 6240
atctgagccc ctgatgacag gaatttctag cctctactat tcttcctaat gggacatgtt 6300
taaataactg tgcaaggggt gatgcaggct ggttcatgcc actttttcag agtatgatga 6360
tatcggctac atatgcagtc tgtgaattat gtaacatact ctatttcttg agggctgcaa 6420
attgctaagt gctcaaaata gagtaagttt taaattgaaa attacataag atttaatgcc 6480
cttcaaatgg tttcatttag ccttgagaat ggttttttga aacttggcca cactaaaatg 6540
tttttttttt tttacgtaga atgtgggata aacttgatga actccaagtt cacagtgtca 6600
tttcttcaga actccccttc attgaatagt gatcatttat taaatgataa attgcactcg 6660
ctgaaagagc acgtcatgaa gcaccatgga atcaaagaga aagatataaa ttcgttccca 6720

cagccttcaa gctgcagtgt tttagattgc ttcaaaaaat gaaaaagttt tgcctttttc 6780
gatatagtga ccttctttgc atattaaaat gtttaccaca atgtcccatt tctagttaag 6840
tcttcgcact tgaaagctaa cattatgaat attatgtgtt ggaggagggg aaggattttc 6900
ttcattctgt gtattttccg g 6921

<210> 118
<211> 946
<212> DNA
<213> Homo sapiens

<400> 118
cttctgactg ggctcaggct gacaggtaga gctcaccatg gcttcttgtg tccttgtccc 60
ctccccatca cagctgtggt gcagtccacc gtctccagtg gctatggcgg tgccagtggt 120
gtcggcagtg gcttaggcct gggtggagga agcagctact cctatggcag tggtcttggc 180
gttggaggtg gcttcagttc cagcagtggc agagccattg ggggtggcct cagctctgtt 240
ggaggcggca gttccaccat caagtacacc accacctcct cctccagcag gaagagctat 300
aagcactaaa gtgcgtctgc tagctctcgg tcccacagtc ctcaggcccc tctctggctg 360
cagagccctc tcctcaggtt gcctgtcctc tcctggcctc cagtctcccc tgctgtccca 420
ggtagagctg gggatgaatg cttagtgccc tcacttcttc tctctctctc tataccatct 480
gagcacccat tgctcaccat cagatcaacc tctgatttta catcatgatg taatcaccac 540
tggagcttca ctgttactaa attattaatt tcttgcctcc agtgttctat ctctgaggct 600
gagcattata agaaaatgac ctctgctcct tttcattgca gaaaattgcc aggggcttat 660
ttcagaacaa cttccactta ctttccactg gctctcaaac tctctaactt ataagtgttg 720
tgaaccccca cccaggcagt atccatgaaa gcacaagtga ctagtcctat gatgtacaaa 780
gcctgtatct ctgtgatgat ttctgtgctc ttcactgttt gcaattgcta aataaagcag 840
atttataata catatattct tttactttgc cttgctttgg ggccaaagtt ttgggcttaa 900
acttttttat ctgataagtg aatagttgtt tttaaaagat aatcta 946

<210> 119
<211> 8948
<212> DNA
<213> Homo sapiens

<400> 119
tcaacagccc ctgctccttg ggcccctcca tgccatgccg taatctctcc cacccgacca 60
acaccaacac ccagctccga cgcagctcct ctgcgccctt gccgccctcc gagccacagc 120
tttcctcccg ctcctgcccc cggcccgtcg ccgtctccgc gctcgcagcg gcctcgggag 180
ggcccaggta gcgagcagcg acctcgcgag ccttccgcac tcccgcccgg ttccccggcc 240
gtccgcctat ccttggcccc ctccgctttc tccgcgccgg cccgcctcgc ttatgcctcg 300
gcgctgagcc gctctcccga ttgcccgccg acatgagctg caacggaggc tcccacccgc 360
ggatcaacac tctgggccgc atgatccgcg ccgagtctgg cccggacctg cgctacgagg 420
tgaccagcgg cggcgggggc accagcagga tgtactattc tcggcgcggc gtgatcaccg 480
accagaactc ggacggctac tgtcaaaccg gcacgatgtc caggcaccag aaccagaaca 540
ccatccagga gctgctgcag aactgctccg actgcttgat gcgagcagag ctcatcgtgc 600
agcctgaatt gaagtatgga gatggaatac aactgactcg gagtcgagaa ttggatgagt 660
gttttgccca ggccaatgac caaatggaaa tcctcgacag cttgatcaga gagatgcggc 720
agatggccca gccctgtgat gcttaccaga aaaggcttct tcagctccaa gagcaaatgc 780
gagcccttta taaagccatc agtgtccctc gagtccgcag ggccagctcc aagggtggtg 840
gaggctacac ttgtcagagt ggctctggct gggatgagtt caccaaacat gtcaccagtg 900
aatgtttggg gtggatgagg cagcaaaggg cggagatgga catggtggcc tggggtgtgg 960
acctggcctc agtggagcag cacattaaca gccaccgggg catccacaac tccatcggcg 1020
actatcgctg gcagctggac aaaatcaaag ccgacctgcg cgagaaatct gcgatctacc 1080
agttggagga ggagtatgaa aacctgctga aagcgtcctt tgagaggatg gatcacctgc 1140
gacagctgca gaacatcatt caggccacgt ccagggagat catgtggatc aatgactgcg 1200
aggaggagga gctgctgtac gactggagcg acaagaacac caacatcgct cagaaacagg 1260
aggccttctc catacgcatg agtcaactgg aagttaaaga aaaagagctc aataagctga 1320

aacaagaaag tgaccaactt gtcctcaatc agcatccagc ttcagacaaa attgaggcct 1380
atatggacac tctgcagacg cagtggagtt ggattcttca gatcaccaag tgcattgatg 1440
ttcatctgaa agaaaatgct gcctactttc agtttttgga agaggcgcag tctactgaag 1500
catacctgaa ggggctccag gactccatca ggaagaagta cccctgcgac aagaacatgc 1560
ccctgcagca cctgctggaa cagatcaagg agctggagaa agaacgagag aaaatccttg 1620
aatacaagcg tcaggtgcag aacttggtaa acaagtctaa gaagattgta cagctgaagc 1680
ctcgtaaccc agactacaga agcaataaac ccattattct cagagctctc tgtgactaca 1740
aacaagatca gaaaatcgtg cataaggggg atgagtgtat cctgaaggac aacaacgagc 1800
gcagcaagtg gtacgtgacg ggcccgggag gcgttgacat gcttgttccc tctgtggggc 1860
tgatcatccc tcctccgaac ccactggccg tggacctctc ttgcaagatt gagcagtact 1920
acgaagccat cttggctctg tggaaccagc tctacatcaa catgaagagc ctggtgtcct 1980
ggcactactg catgattgac atagagaaga tcagggccat gacaatcgcc aagctgaaaa 2040
caatgcggca ggaagattac atgaagacga tagccgacct tgagttacat taccaagagt 2100
tcatcagaaa tagccaaggc tcagagatgt ttggagatga tgacaagcgg aaaatacagt 2160
ctcagttcac cgatgcccag aagcattacc agaccctggt cattcagctc cctggctatc 2220
cccagcacca gacagtgacc acaactgaaa tcactcatca tggaacctgc caagatgtca 2280
accataataa agtaattgaa accaacagag aaaatgacaa gcaagaaaca tggatgctga 2340
tggagctgca gaagattcgc aggcagatag agcactgcga gggcaggatg actctcaaaa 2400
acctccctct agcagaccag gggtcttctc accacatcac agtgaaaatt aacgagctta 2460
agagtgtgca gaatgattca caagcaattg ctgaggttct caaccagctt aaagatatgc 2520
ttgccaactt cagaggttct gaaaagtact gctatttaca gaatgaagta tttggactat 2580
ttcagaaact ggaaaatatc aatggtgtta cagatggcta cttaaatagc ttatgcacag 2640
taagggcact gctccaggct attctccaaa cagaagacat gttaaaggtt tatgaagcca 2700
ggctcactga ggaggaaact gtctgcctgg acctggataa agtggaagct taccgctgtg 2760
gactgaagaa aataaaaaat gacttgaact tgaagaagtc gttgttggcc actatgaaga 2820
cagaactaca gaaagcccag cagatccact ctcagacttc acagcagtat ccactttatg 2880
atctggactt gggcaagttc ggtgaaaaag tcacacagct gacagaccgc tggcaaagga 2940
tagataaaca gatcgacttt agattatggg acctggagaa acaaatcaag caattgagga 3000
attatcgtga taactatcag gctttctgca agtggctcta tgatcgtaaa cgccgccagg 3060
attccttaga atccatgaaa tttggagatt ccaacacagt catgcggttt ttgaatgagc 3120
agaagaactt gcacagtgaa atatctggca aacgagacaa atcagaggaa gtacaaaaaa 3180
ttgctgaact ttgcgccaat tcaattaagg attatgagct ccagctggcc tcatacacct 3240
caggactgga aactctgctg aacataccta tcaagaggac catgattcag tccccttctg 3300
gggtgattct gcaagaggct gcagatgttc atgctcggta cattgaacta cttacaagat 3360
ctggagacta ttacaggttc ttaagtgaga tgctgaagag tttggaagat ctgaagctga 3420
aaaataccaa gatcgaagtt ttggaagagg agctcagact ggcccgagat gccaactcgg 3480
aaaactgtaa taagaacaaa ttcctggatc agaacctgca gaaataccag gcagagtgtt 3540
cccagttcaa agcgaagctt gcgagcctgg aggagctgaa gagacaggct gagctggatg 3600
ggaagtcggc taagcaaaat ctagacaagt gctacggcca aataaaagaa ctcaatgaga 3660
agatcacccg actgacttat gagattgaag atgaaaagag aagaagaaaa tctgtggaag 3720
acagatttga ccaacagaag aatgactatg accaactgca gaaagcaagg caatgtgaaa 3780
aggagaacct tggttggcag aaattagagt ctgagaaagc catcaaggag aaggagtacg 3840
agattgaaag gttgagggtt ctactgcagg aagaaggcac ccggaagaga gaatatgaaa 3900
atgagctggc aaaggtaaga aaccactata atgaggagat gagtaattta aggaacaagt 3960
atgaaacaga gattaacatt acgaagacca ccatcaagga gatatccatg caaaaagagg 4020
atgattccaa aaatcttaga aaccagcttg atagactttc aagggaaaat cgagatctga 4080
aggatgaaat tgtcaggctc aatgacagca tcttgcaggc cactgagcag cgaaggcgag 4140
ctgaagaaaa cgcccttcag caaaaggcct gtggctctga gataatgcag aagaagcagc 4200
atctggagat agaactgaag caggtcatgc agcagcgctc tgaggacaat gcccggcaca 4260
agcagtccct ggaggaggct gccaagacca ttcaggacaa aaataaggag atcgagagac 4320
tcaaagctga gtttcaggag gaggccaagc gccgctggga atatgaaaat gaactgagta 4380
aggtaagaaa caattatgat gaggagatca ttagcttaaa aaatcagttt gagaccgaga 4440
tcaacatcac caagaccacc atccaccagc tcaccatgca gaaggaagag gataccagtg 4500
gctaccgggc tcagatagac aatctcaccc gagaaaacag gagcttatct gaagaaataa 4560
agaggctgaa gaacactcta acccagacca cagagaatct caggagggtg gaagaagaca 4620
tccaacagca aaaggccact ggctctgagg tgtctcagag gaaacagcag ctggaggttg 4680
agctgagaca agtcactcag atgcgaacag aggagagcgt aagatataag caatctcttg 4740
atgatgctgc caaaaccatc caggataaaa acaaggagat agaaaggtta aaacaactga 4800

tcgacaaaga aacaaatgac cggaaatgcc tggaagatga aaacgcgaga ttacaaaggg 4860
tccagtatga cctgcagaaa gcaaacagta gtgcgacgga gacaataaac aaactgaagg 4920
ttcaggagca agaactgaca cgcctgagga tcgactatga aagggtttcc caggagagga 4980
ctgtgaagga ccaggatatc acgcggttcc agaactctct gaaagagctg cagctgcaga 5040
agcagaaggt ggaagaggag ctgaatcggc tgaagaggac cgcgtcagaa gactcctgca 5100
agaggaagaa gctggaggaa gagctggaag gcatgaggag gtcgctgaag gagcaagcca 5160
tcaaaatcac caacctgacc cagcagctgg agcaggcatc cattgttaag aagaggagtg 5220
aggatgacct ccggcagcag agggacgtgc tggatggcca cctgagggaa aagcagagga 5280
cccaggaaga gctgaggagg ctctcttctg aggtcgaggc cctgaggcgg cagttactcc 5340
aggaacagga aagtgtcaaa caagctcact tgaggaatga gcatttccag aaggcgatag 5400
aagataaaag cagaagctta aatgaaagca aaatagaaat tgagaggctg cagtctctca 5460
cagagaacct gaccaaggag cacttgatgt tagaagaaga actgcggaac ctgaggctgg 5520
agtacgatga cctgaggaga ggacgaagcg aagcggacag tgataaaaat gcaaccatct 5580
tggaactaag gagccagctg cagatcagca acaaccggac cctggaactg caggggctga 5640
ttaatgattt acagagagag agggaaaatt tgagacagga aattgagaaa ttccaaaagc 5700
aggctttaga ggcatctaat aggattcagg aatcaaagaa tcagtgtact caggtggtac 5760
aggaaagaga gagccttctg gtgaaaatca aagtcctgga gcaagacaag gcaaggctgc 5820
agaggctgga ggatgagctg aatcgtgcaa aatcaactct agaggcagaa accaggtgaa 5880
aacagcgcct ggagtgtgag aaacagcaaa ttcagaatga cctgaatcag tggaagactc 5940
aatattcccg caaggaggag gctattagga agatagaatc ggaaagagaa aagagtgaga 6000
gagagaagaa cagtcttagg agtgagatcg aaagactcca agcagagatc aagagaattg 6060
aagagaggtg caggcgtaag ctggaggatt ctaccaggga gacacagtca cagttagaaa 6120
cagaacgctc ccgatatcag agggagattg ataaactcag acagcgccca tatgggtccc 6180
atcgagagac ccagactgag tgtgagtgga ccgttgacac ctccaagctg gtgtttgatg 6240
ggctgaggaa gaaggtgaca gcaatgcagc tctatgagtg tcagctgatc gacaaaacaa 6300
ccttggacaa actattgaag gggaagaagt cagtggaaga agttgcttct gaaatccagc 6360
cattccttcg gggtgcagga tctatcgctg gagcatctgc ttctcctaag gaaaaatact 6420
ctttggtaga ggccaagaga aagaaattaa tcagcccaga atccacagtc atgcttctgg 6480
aggcccaggc agctacaggt ggtataattg atccccatcg gaatgagaag ctgactgtcg 6540
acagtgccat agctcgggac ctcattgact tcgatgaccg tcagcagata tatgcagcag 6600
aaaaagctat cactggtttt gatgatccat tttcaggcaa gacagtatct gtttcagaag 6660
ccatcaagaa aaatttgatt gatagagaaa ccggaatgcg cctgctggaa gcccagattg 6720
cttcaggggg tgtagtagac cctgtgaaca gtgtcttttt gccaaaagat gtcgccttgg 6780
cccgggggct gattgataga gatttgtatc gatccctgaa tgatccccga gatagtcaga 6840
aaaactttgt ggatccagtc accaaaaaga aggtcagtta cgtgcagctg aaggaacggt 6900
gcagaatcga accacatact ggtctgctct tgctttcagt acagaagaga agcatgtcct 6960
tccaaggaat cagacaacct gtgaccgtca ctgagctagt agattctggt atattgagac 7020
cgtccactgt caatgaactg gaatctggtc agatttctta tgacgaggtt ggtgagagaa 7080
ttaaggactt cctccagggt tcaagctgca tagcaggcat atacaatgag accacaaaac 7140
agaagcttgg catttatgag gccatgaaaa ttggcttagt ccgacctggt actgctctgg 7200
agttgctgga agcccaagca gctactggct ttatagtgga tcctgttagc aacttgaggt 7260
taccagtgga ggaagcctac aagagaggtc tggtgggcat tgagttcaaa gagaagctcc 7320
tgtctgcaga acgagctgtc actgggtata atgatcctga aacaggaaac atcatctctt 7380
tgttccaagc catgaataag gaactcatcg aaaaggccca cggtattcgc ttattagaag 7440
cacagatcgc aaccgggggg atcattgacc caaaggagag ccatcgttta ccagttgaca 7500
tagcatataa gaggggctat ttcaatgagg aactcagtga gattctctca gatccaagtg 7560
atgataccaa aggatttttt gaccccaaca ctgaagaaaa tcttacctat ctgcaactaa 7620
aagaaagatg cattaaggat gaggaaacag ggctctgtct tctgcctctg aaagaaaaga 7680
agaaacaggt gcagacatca caaaagaata ccctcaggaa gcgtagagtg gtcatagttg 7740
acccagaaac caataaagaa atgtctgttc aggaggccta caagaagggc ctaattgatt 7800
atgaaacctt caaagaactg tgtgagcagg aatgtgaatg ggaagaaata accatcacgg 7860
gatcagatgg ctccaccagg gtggtcctgg tagatagaaa gacaggcagt cagtatgata 7920
ttcaagatgc tattgacaag ggccttgttg acaggaagtt ctttgatcag taccgatccg 7980
gcagcctcag cctcactcaa tttgctgaca tgatctcctt gaaaaatggt gtcggcacca 8040
gcagcagcat gggcagtggt gtcagcgatg atgtttttag cagctcccga catgaatcag 8100
taagtaagat ttccaccata tccagcgtca ggaatttaac cataaggagc agctcttttt 8160
cagacaccct ggaagaatcg agccccattg cagccatctt tgacacagaa aacctggaga 8220
aaatctccat tacagaaggt atagagcggg gcatcgttga cagcatcacg ggtcagaggc 8280

ttctggaggc tcaggcctgc acaggtggca tcatccaccc aaccacgggc cagaagctgt 8340
cacttcagga cgcagtctcc cagggtgtga ttgaccaaga catggccacc agcgtgaagc 8400
ctgctcagaa agccttcata ggcttcgagg gtgtgaaggg aaagaagaag atgtcagcag 8460
cagaggcagt gaaagaaaaa tggctcccgt atgaggctgg ccagcgcttc ctggagttcc 8520
agtacctcac gggaggtctt gttgacccgg aagtgcatgg gaggataagc accgaagaag 8580
ccatccggaa ggggttcata gatggccgcg ccgcacagag gctgcaagac accagcagct 8640
atgccaaaat cctgacctgc cccaaaacca aattaaaaat atcctataag gatgccataa 8700
atcgctccat ggtagaagat atcactgggc tgcgccttct ggaagccgcc tccgtgtcgt 8760
ccaagggctt acccagccct tacaacatgt cttcggctcc ggggtcccgc tccggctccc 8820
gctcgggatc tcgctccgga tctcgctccg ggtcccgcag tgggtcccgg agaggaagct 8880
ttgacgccac agggaattct tcctactctt attcctactc atttagcagt agttctattg 8940
ggcactag 8948

<210> 120
<211> 587
<212> DNA
<213> Homo sapiens

<220>
<221> misc_feature
<222> 91, 131, 256, 263, 332, 392, 400, 403, 461, 496, 497, 499,
510, 511, 518, 519, 539, 554, 560, 576
<223> n = A,T,C or G

<400> 120
cgtcctaagc acttagacta catcagggaa gaacacagac cacatccctg tcctcatgcg 60
gcttatgttt tctggaagaa agtggagacc nagtccttgg ctttagggct ccccggctgg 120
gggctgtgca ntccggtcag ggcgggaagg gaaatgcacc gctgcatgtg aacttacagc 180
ccaggcggat gccccttccc ttagcactac ctggcctcct gcatcccctc gcctcatgtt 240
cctcccacct tcaaanaatg aanaacccca tgggcccagc cccttgccct ggggaaccaa 300
ggcagccttc caaaactcag gggctgaagc anactattag ggcaggggct gactttgggt 360
gacactgccc attccctctc agggcagctc angtcacccn ggnctcttga acccagcctg 420
ttcctttgaa aaagggcaaa actgaaaagg gcttttccta naaaaagaaa aaccagggaa 480
ctttgccagg gcttcnntnt taccaaaacn ncttctcnng gatttttaat tccccattng 540
gcctccactt accnggggcn atgccccaaa attaanaatt tcccatc 587

<210> 121
<211> 619
<212> DNA
<213> Homo sapiens

<220>
<221> misc_feature
<222> 260, 527, 560, 564, 566, 585, 599
<223> n = A,T,C or G

<400> 121
   cactagtagg atagaaacac tgtgtcccga gagtaaggag agaagctact attgattaga 60
   gcctaaccca ggttaactgc aagaagaggc gggatacttt cagctttcca tgtaactgta 120
   tgcataaagc caatgtagtc cagtttctaa gatcatgttc caagctaact gaatcccact 180
   tcaatacaca ctcatgaact cctgatggaa caataacagg cccaagcctg tggtatgatg 240
   tgcacacttg ctagactcan aaaaaatact actctcataa atgggtggga gtattttggt 300
   gacaacctac tttgcttggc tgagtgaagg aatgatattc atatattcat ttattccatg 360
   gacatttagt tagtgctttt tatataccag gcatgatgct gagtgacact cttgtgtata 420
   tttccaaatt tttgtacagt cgctgcacat atttgaaatc atatattaag acttccaaaa 480
   aatgaagtcc ctggtttttc atggcaactt gatcagtaaa ggattcncct ctgtttggta 540
   cttaaaacat ctactatatn gttnanatga aattcctttt ccccncctcc cgaaaaaana 600
   aagtggtggg gaaaaaaaa 619

<210> 122
<211> 1475
<212> DNA
<213> Homo sapiens

<400> 122
tccacctgtc cccgcagcgc cggctcgcgc cctcctgccg cagccaccga gccgccgtct 60
agcgccccga cctcgccacc atgagagccc tgctggcgcg cctgcttctc tgcgtcctgg 120
tcgtgagcga ctccaaaggc agcaatgaac ttcatcaagt tccatcgaac tgtgactgtc 180
taaatggagg aacatgtgtg tccaacaagt acttctccaa cattcactgg tgcaactgcc 240
caaagaaatt cggagggcag cactgtgaaa tagataagtc aaaaacctgc tatgagggga 300
atggtcactt ttaccgagga aaggccagca ctgacaccat gggccggccc tgcctgccct 360
ggaactctgc cactgtcctt cagcaaacgt accatgccca cagatctgat gctcttcagc 420
tgggcctggg gaaacataat tactgcagga acccagacaa ccggaggcga ccctggtgct 480
atgtgcaggt gggcctaaag ccgcttgtcc aagagtgcat ggtgcatgac tgcgcagatg 540
gaaaaaagcc ctcctctcct ccagaagaat taaaatttca gtgtggccaa aagactctga 600
ggccccgctt taagattatt gggggagaat tcaccaccat cgagaaccag ccctggtttg 660
cggccatcta caggaggcac cgggggggct ctgtcaccta cgtgtgtgga ggcagcctca 720
tcagcccttg ctgggtgatc agcgccacac actgcttcat tgattaccca aagaaggagg 780
actacatcgt ctacctgggt cgctcaaggc ttaactccaa cacgcaaggg gagatgaagt 840
ttgaggtgga aaacctcatc ctacacaagg actacagcgc tgacacgctt gctcaccaca 900
acgacattgc cttgctgaag atccgttcca aggagggcag gtgtgcgcag ccatcccgga 960
ctatacagac catctgcctg ccctcgatgt ataacgatcc ccagtttggc acaagctgtg 1020
agatcactgg ctttggaaaa gagaattcta ccgactatct ctatccggag cagctgaaga 1080
tgactgttgt gaagctgatt tcccaccggg agtgtcagca gccccactac tacggctctg 1140
aagtcaccac caaaatgctg tgtgctgctg acccacagtg gaaaacagat tcctgccagg 1200
gagactcagg gggacccctc gtctgttccc tccaaggccg catgactttg actggaattg 1260
tgagctgggg ccgtggatgt gccctgaagg acaagccagg cgtctacacg agagtctcac 1320
acttcttacc ctggatccgc agtcacacca aggaagagaa tggcctggcc ctctgagggt 1380
ccccagggag gaaacgggca ccacccgctt tcttgctggt tgtcattttt gcagtagagt 1440
catctccatc agctgtaaga agagactggg aagat 1475

<210> 123
<211> 2294
<212> DNA
<213> Homo sapiens

<400> 123
cagcgccggc tcgcgccctc ctgccgcagc caccgagccg ccgtctagcg ccccgacctc 60
gccaccatga gagccctgct ggcgcgcctg cttctctgcg tcctggtcgt gagcgactcc 120
aaaggcagca atgaacttca tcaagttcca tcgaactgtg actgtctaaa tggaggaaca 180
tgtgtgtcca acaagtactt ctccaacatt cactggtgca actgcccaaa gaaattcgga 240
gggcagcact gtgaaataga taagtcaaaa acctgctatg aggggaatgg tcacttttac 300
cgaggaaagg ccagcactga caccatgggc cggccctgcc tgccctggaa ctctgccact 360
gtccttcagc aaacgtacca tgcccacaga tctgatgctc ttcagctggg cctggggaaa 420
cataattact gcaggaaccc agacaaccgg aggcgaccct ggtgctatgt gcaggtgggc 480
ctaaagccgc ttgtccaaga gtgcatggtg catgactgcg cagatggaaa aaagccctcc 540
tctcctccag aagaattaaa atttcagtgt ggccaaaaga ctctgaggcc ccgctttaag 600
attattgggg gagaattcac caccatcgag aaccagccct ggtttgcggc catctacagg 660
aggcaccggg ggggctctgt cacctacgtg tgtggaggca gcctcatcag cccttgctgg 720
gtgatcagcg ccacacactg cttcattgat tacccaaaga aggaggacta catcgtctac 780
ctgggtcgct caaggcttaa ctccaacacg caaggggaga tgaagtttga ggtggaaaac 840
ctaatcctac acaaggacta cagcgctgac acgcttgctc accacaacga cattgccttg 900
ctgaagatcc gttccaagga gggcaggtgt gcgcagccat cccggactat acagaccatc 960
tgcctgccct cgatgtataa cgatccccag tttggcacaa gctgtgagat cactggcttt 1020

ggaaaagaga attctaccga ctatctctat ccggagcagc tgaaaatgac tgttgtgaag 1080
ctgatttccc accgggagtg tcagcagccc cactactacg gctctgaagt caccaccaaa 1140
atgctgtgtg ctgctgaccc acagtggaaa acagattcct gccagggaga ctcaggggga 1200
cccctcgtct gttccctcca aggccgcatg actttgactg gaattgtgag ctggggccgt 1260
ggatgtgccc tgaaggacaa gccaggcgtc tacacgagag tctcacactt cttaccctgg 1320
atccgcagtc acaccaagga agagaatggc ctggccctct gagggtcccc agggaggaaa 1380
cgggcaccac ccgctttctt gctggttgct attttgcagt agagtcatct ccatcagctg 1440
taagaagagc tgggaatata ggctctgcac agatggattt gcctgtgcca ccaccagggc 1500
gaacgacaat agctttaccc tcaggcatag gcctgggtgc tggctgccca gacccctctg 1560
gccaggatgg aggggtggtc ctgactcaac atgttactga ccagcaactt gtctttttct 1620
ggactgaagc ctgcaggagt taaaaagggc agggcatctc ctgtgcatgg gctcgaaggg 1680
agagccagct cccccgaccg gtgggcattt gtgaggccca tggttgagaa atgaataatt 1740
tcccaattag gaagtgtaag cagctgaggt ctcttgaggg agcttagcca atgtgggagc 1800
agcggtttgg ggagcagaga cactaacgac ttcagggcag ggctctgata ttccatgaat 1860
gtatcaggaa atatatatgt gtgtgtatgt ttgcacactt gtgtgtgggc tgtgagtgta 1920
agtgtgagta agagctggtg tctgattgtt aagtctaaat atttccttaa actgtgtgga 1980
ctgtgatgcc acacagagtg gtctttctgg agaggttata ggtcactcct ggggcctctt 2040
gggtccccca cgtgacagtg cctgggaatg tattattctg cagcatgacc tgtgaccagc 2100
actgtctcag tttcactttc acatagatgt ccctttcttg gccagttatc ccttcctttt 2160
agcctagttc atccaatcct cactgggtgg ggtgaggacc actcctgtac actgaatatt 2220
tatatttcac tatttttatt tatatttttg taattttaaa taaaagtgat caataaaatg 2280
tgatttttct gatg 2294

<210> 124
<211> 956
<212> DNA
<213> Homo sapiens

<400> 124
gatgagttcc gcaccaagtt tgagacagac caggccctgc gcctgagtgt ggaggccgac 60
atcaatggcc tgcgcagggt gctggatgag ctgaccctgg ccagagccga cctggagatg 120
cagattgaga acctcaagga ggagctggcc tacctgaaga agaaccacga ggaggagatg 180
aacgccctgc gaggccaggt gggtggtgag atcaatgtgg agatggacgc tgccccaggc 240
gtggacctga gccgcatcct caacgagatg cgtgaccagt atgagaagat ggcagagaag 300
aaccgcaagg atgccgagga ttggttcttc agcaagacag aggaactgaa ccgcgaggtg 360
gccaccaaca gtgagctggt gcagagtggc aagagtgaga tctcggagct ccggcgcacc 420
atgcaggcct tggagataga gctgcagtcc cagctcagca tgaaagcatc cctggagggc 480
aacctggcgg agacagagaa ccgctactgc gtgcagctgt cccagatcca ggggctgatt 540
ggcagcgtgg aggagcagct ggcccagctt cgctgcgaga tggagcagca gaaccaggaa 600
tacaaaatcc tgctggatgt gaagacgcgg ctggagcagg agattgccac ctaccgccgc 660
ctgctggagg gagaggatgc ccacctgact cagtacaaga aagaaccggt gaccacccgt 720
caggtgcgta ccattgtgga agaggtccag gatggcaagg tcatctcctc ccgcgagcag 780
gtccaccaga ccacccgctg aggactcagc taccccggcc ggccacccag gaggcaggga 840
cgcagccgcc ccatctgccc cacagtctcc ggcctctcca gcctcagccc cctgcttcag 900
tcccttcccc atgcttcctt gcctgatgac aataaaagct tgttgactca gctatg 956

<210> 125
<211> 486
<212> DNA
<213> Homo sapiens

<220>
<221> misc_feature
<222> 16
<223> n = A,T,C or G

<400> 125
aaattatata tagtgnttca gctcccattg tggtgttcat agtcttctag gaacagataa 60

acttaagtat tcaattcact cttggcattt tttctttaat ataggctttt tagcctattt 120
ttggaaaact gcttttcttc tgagaacctt attctgaatg tcatcaactt taccaaacct 180
tctaagtcca gagctaactt agtactgttt aagttactat tgactgaatt ttcttcattt 240
tctgtttagc cagtgttacc aaggtaagct ggggaatgaa gtataccaac ttctttcaga 300
gcattttagg acattatggc agctttagaa ggctgtcttg tttctagcca agggagagcc 360
agcgcaggtt ttggatacta gagaaagtca tttgcttgta ctattgccat tttagaaagc 420
tctgatgtga attcaaattt tacctctgtt acttaaagcc aacaatttta aggcagtagt 480
tttact 486

<210> 126
<211> 3552
<212> DNA
<213> Homo sapiens

<400> 126
   cggcaggcag gtctcgtctc ggcaccctcc cggcgcccgc gttctcctgg ccctgcccgg 60
   catcccgatg gccgccgctg ggccccggcg ctccgtgcgc ggagccgtct gcctgcatct 120
   gctgctgacc ctcgtgatct tcagtcgtgc tggtgaagcc tgcaaaaagg tgatacttaa 180
   tgtaccttct aaactagagg cagacaaaat aattggcaga gttaatttgg aagagtgctt 240
   caggtctgca gacctcatcc ggtcaagtga tcctgatttc agagttctaa atgatgggtc 300
   agtgtacaca gccagggctg ttgcgctgtc tgataagaaa agatcattta ccatatggct 360
   ttctgacaaa aggaaacaga cacagaaaga ggttactgtg ctgctagaac atcagaagaa 420
   ggtatcgaag acaagacaca ctagagaaac tgttctcagg cgtgccaaga ggagatgggc 480
   acctattcct tgctctatgc aagagaattc cttgggccct ttcccattgt ttcttcaaca 540
   agttgaatct gatgcagcac agaactatac tgtcttctac tcaataagtg gacgtggagt 600
   tgataaagaa cctttaaatt tgttttatat agaaagagac actggaaatc tattttgcac 660
   tcggcctgtg gatcgtgaag aatatgatgt ttttgatttg attgcttatg cgtcaactgc 720
   agatggatat tcagcagatc tgcccctccc actacccatc agggtagagg atgaaaatga 780
   caaccaccct gttttcacag aagcaattta taattttgaa gttttggaaa gtagtagacc 840
   tggtactaca gtgggggtgg tttgtgccac agacagagat gaaccggaca caatgcatac 900
   gcgcctgaaa tacagcattt tgcagcagac accaaggtca cctgggctct tttctgtgca 960
   tcccagcaca ggcgtaatca ccacagtctc tcattatttg gacagagagg ttgtagacaa 1020
   gtactcattg ataatgaaag tacaagacat ggatggccag ttttttggat tgataggcac 1080
   atcaacttgt atcataacag taacagattc aaatgataat gcacccactt tcagacaaaa 1140
   tgcttatgaa gcatttgtag aggaaaatgc attcaatgtg gaaatcttac gaatacctat 1200
   agaagataag gatttaatta acactgccaa ttggagagtc aattttacca ttttaaaggg 1260
   aaatgaaaat ggacatttca aaatcagcac agacaaagaa actaatgaag gtgttctttc 1320
   tgttgtaaag ccactgaatt atgaagaaaa ccgtcaagtg aacctggaaa ttggagtaaa 1380
   caatgaagcg ccatttgcta gagatattcc cagagtgaca gccttgaaca gagccttggt 1440
   tacagttcat gtgagggatc tggatgaggg gcctgaatgc actcctgcag cccaatatgt 1500
   gcggattaaa gaaaacttag cagtggggtc aaagatcaac ggctataagg catatgaccc 1560
   cgaaaataga aatggcaatg gtttaaggta caaaaaattg catgatccta aaggttggat 1620
   caccattgat gaaatttcag ggtcaatcat aacttccaaa atcctggata gggaggttga 1680
   aactcccaaa aatgagttgt ataatattac agtcctggca atagacaaag atgatagatc 1740
   atgtactgga acacttgctg tgaacattga agatgtaaat gataatccac cagaaatact 1800
   tcaagaatat gtagtcattt gcaaaccaaa aatggggtat accgacattt tagctgttga 1860
   tcctgatgaa cctgtccatg gagctccatt ttatttcagt ttgcccaata cttctccaga 1920
   aatcagtaga ctgtggagcc tcaccaaagt taatgataca gctgcccgtc tttcatatca 1980
   gaaaaatgct ggatttcaag aatataccat tcctattact gtaaaagaca gggccggcca 2040
   agctgcaaca aaattattga gagttaatct gtgtgaatgt actcatccaa ctcagtgtcg 2100
   tgcgacttca aggagtacag gagtaatact tggaaaatgg gcaatccttg caatattact 2160
   gggtatagca ctgctctttt ctgtattgct aactttagta tgtggagttt ttggtgcaac 2220
   taaagggaaa cgttttcctg aagatttagc acagcaaaac ttaattatat caaacacaga 2280
   agcacctgga gacgatagag tgtgctctgc caatggattt atgacccaaa ctaccaacaa 2340
   ctctagccaa ggtttttgtg gtactatggg atcaggaatg aaaaatggag ggcaggaaac 2400
   cattgaaatg atgaaaggag gaaaccagac cttggaatcc tgccgggggg ctgggcatca 2460
   tcataccctg gactcctgca ggggaggaca cacggaggtg gacaactgca gatacactta 2520
   ctcggagtgg cacagtttta ctcaaccccg tctcggtgaa aaattgcatc gatgtaatca 2580
   gaatgaagac cgcatgccat cccaagatta tgtcctcact tataactatg agggaagagg 2640
   atctccagct ggttctgtgg gctgctgcag tgaaaagcag gaagaagatg gccttgactt 2700
   tttaaataat ttggaaccca aatttattac attagcagaa gcatgcacaa agagataatg 2760
   tcacagtgct acaattaggt ctttgtcaga cattctggag gtttccaaaa ataatattgt 2820
   aaagttcaat ttcaacatgt atgtatatga tgattttttt ctcaattttg aattatgcta 2880
   ctcaccaatt tatattttta aagcaagttg ttgcttatct tttccaaaaa gtgaaaaatg 2940
   ttaaaacaga caactggtaa atctcaaact ccagcactgg aattaaggtc tctaaagcat 3000
   ctgctctttt ttttttttac agatatttta gtaataaata tgctggataa atattagtcc 3060
   aacaatagct aagttatgct aatatcacat tattatgtat tcactttaag tgatagttta 3120
   aaaaataaac aagaaatatt gagtatcact atgtgaagaa agttttggaa aagaaacaat 3180
   gaagactgaa ttaaattaaa aatgttgcag ctcataaaga attggactca cccctactgc 3240
   actaccaaat tcatttgact ttggaggcaa aatgtgttga agtgccctat gaagtagcaa 3300
   ttttctatag gaatatagtt ggaaataaat gtgtgtgtgt atattattat taatcaatgc 3360
   aatatttaaa tgaaatgaga acaaagagga aaatggtaaa aacttgaaat gaggctgggg 3420
   tatagtttgt cctacaatag aaaaaagaga gagcttccta ggcctgggct cttaaatgct 3480
   gcattataac tgagtctatg aggaaatagt tcctgtccaa tttgtgtaat ttgtttaaaa 3540
   ttgtaaataa at 3552

<210> 127
<211> 754
<212> DNA
<213> Homo sapiens

<400> 127
tttttttttt ttgtcattgt tcattgattt taatgagaaa gctaagagag gaaataagta 60
gcctttcaaa ggtcacacag aagtaagtga cagatccagg attcatatcc aagcattctg 120
gctctagtgt ccatgcttct caaccattat gacccaatat tcaaccaaat caatactgaa 180
ggacacgtga aatgtatccg gtattttact attacaaaca aaaatccaat gaacattctt 240
gaagacatac acaaaaataa tggttacaat agaagttact ggaattgaaa ttttggttca 300
acctatatta aaatgtaagg cttttgatat agctaataga tttttgaaat gatcagtctt 360
aacgtttgta ggggagcaca ctcctgcatg gggaaaagat tcactgtgaa gcacagagca 420
cctttatggt tggatcatct tgtcattaaa gttcaggcgt tatctatcct gtaagtggca 480
gaatcaagac tgcaatatcg cctgcttttc tttttaactc atgttttccc ttgactacac 540
tggtcctcaa agtaaaaccc ctgtgtcagt gtactattca tggaatactc tgcaattata 600
accaccttct aatactttta atacccaatc aaaatttatt atacatatgt atcatagata 660
ctcatctgta aagctgtgct tcaaaatagt gatctcttcc caacattaca atatatatta 720
atgatgtcga acctgcccgg gcggccgctc gaag 754

<210> 128
<211> 374
<212> DNA
<213> Homo sapiens

<400> 128
aggttttgat taaaaaggca aatgatttta ttgttcgata atcttttaaa aaaataagag 60
gaaggagtaa aattaaagat gaaagatgat ttttatttcc ttgtgacctc tatatccccc 120
ttcccctgcc cttggtaagt aactcttgat ggagaaagga ttaaagactc ttatttaacc 180
aaaaaacaga gccagctaat catttccaaa ggttagtatc tccctgctga cctcttcttt 240
ggtttaattg aataaaacta tatgttcata tatgtattaa aacaactcag aataacatct 300
tttcttcctt agttaaggca ttataagggc tatactatca tccataataa ccaaggcaat 360
aacttaaaaa gctg 374

<210> 129
<211> 546
<212> DNA
<213> Homo sapiens

<400> 129

agtgtgatgg atatctgcag aattcggct aagcgtggtc gcggcccgag gtctggaact 60
tcccagcacy tgaaaaggag cctcctgagc tgactcggct aaagccccac tttcgctcct 120
cctcatttct gcctactgat ttccttggag cattcatctg aatattaccg tttgctgtgt 180
aacctggtac atacatagca tgactccctg gaatagagtg ggctggggtg cttatgctgg 240
gagagtgatt gacatgcact ttcaagctat atctaccatt tgcagcaaag gagaaaaaat 300
acctcgagta aattccatca tttttataa catcagcacc tgctccatca tcaaggagtc 360
tcagcgtaac aggatctcca gtctctggct caactgtggc agtgacagtg gcattaagaa 420
tgggataaaa tccctgtttc acattggcat aaatcatcac aggatgagga aaatggaggc 480
tgtctctttc cacaaaggct tccacagtgg ctgggggcac agacctgccc gggcggccgc 540
tcgaaa 546

<210> 130
<211> 5156
<212> DNA
<213> Homo sapiens

<400> 130
accaaccgag gcgccgggca gcgacccctg cagcggagac agagactgag cggcccggca 60
ccgccatgcc tgcgctctgg ctgggctgct gcctctgctt gtcgctcctc ctgcccgcag 120
cccgggccac ctccaggagg gaagtctgtg attgcaatgg gaagtccagg cagtgtatct 180
ttgatcggga acttcacaga caaactggta atggattccg ctgcctcaac tgcaatgaca 240
acactgatgg cattcactgc gagaagtgca agaatggctt ttaccggcac agagaaaggg 300
accgctgttt gccctgcaat tgtaactcca aaggttctct tagtgctcga tgtgacaact 360
ccggacggtg cagctgtaaa ccaggtgtga caggagccag atgcgaccga tgtctgccag 420
gcttccacat gctcacggat gcggggtgca cccaagacca gagactgcta gactccaagt 480
gtgactgtga cccagctggc atcgcagggc cctgtgacgc gggccgctgt gtctgcaagc 540
cagctgtcac tggagaacgc tgtgataggt gtcgatcagg ttactataat ctggatgggg 600
ggaaccctga gggctgtacc cagtgtttct gctatgggca ttcagccagc tgccgcagct 660
ctgcagaata cagtgtccat aagatcacct ctacctttca tcaagatgtt gatggctgga 720
aggctgtcca acgaaatggg tctcctgcaa agctccaatg gtcacagcgc catcaagatg 780
tgtttagctc agcccaacga ctagaccctg tctattttgt ggctcctgcc aaatttcttg 840
ggaatcaaca ggtgagctat ggtcaaagcc tgtcctttga ctaccgtgtg gacagaggag 900
gcagacaccc atctgcccat gatgtgattc tggaaggtgc tggtctacgg atcacagctc 960
ccttgatgcc acttggcaag acactgcctt gtgggctcac caagacttac acattcaggt 1020
taaatgagca tccaagcaat aattggagcc cccagctgag ttactttgag tatcgaaggt 1080
tactgcggaa tctcacagcc ctccgcatcc gagctacata tggagaatac agtactgggt 1140
acattgacaa tgtgaccctg atttcagccc gccctgtctc tggagcccca gcaccctggg 1200
ttgaacagtg tatatgtcct gttgggtaca aggggcaatt ctgccaggat tgtgcttctg 1260
gctacaagag agattcagcg agactggggc cttttggcac ctgtattcct tgtaactgtc 1320
aagggggagg ggcctgtgat ccagacacag gagattgtta ttcaggggat gagaatcctg 1380
acattgagtg tgctgactgc ccaattggtt tctacaacga tccgcacgac ccccgcagct 1440
gcaagccatg tccctgtcat aacgggttca gctgctcagt gatgccggag acggaggagg 1500
tggtgtgcaa taactgccct cccggggtca ccggtgcccg ctgtgagctc tgtgctgatg 1560
gctactttgg ggaccccttt ggtgaacatg gcccagtgag gccttgtcag ccctgtcaat 1620
gcaacaacaa tgtggacccc agtgcctctg ggaattgtga ccggctgaca ggcaggtgtt 1680
tgaagtgtat ccacaacaca gccggcatct actgcgacca gtgcaaagca ggctacttcg 1740
gggacccatt ggctcccaac ccagcagaca agtgtcgagc ttgcaactgt aaccccatgg 1800
gctcagagcc tgtaggatgt cgaagtgatg gcacctgtgt ttgcaagcca ggatttggtg 1860
gccccaactg tgagcatgga gcattcagct gtccagcttg ctataatcaa gtgaagattc 1920
agatggatca gtttatgcag cagcttcaga gaatggaggc cctgatttca aaggctcagg 1980
gtggtgatgg agtagtacct gatacagagc tggaaggcag gatgcagcag gctgagcagg 2040
cccttcagga cattctgaga gatgcccaga tttcagaagg tgctagcaga tcccttggtc 2100
tccagttggc caaggtgagg agccaagaga acagctacca gagccgcctg gatgacctca 2160
agatgactgt ggaaagagtt cgggctctgg gaagtcagta ccagaaccga gttcgggata 2220
ctcacaggct catcactcag atgcagctga gcctggcaga aagtgaagct tccttgggaa 2280
acactaacat tcctgcctca gaccactacg tggggccaaa tggctttaaa agtctggctc 2340
aggaggccac aagattagca gaaagccacg ttgagtcagc cagtaacatg gagcaactga 2400
caaggaaac tgaggactat tccaaacaag ccctctcact ggtgcgcaag gccctgcatg 2460

aaggagtcgg aagcggaagc ggtagcccgg acggtgctgt ggtgcaaggg cttgtggaaa 2520
aattggagaa aaccaagtcc ctggcccagc agttgacaag ggaggccact caagcggaaa 2580
ttgaagcaga taggtcttat cagcacagtc tccgcctcct ggattcagtg tctcggcttc 2640
agggagtcag tgatcagtcc tttcaggtgg aagaagcaaa gaggatcaaa caaaaagcgg 2700
attcactctc aagcctggta accaggcata tggatgagtt caagcgtaca cagaagaatc 2760
tgggaaactg gaaagaagaa gcacagcagc tcttacagaa tggaaaaagt gggagagaga 2820
aatcagatca gctgctttcc cgtgccaatc ttgctaaaag cagagcacaa gaagcactga 2880
gtatgggcaa tgccactttt tatgaagttg agagcatcct taaaaacctc agagagtttg 2940
acctgcaggt ggacaacaga aaagcagaag ctgaagaagc catgaagaga ctctcctaca 3000
tcagccagaa ggtttcagat gccagtgaca agacccagca agcagaaaga gccctgggga 3060
gcgctgctgc tgatgcacag agggcaaaga atggggccgg ggaggccctg gaaatctcca 3120
gtgagattga acaggagatt gggagtctga acttggaagc caatgtgaca gcagatggag 3180
ccttggccat ggaaaaggga ctggcctctc tgaagagtga gatgagggaa gtggaaggag 3240
agctggaaag gaaggagctg gagtttgaca cgaatatgga tgcagtacag atggtgatta 3300
cagaagccca gaaggttgat accagagcca agaacgctgg ggttacaatc caagacacac 3360
tcaacacatt agacggcctc ctgcatctga tggaccagcc tctcagtgta gatgaagagg 3420
ggctggtctt actggagcag aagctttccc gagccaagac ccagatcaac agccaactgc 3480
ggcccatgat gtcagagctg gaagagaggg cacgtcagca gaggggccac ctccatttgc 3540
tggagacaag catagatggg attctggctg atgtgaagaa cttggagaac attagggaca 3600
acctgccccc aggctgctac aatacccagg ctcttgagca acagtgaagc tgccataaat 3660
atttctcaac tgaggttctt gggatacaga tctcagggct cgggagccat gtcatgtgag 3720
tgggtgggat ggggacattt gaacatgttt aatgggtatg ctcaggtcaa ctgacctgac 3780
cccattcctg atcccatggc caggtggttg tcttattgca ccatactcct tgcttcctga 3840
tgctgggcaa tgaggcagat agcactgggt gtgagaatga tcaaggatct ggaccccaaa 3900
gaatagactg gatggaaaga caaactgcac aggcagatgt ttgcctcata atagtcgtaa 3960
gtggagtcct ggaatttgga caagtgctgt tgggatatag tcaacttatt ctttgagtaa 4020
tgtgactaaa ggaaaaaact ttgactttgc ccaggcatga aattcttcct aatgtcagaa 4080
cagagtgcaa cccagtcaca ctgtggccag taaaatacta ttgcctcata ttgtcctctg 4140
caagcttctt gctgatcaga gttcctccta cttacaaccc agggtgtgaa catgttctcc 4200
attttcaagc tggaagaagt gagcagtgtt ggagtgagga cctgtaaggc aggcccattc 4260
agagctatgg tgcttgctgg tgcctgccac cttcaagttc tggacctggg catgacatcc 4320
tttcttttaa tgatgccatg gcaacttaga gattgcattt ttattaaagc atttcctacc 4380
agcaaagcaa atgttgggaa agtatttact ttttcggttt caaagtgata gaaaagtgtg 4440
gcttgggcat tgaaagaggt aaaattctct agatttatta gtcctaattc aatcctactt 4500
ttagaacacc aaaaatgatg cgcatcaatg tattttatct tattttctca atctcctctc 4560
tctttcctcc acccataata agagaatgtt cctactcaca cttcagctgg gtcacatcca 4620
tccctccatt catccttcca tccatctttc catccattac ctccatccat ccttccaaca 4680
tatatttatt gagtacctac tgtgtgccag gggctggtgg gacagtggtg acatagtctc 4740
tgccctcata gagttgattg tctagtgagg aagacaagca tttttaaaaa ataaatttaa 4800
acttacaaac tttgtttgtc acaagtggtg tttattgcaa taaccgcttg gtttgcaacc 4860
tctttgctca acagaacata tgttgcaaga ccctcccatg ggggcacttg agttttggca 4920
aggctgacag agctctgggt tgtgcacatt tctttgcatt ccagctgtca ctctgtgcct 4980
ttctacaact gattgcaaca gactgttgag ttatgataac accagtggga attgctggag 5040
gaaccagagg cacttccacc ttggctggga agactatggt gctgccttgc ttctgtattt 5100
ccttggattt tcctgaaagt gtttttaaat aaagaacaat tgttagaaaa aaaaaa 5156

<210> 131
<211> 671
<212> DNA
<213> Homo sapiens

<400> 131
aggtctggag ggcccacagc cggatgtggg acaccgggaa aaagtggtca tagcacacat 60
ttttgcatcc cggttgcagt gtgttgcaga cgaagtcctc ttgctcgtca ccccacactt 120
cctgggcagc caycacgagg atcatgactc ggaaaataaa gatgactgtg atccacacct 180
tcccgatgct ggtggagtgt ttgttgacac ccccgatgaa agtgtgcagc gtcccccaat 240

ccattgcgct ggtttatccc tgagtcctgt ttccaacgac tgccagtgtt tcagacccaa 300
agaatgaggg caagatccct ctgcgagggt ttcagacctc cttctcctac cccactggag 360
tgcctagaag ccaatgggtg cacagtgatg atacgaatgt caatctttgc tcggtcagtg 420
aggatgtcgc ctggaatatt caaattgaat tacagatgca tgaagagggc gtacaagtta 480
gaatttttct ttcgccatac agaaattgtt tagccagatc ttctgtactt cttttccttc 540
cctgaccctt cctgctcccc aggaagggag gtcagccccg tttgcaaaac acaggatgcc 600
cgtgacaccg gagacaggtc ttcttcaccg acaggaagtg ccttctggtg cctgcacgtt 660
ttaactgcta t 671

<210> 132
<211> 590
<212> DNA
<213> Homo sapiens

<400> 132
ctgaatggaa aagcttatgg ctctgtgatg atattagtga ccagcggaga tgataagctt 60
cttggcaatt gcttacccac tgtgctcagc agtggttcaa caattcactc cattgccctg 120
ggttcatctg cagccccaaa tctggaggaa ttatcacgtc ttacaggagg tttaaagttc 180
tttgttccag atatatcaaa ctccaatagc atgattgatg ctttcagtag aatttcctct 240
ggaactggag acattttcca gcaacatatt cagcttgaaa gtacaggtga aaatgtcaaa 300
cctcaccatc aattgaaaaa cacagtgact gtggataata ctgtgggcaa cgacactatg 360
tttctagtta cgtggcaggc cagtggtcct cctgagatta tattatttga tcctgatgga 420
cgaaaatact acacaaataa ttttatcacc aatctaactt ttcggacagc tagtctttgg 480
attccaggaa cagctaagcc tgggcactgg acttacaccc tgaacaatac ccatcattct 540
ctgcaagccc tgaaagtgac agtgacctct cgcgcctcca actcagacct 590

<210> 133
<211> 581
<212> DNA
<213> Homo sapiens

<400> 133
   aggtcctgtc cgggggcact gagaactccc tctggaattc ttggggggtg ttggggagag 60
   actgtgggcc tggagataaa acttgtctcc tctaccacca ccctgtaccc tagcctgcac 120
   ctgtcctcat ctctgcaaag ttcagcttcc ttccccaggt ctctgtgcac tctgtcttgg 180
   atgctctggg gagctcatgg gtggaggagt ctccaccaga gggaggctca ggggactggt 240
   tgggccaggg atgaatattt gagggataaa aattgtgtaa gagccaaaga attggtagta 300
   gggggagaac agagaggagc tgggctatgg gaaatgattt gaataatgga gctgggaata 360
   tggctggata tctggtacta aaaaagggtc tttaagaacc tacttcctaa tctcttcccc 420
   aatccaaacc atagctgtct gtccagtgct ctcttcctgc ctccagctct gccccaggct 480
   cctcctagac tctgtccctg ggctagggca ggggaggagg gagagcaggg ttgggggaga 540
   ggctgaggag agtgtgacat gtggggagag gaccagacct c 581

<210> 134
<211> 4797
<212> DNA
<213> Homo sapiens

<220>
<221> misc_feature
<222> 135, 501, 4421, 4467, 4468, 4698
<223> n = A,T,C or G

<400> 134
   cctgggacca aagtgctgcc cagagctgag ggtcctggag ccacatgaga aggcttctcc 60
   ctgtgtacct gtgcagcaca gggtagggtg agtccactca gctgtctagg agaggaccca 120
   ggagcagcag agacncgcca agcctttact cataccatat tctgatcctt ttccagcaaa 180
   ttgtggctac taatttgccc cctgaagatc aagatggctc tggggatgac tctgacaact 240
   tctccggctc aggtgcaggt gaggttgtca tgggggcccc ccccacccaa gacggcaaca 300
   ggtcatgcct gggggcagtg gtcaggcagt ctcctgtgtt tactgagcat gtactgagtg 360
   caccctgcct gccctgtctc cacccagctg gctccaaagg gcaatgctga ggagaggaat 420
   ggggtcgtga gctgctgtta aggagagctc atgcttggag gtgaggtgaa ggctgtgagc 480
   tccagaaggc cccagggcgc nctgctgcac gcaggctcat attcactagg aatagcttta 540
   ctcactaaga aacctctgga acccccttca gaaggttatt tgactcctga gcctctattt 600
   tctcatctgc aaaatgggaa taataccttg acctgataag cttgtggagc tgtaaggcag 660
   cacagagcca gctggggtgt agctcttcca tccaagctcc cttccttact tcccctttcc 720
   tgtggggact gggggagaga agtccctgag ctggaggtgg tcagggaagc ttcacagagg 780
   aggtggctct tgagtggacc tcaggaagag gggtgagaga gctaaggaag gaggctgagg 840
   tcatccctgg ggaagtgacc tagcggaggc ctgagagctg caaggtagga tatctgttgt 900
   tggaagtgtc tgttgttgga agtgggggcc tttttttcag ggagggtggg gccagagaag 960
   tgtgtgccct gggataagta ggataaccac agtagttatg cccctaaggg atgcccaccc 1020
   cacccctgtg gtcacagaaa agctttccca ggtggcctag gcacctgtct cgtggctcca 1080
   gagacaggct gcacctgaca cacacaatgg aaggacagct ctccttgtcc attttccaag 1140
   gagcttagcc tcagctgcct tgtccaggta ctagcctccc tcatagcctg agcttggcca 1200
   gcccaggtgc tctggagcct cccccgaccc acccaacaca ctctgcttct ggtcctcccc 1260
   accccccacc tccccaacac actctgcttc tggtcctgca ggtgctttgc aagatatcac 1320
   cttgtcacag cagaccccct ccacttggaa ggacacgcag ctcctgacgg ctattcccac 1380
   gtctccagaa cccaccggcc tggaggctac agctgcctcc acctccaccc tgccggctgg 1440
   agaggggccc aaggagggag aggctgtagt cctgccagaa gtggagcctg gcctcaccgc 1500
   ccgggagcag gaggccaccc cccgacccag ggagaccaca cagctcccga ccactcatca 1560
   ggcctcaacg accacagcca ccacggccca ggagcccgcc acctcccacc cccacaggga 1620
   catgcagcct ggccaccatg agacctcaac ccctgcagga cccagccaag ctgaccttca 1680
   cactccccac acagaggatg gaggtccttc tgccaccgag agggctgctg aggatggagc 1740
   ctccagtcag ctcccagcag cagagggctc tggggagcag gtgagtggcc tctgcattcc 1800
   ttgggaaatt gagtgggttg gtcctaatgc ctggcacttg gcaggcccta cacctgtgcc 1860
   ctgcgcgatc tcgtattcct caccaggaag acagggcaca ggggccgcct tcccctaccc 1920
   ccagggcctc gcagagcagg acagactaac tatgagatca gagcagaagc acccttaaag 1980
   atcacccaag agagggctcc caaactcaca atccaaactt gcagccctcg tcgaagagtg 2040
   aacgttatac cagtcatttt atttatagct tcgtggattt acgcttacac taaatagtct 2100
   gctattcata caaaatgtgt gctttgtatc actttttgtg atatccatgc catggtccag 2160
   ccagggtccg gagttgatgt ggcaagaagg cctggctttc gggccctgtg cgatcctggt 2220
   ttgggtgcat ctgagtgggt ggtggcaaag atcagggagg caggagctgc ttctgggtct 2280
   gtagtggagc tggttgctgc tgctggcggt gacctggcca acccaatctg cccctgccct 2340
   cccacaggac ttcacctttg aaacctcggg ggagaatacg gctgtagtgg ccgtggagcc 2400
   tgaccgccgg aaccagtccc cagtggatca gggggccacg ggggcctcac agggcctcct 2460
   ggacaggaaa gaggtgctgg gaggtgagtt ttctttcagg ggggtagttt ggggtgaatt 2520
   gctgctgtgg ggtcagggtg gggctgacca cagccaaggc cactgctttg ggagggtctg 2580
   cacgagagcc caaggagccg ctgagctgag ctggccccgt ctacctgccc taggggtcat 2640
   tgccggaggc ctcgtggggc tcatctttgc tgtgtgcctg gtgggtttca tgctgtaccg 2700
   catgaagaag aaggacgaag gcagctactc cttggaggag ccgaaacaag ccaacggcgg 2760
   ggcctaccag aagcccacca aacaggagga attctatgcc tgacgcggga gccatgcgcc 2820
   ccctccgccc tgccactcac taggccccca cttgcctctt ccttgaagaa ctgcaggccc 2880
   tggcctcccc tgccaccagg ccacctcccc agcattccag cccctctggt cgctcctgcc 2940
   cacggagtcg tgggtgtgct gggagctcca ctctgcttct ctgacttctg cctggagact 3000
   tagggcacca ggggtttctc gcataggacc tttccaccac agccagcacc tggcatcgca 3060
   ccattctgac tcggtttctc caaactgaag cagcctctcc ccaggtccag ctctggaggg 3120
   gagggggatc cgactgcttt ggacctaaat ggcctcatgt ggctggaaga tcctgcgggt 3180
   ggggcttggg gctcacacac ctgtagcact tactggtagg accaagcatc ttgggggggt 3240
   ggccgctgag tggcagggga caggagtcac tttgtttcgt ggggaggtct aatctagata 3300
   tcgacttgtt tttgcacatg tttcctctag ttctttgttc atagcccagt agaccttgtt 3360
   acttctgagg taagttaagt aagttgattc ggtatccccc catcttgctt ccctaatcta 3420
   tggtcgggag acagcatcag ggttaagaag actttttttt ttttttttaa actaggagaa 3480
   ccaaatctgg aagccaaaat gtaggcttag tttgtgtgtt gtctcttgag tttgtcgctc 3540
   atgtgtgcaa cagggtatgg actatctgtc tggtggcccc gttctggtgg tctgttggca 3600
   ggctggccag tccaggctgc cgtggggccg ccgcctcttt caagcagtcg tgcctgtgtc 3660
   catgcgctca gggccatgct gaggcctggg ccgctgccac gttggagaag cccgtgtgag 3720
   aagtgaatgc tgggactcag ccttcagaca gagaggactg tagggagggc ggcaggggcc 3780
   tggagatcct cctgcaggct cacgcccgtc ctcctgtggc gccgtctcca ggggctgctt 3840
   cctcctggaa attgacgagg ggtgtcttgg gcagagctgg ctctgagcgc ctccatccaa 3900
   ggccaggttc tccgttagct cctgtggccc caccctgggc cctgggctgg aatcaggaat 3960
   attttccaaa gagtgatagt cttttgcttt tggcaaaact ctacttaatc caatgggttt 4020
   ttccctgtac agtagatttt ccaaatgtaa taaactttaa tataaagtag tctgtgaatg 4080
   ccactgcctt cgcttcttgc ctctgtgctg tgtgtgacgt gaccggactt ttctgcaaac 4140
   accaacatgt tgggaaactt ggctcgaatc tctgtgcctt cgtctttccc atggggaggg 4200
   attctggttc cagggtccct ctgtgtattt gcttttttgt tttggctgaa attctcctgg 4260
   aggtcggtag gttcagccaa ggttttataa ggctgatgtc aatttctgtg ttgccaagct 4320
   ccaagcccat cttctaaatg gcaaaggaag gtggatggcc ccagcacagc ttgacctgag 4380
   gctgtggtca cagcggaggt gtggagccga ggcctacccc ncagacacct tggacatcct 4440
   cctcccaccc ggctgcagag gccaganncc agcccagggt cctgcactta cttgcttatt 4500
   tgacaacgtt tcagcgactc cgttggccac tccgagagtg ggccagtctg tggatcagag 4560
   atgcaccacc aagccaaggg aacctgtgtc cggtattcga tactgcgact ttctgcctgg 4620
   agtgtatgac tgcacatgac tcgggggtgg ggaaaggggt cggctgacca tgctcatctg 4680
   ctggtccgtg ggacggtncc caagccagag gtgggttcat ttgtgtaacg acaataaacg 4740
   gtacttgtca tttcgggcaa cggctgctgt ggtggtggtt gagtctcttc ttggcct 4797

<210> 135
<211> 2856
<212> DNA
<213> Homo sapiens

<400> 135
   tagtcgcggg tccccgagtg agcacgccag ggagcaggag accaaacgac gggggtcgga 60
   gtcagagtcg cagtgggagt ccccggaccg gagcacgagc ctgagcggga gagcgccgct 120
   cgcacgcccg tcgccacccg cgtacccggc gcagccagag ccaccagcgc agcgctgcca 180
   tggagcccag cagcaagaag ctgacgggtc gcctcatgct ggctgtggga ggagcagtgc 240
   ttggctccct gcagtttggc tacaacactg gagtcatcaa tgccccccag aaggtgatcg 300
   aggagttcta caaccagaca tgggtccacc gctatgggga gagcatcctg cccaccacgc 360
   tcaccacgct ctggtccctc tcagtggcca tcttttctgt tgggggcatg attggctcct 420
   tctctgtggg ccttttcgtt aaccgctttg gccggcggaa ttcaatgctg atgatgaacc 480
   tgctggcctt cgtgtccgcc gtgctcatgg gcttctcgaa actgggcaag tcctttgaga 540
   tgctgatcct gggccgcttc atcatcggtg tgtactgcgg cctgaccaca ggcttcgtgc 600
   ccatgtatgt gggtgaagtg tcacccacag cctttcgtgg ggccctgggc accctgcacc 660
   agctgggcat cgtcgtcggc atcctcatcg cccaggtgtt cggcctggac tccatcatgg 720
   gcaacaagga cctgtggccc ctgctgctga gcatcatctt catcccggcc ctgctgcagt 780
   gcatcgtgct gcccttctgc cccgagagtc cccgcttcct gctcatcaac cgcaacgagg 840
   agaaccgggc caagagtgtg ctaaagaagc tgcgcgggac agctgacgtg acccatgacc 900
   tgcaggagat gaaggaagag agtcggcaga tgatgcggga gaagaaggtc accatcctgg 960
   agctgttccg ctcccccgcc taccgccagc ccatcctcat cgctgtggtg ctgcagctgt 1020
   cccagcagct gtctggcatc aacgctgtct tctattactc cacgagcatc ttcgagaagg 1080
   cgggggtgca gcagcctgtg tatgccacca ttggctccgg tatcgtcaac acggccttca 1140
   ctgtcgtgtc gctgtttgtg gtggagcgag caggccggcg gaccctgcac ctcataggcc 1200
   tcgctggcat ggcgggttgt gccatactca tgaccatcgc gctagcactg ctggagcagc 1260
   taccctggat gtcctatctg agcatcgtgg ccatctttgg ctttgtggcc ttctttgaag 1320
   tgggtcctgg ccccatccca tggttcatcg tggctgaact cttcagccag ggtccacgtc 1380
   cagctgccat tgccgttgca ggcttctcca actggacctc aaatttcatt gtgggcatgt 1440
   gcttccagta tgtggagcaa ctgtgtggtc cctacgtctt catcatcttc actgtgctcc 1500
   tggttctgtt cttcatcttc acctacttca aagttcctga gactaaaggc cggaccttcg 1560
   atgagatcgc ttccggcttc cggcaggggg gagccagcca aagtgataag acacccgagg 1620
   agctgttcca tcccctgggg gctgattccc aagtgtgagt cgccccagat caccagcccg 1680
   gcctgctccc agcagcccta aggatctctc aggagcacag gcagctggat gagacttcca 1740
   aacctgacag atgtcagccg agccgggcct ggggctcctt tctccagcca gcaatgatgt 1800
   ccagaagaat attcaggact taacggctcc aggattttaa caaaagcaag actgttgctc 1860
   aaatctattc agacaagcaa caggttttat aattttttta ttactgattt tgttattttt 1920
   atatcagcct gagtctcctg tgcccacatc ccaggcttca ccctgaatgg ttccatgcct 1980
   gagggtggag actaagccct gtcgagacac ttgccttctt cacccagcta atctgtaggg 2040
   ctggacctat gtcctaagga cacactaatc gaactatgaa ctacaaagct tctatcccag 2100
   gaggtggcta tggccacccg ttctgctggc ctggatctcc ccactctagg ggtcaggctc 2160
   cattaggatt tgccccttcc catctcttcc tacccaacca ctcaaattaa tctttcttta 2220
   cctgagacca gttgggagca ctggagtgca gggaggagag gggaagggcc agtctgggct 2280
   gccgggttct agtctccttt gcactgaggg ccacactatt accatgagaa gagggcctgt 2340
   gggagcctgc aaactcactg ctcaagaaga catggagact cctgccctgt tgtgtataga 2400
   tgcaagatat ttatatatat ttttggttgt caatattaaa tacagacact aagttatagt 2460
   atatctggac aagccaactt gtaaatacac cacctcactc ctgttactta cctaaacaga 2520
   tataaatggc tggtttttag aaacatggtt ttgaaatgct tgtggattga gggtaggagg 2580
   tttggatggg agtgagacag aagtaagtgg ggttgcaacc actgcaacgg cttagacttc 2640
   gactcaggat ccagtccctt acacgtacct ctcatcagtg tcctcttgct caaaaatctg 2700
   tttgatccct gttacccaga gaatatatac attctttatc ttgacattca aggcatttct 2760
   atcacatatt tgatagttgg tgttcaaaaa aacactagtt ttgtgccagc cgtgatgctc 2820
   aggcttgaaa tcgcattatt ttgaatgtga agggaa 2856

<210> 136
<211> 356
<212> DNA
<213> Homo sapiens

<400> 136
ggtggagcca aatgaagaaa atgaagatga aagagacaga cacctcagtt tttctggatc 60
aggcattgat gatgatgaag attttatctc cagcaccatt tcaaccacac cacgggcttt 120
tgaccacaca aaacagaacc aggactggac tcagtggaac ccaagccatt caaatccgga 180
agtgctactt cagacaacca caaggatgac tgatgtagac agaaatggca ccactgctta 240
tgaaggaaac tggaacccag aagcacaccc tcccctcatt caccatgagc atcatgagga 300
agaagagacc ccacattcta caagcacaat ccaggcaact cctagtagta caacgg 356

<210> 137
<211> 356
<212> DNA
<213> Homo sapiens

<220>
<221> misc_feature
<222> 254, 264, 279, 281, 290, 328, 342
<223> n = A,T,C or G

<400> 137
gcaggtggag aagacatttt attgttcctg gggtctctgg aggcccattg gtggggctgg 60
gtcactggct gcccccggaa cagggcgctg ctccatggct ctgcttgtgg tagtctgtgg 120
ctatgtctcc cagcaaggac agaaactcag aaaaatcaat cttcttatcc tcattcttgt 180
cctttttctc aaagacatcg gcgaggtaat ttgtgccctt tttacctcgg cccgcgacca 240
cgctaaggcc aaanttccag acanayggcc gggccggtnc nataggggan cccaacttgg 300
ggacccaaac tctggcgcgg aaacacangg gcataagctt gnttcctgtg gggaaa 356

<210> 138
<211> 353
<212> DNA
<213> Homo sapiens

<400> 138
aggtccagtc ctccacttgg cctgatgaga gtggggagtg gcaagggacg tttctcctgc 60
aatagacact tagatttctc tcttgtggga agaaaccacc tgtccatcca ctgactcttc 120
tacattgatg tggaaattgc tgctgctacc accacctcct gaagaggctt ccctgatgcc 180
aatgccagcc atcttggcat cctggccctc gagcaggctg cggtaagtag cgatctcctg 240
ctccagccgt gtctttatgt caagcagcat cttgtactcc tggttctgag cctccatctc 300

gcatcggagc tcactcagac ctcgsccgsg mssmcgctam gccgaattcc agc 353

<210> 139
<211> 371
<212> DNA
<213> Homo sapiens

<400> 139
agcgtggtcg cggccgaggt ccatccgaag caagattgca gatggcagtg tgaagagaga 60
agacatattc tacacttcaa agctttggtg caattcccat cgaccagagt tggtccgacc 120
agccttggaa aggtcactga aaaatcttca attggattat gttgacctct accttattca 180
ttttccagtg tctgtaaagc caggtgagga agtgatccca aaagatgaaa atggaaaaat 240
actatttgac acagtggatc tctgtgccac gtgggaggcc gtggagaagt gtaaagatgc 300
aggattggac ctgcccgggc ggccgctcga aagccgaatt ccagcacact ggcggccgtt 360
actagtggat c 371

<210> 140
<211> 370
<212> DNA
<213> Homo sapiens

<400> 140
tagcgtggtc gcggccgagg tccatctccc tttgggaact agggggctgc tggtgggaaa 60
tgggagccag ggcagatgtt gcattccttt gtgtccctgt aaatgtggga ctacaagaag 120
aggagctgcc tgagtggtac tttctcttcc tggtaatcct ctggcccagc ctcatggcag 180
aatagaggta tttttaggct atttttgtaa tatggcttct ggtcaaaatc cctgtgtagc 240
tgaattccca agccctgcat tgtacagccc cccactcccc tcaccaccta ataaaggaat 300
agttaacact caaaaaaaaa aaaaaacctg cccgggcggc cgctcgaaag ccgaattcca 360
gcacactggc 370

<210> 141
<211> 371
<212> DNA
<213> Homo sapiens

<400> 141
tagcgtggtc gcggccgagg tcctctgtgc tgcctgtcac agcccgatgg taccagcgca 60
gggtgtaggc agtgcaggag ccctcatcca gtggcaggga acaggggtca tcactatccc 120
aaggagcttc agggtcctgg tactcctcca cagaatactc ggagtattca gagtactcat 180
catcctcagg gggtacccgc tcttcctcct ctgcatgaga gacgcggagc acaggcacag 240
catggagctg ggagccggca gtgtctgcag cataactagg gaggggtcgt gatccagatg 300
cgatgaactg gccctggcag gcacagtgct gactcatctc ttggcgacct gcccgggcgg 360
ccgctcgaag c 371

<210> 142
<211> 343
<212> DNA
<213> Homo sapiens

<400> 142
gcgttttgag gccaatggtg taaaaggaaa tatcttcaca taaaaactag atggaagcat 60
tgtcagaaac ctctttgtga tgtttgcttt caactcacag agttgaacat tccttttcat 120
agagcagttt tgaaacactc ttttgtagaa tttgcaagcg gatgattgga tcgctatgag 180
gtcttcattg gaaacgggat acctttacat aaaaactaga cagtagcatt ctcagaaatt 240
tctttgggat gtgggcattc aacccacaga ggagaacttc atttgataga gcagttttga 300
aacacccttt ttgtagaatc tacaggtgga catttagagt gct 343

<210> 143

<211> 354
<212> DNA
<213> Homo sapiens

<400> 143
aggtctgatg gcagaaaaac tcagactgtc tgcaacttta cagatggtgc attggttcag 60
catcaggagt gggatgggaa ggaaagcaca ataacaagaa aattgaaaga tgggaaatta 120
gtggtggagt gtgtcatgaa caatgtcacc tgtactcgga tctatgaaaa agtagaataa 180
aaattccatc atcactttgg acaggagtta attaagagaa tgaccaagct cagttcaatg 240
agcaaatctc catactgttt ctttcttttt tttttcatta ctgtgttcaa ttatctttat 300
cataaacatt ttacatgcag ctatttcaaa gtgtgttgga ttaattagga tcat 354

<210> 144
<211> 353
<212> DNA
<213> Homo sapiens

<400> 144
ggtcaaggac ctgggggacc cccaggtcca gcagccacat gattctgcag cagacaggga 60
cctagagcac atctggatct cagccccacc cctggcaacc tgcctgccta gagaactccc 120
aagatgacag actaagtagg attctgccat ttagaataat tctggtatcc tgggcgttgc 180
gttaagttgc ttaactttca ttctgtctta cgatagtctt cagaggtggg aacagatgaa 240
gaaaccatgc cccagagaag gttaagtgac ttcctcttta tggagccagt gttccaacct 300
aggtttgcct gataccagac ctgtggcccc acctcccatg caggtctctg tgg 353

<210> 145
<211> 371
<212> DNA
<213> Homo sapiens

<400> 145
caggtctgtc ataaactggt ctggagtttc tgacgactcc ttgttcacca aatgcaccat 60
ttcctgagac ttgctggcct ctccgttgag tccacttggc tttctgtcct ccacagctcc 120
attgccactg ttgatcacta gctttttctt ctgcccacac cttcttcgac tgttgactgc 180
aatgcaaact gcaagaatca aagccaaggc caagagggat gccaagatga tcagccattc 240
tggaatttgg ggtgtcctta taggaccaga ggttgtgttt gctccacctt cttgactccc 300
atgtgagacc tcggccgcga ccacgctaag ccgaattcca gcacactggc ggcccgttac 360
tagtggatcc g 371

<210> 146
<211> 355
<212> DNA
<213> Homo sapiens

<400> 146
ggtcctccgt cctcttccca gaggtgtcgg ggcttggccc cagcctccat cttcgtctct 60
caggatggcg agtagcagcg gctccaaggc tgaattcatt gtcggaggga aatataaact 120
ggtacggaag atcgggtctg gctccttcgg ggacatctat ttggcgatca acatcaccaa 180
cggcgaggaa gtggcagtga agctagaatc tcagaaggcc aggcatcccc agttgctgta 240
cgagagcaag ctctataaga ttcttcaagg tggggttggc atcccccaca tacggtggta 300
tggtcaggaa aaagactaca atgtactagt catggatctt ctgggaccta gcctc 355

<210> 147
<211> 355
<212> DNA
<213> Homo sapiens

<400> 147

ggtctgttac aaaatgaaga cagacaacac aacatttact ctgtggagat atcctactca 60
tactatgcac gtgctgtgat tttgaacata actcgtccca aaaacttgtc acgatcatcc 120
tgacttttta ggttggctga tccatcaatc ttgcactcaa ctgttacttc tttcccagtg 180
ttgttaggag caaagctgac ctgaacagca accaatggct gtagataccc aacatgcagt 240
tttttcccat aatatgggaa atattttaag tctatcattc cattatgagg ataaactgct 300
acatttggta tatcttcatt ctttgaaaca caatctatcc ttggcactcc ttcag 355

<210> 148
<211> 369
<212> DNA
<213> Homo sapiens

<400> 148
aggtctctct ccccctctcc ctctcctgcc agccaagtga agacatgctt acttcccctt 60
caccttcctt catgatgtgg gaagagtgct gcaacccagc cctagccaac accgcatgag 120
agggagtgtg ccgagggctt ctgagaaggt ttctctcaca tctagaaaga agcgcttaag 180
atgtggcagc ccctcttctt caagtggctc ttgtcctgtt gccctgggag ttctcaaatt 240
gctgcagcag cctccatcca gcctgaggat gacatcaata cacagaggaa gaagagtcag 300
gaaaagatga gagaagttac agactctcct gggcgacccc gagagcttac cattcctcag 360
acttcttca 369

<210> 149
<211> 620
<212> DNA
<213> Homo sapiens

<220>
<221> misc_feature
<222> 169, 171, 222, 472, 528, 559, 599
<223> n = A,T,C or G

<400> 149
actagtcaaa aatgctaaaa taatttggga gaaaatattt tttaagtagt gttatagttt 60
catgtttatc ttttattatg ttttgtgaag ttgtgtcttt tcactaatta cctatactat 120
gccaatattt ccttatatct atccataaca tttatactac atttgtaana naatatgcac 180
gtgaaactta acactttata aggtaaaaat gaggtttcca anatttaata atctgatcaa 240
gttcttgtta tttccaaata gaatggactt ggtctgttaa gggctaagga gaagaggaag 300
ataaggttaa aagttgttaa tgaccaaaca ttctaaaaga aatgcaaaaa aaaagtttat 360
tttcaagcct tcgaactatt taaggaaagc aaaatcattt cctaaatgca tatcatttgt 420
gagaatttct cattaatatc ctgaatcatt catttcacta aggctcatgt tnactccgat 480
atgtctctaa gaaagtacta tttcatggtc caaacctggt tgccatantt gggtaaaggc 540
tttcccttaa gtgtgaaant atttaaaatg aaattttcct ctttttaaaa attctttana 600
agggttaagg gtgttgggga 620

<210> 150
<211> 371
<212> DNA
<213> Homo sapiens

<400> 150
ggtccgatca aaacctgcta cctccccaag actttactag tgccgataaa ctttctcaaa 60
gagcaaccag tatcacttcc ctgtttataa aacctctaac catctctttg ttctttgaac 120
atgctgaaaa ccacctggtc tgcatgtatg cccgaatttg yaattctttt ctctcaaatg 180
aaaatttaat tttagggatt catttctata ttttcacata tgtagtatta ttatttcctt 240
atatgtgtaa ggtgaaattt atggtatttg agtgtgcaag aaaatatatt tttaaagctt 300
tcatttttcc cccagtgaat gatttagaat tttttatgta aatatacaga atgttttttc 360
ttacttttat a 371

<210> 151
<211> 4655
<212> DNA
<213> Homo sapiens

<400> 151
   gggacttgag ttctgttatc ttcttaagta gattcatatt gtaagggtct cggggtgggg 60
   gggttggcaa aatcctggag ccagaagaaa ggacagcagc attgatcaat cttacagcta 120
   acatgttgta cctggaaaac aatgcccaga ctcaatttag tgagccacag tacacgaacc 180
   tggggctcct gaacagcatg gaccagcaga ttcagaacgg ctcctcgtcc accagtccct 240
   ataacacaga ccacgcgcag aacagcgtca cggcgccctc gccctacgca cagcccagct 300
   ccaccttcga tgctctctct ccatcacccg ccatcccctc caacaccgac tacccaggcc 360
   cgcacagttt cgacgtgtcc ttccagcagt cgagcaccgc caagtcggcc acctggacgt 420
   attccactga actgaagaaa ctctactgcc aaattgcaaa gacatgcccc atccagatca 480
   aggtgatgac cccacctcct cagggagctg ttatccgcgc catgcctgtc tacaaaaaag 540
   ctgagcacgt cacggaggtg gtgaagcggt gccccaacca tgagctgagc cgtgaattca 600
   acgaggacaa gattgcccct yctagtcatt tgattcgagt agaggggaac agccatgccc 660
   agtatgtaga agatcccatc acaggaagac agagtgtgct ggtaccttat gagccacccc 720
   aggttggcac tgaattcacg acagtcttgt acaatttcat gtgtaacagc agttgtgttg 780
   gagggatgaa ccgccgtcca attttaatca ttgttactct ggaaaccaga gatgggcaag 840
   tcctgggccg acgctgcttt gaggcccgga tctgtgcttg cccaggaaga gacaggaagg 900
   cggatgaaga tagcatcaga aagcagcaag tttcggacag tacaaagaac ggtgatggta 960
   cgaagcgccc gtttcgtcag aacacacatg gtatccagat gacatccatc aagaaacgaa 1020
   gatccccaga tgatgaactg gtatacttac cagtgagggg ccgtgagact tatgaaatgc 1080
   tggtgaagat caaagagtcc ctggaactca tgcagtacct tcttcagcac acaattgaaa 1140
   cgtacaggca acagcaacag cagcagcacc agcacttact tcagaaacag acctcaatac 1200
   agtctccatc ttcatatggt aacagctccc cacctctgaa caaaatgaac agcatgaaca 1260
   agctgccttc tgtgagccag cttatcaacc ctcagcagcg caacgccctc actcctacaa 1320
   ccattcctga tggcatggga gccaacattc ccatgatggg cacccacatg ccaatggctg 1380
   gagacatgaa tggactcagc cccacccagg cactccctcc cccactctcc atgccatcca 1440
   cctcccactg cacaccccca cctccgtatc ccacagattg cagcattgtc agtttcttag 1500
   cgaggttggg ctgttcatca tgtctggact atttcacgac ccaggggctg accaccatct 1560
   atcagattga gcattactcc atggatgatc tggcaagtct gaaaatccct gagcaatttc 1620
   gacatgcgat ctggaagggc atcctggacc accggcagct ccacgaattc tcctcccctt 1680
   ctcatctcct gcggacccca agcagtgcct ctacagtcag tgtgggctcc agtgagaccc 1740
   ggggtgagcg tgttattgat gctgtgcgat tcaccctccg ccagaccatc tctttcccac 1800
   cccgagatga gtggaatgac ttcaactttg acatggatgc tcgccgcaat aagcaacagc 1860
   gcatcaaaga ggagggggag tgagcctcac catgtgagct cttcctatcc ctctcctaac 1920
   tgccagcccc ctaaaagcac tcctgcttaa tcttcaaagc cttctcccta gctcctcccc 1980
   ttcctcttgt ctgatttctt aggggaagga gaagtaagag gcttacttct taccctaacc 2040
   atctgacctg gcatctaatt ctgattctgg ctttaagcct tcaaaactat agcttgcaga 2100
   actgtagctt gccatggcta ggtagaagtg agcaaaaaag agttgggtgt ctccttaagc 2160
   tgcagagatt tctcattgac ttttataaag catgttcacc cttatagtct aagactatat 2220
   atataaatgt ataaatatac agtatagatt tttgggtggg gggcattgag tattgtttaa 2280
   aatgtaattt aaatgaaaga aaattgagtt gcacttattg accatttttt aatttacttg 2340
   ttttggatgg cttgtctata ctccttccct taaggggtat catgtatggt gataggtatc 2400
   tagagcttaa tgctacatgt gagtgacgat gatgtacaga ttctttcagt tctttggatt 2460
   ctaaatacat gccacatcaa acctttgagt agatccattt ccattgctta ttatgtaggt 2520
   aagactgtag atatgtattc ttttctcagt gttggtatat tttatattac tgacatttct 2580
   tctagtgatg atggttcacg ttggggtgat ttaatccagt tataagaaga agttcatgtc 2640
   caaacgtcct ctttagtttt tggttgggaa tgaggaaaat tcttaaaagg cccatagcag 2700
   ccagttcaaa aacacccgac gtcatgtatt tgagcatatc agtaaccccc ttaaatttaa 2760
   taccagatac cttatcttac aatattgatt gggaaaacat ttgctgccat tacagaggta 2820
   ttaaaactaa atttcactac tagattgact aactcaaata cacatttgct actgttgtaa 2880
   gaattctgat tgatttgatt gggatgaatg ccatctatct agttctaaca gtgaagtttt 2940
   actgtctatt aatattcagg gtaaatagga atcattcaga aatgttgagt ctgtactaaa 3000
   cagtaagata tctcaatgaa ccataaattc aactttgtaa aaatcttttg aagcatagat 3060
   aatattgttt ggtaaatgtt tcttttgttt ggtaaatgtt tcytttaaag accctcctat 3120
   tctataaaac tctgcatgta gaggcttgtt tacctttctc tctctaaggt ttacaatagg 3180
   agtggtgatt tgaaaaatat aaaattatga gattggtttt cctgtggcat aaattgcatc 3240
   actgtatcat tttctttttt aaccggtaag agtttcagtt tgttggaaag taactgtgag 3300
   aacccagttt cccgtccatc tcccttaggg actacccata gacatgaaag gtccccacag 3360
   agcaagagat aagtctttca tggctgctgt tgcttaaacc acttaaacga agagttccct 3420
   tgaaactttg ggaaaacatg ttaatgacaa tattccagat ctttcagaaa tataacacat 3480
   ttttttgcat gcatgcaaat gagctctgaa atcttcccat gcattctggt caagggctgt 3540
   cattgcacat aagcttccat tttaatttta aagtgcaaaa gggccagcgt ggctctaaaa 3600
   ggtaatgtgt ggattgcctc tgaaaagtgt gtatatattt tgtgtgaaat tgcatacttt 3660
   gtattttgat tatttttttt ttcttcttgg gatagtggga tttccagaac cacacttgaa 3720
   accttttttt atcgtttttg tattttcatg aaaataccat ttagtaagaa taccacatca 3780
   aataagaaat aatgctacaa ttttaagagg ggagggaagg gaaagttttt ttttttatta 3840
   tttttttaaa attttgtatg ttaaagagaa tgagtccttg atttcaaagt tttgttgtac 3900
   ttaaatggta ataagcactg taaacttctg caacaagcat gcagctttgc aaacccatta 3960
   aggggaagaa tgaaagctgt tccttggtcc tagtaagaag acaaactgct tcccttactt 4020
   tgctgagggt ttgaataaac ctaggacttc cgagctatgt cagtactatt caggtaacac 4080
   tagggccttg gaaatccctg tactgtgtct catggatttg gcactagcca aagcgaggca 4140
   ccccttactg gcttacctcc tcatggcagc ctactctcct tgagtgtatg agtagccagg 4200
   gtaaggggta aaaggatagt aagcatagaa accactagaa agtgggctta atggagttct 4260
   tgtggcctca gctcaatgca gttagctgaa gaattgaaaa gtttttgttt ggagacgttt 4320
   ataaacagaa atggaaagca gagttttcat taaatccttt tacctttttt ttttcttggt 4380
   aatcccctaa aataacagta tgtgggatat tgaatgttaa aggatatttt ttttctatta 4440
   tttttataat tgtacaaaat taagcaaatg ttaaaagttt tatatgcttt attaatgttt 4500
   tcaaaaggta ttatacatgt gatacatttt ttaagcttca gttgcttgtc ttctggtact 4560
   ttctgttatg ggcttttggg gagccagaag ccaatctaca atctcttttt gtttgccagg 4620
   acatgcaata aaatttaaaa aataaataaa aacta 4655

<210> 152
<211> 586
<212> PRT
<213> Homo sapiens

<400> 152
Met Leu Tyr Leu Glu Asn Asn Ala Gln Thr Gln Phe Ser Glu Pro Gln
1               5                   10                  15
Tyr Thr Asn Leu Gly Leu Leu Asn Ser Met Asp Gln Gln Ile Gln Asn
            20                  25                  30
Gly Ser Ser Ser Thr Ser Pro Tyr Asn Thr Asp His Ala Gln Asn Ser
        35                  40                  45
Val Thr Ala Pro Ser Pro Tyr Ala Gln Pro Ser Ser Thr Phe Asp Ala
    50                  55                  60
Leu Ser Pro Ser Pro Ala Ile Pro Ser Asn Thr Asp Tyr Pro Gly Pro
65                  70                  75                  80
His Ser Phe Asp Val Ser Phe Gln Gln Ser Ser Thr Ala Lys Ser Ala
                85                  90                  95
Thr Trp Thr Tyr Ser Thr Glu Leu Lys Lys Leu Tyr Cys Gln Ile Ala
            100                 105                 110
Lys Thr Cys Pro Ile Gln Ile Lys Val Met Thr Pro Pro Pro Gln Gly
        115                 120                 125
Ala Val Ile Arg Ala Met Pro Val Tyr Lys Lys Ala Glu His Val Thr
    130                 135                 140
Glu Val Val Lys Arg Cys Pro Asn His Glu Leu Ser Arg Glu Phe Asn
145                 150                 155                 160
Glu Gly Gln Ile Ala Pro Ser Ser His Leu Ile Arg Val Glu Gly Asn
                165                 170                 175
Ser His Ala Gln Tyr Val Glu Asp Pro Ile Thr Gly Arg Gln Ser Val
            180                 185                 190
Leu Val Pro Tyr Glu Pro Pro Gln Val Gly Thr Glu Phe Thr Thr Val

195                 200                 205
Leu Tyr Asn Phe Met Cys Asn Ser Ser Cys Val Gly Gly Met Asn Arg
    210                 215                 220
Arg Pro Ile Leu Ile Ile Val Thr Leu Glu Thr Arg Asp Gly Gln Val
225                 230                 235                 240
Leu Gly Arg Arg Cys Phe Glu Ala Arg Ile Cys Ala Cys Pro Gly Arg
                245                 250                 255
Asp Arg Lys Ala Asp Glu Asp Ser Ile Arg Lys Gln Gln Val Ser Asp
            260                 265                 270
Ser Thr Lys Asn Gly Asp Gly Thr Lys Arg Pro Phe Arg Gln Asn Thr
        275                 280                 285
His Gly Ile Gln Met Thr Ser Ile Lys Lys Arg Arg Ser Pro Asp Asp
    290                 295                 300
Glu Leu Val Tyr Leu Pro Val Arg Gly Arg Glu Thr Tyr Glu Met Leu
305                 310                 315                 320
Val Lys Ile Lys Glu Ser Leu Glu Leu Met Gln Tyr Leu Leu Gln His
                325                 330                 335
Thr Ile Glu Thr Tyr Arg Gln Gln Gln Gln Gln Gln His Gln His Leu
            340                 345                 350
Leu Gln Lys Gln Thr Ser Ile Gln Ser Pro Ser Ser Tyr Gly Asn Ser
        355                 360                 365
Ser Pro Pro Leu Asn Lys Met Asn Ser Met Asn Lys Leu Pro Ser Val
    370                 375                 380
Ser Gln Leu Ile Asn Pro Gln Gln Arg Asn Ala Leu Thr Pro Thr Thr
385                 390                 395                 400
Ile Pro Asp Gly Met Gly Ala Asn Ile Pro Met Met Gly Thr His Met
                405                 410                 415
Pro Met Ala Gly Asp Met Asn Gly Leu Ser Pro Thr Gln Ala Leu Pro
            420                 425                 430
Pro Pro Leu Ser Met Pro Ser Thr Ser His Cys Thr Pro Pro Pro Pro
        435                 440                 445
Tyr Pro Thr Asp Cys Ser Ile Val Ser Phe Leu Ala Arg Leu Gly Cys
    450                 455                 460
Ser Ser Cys Leu Asp Tyr Phe Thr Thr Gln Gly Leu Thr Thr Ile Tyr
465                 470                 475                 480
Gln Ile Glu His Tyr Ser Met Asp Asp Leu Ala Ser Leu Lys Ile Pro
                485                 490                 495
Glu Gln Phe Arg His Ala Ile Trp Lys Gly Ile Leu Asp His Arg Gln
            500                 505                 510
Leu His Glu Phe Ser Ser Pro Ser His Leu Leu Arg Thr Pro Ser Ser
        515                 520                 525
Ala Ser Thr Val Ser Val Gly Ser Ser Glu Thr Arg Gly Glu Arg Val
    530                 535                 540
Ile Asp Ala Val Arg Phe Thr Leu Arg Gln Thr Ile Ser Phe Pro Pro
545                 550                 555                 560
Arg Asp Glu Trp Asn Asp Phe Asn Phe Asp Met Asp Ala Arg Arg Asn
                565                 570                 575
Lys Gln Gln Arg Ile Lys Glu Glu Gly Glu
            580                 585

<210> 153
<211> 2007
<212> DNA
<213> Homo sapiens

<400> 153
gaattcgtcg ctgctccagg gaaagttctg ttactccact gactctctct tttcctgata 60

acatggccag caagaaagta attacagtgt ttggagcaac aggagctcaa ggtggctctg 120
tggccagggc aatttggag agcaaaaaat ttgcagtgag agcagtgacc agggatgtga 180
cttgaccaaa tgccctggag ctccagcgcc ttggagctga ggtggtcaaa ggtgacctga 240
atgataaagc atcggtggac agtgccttaa aaggtgtcta tggggccttc ttggtgacca 300
acttctggga ccctctcaac caagataagg aagtgtgtcg ggggaagctg gtggcagact 360
ccgccaagca cctgggtctg aagcacgtgg tgtacagcgg cctggagaac gtcaagcgac 420
tgacggatgg caagctggag gtgccgcact ttgacagcaa gggcgaggtg gaggagtact 480
tctggtccat tggcatcccc atgaccagtg tccgcgtggc ggcctacttt gaaaactttc 540
tcgcggcgtg gcggcccgtg aaagcctctg atggagatta ctacaccttg gctgtaccga 600
tgggagatgt accaatggat ggtatctctg ttgctgatat tggagcagcc gtctctagca 660
tttttaattc tccagaggaa tttttaggca aggccgtggg gctcagtgca gaagcactaa 720
caatacagca atatgctgat gttttgtcca aggctttggg gaaagaagtc cgagatgcaa 780
agattacccc ggaagctttc gagaagctgg gattccctgc agcaaaggaa atagccaata 840
tgtgtcgttt ctatgaaatg aagccagacc gagatgtcaa tctcacccac caactaaatc 900
ccaaagtcaa aagcttcagc cagtttatct cagagaacca gggagccttc aagggcatgt 960
agaaaatcag ctgttcagat aggcctctgc accacacagc ctctttcctc tctgatcctt 1020
ttcctcttta cggcacaaca ttcatgttga cagaacatgc tggaatgcaa ttgtttgcaa 1080
caccgaagga tttcctgcgg tcgcctcttc agtaggaagc actgcattgg tgataggaca 1140
cggtaatttg attcacattt aacttgctag ttagtgataa gggtggtaca actgtttggt 1200
aaaatgagaa gcctcggaac ttggagcttc tctcctacca ctaatgggag ggcagattat 1260
actgggattt ctcctgggtg agtaatttca agccctaatg ctgaaattcc cctaggcagc 1320
tccagttttc tcaactgcat tgcaaaattc ccagtgaact tttaagtact tttaacttaa 1380
aaaaatgaac atctttgtag agaattttct ggggaacatg gtgttcaatg aacaagcaca 1440
agcattggaa atgctaaaat tcagttttgc ctcaagattg gaagtttatt ttctgactca 1500
ttcatgaagt catctattga gccaccattc aattattcat ctattaattc cttgatcctt 1560
catttatcca ttctgcaaac ttttcttgag caccagcacg ggtggccatt tgtggacttc 1620
tcttcattcc tatgtgtttt cttatcaaag tgatccactc tcgaaaggct cctttccagt 1680
ctgtggttgg gttcaagtca tgccagggcc agggggccca tctcctcgtt tagctctagg 1740
caaaatccag gggatctgca gtggggagcg ggggcaggaa gctggaggga aggcctgtga 1800
agggtaggga tgtggaaaga caaggtgaca gaaggaccca ataggacctt tctatatctc 1860
tggcttagca ttttctacat catattgtaa tcgtcttatt tgctagtttt cttccttact 1920
gtgagtgact aacagtcatc tttatcccag tgcctggtac ataataagtg atcaataaat 1980
gttgattgac taaaaaaaaa aaaaaaa 2007

<210> 154
<211> 2148
<212> DNA
<213> Homo sapiens

<400> 154
gaattcgtcg ctgctccagg gaaagttctg ttactccact gactctctct tttcctgata 60
acatggccag caagaaagta attacagtgt ttggagcaac aggagctcaa ggtggctctg 120
tggccagggc aatttggag agcaaaaaat ttgcagtgag agcagtgacc agggatgtga 180
cttgaccaaa tgccctggag ctccagcgcc ttggagctga ggtggtcaaa ggtgacctga 240
atgataaagc atcggtggac agtgccttaa aaggggaagc tggtggcaga ctccgccaag 300
cacctgggtc tgaagcacgt ggtgtacagc ggcctggaga acgtcaagcg actgacggat 360
ggcaagctgg aggtgccgca ctttgacagc aagggcgagg tggaggagta cttctggtcc 420
attggcatcc ccatgaccag tgtccgcgtg gcggcctact ttgaaaactt tctcgcggcg 480
tggcggcccg tgaaagcctc tgatggagat tactacacct tggctgtacc gatgggagat 540
gtaccaatgg atggtatctc tgttgctgat attggagcag ccgtctctag catttttaat 600
tctccagagg aatttttagg caaggccgtg gggctcagtg cagaagcact aacaatacag 660
caatatgctg atgttttgtc caaggctttg gggaaagaag tccgagatgc aaagactatc 720
tgtgctatag atgaccagaa aacagtggaa gaaggtttca tggaagacgt gggcttgagt 780
tggtccttga gggaacatga ccatgtatag acagaggagg catcaagaag gctggcctgg 840
ctaattctgg aataaacacg acaaaccaga ggcagtacgg gaaggaggca aattctggct 900
ctgcctctat ccttgattac cccggaagct ttcgagaagc tgggattccc tgcagcaaag 960
gaaatagcca atatgtgtcg tttctatgaa atgaagccag accgagatgt caatctcacc 1020
caccaactaa atcccaaagt caaaagcttc agccatttta tctcagagaa ccagggagcc 1080

ttcaagggca tgtagaaaat cagctgttca gataggcctc tgcaccacac agcctctttc 1140
ctctctgatc cttttcctct ttacggcaca acattcatgt tgacagaaca tgctggaatg 1200
caattgtttg caacaccgaa ggatttcctg cggtcgcctc ttcagtagga agcactgcat 1260
tggtgatagg acacggtaat ttgattcaca tttaacttgc tagttagtga taagggtggt 1320
acaactgttt ggtaaaatga gaagcctcgg aacttggagc ttctctccta ccactaatgg 1380
gagggcagat tatactggga tttctcctgg gtgagtaatt tcaagcccta atgctgaaat 1440
tcccctaggc agctccagtt ttctcaactg cattgcaaaa ttcccagtga acttttaagt 1500
acttttaact taaaaaaatg aacatctttg tagagaattt tctggggaac atggtgttca 1560
atgaacaagc acaagcattg gaaatgctaa aattcagttt tgcctcaaga ttggaagttt 1620
attttctgac tcattcatga agtcatctat tgagccacca ttcaattatt catctattaa 1680
ttccttgatc cttcatttat ccattctgca aacttttctt gagcaccagc acgggtggcc 1740
atttgtggac ttctcttcat tcctatgtgt tttcttatca aagtgatcca ctctcgaaag 1800
gctcctttcc agtctgtggt tgggttcaag tcatgccagg gccagggggc ccatctcctc 1860
gtttagctct aggcaaaatc caggggatct gcagtgggga gcgggggcag gaagctggag 1920
ggaaggcctg tgaagggtag ggatgtggaa agacaaggtg acagaaggac ccaataggac 1980
ctttctatat ctctggctta gcattttcta catcatattg taatcgtctt atttgctagt 2040
tttcttcctt actgtgagtg actaacagtc atctttatcc cagtgcctgg tacataataa 2100
gtgatcaata aatgttgatt gactaaatga aaaaaaaaaa aaaaaaa 2148

<210> 155
<211> 153
<212> PRT
<213> Homo sapiens

<400> 155
Met Thr Ser Val Arg Val Ala Ala Tyr Phe Glu Asn Phe Leu Ala Ala
1               5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly
        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Ile Thr
                85                  90                  95
Pro Glu Ala Phe Glu Lys Leu Gly Phe Pro Ala Ala Lys Glu Ile Ala
            100                 105                 110
Asn Met Cys Arg Phe Tyr Glu Met Lys Pro Asp Arg Asp Val Asn Leu
        115                 120                 125
Thr His Gln Leu Asn Pro Lys Val Lys Ser Phe Ser Gln Phe Ile Ser
    130                 135                 140
Glu Asn Gln Gly Ala Phe Lys Gly Met
145                 150

<210> 156
<211> 128
<212> PRT
<213> Homo sapiens

<400> 156
Met Thr Ser Val Arg Val Ala Ala Tyr Phe Glu Asn Phe Leu Ala Ala
1               5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly

        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Thr Ile
                85                  90                  95
Cys Ala Ile Asp Asp Gln Lys Thr Val Glu Glu Gly Phe Met Glu Asp
            100                 105                 110
Val Gly Leu Ser Trp Ser Leu Arg Glu His Asp His Val Ala Gly Ala
        115                 120                 125

<210> 157
<211> 424
<212> DNA
<213> Homo sapiens

<220>
<221> misc_feature
<222> 320, 322
<223> n = A,T,C or G

<400> 157
ctgcagcccg ggggatccac tagtccagtg tggtggaatt cattggtctt tacaagactt 60
ggatacatta cagcagacat ggaaatataa ttttaaaaaa tttctctcca acctccttca 120
aattcagtca ccactgttat attaccttct ccaggaaccc tccagtgggg aaggctgcga 180
tattagattt ccttgtatgc aaagtttttg ttgaaagctg tgctcagagg aggtgagagg 240
agaggaagga gaaaactgca tcataacttt acagaattga atctagagtc ttccccgaaa 300
agcccagaaa cttctctgcn gnatctggct tgtccatctg gtctaaggtg gctgcttctt 360
ccccagccat cgagtcagtt tgtgcccatg aataatacac gacctgctat ttcccatgac 420
tgct                                                              424

<210> 158
<211> 2099
<212> DNA
<213> Homo sapiens

<400> 158
   ccgcggttaa aaggcgcagc aggtgggagc cggggccttc acccgaaacc cgacgagagc 60
   ccgacagccg gcggcgcccg agcccgacct gcctgcccag ccggagcgaa gggcgccgcc 120
   ccgcgcagag cccgcgccag ggccgccggc cgcagagcag ttaaaacgtg caggcaccag 180
   aaggcacttc ctgtcggtga agaagacctg tctccggtgt cacgggcatc ctgtgttttg 240
   caaacggggc tgacctccct tcctggggag caggaagggt cagggaagga aaagaagtac 300
   agaagatctg gctaaacaat ttctgtatgg cgaaagaaaa attctaactt gtacgccctc 360
   ttcatgcatc tttaattcaa tttgaatatt ccaggcgaca tcctcactga ccgagcaaag 420
   attgacattc gtatcatcac tgtgcaccat tggcttctag gcactccagt ggggtaggag 480
   aaggaggtct gaaaccctcg cagagggatc ttgccctcat tctttgggtc tgaaacactg 540
   gcagtcgttg gaaacaggac tcagggataa accagcgcaa tggattgggg gacgctgcac 600
   actttcatcg ggggtgtcaa caaacactcc accagcatcg ggaaggtgtg gatcacagtc 660
   atctttattt tccgagtcat gatcctcgtg gtggctgccc aggaagtgtg gggtgacgag 720
   caagaggact tcgtctgcaa cacactgcaa ccgggatgca aaaatgtgtg ctatgaccac 780
   tttttcccgg tgtcccacat ccggctgtgg gccctccagc tgatcttcgt ctccacccca 840
   gcgctgctgg tggccatgca tgtggcctac tacaggcacg aaaccactcg caagttcagg 900
   cgaggagaga agaggaatga tttcaaagac atagaggaca ttaaaaagca gaaggttcgg 960
   atagaggggt cgctgtggtg gacgtacacc agcagcatct ttttccgaat catctttgaa 1020
   gcagccttta tgtatgtgtt ttacttcctt tacaatgggt accacctgcc ctgggtgttg 1080
   aaatgtggga ttgacccctg ccccaacctt gttgactgct ttatttctag gccaacagag 1140
   aagaccgtgt ttaccatttt tatgatttct gcgtctgtga tttgcatgct gcttaacgtg 1200
   gcagagttgt gctacctgct gctgaaagtg tgttttagga gatcaaagag agcacagacg 1260
   caaaaaaatc accccaatca tgccctaaag gagagtaagc agaatgaaat gaatgagctg 1320
   atttcagata gtggtcaaaa tgcaatcaca ggttcccaag ctaaacattt caaggtaaaa 1380
   tgtagctgcg tcataaggag acttctgtct tctccagaag gcaataccaa cctgaaagtt 1440
   ccttctgtag cctgaagagt ttgtaaatga ctttcataat aaatagacac ttgagttaac 1500
   tttttgtagg atacttgctc cattcataca caacgtaatc aaatatgtgg tccatctctg 1560
   aaaacaagag actgcttgac aaaggagcat tgcagtcact ttgacaggtt ccttttaagt 1620
   ggactctctg acaaagtggg tactttctga aaatttatat aactgttgtt gataaggaac 1680
   atttatccag gaattgatac gtttattagg aaaagatatt tttataggct tggatgtttt 1740
   tagttctgac tttgaattta tataaagtat ttttataatg actggtcttc cttacctgga 1800
   aaaacatgcg atgttagttt tagaattaca ccacaagtat ctaaatttgg aacttacaaa 1860
   gggtctatct tgtaaatatt gttttgcatt gtctgttggc aaatttgtga actgtcatga 1920
   tacgcttaag gtggaaagtg ttcattgcac aatatatttt tactgctttc tgaatgtaga 1980
   cggaacagtg tggaagcaga aggctttttt aactcatccg tttgccaatc attgcaaaca 2040
   actgaaatgt ggatgtgatt gcctcaataa agctcgtccc cattgcttaa aaaaaaaaa 2099

<210> 159
<211> 291
<212> PRT
<213> Homo sapiens

<400> 159
Met Asp Trp Gly Thr Leu His Thr Phe Ile Gly Gly Val Asn Lys His
1               5                   10                  15
Ser Thr Ser Ile Gly Lys Val Trp Ile Thr Val Ile Phe Ile Phe Arg
            20                  25                  30
Val Met Ile Leu Val Val Ala Ala Gln Glu Val Trp Gly Asp Glu Gln
        35                  40                  45
Glu Asp Phe Val Cys Asn Thr Leu Gln Pro Gly Cys Lys Asn Val Cys
    50                  55                  60
Tyr Asp His Phe Phe Pro Val Ser His Ile Arg Leu Trp Ala Leu Gln
65                  70                  75                  80
Leu Ile Phe Val Ser Thr Pro Ala Leu Leu Val Ala Met His Val Ala
                85                  90                  95
Tyr Tyr Arg His Glu Thr Thr Arg Lys Phe Arg Arg Gly Glu Lys Arg
            100                 105                 110
Asn Asp Phe Lys Asp Ile Glu Asp Ile Lys Lys Gln Lys Val Arg Ile
        115                 120                 125
Glu Gly Ser Leu Trp Trp Thr Tyr Thr Ser Ser Ile Phe Phe Arg Ile
    130                 135                 140
Ile Phe Glu Ala Ala Phe Met Tyr Val Phe Tyr Phe Leu Tyr Asn Gly
145                 150                 155                 160
Tyr His Leu Pro Trp Val Leu Lys Cys Gly Ile Asp Pro Cys Pro Asn
                165                 170                 175
Leu Val Asp Cys Phe Ile Ser Arg Pro Thr Glu Lys Thr Val Phe Thr
            180                 185                 190
Ile Phe Met Ile Ser Ala Ser Val Ile Cys Met Leu Leu Asn Val Ala
        195                 200                 205
Glu Leu Cys Tyr Leu Leu Leu Lys Val Cys Phe Arg Arg Ser Lys Arg
    210                 215                 220
Ala Gln Thr Gln Lys Asn His Pro Asn His Ala Leu Lys Glu Ser Lys
225                 230                 235                 240
Gln Asn Glu Met Asn Glu Leu Ile Ser Asp Ser Gly Gln Asn Ala Ile
                245                 250                 255
Thr Gly Ser Gln Ala Lys His Phe Lys Val Lys Cys Ser Cys Val Ile
            260                 265                 270
Arg Arg Leu Leu Ser Ser Pro Glu Gly Asn Thr Asn Leu Lys Val Pro

275                280                285
Ser Val Ala
    290

<210> 160
<211> 3951
<212> DNA
<213> Homo sapiens

<400> 160
tctgcatcca tattgaaaac ctgacacaat gtatgcagca ggctcagtgt gagtgaactg 60
gaggcttctc tacaacatga cccaaaggag cattgcaggt cctatttgca acctgaagtt 120
tgtgactctc ctggttgcct taagttcaga actcccattc ctgggagctg gagtacagct 180
tcaagacaat gggtataatg gattgctcat tgcaattaat cctcaggtac ctgagaatca 240
gaacctcatc tcaaacatta aggaaatgat aactgaagct tcattttacc tatttaatgc 300
taccaagaga agagtatttt tcagaaatat aaagatttta atacctgcca catggaaagc 360
taataataac agcaaaataa aacaagaatc atatgaaaag gcaaatgtca tagtgactga 420
ctggtatggg gcacatggag atgatccata caccctacaa tacagagggt gtggaaaaga 480
gggaaaatac attcatttca cacctaattt cctactgaat gataacttaa cagctggcta 540
cggatcacga ggccgagtgt ttgtccatga atgggcccac ctccgttggg gtgtgttcga 600
tgagtataac aatgacaaac ctttctacat aaatgggcaa aatcaaatta aagtgacaag 660
gtgttcatct gacatcacag gcatttttgt gtgtgaaaaa ggtccttgcc cccaagaaaa 720
ctgtattatt agtaagcttt ttaaagaagg atgcaccttt atctacaata gcacccaaaa 780
tgcaactgca tcaataatgt tcatgcaaag tttatcttct gtggttgaat tttgtaatgc 840
aagtacccac aaccaagaag caccaaacct acagaaccag atgtgcagcc tcagaagtgc 900
atgggatgta atcacagact ctgctgactt tcaccacagc tttcccatga acgggactga 960
gcttccacct cctcccacat tctcgcttgt agaggctggt gacaaagtgg tctgtttagt 1020
gctggatgtg tccagcaaga tggcagaggc tgacagactc cttcaactac aacaagccgc 1080
agaattttat ttgatgcaga ttgttgaaat tcataccttc gtgggcattg ccagtttcga 1140
cagcaaagga gagatcagag cccagctaca ccaaattaac agcaatgatg atcgaaagtt 1200
gctggtttca tatctgccca ccactgtatc agctaaaaca gacatcagca tttgttcagg 1260
gcttaagaaa ggatttgagg tggttgaaaa actgaatgga aaagcttatg gctctgtgat 1320
gatattagtg accagcggag atgataagct tcttggcaat tgcttaccca ctgtgctcag 1380
cagtggttca acaattcact ccattgccct gggttcatct gcagccccaa atctggagga 1440
attatcacgt cttacaggag gtttaaagtt ctttgttcca gatatatcaa actccaatag 1500
catgattgat gctttcagta gaatttcctc tggaactgga gacattttcc agcaacatat 1560
tcagcttgaa agtacaggtg aaaatgtcaa acctcaccat caattgaaaa acacagtgac 1620
tgtggataat actgtgggca acgacactat gtttctagtt acgtggcagg ccagtggtcc 1680
tcctgagatt atattatttg atcctgatgg acgaaaatac tacacaaata attttatcac 1740
caatctaact tttcggacag ctagtctttg gattccagga acagctaagc ctgggcactg 1800
gacttacacc ctgaacaata cccatcattc tctgcaagcc ctgaaagtga cagtgacctc 1860
tcgcgcctcc aactcagctg tgcccccagc cactgtggaa gcctttgtgg aaagagacag 1920
cctccatttt cctcatcctg tgatgattta tgccaatgtg aaacagggat tttatcccat 1980
tcttaatgcc actgtcactg ccacagttga gccagagact ggagatcctg ttacgctgag 2040
actccttgat gatggagcag gtgctgatgt tataaaaaat gatggaattt actcgaggta 2100
ttttttctcc tttgctgcaa atggtagata tagcttgaaa gtgcatgtca atcactctcc 2160
cagcataagc accccagccc actctattcc agggagtcat gctatgtatg taccaggtta 2220
cacagcaaac ggtaatattc agatgaatgc tccaaggaaa tcagtaggca gaaatgagga 2280
ggagcgaaag tggggcttta gccgagtcag ctcaggaggc tccttttcag tgctgggagt 2340
tccagctggc ccccaccctg atgtgtttcc accatgcaaa attattgacc tggaagctgt 2400
aaaagtagaa gaggaattga ccctatcttg gacagcacct ggagaagact ttgatcaggg 2460
ccaggctaca agctatgaaa taagaatgag taaaagtcta cagaatatcc aagatgactt 2520
taacaatgct attttagtaa atacatcaaa gcgaaatcct cagcaagctg gcatcaggga 2580
gatatttacg ttctcacccc aaatttccac gaatggacct gaacatcagc caaatggaga 2640
aacacatgaa agccacagaa tttatgttgc aatacgagca atggatagga actccttaca 2700
gtctgctgta tctaacattg cccaggcgcc tctgtttatt ccccccaatt ctgatcctgt 2760
acctgccaga gattatctta tattgaaagg agttttaaca gcaatgggtt tgataggaat 2820

catttgcctt attatagttg tgacacatca tactttaagc aggaaaaaga gagcagacaa 2880
gaaagagaat ggaacaaaat tattataaat aaatatccaa agtgtcttcc ttcttagata 2940
taagacccat ggccttcgac tacaaaaaca tactaacaaa gtcaaattaa catcaaaact 3000
gtattaaaat gcattgagtt tttgtacaat acagataaga tttttacatg gtagatcaac 3060
aaattctttt tgggggtaga ttagaaaacc cttacacttt ggctatgaac aaataataaa 3120
aattattctt taaagtaatg tctttaaagg caaagggaag ggtaaagtcg gaccagtgtc 3180
aaggaaagtt tgttttattg aggtggaaaa atagccccaa gcagagaaaa ggagggtagg 3240
tctgcattat aactgtctgt gtgaagcaat catttagtta ctttgattaa tttttctttt 3300
ctccttatct gtgcagaaca ggttgcttgt ttacaactga agatcatgct atatttcata 3360
tatgaagccc ctaatgcaaa gctctttacc tcttgctatt ttgttatata tattacagat 3420
gaaatctcac tgctaatgct cagagatctt ttttcactgt aagaggtaac ctttaacaat 3480
atgggtatta cctttgtctc ttcataccgg ttttatgaca aaggtctatt gaatttattt 3540
gtttgtaagt ttctactccc atcaaagcag ctttttaagt tattgccttg gttattatgg 3600
atgatagtta tagcccttat aatgccttaa ctaaggaaga aaagatgtta ttctgagttt 3660
gttttaatac atatatgaac atatagtttt attcaattaa accaaagaag aggtcagcag 3720
ggagatacta acctttggaa atgattagct ggctctgttt tttggttaaa taagagtctt 3780
taatcctttc tccatcaaga gttacttacc aagggcaggg gaagggggat atagaggtcc 3840
caaggaaata aaaatcatct ttcatcttta attttactcc ttcctcttat ttttttaaaa 3900
gattatcgaa caataaaatc atttgccttt ttaattaaaa acataaaaaa a 3951

<210> 161
<211> 943
<212> PRT
<213> Homo sapiens

<400> 161

<210> 162
<211> 498
<212> DNA
<213> Homo sapiens

<400> 162
tggagaacca cgtggacagc accatgaaca tgttgggcgg gggaggcagt gctggccgga 60
agcccctcaa gtcgggtatg aaggagctgg ccgtgttccg ggagaaggtc actgagcagc 120
accggcagat gggcaagggt ggcaagcatc accttggcct ggaggagccc aagaagctgc 180
gaccaccccc tgccaggact ccctgccaac aggaactgga ccaggtcctg gagcggatct 240
ccaccatgcg ccttccggat gagcggggcc ctctggagca cctctactcc ctgcacatcc 300
ccaactgtga caagcatggc ctgtacaacc tcaaacagtg gcaagatgtc tctgaacggg 360
cagcgtgggg agtgctggtg tgtgaacccc aacaccggga agctgatcca gggagccccc 420
accatccggg gggaccccga gtgtcatctc ttctacaatg agcagcagga ggctcgcggg 480
gtgcacaccc cagcggat                                                498

<210> 163
<211> 1128
<212> DNA
<213> Homo sapiens

<400> 163
gccacctggc cctcctgatc gacgacacac gcacttgaaa cttgttctca gggtgtgtgg 60
aatcaacttt ccggaagcaa ccagcccacc agaggaggtc ccgagcgcga gcggagacga 120
tgcagcggag actggttcag cagtggagcg tcgcggtgtt cctgctgagc tacgcggtgc 180
cctcctgcgg gcgctcggtg gagggtctca gccgccgcct caaaagagct gtgtctgaac 240
atcagctcct ccatgacaag gggaagtcca tccaagattt acggcgacga ttcttccttc 300
accatctgat cgcagaaatc cacacagctg aaatcagagc tacctcggag gtgtccccta 360

actccaagcc ctctcccaac acaaagaacc accccgtccg atttgggtct gatgatgagg 420
gcagatacct aactcaggaa actaacaagg tggagacgta caaagagcag ccgctcaaga 480
cacctgggaa gaaaaagaaa ggcaagcccg ggaaacgcaa ggagcaggaa aagaaaaaac 540
ggcgaactcg ctctgcctgg ttagactctg gagtgactgg gagtgggcta gaaggggacc 600
acctgtctga cacctccaca acgtcgctgg agctcgattc acggaggcat tgaaattttc 660
agcagagacc ttccaaggac atattgcagg attctgtaat agtgaacata tggaaagtat 720
tagaaatatt tattgtctgt aaatactgta aatgcattgg aataaaactg tctcccccat 780
tgctctatga aactgcacat tggtcattgt gaatattttt ttttttgcca aggctaatcc 840
aattattatt atcacattta ccataattta ttttgtccat tgatgtattt attttgtaaa 900
tgtatcttgg tgctgctgaa tttctatatt ttttgtaaca taatgcactt tagatataca 960
tatcaagtat gttgataaat gacacaatga agtgtctcta ttttgtggtt gattttaatg 1020
aatgcctaaa tataattatc caaattgatt ttcctttgtg catgtaaaaa taacagtatt 1080
ttaaatttgt aaagaatgtc taataaaata taatctaatt acatcatg 1128

<210> 164
<211> 1310
<212> DNA
<213> Homo sapiens

<400> 164
gggcctggtt cgcaaagaag ctgacttcag agggggaaac tttcttcttt taggaggcgg 60
ttagccctgt tccacgaacc caggagaact gctggccaga ttaattagac attgctatgg 120
gagacgtgta aacacactac ttatcattga tgcatatata aaaccatttt attttcgcta 180
ttatttcaga ggaagcgcct ctgatttgtt tcttttttcc ctttttgctc tttctggctg 240
tgtggtttgg agaaagcaca gttggagtag ccggttgcta aataagtccc gagcgcgagc 300
ggagacgatg cagcggagac tggttcagca gtggagcgtc gcggtgttcc tgctgagcta 360
cgcggtgccc tcctgcgggc gctcggtgga gggtctcagc cgccgcctca aaagagctgt 420
gtctgaacat cagctcctcc atgacaaggg gaagtccatc caagatttac ggcgacgatt 480
cttccttcac catctgatcg cagaaatcca cacagctgaa atcagagcta cctcggaggt 540
gtcccctaac tccaagccct ctcccaacac aaagaaccac cccgtccgat ttgggtctga 600
tgatgagggc agatacctaa ctcaggaaac taacaaggtg gagacgtaca aagagcagcc 660
gctcaagaca cctgggaaga aaaagaaagg caagcccggg aaacgcaagg agcaggaaaa 720
gaaaaaacgg cgaactcgct ctgcctggtt agactctgga gtgactggga gtgggctaga 780
aggggaccac ctgtctgaca cctccacaac gtcgctggag ctcgattcac ggaggcattg 840
aaattttcag cagagacctt ccaaggacat attgcaggat tctgtaatag tgaacatatg 900
gaaagtatta gaaatattta ttgtctgtaa atactgtaaa tgcattggaa taaaactgtc 960
tcccccattg ctctatgaaa ctgcacattg gtcattgtga atattttttt ttttgccaag 1020
gctaatccaa ttattattat cacatttacc ataatttatt ttgtccattg atgtatttat 1080
tttgtaaatg tatcttggtg ctgctgaatt tctatatttt ttgtaacata atgcacttta 1140
gatatacata tcaagtatgt tgataaatga cacaatgaag tgtctctatt ttgtggttga 1200
ttttaatgaa tgcctaaata taattatcca aattgatttt cctttgtgcc cgtaaaaata 1260
acagtatttt aaatttgtaa agaatgtcta ataaaatata atctaattac 1310

<210> 165
<211> 177
<212> PRT
<213> Homo sapiens

<400> 165

<210> 166
<211> 177
<212> PRT
<213> Homo sapiens

<400> 166

<210> 167
<211> 3362
<212> DNA
<213> Homo sapiens

<400> 167
cacaatgtat gcagcaggct cagtgtgagt gaactggagg cttctctaca acatgaccca 60
aaggagcatt gcaggtccta tttgcaacct gaagtttgtg actctcctgg ttgccttaag 120
ttcagaactc ccattcctgg gagctggagt acagcttcaa gacaatgggt ataatggatt 180

gctcattgca attaatcctc aggtacctga gaatcagaac ctcatctcaa acattaagga 240
aatgataact gaagcttcat tttacctatt taatgctacc aagagaagag tatttttcag 300
aaatataaag attttaatac ctgccacatg gaaagctaat aataacagca aaataaaaca 360
agaatcatat gaaaaggcaa atgtcatagt gactgactgg tatggggcac atggagatga 420
tccatacacc ctacaataca gagggtgtgg aaaagaggga aaatacattc atttcacacc 480
taatttccta ctgaatgata acttaacagc tggctacgga tcacgaggcc gagtgtttgt 540
ccatgaatgg gcccacctcc gttggggtgt gttcgatgag tataacaatg acaaaccttt 600
ctacataaat gggcaaaatc aaattaaagt gacaaggtgt tcatctgaca tcacaggcat 660
ttttgtgtgt gaaaaaggtc cttgccccca agaaaactgt attattagta agctttttaa 720
agaaggatgc acctttatct acaatagcac ccaaaatgca actgcatcaa taatgttcat 780
gcaaagttta tcttctgtgg ttgaattttg taatgcaagt acccacaacc aagaagcacc 840
aaacctacag aaccagatgt gcagcctcag aagtgcatgg gatgtaatca cagactctgc 900
tgactttcac cacagctttc ccatgaacgg gactgagctt ccacctcctc ccacattctc 960
gcttgtagag gctggtgaca aagtggtctg tttagtgctg gatgtgtcca gcaagatggc 1020
agaggctgac agactccttc aactacaaca agccgcagaa ttttatttga tgcagattgt 1080
tgaaattcat accttcgtgg gcattgccag tttcgacagc aaaggagaga tcagagccca 1140
gctacaccaa attaacagca atgatgatcg aaagttgctg gtttcatatc tgcccaccac 1200
tgtatcagct aaaacagaca tcagcatttg ttcagggctt aagaaaggat ttgaggtggt 1260
tgaaaaactg aatggaaaag cttatggctc tgtgatgata ttagtgacca gcggagatga 1320
taagcttctt ggcaattgct tacccactgt gctcagcagt ggttcaacaa ttcactccat 1380
tgccctgggt tcatctgcag ccccaaatct ggaggaatta tcacgtctta caggaggttt 1440
aaagttcttt gttccagata tatcaaactc caatagcatg attgatgctt tcagtagaat 1500
ttcctctgga actggagaca ttttccagca acatattcag cttgaaagta caggtgaaaa 1560
tgtcaaacct caccatcaat tgaaaaacac agtgactgtg gataatactg tgggcaacga 1620
cactatgttt ctagttacgt ggcaggccag tggtcctcct gagattatat tatttgatcc 1680
tgatggacga aaatactaca caaataattt tatcaccaat ctaacttttc ggacagctag 1740
tctttggatt ccaggaacag ctaagcctgg gcactggact tacaccctga tgtgtttcca 1800
ccatgcaaaa ttattgacct ggaagctgta aaagtagaag aggaattgac cctatcttgg 1860
acagcacctg gagaagactt tgatcagggc caggctacaa gctatgaaat aagaatgagt 1920
aaaagtctac agaatatcca agatgacttt aacaatgcta ttttagtaaa tacatcaaag 1980
cgaaatcctc agcaagctgg catcagggag atatttacgt tctcacccca aatttccacg 2040
aatggacctg aacatcagcc aaatggagaa acacatgaaa gccacagaat ttatgttgca 2100
atacgagcaa tggataggaa ctccttacag tctgctgtat ctaacattgc ccaggcgcct 2160
ctgtttattc cccccaattc tgatcctgta cctgccagag attatcttat attgaaagga 2220
gttttaacag caatgggttt gataggaatc atttgcctta ttatagttgt gacacatcat 2280
actttaagca ggaaaaagag agcagacaag aaagagaatg gaacaaaatt attataaata 2340
aatatccaaa gtgtcttcct tcttagatat aagacccatg gccttcgact acaaaaacat 2400
actaacaaag tcaaattaac atcaaaactg tattaaaatg cattgagttt ttgtacaata 2460
cagataagat ttttacatgg tagatcaaca aattcttttt gggggtagat tagaaaaccc 2520
ttacactttg gctatgaaca aataataaaa attattcttt aaagtaatgt ctttaaaggc 2580
aaagggaagg gtaaagtcgg accagtgtca aggaaagttt gttttattga ggtggaaaaa 2640
tagccccaag cagagaaaag gagggtaggt ctgcattata actgtctgtg tgaagcaatc 2700
atttagttac tttgattaat ttttcttttc tccttatctg tgcagaacag gttgcttgtt 2760
tacaactgaa gatcatgcta tatttcatat atgaagcccc taatgcaaag ctctttacct 2820
cttgctattt tgttatatat attacagatg aaatctcact gctaatgctc agagatcttt 2880
tttcactgta agaggtaacc tttaacaata tgggtattac ctttgtctct tcataccggt 2940
tttatgacaa aggtctattg aatttatttg tttgtaagtt tctactccca tcaaagcagc 3000
tttctaagtt attgccttgg ttattatgga tgatagttat agcccttata atgccttaac 3060
taaggaagaa aagatgttat tctgagtttg ttttaataca tatatgaaca tatagtttta 3120
ttcaattaaa ccaaagaaga ggtcagcagg gagatactaa cctttggaaa tgattagctg 3180
gctctgtttt ttggttaaat aagagtcttt aatcctttct ccatcaagag ttacttacca 3240
agggcagggg aagggggata tagaggtcac aaggaaataa aaatcatctt tcatctttaa 3300
ttttactcct tcctcttatt tttttaaaag attatcgaac aataaaatca tttgcctttt 3360
tt 3362

<210> 168
<211> 2784
<212> DNA

<213> Homo sapiens

<400> 168
tctgcatcca tattgaaaac ctgacacaat gtatgcagca ggctcagtgt gagtgaactg 60
gaggcttctc tacaacatga cccaaaggag cattgcaggt cctatttgca acctgaagtt 120
tgtgactctc ctggttgcct taagttcaga actcccattc ctgggagctg gagtacagct 180
tcaagacaat gggtataatg gattgctcat tgcaattaat cctcaggtac ctgagaatca 240
gaacctcatc tcaaacatta aggaaatgat aactgaagct tcattttacc tatttaatgc 300
taccaagaga agagtatttt tcagaaatat aaagatttta atacctgcca catggaaagc 360
taataataac agcaaaataa aacaagaatc atatgaaaag gcaaatgtca tagtgactga 420
ctggtatggg gcacatggag atgatccata caccctacaa tacagagggt gtggaaaaga 480
gggaaaatac attcatttca cacctaattt cctactgaat gataacttaa cagctggcta 540
cggatcacga ggccgagtgt ttgtccatga atgggcccac ctccgttggg gtgtgttcga 600
tgagtataac aatgacaaac ctttctacat aaatgggcaa aatcaaatta aagtgacaag 660
gtgttcatct gacatcacag gcatttttgt gtgtgaaaaa ggtccttgcc cccaagaaaa 720
ctgtattatt agtaagcttt ttaaagaagg atgcaccttt atctacaata gcacccaaaa 780
tgcaactgca tcaataatgt tcatgcaaag tttatcttct gtggttgaat tttgtaatgc 840
aagtacccac aaccaagaag caccaaacct acagaaccag atgtgcagcc tcagaagtgc 900
atgggatgta atcacagact ctgctgactt tcaccacagc tttcccatga acgggactga 960
gcttccacct cctcccacat tctcgcttgt agaggctggt gacaaagtgg tctgtttagt 1020
gctggatgtg tccagcaaga tggcagaggc tgacagactc cttcaactac aacaagccgc 1080
agaattttat ttgatgcaga ttgttgaaat tcataccttc gtgggcattg ccagtttcga 1140
cagcaaagga gagatcagag cccagctaca ccaaattaac agcaatgatg atcgaaagtt 1200
gctggtttca tatctgccca ccactgtatc agctaaaaca gacatcagca tttgttcagg 1260
gcttaagaaa ggatttgagg tggttgaaaa actgaatgga aaagcttatg gctctgtgat 1320
gatattagtg accagcggag atgataagct tcttggcaat tgcttaccca ctgtgctcag 1380
cagtggttca acaattcact ccattgccct gggttcatct gcagccccaa atctggagga 1440
attatcacgt cttacaggag gtttaaagtt ctttgttcca gatatatcaa actccaatag 1500
catgattgat gctttcagta gaatttcctc tggaactgga gacattttcc agcaacatat 1560
tcagcttgaa agtacaggtg aaaatgtcaa acctcaccat caattgaaaa acacagtgac 1620
tgtggataat actgtgggca acgacactat gtttctagtt acgtggcagg ccagtggtcc 1680
tcctgagatt atattatttg atcctgatgg acgaaaatac tacacaaata attttatcac 1740
caatctaact tttcggacag ctagtctttg gattccagga acagctaagc ctgggcactg 1800
gacttacacc ctgaacaata cccatcattc tctgcaagcc ctgaaagtga cagtgacctc 1860
tcgcgcctcc aactcagctg tgcccccagc cactgtggaa gcctttgtgg aaagagacag 1920
cctccatttt cctcatcctg tgatgattta tgccaatgtg aaacagggat tttatcccat 1980
tcttaatgcc actgtcactg ccacagttga gccagagact ggagatcctg ttacgctgag 2040
actccttgat gatggagcag gtgctgatgt tataaaaaat gatggaattt actcgaggta 2100
ttttttctcc tttgctgcaa atggtagata tagcttgaaa gtgcatgtca atcactctcc 2160
cagcataagc accccagccc actctattcc agggagtcat gctatgtatg taccaggtta 2220
cacagcaaac ggtaatattc agatgaatgc tccaaggaaa tcagtaggca gaaatgagga 2280
ggagcgaaag tggggcttta gccgagtcag ctcaggaggc tccttttcag tgctgggagt 2340
tccagctggc ccccaccctg atgtgtttcc accatgcaaa attattgacc tggaagctgt 2400
aaatagaaga ggaattgacc ctatcttgga cagcacctgg agaagacttt gatcagggcc 2460
aggctacaag ctatgaaata agaatgagta aaagtctaca gaatatccaa gatgacttta 2520
acaatgctat tttagtaaat acatcaaagc gaaatcctca gcaagctggc atcaggagaa 2580
tatttacgtt ctcaccccaa atttccacga atggacctga acatcagcca aatggagaaa 2640
cacatgaaag ccacagaatt tatgttgcaa tacgagcaat ggataggaac tccttacagt 2700
ctgctgtatc taacattgcc caggcgcctc tgtttattcc ccccaattct gatcctgtac 2760
ctgccagaga ttatcttata ttga 2784

<210> 169
<211> 592
<212> PRT
<213> Homo sapiens

<400> 169
Met Thr Gln Arg Ser Ile Ala Gly Pro Ile Cys Asn Leu Lys Phe Val

1               5                   10                  15
Thr Leu Leu Val Ala Leu Ser Ser Glu Leu Pro Phe Leu Gly Ala Gly
                20                  25                  30
Val Gln Leu Gln Asp Asn Gly Tyr Asn Gly Leu Leu Ile Ala Ile Asn
            35                  40                  45
Pro Gln Val Pro Glu Asn Gln Asn Leu Ile Ser Asn Ile Lys Glu Met
    50                  55                  60
Ile Thr Glu Ala Ser Phe Tyr Leu Phe Asn Ala Thr Lys Arg Arg Val
65                  70                  75                  80
Phe Phe Arg Asn Ile Lys Ile Leu Ile Pro Ala Thr Trp Lys Ala Asn
                85                  90                  95
Asn Asn Ser Lys Ile Lys Gln Glu Ser Tyr Glu Lys Ala Asn Val Ile
            100                 105                 110
Val Thr Asp Trp Tyr Gly Ala His Gly Asp Asp Pro Tyr Thr Leu Gln
        115                 120                 125
Tyr Arg Gly Cys Gly Lys Glu Gly Lys Tyr Ile His Phe Thr Pro Asn
    130                 135                 140
Phe Leu Leu Asn Asp Asn Leu Thr Ala Gly Tyr Gly Ser Arg Gly Arg
145                 150                 155                 160
Val Phe Val His Glu Trp Ala His Leu Arg Trp Gly Val Phe Asp Glu
                165                 170                 175
Tyr Asn Asn Asp Lys Pro Phe Tyr Ile Asn Gly Gln Asn Gln Ile Lys
            180                 185                 190
Val Thr Arg Cys Ser Ser Asp Ile Thr Gly Ile Phe Val Cys Glu Lys
        195                 200                 205
Gly Pro Cys Pro Gln Glu Asn Cys Ile Ile Ser Lys Leu Phe Lys Glu
    210                 215                 220
Gly Cys Thr Phe Ile Tyr Asn Ser Thr Gln Asn Ala Thr Ala Ser Ile
225                 230                 235                 240
Met Phe Met Gln Ser Leu Ser Ser Val Val Glu Phe Cys Asn Ala Ser
                245                 250                 255
Thr His Asn Gln Glu Ala Pro Asn Leu Gln Asn Gln Met Cys Ser Leu
            260                 265                 270
Arg Ser Ala Trp Asp Val Ile Thr Asp Ser Ala Asp Phe His His Ser
        275                 280                 285
Phe Pro Met Asn Gly Thr Glu Leu Pro Pro Pro Pro Thr Phe Ser Leu
    290                 295                 300
Val Glu Ala Gly Asp Lys Val Val Cys Leu Val Leu Asp Val Ser Ser
305                 310                 315                 320
Lys Met Ala Glu Ala Asp Arg Leu Leu Gln Leu Gln Gln Ala Ala Glu
                325                 330                 335
Phe Tyr Leu Met Gln Ile Val Glu Ile His Thr Phe Val Gly Ile Ala
            340                 345                 350
Ser Phe Asp Ser Lys Gly Glu Ile Arg Ala Gln Leu His Gln Ile Asn
        355                 360                 365
Ser Asn Asp Asp Arg Lys Leu Leu Val Ser Tyr Leu Pro Thr Thr Val
    370                 375                 380
Ser Ala Lys Thr Asp Ile Ser Ile Cys Ser Gly Leu Lys Lys Gly Phe
385                 390                 395                 400
Glu Val Val Glu Lys Leu Asn Gly Lys Ala Tyr Gly Ser Val Met Ile
                405                 410                 415
Leu Val Thr Ser Gly Asp Asp Lys Leu Leu Gly Asn Cys Leu Pro Thr
            420                 425                 430
Val Leu Ser Ser Gly Ser Thr Ile His Ser Ile Ala Leu Gly Ser Ser
        435                 440                 445
Ala Ala Pro Asn Leu Glu Glu Leu Ser Arg Leu Thr Gly Gly Leu Lys
    450                 455                 460
Phe Phe Val Pro Asp Ile Ser Asn Ser Asn Ser Met Ile Asp Ala Phe

465                 470                 475                 480
Ser Arg Ile Ser Ser Gly Thr Gly Asp Ile Phe Gln Gln His Ile Gln
                485                 490                 495
Leu Glu Ser Thr Gly Glu Asn Val Lys Pro His His Gln Leu Lys Asn
            500                 505                 510
Thr Val Thr Val Asp Asn Thr Val Gly Asn Asp Thr Met Phe Leu Val
        515                 520                 525
Thr Trp Gln Ala Ser Gly Pro Pro Glu Ile Ile Leu Phe Asp Pro Asp
    530                 535                 540
Gly Arg Lys Tyr Tyr Thr Asn Asn Phe Ile Thr Asn Leu Thr Phe Arg
545                 550                 555                 560
Thr Ala Ser Leu Trp Ile Pro Gly Thr Ala Lys Pro Gly His Trp Thr
                565                 570                 575
Tyr Thr Leu Met Cys Phe His His Ala Lys Leu Leu Thr Trp Lys Leu
            580                 585                 590

<210> 170
<211> 791
<212> PRT
<213> Homo sapiens

<400> 170
Met Thr Gln Arg Ser Ile Ala Gly Pro Ile Cys Asn Leu Lys Phe Val
 1               5                  10                  15
Thr Leu Leu Val Ala Leu Ser Ser Glu Leu Pro Phe Leu Gly Ala Gly
            20                  25                  30
Val Gln Leu Gln Asp Asn Gly Tyr Asn Gly Leu Leu Ile Ala Ile Asn
        35                  40                  45
Pro Gln Val Pro Glu Asn Gln Asn Leu Ile Ser Asn Ile Lys Glu Met
    50                  55                  60
Ile Thr Glu Ala Ser Phe Tyr Leu Phe Asn Ala Thr Lys Arg Arg Val
65                  70                  75                  80
Phe Phe Arg Asn Ile Lys Ile Leu Ile Pro Ala Thr Trp Lys Ala Asn
                85                  90                  95
Asn Asn Ser Lys Ile Lys Gln Glu Ser Tyr Glu Lys Ala Asn Val Ile
            100                 105                 110
Val Thr Asp Trp Tyr Gly Ala His Gly Asp Asp Pro Tyr Thr Leu Gln
        115                 120                 125
Tyr Arg Gly Cys Gly Lys Glu Gly Lys Tyr Ile His Phe Thr Pro Asn
    130                 135                 140
Phe Leu Leu Asn Asp Asn Leu Thr Ala Gly Tyr Gly Ser Arg Gly Arg
145                 150                 155                 160
Val Phe Val His Glu Trp Ala His Leu Arg Trp Gly Val Phe Asp Glu
                165                 170                 175
Tyr Asn Asn Asp Lys Pro Phe Tyr Ile Asn Gly Gln Asn Gln Ile Lys
            180                 185                 190
Val Thr Arg Cys Ser Ser Asp Ile Thr Gly Ile Phe Val Cys Glu Lys
        195                 200                 205
Gly Pro Cys Pro Gln Glu Asn Cys Ile Ile Ser Lys Leu Phe Lys Glu
    210                 215                 220
Gly Cys Thr Phe Ile Tyr Asn Ser Thr Gln Asn Ala Thr Ala Ser Ile
225                 230                 235                 240
Met Phe Met Gln Ser Leu Ser Ser Val Val Glu Phe Cys Asn Ala Ser
                245                 250                 255
Thr His Asn Gln Glu Ala Pro Asn Leu Gln Asn Gln Met Cys Ser Leu
            260                 265                 270
Arg Ser Ala Trp Asp Val Ile Thr Asp Ser Ala Asp Phe His His Ser

        275                 280                 285
Phe Pro Met Asn Gly Thr Glu Leu Pro Pro Pro Pro Thr Phe Ser Leu
    290                 295                 300
Val Glu Ala Gly Asp Lys Val Val Cys Leu Val Leu Asp Val Ser Ser
305                 310                 315                 320
Lys Met Ala Glu Ala Asp Arg Leu Leu Gln Leu Gln Gln Ala Ala Glu
                325                 330                 335
Phe Tyr Leu Met Gln Ile Val Glu Ile His Thr Phe Val Gly Ile Ala
            340                 345                 350
Ser Phe Asp Ser Lys Gly Glu Ile Arg Ala Gln Leu His Gln Ile Asn
        355                 360                 365
Ser Asn Asp Asp Arg Lys Leu Leu Val Ser Tyr Leu Pro Thr Thr Val
    370                 375                 380
Ser Ala Lys Thr Asp Ile Ser Ile Cys Ser Gly Leu Lys Lys Gly Phe
385                 390                 395                 400
Glu Val Val Glu Lys Leu Asn Gly Lys Ala Tyr Gly Ser Val Met Ile
                405                 410                 415
Leu Val Thr Ser Gly Asp Asp Lys Leu Leu Gly Asn Cys Leu Pro Thr
            420                 425                 430
Val Leu Ser Ser Gly Ser Thr Ile His Ser Ile Ala Leu Gly Ser Ser
        435                 440                 445
Ala Ala Pro Asn Leu Glu Glu Leu Ser Arg Leu Thr Gly Gly Leu Lys
    450                 455                 460
Phe Phe Val Pro Asp Ile Ser Asn Ser Asn Ser Met Ile Asp Ala Phe
465                 470                 475                 480
Ser Arg Ile Ser Ser Gly Thr Gly Asp Ile Phe Gln Gln His Ile Gln
                485                 490                 495
Leu Glu Ser Thr Gly Glu Asn Val Lys Pro His His Gln Leu Lys Asn
            500                 505                 510
Thr Val Thr Val Asp Asn Thr Val Gly Asn Asp Thr Met Phe Leu Val
        515                 520                 525
Thr Trp Gln Ala Ser Gly Pro Pro Glu Ile Ile Leu Phe Asp Pro Asp
    530                 535                 540
Gly Arg Lys Tyr Tyr Thr Asn Asn Phe Ile Thr Asn Leu Thr Phe Arg
545                 550                 555                 560
Thr Ala Ser Leu Trp Ile Pro Gly Thr Ala Lys Pro Gly His Trp Thr
                565                 570                 575
Tyr Thr Leu Asn Asn Thr His His Ser Leu Gln Ala Leu Lys Val Thr
            580                 585                 590
Val Thr Ser Arg Ala Ser Asn Ser Ala Val Pro Pro Ala Thr Val Glu
        595                 600                 605
Ala Phe Val Glu Arg Asp Ser Leu His Phe Pro His Pro Val Met Ile
    610                 615                 620
Tyr Ala Asn Val Lys Gln Gly Phe Tyr Pro Ile Leu Asn Ala Thr Val
625                 630                 635                 640
Thr Ala Thr Val Glu Pro Glu Thr Gly Asp Pro Val Thr Leu Arg Leu
                645                 650                 655
Leu Asp Asp Gly Ala Gly Ala Asp Val Ile Lys Asn Asp Gly Ile Tyr
            660                 665                 670
Ser Arg Tyr Phe Phe Ser Phe Ala Ala Asn Gly Arg Tyr Ser Leu Lys
        675                 680                 685
Val His Val Asn His Ser Pro Ser Ile Ser Thr Pro Ala His Ser Ile
    690                 695                 700
Pro Gly Ser His Ala Met Tyr Val Pro Gly Tyr Thr Ala Asn Gly Asn
705                 710                 715                 720
Ile Gln Met Asn Ala Pro Arg Lys Ser Val Gly Arg Asn Glu Glu Glu
                725                 730                 735
Arg Lys Trp Gly Phe Ser Arg Val Ser Ser Gly Gly Ser Phe Ser Val

740                 745                 750
Leu Gly Val Pro Ala Gly Pro His Pro Asp Val Phe Pro Pro Cys Lys
        755                 760                 765
Ile Ile Asp Leu Glu Ala Val Asn Arg Arg Gly Ile Asp Pro Ile Leu
    770                 775                 780
Asp Ser Thr Trp Arg Arg Leu
785                 790

<210> 171
<211> 1491
<212> DNA
<213> Homo sapiens

<400> 171
cctcctgcca gccaagtgaa gacatgctta cttccccttc accttccttc atgatgtggg 60
aagagtgctg caacccagcc ctagccaacg ccgcatgaga gggagtgtgc cgagggcttc 120
tgagaaggtt tctctcacat ctagaaagaa gcgcttaaga tgtggcagcc cctcttcttc 180
aagtggctct tgtcctgttg ccctgggagt tctcaaattg ctgcagcagc ctccacccag 240
cctgaggatg acatcaatac acagaggaag aagagtcagg aaaagatgag agaagttaca 300
gactctcctg ggcgaccccg agagcttacc attcctcaga cttcttcaca tggtgctaac 360
agatttgttc ctaaaagtaa agctctagag gccgtcaaat tggcaataga agccgggttc 420
caccatattg attctgcaca tgtttacaat aatgaggagc aggttggact ggccatccga 480
agcaagattg cagatggcag tgtgaagaga gaagacatat tctacacttc aaagctttgg 540
agcaattccc atcgaccaga gttggtccga ccagccttgg aaaggtcact gaaaaatctt 600
caattggact atgttgacct ctatcttatt cattttccag tgtctgtaaa gccaggtgag 660
gaagtgatcc caaaagatga aaatggaaaa atactatttg acacagtgga tctctgtgcc 720
acatggaggg ccatggagaa gtgtaaagat gcaggattgg ccaagtccat cggggtgtcc 780
aacttcaacc acaggctgct ggagatgatc ctcaacaagc cagggctcaa gtacaagcct 840
gtctgcaacc aggtggaatg tcatccttac ttcaaccaga gaaaactgct ggatttctgc 900
aagtcaaaag acattgttct ggttgcctat agtgctctgg gatcccatcg agaagaacca 960
tgggtggacc cgaactcccc ggtgctcttg gaggacccag tcctttgtgc cttggcaaaa 1020
aagcacaagc gaaccccagc cctgattgcc ctgcgctacc agctgcagcg tggggttgtg 1080
gtcctggcca agagctacaa tgagcagcgc atcagacaga acgtgcaggt gtttgaattc 1140
cagttgactt cagaggagat gaaagccata gatggcctaa acagaaatgt gcgatatttg 1200
acccttgata tttttgctgg cccccctaat tatccatttt ctgatgaata ttaacatgga 1260
gggcattgca tgaggtctgc cagaaggccc tgcgtgtgga tggtgacaca gaggatggct 1320
ctatgctggt gactggacac atcgcctctg gttaaatctc tcctgcttgg cgacttcagt 1380
aagctacagc taagcccatc ggccggaaaa gaaagacaat aatttgtttt ttcattttga 1440
aaaaattaaa tgctctctcc taaagattct tcacctaaaa aaaaaaaaaa a 1491

<210> 172
<211> 364
<212> PRT
<213> Homo sapiens

<400> 172
Met Trp Gln Pro Leu Phe Phe Lys Trp Leu Leu Ser Cys Cys Pro Gly
1               5                   10                  15
Ser Ser Gln Ile Ala Ala Ala Ala Ser Thr Gln Pro Glu Asp Asp Ile
            20                  25                  30
Asn Thr Gln Arg Lys Lys Ser Gln Glu Lys Met Arg Glu Val Thr Asp
        35                  40                  45
Ser Pro Gly Arg Pro Arg Glu Leu Thr Ile Pro Gln Thr Ser Ser His
    50                  55                  60
Gly Ala Asn Arg Phe Val Pro Lys Ser Lys Ala Leu Glu Ala Val Lys
65                  70                  75                  80
Leu Ala Ile Glu Ala Gly Phe His His Ile Asp Ser Ala His Val Tyr

85                  90                  95
Asn Asn Glu Glu Gln Val Gly Leu Ala Ile Arg Ser Lys Ile Ala Asp
            100                 105                 110
Gly Ser Val Lys Arg Glu Asp Ile Phe Tyr Thr Ser Lys Leu Trp Ser
        115                 120                 125
Asn Ser His Arg Pro Glu Leu Val Arg Pro Ala Leu Glu Arg Ser Leu
    130                 135                 140
Lys Asn Leu Gln Leu Asp Tyr Val Asp Leu Tyr Leu Ile His Phe Pro
145                 150                 155                 160
Val Ser Val Lys Pro Gly Glu Glu Val Ile Pro Lys Asp Glu Asn Gly
                165                 170                 175
Lys Ile Leu Phe Asp Thr Val Asp Leu Cys Ala Thr Trp Glu Ala Met
            180                 185                 190
Glu Lys Cys Lys Asp Ala Gly Leu Ala Lys Ser Ile Gly Val Ser Asn
        195                 200                 205
Phe Asn His Arg Leu Leu Glu Met Ile Leu Asn Lys Pro Gly Leu Lys
    210                 215                 220
Tyr Lys Pro Val Cys Asn Gln Val Glu Cys His Pro Tyr Phe Asn Gln
225                 230                 235                 240
Arg Lys Leu Leu Asp Phe Cys Lys Ser Lys Asp Ile Val Leu Val Ala
                245                 250                 255
Tyr Ser Ala Leu Gly Ser His Arg Glu Glu Pro Trp Val Asp Pro Asn
            260                 265                 270
Ser Pro Val Leu Leu Glu Asp Pro Val Leu Cys Ala Leu Ala Lys Lys
        275                 280                 285
His Lys Arg Thr Pro Ala Leu Ile Ala Leu Arg Tyr Gln Leu Gln Arg
    290                 295                 300
Gly Val Val Val Leu Ala Lys Ser Tyr Asn Glu Gln Arg Ile Arg Gln
305                 310                 315                 320
Asn Val Gln Val Phe Glu Phe Gln Leu Thr Ser Glu Glu Met Lys Ala
                325                 330                 335
Ile Asp Gly Leu Asn Arg Asn Val Arg Tyr Leu Thr Leu Asp Ile Phe
            340                 345                 350
Ala Gly Pro Pro Asn Tyr Pro Phe Ser Asp Glu Tyr
        355                 360

<210> 173
<211> 1988
<212> DNA
<213> Homo sapiens

<400> 173
cgggagccgc ctccccgcgg cctcttcgct tttgtggcgg cgcccgcgct cgcaggccac 60
tctctgctgt cgcccgtccc gcgcgctcct ccgacccgct ccgctccgct ccgctcggcc 120
ccgcgccgcc cgtcaacatg atccgctgcg gcctggcctg cgagcgctgc cgctggatcc 180
tgcccctgct cctactcagc gccatcgcct tcgacatcat cgcgctggcc ggccgcggct 240
ggttgcagtc tagcgaccac ggccagacgt cctcgctgtg gtggaaatgc tcccaagagg 300
gcggcggcag cgggtcctac gaggagggct gtcagagcct catggagtac gcgtggggta 360
gagcagcggc tgccatgctc ttctgtggct tcatcatcct ggtgatctgt ttcatcctct 420
ccttcttcgc cctctgtgga ccccagatgc ttgtcttcct gagagtgatt ggaggtctcc 480
ttgccttggc tgctgtgttc cagatcatct ccctggtaat ttaccccgtg aagtacaccc 540
agaccttcac ccttcatgcc aaccctgctg tcacttacat ctataactgg gcctacggct 600
ttgggtgggc agccacgatt atcctgatcg gctgtgcctt cttcttctgc tgcctcccca 660
actacgaaga tgaccttctg ggcaatgcca agcccaggta cttctacaca tctgcctaac 720
ttgggaatga atgtgggaga aaatcgctgc tgctgagatg gactccagaa gaagaaactg 780
tttctccagg cgactttgaa cccatttttt ggcagtgttc atattattaa actagtcaaa 840
aatgctaaaa taatttggga gaaaatattt tttaagtagt gttatagttt catgtttatc 900

ttttattatg ttttgtgaag ttgtgtcttt tcactaatta cctatactat gccaatattt 960
ccttatatct atccataaca tttatactac atttgtaaga gaatatgcac gtgaaactta 1020
acactttata aggtaaaaat gaggtttcca agatttaata atctgatcaa gttcttgtta 1080
tttccaaata gaatggactt ggtctgttaa gggctaagga gaagaggaag ataaggttaa 1140
aagttgttaa tgaccaaaca ttctaaaaga aatgcaaaaa aaaagtttat tttcaagcct 1200
tcgaactatt taaggaaagc aaaatcattt cctaaatgca tatcatttgt gagaatttct 1260
cattaatatc ctgaatcatt catttcagct aaggcttcat gttgactcga tatgtcatct 1320
aggaaagtac tatttcatgg tccaaacctg ttgccatagt tggtaaggct ttcctttaag 1380
tgtgaaatat ttagatgaaa ttttctcttt taaagttctt tatagggtta gggtgtggga 1440
aaatgctata ttaataaatc tgtagtgttt tgtgtttata tgttcagaac cagagtagac 1500
tggattgaaa gatggactgg gtctaattta tcatgactga tagatctggt taagttgtgt 1560
agtaaagcat taggagggtc attcytgtca caaaagtgcc actaaaacag cctcaggaga 1620
ataaatgact tgcttttcta aatctcaggt ttatctgggc tctatcatat agacaggctt 1680
ctgatagttt gcarctgtaa gcagaaacct acatatagtt aaaatcctgg tctttcttgg 1740
taaacagatt ttaaatgtct gatataaaac atgccacagg agaattcggg gatttgagtt 1800
tctctgaata gcatatatat gatgcatcgg ataggtcatt atgatttttt accatttcga 1860
cttacataat gaaaaccaat tcattttaaa tatcagatta ttattttgta agttgtggaa 1920
aaagctaatt gtagttttca ttatgaagtt ttcccaataa accaggtatt ctaaaaaaaa 1980
aaaaaaaa 1988

<210> 174
<211> 238
<212> PRT
<213> Homo sapiens

<400> 174
Gly Ala Ala Ser Pro Arg Pro Leu Arg Phe Cys Gly Gly Ala Arg Ala
1               5                   10                  15
Arg Arg Pro Leu Ser Ala Val Ala Arg Pro Ala Arg Ser Ser Asp Pro
            20                  25                  30
Leu Arg Ser Ala Pro Leu Gly Pro Ala Pro Pro Val Asn Met Ile Arg
        35                  40                  45
Cys Gly Leu Ala Cys Glu Arg Cys Arg Trp Ile Leu Pro Leu Leu Leu
    50                  55                  60
Leu Ser Ala Ile Ala Phe Asp Ile Ile Ala Leu Ala Gly Arg Gly Trp
65                  70                  75                  80
Leu Gln Ser Ser Asp His Gly Gln Thr Ser Ser Leu Trp Trp Lys Cys
                85                  90                  95
Ser Gln Glu Gly Gly Gly Ser Gly Ser Tyr Glu Glu Gly Cys Gln Ser
            100                 105                 110
Leu Met Glu Tyr Ala Trp Gly Arg Ala Ala Ala Ala Met Leu Phe Cys
        115                 120                 125
Gly Phe Ile Ile Leu Val Ile Cys Phe Ile Leu Ser Phe Phe Ala Leu
    130                 135                 140
Cys Gly Pro Gln Met Leu Val Phe Leu Arg Val Ile Gly Gly Leu Leu
145                 150                 155                 160
Ala Leu Ala Ala Val Phe Gln Ile Ile Ser Leu Val Ile Tyr Pro Val
                165                 170                 175
Lys Tyr Thr Gln Thr Phe Thr Leu His Ala Asn Pro Ala Val Thr Tyr
            180                 185                 190
Ile Tyr Asn Trp Ala Tyr Gly Phe Gly Trp Ala Ala Thr Ile Ile Leu
        195                 200                 205
Ile Gly Cys Ala Phe Phe Phe Cys Cys Leu Pro Asn Tyr Glu Asp Asp
    210                 215                 220
Leu Leu Gly Asn Ala Lys Pro Arg Tyr Phe Tyr Thr Ser Ala
225                 230                 235

<210> 175
<211> 4181
<212> DNA
<213> Homo sapiens

<220>
<221> misc_feature
<222> 3347, 3502, 3506, 3520, 3538, 3549, 3646, 3940, 3968, 3974,
4036, 4056, 4062, 4080, 4088, 4115
<223> n = A,T,C or G

<400> 175
ggtggatgcg tttgggttgt agctaggctt tttcttttct ttctctttta aaacacatct 60
agacaaggaa aaaacaagcc tcggatctga tttttcactc ctcgttcttg tgcttggttc 120
ttactgtgtt tgtgtatttt aaaggcgaga agacgagggg aacaaaacca gctggatcca 180
tccatcaccg tgggtggttt taatttttcg tttttctcg ttattttttt ttaaacaacc 240
actcttcaca atgaacaaac tgtatatcgg aaacctcagc gagaacgccg ccccctcgga 300
cctagaaagt atcttcaagg acgccaagat cccggtgtcg ggacccttcc tggtgaagac 360
tggctacgcg ttcgtggact gcccggacga gagctgggcc ctcaaggcca tcgaggcgct 420
ttcaggtaaa atagaactgc acgggaaacc catagaagtt gagcactcgg tcccaaaaag 480
gcaaaggatt cggaaacttc agatacgaaa tatcccgcct catttacagt gggaggtgct 540
ggatagttta ctagtccagt atggagtggt ggagagctgt gagcaagtga acactgactc 600
ggaaactgca gttgtaaatg taacctattc cagtaaggac caagctagac aagcactaga 660
caaactgaat ggatttcagt tagagaattt caccttgaaa gtagcctata tccctgatga 720
aatggccgcc cagcaaaacc ccttgcagca gccccgaggt cgccgggggc ttgggcagag 780
gggctcctca aggcaggggt ctccaggatc cgtatccaag cagaaaccat gtgatttgcc 840
tctgcgcctg ctggttccca cccaatttgt tggagccatc ataggaaaag aaggtgccac 900
cattcggaac atcaccaaac agacccagtc taaaatcgat gtccaccgta aagaaaatgc 960
gggggctgct gagaagtcga ttactatcct ctctactcct gaaggcacct ctgcggcttg 1020
taagtctatt ctggagatta tgcataagga agctcaagat ataaaattca cagaagagat 1080
ccccttgaag attttagctc ataataactt tgttggacgt cttattggta aagaaggaag 1140
aaatcttaaa aaaattgagc aagacacaga cactaaaatc acgatatctc cattgcagga 1200
attgacgctg tataatccag aacgcactat tacagttaaa ggcaatgttg agacatgtgc 1260
caaagctgag gaggagatca tgaagaaaat cagggagtct tatgaaaatg atattgcttc 1320
tatgaatctt caagcacatt taattcctgg attaaatctg aacgccttgg gtctgttccc 1380
acccacttca gggatgccac ctcccacctc agggccccct tcagccatga ctcctcccta 1440
cccgcagttt gagcaatcag aaacggagac tgttcatcag tttatcccag ctctatcagt 1500
cggtgccatc atcggcaagc agggccagca catcaagcag ctttctcgct ttgctggagc 1560
ttcaattaag attgctccag cggaagcacc agatgctaaa gtgaggatgg tgattatcac 1620
tggaccacca gaggctcagt tcaaggctca gggaagaatt tatggaaaaa ttaaagaaga 1680
aaactttgtt agtcctaaag aagaggtgaa acttgaagct catatcagag tgccatcctt 1740
tgctgctggc agagttattg gaaaaggagg caaaacggtg aatgaacttc agaatttgtc 1800
aagtgcagaa gttgttgtcc ctcgtgacca gacacctgat gagaatgacc aagtggttgt 1860
caaaataact ggtcacttct atgcttgcca ggttgcccag agaaaaattc aggaaattct 1920
gactcaggta aagcagcacc aacaacagaa ggctctgcaa agtggaccac ctcagtcaag 1980
acggaagtaa aggctcagga aacagcccac cacagaggca gatgccaaac caaagacaga 2040
ttgcttaacc aacagatggg cgctgacccc ctatccagaa tcacatgcac aagtttttac 2100
ctagccagtt gtttctgagg accaggcaac ttttgaactc ctgtctctgt gagaatgtat 2160
actttatgct ctctgaaatg tatgacaccc agctttaaaa caaacaaaca aacaaacaaa 2220
aaaagggtgg gggagggagg gaaagagaag agctctgcac ttccctttgt tgtagtctca 2280
cagtataaca gatattctaa ttcttcttaa tattccccca taatgccaga aattggctta 2340
atgatgcttt cactaaattc atcaaataga ttgctcctaa atccaattgt taaaattgga 2400
tcagaataat tatcacagga acttaaatgt taagccatta gcatagaaaa actgttctca 2460
gttttatttt tacctaacac taacatgagt aacctaaggg aagtgctgaa tggtgttggc 2520
aggggtatta aacgtgcatt tttactcaac tacctcaggt attcagtaat acaatgaaaa 2580
gcaaaattgt tccttttttt tgaaaatttt atatacttta taatgataga agtccaaccg 2640
tttttaaaa aataaattta aaatttaaca gcaatcagct aacaggcaaa ttaagatttt 2700
tacttctggc tggtgacagt aaagctggaa aattaatttc agggtttttt gaggcttttg 2760

acacagttat tagttaaatc aaatgttcaa aaatacggag cagtgcctag tatctggaga 2820
gcagcactac catttattct ttcatttata gttgggaaag tttttgacgg tactaacaaa 2880
gtggtcgcag gagattttgg aacggctggt ttaaatggct tcaggagact tcagtttttt 2940
gtttagctac atgattgaat gcataataaa tgctttgtgc ttctgactat caatacctaa 3000
agaaagtgca tcagtgaaga gatgcaagac tttcaactga ctggcaaaaa gcaagcttta 3060
gcttgtctta taggatgctt agtttgccac tacacttcag accaatggga cagtcataga 3120
tggtgtgaca gtgtttaaac gcaacaaaag gctacatttc catggggcca gcactgtcat 3180
gagcctcact aagctatttt gaagattttt aagcactgat aaattaaaaa aaaaaaaaaa 3240
aaattagact ccaccttaag tagtaaagta taacaggatt tctgtatact gtgcaatcag 3300
ttctttgaaa aaaaagtcaa aagatagaga atacaagaaa agttttnggg atataatttg 3360
aatgactgtg aaaacatatg acctttgata acgaactcat ttgctcactc cttgacagca 3420
aagcccagta cgtacaattg tgttgggtgt gggtggtctc caaggccacg ctgctctctg 3480
aattgatttt ttgagttttg gnttgnaaga tgatcacagn catgttacac tgatcttnaa 3540
ggacatatnt tataaccctt taaaaaaaaa atcccctgcc tcattcttat ttcgagatga 3600
atttcgatac agactagatg tctttctgaa gatcaattag acattntgaa aatgatttaa 3660
agtgttttcc ttaatgttct ctgaaaacaa gtttcttttg tagttttaac caaaaaagtg 3720
ccctttttgt cactggtttc tcctagcatt catgattttt ttttcacaca atgaattaaa 3780
attgctaaaa tcatggactg gctttctggt tggatttcag gtaagatgtg tttaaggcca 3840
gagcttttct cagtatttga tttttttccc caatatttga ttttttaaaa atatacacat 3900
aggagctgca tttaaaacct gctggtttaa attctgtcan atttcacttc tagcctttta 3960
gtatggcnaa tcanaattta cttttactta agcatttgta atttggagta tctggtacta 4020
gctaagaaat aattcnataa ttgagttttg tactcnccaa anatgggtca ttcctcatgn 4080
ataatgtncc cccaatgcag cttcattttc caganacctt gacgcaggat aaattttttc 4140
atcatttagg tccccaaaaa aaaaaaaaaa aaaaaaaaaa a 4181

<210> 176
<211> 579
<212> PRT
<213> Homo sapiens

<400> 176
Met Asn Lys Leu Tyr Ile Gly Asn Leu Ser Glu Asn Ala Ala Pro Ser
1               5                   10                  15
Asp Leu Glu Ser Ile Phe Lys Asp Ala Lys Ile Pro Val Ser Gly Pro
            20                  25                  30
Phe Leu Val Lys Thr Gly Tyr Ala Phe Val Asp Cys Pro Asp Glu Ser
        35                  40                  45
Trp Ala Leu Lys Ala Ile Glu Ala Leu Ser Gly Lys Ile Glu Leu His
    50                  55                  60
Gly Lys Pro Ile Glu Val Glu His Ser Val Pro Lys Arg Gln Arg Ile
65                  70                  75                  80
Arg Lys Leu Gln Ile Arg Asn Ile Pro Pro His Leu Gln Trp Glu Val
                85                  90                  95
Leu Asp Ser Leu Leu Val Gln Tyr Gly Val Val Glu Ser Cys Glu Gln
            100                 105                 110
Val Asn Thr Asp Ser Glu Thr Ala Val Val Asn Val Thr Tyr Ser Ser
        115                 120                 125
Lys Asp Gln Ala Arg Gln Ala Leu Asp Lys Leu Asn Gly Phe Gln Leu
    130                 135                 140
Glu Asn Phe Thr Leu Lys Val Ala Tyr Ile Pro Asp Glu Met Ala Ala
145                 150                 155                 160
Gln Gln Asn Pro Leu Gln Gln Pro Arg Gly Arg Arg Gly Leu Gly Gln
                165                 170                 175
Arg Gly Ser Ser Arg Gln Gly Ser Pro Gly Ser Val Ser Lys Gln Lys
            180                 185                 190
Pro Cys Asp Leu Pro Leu Arg Leu Leu Val Pro Thr Gln Phe Val Gly
        195                 200                 205
Ala Ile Ile Gly Lys Glu Gly Ala Thr Ile Arg Asn Ile Thr Lys Gln

210                 215                 220
Thr Gln Ser Lys Ile Asp Val His Arg Lys Glu Asn Ala Gly Ala Ala
225                 230                 235                 240
Glu Lys Ser Ile Thr Ile Leu Ser Thr Pro Glu Gly Thr Ser Ala Ala
                245                 250                 255
Cys Lys Ser Ile Leu Glu Ile Met His Lys Glu Ala Gln Asp Ile Lys
            260                 265                 270
Phe Thr Glu Glu Ile Pro Leu Lys Ile Leu Ala His Asn Asn Phe Val
        275                 280                 285
Gly Arg Leu Ile Gly Lys Glu Gly Arg Asn Leu Lys Lys Ile Glu Gln
    290                 295                 300
Asp Thr Asp Thr Lys Ile Thr Ile Ser Pro Leu Gln Glu Leu Thr Leu
305                 310                 315                 320
Tyr Asn Pro Glu Arg Thr Ile Thr Val Lys Gly Asn Val Glu Thr Cys
                325                 330                 335
Ala Lys Ala Glu Glu Glu Ile Met Lys Lys Ile Arg Glu Ser Tyr Glu
            340                 345                 350
Asn Asp Ile Ala Ser Met Asn Leu Gln Ala His Leu Ile Pro Gly Leu
        355                 360                 365
Asn Leu Asn Ala Leu Gly Leu Phe Pro Pro Thr Ser Gly Met Pro Pro
    370                 375                 380
Pro Thr Ser Gly Pro Pro Ser Ala Met Thr Pro Pro Tyr Pro Gln Phe
385                 390                 395                 400
Glu Gln Ser Glu Thr Glu Thr Val His Gln Phe Ile Pro Ala Leu Ser
                405                 410                 415
Val Gly Ala Ile Ile Gly Lys Gln Gly Gln His Ile Lys Gln Leu Ser
            420                 425                 430
Arg Phe Ala Gly Ala Ser Ile Lys Ile Ala Pro Ala Glu Ala Pro Asp
        435                 440                 445
Ala Lys Val Arg Met Val Ile Ile Thr Gly Pro Pro Glu Ala Gln Phe
    450                 455                 460
Lys Ala Gln Gly Arg Ile Tyr Gly Lys Ile Lys Glu Glu Asn Phe Val
465                 470                 475                 480
Ser Pro Lys Glu Glu Val Lys Leu Glu Ala His Ile Arg Val Pro Ser
                485                 490                 495
Phe Ala Ala Gly Arg Val Ile Gly Lys Gly Gly Lys Thr Val Asn Glu
            500                 505                 510
Leu Gln Asn Leu Ser Ser Ala Glu Val Val Val Pro Arg Asp Gln Thr
        515                 520                 525
Pro Asp Glu Asn Asp Gln Val Val Val Lys Ile Thr Gly His Phe Tyr
    530                 535                 540
Ala Cys Gln Val Ala Gln Arg Lys Ile Gln Glu Ile Leu Thr Gln Val
545                 550                 555                 560
Lys Gln His Gln Gln Gln Lys Ala Leu Gln Ser Gly Pro Pro Gln Ser
                565                 570                 575
Arg Arg Lys

<210> 177
<211> 401
<212> DNA
<213> Homo sapiens

<400> 177
atgccccgta aatgtcttca gtgttcttca gggtagttgg gatctcaaaa gatttggttc 60
agatccaaac aaatacacat tctgtgtttt agctcagtgt tttctaaaaa aagaaactgc 120
cacacagcaa aaaattgttt actttgttgg acaaaccaaa tcagttctca aaaaatgacc 180

ggtgcttata aaaagttata aatatcgagt agctctaaaa caaaccacct gaccaagagg 240
gaagtgagct tgtgcttagt atttacattg gatgccagtt ttgtaatcac tgacttatgt 300
gcaaactggt gcagaaattc tataaactct ttgctgtttt tgatacctgc tttttgtttc 360
attttgtttt gttttgtaaa aatgataaaa cttcagaaaa t 401

<210> 178
<211> 561
<212> DNA
<213> Homo sapiens

<400> 178
acgcctttca agggtgtacg caaagcactc attgataccc ttttggatgg ctatgaaaca 60
gcccgctatg ggacaggggt ctttggccag aatgagtacc tacgctatca ggaggccctg 120
agtgagctgg ccactgcggt taaagcacga attgggagct ctcagcgaca tcaccagtca 180
gcagccaaag acctaactca gtcccctgag gtctccccaa caaccatcca ggtgacatac 240
ctcccctcca gtcagaagag taaacgtgcc aagcacttcc ttgaattgaa gagctttaag 300
gataactata acacattgga gagtactctg tgacggagct gaaggactct tgccgtagat 360
taagccagtc agttgcaatg tgcaagacag gctgcttgcc gggccgccct cggaacatct 420
ggcccagcag gcccagactg tatccatcca agttcccgtt gtatccagag ttcttagagc 480
ttgtgtctaa agggtaattc cccaaccctt ccttatgagc atttttagaa cattggctaa 540
gactattttc ccccagtagc g 561

<210> 179
<211> 521
<212> DNA
<213> Homo sapiens

<400> 179
cccaacgcgt ttgcaaatat tcccctggta gcctacttcc ttacccccga atattggtaa 60
gatcgagcaa tggcttcagg acatgggttc tcttctcctg tgatcattca agtgctcact 120
gcatgaagac tggcttgtct cagtgtttca acctcaccag ggctgtctct tggtccacac 180
ctcgctccct gttagtgccg tatgacagcc cccatcaaat gaccttggcc aagtcacggt 240
ttctctgtgg tcaaggttgg ttggctgatt ggtggaaagt agggtggacc aaaggaggcc 300
acgtgagcag tcagcaccag ttctgcacca gcagcgcctc cgtcctagtg ggtgttcctg 360
tttctcctgg ccctgggtgg gctagggcct gattcgggaa gatgcctttg cagggagggg 420
aggataagtg ggatctacca attgattctg gcaaaacaat ttctaagatt tttttgcttt 480
atgtgggaaa cagatctaaa tctcatttta tgctgtattt t 521

<210> 180
<211> 417
<212> DNA
<213> Homo sapiens

<400> 180
ggtggaattc gccgaagatg gcggaggtgc aggtcctggt gcttgatggt cgaggccatc 60
tcctgggccg cctggcggcc atcgtggcta aacaggtact gctgggccgg aaggtggtgg 120
tcgtacgctg tgaaggcatc aacatttctg gcaatttcta cagaaacaag ttgaagtacc 180
tggctttcct ccgcaagcgg atgaacacca acccttcccg aggcccctac cacttccggg 240
cccccagccg catcttctgg cggaccgtgc gaggtatgct gccccacaaa accaagcgag 300
gccaggccgc tctggaccgt ctcaaggtgt ttgacggcat cccaccgccc tacgacaaga 360
aaaagcggat ggtggttcct gctgccctca aggtcgtgcg tctgaagcct acaagaa 417

<210> 181
<211> 283
<212> DNA
<213> Homo sapiens

<220>

<221> misc_feature
<222> 35
<223> n = A,T,C or G

<400> 181
gatttcttct aaataggatg taaaacttct ttcanattac tcttcctcag tcctgcctgc 60
caagaactca agtgtaactg tgataaaata acctttccca ggtatattgg caggtatgtg 120
tgtaatctca gaatacacag gtgacataga tatgatatga caactggtaa tggtggattc 180
atttacattg tttacacttc tatgaccagg ccttaaggga aggtcagttt tttaaaaaac 240
caagtagtgt cttcctacct atctccagat acatgtcaaa aaa 283

<210> 182
<211> 401
<212> DNA
<213> Homo sapiens

<400> 182
atattcttgc tgcttatgca gctgacattg ttgccctccc taaagcaacc aagtagcctt 60
tatttcccac agtgaaagaa aacgctggcc tatcagttac attacaaaag gcagatttca 120
agaggattga gtaagtagtt ggatggcttt cataaaaaca agaattcaag aagaggattc 180
atgctttaag aaacatttgt tatacattcc tcacaaatta tacctgggat aaaaactatg 240
tagcaggcag tgtgttttcc ttccatgtct ctctgcacta cctgcagtgt gtcctctgag 300
gctgcaagtc tgtcctatct gaattcccag cagaagcact aagaagctcc accctatcac 360
ctagcagata aaactatggg gaaaacttaa atctgtgcat a 401

<210> 183
<211> 366
<212> DNA
<213> Homo sapiens

<220>
<221> misc_feature
<222> 325
<223> n = A,T,C or G

<400> 183
accgtgtcca agtttttaga acccttgtta gccagaccga ggtgtcctgg tcaccgtttc 60
accatcatgc tttgatgttc ccctgtcttt ctctcttctg ctctcaagag caaaggttaa 120
tttaaggaca aagatgaagt cactgtaaac taatctgtca ttgtttttac cttccttttc 180
tttttcagtg cagaaattaa aagtaagtat aaagcaccgt gattgggagt gtttttgcgt 240
gtgtcggaat cactggtaaa tgttggctga gaacaatccc tccccttgca cttgtgaaaa 300
cactttgagc gctttaagag attancctga gaaataatta aatatctttt ctcttcaaaa 360
aaaaaa 366

<210> 184
<211> 370
<212> DNA
<213> Homo sapiens

<400> 184
tcttacttca aaagaaaaat aaacataaaa aataagttgc tggttcctaa caggaaaaat 60
tttaataatt gtactgagag aaactgctta cgtacacatt gcagatcaaa tatttggagt 120
taaaatgtta gtctacatag atgggtgatt gtaactttat tgccattaaa agatttcaaa 180
ttgcattcat gcttctgtgt acacataatg aaaaatgggc aaataatgaa gatctctcct 240
tcagtctgct ctgtttaatt ctgctgtctg ctcttctcta atgctgcgtc cctaattgta 300
cacagtttag tgatatctag gagtataaag ttgtcgccca tcaataaaaa tcacaaagtt 360
ggtttaaaaa 370

<210> 185
<211> 107
<212> DNA
<213> Homo sapiens

<400> 185
ctcatattat tttccttttg agaaattgga aactctttct gttgctatta tattaataaa 60
gttggtgttt attttctggt agtcaccttc cccatttaaa aaaaaaa 107

<210> 186
<211> 309
<212> DNA
<213> Homo sapiens

<400> 186
gaaaggatgg ctctggttgc cacagagctg ggacttcatg ttcttctaga gagggccaca 60
agagggccac aggggtggcc gggagttgtc agctgatgcc tgctgagagg caggaattgt 120
gccagtgagt gacagtcatg agggagtgtc tcttcttggg gaggaaagaa ggtagagcct 180
ttctgtctga atgaaaggcc aaggctacag tacagggccc cgccccagcc agggtgttaa 240
tgcccacgta gtggaggcct ctggcagatc ctgcattcca aggtcactgg actgtacgtt 300
tttatggtt 309

<210> 187
<211> 477
<212> DNA
<213> Homo sapiens

<400> 187
ttcagtccta gcaagaagcg agaattctga gatcctccag aaagtcgagc agcacccacc 60
tccaacctcg ggccagtgtc ttcaggcttt actggggacc tgcgagctgg cctaatgtgg 120
tggcctgcaa gccaggccat ccctgggcgc cacagacgag ctccgagcca ggtcaggctt 180
cggaggccac aagctcagcc tcaggcccag gcactgattg tggcagaggg gccactaccc 240
aaggtctagc taggcccaag acctagttac ccagacagtg agaagcccct ggaaggcaga 300
aaagttggga gcatggcaga cagggaaggg aaacattttc agggaaaaga catgtatcac 360
atgtcttcag aagcaagtca ggtttcatgt aaccgagtgt cctcttgcgt gtccaaaagt 420
agcccagggc tgtagcacag gcttcacagt gattttgtgt tcagccgtga gtcacac 477

<210> 188
<211> 220
<212> DNA
<213> Homo sapiens

<400> 188
taaatatggt agatattaat attcctctta gatgaccagt gattccaatt gtcccaagtt 60
ttaaataagt accctgtgag tatgagataa attagtgaca atcagaacaa gtttcagtat 120
cagatgttca agaggaagtt gctattgcat tgattttaat atttgtacat aaacactgat 180
ttttttgagc attattttgt atttgttgta ctttaatacc 220

<210> 189
<211> 417
<212> DNA
<213> Homo sapiens

<220>
<221> misc_feature
<222> 76, 77
<223> n = A,T,C or G

<400> 189
accatcttga cagaggatac atgctcccaa aacgtttgtt accacactta aaaatcactg 60
ccatcattaa gcatcnnttt caaaattata gccattcatg atttactttt tccagatgac 120
tatcattatt ctagtccttt gaatttgtaa ggggaaaaaa aacaaaaaca aaaacttacg 180
atgcactttt ctccagcaca tcagatttca aattgaaaat taaagacatg ctatggtaat 240
gcacttgcta gtactacaca ctttgtacaa caaaaaacag aggcaagaaa caacggaaag 300
agaaaagcct tcctttgttg gcccttaaac tgagtcaaga tctgaaatgt agagatgatc 360
tctgacgata cctgtatgtt cttattgtgt aaataaaatt gctggtatga aatgaca 417

<210> 190
<211> 497
<212> DNA
<213> Homo sapiens

<400> 190
gcactgcggc gctctcccgt cccgcggtgg ttgctgctgc tgccgctgct gctgggcctg 60
aacgcaggag ctgtcattga ctggcccaca gaggagggca aggaagtatg ggattatgtg 120
acggtccgca aggatgccta catgttctgg tggctctatt atgccaccaa ctcctgcaag 180
aacttctcag aactgcccct ggtcatgtgg cttcagggcg gtccaggcgg ttctagcact 240
ggatttggaa actttgagga aattgggccc cttgacagtg atctcaaacc acggaaaacc 300
acctggctcc aggctgccag tctcctattt gtggataatc ccgtgggcac tgggttcagt 360
tatgtgaatg gtagtggtgc ctatgccaag gacctggcta tggtggcttc agacatgatg 420
gttctcctga agaccttctt cagttgccac aaagaattcc agacagttcc attctacatt 480
ttctcagagt cctatgg 497

<210> 191
<211> 175
<212> DNA
<213> Homo sapiens

<400> 191
atgttgaata ttttgcttat taactttgtt tattgtcttc tccctcgatt agaatattag 60
ctacttgagt acaaggattt gagcctgtta cattcactgc tgaattttag gctcctggaa 120
gatacccagc attcaataga gaccacacaa taaatatatg tcaaataaaa aaaaa 175

<210> 192
<211> 526
<212> DNA
<213> Homo sapiens

<400> 192
agtaaacatt attatttttt ttatatttgc aaaggaaaca tatctaatcc ttcctataga 60
aagaacagta ttgctgtaat tccttttctt ttcttcctca tttcctctgc cccttaaaag 120
attgaagaaa gagaaacttg tcaactcata tccacgttat ctagcaaagt acataagaat 180
ctatcactaa gtaatgtatc cttcagaatg tgttggttta ccagtgacac cccatattca 240
tcacaaaatt aaagcaagaa gtccatagta atttatttgc taatagtgga tttttaatgc 300
tcagagtttc tgaggtcaaa ttttatcttt tcacttacaa gctctatgat cttaaataat 360
ttacttaatg tattttggtg tattttcctc aaattaatat tggtgttcaa gactatatct 420
aattcctctg atcactttga gaaacaaact tttattaaat gtaaggcact tttctatgaa 480
ttttaaatat aaaaataaat attgttctga ttattactga aaaaaa 526

<210> 193
<211> 553
<212> DNA
<213> Homo sapiens

<220>
<221> misc_feature

<222> 290, 300, 411, 441
<223> n = A,T,C or G

<400> 193
tccattgtgg tggaattcgc tctctggtaa aggcgtgcag gtgttggccg cggcctctga 60
gctgggatga gccgtgctcc cggtggaagc aagggagccc agccggagcc atggccagta 120
cagtggtagc agttggactg accattgctg ctgcaggatt tgcaggccgt tacgttttgc 180
aagccatgaa gcatatggag cctcaagtaa aacaagtttt tcaaagccta ccaaaatctg 240
ccttcagtgg tggctattat agaggtgggt ttgaacccaa aatgacaaan cgggaagcan 300
cattaatact aggtgtaagc cctactgcca ataaagggaa aataagagat gctcatcgac 360
gaattatgct tttaaatcat cctgacaaag gaggatctcc ttatatagca nccaaaatca 420
atgaagctaa agatttacta naaggtcaag ctaaaaaatg aagtaaatgt atgatgaatt 480
ttaagttcgt attagtttat gtatatgagt actaagtttt tataataaaa tgcctcagag 540
ctacaatttt aaa 553

<210> 194
<211> 320
<212> DNA
<213> Homo sapiens

<400> 194
cccttcccaa tccatcagta aagaccccat ctgccttgtc catgccgttt cccaacaggg 60
atgtcacttg atatgagaat ctcaaatctc aatgccttat aagcattcct tcctgtgtcc 120
attaagactc tgataattgt ctcccctcca taggaatttc tcccaggaaa gaaatatatc 180
cccatctccg tttcatatca gaactaccgt ccccgatatt cccttcagag agattaaaga 240
ccagaaaaaa gtgagcctct tcatctgcac ctgtaatagt ttcagttcct attttcttcc 300
attgacccat atttatacct 320

<210> 195
<211> 320
<212> DNA
<213> Homo sapiens

<220>
<221> misc_feature
<222> 203, 218
<223> n = A,T,C or G

<400> 195
aagcatgacc tggggaaatg gtcagacctt gtattgtgtt tttggccttg aaagtagcaa 60
gtgaccagaa tctgccatgg caacaggctt taaaaaagac ccttaaaaag acactgtctc 120
aactgtggtg ttagcaccag ccagctctct gtacatttgc tagcttgtag ttttctaaga 180
ctgagtaaac ttcttatttt tanaaagggg aggctggntt gtaactttcc ttgtacttaa 240
ttgggtaaaa gtcttttcca caaaccacca tctattttgt gaactttgtt agtcatcttt 300
tatttggtaa attatgaact 320

<210> 196
<211> 357
<212> DNA
<213> Homo sapiens

<220>
<221> misc_feature
<222> 36
<223> n = A,T,C or G

<400> 196
atataaaata atacgaaact ttaaaaagca ttggantgtc agtatgttga atcagtagtt 60

tcactttaac tgtaaacaat ttcttaggac accatttggg ctagtttctg tgtaagtgta 120
aatactacaa aaacttattt atactgttct tatgtcattt gttatattca tagatttata 180
tgatgatatg acatctggct aaaaagaaat tattgcaaaa ctaaccacta tgtacttttt 240
tataaatact gtatggacaa aaaatggcat tttttatatt aaattgttta gctctggcaa 300
aaaaaaaaaa ttttaagagc tggtactaat aaaggattat tatgactgtt aaaaaaa 357

<210> 197
<211> 565
<212> DNA
<213> Homo sapiens

<220>
<221> misc_feature
<222> 27
<223> n = A,T,C or G

<400> 197
tcagctgagt accatcagga tatttanccc tttaagtgct gttttgggag tagaaaacta 60
aagcaacaat acttcctctt gacagctttg attggaatgg ggttattaga tcattcacct 120
tggtcctaca ctttttagga tgcttggtga acataacacc acttataatg aacatccctg 180
gttcctatat tttgggctat gtgggtagga attgttactt gttactgcag cagcagccct 240
agaaagtaag cccagggctt cagatctaag ttagtccaaa agctaaatga tttaaagtca 300
agttgtaatg ctaggcataa gcactctata atacattaaa ttataggccg agcaattagg 360
gaatgtttct gaaacattaa acttgtattt atgtcactaa aattctaaca caaacttaaa 420
aaatgtgtct catacatatg ctgtactagg cttcatcatg catttctaaa tttgtgtatg 480
atttgaatat atgaaagaat ttatacaaga gtgttattta aaattattaa aaataaatgt 540
atataatttg tacctattgt aaaaa 565

<210> 198
<211> 484
<212> DNA
<213> Homo sapiens

<400> 198
tatgtaagta ttggtgtctg ctttaaaaaa ggagacccag acttcacctg tcctttttaa 60
acatttgaga acagtgttac tctgagcagt tgggccacct tcaccttatc cgacagctga 120
ctgttggatg tgtccattgt cgccagtttg gctgttgccc ggacaggaca ggacctccat 180
tgggcgcagc agcaggtggc aggggtgtgg cttgaggtgg gtggcagcgt ctggtcctcc 240
tctctggtgc tttctgagag ggtctctaaa gcagagtgtg gttggcctgg gggaaggcag 300
agcacgtatt tctcccctct agtacctctg catttgtgag tgttccctct ggctttctga 360
agggcagcag actcttgagt atactgcaga ggacatgctt tatcagtagg tcctgagggc 420
tccaggggct caactgacca agtaacacag aagttggggt atgtggccta tttgggtcgg 480
aaac 484

<210> 199
<211> 429
<212> DNA
<213> Homo sapiens

<220>
<221> misc_feature
<222> 77, 88, 134, 151, 189, 227, 274, 319
<223> n = A,T,C or G

<400> 199
gcttatgttt tttgttttaa cttttgtttt ttaacattta gaatattaca ttttgtatta 60
tacagtacct ttctcanaca ttttgtanaa ttcatttcgg cagctcacta ggattttgct 120
gaacattaaa aagngtgata gcgatattag ngccaatcaa atggaaaaaa ggtagtctta 180

ataaacaana cacaacgttt ttatacaaca tactttaaaa tattaanaaa actccttaat 240
attgtttcct attaagtatt attctttggg caanattttc tgatgctttt gattttctct 300
caatttagca tttgctttng gtttttttct ctatttagca ttctgttaag gcacaaaaac 360
tatgtactgt atgggaaatg ttgtaaatat taccttttcc acattttaaa cagacaactt 420
tgaatccaa 429

<210> 200
<211> 279
<212> DNA
<213> Homo sapiens

<400> 200
gctttttgga ggaattacag ggaagctcct ggaattgtac atggatatct ttatccctag 60
ggggaaatca aggagctggg cacccctaat tctttatgga agtgtttaaa actattttaa 120
ttttattaca agtattacta gagtagtggt tctactctaa gatttcaaaa gtgcatttaa 180
aatcatacat gttcccgcct gcaaatatat tgttattttg gtggagaaaa aaatagtata 240
ttctacataa aaaattaaag atattaacta agaaaaaaa 279

<210> 201
<211> 569
<212> DNA
<213> Homo sapiens

<400> 201
taggtcagta tttttagaaa ctcttaatag ctcatactct tgataccaaa agcagccctg 60
attgttaaag cacacacctg cacaagaagc agtgatggtt gcatttacat ttcctgggtg 120
cacaaaaaaa aattctcaaa aagcaaggac ttacgctttt tgcaaagcct ttgagaagtt 180
actggatcat aggaagctta taacaagaat ggaagattct taaataactc actttctttg 240
gtatccagta acagtagatg ttcaaaatat gtagctgatt aataccagca ttgtgaacgc 300
tgtacaacct tgtggttatt actaagcaag ttactactag cttctgaaaa gtagcttcat 360
aattaatgtt atttatacac tgccttccat gacttttact ttgccctaag ctaatctcca 420
aaatctgaaa tgctactcca atatcagaaa aaaaggggga ggtggaatta tatttcctgt 480
gattttaaga gtacagagaa tcatgcacat ctctgattag ttcatatatg tctagtgtgt 540
aataaaagtc aaagatgaac tctcaaaaa 569

<210> 202
<211> 501
<212> DNA
<213> Homo sapiens

<400> 202
attaataggc ttaataattg ttggcaagga tccttttgct ttctttggca tgcaagctcc 60
tagcatctgg cagtggggcc aagaaaataa ggtttatgca tgtatgatgg ttttcttctt 120
gagcaacatg attgagaacc agtgtatgtc aacaggtgca tttgagataa ctttaaatga 180
tgtacctgtg tggtctaagc tggaatctgg tcaccttcca tccatgcaac aacttgttca 240
aattcttgac aatgaaatga agctcaatgt gcatatggat tcaatcccac accatcgatc 300
atagcaccac ctatcagcac tgaaaactct tttgcattaa gggatcattg caagagcagc 360
gtgactgaca ttatgaaggc ctgtactgaa gacagcaagc tgttagtaca gaccagatgc 420
tttcttggca ggctcgttgt acctcttgga aaacctcaat gcaagatagt gtttcagtgc 480
tggcatattt tggaattctg c 501

<210> 203
<211> 261
<212> DNA
<213> Homo sapiens

<220>
<221> misc_feature

<222> 36, 96
<223> n = A,T,C or G

<400> 203
gacaagctcc tggtcttgag atgtcttctc gttaangaga tgggcctttt ggaggtaaag 60
gataaaatga atgagttctg tcatgattca ctattntata acttgcatga cctttactgt 120
gttagctctt tgaatgttct tgaaatttta gactttcttt gtaaacaaat gatatgtcct 180
tatcattgta taaaagctgt tatgtgcaac agtgtggaga ttccttgtct gatttaataa 240
aatacttaaa cactgaaaaa a 261

<210> 204
<211> 421
<212> DNA
<213> Homo sapiens

<400> 204
agcatctttt ctacaacgtt aaaattgcag aagtagctta tcattaaaaa acaacaacaa 60
caacaataac aataaatcct aagtgtaaat cagttattct accccctacc aaggatatca 120
gcctgttttt tccctttttt ctcctgggaa taattgtggg cttcttccca aatttctaca 180
gcctctttcc tcttctcatg cttgagcttc cctgtttgca cgcatgcgtg tgcaggactg 240
gcttgtgtgc ttggactcgg ctccaggtgg aagcatgctt tcccttgtta ctgttggaga 300
aactcaaacc ttcaagccct aggtgtagcc attttgtcaa gtcatcaact gtatttttgt 360
actggcatta acaaaaaaag aagataaaat attgtaccat taaactttaa taaaacttta 420
a 421

<210> 205
<211> 460
<212> DNA
<213> Homo sapiens

<400> 205
tactctcaca atgaaggacc tggaatgaaa aatctgtgtc taaacaagtc ctctttagat 60
tttagtgcaa atccagagcc agcgtcggtt gcctcgagta attctttcat gggtaccttt 120
ggaaaagctc tcaggagacc tcacctagat gcctattcaa gctttggaca gccatcagat 180
tgtcagccaa gagcctttta tttgaaagct cattcttccc cagacttgga ctctgggtca 240
gaggaagatg ggaaagaaag gacagatttt caggaagaaa atcacatttg tacctttaaa 300
cagactttag aaaactacag gactccaaat tttcagtctt atgacttgga cacatagact 360
gaatgagacc aaaggaaaag cttaacatac tacctcaagg tgaactttta tttaaaagag 420
agagaatctt atgtttttta aatggagtta tgaattttaa 460

<210> 206
<211> 481
<212> DNA
<213> Homo sapiens

<400> 206
tgtggtggaa ttcgggacgc ccccagaccc tgactttttc ctgcgtgggc cgtctcctcc 60
tgcggaagca gtgacctctg acccctggtg accttcgctt tgagtgcctt ttgaacgctg 120
gtcccgcggg acttggtttt ctcaagctct gtctgtccaa agacgctccg gtcgaggtcc 180
cgcctgccct gggtggatac ttgaacccca gacgcccctc tgtgctgctg tgtccggagg 240
cggccttccc atctgcctgc ccacccggag ctctttccgc cggcgcaggg tcccaagccc 300
acctcccgcc ctcagtcctg cggtgtgcgt ctgggcacgt cctgcacaca caatgcaagt 360
cctggcctcc gcgcccgccc gcccacgcga gccgtacccg ccgccaactc tgttatttat 420
ggtgtgaccc cctggaggtg ccctcggccc accggggcta tttattgttt aatttatttg 480
t 481

<210> 207
<211> 605

<212> DNA
<213> Homo sapiens

<400> 207
accctttttg gattcagggc tcctcacaat taaaatgagt gtaatgaaac aaggtgaaaa 60
tatagaagca tccctttgta tactgttttg ctacttacag tgtacttggc attgctttat 120
ctcactggat tctcacggta ggatttctga gatcttaatc taagctccaa agttgtctac 180
ttttttgatc ctagggtgct ccttttgttt tacagagcag ggtcacttga tttgctagct 240
ggtggcagaa ttggcaccat tacccaggtc tgactgacca ccagtcagag gcactttatt 300
tgtatcatga aatgatttga aatcattgta aagcagcgaa gtctgataat gaatgccagc 360
tttccttgtg ctttgataac aaagactcca aatattctgg agaacctgga taaaagtttg 420
aagggctaga ttgggatttg aagacaaaat tgtaggaaat cttacatttt tgcaataaca 480
aacattaatg aaagcaaaac attataaaag taattttaat tcaccacata cttatcaatt 540
tcttgatgct tccaaatgac atctaccaga tatggttttg tggacatctt tttctgttta 600
cataa 605

<210> 208
<211> 655
<212> DNA
<213> Homo sapiens

<400> 208
ggcgttgttc tggattcccg tcgtaactta aagggaaact ttcacaatgt ccggagccct 60
tgatgtcctg caaatgaagg aggaggatgt ccttaagttc cttgcagcag gaacccactt 120
aggtggcacc aatcttgact tccagatgga acagtacatc tataaaagga aaagtgatgg 180
catctatatc ataaatctca agaggacctg ggagaagctt ctgctggcag ctcgtgcaat 240
tgttgccatt gaaaaccctg ctgatgtcag tgttatatcc tccaggaata ctggccagag 300
ggctgtgctg aagtttgctg ctgccactgg agccactcca attgctggcc gcttcactcc 360
tggaaccttc actaaccaga tccaggcagc cttccgggag ccacggcttc ttgtggttac 420
tgaccccagg gctgaccacc agcctctcac ggaggcatct tatgttaacc tacctaccat 480
tgcgctgtgt aacacagatt ctcctctgcg ctatgtggac attgccatcc catgcaacaa 540
caagggagct cactcagtgg gtttgatgtg gtggatgctg gctcgggaag ttctgcgcat 600
gcgtggcacc atttcccgtg aacacccatg ggaggtcatg cctgatctgt acttc 655

<210> 209
<211> 621
<212> DNA
<213> Homo sapiens

<400> 209
catttagaac atggttatca tccaagacta ctctaccctg caacattgaa ctcccaagag 60
caaatccaca ttcctcttga gttctgcagc ttctgtgtaa atagggcagc tgtcgtctat 120
gccgtagaat cacatgatct gaggaccatt catggaagct gctaaatagc ctagtctggg 180
gagtcttcca taaagttttg catggagcaa acaaacagga ttaaactagg tttggttcct 240
tcagccctct aaaagcatag ggcttagcct gcaggcttcc ttgggctttc tctgtgtgtg 300
tagttttgta aacactatag catctgttaa gatccagtgt ccatggaaac cttcccacat 360
gccgtgactc tggactatat cagtttttgg aaagcagggt tcctctgcct gctaacaagc 420
ccacgtggac cagtctgaat gtctttcctt tacacctatg tttttaaata gtcaaacttc 480
aagaaacaat ctaaacaagt ttctgttgca tatgtgtttg tgaacttgta tttgtattta 540
gtaggcttct atattgcatt taacttgttt ttgtaactcc tgattcttcc ttttcggata 600
ctattgatga ataaagaaat t 621

<210> 210
<211> 533
<212> DNA
<213> Homo sapiens

<220>

<221> misc_feature
<222> 20, 21, 61
<223> n = A,T,C or G

<400> 210
cgccttgggg agccggcggn ngagtccggg acgtggagac ccggggtccc ggcagccggg 60
nggcccgcgg gcccagggtg gggatgcacc gccgcggggt gggagctggc gccatcgcca 120
agaagaaact tgcagaggcc aagtataagg agcgagggac ggtcttggct gaggaccagc 180
tagcccagat gtcaaagcag ttggacatgt tcaagaccaa cctggaggaa tttgccagca 240
aacacaagca ggagatccgg aagaatcctg agttccgtgt gcagttccag gacatgtgtg 300
caaccattgg cgtggatccg ctggcctctg gaaaaggatt ttggtctgag atgctgggcg 360
tgggggactt ctattacgaa ctaggtgtcc aaattatcga agtgtgcctg gcgctgaagc 420
atcggaatgg aggtctgata actttggagg aactacatca acaggtgttg aagggaaggg 480
gcaagttcgc ccaggatgtc agtcaagatg acctgatcag agccatcaag aaa 533

<210> 211
<211> 451
<212> DNA
<213> Homo sapiens

<400> 211
ttagcttgag ccgagaacga ggcgagaaag ctggagaccg aggagaccgc ctagagcgga 60
gtgaacgggg aggggaccgt ggggaccggc ttgatcgtgc gcggacacct gctaccaagc 120
ggagcttcag caaggaagtg gaggagcgga gtagagaacg gccctcccag cctgaggggc 180
tgcgcaaggc agctagcctc acggaggatc gggaccgtgg gcgggatgcc gtgaagcgag 240
aagctgccct acccccagtg agccccctga aggcggctct ctctgaggag gagttagaga 300
agaaatccaa ggctatcatt gaggaatatc tccatctcaa tgacatgaaa gaggcagtcc 360
agtgcgtgca ggagctggcc tcaccctcct tgctcttcat ctttgtacgg catggtgtcg 420
agtctacgct ggagcgcagt gccattgctc g 451

<210> 212
<211> 471
<212> DNA
<213> Homo sapiens

<220>
<221> misc_feature
<222> 54
<223> n = A,T,C or G

<400> 212
gtgattattc ttgatcaggg agaagatcat ttagatttgt tttgcattcc ttanaatgga 60
gggcaacatt ccacagctgc cctggctgtg atgagtgtcc ttgcaggggc cggagtagga 120
gcactggggt gggggcggaa ttggggttac tcgatgtaag ggattccttg ttgttgtgtt 180
gagatccagt gcagttgtga tttctgtgga tcccagcttg gttccaggaa ttttgtgtga 240
ttggcttaaa tccagttttc aatcttcgac agctgggctg gaacgtgaac tcagtagctg 300
aacctgtctg acccggtcac gttcttggat cctcagaact ctttgctctt gtcggggtgg 360
gggtgggaac tcacgtgggg agcggtggct gagaaaatgt aaggattctg gaatacatat 420
tccatgggac tttccttccc tctcctgctt cctcttttcc tgctccctaa c 471

<210> 213
<211> 511
<212> DNA
<213> Homo sapiens

<220>
<221> misc_feature
<222> 27, 63, 337, 442

<223> n = A,T,C or G

<400> 213
ctaattagaa acttgctgta ctttttnttt tcttttaggg gtcaaggacc ctctttatag 60
ctnccatttg cctacaataa attattgcag cagtttgcaa tactaaaata ttttttatag 120
actttatatt tttccttttg ataaagggat gctgcatagt agagttggtg taattaaact 180
atctcagccg tttccctgct ttcccttctg ctccatatgc ctcattgtcc ttccagggag 240
ctcttttaat cttaaagttc tacatttcat gctcttagtc aaattctgtt acctttttaa 300
taactcttcc cactgcatat ttccatcttg aattggnggt tctaaattct gaaactgtag 360
ttgagataca gctatttaat atttctggga gatgtgcatc cctcttcttt gtggttgccc 420
aaggttgttt tgcgtaactg anactccttg atatgcttca gagaatttag gcaaacactg 480
gccatggccg tgggagtact gggagtaaaa t 511

<210> 214
<211> 521
<212> DNA
<213> Homo sapiens

<400> 214
agcattgcca aataatccct aattttccac taaaaatata atgaaatgat gttaagcttt 60
ttgaaaagtt taggttaaac ctactgttgt tagattaatg tatttgttgc ttccctttat 120
ctggaatgtg gcattagctt ttttatttta accctcttta attcttattc aattccatga 180
cttaaggttg gagagctaaa cactgggatt tttggataac agactgacag ttttgcataa 240
ttataatcgg cattgtacat agaaaggata tggctacctt ttgttaaatc tgcactttct 300
aaatatcaaa aaagggaaat gaagtataaa tcaatttttg tataatctgt ttgaaacatg 360
agttttattt gcttaatatt agggctttgc cccttttctg taagtctctt gggatcctgt 420
gtagaagctg ttctcattaa acaccaaaca gttaagtcca ttctctggta ctagctacaa 480
attcggtttc atattctact taacaattta aataaactga a 521

<210> 215
<211> 381
<212> DNA
<213> Homo sapiens

<220>
<221> misc_feature
<222> 17, 20, 60, 61, 365
<223> n = A,T,C or G

<400> 215
gagcggagag cggaccngtn agagccctga gcagccccac cgccgccgcc ggcctagttn 60
ncatcacacc ccgggaggag ccgcagctgc cgcagccggc cccagtcacc atcaccgcaa 120
ccatgagcag cgaggccgag acccagcagc cgcccgccgc cccccccgcc gcccccgccc 180
tcagcgccgc cgacaccaag cccggcacta cgggcagcgg cgcagggagc ggtggcccgg 240
gcggcctcac atcggcggcg cctgccggcg gggacaagaa ggtcatcgca acgaaggttt 300
tgggaacagt aaaatggttc aatgtaagga acggatatgg tttcatcaac aggaatgaca 360
ccaangaaga tgtatttgta c 381

<210> 216
<211> 425
<212> DNA
<213> Homo sapiens

<400> 216
ttactaacta ggtcattcaa ggaagtcaag ttaacttaaa catgtcacct aaatgcactt 60
gatggtgttg aaatgtccac cttcttaaat ttttaagatg aacttagttc taaagaagat 120
aacaggccaa tcctgaaggt actccctgtt tgctgcagaa tgtcagatat tttggatgtt 180
gcataagagt cctatttgcc ccagttaatt caacttttgt ctgcctgttt tgtggactgg 240

ctggctctgt tagaactctg tccaaaaagt gcatggaata taacttgtaa agcttcccac 300
aattgacaat atatatgcat gtgtttaaac caaatccaga aagcttaaac aatagagctg 360
cataatagta tttattaaag aatcacaact gtaaacatga gaataactta aggattctag 420
tttag 425

<210> 217
<211> 181
<212> DNA
<213> Homo sapiens

<400> 217
gagaaaccaa atgataggtt gtagagcctg atgactccaa acaaagccat cacccgcatt 60
cttcctcctt cttctggtgc tacagctcca agggcccttc accttcatgt ctgaaatgga 120
actttggctt tttcagtgga agaatatgtt gaaggtttca ttttgttcta gaaaaaaaaa 180
a 181

<210> 218
<211> 405
<212> DNA
<213> Homo sapiens

<400> 218
caggccttcc agttcactga caaacatggg gaagtgtgcc cagctggctg gaaacctggc 60
agtgatacca tcaagcctga tgtccaaaag agcaaagaat atttctccaa gcagaagtga 120
gcgctgggct gttttagtgc caggctgcgg tgggcagcca tgagaacaaa acctcttctg 180
tatttttttt ttccattagt aaaacacaag acttcagatt cagccgaatt gtggtgtctt 240
acaaggcagg cctttcctac agggggtgga gagaccagcc tttcttcctt tggtaggaat 300
ggcctgagtt ggcgttgtgg gcaggctact ggtttgtatg atgtattagt agagcaaccc 360
attaatcttt tgtagtttgt attaaacttg aactgagaaa aaaaa 405

<210> 219
<211> 216
<212> DNA
<213> Homo sapiens

<220>
<221> misc_feature
<222> 207, 210
<223> n = A,T,C or G

<400> 219
actccaagag ttagggcagc agagtggagc gatttagaaa gaacatttta aaacaatcag 60
ttaatttacc atgtaaaatt gctgtaaatg ataatgtgta cagattttct gttcaaatat 120
tcaattgtaa acttcttgtt aagactgtta cgtttctatt gcttttgtat gggatattgc 180
aaaaataaaa aggaaagaac cctcttnaan aaaaaa 216

<210> 220
<211> 380
<212> DNA
<213> Homo sapiens

<400> 220
cttacaaatt gcccccatgt gtaggggaca cagaaccctt tgagaaaact tagattttttg 60
tctgtacaaa gtctttgcct ttttccttct tcattttttt ccagtacatt aaatttgtca 120
atttcatctt tgagggaaac tgattagatg ggttgtgttt gtgttctgat ggagaaaaca 180
gcaccccaag gactcagaag atgattttaa cagttcagaa cagatgtgtg caatattggt 240
gcatgtaata atgttgagtg gcagtcaaaa gtcatgattt ttatcttagt tcttcattac 300
tgcattgaaa aggaaaacct gtctgagaaa atgcctgaca gtttaattta aaactatggt 360

gtaagtcttt gacaaaaaa                                                380

<210> 221
<211> 398
<212> DNA
<213> Homo sapiens

<400> 221
ggttagtaag ctgtcgactt tgtaaaaaag ttaaaaatga aaaaaaaagg aaaaatgaat 60
tgtatattta atgaatgaac atgtacaatt tgccactggg aggaggttcc tttttgttgg 120
gtgagtctgc aagtgaattt cactgatgtt gatattcatt gtgtgtagtt ttatttcggt 180
cccagccccg tttcctttta ttttggagct aatgccagct gcgtgtctag ttttgagtgc 240
agtaaaatag aatcagcaaa tcactcttat ttttcatcct tttccggtat tttttgggtt 300
gtttctgtgg gagcagtgta caccaactct tcctgtatat tgcctttttg ctggaaaatg 360
ttgtatgttg aataaaattt tctataaaaa ttaaaaaa                          398

<210> 222
<211> 301
<212> DNA
<213> Homo sapiens

<220>
<221> misc_feature
<222> 49, 64
<223> n = A,T,C or G

<400> 222
ttcgataatt gatctcatgg gctttccctg gaggaaaggt tttttttgnt gtttattttt 60
taanaacttg aaacttgtaa actgagatgt ctgtagcttt tttgcccatc tgtagtgtat 120
gtgaagattt caaaacctga gagcactttt tctttgttta gaattatgag aaaggcacta 180
gatgacttta ggatttgcat ttttcccttt attgcctcat ttcttgtgac gccttgttgg 240
ggagggaaat ctgtttattt tttcctacaa ataaaaagct aagattctat atcgcaaaaa 300
a                                                                  301

<210> 223
<211> 200
<212> DNA
<213> Homo sapiens

<400> 223
gtaagtgctt aggaagaaac tttgcaaaca tttaatgagg atacactgtt catttttaaa 60
attccttcac actgtaattt aatgtgtttt atattctttt gtagtaaaac aacataactc 120
agatttctac aggagacagt ggttttattt ggattgtctt ctgtaatagg tttcaataaa 180
gctggatgaa cttaaaaaaa                                              200

<210> 224
<211> 385
<212> DNA
<213> Homo sapiens

<400> 224
gaaaggtttg atccggactc aaagaaagca aaggagtgtg agccgccatc tgctggagca 60
gctgtaactg caagacctgg acaagagatt cgtcagcgaa ctgcagctca aagaaacctt 120
tctccaacac cagcaagccc taaccagggc cctcctccac aagttccagt atctcctgga 180
ccaccaaagg acagttctgc ccctggtgga cccccagaaa ggactgttac tccagcccta 240
tcatcaaatg tgttaccaag acatcttgga tcccctgcta cttcagtgcc tggaatgggt 300
aaacagagca cttaatgtta tttacagttt atattgtttt ctctggttac caataaaacg 360
ggccattttc aggtggtaaa aaaaa                                        385

<210> 225
<211> 560
<212> PRT
<213> Homo sapiens

<400> 225
Met Glu Cys Leu Tyr Tyr Phe Leu Gly Phe Leu Leu Leu Ala Ala Arg
1               5                   10                  15
Leu Pro Leu Asp Ala Ala Lys Arg Phe His Asp Val Leu Gly Asn Glu
            20                  25                  30
Arg Pro Ser Ala Tyr Met Arg Glu His Asn Gln Leu Asn Gly Trp Ser
        35                  40                  45
Ser Asp Glu Asn Asp Trp Asn Glu Lys Leu Tyr Pro Val Trp Lys Arg
    50                  55                  60
Gly Asp Met Arg Trp Lys Asn Ser Trp Lys Gly Gly Arg Val Gln Ala
65                  70                  75                  80
Val Leu Thr Ser Asp Ser Pro Ala Leu Val Gly Ser Asn Ile Thr Phe
                85                  90                  95
Ala Val Asn Leu Ile Phe Pro Arg Cys Gln Lys Glu Asp Ala Asn Gly
            100                 105                 110
Asn Ile Val Tyr Glu Lys Asn Cys Arg Asn Glu Ala Gly Leu Ser Ala
        115                 120                 125
Asp Pro Tyr Val Tyr Asn Trp Thr Ala Trp Ser Glu Asp Ser Asp Gly
    130                 135                 140
Glu Asn Gly Thr Gly Gln Ser His His Asn Val Phe Pro Asp Gly Lys
145                 150                 155                 160
Pro Phe Pro His His Pro Gly Trp Arg Arg Trp Asn Phe Ile Tyr Val
                165                 170                 175
Phe His Thr Leu Gly Gln Tyr Phe Gln Lys Leu Gly Arg Cys Ser Val
            180                 185                 190
Arg Val Ser Val Asn Thr Ala Asn Val Thr Leu Gly Pro Gln Leu Met
        195                 200                 205
Glu Val Thr Val Tyr Arg Arg His Gly Arg Ala Tyr Val Pro Ile Ala
    210                 215                 220
Gln Val Lys Asp Val Tyr Val Val Thr Asp Gln Ile Pro Val Phe Val
225                 230                 235                 240
Thr Met Phe Gln Lys Asn Asp Arg Asn Ser Ser Asp Glu Thr Phe Leu
                245                 250                 255
Lys Asp Leu Pro Ile Met Phe Asp Val Leu Ile His Asp Pro Ser His
            260                 265                 270
Phe Leu Asn Tyr Ser Thr Ile Asn Tyr Lys Trp Ser Phe Gly Asp Asn
        275                 280                 285
Thr Gly Leu Phe Val Ser Thr Asn His Thr Val Asn His Thr Tyr Val
    290                 295                 300
Leu Asn Gly Thr Phe Ser Leu Asn Leu Thr Val Lys Ala Ala Ala Pro
305                 310                 315                 320
Gly Pro Cys Pro Pro Pro Pro Pro Pro Pro Arg Pro Ser Lys Pro Thr
                325                 330                 335
Pro Ser Leu Gly Pro Ala Gly Asp Asn Pro Leu Glu Leu Ser Arg Ile
            340                 345                 350
Pro Asp Glu Asn Cys Gln Ile Asn Arg Tyr Gly His Phe Gln Ala Thr
        355                 360                 365
Ile Thr Ile Val Glu Gly Ile Leu Glu Val Asn Ile Ile Gln Met Thr
    370                 375                 380
Asp Val Leu Met Pro Val Pro Trp Pro Glu Ser Ser Leu Ile Asp Phe
385                 390                 395                 400
Val Val Thr Cys Gln Gly Ser Ile Pro Thr Glu Val Cys Thr Ile Ile

405                 410                 415
Ser Asp Pro Thr Cys Glu Ile Thr Gln Asn Thr Val Cys Ser Pro Val
            420                 425                 430
Asp Val Asp Glu Met Cys Leu Leu Thr Val Arg Arg Thr Phe Asn Gly
        435                 440                 445
Ser Gly Thr Tyr Cys Val Asn Leu Thr Leu Gly Asp Asp Thr Ser Leu
    450                 455                 460
Ala Leu Thr Ser Thr Leu Ile Ser Val Pro Asp Arg Asp Pro Ala Ser
465                 470                 475                 480
Pro Leu Arg Met Ala Asn Ser Ala Leu Ile Ser Val Gly Cys Leu Ala
                485                 490                 495
Ile Phe Val Thr Val Ile Ser Leu Leu Val Tyr Lys Lys His Lys Glu
            500                 505                 510
Tyr Asn Pro Ile Glu Asn Ser Pro Gly Asn Val Val Arg Ser Lys Gly
        515                 520                 525
Leu Ser Val Phe Leu Asn Arg Ala Lys Ala Val Phe Phe Pro Gly Asn
    530                 535                 540
Gln Glu Lys Asp Pro Leu Leu Lys Asn Gln Glu Phe Lys Gly Val Ser
545                 550                 555                 560

<210> 226
<211> 9
<212> PRT
<213> Homo sapiens

<400> 226
Ile Leu Ile Pro Ala Thr Trp Lys Ala
1               5

<210> 227
<211> 9
<212> PRT
<213> Homo sapiens

<400> 227
Phe Leu Leu Asn Asp Asn Leu Thr Ala
1               5

<210> 228
<211> 9
<212> PRT
<213> Homo sapiens

<400> 228
Leu Leu Gly Asn Cys Leu Pro Thr Val
1               5

<210> 229
<211> 10
<212> PRT
<213> Homo sapiens

<400> 229
Lys Leu Leu Gly Asn Cys Leu Pro Thr Val

1                5                   10


<210> 230
<211> 10
<212> PRT
<213> Homo sapiens

<400> 230
Arg Leu Thr Gly Gly Leu Lys Phe Phe Val
1               5                   10


<210> 231
<211> 9
<212> PRT
<213> Homo sapiens

<400> 231
Ser Leu Gln Ala Leu Lys Val Thr Val
1               5


<210> 232
<211> 20
<212> PRT
<213> Homo sapiens

<400> 232
Ala Gly Ala Asp Val Ile Lys Asn Asp Gly Ile Tyr Ser Arg Tyr Phe
1               5                   10                  15
Phe Ser Phe Ala
            20


<210> 233
<211> 21
<212> PRT
<213> Homo sapiens

<400> 233
Phe Phe Ser Phe Ala Ala Asn Gly Arg Tyr Ser Leu Lys Val His Val
1               5                   10                  15
Asn His Ser Pro Ser
            20


<210> 234
<211> 20
<212> PRT
<213> Homo sapiens

<400> 234
Phe Leu Val Thr Trp Gln Ala Ser Gly Pro Pro Glu Ile Ile Leu Phe
1               5                   10                  15
Asp Pro Asp Gly
            20

<210> 235
<211> 20
<212> PRT
<213> Homo sapiens

<400> 235
Leu Gln Ser Ala Val Ser Asn Ile Ala Gln Ala Pro Leu Phe Ile Pro
1               5                   10                  15
Pro Asn Ser Asp
            20


<210> 236
<211> 20
<212> PRT
<213> Homo sapiens

<400> 236
Ile Gln Asp Asp Phe Asn Asn Ala Ile Leu Val Asn Thr Ser Lys Arg
1               5                   10                  15
Asn Pro Gln Gln
            20


<210> 237
<211> 21
<212> PRT
<213> Homo sapiens

<400> 237
Arg Asn Ser Leu Gln Ser Ala Val Ser Asn Ile Ala Gln Ala Pro Leu
1               5                   10                  15
Phe Ile Pro Pro Asn
            20


<210> 238
<211> 20
<212> PRT
<213> Homo sapiens

<400> 238
Thr His Glu Ser His Arg Ile Tyr Val Ala Ile Arg Ala Met Asp Arg
1               5                   10                  15
Asn Ser Leu Gln
            20


<210> 239
<211> 20
<212> PRT
<213> Homo sapiens

<400> 239
Arg Asn Pro Gln Gln Ala Gly Ile Arg Glu Ile Phe Thr Phe Ser Pro
1               5                   10                  15
Gln Ile Ser Thr

            20

<210> 240
<211> 21
<212> PRT
<213> Homo sapiens

<400> 240
Gly Gln Ala Thr Ser Tyr Glu Ile Arg Met Ser Lys Ser Leu Gln Asn
1               5                   10                  15
Ile Gln Asp Asp Phe
            20

<210> 241
<211> 20
<212> PRT
<213> Homo sapiens

<400> 241
Glu Arg Lys Trp Gly Phe Ser Arg Val Ser Ser Gly Gly Ser Phe Ser
1               5                   10                  15
Val Leu Gly Val
            20

<210> 242
<211> 20
<212> PRT
<213> Homo sapiens

<400> 242
Gly Ser His Ala Met Tyr Val Pro Gly Tyr Thr Ala Asn Gly Asn Ile
1               5                   10                  15
Gln Met Asn Ala
            20

<210> 243
<211> 20
<212> PRT
<213> Homo sapiens

<400> 243
Val Asn His Ser Pro Ser Ile Ser Thr Pro Ala His Ser Ile Pro Gly
1               5                   10                  15
Ser His Ala Met
            20

<210> 244
<211> 20
<212> PRT
<213> Homo sapiens

<400> 244
Ala Val Pro Pro Ala Thr Val Glu Ala Phe Val Glu Arg Asp Ser Leu

1               5                   10                  15
His Phe Pro His
            20


<210> 245
<211> 20
<212> PRT
<213> Homo sapiens

<400> 245
Lys Pro Gly His Trp Thr Tyr Thr Leu Asn Asn Thr His His Ser Leu
1               5                   10                  15
Gln Ala Leu Lys
            20


<210> 246
<211> 20
<212> PRT
<213> Homo sapiens

<400> 246
Asn Leu Thr Phe Arg Thr Ala Ser Leu Trp Ile Pro Gly Thr Ala Lys
1               5                   10                  15
Pro Gly His Trp
            20


<210> 247
<211> 20
<212> PRT
<213> Homo sapiens

<400> 247
Leu His Phe Pro His Pro Val Met Ile Tyr Ala Asn Val Lys Gln Gly
1               5                   10                  15
Phe Tyr Pro Ile
            20


<210> 248
<211> 20
<212> PRT
<213> Homo sapiens

<400> 248
Pro Glu Thr Gly Asp Pro Val Thr Leu Arg Leu Leu Asp Asp Gly Ala
1               5                   10                  15
Gly Ala Asp Val
            20


<210> 249
<211> 20
<212> PRT
<213> Homo sapiens

<400> 249
Gly Phe Tyr Pro Ile Leu Asn Ala Thr Val Thr Ala Thr Val Glu Pro
1               5                   10                  15
Glu Thr Gly Asp
            20


<210> 250
<211> 20
<212> PRT
<213> Homo sapiens

<400> 250
Phe Asp Pro Asp Gly Arg Lys Tyr Tyr Thr Asn Asn Phe Ile Thr Asn
1               5                   10                  15
Leu Thr Phe Arg
            20


<210> 251
<211> 20
<212> PRT
<213> Homo sapiens

<400> 251
Leu Gln Ala Leu Lys Val Thr Val Thr Ser Arg Ala Ser Asn Ser Ala
1               5                   10                  15
Val Pro Pro Ala
            20


<210> 252
<211> 153
<212> PRT
<213> Homo sapiens

<400> 252
Met Ala Ser Val Arg Val Ala Ala Tyr Phe Glu Asn Phe Leu Ala Ala
1               5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly
        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Ile Thr
                85                  90                  95
Pro Glu Ala Phe Glu Lys Leu Gly Phe Pro Ala Ala Lys Glu Ile Ala
            100                 105                 110
Asn Met Cys Arg Phe Tyr Glu Met Lys Pro Asp Arg Asp Val Asn Leu
        115                 120                 125
Thr His Gln Leu Asn Pro Lys Val Lys Ser Phe Ser Gln Phe Ile Ser
    130                 135                 140
Glu Asn Gln Gly Ala Phe Lys Gly Met
145                 150

<210> 253
<211> 462
<212> DNA
<213> Homo sapiens

<400> 253
atggccagtg tccgcgtggc ggcctacttt gaaaactttc tcgcggcgtg gcggcccgtg 60
aaagcctctg atggagatta ctacaccttg gctgtaccga tgggagatgt accaatggat 120
ggtatctctg ttgctgatat tggagcagcc gtctctagca tttttaattc tccagaggaa 180
tttttaggca aggccgtggg gctcagtgca gaagcactaa caatacagca atatgctgat 240
gttttgtcca aggctttggg gaaagaagtc cgagatgcaa agattacccc ggaagctttc 300
gagaagctgg gattccctgc agcaaaggaa atagccaata tgtgtcgttt ctatgaaatg 360
aagccagacc gagatgtcaa tctcacccac caactaaatc ccaaagtcaa aagcttcagc 420
cagtttatct cagagaacca gggagccttc aagggcatgt ag 462

<210> 254
<211> 8031
<212> DNA
<213> Homo sapiens

<400> 254
tggcgaatgg gacgcgccct gtagcggcgc attaagcgcg gcgggtgtgg tggttacgcg 60
cagcgtgacc gctacacttg ccagcgccct agcgcccgct cctttcgctt tcttcccttc 120
ctttctcgcc acgttcgccg gctttccccg tcaagctcta aatcgggggc tccctttagg 180
gttccgattt agtgctttac ggcacctcga ccccaaaaaa cttgattagg gtgatggttc 240
acgtagtggg ccatcgccct gatagacggt ttttcgccct ttgacgttgg agtccacgtt 300
ctttaatagt ggactcttgt tccaaactgg aacaacactc aaccctatct cggtctattc 360
ttttgattta taagggattt tgccgatttc ggcctattgg ttaaaaaatg agctgattta 420
acaaaaattt aacgcgaatt ttaacaaaat attaacgttt acaatttcag gtggcacttt 480
tcggggaaat gtgcgcggaa cccctatttg tttatttttc taaatacatt caaatatgta 540
tccgctcatg aattaattct tagaaaaact catcgagcat caaatgaaac tgcaatttat 600
tcatatcagg attatcaata ccatattttt gaaaaagccg tttctgtaat gaaggagaaa 660
actcaccgag gcagttccat aggatggcaa gatcctggta tcggtctgcg attccgactc 720
gtccaacatc aatacaacct attaatttcc cctcgtcaaa aataaggtta tcaagtgaga 780
aatcaccatg agtgacgact gaatccggtg agaatggcaa aagtttatgc atttctttcc 840
agacttgttc aacaggccag ccattacgct cgtcatcaaa atcactcgca tcaaccaaac 900
cgttattcat tcgtgattgc gcctgagcga gacgaaatac gcgatcgctg ttaaaaggac 960
aattacaaac aggaatcgaa tgcaaccggc gcaggaacac tgccagcgca tcaacaatat 1020
tttcacctga atcaggatat tcttctaata cctggaatgc tgttttcccg gggatcgcag 1080
tggtgagtaa ccatgcatca tcaggagtac ggataaaatg cttgatggtc ggaagaggca 1140
taaattccgt cagccagttt agtctgacca tctcatctgt aacatcattg gcaacgctac 1200
ctttgccatg tttcagaaac aactctggcg catcgggctt cccatacaat cgatagattg 1260
tcgcacctga ttgcccgaca ttatcgcgag cccatttata cccatataaa tcagcatcca 1320
tgttggaatt taatcgcggc ctagagcaag acgtttcccg ttgaatatgg ctcataacac 1380
cccttgtatt actgtttatg taagcagaca gttttattgt tcatgaccaa aatcccttaa 1440
cgtgagtttt cgttccactg agcgtcagac cccgtagaaa agatcaaagg atcttcttga 1500
gatccttttt ttctgcgcgt aatctgctgc ttgcaaacaa aaaaaccacc gctaccagcg 1560
gtggtttgtt tgccggatca agagctacca actctttttc cgaaggtaac tggcttcagc 1620
agagcgcaga taccaaatac tgtccttcta gtgtagccgt agttaggcca ccacttcaag 1680
aactctgtag caccgcctac atacctcgct ctgctaatcc tgttaccagt ggctgctgcc 1740
agtggcgata agtcgtgtct taccgggttg gactcaagac gatagttacc ggataaggcg 1800
cagcggtcgg gctgaacggg gggttcgtgc acacagccca gcttggagcg aacgacctac 1860
accgaactga gatacctaca gcgtgagcta tgagaaagcg ccacgcttcc cgaagggaga 1920
aaggcggaca ggtatccggt aagcggcagg gtcggaacag gagagcgcac gagggagctt 1980
ccagggggaa acgcctggta tctttatagt cctgtcgggt ttcgccacct ctgacttgag 2040
cgtcgatttt tgtgatgctc gtcagggggg cggagcctat ggaaaaacgc cagcaacgcg 2100
gcctttttac ggttcctggc cttttgctgg ccttttgctc acatgttctt tcctgcgtta 2160

tcccctgatt ctgtggataa ccgtattacc gcctttgagt gagctgatac cgctcgccgc 2220
agccgaacga ccgagcgcag cgagtcagtg agcgaggaag cggaagagcg cctgatgcgg 2280
tattttctcc ttacgcatct gtgcggtatt tcacaccgca tatatggtgc actctcagta 2340
caatctgctc tgatgccgca tagttaagcc agtatacact ccgctatcgc tacgtgactg 2400
ggtcatggct gcgccccgac acccgccaac acccgctgac gcgccctgac gggcttgtct 2460
gctcccggca tccgcttaca gacaagctgt gaccgtctcc gggagctgca tgtgtcagag 2520
gttttcaccg tcatcaccga aacgcgcgag gcagctgcgg taaagctcat cagcgtggtc 2580
gtgaagcgat tcacagatgt ctgcctgttc atccgcgtcc agctcgttga gtttctccag 2640
aagcgttaat gtctggcttc tgataaagcg ggccatgtta agggcggttt tttcctgttt 2700
ggtcactgat gcctccgtgt aagggggatt tctgttcatg ggggtaatga taccgatgaa 2760
acgagagagg atgctcacga tacgggttac tgatgatgaa catgcccggt tactggaacg 2820
ttgtgagggt aaacaactgg cggtatggat gcggcgggac cagagaaaaa tcactcaggg 2880
tcaatgccag cgcttcgtta atacagatgt aggtgttcca cagggtagcc agcagcatcc 2940
tgcgatgcag atccggaaca taatggtgca gggcgctgac ttccgcgttt ccagacttta 3000
cgaaacacgg aaaccgaaga ccattcatgt tgttgctcag gtcgcagacg ttttgcagca 3060
gcagtcgctt cacgttcgct cgcgtatcgg tgattcattc tgctaaccag taaggcaacc 3120
ccgccagcct agccgggtcc tcaacgacag gagcacgatc atgcgcaccc gtggggccgc 3180
catgccggcg ataatggcct gcttctcgcc gaaacgtttg gtggcgggac cagtgacgaa 3240
ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420
ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatatcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140
ggtcagagac atcaagaaat aacgccggaa cattagtgca ggcagcttcc acagcaatgg 4200
catcctggtc atccagcgga tagttaatga tcagcccact gacgcgttgc gcgagaagat 4260
tgtgcaccgc cgctttacag gcttcgacgc cgcttcgttc taccatcgac accaccacgc 4320
tggcacccag ttgatcggcg cgagatttaa tcgccgcgac aatttgcgac ggcgcgtgca 4380
gggccagact ggaggtggca acgccaatca gcaacgactg tttgcccgcc agttgttgtg 4440
ccacgcggtt gggaatgtaa ttcagctccg ccatcgccgc ttccactttt tcccgcgttt 4500
tcgcagaaac gtggctggcc tggttcacca cgcgggaaac ggtctgataa gagacaccgg 4560
catactctgc gacatcgtat aacgttactg gtttcacatt caccaccctg aattgactct 4620
cttccgggcg ctatcatgcc ataccgcgaa aggttttgcg ccattcgatg gtgtccggga 4680
tctcgacgct ctcccttatg cgactcctgc attaggaagc agcccagtag taggttgagg 4740
ccgttgagca ccgccgccgc aaggaatggt gcatgcaagg agatggcgcc caacagtccc 4800
ccggccacgg ggcctgccac catacccacg ccgaaacaag cgctcatgag cccgaagtgg 4860
cgagcccgat cttccccatc ggtgatgtcg gcgatatagg cgccagcaac cgcacctgtg 4920
gcgccggtga tgccggccac gatgcgtccg gcgtagagga tcgagatctc gatcccgcga 4980
aattaatacg actcactata ggggaattgt gagcggataa caattcccct ctagaaataa 5040
ttttgtttaa ctttaagaag gagatataca tatgcagcat caccaccatc accacggagt 5100
acagcttcaa gacaatgggt ataatggatt gctcattgca attaatcctc aggtacctga 5160
gaatcagaac ctcatctcaa acattaagga aatgataact gaagcttcat tttacctatt 5220
taatgctacc aagagaagag tatttttcag aaatataaag attttaatac ctgccacatg 5280
gaaagctaat aataacagca aaataaaaca agaatcatat gaaaaggcaa atgtcatagt 5340
gactgactgg tatggggcac atggagatga tccatacacc ctacaataca gagggtgtgg 5400
aaaagaggga aaatacattc atttcacacc taatttccta ctgaatgata acttaacagc 5460
tggctacgga tcacgaggcc gagtgtttgt ccatgaatgg gcccacctcc gttggggtgt 5520
gttcgatgag tataacaatg acaaaccttt ctacataaat gggcaaaatc aaattaaagt 5580
gacaaggtgt tcatctgaca tcacaggcat ttttgtgtgt gaaaaaggtc cttgccccca 5640

agaaaactgt attattagta agctttttaa agaaggatgc acctttatct acaatagcac 5700
ccaaaatgca actgcatcaa taatgttcat gcaaagttta tcttctgtgg ttgaattttg 5760
taatgcaagt acccacaacc aagaagcacc aaacctacag aaccagatgt gcagcctcag 5820
aagtgcatgg gatgtaatca cagactctgc tgactttcac cacagctttc ccatgaacgg 5880
gactgagctt ccacctcctc ccacattctc gcttgtagag gctggtgaca aagtggtctg 5940
tttagtgctg gatgtgtcca gcaagatggc agaggctgac agactccttc aactacaaca 6000
agccgcagaa ttttatttga tgcagattgt tgaaattcat accttcgtgg gcattgccag 6060
tttcgacagc aaaggagaga tcagagccca gctacaccaa attaacagca atgatgatcg 6120
aaagttgctg gtttcatatc tgcccaccac tgtatcagct aaaacagaca tcagcatttg 6180
ttcagggctt aagaaaggat ttgaggtggt tgaaaaactg aatggaaaag cttatggctc 6240
tgtgatgata ttagtgacca gcggagatga taagcttctt ggcaattgct tacccactgt 6300
gctcagcagt ggttcaacaa ttcactccat tgccctgggt tcatctgcag ccccaaatct 6360
ggaggaatta tcacgtctta caggaggttt aaagttcttt gttccagata tatcaaactc 6420
caatagcatg attgatgctt tcagtagaat ttcctctgga actggagaca ttttccagca 6480
acatattcag cttgaaagta caggtgaaaa tgtcaaacct caccatcaat tgaaaaacac 6540
agtgactgtg gataatactg tgggcaacga cactatgttt ctagttacgt ggcaggccag 6600
tggtcctcct gagattatat tatttgatcc tgatggacga aaatactaca caaataattt 6660
tatcaccaat ctaacttttc ggacagctag tctttggatt ccaggaacag ctaagcctgg 6720
gcactggact tacaccctga acaataccca tcattctctg caagccctga aagtgacagt 6780
gacctctcgc gcctccaact cagctgtgcc cccagccact gtggaagcct ttgtggaaag 6840
agacagcctc cattttcctc atcctgtgat gatttatgcc aatgtgaaac agggatttta 6900
tcccattctt aatgccactg tcactgccac agttgagcca gagactggag atcctgttac 6960
gctgagactc cttgatgatg gagcaggtgc tgatgttata aaaaatgatg gaatttactc 7020
gaggtatttt ttctcctttg ctgcaaatgg tagatatagc ttgaaagtgc atgtcaatca 7080
ctctcccagc ataagcaccc cagcccactc tattccaggg agtcatgcta tgtatgtacc 7140
aggttacaca gcaaacggta atattcagat gaatgctcca aggaaatcag taggcagaaa 7200
tgaggaggag cgaaagtggg gctttagccg agtcagctca ggaggctcct tttcagtgct 7260
gggagttcca gctggccccc accctgatgt gtttccacca tgcaaaatta ttgacctgga 7320
agctgtaaaa gtagaagagg aattgaccct atcttggaca gcacctggag aagactttga 7380
tcagggccag gctacaagct atgaaataag aatgagtaaa agtctacaga atatccaaga 7440
tgactttaac aatgctattt tagtaaatac atcaaagcga aatcctcagc aagctggcat 7500
cagggagata tttacgttct caccccaaat ttccacgaat ggacctgaac atcagccaaa 7560
tggagaaaca catgaaagcc acagaattta tgttgcaata cgagcaatgg ataggaactc 7620
cttacagtct gctgtatcta acattgccca ggcgcctctg tttattcccc ccaattctga 7680
tcctgtacct gccagagatt atcttatatt gaaaggagtt ttaacagcaa tgggtttgat 7740
aggaatcatt tgccttatta tagttgtgac acatcatact ttaagcagga aaaagagagc 7800
agacaagaaa gagaatggaa caaaattatt ataatgaatt ctgcagatat ccatcacact 7860
ggcggccgct cgagcaccac caccaccacc actgagatcc ggctgctaac aaagcccgaa 7920
aggaagctga gttggctgct gccaccgctg agcaataact agcataaccc cttggggcct 7980
ctaaacgggt cttgaggggt tttttgctga aaggaggaac tatatccgga t 8031

<210> 255
<211> 401
<212> DNA
<213> Homo sapiens

<220>
<221> misc_feature
<222> 9, 67, 247, 275, 277, 397
<223> n = A,T,C or G

<400> 255
gtggccagng actagaaggc gaggcgccgc gggaccatgg cggcggcggc ggacgagcgg 60
agtccanagg acggagaaga cgaggaagag gaggagcagt tggttctggt ggaattatca 120
ggaattattg attcagactt cctctcaaaa tgtgaaaata aatgcaaggt tttgggcatt 180
gacactgaga ggcccattct gcaagtggac agctgtgtct ttgctgggga gtatgaagac 240

actctangga cctgtgttat atttgaagaa aatgntnaac atgctgatac agaaggcaat 300
aataaaacag tgctaaaata taaatgccat acaatgaaga agctcagcat gacaagaact 360
ctcctgacag agaagaagga aggagaagaa aacatangtg g 401

<210> 256
<211> 401
<212> DNA
<213> Homo sapiens

<220>
<221> misc_feature
<222> 7, 37, 51, 79, 96, 98, 103, 104, 107, 116, 167, 181, 183,
194, 206, 276, 303, 307, 308, 310, 323, 332, 341, 353, 374,
376
<223> n = A,T,C or G

<400> 256
tggtggncct gggatgggga accgcggtgg cttccgngga ggtttcggca ntggcatccg 60
gggccggggt cgcggccgng gacggggccg gggccnangc cgnnganctc gcggangcaa 120
ggccgaggat aaggagtgga tgcccgtcac caacttgggc cgcttgncca aggacatgaa 180
nancaagccc ctgnaggaga tctatntctt cttccctgcc ccattaagga atcaagagat 240
catttgattt cttcctgggg gcctctctca aggatnaggt ttttgaagat tatgccagtg 300
canaaannan accccgttgc ccngtccatc tncacccaac ncttccaagg gcnatttttg 360
tttaggcctc attncngggg ggaaccttaa cccaatttgg g 401

<210> 257
<211> 401
<212> DNA
<213> Homo sapiens

<220>
<221> misc_feature
<222> 382, 387
<223> n = A,T,C or G

<400> 257
atgtatgtaa aacacttcat aaaatgtaaa gggctataac aaatatgtta taaagtgatt 60
ctctcagccc tgaggtatac agaatcattt gcctcagact gctgttggat tttaaaattt 120
ttaaaatatc tgctaagtaa tttgctatgt cttctcccac actatcaata tgcctgcttc 180
taacaggctc cccactttct tttaatgtgc tgttatgagc tttggacatg agataaccgt 240
gcctgttcag agtgtctaca gtaagagctg gacaaactct ggagggacac agtctttgag 300
acagctcttt tggttgcttt ccacttttct gaaaggttca cagtaacctt ctagataata 360
gaaactccca gttaaagcct angctancaa ttttttttag t 401

<210> 258
<211> 401
<212> DNA
<213> Homo sapiens

<400> 258
ggagcgctag gtcggtgtac gaccgagatt agggtgcgtg ccagctccgg gaggccgcgg 60
tgaggggccg ggcccaagct gccgacccga gccgatcgtc agggtcgcca gcgcctcagc 120
tctgtggagg agcagcagta gtcggagggt gcaggatatt agaaatggct actccccagt 180
caattttcat ctttgcaatc tgcattttaa tgataacaga attaattctg gcctcaaaaa 240
gctactatga tatcttaggt gtgccaaaat cggcatcaga gcgccaaatc aagaaggcct 300
ttcacaagtt ggccatgaag taccaccctg acaaaaataa gacccagatg ctgaagcaaa 360
attcagagag attgcagaag catatgaaac actctcagat g 401

<210> 259
<211> 401
<212> DNA
<213> Homo sapiens

<400> 259
attgggtttg gagggaggat gatgacagag gaatgccctt tggccatcac ggttttgatt 60
ctccagaata ttgtgggttt gatcatcaat gcagtcatgt taggctgcat tttcatgaaa 120
acagctcagg ctcacagaag ggcagaaact ttgattttca gccgccatgc tgtgattgcc 180
gtccgaaatg gcaagctgtg cttcatgttc cgagtgggtg acctgaggaa aagcatgatc 240
attagtgcct ctgtgcgcat ccaggtggtc aagaaaacaa ctacacctga aggggaggtg 300
gttcctattc accaactgga cattcctgtt gataacccaa tcgagagcaa taacattttt 360
ctggtggccc ctttgatcat ctgccacgtg attgacaagc g 401

<210> 260
<211> 363
<212> DNA
<213> Homo sapiens

<220>
<221> misc_feature
<222> 7, 9, 19, 41,' 63, 73, 106, 111, 113, 116, 119, 156, 158,
162, 187, 247, 288, 289, 290, 292, 298, 299, 300, 340
<223> n = A,T,C or G

<400> 260
aggaganang gagggggana tgaataggga tggagaggga natagtggat gagcagggca 60
canggagagg aancagaaag gagaggcaag acagggagac acacancaca nangangana 120
caggtggggg ctggggtggg gcatggagag cctttnangt cncccaggcc accctgctct 180
cgctggnctg ttgaaaccca ctccatggct tcctgccact gcagttgggc ccagggctgg 240
cttattnctg gaatgcaagt ggctgtggct tggagcctcc cctctggnnn anggaaannn 300
attgctccct tatctgcttg gaatatctga gtttttccan cccggaaata aaacacacac 360
aca 363

<210> 261
<211> 401
<212> DNA
<213> Homo sapiens

<220>
<221> misc_feature
<222> 114, 152
<223> n = A,T,C or G

<400> 261
cggctctccg ccgctctccc ggggtttcgg ggcacttggg tcccacagtc tggtcctgct 60
tcaccttccc ctgacctgag tagtcgccat ggcacaggtt ctcagaggca ctgngactga 120
cttccctgga tttgatgagc gggctgatgc anaaactctt cggaaggcta tgaaaggctt 180
gggcacagat gaggagagca tcctgactct gttgacatcc cgaagtaatg ctcagcgcca 240
ggaaatctct gcagctttta agactctgtt tggcagggat cttctggatg acctgaaatc 300
agaactaact ggaaaatttg aaaaattaat tgtggctctg atgaaaccct ctcggcttta 360
tgatgcttat gaactgaaac atgccttgaa gggagctgga a 401

<210> 262
<211> 401
<212> DNA
<213> Homo sapiens

<220>
<221> misc_feature
<222> 7, 26, 258, 305, 358, 373, 374, 378
<223> n = A,T,C or G

<400> 262
agtctanaac atttctaata ttttgngctt tcatatatca aaggagatta tgtgaaacta 60
tttttaaata ctgtaaagtg acatatagtt ataagatata tttctgtaca gtagagaaag 120
agtttataac atgaagaata ttgtaccatt atacattttc attctcgatc tcataagaaa 180
ttcaaaagaa taatgataga ggtgaaaata tgtttacttt ctctaaatca agcctagttg 240
tcaactcaaa aattatgntg catagtttta ttttgaattt aggttttggg actacttttt 300
tccancttca atgagaaaat aaaatctaca actcaggagt tactacagaa gttctaanta 360
tttttttgct aannagcnaa aaatataaac atatgaaaat g 401

<210> 263
<211> 401
<212> DNA
<213> Homo sapiens

<220>
<221> misc_feature
<222> 232, 290, 304, 326, 383
<223> n = A,T,C or G

<400> 263
ctgtccgacc aagagaggcc ggccgagccc gaggcttggg cttttgcttt ctggcggagg 60
gatctgcggc ggtttaggag gcggcgctga tcctgggagg aagaggcagc tacggcggcg 120
gcggcggtgg cggctagggc ggcggcgaat aaaggggccg ccgccgggtg atgcggtgac 180
cactgcggca ggcccaggag ctgagtgggc cccggccctc agcccgtccc gncggacccg 240
ctttcctcaa ctctccatct tctcctgccg accgagatcg ccgaggcggn ctcaggctcc 300
ctancccctt ccccgtccct tccccncccc cgtccccgcc ccgggggccg ccgccacccg 360
cctcccacca tggctctgaa ganaatccac aaggaattga a 401

<210> 264
<211> 401
<212> DNA
<213> Homo sapiens

<400> 264
aacaccagcc actccaggac ccctgaaggc ctctaccagg tcaccagtgt tctgcgccta 60
aagccacccc ctggcagaaa cttcagctgt gtgttctgga atactcacgt gagggaactt 120
actttggcca gcattgacct tcaaagtcag atggaaccca ggacccatcc aacttggctg 180
cttcacattt tcatcccctc ctgcatcatt gctttcattt tcatagccac agtgatagcc 240
ctaagaaaac aactctgtca aaagctgtat tcttcaaaag acacaacaaa aagacctgtc 300
accacaacaa agagggaagt gaacagtgct gtgaatctga acctgtggtc ttgggagcca 360
gggtgacctg atatgacatc taaagaagct tctggactct g 401

<210> 265
<211> 271
<212> DNA
<213> Homo sapiens

<220>
<221> misc_feature
<222> 59
<223> n = A,T,C or G

<400> 265
   gccacttcct gtggacatgg gcagagcgct gctgccagtt cctggtagcc ttgaccacna 60
   cgctgggggg tctttgtgat ggtcatgggt ctcatttgca cttgggggtg tgggattcaa 120
   gttagaagtt tctagatctg gccgggcgca gtggctcaca cctgtaatcc cagcacttta 180
   ggaggctgag gcaggcggat catgaggtca ggagatcgag accgtcctgg ctaacacagt 240
   gaaaccccgt ctctactaaa aatacaaaaa a 271

<210> 266
<211> 401
<212> DNA
<213> Homo sapiens

<220>
<221> misc_feature
<222> 45
<223> n = A,T,C or G

<400> 266
attcataaat ttagctgaaa gatactgatt caatttgtat acagngaata taaatgagac 60
gacagcaaaa ttttcatgaa atgtaaaata tttttatagt ttgttcatac tatatgaggt 120
tctattttaa atgactttct ggattttaaa aaatttcttt aaatacaatc atttttgtaa 180
tatttatttt atgcttatga tctagataat tgcagaatat cattttatct gactctgtct 240
tcataagaga gctgtggccg aattttgaac atctgttata gggagtgatc aaattagaag 300
gcaatgtgga aaaacaattc tgggaaagat ttctttatat gaagtccctg ccactagcca 360
gccatcctaa ttgatgaaag ttatctgttc acaggcctgc a 401

<210> 267
<211> 401
<212> DNA
<213> Homo sapiens

<220>
<221> misc_feature
<222> 116, 247, 277, 296, 307, 313, 322, 323, 336, 342, 355, 365,
377, 378, 397
<223> n = A,T,C or G

<400> 267
gaagaggcat cacctgatcc cggagacctt tggagttaag aggcggcgga agcgagggcc 60
tgtggagtcg gatcctcttc ggggtgagcc agggtcggcg cgcgcggctg tctcanaact 120
catgcagctg ttcccgcgag gcctgtttga ggacgcgctg ccgcccatcg tgctgaggag 180
ccaggtgtac agccttgtgc ctgacaggac cgtggccgac cggcagctga aggagcttca 240
agagcanggg gagacaaaat cgtccagctg ggcttcnact tggatgccca tggaanttat 300
tctttcnctt ganggactta cnngggaccc aagaanccct tncaaggggc ccttngtgga 360
tgggncccga aaccccnnta tttgcccttg ggggggncca a 401

<210> 268
<211> 223
<212> DNA
<213> Homo sapiens

<400> 268
   tcgccatgtt ggccaggctg gtcttgaact cctgacttta agtgatccac ccgcctcaac 60
   ctcccaaagt gctgggatta caggtgtgag ccaccgcgcc tggcctgata catactttta 120
   gaatcaagta gtcacgcact ttttctgttc atttttctaa aaagtaaata tacaaatgtt 180
   ttgttttttg ttttttttgt ttgtttgttt ctgttttttt ttt 223

<210> 269
<211> 401

<212> DNA
<213> Homo sapiens

<400> 269
actatgtaaa ccacattgta ctttttttta ctttggcaac aaatatttat acatacaaga 60
tgctagttca tttgaatatt tctcccaact tatccaagga tctccagctc taacaaaatg 120
gtttattttt atttaaatgt caatagttgt tttttaaaat ccaaatcaga ggtgcaggcc 180
accagttaaa tgccgtctat caggttttgt gccttaagag actacagagt caaagctcat 240
ttttaaagga gtaggacaaa gttgtcacag gtttttgttg ttgtttttat tgcccccaaa 300
attacatgtt aatttccatt tatatcaggg attctattta cttgaagact gtgaagttgc 360
cattttgtct cattgttttc tttgacataa ctaggatcca t 401

<210> 270
<211> 401
<212> DNA
<213> Homo sapiens

<220>
<221> misc_feature
<222> 240, 382
<223> n = A,T,C or G

<400> 270
tggctgttga ttcacctcag cactgcttgg tatctgcacc ctacctctct ttagaggctg 60
ccttgtcaac tgaaaaatgc acctgacttc gagcaagact ctttccttag gttctggatc 120
tgtttgagcc ccatggcact gagctggaat ctgagggtct tgttccaagg atgtgatgat 180
gtgggagaat gttctttgaa agagcagaaa tccagtctgc atggaaacag cctgtagagn 240
agaagtttcc agtgataagt gttcactgtt ctaaggaggt acaccacagc tacctgaatt 300
ttcccaaaat gagtgcttct gtgcgttaca actggccttt gtacttgact gtgatgactt 360
tgttttttct tttcaattct anatgaacat gggaaaaaat g 401

<210> 271
<211> 329
<212> DNA
<213> Homo sapiens

<400> 271
ccacagcctc caagtcaggt ggggtggagt cccagagctg cacagggttt ggcccaagtt 60
tctaagggag gcacttcctc ccctcgccca tcagtgccag cccctgctgg ctggtgcctg 120
agcccctcag acagccccct gccccgcagg cctgccttct cagggacttc tgcggggcct 180
gaggcaagcc atggagtgag acccaggagc cggacacttc tcaggaaatg gcttttccca 240
acccccagcc cccacccggt ggttcttcct gttctgtgac tgtgtatagt gccaccacag 300
cttatggcat ctcattgagg acaaaaaaa 329

<210> 272
<211> 401
<212> DNA
<213> Homo sapiens

<220>
<221> misc_feature
<222> 1, 7, 12, 21, 61, 62, 66, 72, 78, 88, 90, 92, 98, 117, 119,
128, 130, 134, 142, 144, 151, 159, 162, 164, 168, 169, 177,
184, 185, 188, 194, 202, 204, 209, 213, 218, 223, 231, 260,
272, 299, 300, 306, 321, 322, 323, 331, 335, 336, 338
<223> n = A,T,C or G

<221> misc_feature

<222> 341, 342, 343, 345, 346, 351, 358, 360, 362, 363, 387, 390,
392
<223> n = A,T,C or G

<400> 272
nggctgntaa cntcggaggt nacttcctgg actatcctgg agaccccctc cgcttccacg 60
nncatnatat cnctcatngc tgggcccntn angacacnat cccactccaa cacctgngng 120
atgctggncn cctnggaacc ancntcagaa ngaccctgnt cntntgtnnt ccgcaanctg 180
aagnnaangc gggntacacc tncntgcant ggnccacnct gcngggaact ntacacacct 240
acgggatgtg gctgcgccan gagccaagag cntttctgga tgattcccca gcctcttgnn 300
agggantcta caacattgct nnntaccttt ntccnncngc nnntnntgga ntacaggngn 360
tnntaacact acatcttttt tactgcnccn tncttggtgg g 401

<210> 273
<211> 401
<212> DNA
<213> Homo sapiens

<220>
<221> misc_feature
<222> 399
<223> n = A,T,C or G

<400> 273
cagcaccatg aagatcaaga tcatcgcacc cccagagcgc aagtactcgg tgtggatcgg 60
tggctccatc ctggcctcac tgtccacctt ccagcagatg tggattagca agcaggagta 120
cgacgagtcg ggcccctcca tcgtccaccg caaatgcttc taaacggact cagcagatgc 180
gtagcatttg ctgcatgggt taattgagaa tagaaatttg cccctggcaa atgcacacac 240
ctcatgctag cctcacgaaa ctggaataag ccttcgaaaa gaaattgtcc ttgaagcttg 300
tatctgatat cagcactgga ttgtagaact tgttgctgat tttgaccttg tattgaagtt 360
aactgttccc cttggtatta acgtgtcagg gctgagtgnt c 401

<210> 274
<211> 401
<212> DNA
<213> Homo sapiens

<400> 274
ccacccacac ccaccgcgcc ctcgttcgcc tcttctccgg gagccagtcc gcgccaccgc 60
cgccgcccag gccatcgcca ccctccgcag ccatgtccac caggtccgtg tcctcgtcct 120
cctaccgcag gatgttcggc ggcccgggca ccgcgagccg gccgagctcc agccggagct 180
acgtgactac gtccacccgc acctacagcc tgggcagcgc gctgcgcccc agcaccagcc 240
gcagcctcta cgcctcgtcc ccgggcggcg tgtatgccac gcgctcctct gccgtgcgcc 300
tgcggagcag cgtgcccggg gtgcggctcc tgcaggactc ggtggacttc tcgctggccg 360
acgccatcaa caccgagttc aagaacaccc gcaccaacga g 401

<210> 275
<211> 401
<212> DNA
<213> Homo sapiens

<400> 275
ccacttccac cactttgtgg agcagtgcct tcagcgcaac ccggatgcca ggtatccctg 60
ctggcctggg cctgggcttc gggagagcag agggtgctca ggagggtaag gccagggtgt 120
gaagggactt acctcccaaa ggttctgcag gggaatctgg agctacacac aggagggatc 180
agctcctggg tgtgtcagag gccagcctgg ggagctctgg ccactgcttc ccatgagctg 240
agggagaggg agaggggacc cgaggctgag gcataagtgg caggatttcg ggaagctggg 300
gacacggcag tgatgctgcg gtctctcctc cccttccct ccaggcccag tgccagcacc 360

ctcctgaacc actctttctt caagcagatc aagcgacgtg c 401

<210> 276
<211> 401
<212> DNA
<213> Homo sapiens

<220>
<221> misc_feature
<222> 11
<223> n = A,T,C or G

<400> 276
tctgatattg ntacccttga gccacctaag ttagaagaaa ttggaaatca agaagttgtc 60
attgttgaag aagcacagag ttcagaagac tttaacatgg gctcttcctc tagcagccag 120
tatactttct gtcagccaga aactgtattt tcatctcagc ctagtgatga tgaatcaagt 180
agtgatgaaa ccagtaatca gcccagtcct gcctttagac gacgccgtgc taggaagaag 240
accgtttctg cttcagaatc tgaagaccgg ctagttggtg aacaagaaac tgaaccttct 300
aaggagttga gtaaacgtca gttcagtagt ggtctcaata agtgtgttat acttgctttg 360
gtgattgcaa tcagcatggg atttggccat ttctatggca c 401

<210> 277
<211> 401
<212> DNA
<213> Homo sapiens

<220>
<221> misc_feature
<222> 227, 333
<223> n = A,T,C or G

<400> 277
aactttggca acatatctca gcaaaaacta cagctatgtt attcatgcca aaataaaagc 60
tgtgcagagg agtggctgca atgaggtcac aacggtggtg gatgtaaaag agatcttcaa 120
gtcctcatca cccatccctc gaactcaagt cccgctcatt acaaattctt cttgccagtg 180
tccacacatc ctgccccatc aagatgttct catcatgtgt tacgagnggc gctcaaggat 240
gatgcttctt gaaaattgct tagttgaaaa atggagagat cagcttagta aaagatccat 300
acagtgggaa gagaggctgc aggaacagcg ganaacagtt caggacaaga agaaaacagc 360
cgggcgcacc agtcgtagta atccccccaa accaaaggga a 401

<210> 278
<211> 401
<212> DNA
<213> Homo sapiens

<220>
<221> misc_feature
<222> 322, 354
<223> n = A,T,C or G

<400> 278
aatgagtgtg agaccacaaa tgaatgccgg gaggatgaaa tgtgttggaa ttatcatggc 60
ggcttccgtt gttatccacg aaatccttgt caagatccct acattctaac accagagaac 120
cgatgtgttt gcccagtctc aaatgccatg tgccgagaac tgccccagtc aatagtctac 180
aaatacatga gcatccgatc tgataggtct gtgccatcag acatcttcca gatacaggcc 240
acaactattt atgccaacac catcaatact tttcggatta aatctggaaa tgaaaatgga 300
gagtctacct acgacaacaa anccctgtaa gtgcaatgct tgtgctcgtg aagncattat 360
caggaccaag agaacatatc gtggacctgg agatgctgac a 401

<210> 279
<211> 401
<212> DNA
<213> Homo sapiens

<220>
<221> misc_feature
<222> 30, 35, 81, 88, 180, 212, 378, 384, 391
<223> n = A,T,C or G

<400> 279
aaattattgc ctctgataca tacctaagtn aacanaacat taatacctaa gtaaacataa 60
cattacttgg agggttgcag nttctaantg aaactgtatt tgaaactttt aagtatactt 120
taggaaacaa gcatgaacgg cagtctagaa taccagaaac atctacttgg gtagcttggn 180
gccattatcc tgtggaatct gatatgtctg gnagcatgtc attgatggga catgaagaca 240
tctttggaaa tgatgagatt atttcctgtg ttaaaaaaaa aaaaaatctt aaattcctac 300
aatgtgaaac tgaaactaat aattttgatc ctgatgtatg ggacagcgta tctgtaccag 360
gctctaaata acaaaagnta gggngacaag nacatgttcc t 401

<210> 280
<211> 326
<212> DNA
<213> Homo sapiens

<400> 280
   gaagtggaat tgtataattc aattcgataa ttgatctcat gggctttccc tggaggaaag 60
   gtttttttgg ttgttttttt tttaagaact tgaaacttgt aaactgagat gtctgtagct 120
   tttttgccca tctgtagtgt atgtgaagat ttcaaaacct gagagcactt tttctttgtt 180
   tagaattatg agaaaggcac tagatgactt taggatttgc atttttccct ttattgcctc 240
   atttcttgtg acgccttgtt ggggagggaa atctgtttat tttttcctac aaataaaaag 300
   ctaagattct atatcgcaaa aaaaaa 326

<210> 281
<211> 374
<212> DNA
<213> Homo sapiens

<400> 281
caacgcgttt gcaaatattc ccctggtagc ctacttcctt acccccgaat attggtaaga 60
tcgagcaatg gcttcaggac atgggttctc ttctcctgtg atcattcaag tgctcactgc 120
atgaagactg gcttgtctca gtgtttcaac ctcaccaggg ctgtctcttg gtccacacct 180
cgctccctgt tagtgccgta tgacagcccc catcaaatga ccttggccaa gtcacggttt 240
ctctgtggtc aaggttggtt ggctgattgg tggaaagtag ggtggaccaa aggaggccac 300
gtgagcagtc agcaccagtt ctgcaccagc agcgcctccg tcctagtggg tgttcctgtt 360
tctcctggcc ctgg 374

<210> 282
<211> 404
<212> DNA
<213> Homo sapiens

<220>
<221> misc_feature
<222> 26, 27, 51, 137, 180, 222
<223> n = A,T,C or G

<400> 282

agtgtggtgg aattcccgca tcctanncgc cgactcacac aaggcagagt ngccatggag 60
aaaattccag tgtcagcatt cttgctcctt gtggccctct cctacactct ggccagagat 120
accacagtca aacctgnagc caaaaaggac acaaaggact ctcgacccaa actgccccan 180
accctctcca gaggttgggg tgaccaactc atctggactc anacatatga agaagctcta 240
tataaatcca agacaagcaa caaacccttg atgattattc atcacttgga tgagtgccca 300
cacagtcaag ctttaaagaa agtgtttgct gaaaataaag aaatccagaa attggcagag 360
cagtttgtcc tcctcaatct ggtttatgaa acaactgaca aaca 404

<210> 283
<211> 184
<212> DNA
<213> Homo sapiens

<220>
<221> misc_feature
<222> 26
<223> n = A,T,C or G

<400> 283
agtgtggtgg aattcacttg cttaanttgt gggcaaaaga gaaaaagaag gattgatcag 60
agcattgtgc aatacagttt cattaactcc ttccctcgct cccccaaaaa tttgaatttt 120
tttttcaaca ctcttacacc tgttatggaa aatgtcaacc tttgtaagaa aaccaaaata 180
aaaa 184

<210> 284
<211> 421
<212> DNA
<213> Homo sapiens

<220>
<221> misc_feature
<222> 147, 149
<223> n = A,T,C or G

<400> 284
ctattaatcc tgccacaata tttttaatta cgtacaaaga tctgacatgt cacccaggga 60
cccatttcac ccactgctct gtttggccgc cagtcttttg tctctctctt cagcaatggt 120
gaggcggata ccctttcctc ggggaanana aatccatggt ttgttgccct tgccaataac 180
aaaaatgttg gaaagtcgag tggcaaagct gttgccattg gcatctttca cgtgaaccac 240
gtcaaaagat ccagggtgcc tctctctgtt ggtgatcaca ccaattcttc ctaggttagc 300
acctccagtc accatacaca ggttaccagt gtcgaacttg atgaaatcag taatcttgcc 360
agtctctaaa tcaatctgaa tggtatcatt caccttgatg aggggatcgg ggtagcggat 420
g 421

<210> 285
<211> 361
<212> DNA
<213> Homo sapiens

<220>
<221> misc_feature
<222> 34, 188
<223> n = A,T,C or G

<400> 285
ctgggtggta actctttatt tcattgtccg gaanaaagat gggagtggga acagggtgga 60
cactgtgcag gcttcagctt ccactccggg caggattcag gctatctggg accgcaggga 120
ctgccaggtg cacagccctg gctcccgagg caggcaggca aggtgacggg actggaagcc 180

cttttcanag ccttggagga gctggtccgt ccacaagcaa tgagtgccac tctgcagttt 240
gcaggggatg gataaacagg gaaacactgt gcattcctca cagccaacag tgtaggtctt 300
ggtgaagccc cggcgctgag ctaagctcag gctgttccag ggagccacga aactgcaggt 360
a 361

<210> 286
<211> 336
<212> DNA
<213> Homo sapiens

<220>
<221> misc_feature
<222> 40, 68, 75, 127, 262
<223> n = A,T,C or G

<400> 286
tttgagtggc agcgccttta tttgtggggg ccttcaaggn agggtcgtgg ggggcagcgg 60
ggaggaanag ccganaaact gtgtgaccgg ggcctcaggt ggtgggcatt gggggctcct 120
cttgcanatg cccattggca tcaccggtgc agccattggt ggcagcgggt accggtcctt 180
tcttgttcaa catagggtag gtggcagcca cgggtccaac tcgcttgagg ctgggccctg 240
ggcgctccat tttgtgttcc angagcatgt ggttctgtgg cgggagcccc acgcaggccc 300
tgaggatgtt ctcgatgcag ctgcgctggc ggaaaa 336

<210> 287
<211> 301
<212> DNA
<213> Homo sapiens

<220>
<221> misc_feature
<222> 15, 33, 44, 53, 76, 83, 107, 117, 154, 166, 192, 194, 207,
215, 241, 246
<223> n = A,T,C or G

<400> 287
tgggtaccaa atttntttat ttgaaggaat ggnacaaatc aaanaactta agnggatgtt 60
ttggtacaac ttatanaaaa ggnaaaggaa accccaacat gcatgcnctg ccttggngac 120
cagggaagtc accccacggc tatggggaaa ttancccgag gcttancttt cattatcact 180
gtctcccagg gngngcttgt caaaaanata ttccnccaag ccaaattcgg gcgctcccat 240
nttgcncaag ttggtcacgt ggtcacccaa ttctttgatg gctttcacct gctcattcag 300
g 301

<210> 288
<211> 358
<212> DNA
<213> Homo sapiens

<220>
<221> misc_feature
<222> 39, 143, 226
<223> n = A,T,C or G

<400> 288
aagtttttaa actttttatt tgcatattaa aaaaattgng cattccaata attaaaatca 60
tttgaacaaa aaaaaaaatg gcactctgat taaactgcat tacagcctgc aggacacctt 120
gggccagctt ggttttactc tanatttcac tgtcgtccca ccccacttct tccaccccac 180
ttcttccttc accaacatgc aagttctttc cttccctgcc agccanatag atagacagat 240
gggaaaggca ggcgcggcct tcgttgtcag tagttctttg atgtgaaagg ggcagcacag 300

tcatttaaac ttgatccaac ctctttgcat cttacaaagt taaacagcta aaagaagt 358

<210> 289
<211> 462
<212> DNA
<213> Homo sapiens

<220>
<221> misc_feature
<222> 87, 141, 182, 220, 269, 327
<223> n = A,T,C or G

<400> 289
ggcatcagaa atgctgttta tttctctgct gctcccaagc tggctggcct ttgcagagga 60
gcagacaaca gatgcatagt tggggganaaa gggaggacag gttccaggat agagggtgca 120
ggctgaggga ggaagggtaa naggaaggaa ggccatcctg gatccccaca tttcagtctc 180
anatgaggac aaagggactc ccaagccccc aaatcatcan aaaacaccaa ggagcaggag 240
gagcttgagc aggccccagg gagcctcana gccataccag ccactgtcta cttcccatcc 300
tcctctccca ttccctgtct gcttcanacc acctcccagc taagccccag ctccattccc 360
ccaatcctgg cccttgccag cttgacagtc acagtgcctg gaattccacc actgaggctt 420
ctcccagttg gattaggacg tcgccctgtt agcatgctgc cc 462

<210> 290
<211> 481
<212> DNA
<213> Homo sapiens

<220>
<221> misc_feature
<222> 44, 57, 122, 158, 304, 325, 352, 405
<223> n = A,T,C or G

<400> 290
tactttccta aactttatta aagaaaaaag caataagcaa tggnggtaaa tctctanaac 60
atacccaatt ttctgggctt cctcccccga gaatgtgaca ttttgatttc caaacatgcc 120
anaagtgtat ggttcccaac tgtactaaag taggtganaa gctgaagtcc tcaagtgttc 180
atcttccaac ttttcccagt ctgtggtctg tctttggatc agcaataatt gcctgaacag 240
ctactatggc ttcgttgatt tttgtctgta gctctctgag ctcctctatg tgcagcaatc 300
gcanaatttg agcagcttca ttaanaactg catctcctgt gtcaaaacca anaatatgtt 360
tgtctaaagc aacaggtaag ccctcttttg tttgatttgc cttancaact gcatcctgtg 420
tcaggcgctc ctgaaccaaa atccgaattg ccttaagcat taccaggtaa tcatcatgac 480
g 481

<210> 291
<211> 381
<212> DNA
<213> Homo sapiens

<220>
<221> misc_feature
<222> 79, 166, 187, 208, 219, 315
<223> n = A,T,C or G

<400> 291
tcatagtaat gtaaaaccat ttgtttaatt ctaaatcaaa tcactttcac aacagtgaaa 60
attagtgact ggttaaggng tgccactgta catatcatca ttttctgact ggggtcagga 120
cctggtccta gtccacaagg gtggcaggag gagggtggag gctaanaaca cagaaaacac 180
acaaaanaaa ggaaagctgc cttggcanaa ggatgaggng gtgagcttgc cgaaggatgg 240

tgggaagggg gctccctgtt ggggccgagc caggagtccc aagtcagctc tcctgcctta 300
cttagctcct ggcanagggt gagtggggac ctacgaggtt caaaatcaaa tggcatttgg 360
ccagcctggc tttactaaca g 381

<210> 292
<211> 371
<212> DNA
<213> Homo sapiens

<220>
<221> misc_feature
<222> 32, 55, 72, 151, 189, 292
<223> n = A,T,C or G

<400> 292
gaaaaaataa tccgtttaat tgaaaaacct gnaggatact attccactcc cccanatgag 60
gaggctgagg anaccaaacc cctacatcac ctcgtagcca cttctgatac tcttcacgag 120
gcagcaggca aagacaattc ccaaaacctc nacaaaagca attccaaggg ctgctgcagc 180
taccaccanc acatttttcc tcagccagcc cccaatcttc tccacacagc cctccttatg 240
gatcgccttc tcgttgaaat taatcccaca gcccacagta acattaatgc ancaggagtc 300
ggggactcgg ttcttcgaca tggaagggat tttctcccaa tctgtgtagt tagcagcccc 360
acagcactta a 371

<210> 293
<211> 361
<212> DNA
<213> Homo sapiens

<220>
<221> misc_feature
<222> 75, 196, 222
<223> n = A,T,C or G

<400> 293
gatttaaaag aaaacacttt attgttcagc aattaaaagt tagccaaata tgtatttttc 60
tccataattt attgngatgt tatcaacatc aagtaaaatg ctcattttca tcatttgctt 120
ctgttcatgt tttcttgaac acgtcttcaa ttttccttcc aaaatgctgc atgccacact 180
tgaggtaacg aagcanaagt atttttaaac atgacagcta anaacattca tctacagcaa 240
cctatatgct caatacatgc cgcgtgatcc tagtagtttt ttcacaacct tctacaagtt 300
tttggaaaac atctgttatg atgactttca tacaccttca cctcaaaggc tttcttgcac 360
c 361

<210> 294
<211> 391
<212> DNA
<213> Homo sapiens

<220>
<221> misc_feature
<222> 26, 77, 96, 150, 203, 252, 254, 264, 276
<223> n = A,T,C or G

<400> 294
   tattttaaag tttaattatg attcanaaaa aatcgagcga ataactttct ctgaaaaaat 60
   atattgactc tgtatanacc acagttattg gggganaagg gctggtaggt taaattatcc 120
   tattttttat tctgaaaatg atattaatan aaagtcccgt ttccagtctg attataaaga 180
   tacatatgcc caaaatggct ganaataaat acaacaggaa atgcaaaagc tgtaaagcta 240
   agggcatgca ananaaaatc tcanaatacc caaagnggca acaaggaacg tttggctgga 300
   atttgaagtt atttcagtca tctttgtctt tggctccatg tttcaggatg cgtgtgaact 360
   cgatgtaatt gaaattcccc tttttatcaa t 391

<210> 295
<211> 343
<212> DNA
<213> Homo sapiens

<220>
<221> misc_feature
<222> 145, 174, 205, 232
<223> n = A,T,C or G

<400> 295
ttcttttgtt ttattgataa cagaaactgt gcataattac agatttgatg aggaatctgc 60
aaataataaa gaatgtgtct actgccagca aaatacaatt attccatgcc ctctcaacat 120
acaaatatag agttcttcac accanatggc tctggtgtaa caaagccatt ttanatgttt 180
aattgtgctt ctacaaaacc ttcanagcat gaggtagttt cttttaccta cnatattttc 240
cacatttcca ttattacact tttagtgagc taaaatcctt ttaacatagc ctgcggatga 300
tctttcacaa aagccaagcc tcatttacaa agggtttatt tct 343

<210> 296
<211> 241
<212> DNA
<213> Homo sapiens

<220>
<221> misc_feature
<222> 96, 98, 106, 185
<223> n = A,T,C or G

<400> 296
ttcttggata ttggttgttt ttgtgaaaaa gtttttgttt ttcttctcag tcaactgaat 60
tatttctcta ctttgccctc ctgatgccca catgananaa cttaanataa tttctaacag 120
cttccacttt ggaaaaaaaa aaaacctgtt ttcctcatgg aaccccagga gttgaaagtg 180
gatanatcgc tctcaaaatc taaggctctg ttcagcttta cattatgtta cctgacgttt 240
t 241

<210> 297
<211> 391
<212> DNA
<213> Homo sapiens

<220>
<221> misc_feature
<222> 12, 130
<223> n = A,T,C or G

<400> 297
gttgtggctg anaatgctgg agatgctcag ttctctccct cacaaggtag gccacaaatt 60
cttggtggtg ccctcacatc tggggtcttc aggcaccagc catgcctgcc gaggagtgct 120
gtcaggacan accatgtccg tgctaggccc aggcacagcc caaccactcc tcatccaagt 180
ctctcccagg tttctggtcc cgatgggcaa ggatgacccc tccagtggct ggtaccccac 240
catcccacta cccctcacat gctctcactc tccatcaggt ccccaatcct ggcttccctc 300
ttcacgaact ctcaaagaaa aggaaggata aaacctaaat aaaccagaca gaagcagctc 360
tggaaaagta caaaaagaca gccagaggtg t 391

<210> 298

<211> 321
<212> DNA
<213> Homo sapiens

<220>
<221> misc_feature
<222> 14, 30, 76, 116, 201, 288, 301
<223> n = A,T,C or G

<400> 298
caagccaaac tgtntccagc tttattaaan atactttcca taaacaatca tggtatttca 60
ggcaggacat gggcanacaa tcgttaacag tatacaacaa ctttcaaact cccttnttca 120
atggactacc aaaaatcaaa aagccactat aaaacccaat gaagtcttca tctgatgctc 180
tgaacaggga aagtttaaag ngagggttga catttcacat ttagcatgtt gtttaacaac 240
ttttcacaag ccgaccctga ctttcaggaa gtgaaatgaa aatggcanaa tttatctgaa 300
natccacaat ctaaaaatgg a 321

<210> 299
<211> 401
<212> DNA
<213> Homo sapiens

<220>
<221> misc_feature
<222> 104, 268, 347
<223> n = A,T,C or G

<400> 299
tatcataaag agtgttgaag tttatttatt atagcaccat tgagacattt tgaaattgga 60
attggtaaaa aaataaaaca aaaagcattt gaattgtatt tggnggaaca gcaaaaaaag 120
agaagtatca tttttctttg tcaaattata ctgtttccaa acattttgga aataaataac 180
tggaattttg tcggtcactt gcactggttg acaagattag aacaagagga acacatatgg 240
agttaaattt tttttgttgg gatttcanat agagtttggt ttataaaaag caaacagggc 300
caacgtccac accaaattct tgatcaggac caccaatgtc atagggngca atatctacaa 360
taggtagtct cacagccttg cgtgttcgat attcaaagac t 401

<210> 300
<211> 188
<212> DNA
<213> Homo sapiens

<220>
<221> misc_feature
<222> 48
<223> n = A,T,C or G

<400> 300
tgaatgcttt gtcatattaa gaaagttaaa gtgcaataat gtttgaanac aataagtggt 60
ggtgtatctt gtttctaata agataaactt ttttgtcttt gctttatctt attagggagt 120
tgtatgtcag tgtataaaac atactgtgtg gtataacagg cttaataaat tctttaaaag 180
gaaaaaaa 188

<210> 301
<211> 291
<212> DNA
<213> Homo sapiens

<400> 301

aagattttgt tttattttat tatggctaga aagacactgt tatagccaaa atcggcaatg 60
acactaaaga aatcctctgt gcttttcaat atgcaaatat atttcttcca agagttgccc 120
tggtgtgact tcaagagttc atgttaactt cttttctgga aacttccttt tcttagttgt 180
tgtattcttg aagagcctgg gccatgaaga gcttgcctaa gttttgggca gtgaactcct 240
tgatgttctg gcagtaagtg tttatctggc ctgcaatgag cagcgagtcc a 291

<210> 302
<211> 341
<212> DNA
<213> Homo sapiens

<220>
<221> misc_feature
<222> 25
<223> n = A,T,C or G

<400> 302
tgatttttca taattttatt aaatnatcac tgggaaaact aatggttcgc gtatcacaca 60
attacactac aatctgatag gagtggtaaa accagccaat ggaatccagg taaagtacaa 120
aaacgccacc ttttattgtc ctgtcttatt tctcgggaag gagggttcta ctttacacat 180
ttcatgagcc agcagtggac ttgagttaca atgtgtaggt tccttgtggt tatagctgca 240
gaagaagcca tcaaattctt gaggacttga catctctcgg aaagaagcaa actagtggat 300
cccccgggct gcaggaattc gatatcaagc ttatcgatac c 341

<210> 303
<211> 361
<212> DNA
<213> Homo sapiens

<220>
<221> misc_feature
<222> 15, 27, 92, 124, 127, 183, 198, 244, 320
<223> n = A,T,C or G

<400> 303
tgcagacagt aaatnaattt tatttgngtt cacagaacat actaggcgat ctcgacagtc 60
gctccgtgac agcccaccaa cccccaaccc tntacctcgc agccacccta aaggcgactt 120
caanaanatg gaaggatctc acggatctca ttcctaatgg tccgccgaag tctcacacag 180
tanacagacg gagttganat gctggaggat gcagtcacct cctaaactta cgacccacca 240
ccanacttca tcccagccgg gacgtcctcc cccacccgag tcctccccat ttcttctcct 300
actttgccgc agttccaggn gtcctgcttc caccagtccc acaaagctca ataaatacca 360
a 361

<210> 304
<211> 301
<212> DNA
<213> Homo sapiens

<220>
<221> misc_feature
<222> 23, 104, 192
<223> n = A,T,C or G

<400> 304
ctctttacaa cagcctttat ttncggccct tgatcctgct cggatgctgg tggaggccct 60
tagctccgcc cgccaggctc tgtgccgcct ccccgcaggc gcanattcat gaacacggtg 120
ctcaggggct tgaggccgta ctcccccagc gggagctggt cctccagggg cttcccctcg 180
aaggtcagcc anaacaggtc gtcctgcaca ccctccagcc cgctcacttg ctgcttcagg 240

tgggccacgg tctgcgtcag ccgcacctcg taggtgctgc tgcggccctt gttattcctc 300
a 301

<210> 305
<211> 331
<212> DNA
<213> Homo sapiens

<220>
<221> misc_feature
<222> 3, 36, 60, 193, 223
<223> n = A,T,C or G

<400> 305
ganaggctag taacatcagt tttattgggt tggggnggca accatagcct ggctgggggn 60
ggggctggcc ctcacaggtt gttgagttcc agcagggtct ggtccaaggt ctggtgaatc 120
tcgacgttct cctccttggc actggccaag gtctcttcta ggtcatcgat ggttttctcc 180
aactttgcca canacctctc ggcaaactct gctcgggtct cancctcctt cagcttctcc 240
tccaacagtt tgatctcctc ttcatattta tcttctttgg gggaatactc ctcctctgag 300
gccatcaggg acttgagggc ctggtccatg g 331

<210> 306
<211> 457
<212> DNA
<213> Homo sapiens

<400> 306
aatatgtaaa ggtaataact tttattatat taaagacaat gcaaacgaaa aacagaattg 60
agcagtgcaa aatttaaagg actgttttgt tctcaaagtt gcaagtttca aagccaaaag 120
aattatatgt atcaaatata taagtaaaaa aaagttagac tttcaagcct gtaatcccag 180
cactttggga ggctgaggca ggtggatcac taacattaaa aagacaacat tagattttgt 240
cgatttatag caattttata aatatataac tttgtcactt ggatcctgaa gcaaaataat 300
aaagtgaatt tgggattttt gtacttggta aaaagtttaa caccctaaat tcacaactag 360
tggatccccc gggctgcagg aattcgatat caagcttatc gataccgtcg acctcgaggg 420
ggggcccggt acccaattcg ccctatagtg agtcgta 457

<210> 307
<211> 491
<212> DNA
<213> Homo sapiens

<400> 307
gtgcttggac ggaacccggc gctcgttccc caccccggcc ggccgcccat agccagccct 60
ccgtcacctc ttcaccgcac cctcggactg ccccaaggcc cccgccgccg ctccagcgcc 120
gcgcagccac cgccgccgcc gccgcctctc cttagtcgcc gccatgacga ccgcgtccac 180
ctcgcaggtg cgccagaact accaccagga ctcagaggcc gccatcaacc gccagatcaa 240
cctggagctc tacgcctcct acgtttacct gtccatgtct tactactttg accgcgatga 300
tgtggctttg aagaactttg ccaaatactt tcttcaccaa tctcatgagg agagggaaca 360
tgctgagaaa ctgatgaagc tgcagaacca acgaggtggc cgaatcttcc ttcaggatat 420
caagaaacca gactgtgatg actgggagag cgggctgaat gcaatggagt gtgcattaca 480
tttggaaaaa a 491

<210> 308
<211> 421
<212> DNA
<213> Homo sapiens

<400> 308

ctcagcgctt cttctttctt ggtttgatcc tgactgctgt catggcgtgc cctctggaga 60
aggccctgga tgtgatggtg tccaccttcc acaagtactc gggcaaagag ggtgacaagt 120
tcaagctcaa caagtcagaa ctaaaggagc tgctgacccg ggagctgccc agcttcttgg 180
ggaaaaggac agatgaagct gctttccaga agctgatgag caacttggac agcaacaggg 240
acaacgaggt ggacttccaa gagtactgtg tcttcctgtc ctgcatcgcc atgatgtgta 300
acgaattctt tgaaggcttc ccagataagc agcccaggaa gaaatgaaaa ctcctctgat 360
gtggttgggg ggtctgccag ctggggccct ccctgtcgcc agtgggcact tttttttttc 420
c 421

<210> 309
<211> 321
<212> DNA
<213> Homo sapiens

<400> 309
accaaatggc ggatgacgcc ggtgcagcgg gggggcccgg gggccctggt ggccctggga 60
tggggaaccg cggtggcttc cgcggaggtt tcggcagtgg catccggggc cggggtcgcg 120
gccgtggacg gggccggggc cgaggccgcg gagctcgcgg aggcaaggcc gaggataagg 180
agtggatgcc cgtcaccaag ttgggccgct tggtcaagga catgaagatc aagtccctgg 240
aggagatcta tctcttctcc ctgcccatta aggaatcaga gatcattgat ttcttcctgg 300
gggcctctct caaggatgag g 321

<210> 310
<211> 381
<212> DNA
<213> Homo sapiens

<400> 310
ttaaccagcc atattggctc aataaatagc ttcggtaagg agttaatttc cttctagaaa 60
tcagtgccta tttttcctgg aaactcaatt ttaaatagtc caattccatc tgaagccaag 120
ctgttgtcat tttcattcgg tgacattctc tcccatgaca cccagaaggg gcagaagaac 180
cacatttttc atttatagat gtttgcatcc tttgtattaa aattattttg aaggggttgc 240
ctcattggat ggcttttttt tttttcctcc agggagaagg ggagaaatgt acttggaaat 300
taatgtatgt ttacatctct ttgcaaattc ctgtacatag agatatattt tttaagtgtg 360
aatgtaacaa catactgtga a 381

<210> 311
<211> 538
<212> DNA
<213> Homo sapiens

<400> 311
tttgaattta caccaagaac ttctcaataa aagaaaatca tgaatgctcc acaatttcaa 60
cataccacaa gagaagttaa tttcttaaca ttgtgttcta tgattatttg taagaccttc 120
accaagttct gatatctttt aaagacatag ttcaaaattg cttttgaaaa tctgtattct 180
tgaaaatatc cttgttgtgt attaggtttt taaataccag ctaaaggatt acctcactga 240
gtcatcagta ccctcctatt cagctcccca agatgatgtg tttttgctta ccctaagaga 300
ggttttcttc ttatttttag ataattcaag tgcttagata aattatgttt tctttaagtg 360
tttatggtaa actcttttaa agaaaattta atatgttata gctgaatctt tttggtaact 420
ttaaatcttt atcatagact ctgtacatat gttcaaatta gctgcttgcc tgatgtgtgt 480
atcatcggtg ggatgacaga acaaacatat ttatgatcat gaataatgtg ctttgtaa 538

<210> 312
<211> 176
<212> DNA
<213> Homo sapiens

<400> 312

ggaggagcag ctgagagata gggtcagtga atgcggttca gcctgctacc tctcctgtct 60
tcatagaacc attgccttag aattattgta tgacacgttt tttgttggtt aagctgtaag 120
gttttgttct ttgtgaacat gggtattttg aggggagggt ggagggagta gggaag 176

<210> 313
<211> 396
<212> DNA
<213> Homo sapiens

<400> 313
   ccagcacccc caggccctgg gggacctggg ttctcagact gccaaagaag ccttgccatc 60
   tggcgctccc atggctcttg caacatctcc ccttcgtttt tgagggggtc atgccggggg 120
   agccaccagc ccctcactgg gttcggagga gagtcaggaa gggccaagca cgacaaagca 180
   gaaacatcgg atttggggaa cgcgtgtcaa tcccttgtgc cgcagggctg ggcgggagag 240
   actgttctgt tccttgtgta actgtgttgc tgaaagacta cctcgttctt gtcttgatgt 300
   gtcaccgggg caactgcctg ggggcgggga tgggggcagg gtggaagcgg ctccccattt 360
   tataccaaag gtgctacatc tatgtgatgg gtgggg 396

<210> 314
<211> 311
<212> DNA
<213> Homo sapiens

<400> 314
   cctcaacatc ctcagagagg actggaagcc agtccttacg ataaactcca taatttatgg 60
   cctgcagtat ctcttcttgg agcccaaccc cgaggaccca ctgaacaagg aggccgcaga 120
   ggtcctgcag aacaaccggc ggctgtttga gcagaacgtg cagcgctcca tgcggggtgg 180
   ctacatcggc tccacctact ttgagcgctg cctgaaatag ggttggcgca tacccacccc 240
   cgccacggcc acaagccctg gcatcccctg caaatattta ttgggggcca tgggtagggg 300
   tttgggggcg g 311

<210> 315
<211> 336
<212> DNA
<213> Homo sapiens

<400> 315
tttagaacat ggttatcatc caagactact ctaccctgca acattgaact cccaagagca 60
aatccacatt cctcttgagt tctgcagctt ctgtgtaaat agggcagctg tcgtctatgc 120
cgtagaatca catgatctga ggaccattca tggaagctgc taaatagcct agtctgggga 180
gtcttccata aagttttgca tggagcaaac aaacaggatt aaactaggtt tggttccttc 240
agccctctaa aagcataggg cttagcctgc aggcttcctt gggctttctc tgtgtgtgta 300
gttttgtaaa cactatagca tctgttaaga tccagt 336

<210> 316
<211> 436
<212> DNA
<213> Homo sapiens

<400> 316
aacatggtct gcgtgcctta agagagacgc ttcctgcaga acaggacctg actacaaaga 60
atgtttccat tggaattgtt ggtaaagact tggagtttac aatctatgat gatgatgatg 120
tgtctccatt cctggaaggt cttgaagaaa gaccacagag aaaggcacag cctgctcaac 180
ctgctgatga acctgcagaa aaggctgatg aaccaatgga acattaagtg ataagccagt 240
ctatatatgt attatcaaat atgtaagaat acaggcacca catactgatg acaataatct 300
atactttgaa ccaaaagttg cagagtggtg gaatgctatg ttttaggaat cagtccagat 360
gtgagttttt tccaagcaac ctcactgaaa cctatataat ggaatacatt tttctttgaa 420
agggtctgta taatca 436

<210> 317
<211> 196
<212> DNA
<213> Homo sapiens

<400> 317
   tattccttgt gaagatgata tactattttt gttaagcgtg tctgtattta tgtgtgagga 60
   gctgctggct tgcagtgcgc gtgcacgtgg agagctggtg cccggagatt ggacggcctg 120
   atgctccctc ccctgccctg gtccagggaa gctggccgag ggtcctggct cctgaggggc 180
   atctgcccct ccccca 196

<210> 318
<211> 381
<212> DNA
<213> Homo sapiens

<220>
<221> misc_feature
<222> 8, 9, 102, 122, 167, 182, 193, 235, 253, 265, 266, 290, 321,
378
<223> n = A,T,C or G

<400> 318
gacgcttnng ccgtaacgat gatcggagac atcctgctgt tcgggacgtt gctgatgaat 60
gccggggcgg tgctgaactt taagctgaaa aagaaggaca cncagggctt tggggaggag 120
tncagggagc ccaacacagg tgacaacatc cgggaattct tgctgancct cagatacttt 180
cnaatcttca tcnccctgtg gaacatcttc atgatgttct gcatgattgt gctgntcggc 240
tcttgaatcc cancgatgaa accannaact cactttcccg ggatgccgan tctccattcc 300
tccattcctg atgacttcaa naatgttttt gaccaaaaaa ccgacaacct tcccagaaag 360
tccaagctcg tggtgggngg a 381

<210> 319
<211> 506
<212> DNA
<213> Homo sapiens

<400> 319
ctaagcttta cgaatggggt gacaacttat gataaaaact agagctagtg aattagccta 60
tttgtaaata cctttgttat aattgatagg atacatcttg gacatggaat tgttaagcca 120
cctctgagca gtgtatgtca ggacttgttc attaggttgg cagcagaggg gcagaaggaa 180
ttatacaggt agagatgtat gcagatgtgt ccatatatgt ccatatttac attttgatag 240
ccattgatgt atgcatctct tggctgtact ataagaacac attaattcaa tggaaataca 300
ctttgctaat attttaatgg tatagatctg ctaatgaatt ctcttaaaaa catactgtat 360
tctgttgctg tgtgtttcat tttaaattga gcattaaggg aatgcagcat ttaaatcaga 420
actctgccaa tgcttttatc tagaggcgtg ttgccatttt tgtcttatat gaaatttctg 480
tcccaagaaa ggcaggatta catctt 506

<210> 320
<211> 351
<212> DNA
<213> Homo sapiens

<400> 320
ctgacctgca ggacgaaacc atgaagagcc tgatccttct tgccatcctg gccgccttag 60
cggtagtaac tttgtgttat gaatcacatg aaagcatgga atcttatgaa cttaatccct 120
tcattaacag gagaaatgca aataccttca tatcccctca gcagagatgg agagctaaag 180
tccaagagag gatccgagaa cgctctaagc ctgtccacga gctcaatagg gaagcctgtg 240

atgactacag actttgcgaa cgctacgcca tggtttatgg atacaatgct gcctataatc 300
gctacttcag gaagcgccga gggaccaaat gagactgagg gaagaaaaaa a 351

<210> 321
<211> 421
<212> DNA
<213> Homo sapiens

<400> 321
ctcggaggcg ttcagctgct tcaagatgaa gctgaacatc tccttcccag ccactggctg 60
ccagaaactc attgaagtgg acgatgaacg caaacttcgt actttctatg agaagcgtat 120
ggccacagaa gttgctgctg acgctctggg tgaagaatgg aagggttatg tggtccgaat 180
cagtggtggg aacgacaaac aaggtttccc catgaagcag ggtgtcttga cccatggccg 240
tgtccgcctg ctactgagta aggggcattc ctgttacaga ccaaggagaa ctggagaaag 300
aaagagaaaa tcagttcgtg gttgcattgt ggatgcaaat ctgagcgttc tcaacttggt 360
tattgtaaaa aaaggagaga aggatattcc tggactgact gatactacag tgcctcgccg 420
c 421

<210> 322
<211> 521
<212> DNA
<213> Homo sapiens

<400> 322
agcagctctc ctgccacagc tcctcacccc ctgaaaatgt tcgcctgctc caagtttgtc 60
tccactccct ccttggtcaa gagcacctca cagctgctga gccgtccgct atctgcagtg 120
gtgctgaaac gaccggagat actgacagat gagagcctca gcagcttggc agtctcatgt 180
ccccttacct cacttgtctc tagccgcagc ttccaaacca gcgccatttc aagggacatc 240
gacacagcag ccaagttcat tggagctggg gctgccacag ttggggtggc tggttctggg 300
gctgggattg gaactgtgtt tgggagcctc atcattggtt atgccaggaa cccttctctg 360
aagcaacagc tcttctccta cgccattctg ggctttgccc tctcggaggc catggggctc 420
ttttgtctga tggtagcctt tctcatcctc tttgccatgt gaaggagccg tctccacctc 480
ccatagttct cccgcgtctg gttggccccg tgtgttcctt t 521

<210> 323
<211> 435
<212> DNA
<213> Homo sapiens

<400> 323
   ccgaggtcgc acgcgtgaga cttctccgcc gcagacgccg ccgcgatgcg ctacgtcgcc 60
   tcctacctgc tggctgccct agggggcaac tcctccccca gcgccaagga catcaagaag 120
   atcttggaca gcgtgggtat cgaggcggac gacgaccggc tcaacaaggt tatcagtgag 180
   ctgaatggaa aaaacattga agacgtcatt gcccagggta ttggcaagct tgccagtgta 240
   cctgctggtg gggctgtagc cgtctctgct gccccaggct ctgcagcccc tgctgctggt 300
   tctgcccctg ctgcagcaga ggagaagaaa gatgagaaga aggaggagtc tgaagagtca 360
   gatgatgaca tgggatttgg cctttttgat taaattcctg ctcccctgca aataaagcct 420
   ttttacacat ctcaa 435

<210> 324
<211> 521
<212> DNA
<213> Homo sapiens

<400> 324
aggagatcga ctttcggtgc ccgcaagacc agggctggaa cgccgagatc acgctgcaga 60
tggtgcagta caagaatcgt caggccatcc tggcggtcaa atccacgcgg cagaagcagc 120
agcacctggt ccagcagcag cccccctcgc agccgcagcc gcagccgcag ctccagcccc 180

aaccccagcc tcagcctcag ccgcaacccc agccccaatc acaaccccag cctcagcccc 240
aacccaagcc tcagccccag cagctccacc cgtatccgca tccacatcca catccacact 300
ctcatcctca ctcgcaccca caccctcacc cgcacccgca tccgcaccaa ataccgcacc 360
cacacccaca gccgcactcg cagccgcacg ggcaccggct tctccgcagc acctccaact 420
ctgcctgaaa ggggcagctc ccgggcaaga caaggttttg aggacttgag gaagtgggac 480
gagcacattt ctattgtctt cacttggatc aaaagcaaaa c 521

<210> 325
<211> 451
<212> DNA
<213> Homo sapiens

<400> 325
attttcattt ccattaacct ggaagctttc atgaatattc tcttctttta aaacatttta 60
acattattta aacagaaaaa gatgggctct ttctggttag ttgttacatg atagcagaga 120
tatttttact tagattactt tgggaatgag agattgttgt cttgaactct ggcactgtac 180
agtgaatgtg tctgtagttg tgttagtttg cattaagcat gtataacatt caagtatgtc 240
atccaaataa gaggcatata cattgaattg tttttaatcc tctgacaagt tgactcttcg 300
acccccaccc ccacccaaga cattttaata gtaaatagag agagagagaa gagttaatga 360
acatgaggta gtgttccact ggcaggatga cttttcaata gctcaaatca atttcagtgc 420
ctttatcact tgaattatta acttaatttg a 451

<210> 326
<211> 421
<212> DNA
<213> Homo sapiens

<220>
<221> misc_feature
<222> 296
<223> n = A,T,C or G

<400> 326
cgcggtcgta agggctgagg atttttggtc cgcacgctcc tgctcctgac tcaccgctgt 60
tcgctctcgc cgaggaacaa gtcggtcagg aagcccgcgc gcaacagcca tggcttttaa 120
ggataccgga aaaacacccg tggagccgga ggtggcaatt caccgaattc gaatcaccct 180
aacaagccgc aacgtaaaat ccttggaaaa ggtgtgtgct gacttgataa gaggcgcaaa 240
agaaaagaat ctcaaagtga aaggaccagt tcgaatgcct accaagactt tgagantcac 300
tacaagaaaa actccttgtg gtgaaggttc taagacgtgg gatcgtttcc agatgagaat 360
tcacaagcga ctcattgact tgcacagtcc ttctgagatt gttaagcaga ttacttccat 420
c 421

<210> 327
<211> 456
<212> DNA
<213> Homo sapiens

<400> 327
atcttgacga ggctgcggtg tctgctgcta ttctccgagc ttcgcaatgc cgcctaagga 60
cgacaagaag aagaaggacg ctggaaagtc ggccaagaaa gacaaagacc cagtgaacaa 120
atccgggggc aaggccaaaa agaagaagtg gtccaaaggc aaagttcggg acaagctcaa 180
taacttagtc ttgtttgaca aagctaccta tgataaactc tgtaaggaag ttcccaacta 240
taaacttata accccagctg tggtctctga gagactgaag attcgaggct ccctggccag 300
ggcagccctt caggagctcc ttagtaaagg acttatcaaa ctggtttcaa agcacagagc 360
tcaagtaatt tacaccagaa ataccaaggg tggagatgct ccagctgctg gtgaagatgc 420
atgaataggt ccaaccagct gtacatttgg aaaaat 456

<210> 328

<211> 471
<212> DNA
<213> Homo sapiens

<400> 328
gtggaagtga catcgtcttt aaaccctgcg tggcaatccc tgacgcaccg ccgtgatgcc 60
cagggaagac agggcgacct ggaagtccaa ctacttcctt aagatcatcc aactattgga 120
tgattatccg aaatgtttca ttgtgggagc agacaatgtg ggctccaagc agatgcagca 180
gatccgcatg tcccttcgcg ggaaggctgt ggtgctgatg ggcaagaaca ccatgatgcg 240
caaggccatc cgagggcacc tggaaaacaa cccagctctg gagaaactgc tgcctcatat 300
ccgggggaat gtgggctttg tgttcaccaa ggaggacctc actgagatca gggacatgtt 360
gctggccaat aaggtgccag ctgctgcccg tgctggtgcc attgccccat gtgaagtcac 420
tgtgccagcc cagaacactg gtctcgggcc cgagaagacc tcctttttcc a 471

<210> 329
<211> 278
<212> DNA
<213> Homo sapiens

<220>
<221> misc_feature
<222> 154, 204
<223> n = A,T,C or G

<400> 329
gtttaaactt aagcttggta ccgagctcgg atccactagt ccagtgtggt ggaattctag 60
aaattgagat gcccccccag gccagcaaat gttccttttt gttcaaagtc tatttttatt 120
ccttgatatt tttctttttt tttttttttt ttgnggatgg ggacttgtga atttttctaa 180
aggtgctatt taacatggga gganagcgtg tgcggctcca gcccagcccg ctgctcactt 240
tccaccctct ctccacctgc ctctggcttc tcaggcct 278

<210> 330
<211> 338
<212> DNA
<213> Homo sapiens

<400> 330
ctcaggcttc aacatcgaat acgccgcagg ccccttcgcc ctattcttca tagccgaata 60
cacaaacatt attataataa acaccctcac cactacaatc ttcctaggaa caacatatga 120
cgcactctcc cctgaactct acacaacata ttttgtcacc aagaccctac ttctaacctc 180
cctgttctta tgaattcgaa cagcataccc ccgattccgc tacgaccaac tcatacacct 240
cctatgaaaa aacttcctac cactcaccct agcattactt atatgatatg tctccatacc 300
cattacaatc tccagcattc cccctcaaac ctaaaaaa 338

<210> 331
<211> 2820
<212> DNA
<213> Homo sapiens

<400> 331
   tggcaaaatc ctggagccag aagaaaggac agcagcattg atcaatctta cagctaacat 60
   gttgtacctg gaaaacaatg cccagactca atttagtgag ccacagtaca cgaacctggg 120
   gctcctgaac agcatggacc agcagattcg gaacggctcc tcgtccacca gtccctataa 180
   cacagaccac gcgcagaaca gcgtcacggc gccctcgccc tacgcacagc ccagccccac 240
   cttcgatgct ctctctccat cacccgccat cccctccaac accgactacc caggcccgca 300
   cagttccgac gtgtccttcc agcagtcgag caccgccaag tcggccacct ggacgtattc 360
   cactgaactg aagaaactct actgccaaat tgcaaagaca tgccccatcc agatcaaggt 420
   gatgacccca cctcctcagg gagctgttat ccgcgccatg cctgtctaca aaaaagctga 480
   gcacgtcacg gaggtggtga agcggtgccc caaccatgag ctgagccgtg agttcaacga 540
   gggacagatt gcccctccta gtcatttgat tcgagtagag gggaacagcc atgcccagta 600
   tgtagaagat cccatcacag gaagacagag tgtgctggta ccttatgagc caccccaggt 660
   tggcactgaa ttcacgacag tcttgtacaa tttcatgtgt aacagcagtt gtgttggagg 720
   gatgaaccgc cgtccaattt taatcattgt tactctggaa accagagatg ggcaagtcct 780
   gggccgacgc tgctttgagg cccggatctg tgcttgccca ggaagagaca ggaaggcgga 840
   tgaagatagc atcagaaagc agcaagtttc ggacagtaca aagaacggtg atggtacgaa 900
   gcgcccgttt cgtcagaaca cacatggtat ccagatgaca tccatcaaga aacgaagatc 960
   cccagatgat gaactgttat acttaccagt gaggggccgt gagacttatg aaatgctgtt 1020
   gaagatcaaa gagtccctgg aactcatgca gtaccttcct cagcacacaa ttgaaacgta 1080
   caggcaacag caacagcagc agcaccagca cttacttcag aaacagacct caatacagtc 1140
   tccatcttca tatggtaaca gctccccacc tctgaacaaa atgaacagca tgaacaagct 1200
   gccttctgtg agccagctta tcaaccctca gcagcgcaac gccctcactc ctacaaccat 1260
   tcctgatggc atgggagcca acattcccat gatgggcacc cacatgccaa tggctggaga 1320
   catgaatgga ctcagcccca cccaggcact ccctccccca ctctccatgc catccacctc 1380
   ccactgcaca cccccacctc cgtatcccac agattgcagc attgtcagtt tcttagcgag 1440
   gttgggctgt tcatcatgtc tggactattt cacgacccag gggctgacca ccatctatca 1500
   gattgagcat tactccatgg atgatctggc aagtctgaaa atccctgagc aatttcgaca 1560
   tgcgatctgg aagggcatcc tggaccaccg gcagctccac gaattctcct ccccttctca 1620
   tctcctgcgg accccaagca gtgcctctac agtcagtgtg ggctccagtg agacccgggg 1680
   tgagcgtgtt attgatgctg tgcgattcac cctccgccag accatctctt tcccaccccg 1740
   agatgagtgg aatgacttca actttgacat ggatgctcgc cgcaataagc aacagcgcat 1800
   caaagaggag ggggagtgag cctcaccatg tgagctcttc ctatccctct cctaactgcc 1860
   agccccctaa aagcactcct gcttaatctt caaagccttc tccctagctc ctccccttcc 1920
   tcttgtctga tttcttaggg gaaggagaag taagaggcta cctcttacct aacatctgac 1980
   ctggcatcta attctgattc tggctttaag ccttcaaaac tatagcttgc agaactgtag 2040
   ctgccatggc taggtagaag tgagcaaaaa agagttgggt gtctccttaa gctgcagaga 2100
   tttctcattg actttataaa agcatgttca cccttatagt ctaagactat atatataaat 2160
   gtataaatat acagtataga tttttgggtg gggggcattg agtattgttt aaaatgtaat 2220
   ttaaatgaaa gaaaattgag ttgcacttat tgaccatttt ttaatttact tgttttggat 2280
   ggcttgtcta tactccttcc cttaaggggt atcatgtatg gtgataggta tctagagctt 2340
   aatgctacat gtgagtgcga tgatgtacag attctttcag ttctttggat tctaaataca 2400
   tgccacatca aacctttgag tagatccatt tccattgctt attatgtagg taagactgta 2460
   gatatgtatt cttttctcag tgttggtata ttttatatta ctgacatttc ttctagtgat 2520
   gatggttcac gttggggtga tttaatccag ttataagaag aagttcatgt ccaaacggtc 2580
   ctctttagtt tttggttggg aatgaggaaa attcttaaaa ggcccatagc agccagttca 2640
   aaaacacccg acgtcatgta tttgagcata tcagtaaccc ccttaaattt aatacccaga 2700
   taccttatct tacaatgttg attgggaaaa catttgctgc ccattacaga ggtattaaaa 2760
   ctaaatttca ctactagatt gactaactca aatacacatt tgctactgtt gtaagaattc 2820

<210> 332
<211> 2270
<212> DNA
<213> Homo sapiens

<400> 332
tcgttgatat caaagacagt tgaaggaaat gaattttgaa acttcacggt gtgccaccct 60
acagtactgc cctgaccctt acatccagcg tttcgtagaa acccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660

cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgagggccgc tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacagacc 1380
tcaatacagt ctccatcttc atatggtaac agctccccac ctctgaacaa aatgaacagc 1440
atgaacaagc tgccttctgt gagccagctt atcaaccctc agcagcgcaa cgccctcact 1500
cctacaacca ttcctgatgg catgggagcc aacattccca tgatgggcac ccacatgcca 1560
atggctggag acatgaatgg actcagcccc acccaggcac tccctccccc actctccatg 1620
ccatccacct cccactgcac acccccacct ccgtatccaa cagattgcag cattgtcggt 1680
ttcttagcga ggttgggctg ttcatcatgt ctggactatt tcacgaccca ggggctgacc 1740
accatctatc agattgagca ttactccatg gatgatctgg caagtctgaa aatccctgag 1800
caatttcgac atgcgatctg gaagggcatc ctggaccacc ggcagctcca cgaattctcc 1860
tccccttctc atctcctgcg gaccccaagc agtgcctcta cagtcagtgt gggctccagt 1920
gagacccggg gtgagcgtgt tattgatgct gtgcgattca ccctccgcca gaccatctct 1980
ttcccacccc gagatgagtg gaatgacttc aactttgaca tggatgctcg ccgcaataag 2040
caacagcgca tcaaagagga gggggagtga gcctcaccat gtgagctctt cctatccctc 2100
tcctaactgc cagcccccta aaagcactcc tgcttaatct tcaaagcctt ctccctagct 2160
cctccccttc ctcttgtctg atttcttagg ggaaggagaa gtaagaggct acctcttacc 2220
taacatctga cctggcatct aattctgatt ctggctttaa gccttcaaaa 2270

<210> 333
<211> 2816
<212> DNA
<213> Homo sapiens

<400> 333
tcgttgatat caaagacagt tgaaggaaat gaattttgaa acttcacggt gtgccaccct 60
acagtactgc cctgaccctt acatccagcg tttcgtagaa acccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgagggccgc tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440

gacgtcttct ttagacattc caagccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt ttttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400
aatttgctta attagagctt ctatccctca agcctaccta ccataaaacc agccatatta 2460
ctgatactgt tcagtgcatt tagccaggag acttacgttt tgagtaagtg agatccaagc 2520
agacgtgtta aaatcagcac tcctggactg gaaattaaag attgaaaggg tagactactt 2580
ttcttttttt tactcaaaag tttagagaat ctctgtttct ttccatttta aaaacatatt 2640
ttaagataat agcataaaga ctttaaaaat gttcctcccc tccatcttcc cacacccagt 2700
caccagcact gtattttctg tcaccaagac aatgatttct tgttattgag gctgttgctt 2760
ttgtggatgt gtgattttaa ttttcaataa acttttgcat cttggtttaa aagaaa 2816

<210> 334
<211> 2082
<212> DNA
<213> Homo sapiens

<400> 334
agatgctaca gcgactgcac acccaggctg tatgatacag cctattgctc ccgggctgca 60
aacctgtcca gcatgtgatg tggtgggata ctgaattgaa taccgaatac tgtaggcaat 120
tgtaacacag tggtaagtct ttgtgtatct aaacatagct aaacaccaaa aggtatagta 180
agaatatggt attataatct tatggaacta tcattgtata tgtggtttgt caaccagaat 240
gtagttatac agcacaggac tgtgcttatg atgtgccaag cacagctctc agtactaact 300
cctttaatct tcatatcaac cctaggaggt aacttcttaa gtagattcat attgtaaggg 360
tctcggggtg ggggggttgg caaaatcctg gagccagaag aaaggacagc agcattgatc 420
aatcttacag ctaacatgtt gtacctggaa aacaatgccc agactcaatt tagtgagcca 480
cagtacacga acctggggct cctgaacagc atggaccagc agattcagaa cggctcctcg 540
tccaccagtc cctataacac agaccacgcg cagaacagcg tcacggcgcc ctcgccctac 600
gcacagccca gctccacctt cgatgctctc tctccatcac ccgccatccc ctccaacacc 660
gactacccag gcccgcacag tttcgacgtg tccttccagc agtcgagcac cgccaagtcg 720
gccacctgga cgtattccac tgaactgaag aaactctact gccaaattgc aaagacatgc 780
cccatccaga tcaaggtgat gaccccacct cctcagggag ctgttatccg cgccatgcct 840
gtctacaaaa aagctgagca cgtcacggag gtggtgaagc ggtgccccaa ccatgagctg 900
agccgtgaat tcaacgaggg acagattgcc cctcctagtc atttgattcg agtagagggg 960
aacagccatg cccagtatgt agaagatccc atcacaggaa gacagagtgt gctggtacct 1020
tatgagccac cccaggttgg cactgaattc acgacagtct tgtacaattt catgtgtaac 1080
agcagttgtg ttggagggat gaaccgccgt ccaattttaa tcattgttac tctggaaacc 1140
agagatgggc aagtcctggg ccgacgctgc tttgaggccc ggatctgtgc ttgcccagga 1200
agagacagga aggcggatga agatagcatc agaaagcagc aagtttcgga cagtacaaag 1260
aacggtgatg gtacgaagcg cccgtctcgt cagaacacac atggtatcca gatgacatcc 1320
atcaagaaac gaagatcccc agatgatgaa ctgttatact taccagtgag gggccgtgag 1380
acttatgaaa tgctgttgaa gatcaaagag tccctggaac tcatgcagta ccttcctcag 1440
cacacaattg aaacgtacag gcaacagcaa cagcagcagc accagcactt acttcagaaa 1500
cagtgagtgt atcaacgtgt cattttagga ggcatgagtg acggtgactt tatttggatc 1560
agcaataggg tgattgatga gcaatgtgga acataatggg agatagcaga ttgtcataga 1620
ttcagatgac ctggtatggc aaccctcttt cagttgcaac ctttttacg tgtcttatta 1680

taaccttccc ttcagaattc cacttatgtt ctgaaattaa atacaaacca tttctggtga 1740
attacaaaga aactcacact aacagttctc ttctctatat gcctggtcca tacacactaa 1800
cagtaagtac acactctatt tggtagtgat gtgtatattt gaaaacatga aatcttttct 1860
catcccaatg gattgtctta taaatctcct gggatgcaca ctatccactt ttgggaataa 1920
cactgtagac cagggatagc aaataggctt tactataata taaagtgact tgtttgaatg 1980
ctgtaatgag aagaattctg agacctagtg catgataatt ggggaaatat ctgggtgcag 2040
aaggataagg tagcatcatg ttgccgtatt ttagcatctc tg 2082

<210> 335
<211> 4849
<212> DNA
<213> Homo sapiens

<400> 335
cgttgatatc aaagacagtt gaaggaaatg aattttgaaa cttcacggtg tgccacccta 60
cagtactgcc ctgaccctta catccagcgt ttcgtagaaa ccccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacagacc 1380
tcaatacagt ctccatcttc atatggtaac agctccccac ctctgaacaa aatgaacagc 1440
atgaacaagc tgccttctgt gagccagctt atcaaccctc agcagcgcaa cgccctcact 1500
cctacaacca ttcctgatgg catgggagcc aacattccca tgatgggcac ccacatgcca 1560
atggctggag acatgaatgg actcagcccc acccaggcac tccctccccc actctccatg 1620
ccatccacct cccagtgcac acccccacct ccgtatccca cagattgcag cattgtcagt 1680
ttcttagcga ggttgggctg ttcatcatgt ctggactatt tcacgaccca ggggctgacc 1740
accatctatc agattgagca ttactccatg gatgatctgg caagtctgaa aatccctgag 1800
caatttcgac atgcgatctg gaagggcatc ctggaccacc ggcagctcca cgaattctcc 1860
tccccttctc atctcctgcg gaccccaagc agtgcctcta cagtcagtgt gggctccagt 1920
gagacccggg gtgagcgtgt tattgatgct gtgcgattca ccctccgcca gaccatctct 1980
ttcccacccc gagatgagtg gaatgacttc aactttgaca tggatgctcg ccgcaataag 2040
caacagcgca tcaaagagga gggggagtga gcctcaccat gtgagctctt cctatccctc 2100
tcctaactgc cagcycccta aaagcactcc tgcttaatct tcaaagcctt ctccctagct 2160
cctccccttc ctcttgtctg atttcttagg ggaaggagaa gtaagaggct acctcttacc 2220
taacatctga cctggcatct aattctgatt ctggctttaa gccttcaaaa ctatagcttg 2280
cagaactgta gctgccatgg ctaggtagaa gtgagcaaaa aagagttggg tgtctcctta 2340
agctgcagag atttctcatt gacttttata aagcatgttc acccttatag tctaagacta 2400
tatatataaa tgtataaata tacagtatag atttttgggt ggggggcatt gagtattgtt 2460
taaaatgtaa tttaaatgaa agaaaattga gttgcactta ttgaccattt tttaatttac 2520
ttgttttgga tggcttgtct atactccttc ccttaagggg tatcatgtat ggtgataggt 2580
atctagagct taatgctaca tgtgagtgac gatgatgtac agattctttc agttctttgg 2640

attctaaata catgccacat caaacctttg agtagatcca tttccattgc ttattatgta 2700
ggtaagactg tagatatgta ttcttttctc agtgttggta tattttatat tactgacatt 2760
tcttctagtg atgatggttc acgttggggt gatttaatcc agttataaga agaagttcat 2820
gtccaaacgt cctctttagt ttttggttgg gaatgaggaa aattcttaaa aggcccatag 2880
cagccagttc aaaaacaccc gacgtcatgt atttgagcat atcagtaacc cccttaaatt 2940
taataccaga taccttatct tacaatattg attgggaaaa catttgctgc cattacagag 3000
gtattaaaac taaatttcac tactagattg actaactcaa atacacattt gctactgttg 3060
taagaattct gattgatttg attgggatga atgccatcta tctagttcta acagtgaagt 3120
tttactgtct attaatattc agggtaaata ggaatcattc agaaatgttg agtctgtact 3180
aaacagtaag atatctcaat gaaccataaa ttcaactttg taaaaatctt ttgaagcata 3240
gataatattg tttggtaaat gtttcttttg tttggtaaat gtttctttta aagaccctcc 3300
tattctataa aactctgcat gtagaggctt gtttaccttt ctctctctaa ggtttacaat 3360
aggagtggtg atttgaaaaa tataaaatta tgagattggt tttcctgtgg cataaattgc 3420
atcactgtat cattttcttt tttaaccggt aagagtttca gtttgttgga aagtaactgt 3480
gagaacccag tttcccgtcc atctccctta gggactaccc atagacatga aaggtcccca 3540
cagagcaaga gataagtctt tcatggctgc tgttgcttaa accacttaaa cgaagagttc 3600
ccttgaaact ttgggaaaac atgttaatga caatattcca gatctttcag aaatataaca 3660
catttttttg catgcatgca aatgagctct gaaatcttcc catgcattct ggtcaagggc 3720
tgtcattgca cataagcttc cattttaatt ttaaagtgca aaagggccag cgtggctcta 3780
aaaggtaatg tgtggattgc ctctgaaaag tgtgtatata ttttgtgtga aattgcatac 3840
tttgtatttt gattattttt tttttcttct tgggatagtg ggatttccag aaccacactt 3900
gaaacctttt tttatcgttt ttgtattttc atgaaaatac catttagtaa gaataccaca 3960
tcaaataaga aataatgcta caattttaag aggggaggga agggaaagtt tttttttatt 4020
atttttttaa aattttgtat gttaaagaga atgagtcctt gatttcaaag ttttgttgta 4080
cttaaatggt aataagcact gtaaacttct gcaacaagca tgcagctttg caaacccatt 4140
aaggggaaga atgaaagctg ttccttggtc ctagtaagaa gacaaactgc ttcccttact 4200
ttgctgaggg tttgaataaa cctaggactt ccgagctatg tcagtactat tcaggtaaca 4260
ctagggcctt ggaaattcct gtactgtgtc tcatggattt ggcactagcc aaagcgaggc 4320
acccttactg gcttacctcc tcatggcagc ctactctcct tgagtgtatg agtagccagg 4380
gtaaggggta aaaggatagt aagcatagaa accactagaa agtgggctta atggagttct 4440
tgtggcctca gctcaatgca gttagctgaa gaattgaaaa gtttttgttt ggagacgttt 4500
ataaacagaa atggaaagca gagttttcat taaatccttt tacctttttt ttttcttggt 4560
aatcccctaa aataacagta tgtgggatat tgaatgttaa agggatattt tttttctatt 4620
atttttataa ttgtacaaaa ttaagcaaat gttaaaagtt ttatatgctt tattaatgtt 4680
ttcaaaaggt attatacatg tgatacattt tttaagcttc agttgcttgt cttctggtac 4740
tttctgttat gggcttttgg ggagccagaa gccaatctac aatctctttt tgtttgccag 4800
gacatgcaat aaaatttaaa aaataaataa aaactaatta agaaataaa 4849

<210> 336
<211> 1386
<212> DNA
<213> Homo sapiens

<400> 336
atgttgtacc tggaaaacaa tgcccagact caatttagtg agccacagta cacgaacctg 60
gggctcctga acagcatgga ccagcagatt cagaacggct cctcgtccac cagtccctat 120
aacacagacc acgcgcagaa cagcgtcacg gcgccctcgc cctacgcaca gcccagctcc 180
accttcgatg ctctctctcc atcacccgcc atcccctcca acaccgacta cccaggcccg 240
cacagtttcg acgtgtcctt ccagcagtcg agcaccgcca agtcggccac ctggacgtat 300
tccactgaac tgaagaaact ctactgccaa attgcaaaga catgccccat ccagatcaag 360
gtgatgaccc cacctcctca gggagctgtt atccgcgcca tgcctgtcta caaaaaagct 420
gagcacgtca cggaggtggt gaagcggtgc cccaaccatg agctgagccg tgaattcaac 480
gagggacaga ttgcccctcc tagtcatttg attcgagtag aggggaacag ccatgcccag 540
tatgtagaag atcccatcac aggaagacag agtgtgctgg taccttatga gccaccccag 600
gttggcactg aattcacgac agtcttgtac aatttcatgt gtaacagcag ttgtgttgga 660
gggatgaacc gccgtccaat tttaatcatt gttactctgg aaaccagaga tgggcaagtc 720
ctgggccgac gctgctttga ggcccggatc tgtgcttgcc caggaagaga caggaaggcg 780
gatgaagata gcatcagaaa gcagcaagtt tcggacagta caaagaacgg tgatggtacg 840

aagcgcccgt ttcgtcagaa cacacatggt atccagatga catccatcaa gaaacgaaga 900
tccccagatg atgaactgtt atacttacca gtgaggggcc gtgagactta tgaaatgctg 960
ttgaagatca aagagtccct ggaactcatg cagtaccttc ctcagcacac aattgaaacg 1020
tacaggcaac agcaacagca gcagcaccag cacttacttc agaaacagac ctcaatacag 1080
tctccatctt catatggtaa cagctcccca cctctgaaca aaatgaacag catgaacaag 1140
ctgccttctg tgagccagct tatcaaccct cagcagcgca acgccctcac tcctacaacc 1200
attcctgatg gcatgggagc caacattccc atgatgggca cccacatgcc aatggctgga 1260
gacatgaatg gactcagccc cacccaggca ctccctcccc cactctccat gccatccacc 1320
tcccactgca cacccccacc tccgtatccc acagattgca gcattgtcag gatctggcaa 1380
gtctga 1386

<210> 337
<211> 1551
<212> DNA
<213> Homo sapiens

<400> 337
atgtcccaga gcacacagac aaatgaattc ctcagtccag aggttttcca gcatatctgg 60
gattttctgg aacagcctat atgttcagtt cagcccattg acttgaactt tgtggatgaa 120
ccatcagaag atggtgcgac aaacaagatt gagattagca tggactgtat ccgcatgcag 180
gactcggacc tgagtgaccc catgtggcca cagtacacga acctggggct cctgaacagc 240
atggaccagc agattcagaa cggctcctcg tccaccagtc cctataacac agaccacgcg 300
cagaacagcg tcacggcgcc ctcgccctac gcacagccca gctccacctt cgatgctctc 360
tctccatcac ccgccatccc ctccaacacc gactacccag gcccgcacag tttcgacgtg 420
tccttccagc agtcgagcac cgccaagtcg gccacctgga cgtattccac tgaactgaag 480
aaactctact gccaaattgc aaagacatgc cccatccaga tcaaggtgat gaccccacct 540
cctcagggag ctgttatccg cgccatgcct gtctacaaaa aagctgagca cgtcacggag 600
gtggtgaagc ggtgccccaa ccatgagctg agccgtgaat tcaacgaggg acagattgcc 660
cctcctagtc atttgattcg agtagagggg aacagccatg cccagtatgt agaagatccc 720
atcacaggaa gacagagtgt gctggtacct tatgagccac cccaggttgg cactgaattc 780
acgacagtct tgtacaattt catgtgtaac agcagttgtg ttggagggat gaaccgccgt 840
ccaattttaa tcattgttac tctggaaacc agagatgggc aagtcctggg ccgacgctgc 900
tttgaggccc ggatctgtgc ttgcccagga agagacagga aggcggatga agatagcatc 960
agaaagcagc aagtttcgga cagtacaaag aacggtgatg gtacgaagcg cccgtttcgt 1020
cagaacacac atggtatcca gatgacatcc atcaagaaac gaagatcccc agatgatgaa 1080
ctgttatact taccagtgag gggccgtgag acttatgaaa tgctgttgaa gatcaaagag 1140
tccctggaac tcatgcagta ccttcctcag cacacaattg aaacgtacag gcaacagcaa 1200
cagcagcagc accagcactt acttcagaaa cagacctcaa tacagtctcc atcttcatat 1260
ggtaacagct ccccacctct gaacaaaatg aacagcatga acaagctgcc ttctgtgagc 1320
cagcttatca accctcagca gcgcaacgcc ctcactccta caaccattcc tgatggcatg 1380
ggagccaaca ttcccatgat gggcacccac atgccaatgg ctggagacat gaatggactc 1440
agccccaccc aggcactccc tcccccactc tccatgccat ccacctccca ctgcacaccc 1500
ccacctccgt atcccacaga ttgcagcatt gtcaggatct ggcaagtctg a 1551

<210> 338
<211> 586
<212> PRT
<213> Homo sapiens

<400> 338

<210> 339
<211> 641
<212> PRT
<213> Homo sapiens

<400> 339

<210> 340
<211> 448
<212> PRT
<213> Homo sapiens

<400> 340
Met Ser Gln Ser Thr Gln Thr Asn Glu Phe Leu Ser Pro Glu Val Phe
1               5                   10                  15
Gln His Ile Trp Asp Phe Leu Glu Gln Pro Ile Cys Ser Val Gln Pro
            20                  25                  30
Ile Asp Leu Asn Phe Val Asp Glu Pro Ser Glu Asp Gly Ala Thr Asn
        35                  40                  45
Lys Ile Glu Ile Ser Met Asp Cys Ile Arg Met Gln Asp Ser Asp Leu
    50                  55                  60
Ser Asp Pro Met Trp Pro Gln Tyr Thr Asn Leu Gly Leu Leu Asn Ser
65                  70                  75                  80

Met Asp Gln Gln Ile Gln Asn Gly Ser Ser Ser Thr Ser Pro Tyr Asn
                85                  90                  95
Thr Asp His Ala Gln Asn Ser Val Thr Ala Pro Ser Pro Tyr Ala Gln
                100                 105                 110
Pro Ser Ser Thr Phe Asp Ala Leu Ser Pro Ser Pro Ala Ile Pro Ser
        115                 120                 125
Asn Thr Asp Tyr Pro Gly Pro His Ser Phe Asp Val Ser Phe Gln Gln
    130                 135                 140
Ser Ser Thr Ala Lys Ser Ala Thr Trp Thr Tyr Ser Thr Glu Leu Lys
145                 150                 155                 160
Lys Leu Tyr Cys Gln Ile Ala Lys Thr Cys Pro Ile Gln Ile Lys Val
                165                 170                 175
Met Thr Pro Pro Pro Gln Gly Ala Val Ile Arg Ala Met Pro Val Tyr
                180                 185                 190
Lys Lys Ala Glu His Val Thr Glu Val Val Lys Arg Cys Pro Asn His
        195                 200                 205
Glu Leu Ser Arg Glu Phe Asn Glu Gly Gln Ile Ala Pro Pro Ser His
    210                 215                 220
Leu Ile Arg Val Glu Gly Asn Ser His Ala Gln Tyr Val Glu Asp Pro
225                 230                 235                 240
Ile Thr Gly Arg Gln Ser Val Leu Val Pro Tyr Glu Pro Pro Gln Val
                245                 250                 255
Gly Thr Glu Phe Thr Thr Val Leu Tyr Asn Phe Met Cys Asn Ser Ser
                260                 265                 270
Cys Val Gly Gly Met Asn Arg Arg Pro Ile Leu Ile Ile Val Thr Leu
        275                 280                 285
Glu Thr Arg Asp Gly Gln Val Leu Gly Arg Arg Cys Phe Glu Ala Arg
    290                 295                 300
Ile Cys Ala Cys Pro Gly Arg Asp Arg Lys Ala Asp Glu Asp Ser Ile
305                 310                 315                 320
Arg Lys Gln Gln Val Ser Asp Ser Thr Lys Asn Gly Asp Gly Thr Lys
                325                 330                 335
Arg Pro Phe Arg Gln Asn Thr His Gly Ile Gln Met Thr Ser Ile Lys
                340                 345                 350
Lys Arg Arg Ser Pro Asp Asp Glu Leu Leu Tyr Leu Pro Val Arg Gly
        355                 360                 365
Arg Glu Thr Tyr Glu Met Leu Leu Lys Ile Lys Glu Ser Leu Glu Leu
    370                 375                 380
Met Gln Tyr Leu Pro Gln His Thr Ile Glu Thr Tyr Arg Gln Gln Gln
385                 390                 395                 400
Gln Gln Gln His Gln His Leu Leu Gln Lys His Leu Leu Ser Ala Cys
                405                 410                 415
Phe Arg Asn Glu Leu Val Glu Pro Arg Arg Glu Thr Pro Lys Gln Ser
                420                 425                 430
Asp Val Phe Phe Arg His Ser Lys Pro Pro Asn Arg Ser Val Tyr Pro
        435                 440                 445


<210> 341
<211> 356
<212> PRT
<213> Homo sapiens

<400> 341


<210> 342
<211> 680
<212> PRT
<213> Homo sapiens

<400> 342
Met Asn Phe Glu Thr Ser Arg Cys Ala Thr Leu Gln Tyr Cys Pro Asp
1               5                   10                  15
Pro Tyr Ile Gln Arg Phe Val Glu Thr Pro Ala His Phe Ser Trp Lys
            20                  25                  30
Glu Ser Tyr Tyr Arg Ser Thr Met Ser Gln Ser Thr Gln Thr Asn Glu
        35                  40                  45
Phe Leu Ser Pro Glu Val Phe Gln His Ile Trp Asp Phe Leu Glu Gln
    50                  55                  60

Pro Ile Cys Ser Val Gln Pro Ile Asp Leu Asn Phe Val Asp Glu Pro
65                  70                  75                  80
Ser Glu Asp Gly Ala Thr Asn Lys Ile Glu Ile Ser Met Asp Cys Ile
                85                  90                  95
Arg Met Gln Asp Ser Asp Leu Ser Asp Pro Met Trp Pro Gln Tyr Thr
            100                 105                 110
Asn Leu Gly Leu Leu Asn Ser Met Asp Gln Gln Ile Gln Asn Gly Ser
        115                 120                 125
Ser Ser Thr Ser Pro Tyr Asn Thr Asp His Ala Gln Asn Ser Val Thr
    130                 135                 140
Ala Pro Ser Pro Tyr Ala Gln Pro Ser Ser Thr Phe Asp Ala Leu Ser
145                 150                 155                 160
Pro Ser Pro Ala Ile Pro Ser Asn Thr Asp Tyr Pro Gly Pro His Ser
                165                 170                 175
Phe Asp Val Ser Phe Gln Gln Ser Ser Thr Ala Lys Ser Ala Thr Trp
            180                 185                 190
Thr Tyr Ser Thr Glu Leu Lys Lys Leu Tyr Cys Gln Ile Ala Lys Thr
        195                 200                 205
Cys Pro Ile Gln Ile Lys Val Met Thr Pro Pro Pro Gln Gly Ala Val
    210                 215                 220
Ile Arg Ala Met Pro Val Tyr Lys Lys Ala Glu His Val Thr Glu Val
225                 230                 235                 240
Val Lys Arg Cys Pro Asn His Glu Leu Ser Arg Glu Phe Asn Glu Gly
                245                 250                 255
Gln Ile Ala Pro Pro Ser His Leu Ile Arg Val Glu Gly Asn Ser His
            260                 265                 270
Ala Gln Tyr Val Glu Asp Pro Ile Thr Gly Arg Gln Ser Val Leu Val
        275                 280                 285
Pro Tyr Glu Pro Pro Gln Val Gly Thr Glu Phe Thr Thr Val Leu Tyr
    290                 295                 300
Asn Phe Met Cys Asn Ser Ser Cys Val Gly Gly Met Asn Arg Arg Pro
305                 310                 315                 320
Ile Leu Ile Ile Val Thr Leu Glu Thr Arg Asp Gly Gln Val Leu Gly
                325                 330                 335
Arg Arg Cys Phe Glu Ala Arg Ile Cys Ala Cys Pro Gly Arg Asp Arg
            340                 345                 350
Lys Ala Asp Glu Asp Ser Ile Arg Lys Gln Gln Val Ser Asp Ser Thr
        355                 360                 365
Lys Asn Gly Asp Gly Thr Lys Arg Pro Phe Arg Gln Asn Thr His Gly
    370                 375                 380
Ile Gln Met Thr Ser Ile Lys Lys Arg Arg Ser Pro Asp Asp Glu Leu
385                 390                 395                 400
Leu Tyr Leu Pro Val Arg Gly Arg Glu Thr Tyr Glu Met Leu Leu Lys
                405                 410                 415
Ile Lys Glu Ser Leu Glu Leu Met Gln Tyr Leu Pro Gln His Thr Ile
            420                 425                 430
Glu Thr Tyr Arg Gln Gln Gln Gln Gln Gln His Gln His Leu Leu Gln
        435                 440                 445
Lys Gln Thr Ser Ile Gln Ser Pro Ser Ser Tyr Gly Asn Ser Ser Pro
    450                 455                 460
Pro Leu Asn Lys Met Asn Ser Met Asn Lys Leu Pro Ser Val Ser Gln
465                 470                 475                 480
Leu Ile Asn Pro Gln Gln Arg Asn Ala Leu Thr Pro Thr Thr Ile Pro
                485                 490                 495
Asp Gly Met Gly Ala Asn Ile Pro Met Met Gly Thr His Met Pro Met
            500                 505                 510
Ala Gly Asp Met Asn Gly Leu Ser Pro Thr Gln Ala Leu Pro Pro Pro
        515                 520                 525

Leu Ser Met Pro Ser Thr Ser Gln Cys Thr Pro Pro Pro Pro Tyr Pro
    530                 535                 540
Thr Asp Cys Ser Ile Val Ser Phe Leu Ala Arg Leu Gly Cys Ser Ser
545                 550                 555                 560
Cys Leu Asp Tyr Phe Thr Thr Gln Gly Leu Thr Thr Ile Tyr Gln Ile
                565                 570                 575
Glu His Tyr Ser Met Asp Asp Leu Ala Ser Leu Lys Ile Pro Glu Gln
            580                 585                 590
Phe Arg His Ala Ile Trp Lys Gly Ile Leu Asp His Arg Gln Leu His
        595                 600                 605
Glu Phe Ser Ser Pro Ser His Leu Leu Arg Thr Pro Ser Ser Ala Ser
    610                 615                 620
Thr Val Ser Val Gly Ser Ser Glu Thr Arg Gly Glu Arg Val Ile Asp
625                 630                 635                 640
Ala Val Arg Phe Thr Leu Arg Gln Thr Ile Ser Phe Pro Pro Arg Asp
                645                 650                 655
Glu Trp Asn Asp Phe Asn Phe Asp Met Asp Ala Arg Arg Asn Lys Gln
            660                 665                 670
Gln Arg Ile Lys Glu Glu Gly Glu
        675                 680

<210> 343
<211> 461
<212> PRT
<213> Homo sapiens

<400> 343
Met Leu Tyr Leu Glu Asn Asn Ala Gln Thr Gln Phe Ser Glu Pro Gln
 1               5                  10                  15
Tyr Thr Asn Leu Gly Leu Leu Asn Ser Met Asp Gln Gln Ile Gln Asn
            20                  25                  30
Gly Ser Ser Ser Thr Ser Pro Tyr Asn Thr Asp His Ala Gln Asn Ser
        35                  40                  45
Val Thr Ala Pro Ser Pro Tyr Ala Gln Pro Ser Ser Thr Phe Asp Ala
    50                  55                  60
Leu Ser Pro Ser Pro Ala Ile Pro Ser Asn Thr Asp Tyr Pro Gly Pro
65                  70                  75                  80
His Ser Phe Asp Val Ser Phe Gln Gln Ser Ser Thr Ala Lys Ser Ala
                85                  90                  95
Thr Trp Thr Tyr Ser Thr Glu Leu Lys Lys Leu Tyr Cys Gln Ile Ala
            100                 105                 110
Lys Thr Cys Pro Ile Gln Ile Lys Val Met Thr Pro Pro Pro Gln Gly
        115                 120                 125
Ala Val Ile Arg Ala Met Pro Val Tyr Lys Lys Ala Glu His Val Thr
    130                 135                 140
Glu Val Val Lys Arg Cys Pro Asn His Glu Leu Ser Arg Glu Phe Asn
145                 150                 155                 160
Glu Gly Gln Ile Ala Pro Pro Ser His Leu Ile Arg Val Glu Gly Asn
                165                 170                 175
Ser His Ala Gln Tyr Val Glu Asp Pro Ile Thr Gly Arg Gln Ser Val
            180                 185                 190
Leu Val Pro Tyr Glu Pro Pro Gln Val Gly Thr Glu Phe Thr Thr Val
        195                 200                 205
Leu Tyr Asn Phe Met Cys Asn Ser Ser Cys Val Gly Gly Met Asn Arg
    210                 215                 220
Arg Pro Ile Leu Ile Ile Val Thr Leu Glu Thr Arg Asp Gly Gln Val
225                 230                 235                 240

Leu Gly Arg Arg Cys Phe Glu Ala Arg Ile Cys Ala Cys Pro Gly Arg
                245                 250                 255
Asp Arg Lys Ala Asp Glu Asp Ser Ile Arg Lys Gln Gln Val Ser Asp
            260                 265                 270
Ser Thr Lys Asn Gly Asp Gly Thr Lys Arg Pro Phe Arg Gln Asn Thr
        275                 280                 285
His Gly Ile Gln Met Thr Ser Ile Lys Lys Arg Arg Ser Pro Asp Asp
    290                 295                 300
Glu Leu Leu Tyr Leu Pro Val Arg Gly Arg Glu Thr Tyr Glu Met Leu
305                 310                 315                 320
Leu Lys Ile Lys Glu Ser Leu Glu Leu Met Gln Tyr Leu Pro Gln His
                325                 330                 335
Thr Ile Glu Thr Tyr Arg Gln Gln Gln Gln Gln Gln His Gln His Leu
            340                 345                 350
Leu Gln Lys Gln Thr Ser Ile Gln Ser Pro Ser Ser Tyr Gly Asn Ser
        355                 360                 365
Ser Pro Pro Leu Asn Lys Met Asn Ser Met Asn Lys Leu Pro Ser Val
    370                 375                 380
Ser Gln Leu Ile Asn Pro Gln Gln Arg Asn Ala Leu Thr Pro Thr Thr
385                 390                 395                 400
Ile Pro Asp Gly Met Gly Ala Asn Ile Pro Met Met Gly Thr His Met
                405                 410                 415
Pro Met Ala Gly Asp Met Asn Gly Leu Ser Pro Thr Gln Ala Leu Pro
            420                 425                 430
Pro Pro Leu Ser Met Pro Ser Thr Ser His Cys Thr Pro Pro Pro Pro
        435                 440                 445
Tyr Pro Thr Asp Cys Ser Ile Val Arg Ile Trp Gln Val
    450                 455                 460

<210> 344
<211> 516
<212> PRT
<213> Homo sapiens

<400> 344
Met Ser Gln Ser Thr Gln Thr Asn Glu Phe Leu Ser Pro Glu Val Phe
1               5                   10                  15
Gln His Ile Trp Asp Phe Leu Glu Gln Pro Ile Cys Ser Val Gln Pro
            20                  25                  30
Ile Asp Leu Asn Phe Val Asp Glu Pro Ser Glu Asp Gly Ala Thr Asn
        35                  40                  45
Lys Ile Glu Ile Ser Met Asp Cys Ile Arg Met Gln Asp Ser Asp Leu
    50                  55                  60
Ser Asp Pro Met Trp Pro Gln Tyr Thr Asn Leu Gly Leu Leu Asn Ser
65                  70                  75                  80
Met Asp Gln Gln Ile Gln Asn Gly Ser Ser Ser Thr Ser Pro Tyr Asn
                85                  90                  95
Thr Asp His Ala Gln Asn Ser Val Thr Ala Pro Ser Pro Tyr Ala Gln
            100                 105                 110
Pro Ser Ser Thr Phe Asp Ala Leu Ser Pro Ser Pro Ala Ile Pro Ser
        115                 120                 125
Asn Thr Asp Tyr Pro Gly Pro His Ser Phe Asp Val Ser Phe Gln Gln
    130                 135                 140
Ser Ser Thr Ala Lys Ser Ala Thr Trp Thr Tyr Ser Thr Glu Leu Lys
145                 150                 155                 160
Lys Leu Tyr Cys Gln Ile Ala Lys Thr Cys Pro Ile Gln Ile Lys Val
                165                 170                 175

Met Thr Pro Pro Pro Gln Gly Ala Val Ile Arg Ala Met Pro Val Tyr
            180                 185                 190
Lys Lys Ala Glu His Val Thr Glu Val Val Lys Arg Cys Pro Asn His
        195                 200                 205
Glu Leu Ser Arg Glu Phe Asn Glu Gly Gln Ile Ala Pro Pro Ser His
    210                 215                 220
Leu Ile Arg Val Glu Gly Asn Ser His Ala Gln Tyr Val Glu Asp Pro
225                 230                 235                 240
Ile Thr Gly Arg Gln Ser Val Leu Val Pro Tyr Glu Pro Pro Gln Val
                245                 250                 255
Gly Thr Glu Phe Thr Thr Val Leu Tyr Asn Phe Met Cys Asn Ser Ser
            260                 265                 270
Cys Val Gly Gly Met Asn Arg Arg Pro Ile Leu Ile Ile Val Thr Leu
        275                 280                 285
Glu Thr Arg Asp Gly Gln Val Leu Gly Arg Arg Cys Phe Glu Ala Arg
    290                 295                 300
Ile Cys Ala Cys Pro Gly Arg Asp Arg Lys Ala Asp Glu Asp Ser Ile
305                 310                 315                 320
Arg Lys Gln Gln Val Ser Asp Ser Thr Lys Asn Gly Asp Gly Thr Lys
                325                 330                 335
Arg Pro Phe Arg Gln Asn Thr His Gly Ile Gln Met Thr Ser Ile Lys
            340                 345                 350
Lys Arg Arg Ser Pro Asp Asp Glu Leu Leu Tyr Leu Pro Val Arg Gly
        355                 360                 365
Arg Glu Thr Tyr Glu Met Leu Leu Lys Ile Lys Glu Ser Leu Glu Leu
    370                 375                 380
Met Gln Tyr Leu Pro Gln His Thr Ile Glu Thr Tyr Arg Gln Gln Gln
385                 390                 395                 400
Gln Gln Gln His Gln His Leu Leu Gln Lys Gln Thr Ser Ile Gln Ser
                405                 410                 415
Pro Ser Ser Tyr Gly Asn Ser Ser Pro Pro Leu Asn Lys Met Asn Ser
            420                 425                 430
Met Asn Lys Leu Pro Ser Val Ser Gln Leu Ile Asn Pro Gln Gln Arg
        435                 440                 445
Asn Ala Leu Thr Pro Thr Thr Ile Pro Asp Gly Met Gly Ala Asn Ile
    450                 455                 460
Pro Met Met Gly Thr His Met Pro Met Ala Gly Asp Met Asn Gly Leu
465                 470                 475                 480
Ser Pro Thr Gln Ala Leu Pro Pro Pro Leu Ser Met Pro Ser Thr Ser
                485                 490                 495
His Cys Thr Pro Pro Pro Pro Tyr Pro Thr Asp Cys Ser Ile Val Arg
            500                 505                 510
Ile Trp Gln Val
        515

<210> 345
<211> 1800
<212> DNA
<213> Homo sapiens

<400> 345
gcgcctcatt gccactgcag tgactaaagc tgggaagacg ctggtcagtt cacctgcccc 60
actggttgtt ttttaaacaa attctgatac aggcgacatc ctcactgacc gagcaaagat 120
tgacattcgt atcatcactg tgcaccattg gcttctaggc actccagtgg ggtaggagaa 180
ggaggtctga aaccctcgca gagggatctt gccctcattc tttgggtctg aaacactggc 240
agtcgttgga aacaggactc agggataaac cagcgcaatg gattggggga cgctgcacac 300
tttcatcggg ggtgtcaaca aacactccac cagcatcggg aaggtgtgga tcacagtcat 360

ctttattttc cgagtcatga tcctagtggt ggctgcccag gaagtgtggg gtgacgagca 420
agaggacttc gtctgcaaca cactgcaacc gggatgcaaa aatgtgtgct atgaccactt 480
tttcccggtg tcccacatcc ggctgtgggc cctccagctg atcttcgtct ccaccccagc 540
gctgctggtg gccatgcatg tggcctacta caggcacgaa accactcgca agttcaggcg 600
aggagagaag aggaatgatt tcaaagacat agaggacatt aaaaagcaca aggttcggat 660
agaggggtcg ctgtggtgga cgtacaccag cagcatcttt ttccgaatca tctttgaagc 720
agcctttatg tatgtgtttt acttccttta caatgggtac cacctgccct gggtgttgaa 780
atgtgggatt gacccctgcc ccaaccttgt tgactgcttt atttctaggc caacagagaa 840
gaccgtgttt accattttta tgatttctgc gtctgtgatt tgcatgctgc ttaacgtggc 900
agagttgtgc tacctgctgc tgaaagtgtg ttttaggaga tcaaagagag cacagacgca 960
aaaaaatcac cccaatcatg ccctaaagga gagtaagcag aatgaaatga atgagctgat 1020
ttcagatagt ggtcaaaatg caatcacagg tttcccaagc taaacatttc aaggtaaaat 1080
gtagctgcgt cataaggaga cttctgtctt ctccagaagg caataccaac ctgaaagttc 1140
cttctgtagc ctgaagagtt tgtaaatgac tttcataata aatagacact tgagttaact 1200
ttttgtagga tacttgctcc attcatacac aacgtaatca aatatgtggt ccatctctga 1260
aaacaagaga ctgcttgaca aaggagcatt gcagtcactt tgacaggttc cttttaagtg 1320
gactctctga caaagtgggt actttctgaa aatttatata actgttgttg ataaggaaca 1380
tttatccagg aattgatacg tttattagga aaagatattt ttataggctt ggatgttttt 1440
agttccgact ttgaatttat ataaagtatt tttataatga ctggtcttcc ttacctggaa 1500
aaacatgcga tgttagtttt agaattacac cacaagtatc taaatttcca acttacaaag 1560
ggtcctatct tgtaaatatt gttttgcatt gtctgttggc aaatttgtga actgtcatga 1620
tacgcttaag gtgggaaagt gttcattgca caatatattt ttactgcttt ctgaatgtag 1680
acggaacagt gtggaagcag aaggcttttt taactcatcc gtttggccga tcgttgcaga 1740
ccactgggag atgtggatgt ggttgcctcc ttttgctcgt ccccgtggct taacccttct 1800

<210> 346
<211> 261
<212> PRT
<213> Homo sapiens

<400> 346
Met Asp Trp Gly Thr Leu His Thr Phe Ile Gly Gly Val Asn Lys His
1               5                   10                  15
Ser Thr Ser Ile Gly Lys Val Trp Ile Thr Val Ile Phe Ile Phe Arg
            20                  25                  30
Val Met Ile Leu Val Val Ala Ala Gln Glu Val Trp Gly Asp Glu Gln
        35                  40                  45
Glu Asp Phe Val Cys Asn Thr Leu Gln Pro Gly Cys Lys Asn Val Cys
    50                  55                  60
Tyr Asp His Phe Phe Pro Val Ser His Ile Arg Leu Trp Ala Leu Gln
65                  70                  75                  80
Leu Ile Phe Val Ser Thr Pro Ala Leu Leu Val Ala Met His Val Ala
                85                  90                  95
Tyr Tyr Arg His Glu Thr Thr Arg Lys Phe Arg Arg Gly Glu Lys Arg
            100                 105                 110
Asn Asp Phe Lys Asp Ile Glu Asp Ile Lys Lys His Lys Val Arg Ile
        115                 120                 125
Glu Gly Ser Leu Trp Trp Thr Tyr Thr Ser Ser Ile Phe Phe Arg Ile
    130                 135                 140
Ile Phe Glu Ala Ala Phe Met Tyr Val Phe Tyr Phe Leu Tyr Asn Gly
145                 150                 155                 160
Tyr His Leu Pro Trp Val Leu Lys Cys Gly Ile Asp Pro Cys Pro Asn
                165                 170                 175
Leu Val Asp Cys Phe Ile Ser Arg Pro Thr Glu Lys Thr Val Phe Thr
            180                 185                 190
Ile Phe Met Ile Ser Ala Ser Val Ile Cys Met Leu Leu Asn Val Ala
        195                 200                 205

Glu Leu Cys Tyr Leu Leu Leu Lys Val Cys Phe Arg Arg Ser Lys Arg
    210                 215                 220
Ala Gln Thr Gln Lys Asn His Pro Asn His Ala Leu Lys Glu Ser Lys
225                 230                 235                 240
Gln Asn Glu Met Asn Glu Leu Ile Ser Asp Ser Gly Gln Asn Ala Ile
                245                 250                 255
Thr Gly Phe Pro Ser
            260

<210> 347
<211> 1740
<212> DNA
<213> Homo sapiens

<400> 347
   atgaacaaac tgtatatcgg aaacctcagc gagaacgccg ccccctcgga cctagaaagt 60
   atcttcaagg acgccaagat cccggtgtcg ggacccttcc tggtgaagac tggctacgcg 120
   ttcgtggact gcccggacga gagctgggcc ctcaaggcca tcgaggcgct ttcaggtaaa 180
   atagaactgc acgggaaacc catagaagtt gagcactcgg tcccaaaaag gcaaaggatt 240
   cggaaacttc agatacgaaa tatcccgcct catttacagt gggaggtgct ggatagttta 300
   ctagtccagt atggagtggt ggagagctgt gagcaagtga acactgactc ggaaactgca 360
   gttgtaaatg taacctattc cagtaaggac caagctagac aagcactaga caaactgaat 420
   ggatttcagt tagagaattt caccttgaaa gtagcctata tccctgatga aacggccgcc 480
   cagcaaaacc ccttgcagca gccccgaggt cgccgggggc ttgggcagag gggctcctca 540
   aggcaggggt ctccaggatc cgtatccaag cagaaaccat gtgatttgcc tctgcgcctg 600
   ctggttccca cccaatttgt tggagccatc ataggaaaag aaggtgccac cattcggaac 660
   atcaccaaac agacccagtc taaaatcgat gtccaccgta aagaaaatgc gggggctgct 720
   gagaagtcga ttactatcct ctctactcct gaaggcacct ctgcggcttg taagtctatt 780
   ctggagatta tgcataagga agctcaagat ataaaattca cagaagagat ccccttgaag 840
   attttagctc ataataactt tgttggacgt cttattggta aagaaggaag aaatcttaaa 900
   aaaattgagc aagacacaga cactaaaatc acgatatctc cattgcagga attgacgctg 960
   tataatccag aacgcactat tacagttaaa ggcaatgttg agacatgtgc caaagctgag 1020
   gaggagatca tgaagaaaat cagggagtct tatgaaaatg atattgcttc tatgaatctt 1080
   caagcacatt taattcctgg attaaatctg aacgccttgg gtctgttccc acccacttca 1140
   gggatgccac ctcccacctc agggccccct tcagccatga ctcctcccta cccgcagttt 1200
   gagcaatcag aaacggagac tgttcatctg tttatcccag ctctatcagt cggtgccatc 1260
   atcggcaagc agggccagca catcaagcag ctttctcgct ttgctggagc ttcaattaag 1320
   attgctccag cggaagcacc agatgctaaa gtgaggatgg tgattatcac tggaccacca 1380
   gaggctcagt tcaaggctca gggaagaatt tatggaaaaa ttaaagaaga aaactttgtt 1440
   agtcctaaag aagaggtgaa acttgaagct catatcagag tgccatcctt tgctgctggc 1500
   agagttattg gaaaaggagg caaaacggtg aatgaacttc agaatttgtc aagtgcagaa 1560
   gttgttgtcc ctcgtgacca gacacctgat gagaatgacc aagtggttgt caaaataact 1620
   ggtcacttct atgcttgcca ggttgcccag agaaaaattc aggaaattct gactcaggta 1680
   aagcagcacc aacaacagaa ggctctgcaa agtggaccac ctcagtcaag acggaagtaa 1740

<210> 348
<211> 579
<212> PRT
<213> Homo sapiens

<400> 348
Met Asn Lys Leu Tyr Ile Gly Asn Leu Ser Glu Asn Ala Ala Pro Ser
1               5                   10                  15
Asp Leu Glu Ser Ile Phe Lys Asp Ala Lys Ile Pro Val Ser Gly Pro
            20                  25                  30
Phe Leu Val Lys Thr Gly Tyr Ala Phe Val Asp Cys Pro Asp Glu Ser

        35                  40                  45
Trp Ala Leu Lys Ala Ile Glu Ala Leu Ser Gly Lys Ile Glu Leu His
    50                  55                  60
Gly Lys Pro Ile Glu Val Glu His Ser Val Pro Lys Arg Gln Arg Ile
65                  70                  75                  80
Arg Lys Leu Gln Ile Arg Asn Ile Pro Pro His Leu Gln Trp Glu Val
                85                  90                  95
Leu Asp Ser Leu Leu Val Gln Tyr Gly Val Val Glu Ser Cys Glu Gln
            100                 105                 110
Val Asn Thr Asp Ser Glu Thr Ala Val Val Asn Val Thr Tyr Ser Ser
        115                 120                 125
Lys Asp Gln Ala Arg Gln Ala Leu Asp Lys Leu Asn Gly Phe Gln Leu
    130                 135                 140
Glu Asn Phe Thr Leu Lys Val Ala Tyr Ile Pro Asp Glu Thr Ala Ala
145                 150                 155                 160
Gln Gln Asn Pro Leu Gln Gln Pro Arg Gly Arg Arg Gly Leu Gly Gln
                165                 170                 175
Arg Gly Ser Ser Arg Gln Gly Ser Pro Gly Ser Val Ser Lys Gln Lys
            180                 185                 190
Pro Cys Asp Leu Pro Leu Arg Leu Leu Val Pro Thr Gln Phe Val Gly
        195                 200                 205
Ala Ile Ile Gly Lys Glu Gly Ala Thr Ile Arg Asn Ile Thr Lys Gln
    210                 215                 220
Thr Gln Ser Lys Ile Asp Val His Arg Lys Glu Asn Ala Gly Ala Ala
225                 230                 235                 240
Glu Lys Ser Ile Thr Ile Leu Ser Thr Pro Glu Gly Thr Ser Ala Ala
                245                 250                 255
Cys Lys Ser Ile Leu Glu Ile Met His Lys Glu Ala Gln Asp Ile Lys
            260                 265                 270
Phe Thr Glu Glu Ile Pro Leu Lys Ile Leu Ala His Asn Asn Phe Val
        275                 280                 285
Gly Arg Leu Ile Gly Lys Glu Gly Arg Asn Leu Lys Lys Ile Glu Gln
    290                 295                 300
Asp Thr Asp Thr Lys Ile Thr Ile Ser Pro Leu Gln Glu Leu Thr Leu
305                 310                 315                 320
Tyr Asn Pro Glu Arg Thr Ile Thr Val Lys Gly Asn Val Glu Thr Cys
                325                 330                 335
Ala Lys Ala Glu Glu Glu Ile Met Lys Lys Ile Arg Glu Ser Tyr Glu
            340                 345                 350
Asn Asp Ile Ala Ser Met Asn Leu Gln Ala His Leu Ile Pro Gly Leu
        355                 360                 365
Asn Leu Asn Ala Leu Gly Leu Phe Pro Pro Thr Ser Gly Met Pro Pro
    370                 375                 380
Pro Thr Ser Gly Pro Pro Ser Ala Met Thr Pro Pro Tyr Pro Gln Phe
385                 390                 395                 400
Glu Gln Ser Glu Thr Glu Thr Val His Leu Phe Ile Pro Ala Leu Ser
                405                 410                 415
Val Gly Ala Ile Ile Gly Lys Gln Gly Gln His Ile Lys Gln Leu Ser
            420                 425                 430
Arg Phe Ala Gly Ala Ser Ile Lys Ile Ala Pro Ala Glu Ala Pro Asp
        435                 440                 445
Ala Lys Val Arg Met Val Ile Ile Thr Gly Pro Pro Glu Ala Gln Phe
    450                 455                 460
Lys Ala Gln Gly Arg Ile Tyr Gly Lys Ile Lys Glu Glu Asn Phe Val
465                 470                 475                 480
Ser Pro Lys Glu Glu Val Lys Leu Glu Ala His Ile Arg Val Pro Ser
                485                 490                 495
Phe Ala Ala Gly Arg Val Ile Gly Lys Gly Gly Lys Thr Val Asn Glu

500                 505                 510
Leu Gln Asn Leu Ser Ser Ala Glu Val Val Val Pro Arg Asp Gln Thr
        515                 520                 525
Pro Asp Glu Asn Asp Gln Val Val Val Lys Ile Thr Gly His Phe Tyr
    530                 535                 540
Ala Cys Gln Val Ala Gln Arg Lys Ile Gln Glu Ile Leu Thr Gln Val
545                 550                 555                 560
Lys Gln His Gln Gln Gln Lys Ala Leu Gln Ser Gly Pro Pro Gln Ser
                565                 570                 575
Arg Arg Lys

<210> 349
<211> 207
<212> DNA
<213> Homo sapiens

<400> 349
atgtggcagc ccctcttctt caagtggctc ttgtcctgtt gccctgggag ttctcaaatt 60
gctgcagcag cctccaccca gcctgaggat gacatcaata cacagaggaa gaagagtcag 120
gaaaagatga gagaagttac agactctcct gggcgacccc gagagcttac cattcctcag 180
acttcttcac atggtgctaa cagattt                                     207

<210> 350
<211> 69
<212> PRT
<213> Homo sapiens

<400> 350
Met Trp Gln Pro Leu Phe Phe Lys Trp Leu Leu Ser Cys Cys Pro Gly
 1               5                  10                  15
Ser Ser Gln Ile Ala Ala Ala Ala Ser Thr Gln Pro Glu Asp Asp Ile
            20                  25                  30
Asn Thr Gln Arg Lys Lys Ser Gln Glu Lys Met Arg Glu Val Thr Asp
        35                  40                  45
Ser Pro Gly Arg Pro Arg Glu Leu Thr Ile Pro Gln Thr Ser Ser His
    50                  55                  60
Gly Ala Asn Arg Phe
65

<210> 351
<211> 1012
<212> DNA
<213> Homo sapiens

<400> 351
ccctctagaa ataattttgt ttaactttaa gaaggagata tacatatgca tcaccatcac 60
catcacacgg ccgcgtccga taacttccag ctgtcccagg gtgggcaggg attcgccatt 120
ccgatcgggc aggcgatggc gatcgcgggc cagatcaagc ttcccaccgt tcatatcggg 180
cctaccgcct tcctcggctt gggtgttgtc gacaacaacg gcaacggcgc acgagtccaa 240
cgcgtggtcg ggagcgctcc ggcggcaagt ctcggcatct ccaccggcga cgtgatcacc 300
gcggtcgacg gcgctccgat caactcggcc accgcgatgg cggacgcgct taacgggcat 360
catcccggtg acgtcatctc ggtgacctgg caaaccaagt cgggcggcac gcgtacaggg 420
aacgtgacat tggccgaggg acccccggcc gaattcatgg attgggggac gctgcacact 480
ttcatcgggg gtgtcaacaa acactccacc agcatcggga aggtgtggat cacagtcatc 540
tttattttcc gagtcatgat cctcgtggtg gctgcccagg aagtgtgggg tgacgagcaa 600

gaggacttcg tctgcaacac actgcaaccg ggatgcaaaa atgtgtgcta tgaccacttt 660
ttcccggtgt cccacatccg gctgtgggcc ctccagctga tcttcgtctc caccccagcg 720
ctgctggtgg ccatgcatgt ggcctactac aggcacgaaa ccactcgcaa gttcaggcga 780
ggagagaaga ggaatgattt caaagacata gaggacatta aaaagcagaa ggttcggata 840
gaggggtgac tcgagcacca ccaccaccac cactgagatc cggctgctaa caaagcccga 900
aaggaagctg agttggctgc tgccaccgct gagcaataac tagcataacc ccttggggcc 960
tctaaacggg tcttgagggg ttttttgctg aaaggaggaa ctatatccgg at 1012

<210> 352
<211> 267
<212> PRT
<213> Homo sapiens

<400> 352
Met His His His His His His Thr Ala Ala Ser Asp Asn Phe Gln Leu
1               5                   10                  15
Ser Gln Gly Gly Gln Gly Phe Ala Ile Pro Ile Gly Gln Ala Met Ala
            20                  25                  30
Ile Ala Gly Gln Ile Lys Leu Pro Thr Val His Ile Gly Pro Thr Ala
        35                  40                  45
Phe Leu Gly Leu Gly Val Val Asp Asn Asn Gly Asn Gly Ala Arg Val
    50                  55                  60
Gln Arg Val Val Gly Ser Ala Pro Ala Ala Ser Leu Gly Ile Ser Thr
65                  70                  75                  80
Gly Asp Val Ile Thr Ala Val Asp Gly Ala Pro Ile Asn Ser Ala Thr
                85                  90                  95
Ala Met Ala Asp Ala Leu Asn Gly His His Pro Gly Asp Val Ile Ser
            100                 105                 110
Val Thr Trp Gln Thr Lys Ser Gly Gly Thr Arg Thr Gly Asn Val Thr
        115                 120                 125
Leu Ala Glu Gly Pro Pro Ala Glu Phe Met Asp Trp Gly Thr Leu His
    130                 135                 140
Thr Phe Ile Gly Gly Val Asn Lys His Ser Thr Ser Ile Gly Lys Val
145                 150                 155                 160
Trp Ile Thr Val Ile Phe Ile Phe Arg Val Met Ile Leu Val Val Ala
                165                 170                 175
Ala Gln Glu Val Trp Gly Asp Glu Gln Glu Asp Phe Val Cys Asn Thr
            180                 185                 190
Leu Gln Pro Gly Cys Lys Asn Val Cys Tyr Asp His Phe Phe Pro Val
        195                 200                 205
Ser His Ile Arg Leu Trp Ala Leu Gln Leu Ile Phe Val Ser Thr Pro
    210                 215                 220
Ala Leu Leu Val Ala Met His Val Ala Tyr Tyr Arg His Glu Thr Thr
225                 230                 235                 240
Arg Lys Phe Arg Arg Gly Glu Lys Arg Asn Asp Phe Lys Asp Ile Glu
                245                 250                 255
Asp Ile Lys Lys Gln Lys Val Arg Ile Glu Gly
            260                 265

<210> 353
<211> 900
<212> DNA
<213> Homo sapiens

<400> 353
atgcatcacc atcaccatca cacggccgcg tccgataact tccagctgtc ccagggtggg 60
cagggattcg ccattccgat cgggcaggcg atggcgatcg cgggccagat caagcttccc 120

accgttcata tcgggcctac cgccttcctc ggcttgggtg ttgtcgacaa caacggcaac 180
ggcgcacgag tccaacgcgt ggtcgggagc gctccggcgg caagtctcgg catctccacc 240
ggcgacgtga tcaccgcggt cgacggcgct ccgatcaact cggccaccgc gatggcggac 300
gcgcttaacg ggcatcatcc cggtgacgtc atctcggtga cctggcaaac caagtcgggc 360
ggcacgcgta cagggaacgt gacattggcc gagggacccc cggccgaatt ccacgaaacc 420
actcgcaagt tcaggcgagg agagaagagg aatgatttca aagacataga ggacattaaa 480
aagcagaagg ttcggataga ggggtcgctg tggtggacgt acaccagcag catctttttc 540
cgaatcatct ttgaagcagc ctttatgtat gtgttttact tcctttacaa tgggtaccac 600
ctgccctggg tgttgaaatg tgggattgac cctgccccca accttgttga ctgctttatt 660
tctaggccaa cagagaagac cgtgtttacc atttttatga tttctgcgtc tgtgatttgc 720
atgctgctta acgtggcaga gttgtgctac ctgctgctga aagtgtgttt taggagatca 780
aagagagcac agacgcaaaa aaatcacccc aatcatgccc taaaggagag taagcagaat 840
gaaatgaatg agctgatttc agatagtggt caaaatgcaa tcacaggttt cccaagctaa 900

<210> 354
<211> 299
<212> PRT
<213> Homo sapiens

<400> 354
Met His His His His His His Thr Ala Ala Ser Asp Asn Phe Gln Leu
1               5                   10                  15
Ser Gln Gly Gly Gln Gly Phe Ala Ile Pro Ile Gly Gln Ala Met Ala
            20                  25                  30
Ile Ala Gly Gln Ile Lys Leu Pro Thr Val His Ile Gly Pro Thr Ala
        35                  40                  45
Phe Leu Gly Leu Gly Val Val Asp Asn Asn Gly Asn Gly Ala Arg Val
    50                  55                  60
Gln Arg Val Val Gly Ser Ala Pro Ala Ala Ser Leu Gly Ile Ser Thr
65                  70                  75                  80
Gly Asp Val Ile Thr Ala Val Asp Gly Ala Pro Ile Asn Ser Ala Thr
                85                  90                  95
Ala Met Ala Asp Ala Leu Asn Gly His His Pro Gly Asp Val Ile Ser
            100                 105                 110
Val Thr Trp Gln Thr Lys Ser Gly Gly Thr Arg Thr Gly Asn Val Thr
        115                 120                 125
Leu Ala Glu Gly Pro Pro Ala Glu Phe His Glu Thr Thr Arg Lys Phe
    130                 135                 140
Arg Arg Gly Glu Lys Arg Asn Asp Phe Lys Asp Ile Glu Asp Ile Lys
145                 150                 155                 160
Lys Gln Lys Val Arg Ile Glu Gly Ser Leu Trp Trp Thr Tyr Thr Ser
                165                 170                 175
Ser Ile Phe Phe Arg Ile Ile Phe Glu Ala Ala Phe Met Tyr Val Phe
            180                 185                 190
Tyr Phe Leu Tyr Asn Gly Tyr His Leu Pro Trp Val Leu Lys Cys Gly
        195                 200                 205
Ile Asp Pro Cys Pro Asn Leu Val Asp Cys Phe Ile Ser Arg Pro Thr
    210                 215                 220
Glu Lys Thr Val Phe Thr Ile Phe Met Ile Ser Ala Ser Val Ile Cys
225                 230                 235                 240
Met Leu Leu Asn Val Ala Glu Leu Cys Tyr Leu Leu Leu Lys Val Cys
                245                 250                 255
Phe Arg Arg Ser Lys Arg Ala Gln Thr Gln Lys Asn His Pro Asn His
            260                 265                 270
Ala Leu Lys Glu Ser Lys Gln Asn Glu Met Asn Glu Leu Ile Ser Asp
        275                 280                 285
Ser Gly Gln Asn Ala Ile Thr Gly Phe Pro Ser

290                 295

<210> 355
<211> 24
<212> DNA
<213> Artificial Sequence

<220>
<223> PCR primer

<400> 355
ggagtacagc ttcaagacaa tggg                                              24

<210> 356
<211> 31
<212> DNA
<213> Artificial Sequence

<220>
<223> PCR primer

<400> 356
ccatgggaat tcattataat aattttgttc c                                      31

<210> 357
<211> 920
<212> PRT
<213> Homo sapiens

<400> 357
Met Gln His His His His His His Gly Val Gln Leu Gln Asp Asn Gly
1               5                   10                  15
Tyr Asn Gly Leu Leu Ile Ala Ile Asn Pro Gln Val Pro Glu Asn Gln
            20                  25                  30
Asn Leu Ile Ser Asn Ile Lys Glu Met Ile Thr Glu Ala Ser Phe Tyr
        35                  40                  45
Leu Phe Asn Ala Thr Lys Arg Arg Val Phe Phe Arg Asn Ile Lys Ile
    50                  55                  60
Leu Ile Pro Ala Thr Trp Lys Ala Asn Asn Asn Ser Lys Ile Lys Gln
65                  70                  75                  80
Glu Ser Tyr Glu Lys Ala Asn Val Ile Val Thr Asp Trp Tyr Gly Ala
                85                  90                  95
His Gly Asp Asp Pro Tyr Thr Leu Gln Tyr Arg Gly Cys Gly Lys Glu
            100                 105                 110
Gly Lys Tyr Ile His Phe Thr Pro Asn Phe Leu Leu Asn Asp Asn Leu
        115                 120                 125
Thr Ala Gly Tyr Gly Ser Arg Gly Arg Val Phe Val His Glu Trp Ala
    130                 135                 140
His Leu Arg Trp Gly Val Phe Asp Glu Tyr Asn Asn Asp Lys Pro Phe
145                 150                 155                 160
Tyr Ile Asn Gly Gln Asn Gln Ile Lys Val Thr Arg Cys Ser Ser Asp
                165                 170                 175
Ile Thr Gly Ile Phe Val Cys Glu Lys Gly Pro Cys Pro Gln Glu Asn
            180                 185                 190
Cys Ile Ile Ser Lys Leu Phe Lys Glu Gly Cys Thr Phe Ile Tyr Asn
        195                 200                 205
Ser Thr Gln Asn Ala Thr Ala Ser Ile Met Phe Met Gln Ser Leu Ser

210                 215                 220
Ser Val Val Glu Phe Cys Asn Ala Ser Thr His Asn Gln Glu Ala Pro
225                 230                 235                 240
Asn Leu Gln Asn Gln Met Cys Ser Leu Arg Ser Ala Trp Asp Val Ile
                245                 250                 255
Thr Asp Ser Ala Asp Phe His His Ser Phe Pro Met Asn Gly Thr Glu
            260                 265                 270
Leu Pro Pro Pro Pro Thr Phe Ser Leu Val Glu Ala Gly Asp Lys Val
        275                 280                 285
Val Cys Leu Val Leu Asp Val Ser Ser Lys Met Ala Glu Ala Asp Arg
    290                 295                 300
Leu Leu Gln Leu Gln Gln Ala Ala Glu Phe Tyr Leu Met Gln Ile Val
305                 310                 315                 320
Glu Ile His Thr Phe Val Gly Ile Ala Ser Phe Asp Ser Lys Gly Glu
                325                 330                 335
Ile Arg Ala Gln Leu His Gln Ile Asn Ser Asn Asp Asp Arg Lys Leu
            340                 345                 350
Leu Val Ser Tyr Leu Pro Thr Thr Val Ser Ala Lys Thr Asp Ile Ser
        355                 360                 365
Ile Cys Ser Gly Leu Lys Lys Gly Phe Glu Val Val Glu Lys Leu Asn
    370                 375                 380
Gly Lys Ala Tyr Gly Ser Val Met Ile Leu Val Thr Ser Gly Asp Asp
385                 390                 395                 400
Lys Leu Leu Gly Asn Cys Leu Pro Thr Val Leu Ser Ser Gly Ser Thr
                405                 410                 415
Ile His Ser Ile Ala Leu Gly Ser Ser Ala Ala Pro Asn Leu Glu Glu
            420                 425                 430
Leu Ser Arg Leu Thr Gly Gly Leu Lys Phe Phe Val Pro Asp Ile Ser
        435                 440                 445
Asn Ser Asn Ser Met Ile Asp Ala Phe Ser Arg Ile Ser Ser Gly Thr
    450                 455                 460
Gly Asp Ile Phe Gln Gln His Ile Gln Leu Glu Ser Thr Gly Glu Asn
465                 470                 475                 480
Val Lys Pro His His Gln Leu Lys Asn Thr Val Thr Val Asp Asn Thr
                485                 490                 495
Val Gly Asn Asp Thr Met Phe Leu Val Thr Trp Gln Ala Ser Gly Pro
            500                 505                 510
Pro Glu Ile Ile Leu Phe Asp Pro Asp Gly Arg Lys Tyr Tyr Thr Asn
        515                 520                 525
Asn Phe Ile Thr Asn Leu Thr Phe Arg Thr Ala Ser Leu Trp Ile Pro
    530                 535                 540
Gly Thr Ala Lys Pro Gly His Trp Thr Tyr Thr Leu Asn Asn Thr His
545                 550                 555                 560
His Ser Leu Gln Ala Leu Lys Val Thr Val Thr Ser Arg Ala Ser Asn
                565                 570                 575
Ser Ala Val Pro Pro Ala Thr Val Glu Ala Phe Val Glu Arg Asp Ser
            580                 585                 590
Leu His Phe Pro His Pro Val Met Ile Tyr Ala Asn Val Lys Gln Gly
        595                 600                 605
Phe Tyr Pro Ile Leu Asn Ala Thr Val Thr Ala Thr Val Glu Pro Glu
    610                 615                 620
Thr Gly Asp Pro Val Thr Leu Arg Leu Leu Asp Asp Gly Ala Gly Ala
625                 630                 635                 640
Asp Val Ile Lys Asn Asp Gly Ile Tyr Ser Arg Tyr Phe Phe Ser Phe
                645                 650                 655
Ala Ala Asn Gly Arg Tyr Ser Leu Lys Val His Val Asn His Ser Pro
            660                 665                 670
Ser Ile Ser Thr Pro Ala His Ser Ile Pro Gly Ser His Ala Met Tyr

        675                 680                 685
Val Pro Gly Tyr Thr Ala Asn Gly Asn Ile Gln Met Asn Ala Pro Arg
    690                 695                 700
Lys Ser Val Gly Arg Asn Glu Glu Glu Arg Lys Trp Gly Phe Ser Arg
705                 710                 715                 720
Val Ser Ser Gly Gly Ser Phe Ser Val Leu Gly Val Pro Ala Gly Pro
                725                 730                 735
His Pro Asp Val Phe Pro Pro Cys Lys Ile Ile Asp Leu Glu Ala Val
            740                 745                 750
Lys Val Glu Glu Glu Leu Thr Leu Ser Trp Thr Ala Pro Gly Glu Asp
        755                 760                 765
Phe Asp Gln Gly Gln Ala Thr Ser Tyr Glu Ile Arg Met Ser Lys Ser
    770                 775                 780
Leu Gln Asn Ile Gln Asp Asp Phe Asn Asn Ala Ile Leu Val Asn Thr
785                 790                 795                 800
Ser Lys Arg Asn Pro Gln Gln Ala Gly Ile Arg Glu Ile Phe Thr Phe
                805                 810                 815
Ser Pro Gln Ile Ser Thr Asn Gly Pro Glu His Gln Pro Asn Gly Glu
            820                 825                 830
Thr His Glu Ser His Arg Ile Tyr Val Ala Ile Arg Ala Met Asp Arg
        835                 840                 845
Asn Ser Leu Gln Ser Ala Val Ser Asn Ile Ala Gln Ala Pro Leu Phe
    850                 855                 860
Ile Pro Pro Asn Ser Asp Pro Val Pro Ala Arg Asp Tyr Leu Ile Leu
865                 870                 875                 880
Lys Gly Val Leu Thr Ala Met Gly Leu Ile Gly Ile Ile Cys Leu Ile
                885                 890                 895
Ile Val Val Thr His His Thr Leu Ser Arg Lys Lys Arg Ala Asp Lys
            900                 905                 910
Lys Glu Asn Gly Thr Lys Leu Leu
        915                 920

<210> 358
<211> 2773
<212> DNA
<213> Homo sapiens

<400> 358
catatgcagc atcaccacca tcaccacgga gtacagcttc aagacaatgg gtataatgga 60
ttgctcattg caattaatcc tcaggtacct gagaatcaga acctcatctc aaacattaag 120
gaaatgataa ctgaagcttc attttaccta tttaatgcta ccaagagaag agtatttttc 180
agaaatataa agattttaat acctgccaca tggaaagcta ataataacag caaaataaaa 240
caagaatcat atgaaaaggc aaatgtcata gtgactgact ggtatggggc acatggagat 300
gatccataca ccctacaata cagagggtgt ggaaaagagg gaaaatacat tcatttcaca 360
cctaatttcc tactgaatga taacttaaca gctggctacg gatcacgagg ccgagtgttt 420
gtccatgaat gggcccacct ccgttggggt gtgttcgatg agtataacaa tgacaaacct 480
ttctacataa atgggcaaaa tcaaattaaa gtgacaaggt gttcatctga catcacaggc 540
atttttgtgt gtgaaaaagg tccttgcccc caagaaaact gtattattag taagcttttt 600
aaagaaggat gcacctttat ctacaatagc acccaaaatg caactgcatc aataatgttc 660
atgcaaagtt tatcttctgt ggttgaattt tgtaatgcaa gtacccacaa ccaagaagca 720
ccaaacctac agaaccagat gtgcagcctc agaagtgcat gggatgtaat cacagactct 780
gctgactttc accacagctt tcccatgaac gggactgagc ttccacctcc tcccacattc 840
tcgcttgtag aggctggtga caaagtggtc tgtttagtgc tggatgtgtc cagcaagatg 900
gcagaggctg acagactcct tcaactacaa caagccgcag aattttattt gatgcagatt 960
gttgaaattc ataccttcgt gggcattgcc agtttcgaca gcaaaggaga gatcagagcc 1020
cagctacacc aaattaacag caatgatgat cgaaagttgc tggtttcata tctgcccacc 1080
actgtatcag ctaaaacaga catcagcatt tgttcagggc ttaagaaagg atttgaggtg 1140

gttgaaaaac tgaatggaaa agcttatggc tctgtgatga tattagtgac cagcggagat 1200
gataagcttc ttggcaattg cttacccact gtgctcagca gtggttcaac aattcactcc 1260
attgccctgg gttcatctgc agccccaaat ctggaggaat tatcacgtct tacaggaggt 1320
ttaaagttct ttgttccaga tatatcaaac tccaatagca tgattgatgc tttcagtaga 1380
atttcctctg gaactggaga cattttccag caacatattc agcttgaaag tacaggtgaa 1440
aatgtcaaac ctcaccatca attgaaaaac acagtgactg tggataatac tgtgggcaac 1500
gacactatgt ttctagttac gtggcaggcc agtggtcctc ctgagattat attatttgat 1560
cctgatggac gaaaatacta cacaaataat tttatcacca atctaacttt tcggacagct 1620
agtctttgga ttccaggaac agctaagcct gggcactgga cttacaccct gaacaatacc 1680
catcattctc tgcaagccct gaaagtgaca gtgacctctc gcgcctccaa ctcagctgtg 1740
cccccagcca ctgtggaagc ctttgtggaa agagacagcc tccattttcc tcatcctgtg 1800
atgatttatg ccaatgtgaa acagggattt tatcccattc ttaatgccac tgtcactgcc 1860
acagttgagc cagagactgg agatcctgtt acgctgagac tccttgatga tggagcaggt 1920
gctgatgtta taaaaaatga tggaatttac tcgaggtatt ttttctcctt tgctgcaaat 2040
ggtagatata gcttgaaagt gcatgtcaat cactctccca gcataagcac cccagcccac 2040
tctattccag ggagtcatgc tatgtatgta ccaggttaca cagcaaacgg taatattcag 2100
atgaatgctc caaggaaatc agtaggcaga aatgaggagg agcgaaagtg gggctttagc 2160
cgagtcagct caggaggctc cttttcagtg ctgggagttc cagctggccc ccaccctgat 2220
gtgtttccac catgcaaaat tattgacctg gaagctgtaa aagtagaaga ggaattgacc 2280
ctatcttgga cagcacctgg agaagacttt gatcagggcc aggctacaag ctatgaaata 2340
agaatgagta aaagtctaca gaatatccaa gatgacttta acaatgctat tttagtaaat 2400
acatcaaagc gaaatcctca gcaagctggc atcagggaga tatttacgtt ctcaccccaa 2460
atttccacga atggacctga acatcagcca aatggagaaa cacatgaaag ccacagaatt 2520
tatgttgcaa tacgagcaat ggataggaac tccttacagt ctgctgtatc taacattgcc 2580
caggcgcctc tgtttattcc ccccaattct gatcctgtac ctgccagaga ttatcttata 2640
ttgaaaggag ttttaacagc aatgggtttg ataggaatca tttgccttat tatagttgtg 2700
acacatcata ctttaagcag gaaaaagaga gcagacaaga aagagaatgg aacaaaatta 2760
ttataatgaa ttc 2773

<210> 359
<211> 25
<212> DNA
<213> Artificial Sequence

<220>
<223> PCR primer

<400> 359
tggcagcccc tcttcttcaa gtggc 25

<210> 360
<211> 33
<212> DNA
<213> Artificial Sequence

<220>
<223> PCR primer

<400> 360
cgccagaatt catcaaacaa atctgttagc acc 33

<210> 361
<211> 77
<212> PRT
<213> Homo sapiens

<400> 361
Met Gln His His His His His His Trp Gln Pro Leu Phe Phe Lys Trp

1                   5                   10                  15
Leu Leu Ser Cys Cys Pro Gly Ser Ser Gln Ile Ala Ala Ala Ala Ser
                20                  25                  30
Thr Gln Pro Glu Asp Asp Ile Asn Thr Gln Arg Lys Lys Ser Gln Glu
            35                  40                  45
Lys Met Arg Glu Val Thr Asp Ser Pro Gly Arg Pro Arg Glu Leu Thr
    50                  55                  60
Ile Pro Gln Thr Ser Ser His Gly Ala Asn Arg Phe Val
65                  70                  75


<210> 362
<211> 244
<212> DNA
<213> Homo sapiens

<400> 362
catatgcagc atcaccacca tcaccactgg cagcccctct tcttcaagtg gctcttgtcc 60
tgttgccctg ggagttctca aattgctgca gcagcctcca cccagcctga ggatgacatc 120
aatacacaga ggaagaagag tcaggaaaag atgagagaag ttacagactc tcctgggcga 180
ccccgagagc ttaccattcc tcagacttct tcacatggtg ctaacagatt tgtttgatga 240
attc                                                              244

<210> 363
<211> 20
<212> PRT
<213> Homo sapiens

<400> 363
Met Trp Gln Pro Leu Phe Phe Lys Trp Leu Leu Ser Cys Cys Pro Gly
1               5                   10                  15
Ser Ser Gln Ile
            20


<210> 364
<211> 60
<212> DNA
<213> Homo sapiens

<400> 364
atgtggcagc ccctcttctt caagtggctc ttgtcctgtt gccctgggag ttctcaaatt 60


<210> 365
<211> 20
<212> PRT
<213> Homo sapiens

<400> 365
Gly Ser Ser Gln Ile Ala Ala Ala Ala Ser Thr Gln Pro Glu Asp Asp
1               5                   10                  15
Ile Asn Thr Gln
            20


<210> 366
<211> 60

<212> DNA
<213> Homo sapiens

<400> 366
gggagttctc aaattgctgc agcagcctcc acccagcctg aggatgacat caatacacag 60


<210> 367
<211> 20
<212> PRT
<213> Homo sapiens

<400> 367
Lys Pro Gly His Trp Thr Tyr Thr Leu Asn Asn Thr His His Ser Leu
1               5                   10                  15
Gln Ala Leu Lys
            20


<210> 368
<211> 2343
<212> DNA
<213> Homo sapiens

<400> 368
attccggagc gtttgcggct tcgcttcatg gccgctctcc cgcccctcct gggatctgtg 60
gggagctggg gagcccgcag cggcccggag ccggagctgg cgagccgagc ggagacctgt 120
gcgccgcgcc tctgaggcgc agcatgtgaa gcggagacgg catccagtgg ggggcgagcc 180
tctcagccgg ccgggatggc taccacggcc gagctcttcg aggagccttt tgtggcagat 240
gaatatattg aacgtcttgt atggagaacc ccaggaggag gctctagagg tggacctgaa 300
gcttttgatc ctaaaagatt attagaagaa tttgtaaatc atattcagga actccagata 360
atggatgaaa ggattcagag gaaagtagag aaactagagc aacaatgtca gaaagaagcc 420
aaggaatttg ccaagaaggt acaagagctg cagaaaagca atcaggttgc cttccaacat 480
ttccaagaac tagatgagca cattagctat gtagcaacta aagtctgtca ccttggagac 540
cagttagagg gggtaaacac acccagacaa cgggcagtgg aggctcagaa attgatgaaa 600
tactttaatg agtttctaga tggagaattg aaatctgatg tttttacaaa ttctgaaaag 660
ataaaggaag cagcagacat cattcagaag ttgcacctaa ttgcccaaga gttacctttt 720
gatagatttt cagaagttaa atccaaaatt gcaagtaaat accatgattt agaatgccag 780
ctgattcagg agtttaccag tgctcaaaga agaggtgaaa tctccagaat gagagaagta 840
gcagcagttt tacttcattt taagggttat tcccattgtg ttgatgttta tataaagcag 900
tgccaggagg gtgcttattt gagaaatgat atatttgaag acgctggaat actctgtcaa 960
agagtgaaca aacaagttgg agatatcttc agtaatccag aaacagtcct ggctaaactt 1020
attcaaaatg tatttgaaat caaactacag agttttgtga aagagcagtt agaagaatgt 1080
aggaagtccg atgcagagca atatctcaaa aatctctatg atctgtatac aagaaccacc 1140
aatctttcca gcaagctgat ggagtttaat ttaggtactg ataaacagac tttcttgtct 1200
aagcttatca aatccatttt catttcctat ttggagaact atattgaggt ggagactgga 1260
tatttgaaaa gcagaagtgc tatgatccta cagcgctatt atgattcgaa aaaccatcaa 1320
aagagatcca ttggcacagg aggtattcaa gatttgaagg aaagaattag acagcgtacc 1380
aacttaccac ttgggccaag tatcgatact catggggaga cttttctatc ccaagaagtg 1440
gtggttaatc ttttacaaga aaccaaacaa gcctttgaaa gatgtcatag gctctctgat 1500
ccttctgact taccaaggaa tgccttcaga atttttacca ttcttgtgga atttttatgt 1560
attgagcata ttgattatgc tttggaaaca ggacttgctg gaattccctc ttcagattct 1620
aggaatgcaa atctttattt tttggacgtt gtgcaacagg ccaatactat ttttcatctt 1680
tttgacaaac agtttaatga tcaccttatg ccactaataa gctcttctcc taagttatct 1740
gaatgccttc agaagaaaaa agaaataatt gaacaaatgg agatgaaatt ggatactggc 1800
attgatagga cattaaattg tatgattgga cagatgaagc atattttggc tgcagaacag 1860
aagaaaacag attttaagcc agaagatgaa aacaatgttt tgattcaata tactaatgcc 1920
tgtgtaaaag tctgtgctta cgtaagaaaa caagtggaga agattaaaaa ttccatggat 1980

gggaagaatg tggatacagt tttgatggaa cttggagtac gttttcatcg acttatctat 2040
gagcatcttc aacaatattc ctacagttgt atgggtggca tgttggccat ttgtgatgta 2100
gccgaatata ggaagtgtgc caaagacttc aagattccaa tggtattaca tctttttgat 2160
actctgcatg ctctttgcaa tcttctggta gttgccccag ataatttaaa gcaagtctgc 2220
tcaggagaac aacttgctaa tctggacaag aatatacttc actccttcgt acaacttcgt 2280
gctgattata gatctgcccg ccttgctcga cacttcagct gagattgaat ttacaaagga 2340
att 2343

<210> 369
<211> 708
<212> PRT
<213> Homo sapiens

<400> 369
Met Ala Thr Thr Ala Glu Leu Phe Glu Glu Pro Phe Val Ala Asp Glu
1               5                   10                  15
Tyr Ile Glu Arg Leu Val Trp Arg Thr Pro Gly Gly Gly Ser Arg Gly
            20                  25                  30
Gly Pro Glu Ala Phe Asp Pro Lys Arg Leu Leu Glu Glu Phe Val Asn
        35                  40                  45
His Ile Gln Glu Leu Gln Ile Met Asp Glu Arg Ile Gln Arg Lys Val
    50                  55                  60
Glu Lys Leu Glu Gln Gln Cys Gln Lys Glu Ala Lys Glu Phe Ala Lys
65                  70                  75                  80
Lys Val Gln Glu Leu Gln Lys Ser Asn Gln Val Ala Phe Gln His Phe
                85                  90                  95
Gln Glu Leu Asp Glu His Ile Ser Tyr Val Ala Thr Lys Val Cys His
            100                 105                 110
Leu Gly Asp Gln Leu Glu Gly Val Asn Thr Pro Arg Gln Arg Ala Val
        115                 120                 125
Glu Ala Gln Lys Leu Met Lys Tyr Phe Asn Glu Phe Leu Asp Gly Glu
    130                 135                 140
Leu Lys Ser Asp Val Phe Thr Asn Ser Glu Lys Ile Lys Glu Ala Ala
145                 150                 155                 160
Asp Ile Ile Gln Lys Leu His Leu Ile Ala Gln Glu Leu Pro Phe Asp
                165                 170                 175
Arg Phe Ser Glu Val Lys Ser Lys Ile Ala Ser Lys Tyr His Asp Leu
            180                 185                 190
Glu Cys Gln Leu Ile Gln Glu Phe Thr Ser Ala Gln Arg Arg Gly Glu
        195                 200                 205
Ile Ser Arg Met Arg Glu Val Ala Ala Val Leu Leu His Phe Lys Gly
    210                 215                 220
Tyr Ser His Cys Val Asp Val Tyr Ile Lys Gln Cys Gln Glu Gly Ala
225                 230                 235                 240
Tyr Leu Arg Asn Asp Ile Phe Glu Asp Ala Gly Ile Leu Cys Gln Arg
                245                 250                 255
Val Asn Lys Gln Val Gly Asp Ile Phe Ser Asn Pro Glu Thr Val Leu
            260                 265                 270
Ala Lys Leu Ile Gln Asn Val Phe Glu Ile Lys Leu Gln Ser Phe Val
        275                 280                 285
Lys Glu Gln Leu Glu Glu Cys Arg Lys Ser Asp Ala Glu Gln Tyr Leu
    290                 295                 300
Lys Asn Leu Tyr Asp Leu Tyr Thr Arg Thr Thr Asn Leu Ser Ser Lys
305                 310                 315                 320
Leu Met Glu Phe Asn Leu Gly Thr Asp Lys Gln Thr Phe Leu Ser Lys
                325                 330                 335
Leu Ile Lys Ser Ile Phe Ile Ser Tyr Leu Glu Asn Tyr Ile Glu Val
            340                 345                 350

Glu Thr Gly Tyr Leu Lys Ser Arg Ser Ala Met Ile Leu Gln Arg Tyr
        355                 360                 365
Tyr Asp Ser Lys Asn His Gln Lys Arg Ser Ile Gly Thr Gly Gly Ile
    370                 375                 380
Gln Asp Leu Lys Glu Arg Ile Arg Gln Arg Thr Asn Leu Pro Leu Gly
385                 390                 395                 400
Pro Ser Ile Asp Thr His Gly Glu Thr Phe Leu Ser Gln Glu Val Val
                405                 410                 415
Val Asn Leu Leu Gln Glu Thr Lys Gln Ala Phe Glu Arg Cys His Arg
            420                 425                 430
Leu Ser Asp Pro Ser Asp Leu Pro Arg Asn Ala Phe Arg Ile Phe Thr
        435                 440                 445
Ile Leu Val Glu Phe Leu Cys Ile Glu His Ile Asp Tyr Ala Leu Glu
    450                 455                 460
Thr Gly Leu Ala Gly Ile Pro Ser Ser Asp Ser Arg Asn Ala Asn Leu
465                 470                 475                 480
Tyr Phe Leu Asp Val Val Gln Gln Ala Asn Thr Ile Phe His Leu Phe
                485                 490                 495
Asp Lys Gln Phe Asn Asp His Leu Met Pro Leu Ile Ser Ser Ser Pro
            500                 505                 510
Lys Leu Ser Glu Cys Leu Gln Lys Lys Lys Glu Ile Ile Glu Gln Met
        515                 520                 525
Glu Met Lys Leu Asp Thr Gly Ile Asp Arg Thr Leu Asn Cys Met Ile
    530                 535                 540
Gly Gln Met Lys His Ile Leu Ala Ala Glu Gln Lys Lys Thr Asp Phe
545                 550                 555                 560
Lys Pro Glu Asp Glu Asn Asn Val Leu Ile Gln Tyr Thr Asn Ala Cys
                565                 570                 575
Val Lys Val Cys Ala Tyr Val Arg Lys Gln Val Glu Lys Ile Lys Asn
            580                 585                 590
Ser Met Asp Gly Lys Asn Val Asp Thr Val Leu Met Glu Leu Gly Val
        595                 600                 605
Arg Phe His Arg Leu Ile Tyr Glu His Leu Gln Gln Tyr Ser Tyr Ser
    610                 615                 620
Cys Met Gly Gly Met Leu Ala Ile Cys Asp Val Ala Glu Tyr Arg Lys
625                 630                 635                 640
Cys Ala Lys Asp Phe Lys Ile Pro Met Val Leu His Leu Phe Asp Thr
                645                 650                 655
Leu His Ala Leu Cys Asn Leu Leu Val Val Ala Pro Asp Asn Leu Lys
            660                 665                 670
Gln Val Cys Ser Gly Glu Gln Leu Ala Asn Leu Asp Lys Asn Ile Leu
        675                 680                 685
His Ser Phe Val Gln Leu Arg Ala Asp Tyr Arg Ser Ala Arg Leu Ala
    690                 695                 700
Arg His Phe Ser
705


<210> 370
<211> 60
<212> DNA
<213> Homo sapiens

<400> 370
gtcaatcact ctcccagcat aagcacccca gcccactcta ttccagggag tcatgctatg 60


<210> 371

<211> 60
<212> DNA
<213> Homo sapiens

<400> 371
agtagaattt cctctggaac tggagacatt ttccagcaac atattcagct tgaaagtaca 60


<210> 372
<211> 60
<212> DNA
<213> Homo sapiens

<400> 372
ccagagactg gagatcctgt tacgctgaga ctccttgatg atggagcagg tgctgatgtt 60


<210> 373
<211> 60
<212> DNA
<213> Homo sapiens

<400> 373
ttacagtctg ctgtatctaa cattgcccag gcgcctctgt ttattccccc caattctgat 60


<210> 374
<211> 60
<212> DNA
<213> Homo sapiens

<400> 374
gctgtgcccc cagccactgt ggaagccttt gtggaaagag acagcctcca ttttcctcat 60


<210> 375
<211> 60
<212> DNA
<213> Homo sapiens

<400> 375
aaaaacacag tgactgtgga taatactgtg ggcaacgaca ctatgtttct agttacgtgg 60


<210> 376
<211> 20
<212> PRT
<213> Homo sapiens

<400> 376
Leu Gln Ser Ala Val Ser Asn Ile Ala Gln Ala Pro Leu Phe Ile Pro
1               5                   10                  15
Pro Asn Ser Asp
            20


<210> 377
<211> 20

<212> PRT
<213> Homo sapiens

<400> 377
Val Asn His Ser Pro Ser Ile Ser Thr Pro Ala His Ser Ile Pro Gly
1               5                   10                  15
Ser His Ala Met
            20


<210> 378
<211> 20
<212> PRT
<213> Homo sapiens

<400> 378
Pro Glu Thr Gly Asp Pro Val Thr Leu Arg Leu Leu Asp Asp Gly Ala
1               5                   10                  15
Gly Ala Asp Val
            20


<210> 379
<211> 20
<212> PRT
<213> Homo sapiens

<400> 379
Ala Val Pro Pro Ala Thr Val Glu Ala Phe Val Glu Arg Asp Ser Leu
1               5                   10                  15
His Phe Pro His
            20


<210> 380
<211> 20
<212> PRT
<213> Homo sapiens

<400> 380
Ser Arg Ile Ser Ser Gly Thr Gly Asp Ile Phe Gln Gln His Ile Gln
1               5                   10                  15
Leu Glu Ser Thr
            20


<210> 381
<211> 20
<212> PRT
<213> Homo sapiens

<400> 381
Lys Asn Thr Val Thr Val Asp Asn Thr Val Gly Asn Asp Thr Met Phe
1               5                   10                  15
Leu Val Thr Trp
            20

<210> 382
<211> 20
<212> PRT
<213> Homo sapiens

<400> 382
Lys Pro Gly His Trp Thr Tyr Thr Leu Asn Asn Thr His His Ser Leu
1               5                   10                  15
Gln Ala Leu Lys
            20


<210> 383
<211> 29
<212> DNA
<213> Artificial Sequence

<220>
<223> PCR primer

<400> 383
cggcgaattc atggattggg ggacgctgc                                        29

<210> 384
<211> 35
<212> DNA
<213> Artificial Sequence

<220>
<223> PCR primer

<400> 384
cggcctcgag tcacccctct atccgaacct tctgc                                 35

<210> 385
<211> 32
<212> DNA
<213> Artificial Sequence

<220>
<223> PCR primer

<400> 385
cggcgaattc cacgaaccac tcgcaagttc ag                                    32

<210> 386
<211> 30
<212> DNA
<213> Artificial Sequence

<220>
<223> PCR primer

<400> 386
cggctcgagt tagcttgggc ctgtgattgc                                       30

<210> 387
<211> 20

<212> PRT
<213> Homo sapiens

<400> 387
Phe Phe Lys Trp Leu Leu Ser Cys Cys Pro Gly Ser Ser Gln Ile Ala
1               5                   10                  15
Ala Ala Ala Ser
            20


<210> 388
<211> 19
<212> PRT
<213> Homo sapiens

<400> 388
Leu Ser Cys Cys Pro Gly Ser Ser Gln Ile Ala Ala Ala Ser Thr Gln
1               5                   10                  15
Pro Glu Asp


<210> 389
<211> 20
<212> PRT
<213> Homo sapiens

<400> 389
Ala Ala Ala Ala Ser Thr Gln Pro Glu Asp Asp Ile Asn Thr Gln Arg
1               5                   10                  15
Lys Lys Ser Gln
            20


<210> 390
<211> 20
<212> PRT
<213> Homo sapiens

<400> 390
Thr Gln Pro Glu Asp Asp Ile Asn Thr Gln Arg Lys Lys Ser Gln Glu
1               5                   10                  15
Lys Met Arg Glu
            20


<210> 391
<211> 20
<212> PRT
<213> Homo sapiens

<400> 391
Asp Ile Asn Thr Gln Arg Lys Lys Ser Gln Glu Lys Met Arg Glu Val
1               5                   10                  15
Thr Asp Ser Pro
            20

<210> 392
<211> 20
<212> PRT
<213> Homo sapiens

<400> 392
Arg Lys Lys Ser Gln Glu Lys Met Arg Glu Val Thr Asp Ser Pro Gly
1               5                   10                  15
Arg Pro Arg Glu
            20

<210> 393
<211> 20
<212> PRT
<213> Homo sapiens

<400> 393
Glu Lys Met Arg Glu Val Thr Asp Ser Pro Gly Arg Pro Arg Glu Leu
1               5                   10                  15
Thr Ile Pro Gln
            20

<210> 394
<211> 20
<212> PRT
<213> Homo sapiens

<400> 394
Val Thr Asp Ser Pro Gly Arg Pro Arg Glu Leu Thr Ile Pro Gln Thr
1               5                   10                  15
Ser Ser His Gly
            20

<210> 395
<211> 19
<212> PRT
<213> Homo sapiens

<400> 395
Gly Arg Pro Arg Glu Leu Thr Ile Pro Gln Thr Ser Ser His Gly Ala
1               5                   10                  15
Asn Arg Phe

<210> 396
<211> 19
<212> PRT
<213> Homo sapiens

<400> 396
Met Asn Lys Leu Tyr Ile Gly Asn Leu Ser Glu Asn Ala Ala Pro Ser
1               5                   10                  15
Asp Leu Glu

<210> 397
<211> 20
<212> PRT
<213> Homo sapiens

<400> 397
Ser Glu Asn Ala Ala Pro Ser Asp Leu Glu Ser Ile Phe Lys Asp Ala
1               5                   10                  15
Lys Ile Pro Val
            20

<210> 398
<211> 20
<212> PRT
<213> Homo sapiens

<400> 398
Ser Ile Phe Lys Asp Ala Lys Ile Pro Val Ser Gly Pro Phe Leu Val
1               5                   10                  15
Lys Thr Gly Tyr
            20

<210> 399
<211> 20
<212> PRT
<213> Homo sapiens

<400> 399
Ser Gly Pro Phe Leu Val Lys Thr Gly Tyr Ala Phe Val Asp Cys Pro
1               5                   10                  15
Asp Glu Ser Trp
            20

<210> 400
<211> 20
<212> PRT
<213> Homo sapiens

<400> 400
Ala Phe Val Asp Cys Pro Asp Glu Ser Trp Ala Leu Lys Ala Ile Glu
1               5                   10                  15
Ala Leu Ser Gly
            20

<210> 401
<211> 20
<212> PRT
<213> Homo sapiens

<400> 401
Ala Leu Lys Ala Ile Glu Ala Leu Ser Gly Lys Ile Glu Leu His Gly
1               5                   10                  15

Lys Pro Ile Glu
            20

<210> 402
<211> 20
<212> PRT
<213> Homo sapiens

<400> 402
Lys Ile Glu Leu His Gly Lys Pro Ile Glu Val Glu His Ser Val Pro
1               5                   10                  15
Lys Arg Gln Arg
            20

<210> 403
<211> 20
<212> PRT
<213> Homo sapiens

<400> 403
Val Glu His Ser Val Pro Lys Arg Gln Arg Ile Arg Lys Leu Gln Ile
1               5                   10                  15
Arg Asn Ile Pro
            20

<210> 404
<211> 20
<212> PRT
<213> Homo sapiens

<400> 404
Ile Arg Lys Leu Gln Ile Arg Asn Ile Pro Pro His Leu Gln Trp Glu
1               5                   10                  15
Val Leu Asp Ser
            20

<210> 405
<211> 20
<212> PRT
<213> Homo sapiens

<400> 405
Ala Val Val Asn Val Thr Tyr Ser Ser Lys Asp Gln Ala Arg Gln Ala
1               5                   10                  15
Leu Asp Lys Leu
            20

<210> 406
<211> 20
<212> PRT
<213> Homo sapiens

<400> 406

Asp Gln Ala Arg Gln Ala Leu Asp Lys Leu Asn Gly Phe Gln Leu Glu
1               5                   10                  15
Asn Phe Thr Leu
            20

<210> 407
<211> 20
<212> PRT
<213> Homo sapiens

<400> 407
Asn Gly Phe Gln Leu Glu Asn Phe Thr Leu Lys Val Ala Tyr Ile Pro
1               5                   10                  15
Asp Glu Thr Ala
            20

<210> 408
<211> 20
<212> PRT
<213> Homo sapiens

<400> 408
Lys Val Ala Tyr Ile Pro Asp Glu Thr Ala Ala Gln Gln Asn Pro Leu
1               5                   10                  15
Gln Gln Pro Arg
            20

<210> 409
<211> 20
<212> PRT
<213> Homo sapiens

<400> 409
Ala Gln Gln Asn Pro Leu Gln Gln Pro Arg Gly Arg Arg Gly Leu Gly
1               5                   10                  15
Gln Arg Gly Ser
            20

<210> 410
<211> 20
<212> PRT
<213> Homo sapiens

<400> 410
Gly Arg Arg Gly Leu Gly Gln Arg Gly Ser Ser Arg Gln Gly Ser Pro
1               5                   10                  15
Gly Ser Val Ser
            20

<210> 411
<211> 20
<212> PRT
<213> Homo sapiens

<400> 411
Ser Arg Gln Gly Ser Pro Gly Ser Val Ser Lys Gln Lys Pro Cys Asp
1               5                   10                  15
Leu Pro Leu Arg
            20


<210> 412
<211> 20
<212> PRT
<213> Homo sapiens

<400> 412
Lys Gln Lys Pro Cys Asp Leu Pro Leu Arg Leu Leu Val Pro Thr Gln
1               5                   10                  15
Phe Val Gly Ala
            20


<210> 413
<211> 20
<212> PRT
<213> Homo sapiens

<400> 413
Leu Leu Val Pro Thr Gln Phe Val Gly Ala Ile Ile Gly Lys Glu Gly
1               5                   10                  15
Ala Thr Ile Arg
            20


<210> 414
<211> 20
<212> PRT
<213> Homo sapiens

<400> 414
Ile Ile Gly Lys Glu Gly Ala Thr Ile Arg Asn Ile Thr Lys Gln Thr
1               5                   10                  15
Gln Ser Lys Ile
            20


<210> 415
<211> 20
<212> PRT
<213> Homo sapiens

<400> 415
Asn Ile Thr Lys Gln Thr Gln Ser Lys Ile Asp Val His Arg Lys Glu
1               5                   10                  15
Asn Ala Gly Ala
            20


<210> 416
<211> 20

<212> PRT
<213> Homo sapiens

<400> 416
Asp Val His Arg Lys Glu Asn Ala Gly Ala Ala Glu Lys Ser Ile Thr
1               5                   10                  15
Ile Leu Ser Thr
            20


<210> 417
<211> 20
<212> PRT
<213> Homo sapiens

<400> 417
Ala Glu Lys Ser Ile Thr Ile Leu Ser Thr Pro Glu Gly Thr Ser Ala
1               5                   10                  15
Ala Cys Lys Ser
            20


<210> 418
<211> 20
<212> PRT
<213> Homo sapiens

<400> 418
Pro Glu Gly Thr Ser Ala Ala Cys Lys Ser Ile Leu Glu Ile Met His
1               5                   10                  15
Lys Glu Ala Gln
            20


<210> 419
<211> 20
<212> PRT
<213> Homo sapiens

<400> 419
Ile Leu Glu Ile Met His Lys Glu Ala Gln Asp Ile Lys Phe Thr Glu
1               5                   10                  15
Glu Ile Pro Leu
            20


<210> 420
<211> 455
<212> DNA
<213> Homo sapiens

<400> 420
gaagacatgc ttacttcccc ttcaccttcc ttcatgatgt gggaagagtg ctgcaaccca 60
gccctagcca acgccgcatg agagggagtg tgccgagggc ttctgagaag gtttctctca 120
catctagaaa gaagcgctta agatgtggca gcccctcttc ttcaagtggc tcttgtcctg 180
ttgccctggg agttctcaaa ttgctgcagc agcctccacc cagcctgagg atgacatcaa 240
tacacagagg aagaagagtc aggaaaagat gagagaagtt acagactctc ctgggcgacc 300
ccgagagctt accattcctc agacttcttc acatggtgct aacagatttg ttcctaaaag 360

taaagctcta gaggccgtca aattggcaat agaagccggg ttccaccata ttgattctgc 420
acatgtttac aataatgagg agcaggttgg actgg 455

<210> 421
<211> 24
<212> DNA
<213> Artificial Sequence

<220>
<223> PCR primer

<400> 421
actagtgtcc gcgtggcggc ctac 24

<210> 422
<211> 34
<212> DNA
<213> Artificial Sequence

<220>
<223> PCR primer

<400> 422
catgagaatt catcacatgc ccttgaaggc tccc 34

<210> 423
<211> 161
<212> PRT
<213> Homo sapiens

<400> 423
Met Gln His His His His His His His Thr Ser Val Arg Val Ala Ala
1               5                   10                  15
Tyr Phe Glu Asn Phe Leu Ala Ala Trp Arg Pro Val Lys Ala Ser Asp
            20                  25                  30
Gly Asp Tyr Tyr Thr Leu Ala Val Pro Met Gly Asp Val Pro Met Asp
        35                  40                  45
Gly Ile Ser Val Ala Asp Ile Gly Ala Ala Val Ser Ser Ile Phe Asn
    50                  55                  60
Ser Pro Glu Glu Phe Leu Gly Lys Ala Val Gly Leu Ser Ala Glu Ala
65                  70                  75                  80
Leu Thr Ile Gln Gln Tyr Ala Asp Val Leu Ser Lys Ala Leu Gly Lys
                85                  90                  95
Glu Val Arg Asp Ala Lys Ile Thr Pro Glu Ala Phe Glu Lys Leu Gly
            100                 105                 110
Phe Pro Ala Ala Lys Glu Ile Ala Asn Met Cys Arg Phe Tyr Glu Met
        115                 120                 125
Lys Pro Asp Arg Asp Val Asn Leu Thr His Gln Leu Asn Pro Lys Val
    130                 135                 140
Lys Ser Phe Ser Gln Phe Ile Ser Glu Asn Gln Gly Ala Phe Lys Gly
145                 150                 155                 160
Met

<210> 424
<211> 489
<212> DNA

<213> Homo sapiens

<400> 424
atgcagcatc accaccatca ccaccacact agtgtccgcg tggcggccta ctttgaaaac 60
tttctcgcgg cgtggcggcc cgtgaaagcc tctgatggag attactacac cttggctgta 120
ccgatgggag atgtaccaat ggatggtatc tctgttgctg atattggagc agccgtctct 180
agcattttta attctccaga ggaattttta ggcaaggccg tggggctcag tgcagaagca 240
ctaacaatac agcaatatgc tgatgttttg tccaaggctt tggggaaaga agtccgagat 300
gcaaagatta ccccggaagc tttcgagaag ctgggattcc ctgcagcaaa ggaaatagcc 360
aatatgtgtc gtttctatga aatgaagcca gaccgagatg tcaatctcac ccaccaacta 420
aatcccaaag tcaaaagctt cagccagttt atctcagaga accagggagc cttcaagggc 480
atgtgatga 489

<210> 425
<211> 32
<212> DNA
<213> Artificial Sequence

<220>
<223> PCR primer

<400> 425
aacaaactgt atatcggaaa cctcagcgag aa 32

<210> 426
<211> 33
<212> DNA
<213> Artificial Sequence

<220>
<223> PCR primer

<400> 426
ccatagaatt cattacttcc gtcttgactg agg 33

<210> 427
<211> 586
<212> PRT
<213> Homo sapiens

<400> 427
Met Gln His His His His His His Asn Lys Leu Tyr Ile Gly Asn Leu
1               5                   10                  15
Ser Glu Asn Ala Ala Pro Ser Asp Leu Glu Ser Ile Phe Lys Asp Ala
            20                  25                  30
Lys Ile Pro Val Ser Gly Pro Phe Leu Val Lys Thr Gly Tyr Ala Phe
        35                  40                  45
Val Asp Cys Pro Asp Glu Ser Trp Ala Leu Lys Ala Ile Glu Ala Leu
    50                  55                  60
Ser Gly Lys Ile Glu Leu His Gly Lys Pro Ile Glu Val Glu His Ser
65                  70                  75                  80
Val Pro Lys Arg Gln Arg Ile Arg Lys Leu Gln Ile Arg Asn Ile Pro
                85                  90                  95
Pro His Leu Gln Trp Glu Val Leu Asp Ser Leu Leu Val Gln Tyr Gly
            100                 105                 110
Val Val Glu Ser Cys Glu Gln Val Asn Thr Asp Ser Glu Thr Ala Val
        115                 120                 125
Val Asn Val Thr Tyr Ser Ser Lys Asp Gln Ala Arg Gln Ala Leu Asp

130                 135                 140
Lys Leu Asn Gly Phe Gln Leu Glu Asn Phe Thr Leu Lys Val Ala Tyr
145                 150                 155                 160
Ile Pro Asp Glu Thr Ala Ala Gln Gln Asn Pro Leu Gln Gln Pro Arg
                165                 170                 175
Gly Arg Arg Gly Leu Gly Gln Arg Gly Ser Ser Arg Gln Gly Ser Pro
            180                 185                 190
Gly Ser Val Ser Lys Gln Lys Pro Cys Asp Leu Pro Leu Arg Leu Leu
        195                 200                 205
Val Pro Thr Gln Phe Val Gly Ala Ile Ile Gly Lys Glu Gly Ala Thr
    210                 215                 220
Ile Arg Asn Ile Thr Lys Gln Thr Gln Ser Lys Ile Asp Val His Arg
225                 230                 235                 240
Lys Glu Asn Ala Gly Ala Ala Glu Lys Ser Ile Thr Ile Leu Ser Thr
                245                 250                 255
Pro Glu Gly Thr Ser Ala Ala Cys Lys Ser Ile Leu Glu Ile Met His
            260                 265                 270
Lys Glu Ala Gln Asp Ile Lys Phe Thr Glu Glu Ile Pro Leu Lys Ile
        275                 280                 285
Leu Ala His Asn Asn Phe Val Gly Arg Leu Ile Gly Lys Glu Gly Arg
    290                 295                 300
Asn Leu Lys Lys Ile Glu Gln Asp Thr Asp Thr Lys Ile Thr Ile Ser
305                 310                 315                 320
Pro Leu Gln Glu Leu Thr Leu Tyr Asn Pro Glu Arg Thr Ile Thr Val
                325                 330                 335
Lys Gly Asn Val Glu Thr Cys Ala Lys Ala Glu Glu Glu Ile Met Lys
            340                 345                 350
Lys Ile Arg Glu Ser Tyr Glu Asn Asp Ile Ala Ser Met Asn Leu Gln
        355                 360                 365
Ala His Leu Ile Pro Gly Leu Asn Leu Asn Ala Leu Gly Leu Phe Pro
    370                 375                 380
Pro Thr Ser Gly Met Pro Pro Pro Thr Ser Gly Pro Pro Ser Ala Met
385                 390                 395                 400
Thr Pro Pro Tyr Pro Gln Phe Glu Gln Ser Glu Thr Glu Thr Val His
                405                 410                 415
Leu Phe Ile Pro Ala Leu Ser Val Gly Ala Ile Ile Gly Lys Gln Gly
            420                 425                 430
Gln His Ile Lys Gln Leu Ser Arg Phe Ala Gly Ala Ser Ile Lys Ile
        435                 440                 445
Ala Pro Ala Glu Ala Pro Asp Ala Lys Val Arg Met Val Ile Ile Thr
    450                 455                 460
Gly Pro Pro Glu Ala Gln Phe Lys Ala Gln Gly Arg Ile Tyr Gly Lys
465                 470                 475                 480
Ile Lys Glu Glu Asn Phe Val Ser Pro Lys Glu Glu Val Lys Leu Glu
                485                 490                 495
Ala His Ile Arg Val Pro Ser Phe Ala Ala Gly Arg Val Ile Gly Lys
            500                 505                 510
Gly Gly Lys Thr Val Asn Glu Leu Gln Asn Leu Ser Ser Ala Glu Val
        515                 520                 525
Val Val Pro Arg Asp Gln Thr Pro Asp Glu Asn Asp Gln Val Val Val
    530                 535                 540
Lys Ile Thr Gly His Phe Tyr Ala Cys Gln Val Ala Gln Arg Lys Ile
545                 550                 555                 560
Gln Glu Ile Leu Thr Gln Val Lys Gln His Gln Gln Gln Lys Ala Leu
                565                 570                 575
Gln Ser Gly Pro Pro Gln Ser Arg Arg Lys
            580                 585

<210> 428
<211> 1764
<212> DNA
<213> Homo sapiens

<400> 428
atgcagcatc accaccatca ccacaacaaa ctgtatatcg gaaacctcag cgagaacgcc 60
gccccctcgg acctagaaag tatcttcaag gacgccaaga tcccggtgtc gggacccttc 120
ctggtgaaga ctggctacgc gttcgtggac tgcccggacg agagctgggc cctcaaggcc 180
atcgaggcgc tttcaggtaa aatagaactg cacgggaaac ccatagaagt tgagcactcg 240
gtcccaaaaa ggcaaaggat tcggaaactt cagatacgaa atatcccgcc tcatttacag 300
tgggaggtgc tggatagttt actagtccag tatggagtgg tggagagctg tgagcaagtg 360
aacactgact cggaaactgc agttgtaaat gtaacctatt ccagtaagga ccaagctaga 420
caagcactag acaaactgaa tggatttcag ttagagaatt tcaccttgaa agtagcctat 480
atccctgatg aaacggccgc ccagcaaaac cccttgcagc agccccgagg tcgccggggg 540
cttgggcaga ggggctcctc aaggcagggg tctccaggat ccgtatccaa gcagaaacca 600
tgtgatttgc ctctgcgcct gctggttccc acccaatttg ttggagccat cataggaaaa 660
gaaggtgcca ccattcggaa catcaccaaa cagacccagt ctaaaatcga tgtccaccgt 720
aaagaaaatg cgggggctgc tgagaagtcg attactatcc tctctactcc tgaaggcacc 780
tctgcggctt gtaagtctat tctggagatt atgcataagg aagctcaaga tataaaattc 840
acagaagaga tccccttgaa gattttagct cataataact ttgttggacg tcttattggt 900
aaagaaggaa gaaatcttaa aaaaattgag caagacacag acactaaaat cacgatatct 960
ccattgcagg aattgacgct gtataatcca gaacgcacta ttacagttaa aggcaatgtt 1020
gagacatgtg ccaaagctga ggaggagatc atgaagaaaa tcagggagtc ttatgaaaat 1080
gatattgctt ctatgaatct tcaagcacat ttaattcctg gattaaatct gaacgccttg 1140
ggtctgttcc cacccacttc agggatgcca cctcccacct caggcccccc ttcagccatg 1200
actcctccct acccgcagtt tgagcaatca gaaacggaga ctgttcatct gtttatccca 1260
gctctatcag tcggtgccat catcggcaag cagggccagc acatcaagca gctttctcgc 1320
tttgctggag cttcaattaa gattgctcca gcggaagcac cagatgctaa agtgaggatg 1380
gtgattatca ctggaccacc agaggctcag ttcaaggctc agggaagaat ttatggaaaa 1440
attaaagaag aaaactttgt tagtcctaaa gaagaggtga aacttgaagc tcatatcaga 1500
gtgccatcct ttgctgctgg cagagttatt ggaaaaggag gcaaaacggt gaatgaactt 1560
cagaatttgt caagtgcaga agttgttgtc cctcgtgacc agacacctga tgagaatgac 1620
caagtggttg tcaaaataac tggtcacttc tatgcttgcc aggttgccca gagaaaaatt 1680
caggaaattc tgactcaggt aaagcagcac caacaacaga aggctctgca aagtggacca 1740
cctcagtcaa gacggaagta atga 1764

<210> 429
<211> 35
<212> DNA
<213> Artificial Sequence

<220>
<223> PCR primer

<400> 429
ccatggaatt cattatttca atataagata atctc 35

<210> 430
<211> 881
<212> PRT
<213> Homo sapiens

<400> 430
Met Gln His His His His His His Gly Val Gln Leu Gln Asp Asn Gly
1               5                   10                  15
Tyr Asn Gly Leu Leu Ile Ala Ile Asn Pro Gln Val Pro Glu Asn Gln

                20                  25                  30
Asn Leu Ile Ser Asn Ile Lys Glu Met Ile Thr Glu Ala Ser Phe Tyr
        35                  40                  45
Leu Phe Asn Ala Thr Lys Arg Arg Val Phe Phe Arg Asn Ile Lys Ile
    50                  55                  60
Leu Ile Pro Ala Thr Trp Lys Ala Asn Asn Asn Ser Lys Ile Lys Gln
65                  70                  75                  80
Glu Ser Tyr Glu Lys Ala Asn Val Ile Val Thr Asp Trp Tyr Gly Ala
                85                  90                  95
His Gly Asp Asp Pro Tyr Thr Leu Gln Tyr Arg Gly Cys Gly Lys Glu
            100                 105                 110
Gly Lys Tyr Ile His Phe Thr Pro Asn Phe Leu Leu Asn Asp Asn Leu
        115                 120                 125
Thr Ala Gly Tyr Gly Ser Arg Gly Arg Val Phe Val His Glu Trp Ala
    130                 135                 140
His Leu Arg Trp Gly Val Phe Asp Glu Tyr Asn Asn Asp Lys Pro Phe
145                 150                 155                 160
Tyr Ile Asn Gly Gln Asn Gln Ile Lys Val Thr Arg Cys Ser Ser Asp
                165                 170                 175
Ile Thr Gly Ile Phe Val Cys Glu Lys Gly Pro Cys Pro Gln Glu Asn
            180                 185                 190
Cys Ile Ile Ser Lys Leu Phe Lys Glu Gly Cys Thr Phe Ile Tyr Asn
        195                 200                 205
Ser Thr Gln Asn Ala Thr Ala Ser Ile Met Phe Met Gln Ser Leu Ser
    210                 215                 220
Ser Val Val Glu Phe Cys Asn Ala Ser Thr His Asn Gln Glu Ala Pro
225                 230                 235                 240
Asn Leu Gln Asn Gln Met Cys Ser Leu Arg Ser Ala Trp Asp Val Ile
                245                 250                 255
Thr Asp Ser Ala Asp Phe His His Ser Phe Pro Met Asn Gly Thr Glu
            260                 265                 270
Leu Pro Pro Pro Pro Thr Phe Ser Leu Val Glu Ala Gly Asp Lys Val
        275                 280                 285
Val Cys Leu Val Leu Asp Val Ser Ser Lys Met Ala Glu Ala Asp Arg
    290                 295                 300
Leu Leu Gln Leu Gln Gln Ala Ala Glu Phe Tyr Leu Met Gln Ile Val
305                 310                 315                 320
Glu Ile His Thr Phe Val Gly Ile Ala Ser Phe Asp Ser Lys Gly Glu
                325                 330                 335
Ile Arg Ala Gln Leu His Gln Ile Asn Ser Asn Asp Asp Arg Lys Leu
            340                 345                 350
Leu Val Ser Tyr Leu Pro Thr Thr Val Ser Ala Lys Thr Asp Ile Ser
        355                 360                 365
Ile Cys Ser Gly Leu Lys Lys Gly Phe Glu Val Val Glu Lys Leu Asn
    370                 375                 380
Gly Lys Ala Tyr Gly Ser Val Met Ile Leu Val Thr Ser Gly Asp Asp
385                 390                 395                 400
Lys Leu Leu Gly Asn Cys Leu Pro Thr Val Leu Ser Ser Gly Ser Thr
                405                 410                 415
Ile His Ser Ile Ala Leu Gly Ser Ser Ala Ala Pro Asn Leu Glu Glu
            420                 425                 430
Leu Ser Arg Leu Thr Gly Gly Leu Lys Phe Phe Val Pro Asp Ile Ser
        435                 440                 445
Asn Ser Asn Ser Met Ile Asp Ala Phe Ser Arg Ile Ser Ser Gly Thr
    450                 455                 460
Gly Asp Ile Phe Gln Gln His Ile Gln Leu Glu Ser Thr Gly Glu Asn
465                 470                 475                 480
Val Lys Pro His His Gln Leu Lys Asn Thr Val Thr Val Asp Asn Thr

485                 490                 495
Val Gly Asn Asp Thr Met Phe Leu Val Thr Trp Gln Ala Ser Gly Pro
            500                 505                 510
Pro Glu Ile Ile Leu Phe Asp Pro Asp Gly Arg Lys Tyr Tyr Thr Asn
        515                 520                 525
Asn Phe Ile Thr Asn Leu Thr Phe Arg Thr Ala Ser Leu Trp Ile Pro
    530                 535                 540
Gly Thr Ala Lys Pro Gly His Trp Thr Tyr Thr Leu Asn Asn Thr His
545                 550                 555                 560
His Ser Leu Gln Ala Leu Lys Val Thr Val Thr Ser Arg Ala Ser Asn
                565                 570                 575
Ser Ala Val Pro Pro Ala Thr Val Glu Ala Phe Val Glu Arg Asp Ser
            580                 585                 590
Leu His Phe Pro His Pro Val Met Ile Tyr Ala Asn Val Lys Gln Gly
        595                 600                 605
Phe Tyr Pro Ile Leu Asn Ala Thr Val Thr Ala Thr Val Glu Pro Glu
    610                 615                 620
Thr Gly Asp Pro Val Thr Leu Arg Leu Leu Asp Asp Gly Ala Gly Ala
625                 630                 635                 640
Asp Val Ile Lys Asn Asp Gly Ile Tyr Ser Arg Tyr Phe Phe Ser Phe
                645                 650                 655
Ala Ala Asn Gly Arg Tyr Ser Leu Lys Val His Val Asn His Ser Pro
            660                 665                 670
Ser Ile Ser Thr Pro Ala His Ser Ile Pro Gly Ser His Ala Met Tyr
        675                 680                 685
Val Pro Gly Tyr Thr Ala Asn Gly Asn Ile Gln Met Asn Ala Pro Arg
    690                 695                 700
Lys Ser Val Gly Arg Asn Glu Glu Glu Arg Lys Trp Gly Phe Ser Arg
705                 710                 715                 720
Val Ser Ser Gly Gly Ser Phe Ser Val Leu Gly Val Pro Ala Gly Pro
                725                 730                 735
His Pro Asp Val Phe Pro Pro Cys Lys Ile Ile Asp Leu Glu Ala Val
            740                 745                 750
Lys Val Glu Glu Glu Leu Thr Leu Ser Trp Thr Ala Pro Gly Glu Asp
        755                 760                 765
Phe Asp Gln Gly Gln Ala Thr Ser Tyr Glu Ile Arg Met Ser Lys Ser
    770                 775                 780
Leu Gln Asn Ile Gln Asp Asp Phe Asn Asn Ala Ile Leu Val Asn Thr
785                 790                 795                 800
Ser Lys Arg Asn Pro Gln Gln Ala Gly Ile Arg Glu Ile Phe Thr Phe
                805                 810                 815
Ser Pro Gln Ile Ser Thr Asn Gly Pro Glu His Gln Pro Asn Gly Glu
            820                 825                 830
Thr His Glu Ser His Arg Ile Tyr Val Ala Ile Arg Ala Met Asp Arg
        835                 840                 845
Asn Ser Leu Gln Ser Ala Val Ser Asn Ile Ala Gln Ala Pro Leu Phe
    850                 855                 860
Ile Pro Pro Asn Ser Asp Pro Val Pro Ala Arg Asp Tyr Leu Ile Leu
865                 870                 875                 880
Lys

<210> 431
<211> 2646
<212> DNA
<213> Homo sapiens

<400> 431
atgcagcatc accaccatca ccacggagta cagcttcaag acaatgggta taatggattg 60
ctcattgcaa ttaatcctca ggtacctgag aatcagaacc tcatctcaaa cattaaggaa 120
atgataactg aagcttcatt ttacctattt aatgctacca agagaagagt attttcaga 180
aatataaaga ttttaatacc tgccacatgg aaagctaata ataacagcaa aataaaacaa 240
gaatcatatg aaaaggcaaa tgtcatagtg actgactggt atggggcaca tggagatgat 300
ccatacaccc tacaatacag agggtgtgga aaagagggaa aatacattca tttcacacct 360
aatttcctac tgaatgataa cttaacagct ggctacggat cacgaggccg agtgtttgtc 420
catgaatggg cccacctccg ttggggtgtg ttcgatgagt ataacaatga caaacctttc 480
tacataaatg ggcaaaatca aattaaagtg acaaggtgtt catctgacat cacaggcatt 540
tttgtgtgtg aaaaaggtcc ttgcccccaa gaaaactgta ttattagtaa gctttttaaa 600
gaaggatgca cctttatcta caatagcacc caaaatgcaa ctgcatcaat aatgttcatg 660
caaagtttat cttctgtggt tgaattttgt aatgcaagta cccacaacca agaagcacca 720
aacctacaga accagatgtg cagcctcaga agtgcatggg atgtaatcac agactctgct 780
gactttcacc acagctttcc catgaacggg actgagcttc cacctcctcc cacattctcg 840
cttgtagagg ctggtgacaa agtggtctgt ttagtgctgg atgtgtccag caagatggca 900
gaggctgaca gactccttca actacaacaa gccgcagaat tttatttgat gcagattgtt 960
gaaattcata ccttcgtggg cattgccagt ttcgacagca aaggagagat cagagcccag 1020
ctacaccaaa ttaacagcaa tgatgatcga aagttgctgg tttcatatct gcccaccact 1080
gtatcagcta aaacagacat cagcatttgt tcagggctta agaaaggatt tgaggtggtt 1140
gaaaaactga atggaaaagc ttatggctct gtgatgatat tagtgaccag cggagatgat 1200
aagcttcttg gcaattgctt acccactgtg ctcagcagtg gttcaacaat tcactccatt 1260
gccctgggtt catctgcagc cccaaatctg gaggaattat cacgtcttac aggaggttta 1320
aagttctttg ttccagatat atcaaactcc aatagcatga ttgatgcttt cagtagaatt 1380
tcctctggaa ctggagacat tttccagcaa catattcagc ttgaaagtac aggtgaaaat 1440
gtcaaacctc accatcaatt gaaaaacaca gtgactgtgg ataatactgt gggcaacgac 1500
actatgtttc tagttacgtg gcaggccagt ggtcctcctg agattatatt atttgatcct 1560
gatggacgaa aatactacac aaataatttt atcaccaatc taacttttcg gacagctagt 1620
ctttggattc caggaacagc taagcctggg cactggactt acaccctgaa caatacccat 1680
cattctctgc aagccctgaa agtgacagtg acctctcgcg cctccaactc agctgtgccc 1740
ccagccactg tggaagcctt tgtggaaaga gacagcctcc attttcctca tcctgtgatg 1800
atttatgcca atgtgaaaca gggattttat cccattctta atgccactgt cactgccaca 1860
gttgagccag agactggaga tcctgttacg ctgagactcc ttgatgatgg agcaggtgct 1920
gatgttataa aaaatgatgg aatttactcg aggtattttt tctcctttgc tgcaaatggt 1980
agatatagct tgaaagtgca tgtcaatcac tctcccagca taagcacccc agcccactct 2040
attccaggga gtcatgctat gtatgtacca ggttacacag caaacggtaa tattcagatg 2100
aatgctccaa ggaaatcagt aggcagaaat gaggaggagc gaaagtgggg ctttagccga 2160
gtcagctcag gaggctcctt ttcagtgctg ggagttccag ctggccccca ccctgatgtg 2220
tttccaccat gcaaaattat tgacctggaa gctgtaaaag tagaagagga attgacccta 2280
tcttggacag cacctggaga agactttgat cagggccagg ctacaagcta tgaaataaga 2340
atgagtaaaa gtctacagaa tatccaagat gactttaaca atgctatttt agtaaataca 2400
tcaaagcgaa atcctcagca agctggcatc agggagatat ttacgttctc accccaaatt 2460
tccacgaatg gacctgaaca tcagccaaat ggagaaacac atgaaagcca cagaatttat 2520
gttgcaatac gagcaatgga taggaactcc ttacagtctg ctgtatctaa cattgcccag 2580
gcgcctctgt ttattccccc caattctgat cctgtacctg ccagagatta tcttatattg 2640
aaataa 2646

<210> 432
<211> 36
<212> DNA
<213> Artificial Sequence

<220>
<223> PCR primer

<400> 432
cgcctgctcg agtcattaat attcatcaga aaatgg 36

<210> 433
<211> 371
<212> PRT
<213> Homo sapiens

<400> 433

<210> 434
<211> 1119

<212> DNA
<213> Homo sapiens

<400> 434
atgcagcatc accaccatca ccactggcag cccctcttct tcaagtggct cttgtcctgt 60
tgccctggga gttctcaaat tgctgcagca gcctccaccc agcctgagga tgacatcaat 120
acacagagga agaagagtca ggaaaagatg agagaagtta cagactctcc tgggcgaccc 180
cgagagctta ccattcctca gacttcttca catggtgcta acagatttgt tcctaaaagt 240
aaagctctag aggccgtcaa attggcaata gaagccgggt tccaccatat tgattctgca 300
catgtttaca ataatgagga gcaggttgga ctggccatcc gaagcaagat tgcagatggc 360
agtgtgaaga gagaagacat attctacact tcaaagcttt ggagcaattc ccatcgacca 420
gagttggtcc gaccagcctt ggaaaggtca ctgaaaaatc ttcaattgga ctatgttgac 480
ctctatctta ttcattttcc agtgtctgta aagccaggtg aggaagtgat cccaaaagat 540
gaaaatggaa aaatactatt tgacacagtg gatctctgtg ccacatggga ggccatggag 600
aagtgtaaag atgcaggatt ggccaagtcc atcggggtgt ccaacttcaa ccacaggctg 660
ctggagatga tcctcaacaa gccagggctc aagtacaagc ctgtctgcaa ccaggtggaa 720
tgtcatcctt acttcaacca gagaaaactg ctggatttct gcaagtcaaa agacattgtt 780
ctggttgcct atagtgctct gggatcccat cgagaagaac catgggtgga cccgaactcc 840
ccggtgctct tggaggaccc agtcctttgt gccttggcaa aaaagcacaa gcgaacccca 900
gccctgattg ccctgcgcta ccagctgcag cgtggggttg tggtcctggc caagagctac 960
aatgagcagc gcatcagaca gaacgtgcag gtgtttgaat tccagttgac ttcagaggag 1020
atgaaagcca tagatggcct aaacagaaat gtgcgatatt tgacccttga tatttttgct 1080
ggccccccta attatccatt ttctgatgaa tattaatga 1119

<210> 435
<211> 36
<212> DNA
<213> Artificial Sequence

<220>
<223> Primer

<400> 435
ggatccgccg ccaccatgac atccattcga gctgta 36

<210> 436
<211> 27
<212> DNA
<213> Artificial Sequence

<220>
<223> Primer

<400> 436
gtcgactcag ctggaccaca gccgcag 27

<210> 437
<211> 37
<212> DNA
<213> Artificial Sequence

<220>
<223> Primer

<400> 437
ggatccgccg ccaccatgga ctcctggacc ttctgct 37

<210> 438

<211> 27
<212> DNA
<213> Artificial Sequence

<220>
<223> Primer

<400> 438
gtcgactcag aaatcctttc tcttgac                                          27

<210> 439
<211> 933
<212> DNA
<213> Homo sapiens

<400> 439
atggactcct ggaccttctg ctgtgtgtcc ctttgcatcc tggtagcaaa gcacacagat      60
gctggagtta tccagtcacc ccggcacgag gtgacagaga tgggacaaga agtgactctg     120
agatgtaaac caatttcagg acacgactac cttttctggt acagacagac catgatgcgg     180
ggactggagt tgctcattta ctttaacaac aacgttccga tagatgattc agggatgccc     240
gaggatcgat tctcagctaa gatgcctaat gcatcattct ccactctgaa gatccagccc     300
tcagaaccca gggactcagc tgtgtacttc tgtgccagca gtttagttgg agcaaacact     360
gaagctttct ttggacaagg caccagactc acagttgtag aggacctgaa caaggtgttc     420
ccacccgagg tcgctgtgtt tgagccatca gaagcagaga tctcccacac ccaaaaggcc     480
acactggtgt gcctggccac aggcttcttc cctgaccacg tggagctgag ctggtgggtg     540
aatgggaagg aggtgcacag tggggtcagc acggacccgc agcccctcaa ggagcagccc     600
gccctcaatg actccagata ctgcctgagc agccgcctga gggtctcggc caccttctgg     660
cagaaccccc gcaaccactt ccgctgtcaa gtccagttct acgggctctc ggagaatgac     720
gagtggaccc aggatagggc caaacccgtc acccagatcg tcagcgccga ggcctggggt     780
agagcagact gtggctttac ctcggtgtcc taccagcaag gggtcctgtc tgccaccatc     840
ctctatgaga tcctgctagg gaaggccacc ctgtatgctg tgctggtcag cgcccttgtg     900
ttgatggcca tggtcaagag aaaggatttc tga                                  933

<210> 440
<211> 822
<212> DNA
<213> Homo sapiens

<400> 440
atgacatcca ttcgagctgt atttatattc ctgtggctgc agctggactt ggtgaatgga      60
gagaatgtgg agcagcatcc ttcaaccctg agtgtccagg agggagacag cgctgttatc     120
aagtgtactt attcagacag tgcctcaaac tacttccctt ggtataagca agaacttgga     180
aaaagacctc agcttattat agacattcgt tcaaatgtgg gcgaaaagaa agaccaacga     240
attgctgtta cattgaacaa gacagccaaa catttctccc tgcacatcac agagacccaa     300
cctgaagact cggctgtcta cttctgtgca gcaagtatac tgaacaccgg taaccagttc     360
tattttggga cagggacaag tttgacggtc attccaaata tccagaaccc tgaccctgcc     420
gtgtaccagc tgagagactc taaatccagt gacaagtctg tctgcctatt caccgatttt     480
gattctcaaa caaatgtgtc acaaagtaag gattctgatg tgtatatcac agacaaaact     540
gtgctagaca tgaggtctat ggacttcaag agcaacagtg ctgtggcctg gagcaacaaa     600
tctgactttg catgtgcaaa cgccttcaac aacagcatta ttccagaaga caccttcttc     660
cccagcccag aaagttcctg tgatgtcaag ctggtcgaga aaagctttga aacagatacg     720
aacctaaact ttcaaaacct gtcagtgatt gggttccgaa tcctcctcct gaaagtggcc     780
gggtttaatc tgctcatgac gctgcggctg tggtccagct ga                        822

<210> 441
<211> 2311
<212> DNA

<213> Homo sapiens

<400> 441
gatttaatcc tatgacaaac taagttggtt ctgtcttcac ctgttttggt gaggttgtgt 60
aagagttggt gtttgctcag gaagagattt aagcatgctt gcttacccag actcagagaa 120
gtctccctgt tctgtcctag ctatgttcct gtgttgtgtg cattcgtctt ttccagagca 180
aaccgcccag agtagaagat ggattggggc acgctgcaga cgatcctggg gggtgtgaac 240
aaacactcca ccagcattgg aaagatctgg ctcaccgtcc tcttcatttt tcgcattatg 300
atcctcgttg tggctgcaaa ggaggtgtgg ggagatgagc aggccgactt tgtctgcaac 360
accctgcagc caggctgcaa gaacgtgtgc tacgatcact acttccccat ctcccacatc 420
cggctatggg ccctgcagct gatcttcgtg tccagcccag cgctcctagt ggccatgcac 480
gtggcctacc ggagacatga gaagaagagg aagttcatca agggggagat aaagagtgaa 540
tttaaggaca tcgaggagat caaaacccag aaggtccgca tcgaaggctc cctgtggtgg 600
acctacacaa gcagcatctt cttccgggtc atcttcgaag ccgccttcat gtacgtcttc 660
tatgtcatgt acgacggctt ctccatgcag cggctggtga agtgcaacgc ctggccttgt 720
cccaacactg tggactgctt tgtgtcccgg cccacggaga agactgtctt cacagtgttc 780
atgattgcag tgtctggaat ttgcatcctg ctgaatgtca ctgaattgtg ttatttgcta 840
attagatatt gttctgggaa gtcaaaaaag ccagtttaac gcattgccca gttgttagat 900
taagaaatag acagcatgag agggatgagg caacccgtgc tcagctgtca aggctcagtc 960
gccagcattt cccaacacaa agattctgac cttaaatgca accatttgaa acccctgtag 1020
gcctcaggtg aaactccaga tgccacaatg agctctgctc ccctaaagcc tcaaaacaaa 1080
ggcctaattc tatgcctgtc ttaattttct ttcacttaag ttagttccac tgagacccca 1140
ggctgttagg ggttattggt gtaaggtact ttcatatttt aaacagagga tatcggcatt 1200
tgtttctttc tctgaggaca agagaaaaaa gccaggttcc acagaggaca cagagaaggt 1260
ttgggtgtcc tcctggggtt ctttttgcca actttcccca cgttaaaggt gaacattggt 1320
tctttcattt gctttggaag ttttaatctc taacagtgga caaagttacc agtgccttaa 1380
actctgttac actttttgga agtgaaaact ttgtagtatg ataggttatt ttgatgtaaa 1440
gatgttctgg ataccattat atgttccccc tgtttcagag gctcagattg taatatgtaa 1500
atggtatgtc attcgctact atgatttaat ttgaaatatg gtcttttggt tatgaatact 1560
ttgcagcaca gctgagagag gctgtctgtt gtattcattg tggtcatagc acctaacaac 1620
attgtagcct caatcgagtg agacagacta gaagttccta gttggcttat gatagcaaat 1680
ggcctcatgt caaatattag atgtaatttt gtgtaagaaa tacagactgg atgtaccacc 1740
aactactacc tgtaatgaca ggcctgtcca acacatctcc cttttccatg ctgtggtagc 1800
cagcatcgga aagaacgctg atttaaagag gtgagcttgg gaattttatt gacacagtac 1860
catttaatgg ggagacaaaa atgggggcca ggggagggag aagtttctgt cgttaaaaac 1920
gagtttggaa agactggact ctaaattctg ttgattaaag atgagctttg tctaccttca 1980
aaagtttgtt tggcttaccc ccttcagcct ccaatttttt aagtgaaaat ataactaata 2040
acatgtgaaa agaatagaag ctaaggttta gataaatatt gagcagatct ataggaagat 2100
tgaacctgaa tattgccatt atgcttgaca tggtttccaa aaaatggtac tccacatact 2160
tcagtgaggg taagtatttt cctgttgtca agaatagcat tgtaaaagca ttttgtaata 2220
ataaagaata gctttaatga tatgcttgta actaaaataa ttttgtaatg tatcaaatac 2280
atttaaaaca ttaaaatata atctctataa t 2311

<210> 442
<211> 226
<212> PRT
<213> Homo sapiens

<400> 442
Met Asp Trp Gly Thr Leu Gln Thr Ile Leu Gly Gly Val Asn Lys His
1               5                   10                  15

Ser Thr Ser Ile Gly Lys Ile Trp Leu Thr Val Leu Phe Ile Phe Arg
            20                  25                  30

Ile Met Ile Leu Val Val Ala Ala Lys Glu Val Trp Gly Asp Glu Gln
        35                  40                  45

Ala Asp Phe Val Cys Asn Thr Leu Gln Pro Gly Cys Lys Asn Val Cys
    50                  55                  60

Tyr Asp His Tyr Phe Pro Ile Ser His Ile Arg Leu Trp Ala Leu Gln
65                  70                  75                  80

Leu Ile Phe Val Ser Ser Pro Ala Leu Leu Val Ala Met His Val Ala
                85                  90                  95

Tyr Arg Arg His Glu Lys Lys Arg Lys Phe Ile Lys Gly Glu Ile Lys
            100                 105                 110

Ser Glu Phe Lys Asp Ile Glu Glu Ile Lys Thr Gln Lys Val Arg Ile
        115                 120                 125

Glu Gly Ser Leu Trp Trp Thr Tyr Thr Ser Ser Ile Phe Phe Arg Val
    130                 135                 140

Ile Phe Glu Ala Ala Phe Met Tyr Val Phe Tyr Val Met Tyr Asp Gly
145                 150                 155                 160

Phe Ser Met Gln Arg Leu Val Lys Cys Asn Ala Trp Pro Cys Pro Asn
                165                 170                 175

Thr Val Asp Cys Phe Val Ser Arg Pro Thr Glu Lys Thr Val Phe Thr
            180                 185                 190

Val Phe Met Ile Ala Val Ser Gly Ile Cys Ile Leu Leu Asn Val Thr
        195                 200                 205

Glu Leu Cys Tyr Leu Leu Ile Arg Tyr Cys Ser Gly Lys Ser Lys Lys
    210                 215                 220

Pro Val
225

<210> 443
<211> 23
<212> PRT
<213> Homo sapiens

<400> 443
Val Lys Leu Cys Gly Ile Asp Pro Cys Pro Asn Leu Val Asp Cys Phe
                5                   10                  15

Ile Ser Arg Pro Gly Cys Gly
            20

<210> 444
<211> 36
<212> DNA
<213> Artificial Sequence

<220>
<223> PCR primer

<400> 444
caatcaggca tgcacaacaa actgtatatc ggaaac                                36

<210> 445
<211> 30
<212> DNA
<213> Artificial Sequence

<220>
<223> PCR primer

<400> 445
cgtcaagatc ttcattactt ccgtcttgac                                       30

<210> 446
<211> 579
<212> PRT
<213> Homo sapiens

<400> 446
Met Asn Lys Leu Tyr Ile Gly Asn Leu Ser Glu Asn Ala Ala Pro Ser
                5                   10                  15

Asp Leu Glu Ser Ile Phe Lys Asp Ala Lys Ile Pro Val Ser Gly Pro
            20                  25                  30

Phe Leu Val Lys Thr Gly Tyr Ala Phe Val Asp Cys Pro Asp Glu Ser
        35                  40                  45

Trp Ala Leu Lys Ala Ile Glu Ala Leu Ser Gly Lys Ile Glu Leu His
    50                  55                  60

Gly Lys Pro Ile Glu Val Glu His Ser Val Pro Lys Arg Gln Arg Ile
65                  70                  75                  80

Arg Lys Leu Gln Ile Arg Asn Ile Pro Pro His Leu Gln Trp Glu Val
                85                  90                  95

Leu Asp Ser Leu Leu Val Gln Tyr Gly Val Val Glu Ser Cys Glu Gln
            100                 105                 110

Val Asn Thr Asp Ser Glu Thr Ala Val Val Asn Val Thr Tyr Ser Ser
        115                 120                 125

Lys Asp Gln Ala Arg Gln Ala Leu Asp Lys Leu Asn Gly Phe Gln Leu
    130                 135                 140

Glu Asn Phe Thr Leu Lys Val Ala Tyr Ile Pro Asp Glu Thr Ala Ala
145                 150                 155                 160

Gln Gln Asn Pro Leu Gln Gln Pro Arg Gly Arg Arg Gly Leu Gly Gln
                165                 170                 175

Arg Gly Ser Ser Arg Gln Gly Ser Pro Gly Ser Val Ser Lys Gln Lys
            180                 185                 190

Pro Cys Asp Leu Pro Leu Arg Leu Leu Val Pro Thr Gln Phe Val Gly
        195                 200                 205

Ala Ile Ile Gly Lys Glu Gly Ala Thr Ile Arg Asn Ile Thr Lys Gln
    210                 215                 220

Thr Gln Ser Lys Ile Asp Val His Arg Lys Glu Asn Ala Gly Ala Ala
225                 230                 235                 240
Glu Lys Ser Ile Thr Ile Leu Ser Thr Pro Glu Gly Thr Ser Ala Ala
                245                 250                 255

Cys Lys Ser Ile Leu Glu Ile Met His Lys Glu Ala Gln Asp Ile Lys
            260                 265                 270

Phe Thr Glu Glu Ile Pro Leu Lys Ile Leu Ala His Asn Asn Phe Val
        275                 280                 285

Gly Arg Leu Ile Gly Lys Glu Gly Arg Asn Leu Lys Lys Ile Glu Gln
    290                 295                 300

Asp Thr Asp Thr Lys Ile Thr Ile Ser Pro Leu Gln Glu Leu Thr Leu
305                 310                 315                 320

Tyr Asn Pro Glu Arg Thr Ile Thr Val Lys Gly Asn Val Glu Thr Cys
                325                 330                 335

Ala Lys Ala Glu Glu Glu Ile Met Lys Lys Ile Arg Glu Ser Tyr Glu
            340                 345                 350

Asn Asp Ile Ala Ser Met Asn Leu Gln Ala His Leu Ile Pro Gly Leu
        355                 360                 365

Asn Leu Asn Ala Leu Gly Leu Phe Pro Pro Thr Ser Gly Met Pro Pro
    370                 375                 380

Pro Thr Ser Gly Pro Pro Ser Ala Met Thr Pro Pro Tyr Pro Gln Phe
385                 390                 395                 400

Glu Gln Ser Glu Thr Glu Thr Val His Leu Phe Ile Pro Ala Leu Ser
                405                 410                 415

Val Gly Ala Ile Ile Gly Lys Gln Gly Gln His Ile Lys Gln Leu Ser
            420                 425                 430

Arg Phe Ala Gly Ala Ser Ile Lys Ile Ala Pro Ala Glu Ala Pro Asp
        435                 440                 445

Ala Lys Val Arg Met Val Ile Ile Thr Gly Pro Pro Glu Ala Gln Phe
    450                 455                 460

Lys Ala Gln Gly Arg Ile Tyr Gly Lys Ile Lys Glu Glu Asn Phe Val
465                 470                 475                 480

Ser Pro Lys Glu Glu Val Lys Leu Glu Ala His Ile Arg Val Pro Ser
                485                 490                 495

Phe Ala Ala Gly Arg Val Ile Gly Lys Gly Gly Lys Thr Val Asn Glu
            500                 505                 510

Leu Gln Asn Leu Ser Ser Ala Glu Val Val Val Pro Arg Asp Gln Thr
        515                 520                 525

Pro Asp Glu Asn Asp Gln Val Val Val Lys Ile Thr Gly His Phe Tyr
    530                 535                 540

Ala Cys Gln Val Ala Gln Arg Lys Ile Gln Glu Ile Leu Thr Gln Val
545                 550                 555                 560

Lys Gln His Gln Gln Gln Lys Ala Leu Gln Ser Gly Pro Pro Gln Ser
                565                 570                 575

Arg Arg Lys

<210> 447
<211> 1743
<212> DNA
<213> Homo sapiens

<400> 447
atgaacaaac tgtatatcgg aaacctcagc gagaacgccg ccccctcgga cctagaaagt 60
atcttcaagg acgccaagat cccggtgtcg ggacccttcc tggtgaagac tggctacgcg 120
ttcgtggact gcccggacga gagctgggcc ctcaaggcca tcgaggcgct ttcaggtaaa 180
atagaactgc acgggaaacc catagaagtt gagcactcgg tcccaaaaag gcaaaggatt 240
cggaaacttc agatacgaaa tatcccgcct catttacagt gggaggtgct ggatagttta 300
ctagtccagt atggagtggt ggagagctgt gagcaagtga acactgactc ggaaactgca 360
gttgtaaatg taacctattc cagtaaggac caagctagac aagcactaga caaactgaat 420
ggatttcagt tagagaattt caccttgaaa gtagcctata tccctgatga aacggccgcc 480
cagcaaaacc ccttgcagca gccccgaggt cgccgggggc ttgggcagag gggctcctca 540
aggcaggggt ctccaggatc cgtatccaag cagaaaccat gtgatttgcc tctgcgcctg 600
ctggttccca cccaatttgt tggagccatc ataggaaaag aaggtgccac cattcggaac 660
atcaccaaac agacccagtc taaaatcgat gtccaccgta aagaaaatgc gggggctgct 720
gagaagtcga ttactatcct ctctactcct gaaggcacct ctgcggcttg taagtctatt 780
ctggagatta tgcataagga agctcaagat ataaaattca cagaagagat ccccttgaag 840
attttagctc ataataactt tgttggacgt cttattggta aagaaggaag aaatcttaaa 900
aaaattgagc aagacacaga cactaaaatc acgatatctc cattgcagga attgacgctg 960
tataatccag aacgcactat tacagttaaa ggcaatgttg agacatgtgc caaagctgag 1020
gaggagatca tgaagaaaat cagggagtct tatgaaaatg atattgcttc tatgaatctt 1080
caagcacatt taattcctgg attaaatctg aacgccttgg gtctgttccc acccacttca 1140
gggatgccac ctcccacctc agggccccct tcagccatga ctcctcccta cccgcagttt 1200
gagcaatcag aaacggagac tgttcatctg tttatcccag ctctatcagt cggtgccatc 1260
atcggcaagc agggccagca catcaagcag ctttctcgct ttgctggagc ttcaattaag 1320
attgctccag cggaagcacc agatgctaaa gtgaggatgg tgattatcac tggaccacca 1380
gaggctcagt tcaaggctca gggaagaatt tatggaaaaa ttaaagaaga aaactttgtt 1440
agtcctaaag aagaggtgaa acttgaagct catatcagag tgccatcctt tgctgctggc 1500
agagttattg gaaaaggagg caaaacggtg aatgaacttc agaatttgtc aagtgcagaa 1560
gttgttgtcc ctcgtgacca gacacctgat gagaatgacc aagtggttgt caaaataact 1620
ggtcacttct atgcttgcca ggttgcccag agaaaaattc aggaaattct gactcaggta 1680
aagcagcacc aacaacagaa ggctctgcaa agtggaccac ctcagtcaag acggaagtaa 1740
tga 1743

<210> 448
<211> 35
<212> DNA

<213> Artificial Sequence

<220>
<223> PCR primer

<400> 448
cgtactagca tatgaacaaa ctgtatatcg gaaac                                35


<210> 449
<211> 579
<212> PRT
<213> Homo sapiens

<400> 449
Met Asn Lys Leu Tyr Ile Gly Asn Leu Ser Glu Asn Ala Ala Pro Ser
                5                   10                  15

Asp Leu Glu Ser Ile Phe Lys Asp Ala Lys Ile Pro Val Ser Gly Pro
            20                  25                  30

Phe Leu Val Lys Thr Gly Tyr Ala Phe Val Asp Cys Pro Asp Glu Ser
        35                  40                  45

Trp Ala Leu Lys Ala Ile Glu Ala Leu Ser Gly Lys Ile Glu Leu His
    50                  55                  60

Gly Lys Pro Ile Glu Val Glu His Ser Val Pro Lys Arg Gln Arg Ile
65                  70                  75                  80

Arg Lys Leu Gln Ile Arg Asn Ile Pro Pro His Leu Gln Trp Glu Val
                85                  90                  95

Leu Asp Ser Leu Leu Val Gln Tyr Gly Val Val Glu Ser Cys Glu Gln
            100                 105                 110

Val Asn Thr Asp Ser Glu Thr Ala Val Val Asn Val Thr Tyr Ser Ser
        115                 120                 125

Lys Asp Gln Ala Arg Gln Ala Leu Asp Lys Leu Asn Gly Phe Gln Leu
    130                 135                 140

Glu Asn Phe Thr Leu Lys Val Ala Tyr Ile Pro Asp Glu Thr Ala Ala
145                 150                 155                 160

Gln Gln Asn Pro Leu Gln Gln Pro Arg Gly Arg Arg Gly Leu Gly Gln
                165                 170                 175

Arg Gly Ser Ser Arg Gln Gly Ser Pro Gly Ser Val Ser Lys Gln Lys
            180                 185                 190

Pro Cys Asp Leu Pro Leu Arg Leu Leu Val Pro Thr Gln Phe Val Gly
        195                 200                 205

Ala Ile Ile Gly Lys Glu Gly Ala Thr Ile Arg Asn Ile Thr Lys Gln
    210                 215                 220
Thr Gln Ser Lys Ile Asp Val His Arg Lys Glu Asn Ala Gly Ala Ala
225                 230                 235                 240

Glu Lys Ser Ile Thr Ile Leu Ser Thr Pro Glu Gly Thr Ser Ala Ala
                245                 250                 255

Cys Lys Ser Ile Leu Glu Ile Met His Lys Glu Ala Gln Asp Ile Lys
            260                 265                 270

Phe Thr Glu Glu Ile Pro Leu Lys Ile Leu Ala His Asn Asn Phe Val
        275                 280                 285

Gly Arg Leu Ile Gly Lys Glu Gly Arg Asn Leu Lys Lys Ile Glu Gln
    290                 295                 300

Asp Thr Asp Thr Lys Ile Thr Ile Ser Pro Leu Gln Glu Leu Thr Leu
305                 310                 315                 320

Tyr Asn Pro Glu Arg Thr Ile Thr Val Lys Gly Asn Val Glu Thr Cys
                325                 330                 335

Ala Lys Ala Glu Glu Glu Ile Met Lys Lys Ile Arg Glu Ser Tyr Glu
            340                 345                 350

Asn Asp Ile Ala Ser Met Asn Leu Gln Ala His Leu Ile Pro Gly Leu
        355                 360                 365

Asn Leu Asn Ala Leu Gly Leu Phe Pro Pro Thr Ser Gly Met Pro Pro
    370                 375                 380

Pro Thr Ser Gly Pro Pro Ser Ala Met Thr Pro Pro Tyr Pro Gln Phe
385                 390                 395                 400

Glu Gln Ser Glu Thr Glu Thr Val His Leu Phe Ile Pro Ala Leu Ser
                405                 410                 415

Val Gly Ala Ile Ile Gly Lys Gln Gly Gln His Ile Lys Gln Leu Ser
            420                 425                 430

Arg Phe Ala Gly Ala Ser Ile Lys Ile Ala Pro Ala Glu Ala Pro Asp
        435                 440                 445

Ala Lys Val Arg Met Val Ile Ile Thr Gly Pro Pro Glu Ala Gln Phe
    450                 455                 460
Lys Ala Gln Gly Arg Ile Tyr Gly Lys Ile Lys Glu Glu Asn Phe Val
465                 470                 475                 480

Ser Pro Lys Glu Glu Val Lys Leu Glu Ala His Ile Arg Val Pro Ser
                485                 490                 495

Phe Ala Ala Gly Arg Val Ile Gly Lys Gly Gly Lys Thr Val Asn Glu
            500                 505                 510

Leu Gln Asn Leu Ser Ser Ala Glu Val Val Val Pro Arg Asp Gln Thr
        515                 520                 525

Pro Asp Glu Asn Asp Gln Val Val Val Lys Ile Thr Gly His Phe Tyr
    530                 535                 540

Ala Cys Gln Val Ala Gln Arg Lys Ile Gln Glu Ile Leu Thr Gln Val

545                 550                 555                 560

Lys Gln His Gln Gln Gln Lys Ala Leu Gln Ser Gly Pro Pro Gln Ser
                565                 570                 575

Arg Arg Lys


<210> 450
<211> 1743
<212> DNA
<213> Homo sapiens

<400> 450
   atgaacaaac tgtatatcgg aaacctcagc gagaacgccg ccccctcgga cctagaaagt 60
   atcttcaagg acgccaagat cccggtgtcg ggacccttcc tggtgaagac tggctacgcg 120
   ttcgtggact gcccggacga gagctgggcc ctcaaggcca tcgaggcgct ttcaggtaaa 180
   atagaactgc acgggaaacc catagaagtt gagcactcgg tcccaaaaag gcaaaggatt 240
   cggaaacttc agatacgaaa tatcccgcct catttacagt gggaggtgct ggatagttta 300
   ctagtccagt atggagtggt ggagagctgt gagcaagtga acactgactc ggaaactgca 360
   gttgtaaatg taacctattc cagtaaggac caagctagac aagcactaga caaactgaat 420
   ggatttcagt tagagaattt caccttgaaa gtagcctata tccctgatga aacggccgcc 480
   cagcaaaacc ccttgcagca gccccgaggt cgccgggggc ttgggcagag gggctcctca 540
   aggcaggggt ctccaggatc cgtatccaag cagaaaccat gtgatttgcc tctgcgcctg 600
   ctggttccca cccaatttgt tggagccatc ataggaaaag aaggtgccac cattcggaac 660
   atcaccaaac agacccagtc taaaatcgat gtccaccgta aagaaaatgc gggggctgct 720
   gagaagtcga ttactatcct ctctactcct gaaggcacct ctgcggcttg taagtctatt 780
   ctggagatta tgcataagga agctcaagat ataaaattca cagaagagat ccccttgaag 840
   attttagctc ataataactt tgttggacgt cttattggta aagaaggaag aaatcttaaa 900
   aaaattgagc aagacacaga cactaaaatc acgatatctc cattgcagga attgacgctg 960
   tataatccag aacgcactat tacagttaaa ggcaatgttg agacatgtgc caaagctgag 1020
   gaggagatca tgaagaaaat cagggagtct tatgaaaatg atattgcttc tatgaatctt 1080
   caagcacatt taattcctgg attaaatctg aacgccttgg gtctgttccc acccacttca 1140
   gggatgccac ctcccacctc agggccccct tcagccatga ctcctcccta cccgcagttt 1200
   gagcaatcag aaacggagac tgttcatctg tttatcccag ctctatcagt cggtgccatc 1260
   atcggcaagc agggccagca catcaagcag ctttctcgct ttgctggagc ttcaattaag 1320
   attgctccag cggaagcacc agatgctaaa gtgaggatgg tgattatcac tggaccacca 1380
   gaggctcagt tcaaggctca gggaagaatt tatggaaaaa ttaaagaaga aaactttgtt 1440
   agtcctaaag aagaggtgaa acttgaagct catatcagag tgccatcctt tgctgctggc 1500
   agagttattg gaaaaggagg caaaacggtg aatgaacttc agaatttgtc aagtgcagaa 1560
   gttgttgtcc ctcgtgacca gacacctgat gagaatgacc aagtggttgt caaaataact 1620
   ggtcacttct atgcttgcca ggttgcccag agaaaaattc aggaaattct gactcaggta 1680
   aagcagcacc aacaacagaa ggctctgcaa agtggaccac ctcagtcaag acggaagtaa 1740
   tga                                                               1743


<210> 451
<211> 25
<212> PRT
<213> Homo sapiens

<400> 451
Leu Gly Lys Glu Val Arg Asp Ala Lys Ile Thr Pro Glu Ala Phe Glu
                5                   10                  15

Lys Leu Gly Phe Pro Ala Ala Lys Glu
            20                  25

<210> 452
<211> 25
<212> PRT
<213> Homo sapiens

<400> 452
Lys Ala Ser Asp Gly Asp Tyr Tyr Thr Leu Ala Val Pro Met Gly Asp
                5                   10                  15

Val Pro Met Asp Gly Ile Ser Val Ala
            20                  25


<210> 453
<211> 16
<212> PRT
<213> Homo sapiens

<400> 453
Pro Asp Arg Asp Val Asn Leu Thr His Gln Leu Asn Pro Lys Val Lys
                5                   10                  15


<210> 454
<211> 20
<212> PRT
<213> Homo sapiens

<400> 454
Lys Ile Ala Pro Ala Glu Ala Pro Asp Ala Lys Val Arg Met Val Ile
                5                   10                  15

Ile Thr Gly Pro
            20


<210> 455
<211> 20
<212> PRT
<213> Homo sapiens

<400> 455
Pro Asp Glu Thr Ala Ala Gln Gln Asn Pro Leu Gln Gln Pro Arg Gly
                5                   10                  15

Arg Arg Gly Leu
            20


<210> 456
<211> 20
<212> PRT
<213> Homo sapiens

<400> 456
Arg Thr Ile Thr Val Lys Gly Asn Val Glu Thr Cys Ala Lys Ala Glu

5                   10                   15

Glu Glu Ile Met
            20

<210> 457
<211> 20
<212> PRT
<213> Homo sapiens

<400> 457
Ala Phe Val Asp Cys Pro Asp Glu Ser Trp Ala Leu Lys Ala Ile Glu
                5                   10                  15

Ala Leu Ser Gly
            20


<210> 458
<211> 20
<212> PRT
<213> Homo sapiens

<400> 458
Ile Arg Lys Leu Gln Ile Arg Asn Ile Pro Pro His Leu Gln Trp Glu
                5                   10                  15

Val Leu Asp Ser
            20


<210> 459
<211> 20
<212> PRT
<213> Homo sapiens

<400> 459
Ala Gln Gln Asn Pro Leu Gln Gln Pro Arg Gly Arg Arg Gly Leu Gly
                5                   10                  15

Gln Arg Gly Ser
            20


<210> 460
<211> 20
<212> PRT
<213> Homo sapiens

<400> 460
Asp Val His Arg Lys Glu Asn Ala Gly Ala Ala Glu Lys Ser Ile Thr
                5                   10                  15

Ile Leu Ser Thr
            20


<210> 461

<211> 20
<212> PRT
<213> Homo sapiens

<400> 461
Leu Tyr Asn Pro Glu Arg Thr Ile Thr Val Lys Gly Asn Val Glu Thr
                5                   10                  15

Cys Ala Lys Ala
            20


<210> 462
<211> 20
<212> PRT
<213> Homo sapiens

<400> 462
Glu Glu Glu Ile Met Lys Lys Ile Arg Glu Ser Tyr Glu Asn Asp Ile
                5                   10                  15

Ala Ser Met Asn
            20


<210> 463
<211> 20
<212> PRT
<213> Homo sapiens

<400> 463
Leu Asn Ala Leu Gly Leu Phe Pro Pro Thr Ser Gly Met Pro Pro Pro
                5                   10                  15

Thr Ser Gly Pro
            20


<210> 464
<211> 20
<212> PRT
<213> Homo sapiens

<400> 464
Lys Ile Ala Pro Ala Glu Ala Pro Asp Ala Lys Val Arg Met Val Ile
                5                   10                  15

Ile Thr Gly Pro
            20

<210> 465
<211> 18
<212> PRT
<213> Homo sapiens

<400> 465
Thr Gly Tyr Ala Phe Val Asp Cys Pro Asp Glu Ser Trp Ala Leu Lys Ile
                5                   10                  15

Glu


<210> 466
<211> 11
<212> PRT
<213> Homo sapiens

<400> 466
Phe Val Asp Cys Pro Asp Glu Ser Trp Ala Leu
                5                   10

<210> 467
<211> 33
<212> DNA
<213> Homo sapiens

<400> 467
ttcgtggact gcccggacga gagctgggcc ctc                                    33


<210> 468
<211> 24
<212> PRT
<213> Homo sapiens

<400> 468
Ile Pro Asp Glu Met Ala Ala Gln Gln Asn Pro Leu Gln Gln Pro Arg
                5                   10                  15

Gly Arg Arg Gly Leu Gly Gln Arg
            20


<210> 469
<211> 24
<212> PRT
<213> Homo sapiens

<400> 469
Ile Pro Asp Glu Thr Ala Ala Gln Gln Asn Pro Ser Pro Gln Leu Arg
                5                   10                  15

Gly Arg Arg Gly Pro Gly Gln Arg
            20

## Description

### TECHNICAL FIELD OF THE INVENTION

The present invention relates generally to therapy and diagnosis of cancel, such as lung cancer. The invention is more specifically related to polypeptides, comprising at least a portion of a lung tumor protein, and to polynucleotides encoding such polypeptides. Such polypeptides and polynucleotides are useful in pharmaceutical compositions, *e*.*g*., vaccines, and other compositions for the diagnosis and treatment of lung cancer

### BACKGROUND OF THE INVENTION

### Field of the Invention

Cancer is a significant health problem throughout the world. Although advances have been made in detection and therapy of cancel, no vaccine or other universally successful method for prevention and/or treatment is currently available. Current therapies, which are generally based on a combination of chemotherapy or surgery and radiation, continue to prove inadequate in many patients.

### Description of Related Art

Lung cancer is the primary cause of cancer death among both men and women in the US., with an estimated 172,000 new cases being reported in 1994. The five-year survival rate among all lung cancer patients, regardless of the stage of disease at diagnosis, is only 13%. This contrasts with a five-year survival rate of 46% among cases detected while the disease is still localized. However, only 16% of lung cancers are discovered before the disease has spread.

In spite of considerable research into therapies for these and other cancers, lung cancer remains difficult to diagnose and treat effectively. Accordingly, there is a need in the art for improved methods for detecting and treating such cancers. The present invention fulfills these needs and further provides other related advantages.

### SUMMARY OF THE INVENTION

In one aspect, the present invention provides polynucleotide compositions comprising a sequence selected from the group consisting of:
(a) sequences provided in SEQ ID NO:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467;
(b) complements of the sequences provided in SEQ ID NO:1-3, 6-8, 10-13, 15-27, 29, 30, 32, 34-49, 51,52,54,55,57-59, 61-69, 71, 73, 74, 77, 78, 80-82, 84, 86-96, 107-109, 111, 113, 125, 127, 128, 129, 131-133, 142, 144, 148-151, 15.3, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467;
(c) sequences consisting of at least 10, 15, 20, 25, 30, 35, 40, 45, 50, 75 and 100 contiguous residues of a sequence provided in SEQ ID NO: 1-3, 6-8,10-13, 15-27,29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467;
(d) sequences that hybridize to a sequence provided in SEQ ID NO:1-3, 6-8, 10-13,15-27,29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, under moderate or highly stringent conditions;
(e) sequences having at least 75%, 80%, 85%, 90%, 95%, 96%, 97%, 98% or 99% identity to a sequence of SEQ ID NO:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467; and
(f) degenerate variants of a sequence provides in SEQ ID NO:1-3, 6-8, 10-13, 15-27, 29, 30, 32,34-49,51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362,364,365,368,370-375, 420, 424, 428, 431, 434, 442, 447, 450 and 467

In one preferred embodiment, the polynucleotide compositions of the invention are expressed in at least about 20%, more preferably in at least about 30%, and most preferably in at least about 50% of lung tumors samples tested, at a level that is at least about 2-fold, preferably at least about 5-fold, and most preferably, at least about 10-fold higher than that for normal tissues.

The present invention, in another aspect, provides polypeptide compositions comprising an amino acid sequence that is encoded by a polynucleotide sequence described above.

The present invention further provides polypeptide compositions comprising an amino acid sequence selected from the group consisting of sequences recited in SEQ ID NO:152, 155, 156, 165, 166, 169, 170, 172, 174, 176, 226-252, 338. 344, 346, 350, 357, 361, 363, 365, 367, 369, 376-382, 387-419, 423, 427, 430, 433, 441,443,446,449,451-466 and 468-469.

In certain preferred embodiments, the polypeptides and/or polynucleotides of the present invention are immunogenic, *i*.*e*., they are capable of eliciting an immune response, particularly a humoral and/on cellular immune response, as further described herein.

The present invention further provides fragments, variants and/or derivatives of the disclosed polypeptide and/or polynucleotide sequences, wherein the fragment, variants and/or derivatives preferably have a level of immunogenic activity of at least about 50%, preferably at least about 70% and more preferably at least about 90% of the level of immunogenic activity of a polypeptide sequence set forth in SEQ ID NO:152, 155,156,165,166,169,170,172,174,176,226-252,338-344,346,350,357, 361,363, 365, 367, 369, 376-382, 387-419,423,427,430,433,441, 443, 446, 449 and 451-466, or a polypeptide sequence encoded by a polynucleotide sequence set forth in SEQ ID NO:1-3, 68,10-13,15-27,29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467.

The present invention further provides polynucleotides that encode a polypeptide described above, expression vectors comprising such polynucleotides and host cells transformed or transfected with such expression vectors.

Within other aspects, the present invention provides pharmaceutical compositions comprising a polypeptide or polynucleotide as described above and a physiologically acceptable carrier.

Within a related aspect of the present invention, the pharmaceutical compositions, *e*.*g*., vaccine compositions, are provided for prophylactic or therapeutic applications. Such compositions generally comprise an immunogenic polypeptide or polynucleotide of the invention and an immunostimulant, such as an adjuvant.

The present invention further provides pharmaceutical compositions that comprise: (a) an antibody or antigen- binding fragment thereof that specifically binds to a polypeptide of the present invention, or a fragment thereof; and (b) a physiologically acceptable carrier.

Within further aspects, the present invention provides pharmaceutical compositions comprising: (a) an antigen presenting cell that expresses a polypeptide as described above and (b) a pharmaceutically acceptable carrier or excipient Illustrative antigen presenting cells include dendritic cells, macrophages, monocytes, fibroblasts and B cells.

Within related aspects, pharmaceutical compositions are provided that comprise: (a) an antigen presenting cell that expresses a polypeptide as described above and (b) an immunostimulant.

The present invention further provides, in other aspects, fusion proteins that comprise at least one polypeptide as described above, as well as polynucleotides encoding such fusion proteins, typically in the form of pharmaceutical compositions, *e.g*., vaccine compositions, comprising a physiologically acceptable carrier and/or an immunostimulant. The fusions proteins may comprise multiple immunogenic polypeptides or portions/variants thereof, as described herein, and may further comprise one or more polypeptide segments for facilitating the expression, purification and/or immunogenicity of the polypeptide(s).

Within further aspects, the present invention provides methods for stimulating an immune response in a patient, preferably a T cell response in a human patient, comprising administering a pharmaceutical composition described herein. The patient may be afflicted with lung cancer, in which case the methods provide treatment for the disease, or patient considered at risk for such a disease may be treated prophylactically.

Within further aspects, the present invention provides methods for inhibiting the development of a cancer in a patient, comprising administering to a patient a pharmaceutical composition as recited above. The patient may be afflicted with lung cancer, in which case the methods provide treatment for the disease, or patient considered at risk for such a disease may be treated prophylactically.

The present invention further provides, within other aspects, methods for removing tumor cells from a biological sample, comprising contacting a biological sample with T cells that specifically react with a polypeptide of the present invention, wherein the step of contacting is performed under conditions and for a time sufficient to permit the removal of cells expressing the protein from the sample..

Within related aspects, methods are provided for inhibiting the development of a cancer in a patient, comprising administering to a patient a biological sample treated as described above.

Methods are further provided, within other aspects, for stimulating and/or expanding T cells specific for a polypeptide of the present invention, comprising contacting T cells with one or more of: (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Isolated T cell populations comprising T cells prepared as described above are also provided.

Within further aspects, the present invention provides methods for inhibiting the development of a cancer in a patient, comprising administering to a patient an effective amount of a T cell population as described above.

The present invention further provides methods for inhibiting the development of a cancer in a patient, comprising the steps of: (a) incubating CD4⁺ and/or CD8⁺ T cells isolated from a patient with one or more of: (i) a polypeptide comprising at least an immunogenic portion of polypeptide disclosed herein; (ii) a polynucleotide encoding such a polypeptide; and (iii) an antigen presenting cell that expressed such a polypeptide; and (b) administering to the patient an effective amount of the proliferated T cells, and thereby inhibiting the development of a cancer in the patient Proliferated cells may, but need not, be cloned prior to administration to the patient.

Within further aspects, the present invention provides methods for determining the presence or absence of a cancer, preferably a lung cancer, in a patient comprising: (a) contacting a biological sample obtained from a patient with a binding agent that binds to a polypeptide as recited above; (b) detecting in the sample an amount of polypeptide that binds to the binding agent; and (c) comparing the amount of polypeptide with a predetermined cut off value, and therefrom determining the presence or absence of a cancer in the patient. Within preferred embodiments, the binding agent is an antibody, more preferably a monoclonal antibody..

The present invention also provides, within other aspects, methods for monitoring the progression of a cancer in a patient. Such methods comprise the steps of: (a) contacting a biological sample obtained from a patient at a first point in time with a binding agent that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b) and therefrom monitoring the progression of the cancer in the patient.

The present invention further provides, within other aspects, methods for determining the presence or absence of a cancer in a patient, comprising the steps of: (a) contacting a biological sample obtained from a patient with an oligonucleotide that hybridizes to a polynucleotide that encodes a polypeptide of the present invention; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are provided for monitoring the progression of a cancer in a patient, comprising the steps of: (a) contacting a biological sample obtained from a patient with an oligonucleotide that hybridizes to a polynucleotide that encodes a polypeptide of the present invention; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Within further aspects, the present invention provides antibodies, such as monoclonal antibodies, that bind to a polypeptide as described above, as well as diagnostic kits comprising such antibodies. Diagnostic kits comprising one or more oligonucleotide probes or primers as described above are also provided

These and other aspects of the present invention will become apparent upon reference to the following detailed description. All references disclosed herein are hereby incorporated by reference in their entirety as if each was incorporated individually.

### SEQUENCE IDENTIFIERS

SEQ ID NO:1 is the determined cDNA sequence for LST-S1-2
SEQ ID NO:2 is the determined cDNA sequence for LST-S1-28
SEQ ID NO:3 is the determined cDNA sequence for LST-S1-90
SEQ ID NO:4 is the determined cDNA sequence for LST-S1-144
SEQ ID NO:5 is the determined cDNA sequence for LST-S1-133
SEQ ID NO:6 is the determined cDNA sequence for LST-S1-169
SEQ ID NO:7 is the determined cDNA sequence for LST-S2-6
SEQ ID NO:8 is the determined cDNA sequence for LST-S2-11
SEQ ID NO:9 is the determined cDNA sequence for LST-S2-17
SEQ ID NO:10 is the determined cDNA sequence for LST-S2-25
SEQ ID NO:11 is the determined cDNA sequence for LST-S2 -39
SEQ ID NO:12 is a first determined cDNA sequence for LST-S2-43
SEQ ID NO:13 is a second determined cDNA sequence for LST-S2-43
SEQ ID NO:14 is the determined cDNA sequence for LST-S2-65
SEQ ID NO:15 is the determined cDNA sequence for LST- S2-68
SEQ ID NO:16 is the determined cDNA sequence for LST-S2-72
SEQ ID NO:17 is the determined cDNA sequence for LST-S2-74
SEQ ID NO:18 is the determined cDNA sequence for LST-S2-103
SEQ ID NO:19 is the determined cDNA sequence for LST-S2-N1-1F
SEQ ID NO:20 is the determined cDNA sequence for LST-S2-N1-2A
SEQ ID NO:21 is the determined cDNA sequence for LST-S2-N1-4H
SEQ ID NO:22 is the determined cDNA sequence for LST-S2-N1-5A
SEQ ID NO:23 is the determined cDNA sequence for LST-S2-N1-6B
SEQ ID NO:24 is the determined cDNA sequence for LST-S2-N1-7B
SEQ ID NO:25 is the determined cDNA sequence for LST-S2-N1-7H
SEQ ID NO:26 is the determined cDNA sequence for LSI-S2-N1-8A
SEQ ID NO:27 is the determined cDNA sequence for LST-S2-N1-8D
SEQ ID NO:28 is the determined cDNA sequence for LST-S2-N1 -9A
SEQ ID NO:29 is the determined cDNA sequence for LST-S2-N1-9E
SEQ ID NO:30 is the determined cDNA sequence for LST-S2-N1-10A
SEQ ID NO:31 is the determined cDNA sequence for LST-S2-N1-10G
SEQ ID NO:32 is the determined cDNA sequence for LST-S2-N1-11A
SEQ ID NO:33 is the determined cDNA sequence for LST-S2-N1-12C
SEQ ID NO:34 is the determined cDNA sequence foz LST-S2-N1-12E
SEQ ID NO:35 is the determined cDNA sequence fox LST-S2-B1-3D
SEQ ID NO:36 is the determined cDNA sequence for LST-S2-B1-6C
SEQ ID NO:37 is the determined cDNA sequence for LST-S2-B1-5D
SEQ ID NO:38 is the determined cDNA sequence for LSI-S2-B1-5F
SEQ ID NO:39 is the determined cDNA sequence for LST-S2-B1-6G
SEQ ID NO:40 is the determined cDNA sequence for LST-S2-B1-8A
SEQ ID NO:41 is the determined cDNA sequence for LST-S2-B1-8D
SEQ ID NO:42 is the determined cDNA sequence for LST-S2-B1-10A
SEQ ID NO:43 is the determined cDNA sequence for LST-S2-B1-9B
SEQ ID NO:44 is the determined cDNA sequence for LST-S2-B1-9F
SEQ ID NO:45 is the determined cDNA sequence for LST-S2-B1-12D
SEQ ID NO:46 is the determined cDNA sequence for LST-S2-I2-2B
SEQ ID NO:47 is the determined cDNA sequence for LST-S2-I2-5F
SEQ ID NO:48 is the determined cDNA sequence for LST-S2-I2-6B
SEQ ID NO:49 is the determined cDNA sequence for LST-S2-I2-7F
SEQ ID NO:50 is the determined cDNA sequence for LST-S2-I2-8G
SEQ ID NO:51 is the determined cDNA sequence for LST-S2-I2-9E
SEQ ID NO:52 is the determined cDNA sequence for LST-S2-I2-12B
SEQ ID NO:53 is the determined cDNA sequence for LST-S2-H2-2C
SEQ ID NO:54 is the determined cDNA sequence for LSI-S2-H2-1G
SEQ ID NO:55 is the determined cDNA sequence for LST-S2-H2-40
SEQ ID NO:56 is the determined cDNA sequence for LST-S2-H2-3H
SEQ ID NO:57 is the determined cDNA sequence for LST-S2-H2-5G
SEQ ID NO:58 is the determined cDNA sequence for LST-S2-H2-9B
SEQ ID NO:59 is the determined cDNA sequence for LST-S2-H2-10H
SEQ ID NO:60 is the determined cDNA sequence for LST-S2-H2-12D
SEQ ID NO: 61 is the determined cDNA sequence for LST-S3-2
SEQ ID NO: 62 is the determined cDNA sequence for LST-S3-4
SEQ ID NO: 63 is the determined cDNA sequence for LST-S3-7
SEQ ID NO: 64 is the determined cDNA sequence for LST-S3-8
SEQ ID NO: 65 is the determined cDNA sequence for LST-S3-12
SEQ ID NO: 66 is the determined cDNA sequence for LST-S3-13
SEQ ID NO: 67 is the determined cDNA sequence for LST-S3-14
SEQ ID NO: 68 is the determined cDNA sequence for LST-S3-16
SEQ ID NO: 69 is the determined cDNA sequence for LST-S3-21
SEQ ID NO: 70 is the determined cDNA sequence for LST-S3-22
SEQ ID NO: 71 is the determined cDNA sequence for LST-S1-7
SEQ ID NO: 72 is the determined cDNA sequence for LST-S1-A-1E
SEQ ID NO: 73 is the determined cDNA sequence for LST-S1-A-1G
SEQ ID NO: 74 is the determined cDNA sequence for LST-S1-A-3E
SEQ ID NO: 75 is the determined cDNA sequence for LST-S1-A-4E
SEQ ID NO: 76 is the determined cDNA sequence for LST-S1-A-6D
SEQ ID NO: 77 is the determined cDNA sequence for LST-S1-A-8D
SEQ ID NO: 78 is the determined cDNA sequence for LST-S1-A-10A
SEQ ID NO: 79 is the determined cDNA sequence for LST-S1-A-10C
SEQ ID NO: 80 is the determined cDNA sequence for LST-S1-A-9D
SEQ ID NO: 81 is the determined cDNA sequence for LST-S1-A-10D
SEQ ID NO: 82 is the determined cDNA sequence for LST-S1-A-9H
SEQ ID NO: 83 is the determined cDNA sequence for LST-S1-A-11D
SEQ ID NO: 84 is the determined cDNA sequence for LST-S1-A-12D
SEQ ID NO: 85 is the determined cDNA sequence for LST-S1-A-11E
SEQ ID NO: 86 is the determined cDNA sequence for LST-S1-A-12E
SEQ ID NO: 87 is the determined cDNA sequence for L513S (T3).
SEQ ID NO: 88 is the determined cDNA sequence for L513S contig 1.
SEQ ID NO: 89 is a first determined cDNA sequence for L514S..
SEQ ID NO: 90 is a second determined cDNA sequence for L514S..
SEQ ID NO: 91 is a first determined cDNA sequence for L516S.
SEQ ID NO: 92 is a second determined cDNA sequence for L516S.
SEQ ID NO: 93 is the determined cDNA sequence for L517S.
SEQ ID NO: 94 is the extended cDNA sequence for LST-S1-169 (also known as L519S).
SEQ ID NO: 95 is a first determined cDNA sequence for L520S.
SEQ ID NO: 96 is a second determined cDNA sequence for L520S.
SEQ ID NO: 97 is a first determined cDNA sequence for L521S.
SEQ ID NO: 98 is a second determined cDNA sequence for L521S.
SEQ ID NO: 99 is the determiner cDNA sequence for L522S.
SEQ ID NO: 100 is the determined cDNA sequence for L523S.
SEQ ID NO: 101 is the determined cDNA sequence for L524S.
SEQ ID NO: 102 is the determined cDNA sequence for L525S.
SEQ ID NO: 103 is the determined cDNA sequence for L526S.
SEQ ID NO: 104 is the determined cDNA sequence for L527S.
SEQ ID NO: 105 is the determined cDNA sequence for L528S.
SEQ ID NO: 106 is the determined cDNA sequence for L529S.
SEQ ID NO: 107 is a first determined cDNA sequence for L530S.
SEQ ID NO: 108 is a second determine cDNA sequence for L530S.
SEQ ID NO: 109 is the determined full-length cDNA sequence for L531S short form
SEQ ID NO: 110 is the amino acid sequence encoded by SEQ ID NO: 109.
SEQ ID NO: 111 is the determined full-length cDNA sequence for L531S long form
SEQ ID NO: 112 is the amino acid sequence encoded by SEQ NO: 111.
SEQ ID NO: 113 is the determined full-length cDNA sequence for L520S.
SEQ ID NO: 114 is the amino acid sequence encoded by SEQ ID NO: 113.
SEQ ID NO: 115 is the determined cDNA sequence for contig 1.
SEQ ID NO: 116 is the determined cDNA sequence for contig 3.
SEQ ID NO: 117 is the determined cDNA sequence for contig 4.
SEQ ID NO: 118 is the determined cDNA sequence for contig 5.
SEQ ID NO: 119 is the determined cDNA sequence for contig 7.
SEQ ID NO: 120 is the determined cDNA sequence for contig 8.
SEQ ID NO: 121 is the determined cDNA sequence for contig 9.
SEQ ID NO: 122 is the determined cDNA sequence for contig 10.
SEQ ID NO: 123 is the determined cDNA sequence for contig 12.
SEQ ID NO: 124 is the determined cDNA sequence for contig 11.
SEQ ID NO: 125 is the determined cDNA sequence for contig 13 (also known as L761P)
SEQ ID NO: 126 is the determined cDNA sequence for contig 15.
SEQ ID NO: 127 is the determined cDNA sequence for contig 16.
SEQ ID NO: 128 is the determined cDNA sequence for contig 17.
SEQ ID NO: 129 is the determined cDNA sequence for contig 19.
SEQ ID NO: 130 is the determined cDNA sequence for contig 20.
SEQ ID NO: 131 is the determined cDNA sequence for contig 22.
SEQ ID NO: 132 is the determined cDNA sequence for contig 24.
SEQ ID NO: 133 is the determined cDNA sequence for contig 29.
SEQ ID NO: 134 is the determined cDNA sequence for contig 31.
SEQ ID NO: 135 is the determined cDNA sequence for contig 33.
SEQ ID NO: 136 is the determined cDNA sequence for contig 38.
SEQ ID NO: 137 is the determined cDNA sequence for contig 39.
SEQ ID NO: 138 is the determined cDNA sequence for contig 41.
SEQ ID NO: 139 is the determined cDNA sequence for contig 43.
SEQ ID NO: 140 is the determined cDNA sequence for contig 44.
SEQ ID NO: 141 is the determined cDNA sequence for contig 45.
SEQ ID NO: 142 is the determined cDNA sequence for contig 47.
SEQ ID NO: 143 is the determined cDNA sequence for contig 48.
SEQ ID NO: 144 is the determined cDNA sequence for contig 49.
SEQ ID NO: 145 is the determined cDNA sequence for contig 50..
SEQ ID NO: 146 is the determined cDNA sequence for contig 53.
SEQ ID NO: 147 is the determined cDNA sequence for contig 54.
SEQ ID NO: 148 is the determined cDNA sequence for contig 56.
SEQ ID NO: 149 is the determined cDNA sequence for contig 57.
SEQ ID NO: 150 is the determined cDNA sequence for contig 58.
SEQ ID NO: 151 is the full-length cDNA sequence for L530S.
SEQ ID NO: 152 is the amino acid sequence encoded by SEQ ID NO: 151
SEQ ID NO: 153 is the full-length cDNA sequence of a first variant of L514S
SEQ ID NO: 154 is the full-length cDNA sequence of a second variant of L514S
SEQ ID NO: 15 5 is the amino acid sequence encoded by SEQ ID NO: 153.
SEQ ID NO: 156 is the amino acid sequence encoded by SEQ NO: 154.
SEQ ID NO: 157 is the determined cDNA sequence for contig 59.
SEQ ID NO: 158 is the full-length cDNA sequence for L763P (also referred to as contig 22)..
SEQ ID NO: 159 is the amino acid sequence encoded by SEQ ID NO: 158
SEQ ID NO: 160 is the full-length cDNA sequence for L762P (also referred to as contig 17).
SEQ ID NO: 161 is the amino acid sequence encoded by SEQ ID NO: 160
SEQ ID NO: 162 is the determined cDNA sequence for L515S.
SEQ ID NO: 163 is the full-length cDNA sequence of a first variant of L524S.
SEQ ID NO: 164 is the full-length cDNA sequence of a second variant of L524S.
SEQ ID NO: 165 is the amino acid sequence encoded by SEQ ID NO: 163.
SEQ ID NO: 166 is the amino acid sequence encoded by SEQ ID NO: 164.
SEQ ID NO: 167 is the full-length cDNA sequence of a first variant of L762P.
SEQ ID NO: 168 is the full-length cDNA sequence of a second variant of L762P.
SEQ ID NO: 169 is the amino acid sequence encoded by SEQ ID NO: 167.
SEQ ID NO: 170 is the amino acid sequence encoded by SEQ ID NO: 168.
SEQ ID NO: 171 is the full-length cDNA sequence for L773P (also referred to as contig 56).
SEQ ID NO: 172 is the amino acid sequence encoded by SEQ ID NO: 171.
SEQ ID NO: 173 is an extended cDNA sequence for L519S.
SEQ ID NO: 174 is the amino acid sequence encoded by SEQ ID NO: 174.
SEQ ID NO: 175 is the full-length cDNA sequence for L523S.
SEQ ID NO: 176 is the amino acid sequence encoded by SEQ ID NO: 175.
SEQ ID NO: 177 is the determined cDNA sequence for LST-sub5-7A.
SEQ ID NO: 178 is the determined cDNA sequence for LST-sub5-8G.
SEQ ID NO: 179 is the determined cDNA sequence for LST-sub5-8H.
SEQ ID NO: 180 is the determined cDNA sequence for LST-sub5-10B.
SEQ ID NO: 181 is the determined cDNA sequence for LST-sub5-10H.
SEQ ID NO: 182 is the determined cDNA sequence for LST-sub5-12B.
SEQ ID NO: 183 is the determined cDNA sequence for LST-sub5-11C.
SEQ ID NO: 184 is the determined cDNA sequence for LST-sub6-1c.
SEQ ID NO: 185 is the determined cDNA sequence for LST-sub6-2f.
SEQ ID NO: 186 is the determined cDNA sequence for LST-sub6-2G.
SEQ ID NO: 187 is the determined cDNA sequence for LST-sub6-4d.
SEQ ID NO: 188 is the determined cDNA sequence for LST-sub6-4e.
SEQ ID NO: 189 is the determined cDNA sequence for LSI-sub6-4f.
SEQ ID NO: 190 is the determined cDNA sequence for LST-sub6-3h.
SEQ ID NO: 191 is the determined cDNA sequence for LST-sub6-5d.
SEQ ID NO: 192 is the determined cDNA sequence for LST-sub6-5h.
SEQ ID NO: 193 is the determined cDNA sequence for LST-sub6-6h.
SEQ ID NO: 194 is the determined cDNA sequence for LST-sub6-7a.
SEQ ID NO: 195 is the determined cDNA sequence for LST-sub6-8a.
SEQ ID NO: 196 is the determined cDNA sequence for LST-sub6-7d.
SEQ ID NO: 197 is the determined cDNA sequence for LST-sub6-7e.
SEQ ID NO: 198 is the determined cDNA sequence for LST-sub6-8e.
SEQ ID NO: 199 is the determined cDNA sequence for LST-sub6-7g.
SEQ ID NO: 200 is the determined cDNA sequence for LSI-sub6-9f.
SEQ ID NO: 201 is the determined cDNA sequence for LST-sub6-9h.
SEQ ID NO: 202 is the determined cDNA sequence for LST-sub6-11b.
SEQ ID NO: 203 is the determined cDNA sequence for LST-suh6-11c.
SEQ ID NO: 204 is the determined cDNA sequence for LST-sub6-12c.
SEQ ID NO: 205 is the determined cDNA sequence for LST-sub6-12e.
SEQ ID NO: 206 is the determined cDNA sequence for LST-sub6-12f.
SEQ ID NO: 207 is the determined cDNA sequence for LST-sub6-11g.
SEQ ID NO: 208 is the determined cDNA sequence for LST-sub6-12g.
SEQ ID NO: 209 is the determined cDNA sequence for LST-sub6-12h.
SEQ ID NO: 210 is the determined cDNA sequence for LST-sub6-II-1a.
SEQ ID NO: 211 is the determined cDNA sequence for LST-sub6-II-2b.
SEQ ID NO: 212 is the determined cDNA sequence for LST-sub6-II-2g.
SEQ ID NO: 213 is the determined cDNA sequence for LST-sub6-II-1h.
SEQ ID NO: 214 is the determined cDNA sequence for LST-sub6-II-4a.
SEQ ID NO: 215 is the determined cDNA sequence for LST-sub6-II-4b.
SEQ ID NO: 216 is the determined cDNA sequence for LST-sub6-II-3e.
SEQ ID NO: 217 is the determined cDNA sequence for LST-sub6-II-4f.
SEQ ID NO: 218 is the determined cDNA sequence for LST-sub6-II-4g.
SEQ ID NO: 219 is the determined cDNA sequence for LST-sub6-II-4h.
SEQ ID NO: 220 is the determined cDNA sequence for LST-sub6-II-5c.
SEQ ID NO: 221 is the determined cDNA sequence for LST-sub6-II-5e.
SEQ ID NO: 222 is the determined cDNA sequence for LST-sub6-II-6f.
SEQ ID NO: 223 is the determined cDNA sequence for LST-sub6-II-5g.
SEQ ID NO: 224 is the determined cDNA sequence for LST-sub6-II-6g.
SEQ ID NO: 225 is the amino acid sequence for L528S.
SEQ ID NO: 226-251 are synthetic peptides derived from L762P.
SEQ ID NO: 252 is the expressed amino acid sequence of L514S.
SEQ ID NO: 253 is the DNA sequence corresponding to SEQ ID NO: 252.
SEQ ID NO: 254 is the DNA sequence of a L762P expression construct.
SEQ ID NO: 255 is the determined cDNA sequence for clone 23785.
SEQ ID NO: 256 is the determined cDNA sequence for clone 23786.
SEQ ID NO: 257 is the determined cDNA sequence for clone 23788.
SEQ ID NO: 258 is the determined cDNA sequence for clone 23790.
SEQ ID NO: 259 is the determined cDNA sequence for clone 23793.
SEQ ID NO: 260 is the determined cDNA sequence for clone 23794.
SEQ ID NO: 261 is the determined cDNA sequence for clone 23795.
SEQ ID NO: 262 is the determined cDNA sequence for clone 23796.
SEQ ID NO: 263 is the determined cDNA sequence for clone 23797.
SEQ ID NO: 264 is the determined cDNA sequence for clone 23798..
SEQ ID NO: 265 is the determined cDNA sequence for clone 23799.
SEQ ID NO: 266 is the determined cDNA sequence for clone 23800.
SEQ ID NO: 267 is the determined cDNA sequence for clone 23802.
SEQ ID NO: 268 is the determined cDNA sequence for clone 23803.
SEQ ID NO: 269 is the determined cDNA sequence for clone 23804.
SEQ ID NO: 270 is the determined cDNA sequence for clone 23805.
SEQ ID NO: 271 is the determined cDNA sequence for clone 23806.
SEQ ID NO: 272 is the determined cDNA sequence for clone 23807.
SEQ ID NO: 273 is the determined cDNA sequence for clone 23808.
SEQ ID NO: 274 is the determined cDNA sequence for clone 23809.
SEQ ID NO: 275 is the determined cDNA sequence for clone 23810.
SEQ ID NO: 276 is the determined cDNA sequence for clone 23811.
SEQ ID NO: 277 is the determine cDNA sequence for clone 23812.
SEQ ID NO: 278 is the determined cDNA sequence for clone 23813.
SEQ ID NO: 279 is the determined cDNA sequence for clone 23815.
SEQ ID NO: 280 is the determined cDNA sequence for clone 25298.
SEQ ID NO: 281 is the determined cDNA sequence for clone 25299.
SEQ ID NO: 282 is the determined cDNA sequence for clone 25300.
SEQ ID NO: 283 is the determined cDNA sequence for clone 25301
SEQ ID NO: 284 is the determined cDNA sequence for clone 25304
SEQ ID NO: 285 is the determined cDNA sequence for clone 25309.
SEQ ID NO: 286 is the determined cDNA sequence for clone 25312.
SEQ ID NO: 287 is the determined cDNA sequence for clone 25317.
SEQ ID NO:288 is the determined cDNA sequence for clone 25321.
SEQ ID NO:289 is the determined cDNA sequence for clone 25323.
SEQ ID NO:290 is the determined cDNA sequence for clone 25327.
SEQ ID NO:291 is the determined cDNA sequence for clone 25328.
SEQ ID NO:292 is the determined cDNA sequence for clone 25332.
SEQ ID NO:293 is the determined cDNA sequence for clone 25333.
SEQ ID NO:294 is the determined cDNA sequence for clone 25336.,
SEQ ID NO:295 is the determined cDNA sequence for clone 25340.
SEQ ID NO:296 is the determined cDNA sequence for clone 25342.
SEQ ID NO:297 is the determined cDNA sequence for clone 25356.
SEQ ID NO:298 is the determined cDNA sequence for clone 25357
SEQ ID NO:299 is the determined cDNA sequence for clone 25361.
SEQ ID NO:300 is the determined cDNA sequence for clone 25363.
SEQ ID NO:301 is the determined cDNA sequence for clone 25397.
SEQ ID NO:302 is the determined cDNA sequence for clone 25402.
SEQ ID NO:303 is the determined cDNA sequence for clone 25403.
SEQ ID NO:304 is the determined cDNA sequence for clone 25405.
SEQ ID NO:305 is the determined cDNA sequence for clone 25407.
SEQ ID NO:306 is the determined cDNA sequence for clone 25409.
SEQ ID NO:307 is the determined cDNA sequence for clone 25396.
SEQ ID NO:308 is the determined cDNA sequence for clone 25414.
SEQ ID NO:309 is the determined cDNA sequence for clone 25410.
SEQ ID NO:310 is the determined cDNA sequence for clone 25406.
SEQ ID NO:311 is the determined cDNA sequence for clone 25306.
SEQ ID NO:312 is the determined cDNA sequence for clone 25362.
SEQ ID NO:313 is the determined cDNA sequence for clone 25360.
SEQ ID NO:314 is the determined cDNA sequence for clone 25398.
SEQ ID NO:315 is the determined cDNA sequence for clone 25355.
SEQ ID NO:316 is the determined cDNA sequence for clone 25351.
SEQ ID NO:317 is the determined cDNA sequence for clone 25331.
SEQ ID NO:318 is the determined cDNA sequence for clone 25338.
SEQ ID NO:319 is the determined cDNA sequence for clone 25335.
SEQ ID NO:320 is the determined cDNA sequence for clone 25329.
SEQ ID NO:321 is the determined cDNA sequence for clone 25324.
SEQ ID NO:322 is the determined cDNA sequence for clone 25322.
SEQ ID NO:323 is the determined cDNA sequence for clone 25319.
SEQ ID NO:324 is the determined cDNA sequence for clone 25316.
SEQ ID NO:325 is the determined cDNA sequence for clone 25311.
SEQ ID NO:326 is the determined cDNA sequence for clone 25310.
SEQ ID NO:327 is the determined cDNA sequence for clone 25302.
SEQ ID NO:328 is the determined cDNA sequence for clone 25315.
SEQ ID NO:329 is the determined cDNA sequence for clone 25308.
SEQ ID NO:330 is the determined cDNA sequence for clone 25303.
SEQ ID NO:331-337 are the cDNA sequences of isoforms of the p53 tumor suppressor homologue, p63 (also referred to as L530S).
SEQ ID NO:338-344 are the amino acid sequences encoded by SEQ ID NO:331-337, respectively
SEQ ID NO:345 is a second cDNA sequence for the antigen L763P.
SEQ ID NO:346 is the amino acid sequence encoded by the sequence of SEQ ID NO: 345.
SEQ ID NO:347 is a determined full-length cDNA sequence for L523S.
SEQ ID NO:348 is the amino acid sequence encoded by SEQ ID NO: 347.
SEQ ID NO:349 is the cDNA sequence encoding the N-terminal portion of L773P.
SEQ ID NO:350 is the amino acid sequence of the N-terminal portion of L773P.
SEQ ID NO:351 is the DNA sequence for a fusion of Ra12 and the N-terminal portion of L763P.
SEQ ID NO:352 is the amino acid sequence of the fusion of Ra12 and the N-terminal portion of L763P.
SEQ ID NO:353 is the DNA sequence for a fusion of Ra12 and the C-terminal portion of L763P.
SEQ ID NO:354 is the amino acid sequence of the fusion of Ra12 and the C-terminal portion of L763P.
SEQ ID NO:355 is a primer.
SEQ ID NO:356 is a primer.
SEQ ID NO:357 is the protein sequence of expressed recombinant L762P.
SEQ ID NO:358 is the DNA sequence of expressed recombinant L762P.
SEQ ID NO:359 is a primer.
SEQ ID NO:360 is a primer.
SEQ ID NO:361 is the protein sequence of expressed recombinant L773P A.
SEQ ID NO:362 is the DNA sequence of expressed recombinant L773P A.
SEQ ID NO:363 is an epitope derived from clone L773P polypeptide.
SEQ ID NO:364 is a polynucleotide encoding the polypeptide of SEQ ID NO:363.
SEQ ID NO:365 is an epitope derived from clone L773P polypeptide..
SEQ ID NO:366 is a polynucleotide encoding the polypeptide of SEQ ID NO:365.
SEQ ID NO:367 is an epitope consisting of amino acids 571-590 of SEQ ID NO:161, clone L762P.
SEQ ID NO:368 is the full-length DNA sequence for contig 13 (SEQ ID NO:125), also referred to as L761P.
SEQ ID NO:369 is the protein sequence encoded by the DNA sequence of SEQ ID NO:368.
SEQ ID NO:370 is an L762P DNA sequence from nucleotides 2071-2130.
SEQ ID NO:371 is an L762P DNA sequence from nucleotides 1441-1500.
SEQ ID NO:372 is an L762P DNA sequence from nucleotides 1936-1955.
SEQ ID NO:373 is an L762P DNA sequence from nucleotides 2620-2679.
SEQ ID NO:374 is an L762P DNA sequence from nucleotides 1801-1860.
SEQ ID NO:375 is an L762P DNA sequence from nucleotides 1531-1591.
SEQ ID NO:376 is the amino acid sequence of the L762P peptide encoded by SEQ ID NO:373.
SEQ ID NO:377 is the amino acid sequence of the L762P peptide encoded by SEQ ID NO:370.
SEQ ID NO:378 is the amino acid sequence of the L762P peptide encoded by SEQ ID NO:372.
SEQ ID NO:379 is the amino acid sequence of the L762P peptide encoded by SEQ ID NO:374.
SEQ ID NO:380 is the amino acid sequence of the L762P peptide encoded by SEQ ID NO:371.
SEQ ID NO:381 is the amino acid sequence of the L762P peptide encoded by SEQ ID NO:375.
SEQ ID NO:382 is the amino acid sequence of an epitope of L762P.
SEQ ID NO:383-386 are PCR primers.
SEQ ID NO:387-395 are the amino acid sequences of L773P peptides.
SEQ ID NO:396-419 are the amino acid sequences of L523S peptides.
SEQ ID NO:420 is the determined cDNA sequence for clone #19014.
SEQ ID NO:421 is the forward primer PDM-278 for the L514S-13160 coding region.
SEQ ID NO:422 is the reverse primer PDM-278 for the L514S-13160 coding region.
SEQ ID NO:423 is the amino acid sequence for the expressed recombinant L514S.
SEQ ID NO:424 is the DNA coding sequence for the recombinant L514S.
SEQ ID NO:425 is the forward primer PDM-414 for the L523S coding region.
SEQ ID NO:426 is the reverse primer PDM-414 for the L523S coding region.
SEQ ID NO:427 is the amino acid sequence for the expressed recombinant L523S.
SEQ ID NO:428 is the DNA coding sequence for the recombinant L523S.
SEQ ID NO:429 is the reverse primer PDM-279 for the L762PA coding region.
SEQ ID NO:430 is the amino acid sequence for the expressed recombinant L762PA.
SEQ ID NO:431 is the DNA coding sequence for the recombinant L762PA.
SEQ ID NO:432 is the reverse primer PDM-300 for the L773P coding region.
SEQ ID NO:433 is the amino acid sequence of the expressed recombinant L773P.
EQ ID NO:434 is the DNA coding sequence for the recombinant L773P.
SEQ ID NO:435 is the forward primer for TCR Valpha8.
SEQ ID NO:436 is the reverse primer for TCR Valpha8.
SEQ ID NO:437 is the forward primer for TCR Vbeta8.
SEQ ID NO:438 is the reverse primer for TCR Vbeta8.
SEQ ID NO:439 is the TCR Valpha DNA sequence of the TCR clone specific for the lung antigen L762P.
SEQ ID NO:440 is the TCR Vbeta DNA sequence of the TCR clone specific for the lung antigen L762P.
SEQ ID NO:441 is the amino acid sequence of L763 peptide #2684.
SEQ ID NO:442 is the predicted full-length cDNA for the cloned partial sequence of clone L529S (SEQ ID NO:106).
SEQ ID NO:443 is the deduced amino acid sequence encoded by SEQ ID NO:442.
SEQ ID NO:444 is the forward primer PDM-734 for the coding region of clone L523S.
SEQ ID NO:445 is the reverse primer PDM-735 for the coding region of clone L523S.
SEQ ID NO:446 is the amino acid sequence for the expressed recombinant L523S.
SEQ ID NO:447 is the DNA coding sequence for the recombinant L523S.
SEQ ID NO:448 is another forward primer PDM-733 for the coding region of clone L523S.
SEQ ID NO:449 is the amino acid sequence for a second expressed recombinant L523S.
SEQ ID NO:450 is the DNA coding sequence for a second recombinant L523S.
SEQ ID NO:451 corresponds to amino acids 86-110, an epitope of L514S-specific in the generation of antibodies.
SEQ ID NO:452 corresponds to amino acids 21-45, an epitope of L514S-specific in the generation of antibodies.
SEQ ID NO:453 corresponds to amino acids 121-135, an epitope of L514S-specific in the generation of antibodies.
SEQ ID NO:454 corresponds to amino acids 440-460, an epitope of L523S-specific in the generation of antibodies.
SEQ ID NO:455 corresponds to amino acids 156-175, an epitope of L523S-specific in the generation of antibodies.
SEQ ID NO:456 corresponds to amino acids 326-345, an epitope of L523S-specific in the generation of antibodies.
SEQ ID NO:457 corresponds to amino acids 40-59, an epitope of L523S-specifc in the generation of antibodies.
SEQ ID NO:458 corresponds to amino acids 80-99, an epitope of L523S-specific in the generation of antibodies.
SEQ ID NO:459 corresponds to amino acids 160-179, an epitope of L523S-specifc in the generation of antibodies.
SEQ ID NO:460 corresponds to amino acids 180-199, an epitope of L523S-specific in the generation of antibodies..
SEQ ID NO:461 corresponds to amino acids 320-339, an epitope of L523S-specific in the generation of antibodies.
SEQ ID NO:462 corresponds to amino acids 340-359, an epitope of L523S-specific in the generation of antibodies.
SEQ ID NO:463 corresponds to amino acids 370-389, an epitope of L523S-specific in the generation of antibodies.
SEQ ID NO:464 corresponds to amino acids 380-399, an epitope of L523S-specific in the generation of antibodies.
SEQ ID NO:465 corresponds to amino acids 37-55, an epitope of L523S-recognized by the L523S-specific CTL line 6B1.
SEQ ID NO:466 corresponds to amino acids 41-51, the mapped antigenic epitope of L523S-recognized by the L523S-specific CTL line 6B1.
SEQ ID NO:467 corresponds to the DNA sequence which encodes SEQ ID NO:466.
SEQ ID NO:468 corresponds to the amino acids of peptide 16, 17 of hL523S.
SEQ ID NO:469 corresponds to the amino acids of peptide 16, 17 of mL523S

### DETAILED DESCRIPTION OF THE INVENTION

The present invention is directed generally to compositions and their use in the therapy and diagnosis of cancer, particularly lung cancer. As described further below, illustrative compositions of the present invention include, but are not restricted to, polypeptides, particularly immunogenic polypeptides, polynucleotides encoding such polypeptides, antibodies and other binding agents, antigen presenting cells (APCs) and immune system cells (*e*.*g*., T cells).

The practice of the present invention will employ, unless indicated specifically to the contrary, conventional methods of virology, immunology, microbiology, molecular biology and recombinant DNA techniques within the skill of the art, many of which are described below for the purpose of illustration.. Such techniques are explained fully in the literature. See, *e*.*g*., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D.. Glover, ed.); Oligonucleotide Synthesis (N. Gait, ed., 1984); Nucleic Acid Hybridization (B. Hames & S. Higgins, eds., 1985); Transcription and Tianslation (B. Hames & S. Higgins, eds., 1984); Animal Cell Culture (R. Freshney, ed., 1986); Perbal, A Practical Guide to Molecular Cloning (1984).

All publications, patents and patent applications cited herein, whether supra or infra, are hereby incorporated by reference in their entirety.

As used in this specification and the appended claims, the singular forms "a," "an" and "the" include plural references unless the content clearly dictates otherwise.

### Polypeptide Compositions

As used herein, the term "polypeptide" " is used in its conventional meaning, i.e., as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-natulally occurring. A polypeptide may be an entire protein, or a subsequence thereof Particular polypeptides of interest in the context of this invention are amino acid subsequences comprising epitopes, i..e., antigenic determinants substantially responsible for the immunogenic properties of a polypeptide and being capable of evoking an immune response..

Particularly illustrative polypeptides of the present invention comprise those encoded by a polynucleotide sequence set forth in any one of SEQ ID NO:1-3, 6-8, 10-13, 15-27, 29, 30, 32, 34-49, 51,52, 54, 55, 57-59, 61-69,71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, or a sequence that hybridizes under moderately stringent conditions, or, alternatively, under highly stringent conditions, to a polynucleotide sequence set forth in any one of SEQ ID NO:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467. Certain illustrative polypeptides of the invention comprise amino acid sequences as set forth in any one of SEQ ID NO:152, 155, 156, 165, 166, 169, 170, 172, 174, 176, 226-252, 338-344, 346, 350, 357, 361, 363, 365, 367, 369, 376-382, 387-419, 423, 427, 430, 433, 441, 443, 446, 449, 451-466 and 468-469..

The polypeptides of the present invention are sometimes herein referred to as lung tumor proteins or lung tumor polypeptides, as an indication that their identification has been based at least in part upon their increased levels of expression in lung tumor samples. Thus, a "lung tumor polypeptide" or "lung tumor protein," refers generally to a polypeptide sequence of the present invention, or a polynucleotide sequence encoding such a polypeptide, that is expressed in a substantial proportion of lung tumor samples, for example preferably greater than about 20%, more preferably greater than about 30%, and most preferably greater than about 50% or more of lung tumor samples tested, at a level that is at least two fold, and preferably at least five fold, greater than the level of expression in normal tissues, as determined using a representative assay provided herein.. A lung tumor polypeptide sequence of the invention, based upon its increased level of expression in tumor cells, has particular utility both as a diagnostic marker as well as a therapeutic target, as further described below.

In certain preferred embodiments, the polypeptides of the invention are immunogenic, *i*.*e*., they react detectably within an immunoassay (such as an ELISA or T-cell stimulation assay) with antisera and/or T-cells from a patient with lung cancer. Screening for immunogenic activity can be performed using techniques well known to the skilled artisan. For example, such screens can be performed using methods such as those described in Harlow and Lane, *Antibodies. A Laboratory Manual,* Cold Spring Harbor Laboratory, 1988.. In one illustrative example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As would be recognized by the skilled artisan, immunogenic portions of the polypeptides disclosed herein are also encompassed by the present invention. An "immunogenic portion," as used herein, is a fragment of an immunogenic polypeptide of the invention that itself is immunologically reactive (*i*.*e*., specifically binds) with the B-cells and/or T-cell surface antigen receptors that recognize the polypeptide. Immunogenic portions may generally be identified using well known techniques, such as those summarized in Paul, Fundamental Immunology, 3rd ed.., 243-247 (Raven Press, 1993) and references cited therein. Such techniques include screening polypeptides for the ability to react with antigen-specific antibodies, antisera and/or T-cell lines or clones.. As used herein, antisera and antibodies are "antigen-specific" if they specifically bind to an antigen (i e., they react with the protein in an ELISA or other immunoassay, and do not react detectably with unrelated proteins). Such antisera and antibodies may be prepared as described herein, and using well-known techniques.

In one preferred embodiment, an immunogenic portion of a polypeptide of the present invention is a portion that reacts with antisera and/or T-cells at a level that is not substantially less than the reactivity of the full-length polypeptide (*e.g.,* in an ELISA and/or T-cell reactivity assay). Preferably, the level of immunogenic activity of the immunogenic portion is at least about 50%, preferably at least about 70% and most preferably greater than about 90% of the immunogenicity for the full-length polypeptide. In some instances, preferred immunogenic portions will be identified that have a level of immunogenic activity greater than that of the corresponding full-length polypeptide, *e*.*g*., having greater than about 100% or 150% or more immunogenic activity.

In certain other embodiments, illustrative immunogenic portions may include peptides in which an N-terminal leader sequence and/or transmembrane domain have been deleted. Other illustrative immunogenic portions will contain a small N-and/or C-terminal deletion (*e*.*g*., 1-30 amino acids, preferably, 5-15 amino acids), relative to the mature protein.

In another embodiment, a polypeptide composition of the invention may also comprise one or more polypeptides that are immunologically reactive with T cells and/or antibodies generated against a polypeptide of the invention, particularly a polypeptide having an amino acid sequence disclosed herein, or to an immunogenic fragment or variant thereof.

In another embodiment of the invention, polypeptides are provided that comprise one or more polypeptides that are capable of eliciting T cells and/or antibodies that are immunologically reactive with one or more polypeptides described herein, or one or more polypeptides encoded by contiguous nucleic acid sequences contained in the polynucleotide sequences disclosed herein, or immunogenic fragments or variants thereof, or to one or more nucleic acid sequences which hybridize to one or more of these sequences under conditions of moderate to high stringency.

The present invention, in another aspect, provides polypeptide fragments comprising at least about 5, 10, 15, 20, 25, 50, or 100 contiguous amino acids, or more, including all intermediate lengths, of a polypeptide compositions set forth herein, such as those set forth in SEQ ID NO:152, 155, 156, 165, 166, 169, 170, 172, 174, 176, 226-252, 338-344, 346, 350, 357, 361, 363, 365, 367, 369, 376-382 and 387-419, 441, 443, 446, 449, 451-466 and 468-469, or those encoded by a polynucleotide sequence set forth in a sequence of SEQ ID NO:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467.

In another aspect, the present invention provides variants of the polypeptide compositions described herein. Polypeptide variants generally encompassed by the present invention will typically exhibit at least about 70%, 75%, 80%, 85%, 90%, 91%, 92%, 93%, 94%, 95%, 96%, 97%, 98%, or 99% or more identity (determined as described below), along its length, to a polypeptide sequences set forth herein.

In one preferred embodiment, the polypeptide fragments and variants provide by the present invention are immunologically reactive with an antibody and/or T-cell that reacts with a full-length polypeptide specifically set for the herein.

In another preferred embodiment, the polypeptide fragments and variants provided by the present invention exhibit a level of immunogenic activity of at least about 50%, preferably at least about 70%, and most preferably at least about 90% or more of that exhibited by a full-length polypeptide sequence specifically set forth herein.

A polypeptide "variant," as the term is used herein, is a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences of the invention and evaluating their immunogenic activity as described herein and/or using any of a number of techniques well known in the art.

For example, certain illustrative variants of the polypeptides of the invention include those in which one or more portions, such as an N-terminal leader sequence or transmembrane domain, have been removed. Other illustrative Variants include variants in which a small portion (*e*.*g*., 1-30 amino acids, preferably 5-15 amino acids) has been removed from the N- and/or C-terminal of the mature protein.

In many instances, a variant will contain conservative substitutions A "conservative substitution" is one in which an amino acid is substituted for another amino acid that has similar properties, such that one skilled in the art of peptide chemistry would expect the secondary structure and hydropathic nature of the polypeptide to be substantially unchanged. As described above, modifications may be made in the structure of the polynucleotides and polypeptides of the present invention and still obtain a functional molecule that encodes a variant or derivative polypeptide with desirable characteristics, *e*.*g*., with immunogenic characteristics. When it is desired to alter the amino acid sequence of a polypeptide to create an equivalent, or even an improved, immunogenic variant or portion of a polypeptide of the invention, one skilled in the art will typically change one or more of the codons of the encoding DNA sequence according to Table 1.

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated that various changes may be made in the peptide sequences of the disclosed compositions, or corresponding DNA sequences which encode said peptides without appreciable loss of their biological utility or activity.

**Table 1**

| **Amino Acids** | | **Codons** | | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | I | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

In making such changes, the hydropathic index of amino acids may be considered. The importance of the hydropathic amino acid index in conferring interactive biologic function on a protein is generally understood in the art (Kyte and Doolittle, 1982, incorporated herein by reference). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that certain amino acids may be substituted by other amino acids having a similar hydropathic index or score and still result in a protein with similar biological activity, *i e*. still obtain a biological functionally equivalent protein. In making such changes, the substitution of amino acids whose hydropathic indices are within ±2 is Preferred, those within ±1 are particularly preferred, and those within ±0.5 are even more particularly referred. It is also understood in the art that the substitution of like amino acids can be made effectively on the basis of hydrophilicity. U. S. Patent 4,554,101 (specifically incorporated herein by reference in its entirety), states that the greatest local average hydrophilicity of a protein, as governed by the hydrophilicity of its adjacent amino acids, correlates with a biological property of the protein.

As detailed in U. S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those within ±1 are particularly preferred, and those within ±0.5 are even more particularly referred.

As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine.

In addition, any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetylmethyl-, thio- and other modified forms of adenine, cytidine, guanine, thymine and uridine.

Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain non-conservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein, which co-translationally or post-translationally directs transfer of the protein. The polypeptide may also be conjugated to a linker or other sequence for ease of synthesis, purification or identification of the polypeptide (*e*.*g*., poly-His), or to enhance binding of the polypeptide to a solid support. For example, a polypeptide may be conjugated to an immunoglobulin Fc region.

When comparing polypeptide sequences, two sequences are said to be "identical" if the sequence of amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASIAR, Inc., Madison, WI), using default parameters. This program embodies several alignment schemes described in the following references: Dayhoff, M.O.. (1978) A model of evolutionary change in proteins - Matrices for detecting distant relationship. In Dayhoff, M.O. (ed.) Atlas of Protein Sequence and Structure, National Biomedical Research Foundation, Washington DC Vol.. 5, Suppl. 3, pp.. 345-358; Hein J. (1990) Unified Approach to Alignment and Phylogenes pp. 626. 645 Methods in Enzymology vol. 183, Academic Press, Inc.., San Diego, CA; Higgins, D.G. and Sharp, P.M.. (1989) CABIOS 5:151-153; Myers, E..W. and Muller W. (1988) CABIOS 4:11-17; Robinson, E..D. (1971) Comb. Theor 11:105; Santou, N. Nes, M.. (1987) Mol. Biol. Evol. 4:406-425; Sneath, P..HA. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl Acad., Sci. USA 80:726-730.

Alternatively, optimal alignment of sequences for comparison may be conducted by the local identity algorithm of Smith and Waterman (1981) *Add. APL. Math* 2:482, by the identity alignment algorithm of Needleman and Wunsch (1970) J. Mol. Biol. 48:443, by the search for similarity methods of Pearson and Lipman (1988) Proc. Natl. Acad. Sci. USA 85: 2444, by computerized implementations of these algorithms (GAP, BESTFIT, BLASI, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al., (1977) Nucl. Acids Res. 25:3389-3402 and Altschul et al. (1990) J. Mol. Biol. 215:403-410, respectively. BLASI and BLAST 2.0 can be used, for example with the parameters described herein, to determine percent sequence identity for the polynucleotides and polypeptides of the invention. Software for performing BLAST analyses is publicly available through the National Center for Biotechnology Information. For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative scoring residue alignments; or the end of either sequence is reached.. The BLAST algorithm parameters W, T and X determine the sensitivity and speed of the alignment..

In one preferred approach, the "percentage of sequence identity" is determined by comparing two optimally aligned sequences over a window of comparison of at least 20 positions, wherein the portion of the polypeptide sequence in the comparison window may comprise additions or deletions (*i.e.,* gaps) of 20 percent or less, usually 5 to 15 percent, on 10 to 12 percent, as compared to the reference sequences (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the reference sequence *(i.e.,* the window size) and multiplying the results by 100 to yield the percentage of sequence identity.

Within other illustrative embodiments, a polypeptide may be a fusion polypeptide that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably I helper epitopes recognized by humans, or may assist in expressing the protein (an expression enhancer) at higher yields than the native recombinant protein. Certain preferred fusion partners are both immunological and expression enhancing fusion partner. Other fusion partners may be selected so as to increase the solubility of the polypeptide or to enable the polypeptide to be targeted to desired intracellular compartments. Still further fusion partners include affinity tags, which facilitate purification of the polypeptide.

Fusion polypeptides may generally be prepared using standard techniques, including chemical conjugation Preferably, a fusion polypeptide is expressed as a recombinant polypeptide, allowing the production of increased levels, relative to a non-fused polypeptide, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion polypeptide that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion polypeptide using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues.. Other near neutral amino acids, such as Thr and Ala may also be used in the linker sequence. Amino acid sequences which may be usefully employed as linkers include those disclosed in Maratea et al.., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements.. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

The fusion polypeptide can comprise a polypeptide as described herein together with an unrelated immunogenic protein, such as an immunogenic protein capable of eliciting a recall response.. Examples of such proteins include tetanus, tuberculosis and hepatitis proteins (*see,* for example, Stoute et al. New Engl J. Med., 336:86-91, 1997)..

In one preferred embodiment, the immunological fusion partner is derived from a Mycobacterium sp., such as a Mycobacterium tuberculosis-derived Ra12 fragment Ra12 compositions and methods for their use in enhancing the expression and/or immunogenicity of heterologous polynucleotide/polypeptide sequences is described in U.S. Patent Application 60/158,585, the disclosure of which is incorporated herein by reference in its entirety. Briefly, Ra12 refers to a polynucleotide region that is a subsequence of a *Mycobacterium tuberculosis* MTB32A nucleic acid. MTB32A is a serine protease of 32 KD molecular weight encoded by a gene in virulent and avirulent strains of *M. tuberculosis.* The nucleotide sequence and amino acid sequence of MTB32A have been described (for example, U.S. Patent Application 60/158,585; see also, Skeiky et al., Infection and Immun. (1999) 67:3998-4007, incorporated herein by reference). C-terminal fragments of the MTB32A coding sequence express at high levels and remain as a soluble polypeptides throughout the purification process.. Moreover, Ra12 may enhance the immunogenicity of heterologous immunogenic polypeptides with which it is fused. One preferred Ra12 fusion polypeptide comprises a 14 KD C-terminal fragment corresponding to amino acid residues 192 to 323 of MTB32A.

Other preferred Ra12 polynucleotides generally comprise at least about 15 consecutive nucleotides, at least about 30 nucleotides, at least about 60 nucleotides, at least about 100 nucleotides, at least about 200 nucleotides, or at least about 300 nucleotides that encode a portion of a Ra12 polypeptide..

Ra12 polynucleotides may comprise a native sequence (i..e.., an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide or a portion thereof.

Within other preferred embodiments, an immunological fusion partner is derived from protein D, a surface protein of the gram-negative bacterium Haemophilus influenza B (WO 91/18926). Preferably, a protein D derivative comprises approximately the first third of the protein (*e.g.,* the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYIA (encoded by the LytA gene; *Gene 43*:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (*see Biotechnology 10*:795-798, 1992). Within a preferred embodiment, a repeat portion of LYTA may be incorporated into a fusion polypeptide. A repeat portion is found in the C-terminal region starting at residue 178. A particularly preferred repeat portion incorporates residues 188-305.

Yet another illustrative embodiment involves fusion polypeptides, and the polynucleotides encoding them, wherein the fusion partner comprises a targeting signal capable of directing a polypeptide to the endosomal/lysosomal compartment, as described in U.S. Patent No. 5,633,234 An immunogenic polypeptide of the invention, when fused with this targeting signal, will associate more efficiently with MHC class II molecules and thereby provide enhanced in vivo stimulation of CD4⁺ T cells specific for the polypeptide.

Polypeptides of the invention are prepared using any of a variety of well known synthetic and/or recombinant techniques, the latter of which are further described below. Polypeptides, portions and other variants generally less than about 150 amino acids can be generated by synthetic means, using techniques well known to those of ordinary skill in the art. In one illustrative example, such polypeptides are synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division (Foster City, CA), and may be operated according to the manufacturer's instructions.

In general, polypeptide compositions (including fusion polypeptides) of the invention are isolated. An "isolated" polypeptide is one that is removed from its original environment. For example, a naturally-occurring protein or polypeptide is isolated if it is separated from some or all of the coexisting materials in the natural system. Preferably, such polypeptides are also purified, *e.g.*, are at least about 90% pure, more preferably at least about 95% pure and most preferably at least about 99% pure.

### Polynucleotide Compositions

The present invention, in other aspects, provides polynucleotide compositions. The terms "DNA" and "polynucleotide" are used essentially interchangeably herein to refer to a DNA molecule that has been isolated free of total genomic DNA of a particular species. "Isolated," as used herein, means that a polynucleotide is substantially away from other coding sequences, and that the DNA molecule does not contain large portions of unrelated coding DNA, such as large chromosomal fragments or other functional genes or polypeptide coding regions. Of course, this refers to the DNA molecule as originally isolated, and does not exclude genes or coding regions later added to the segment by the hand of man.

As will be understood by those skilled in the art, the polynucleotide compositions of this invention can include genomic sequences, extra-genomic and plasmid-encoded sequences and smaller engineered gene segments that express, or may be adapted to express, proteins, polypeptides, peptides and the like. Such segments may be naturally isolated, or modified synthetically by the hand of man..

As will be also recognized by the skilled artisan, polynucleotides of the invention may be single-stranded (coding or antisense) or double-stranded, and may be DNA (genomic, cDNA or synthetic) or RNA molecules. RNA molecules may include HnRNA molecules, which contain introns and correspond to a DNA molecule in a one to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i.e*., an endogenous sequence that encodes a polypeptide/protein of the invention or a portion thereof) or may comprise a sequence that encodes a variant or derivative, preferably and immunogenic variant or derivative, of such a sequence.

Therefore, according to another aspect of the present invention, polynucleotide compositions are provided that comprise some or all of a polynucleotide sequence set forth in any one of SEQ ID NO:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, complements of a polynucleotide sequence set forth in any one of SEQ ID NO:1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153,154, 157, 158, 160, 167, 168, 171, 179, 182, 184-186, 188-191, 193, 194, 198-207, 209, 210, 213, 214, 217, 220-224, 253-337, 345, 347, 349, 358, 362, 364, 365, 368, 370-375, 420, 424, 428, 431, 434, 442, 447, 450 and 467, and degenerate variants of a polynucleotide sequence set forth in any one of SEQ ID NO:1-3, 68, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186,188-191, 193, 194, 198-207, 209, 210, 213,214,217,220-224,253-337, 345, 347, 349, 358, 362, 364, 365, 368, 370-375, 420, 424, 428, 431, 434, 442, 447, 450 and 467. In certain preferred embodiments, the polynucleotide sequences set forth herein encode immunogenic polypeptides, as described above.

In other related embodiments, the present invention provides polynucleotide variants having substantial identity to the sequences disclosed herein in SEQ ID NO:1-3, 6-8,10-13,15-27, 29,30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-3.37, 345, 347, 349, 358, 362, 364, 365, 368, 370-375, 420, 424, 428, 431, 434, 442, 447, 450 and 467, for example those comprising at least 70% sequence identity, preferably at least 75%, 80%, 85%, 90%, 95%, 96%, 97%, 98%, or 99% or higher, sequence identity compared to a polynucleotide sequence of this invention using the methods described herein, (*e.g.*, BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like..

Typically, polynucleotide variants will contain one or more substitutions, additions, deletions and/or insertions, preferably such that the immunogenicity of the polypeptide encoded by the variant polynucleotide is not substantially diminished relative to a polypeptide encoded by a polynucleotide sequence specifically set forth herein) The term "variant" should also be understood to encompasses homologous genes of xenogenic origin.

In additional embodiments, the present invention provides polynucleotide fragments comprising various lengths of contiguous stretches of sequence identical to or complementary to one or more of the sequences disclosed herein For example, polynucleotides are provided by this invention that comprise at least about 10, 15, 20, 30, 40, 50, 75, 100, 150, 200, 300, 400, 500 or 1000 or more contiguous nucleotides of one or more of the sequences disclosed herein as well as all intermediate lengths there between. It will be readily understood that "intermediate lengths", in this context, means any length between the quoted values, such as 16, 17, 18, 19, *etc.;* 21, 22, 23, *etc.;* 30, 31, 32, *etc.;* 50, 51, 52, 53, *etc.;* 100, 101, 102, 103, *etc.;* 150,151, 152, 153, *etc.;* including all integers through 200-500; 500-1,000, and the like.

In another embodiment of the invention, polynucleotide compositions are provided that are capable of hybridizing under moderate to high stringency conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5 X SSC, 0.5% SDS, 1.0 mM EDTA (pH 8..0); hybridizing at 50°C-60°C, 5 X SSC, overnight; followed by washing twice at 65°C for 20 minutes with each of 2X, 0.5X and 0.2X SSC containing 0.1% SDS.. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, *e.g.*, to 60-65°C or 65-70°C.

In certain preferred embodiments, the polynucleotides described above, eg., polynucleotide variants, fragments and hybridizing sequences, encode polypeptides that are immunologically cross-reactive with a polypeptide sequence specifically set forth herein. In other referred embodiments, such polynucleotides encode polypeptides that have a level of immunogenic activity of at least about 50%, preferably at least about 70%, and more preferably at least about 90% of that for a polypeptide sequence specifically set forth herein.

The polynucleotides of the present invention, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol. For example, illustrative polynucleotide segments with total lengths of about 10,000, about 5000, about 3000, about 2,000, about 1,000, about 500, about 200, about 100, about 50 base pairs in length, and the like, (including all intermediate lengths) are contemplated to be useful in many implementations of this invention.

When comparing polynucleotide sequences, two sequences are said to be "identical" if the sequence of nucleotides in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff. M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc.., San Diego, CA; Higgins, D.G. and Sharp, P.M. (1989) CABIOS 5:151-153; Myers, E.W.. and Muller W. (1988) CABIOS 4:11-17; Robinson, E.D. (1971) Comb. Theor 11:105; Santou, N. Nes, M. (1987) Mol. Biol Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Alternatively, optimal alignment of sequences for comparison may be conducted by the local identity algorithm of Smith and Waterman (1981) Add. APL. Math 2:482, by the identity alignment algorithm of Needleman and Wunsch (1970) J Mol Biol. 48:443, by the search for similarity methods of Pearson and Lipman (1988) Proc. Natl. Acad Sci. USA 85: 2444, by computerized implementations of these algorithms (GAP, BESIFIT, BLASI, FASTA, and TFASTA in the Wisconsin Genetics Software Package, Genetics Computer Group (GCG), 575 Science Dr, Madison, WI), or by inspection.

One preferred example of algorithms that are suitable for determining percent sequence identity and sequence similarity are the BLAST and BLAST 2.0 algorithms, which are described in Altschul et al.. (1977) Nucl. Acids Res. 25:3389-3402 and Altschul et al. (1990) J. Mol. Biol. 215:403-410, respectively. BLAST and BLAST 2.0 can be used, for example with the parameters described herein, to determine percent sequence identity for the polynucleotides of the invention. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) Proc. Natl. Acad. Sci. USA 89:10915) alignments, (B) of 50, expectation (E) of 10, M=5, N=-4 and a comparison of both strands

Preferably, the "percentage of sequence identity" is determined by comparing two optimally aligned sequences over a window of comparison of at least 20 positions, wherein the portion of the polynucleotide sequence in the comparison window may comprise additions or deletions (*i.e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases occurs in both sequences to yield the number of matched positions, dividing the number of matched positions by the total number of positions in the reference sequence (*i.e.,* the window size) and multiplying the results by 100 to yield the percentage of sequence identity.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Therefore, in another embodiment of the invention, a mutagenesis approach, such as site-specific mutagenesis, is employed for the preparation of immunogenic variants and/or derivatives of the polypeptides described herein, By this approach, specific modifications in a polypeptide sequence can be made through mutagenesis of the underlying polynucleotides that encode them. These techniques provides a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

In certain embodiments of the present invention, the inventors contemplate the mutagenesis of the disclosed polynucleotide sequences to alter one or more properties of the encoded polypeptide, such as the immunogenicity of a polypeptide vaccine. I he techniques of site-specific mutagenesis are well-known in the art, and are widely used to create variants of both polypeptides and polynucleotides. For example, site-specific mutagenesis is often used to alter a specific portion of a DNA molecule. In such embodiments, a primer comprising typically about 14 to about 25 nucleotides or so in length is employed, with about 5 to about 10 residues on both sides of the junction of the sequence being altered.

As will be appreciated by those of skill in the art, site-specific mutagenesis techniques have often employed a phage vector that exists in both a single stranded and double stranded form. Typical vectors useful in site-directed mutagenesis include vectors such as the M13 phage. These phage are readily commercially-available and their use is generally well known to those skilled in the art. Double-stranded plasmids are also routinely employed in site directed mutagenesis that eliminates the step of transferring the gene of interest from a plasmid to a phage.

In general, site-directed mutagenesis in accordance herewith is performed by first obtaining a single-stranded vector or melting apart of two strands of a double-stranded vector that includes within its sequence a DNA sequence that encodes the desired peptide. An oligonucleotide primer bearing the desired mutated sequence is prepared, generally synthetically. This primer is then annealed with the single-stranded vector, and subjected to DNA polymerizing enzymes such as *E. coli* polymerase I Klenow fragment, in order to complete the synthesis of the mutation-bearing strand. Thus, a heteroduplex is formed wherein one strand encodes the original non-mutated sequence and the second strand bears the desired mutation This heteroduplex vector is then used to transform appropriate cells, such as *E. coli* cells, and clones are selected which include recombinant vectors bearing the mutated sequence arrangement.

The preparation of sequence variants of the selected peptide-encoding DNA segments using site-directed mutagenesis provides a means of producing potentially useful species and is not meant to be limiting as there are other ways in which sequence variants of peptides and the DNA sequences encoding them may be obtained. For example, recombinant vectors encoding the desired peptide sequence may be treated with mutagenic agents, such as hydroxylamine, to obtain sequence variants. Specific details regarding these methods and protocols are found in the teachings of Maloy *et al*., 1994; Segal, 1976; Prokop and Bajpai, 1991; Kuby, 1994; and Maniatis *et al*, 1982, each incorporated herein by reference, for that purpose.

As used herein, the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" is intended to refer to a process that involves the template-dependent extension of a primer molecule. Ihe term template dependent process refers to nucleic acid synthesis of an RNA or a DNA molecule wherein the sequence of the newly synthesized strand of nucleic acid is dictated by the well-known rules of complementary base pairing (see, for example, Watson, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by U. S.. Patent No. 4,237,224, specifically incorporated herein by reference in its entirety..

In another approach for the production of polypeptide variants of the present invention, recursive sequence recombination, as described in U..S. Patent No. 5,837,458, may be employed. In this approach, iterative cycles of recombination and screening or selection are performed to "evolve" individual polynucleotide variants of the invention having, for example, enhanced immunogenic activity.

In other embodiments of the present invention, the polynucleotide sequences provided herein can be advantageously used as probes or primers for nucleic acid Hybridization. As such, it is contemplated that nucleic acid segments that comprise a sequence region of at least about 15 nucleotide long contiguous sequence that has the same sequence as, or is complementary to, a 15 nucleotide long contiguous sequence disclosed herein will find particular utility. Longer contiguous identical or complementary sequences, e..g., those of about 20, 30, 40, 50, 100, 200, 500, 1000 (including all intermediate lengths) and even up to full length sequences will also be of use in certain embodiments.

The ability of such nucleic acid probes to specifically hybridize to a sequence of interest will enable them to be of use in detecting the presence of complementary sequences in a given sample. However, other uses are also envisioned, such as the use of the sequence information for the preparation of mutant species primers, or primers for use in preparing other genetic constructions..

Polynucleotide molecules having sequence regions consisting of contiguous nucleotide stretches of 10-14, 15-20, 30, 50, or even of 100-200 nucleotides or so (including intermediate lengths as well), identical or complementary to a polynucleotide sequence disclosed herein, are particularly contemplated as hybridization probes for use in, *e*.*g*., Southern and Northern blotting. This would allow a gene product, or fragment thereof, to be analyzed, both in diverse cell types and also in various bacterial cells. The total size of fragment, as well as the size of the complementary stretch(es), will ultimately depend on the intended use or application of the particular nucleic acid segment. Smaller fragments will generally find use in hybridization embodiments, wherein the length of the contiguous complementary region may be varied, such as between about 15 and about 100 nucleotides, but larger contiguous complementarity stretches may be used, according to the length complementary sequences one wishes to detect..

The use of a hybridization probe of about 15-25 nucleotides in length allows the formation of a duplex molecule that is both stable and selective. Molecules having contiguous complementary sequences over stretches greater than 15 bases in length are generally preferred, though, in order to increase stability and selectivity of the hybrid, and thereby improve the quality and degree of specific hybrid molecules obtained. One will generally prefer to design nucleic acid molecules having gene-complementary stretches of 15 to 25 contiguous nucleotides, or even longer where desired.

Hybridization probes may be selected from any portion of any of the sequences disclosed herein. All that is required is to review the sequences set forth herein, or to any continuous portion of the sequences, from about 15-25 nucleotides in length up to and including the full length sequence, that one wishes to utilize as a probe or primer. The choice of probe and primer sequences may be governed by various factors. For example, one may wish to employ primers from towards the termini of the total sequence.

Small polynucleotide segments or fragments may be readily prepared by, for example, directly synthesizing the fragment by chemical means, as is commonly practiced using an automated oligonucleotide synthesizer. Also, fragments may be obtained by application of nucleic acid reproduction technology, such as the PCR™ technology of U. S. Patent 4,683,202 (incorporated herein by reference), by introducing selected sequences into recombinant vectors for recombinant production, and by other recombinant DNA techniques generally known to those of skill in the art of molecular biology.

The nucleotide sequences of the invention may be used for their ability to selectively form duplex molecules with complementary stretches of the entire gene or gene fragments of interest. Depending on the application envisioned, one will typically desire to employ varying conditions of hybridization to achieve varying degrees of selectivity of probe towards target sequence. For applications requiring high selectivity, one will typically desire to employ relatively stringent conditions to form the hybrids, *e*.*g*., one will select relatively low salt and/or high temperature conditions, such as provided by a salt concentration of from about 0.02 M to about 0.15 M salt at temperatures of from about 50°C to about 70°C. Such selective conditions tolerate little, if any, mismatch between the probe and the template or target strand, and would be particularly suitable for isolating related sequences.

Of course, for some applications, for example, where one desires to prepare mutants employing a mutant primer strand hybridized to an underlying template, less stringent (reduced stringency) hybridization conditions will typically be needed in order to allow formation of the heteroduplex. In these circumstances, one may desire to employ salt conditions such as those of from about 0.15 M to about 0.9 M salt, at temperatures ranging from about 20°C to about 55°C. Cross-hybridizing species can thereby be readily identified as positively hybridizing signals with respect to control hybridizations. In any case, it is generally appreciated that conditions can be rendered more stringent by the addition of increasing amounts of formamide, which serves to destabilize the hybrid duplex in the same manner as increased temperature. Thus, hybridization conditions can be readily manipulated, and thus will generally be a method of choice depending on the desired results.

According to another embodiment of the present invention, polynucleotide compositions comprising antisense oligonucleotides are provided. Antisense oligonucleotides have been demonstrated to be effective and targeted inhibitors of protein synthesis, and, consequently, provide a therapeutic approach by which a disease can be treated by inhibiting the synthesis of proteins that contribute to the disease. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal GABA_{A} receptor and human EGF (Jaskulski et al., Science. 1988 Jun 10;240(4858):1544-6; Vasanthakumar and Ahmed, Cancer Commun. 1989;1(4):225-32; Peris et al., Brain Res Mol Brain Res. 1998 Jun 15;57(2):310-20; U. S. Patent 5,801,154; U.S. Patent 5,789,573; U. S. Patent 5,718,709 and U.S. Patent 5,610,288).. Antisense constructs have also been described that inhibit and can be used to treat a variety of abnormal cellular proliferations, *e*.*g*. cancer (U. S. Patent 5,747,470; U. S. Patent 5,591,317 and U. S. Patent 5,783,683).

Therefore, in certain embodiments, the present invention provides oligonucleotide sequences that comprise all, or a portion of, any sequence that is capable of specifically binding to polynucleotide sequence described herein, or a complement thereof. In one embodiment, the antisense oligonucleotides comprise DNA or derivatives thereof. In another embodiment, the oligonucleotides comprise RNA or derivatives thereof. In a third embodiment, the oligonucleotides are modified DNAs comprising a phosphorothioated modified backbone. In a fourth embodiment, the oligonucleotide sequences comprise peptide nucleic acids or derivatives thereof. In each case, referred compositions comprise a sequence region that is complementary, and more preferably substantially-complementary, and even more preferably, completely complementary to one or more portions of polynucleotides disclosed herein. Selection of antisense compositions specific for a given gene sequence is based upon analysis of the chosen target sequence and determination of secondary structure, Tₘ, binding energy, and relative stability. Antisense compositions may be selected based upon their relative inability to form dimers, hairpins, or other secondary structures that would reduce or prohibit specific binding to the target mRNA in a host cell. Highly preferred target regions of the mRNA, are those which are at or near the AUG translation initiation codon, and those sequences which are substantially complementary to 5' regions of the mRNA. These secondary structure analyses and target site selection considerations can be performed, for example, using v.4 of the OLIGO primer analysis software and/or the BLASTN 20.5 algorithm software (Altschul et al., Nucleic Acids Res.. 1997,25(17):3389-402)

The use of an antisense delivery method employing a short peptide vector, termed MPG (27 residues), is also contemplated. The MPG peptide contains a hydrophobic domain derived from the fusion sequence of HIV gp41 and a hydrophilic domain from the nuclear localization sequence of SV40 T-antigen (Morris et al., Nucleic Acids Res.. 1997 Jul 15;25(14):2730-6). It has been demonstrated that several molecules of the MPG peptide coat the antisense oligonucleotides and can be delivered into cultured mammalian cells in less than 1 hour with relatively high efficiency (90%). Further, the interaction with MPG strongly increases both the stability of the oligonucleotide to nuclease and the ability to cross the plasma membrane.

According to another embodiment of the invention, the polynucleotide compositions described herein are used in the design and preparation of ribozyme molecules for inhibiting expression of the tumor polypeptides and proteins of the present invention in tumor cells. Ribozymes are RNA-protein complexes that cleave nucleic acids in a site-specific fashion Ribozymes have specific catalytic domains that possess endonuclease activity (Kim and Cech, Proc Natl Acad Sci U S A. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech et al., Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction..

Six basic varieties of naturally-occurring enzymatic RNAs are know presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nature of a ribozyme is advantageous over many technologies, such as antisense technology (where a nucleic acid molecule simply binds to a nucleic acid target to block its translation) since the concentration of ribozyme necessary to affect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding to the target RNA, but also on the mechanism of target RNA cleavage. Single mismatches, or base-substitutions, near the site of cleavage can completely eliminate catalytic activity of a ribozyme. Similar mismatches in antisense molecules do not prevent their action (Woolf et al., Proc Natl Acad Sci U S A. 1992 Aug 15;89(16):7305-9). Thus, the specificity of action of a ribozyme is greater than that of an antisense oligonucleotide binding the same RNA site.

The enzymatic nucleic acid molecule may be formed in a hammerhead, hairpin, a hepatitis δ virus, group I intron or RNaseP RNA (in association with an RNA guide sequence) or Neurospora VS RNA motif. Examples of hammerhead motifs are described by Rossi et al. Nucleic Acids Res. 1992 Sep 11;20(17):4559-65 Examples of hairpin motifs are described by Hampel *et al*. (Eur. Pat. Appl. Publ. No- EP 0360257), Hampel and Tritz, Biochemistry 1989 Jun 13;28(12):4929-33; Hampel et al., Nucleic Acids Res. 1990 Jan 25;18(2):299-304 and U. S. Patent 5,631,359. An example of the hepatitis δ virus motif is described by Perrotta and Been, Biochemistry. 1992 Dec 1;31(47):11843-52; an example of the RNaseP motif is described by Guerrier-Takada et al., Cell.. 1983 Dec;.35(3 Pt 2):849-57; Neurospora VS RNA ribozyme motif is described by Collins (Saville and Collins, Cell. 1990 May 18;61(4):685-96; Saville and Collins, Proc Natl Acad Sci U S A. 1991 Oct 1;88(19):8826-30; Collins and Olive, Biochemistry. 1993 Mar 23;32(11):2795-9); and an example of the Group I intron is described in (U. S. Patent 4,987,071). All that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule. Thus the ribozyme constructs need not be limited to specific motifs mentioned herein.

Ribozymes may be designed as described in Int. Pat. Appl. Publ. No.. WO 93/23569 and Int. Pat. Appl. Publ. No. WO 94/02595, each specifically incorporated herein by reference) and synthesized to be tested *in vitro* and *in vivo*, as described. Such ribozymes can also be optimized for delivery. While specific examples are provided, those in the art will recognize that equivalent RNA targets in other species can be utilized when necessary.

Ribozyme activity can be optimized by altering the length of the ribozyme binding arms, or chemically synthesizing ribozymes with modifications that prevent their degradation by serum ribonucleases (see *e*.*g*., Int. Pat. Appl. Publ. No. WO 92/07065; Int. Pat.. Appl.. Publ. No. WO 93/15187; Int. Pat. Appl. Publ. No. WO 91/03162; Eur. Pat. Appl.. Publ. No.. 92110298.4; U. S. Patent 5,334,711; and Int. Pat. Appl. Publ. No.. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

Sullivan *et al.* (Int. Pat. Appl. Publ. No. WO 94/02595) describes the general methods for delivery of enzymatic RNA molecules.. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination may be locally delivered by direct inhalation, by direct injection or by use of a catheter, infusion pump or stint. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Int. Pat. Appl. Publ. No.. WO 94/02595 and Int.. Pat.. Appl.. Publ. No. WO 93/23569, each specifically incorporated herein by reference.

Another means of accumulating high concentrations of a ribozyme(s) within cells is to incorporate the ribozyme-encoding sequences into a DNA expression vector. Transcription of the ribozyme sequences are driven from a promoter for eukaryotic RNA polymerase I (pol I), RNA polymerase II (pol II), or RNA polymerase III (pol III) Transcripts from pol II or pol III promoters will be expressed at high levels in all cells; the levels of a given pol II promoter in a given cell type will depend on the nature of the gene regulatory sequences (enhancers, silencers, *etc*.) present nearby. Prokaryotic RNA polymerase promoters may also be used, providing that the prokaryotic RNA polymerase enzyme is expressed in the appropriate cells. Ribozymes expressed from such promoters have been shown to function in mammalian cells. Such transcription units can be incorporated into a variety of vectors for introduction into mammalian cells, including but not restricted to, plasmid DNA vectors, viral DNA vectors (such as adenovirus or adeno-associated vectors), or viral RNA vectors (such as retroviral, semliki forest virus, sindbis virus vectors).

In another embodiment of the invention, peptide nucleic acids (PNAs) compositions are provided. PNA is a DNA mimic in which the nucleobases are attached to a pseudopeptide backbone (Good and Nielsen, Antisense Nucleic Acid Drug Dev. 1997 7(4) 431-37). PNA is able to be utilized in a number methods that traditionally have used RNA or DNA Often PNA sequences perform better in techniques than the corresponding RNA or DNA sequences and have utilities that are not inherent to RNA or DNA A review of PNA including methods of making, characteristics of, and methods of using, is provided by Corey (Trends Biotechnol 1997 Jun;15(6):224-9). As such, in certain embodiments, one may prepare PNA sequences that are complementary to one or more portions of the ACE mRNA sequence, and such PNA compositions may be used to regulate, alter, decrease, or reduce the translation of ACE-specific mRNA, and thereby alter the level of ACE activity in a host cell to which such PNA compositions have been administered..

PNAs have 2-aminoethyl-glycine linkages replacing the normal phosphodiester backbone of DNA (Nielsen et al., Science 1991 Dec 6;254(5037):1497-500; Hanvey et al., Science.. 1992 Nov 27;258(5087):1481-5; Hyrup and Nielsen, Bioorg Med Chem. 1996 Jan;4(1):5-23). This chemistry has three important consequences: firstly, in contrast to DNA or phosphorothioate oligonucleotides, PNAs are neutral molecules; secondly, PNAs are achiral, which avoids the need to develop a stereoselective synthesis; and thirdly, PNA synthesis uses standard Boc or Fmoc protocols for solid-phase peptide synthesis, although other methods, including a modified Merrifield method, have been used.

PNA monomers or mady-made oligomers are commercially available from PerSeptive Biosystems (Framingham, MA). PNA syntheses by either Boc or Fmoc protocols are straightforward using manual or automated protocols (Norton et al., Bioorg Med Chem.. 1995 Apr;3(4):437-45). The manual protocol lends itself to the production of chemically modified PNAs or the simultaneous synthesis of families of closely related PNAs.

As with peptide synthesis, the success of a particular PNA synthesis will depend on the properties of the chosen sequence. For example, while in theory PNAs can incorporate any combination of nucleotide bases, the presence of adjacent purines can lead to deletions of one or more residues in the product. In expectation of this difficulty, it is suggested that, in producing PNAs with adjacent purines, one should repeat the coupling of residues likely to be added inefficiently. This should be followed by the purification of PNAs by reveres phase high-pressure liquid chromatography, providing yields and purity of product similar to those observed during the synthesis of peptides.

Modifications of PNAs for a given application may be accomplished by coupling amino acids during solid-phase synthesis or by attaching compounds that contain a carboxylic acid group to the exposed N-terminal amine. Alternatively, PNAs can be modified after synthesis by coupling to an introduced lysine or cysteine . The ease with which PNAs can be modified facilitates optimization for better solubility or for specific functional requirements. Once synthesized, the identity of PNAs and their derivatives can be confirmed by mass spectrometry. Several studies have made and utilized modifications of PNAs (for example, Norton et al., Bioorg Med Chem. 1995 Apr;3(4):437-45; Petersen et al., J Pept Sci.. 1995 May-Jun;1(3):175-83; Orum et al., Biotechniques.. 1995 Sep;19(3):472-80; Footer et al., Biochemistry. 1996 Aug 20;35(33):10673-9; Griffith et al., Nucleic Acids Res. 1995 Aug 11;23(15):3003-8; Pardridge et al., Proc Natl Acad Sci U S A. 1995 Jun 6;92(12):5592-6; Boffa et al., Proc Natl Acad Sci U S A. 1995 Mar 14;92(6):1901-5; Gambacorti-Passerini et al, Blood. 1996 Aug 15;88(4):1411-7; Arrnitage et al., Proc Natl Acad Sci U S A. 1997 Nov 11;94(23):12320-5; Seeger et al., Biotechniques. 1997 Sep;23(3):512-7). U.S. Patent No. 5,700,922 discusses PNA-DNA-PNA chimeric molecules and their uses in diagnostics, modulating protein in organisms, and treatment of conditions susceptible to therapeutics.

Methods of characterizing the antisense binding properties of PNAs are discussed in Rose (Anal Chem. 1993 Dec 15;65(24):3545-9) and Jensen et al. (Biochemistry. 1997 Apr 22;36(16):5072-7). Rose uses capillary gel electrophoresis to determine binding of PNAs to their complementary oligonucleotide, measuring the relative binding kinetics and stoichiometry.. Similar types of measurements were made by Jensen *et al*. using BIAcore™ technology.

Other applications of PNAs that have been described and will be apparent to the skilled artisan include use in DNA strand invasion, antisense inhibition, mutational analysis, enhancers of transcription, nucleic acid purification, isolation of transcriptionally active genes, blocking of transcription factor binding, genome cleavage, biosensors, *in situ* hybridization, and the like.

### Polynucleotide Identification, Characterization and Expression

Polynucleotides compositions of the present invention may be identified, prepared and/or manipulated using any of a variety of well established techniques (see generally, Sambrook et al., *Molecular Cloning: A Laboratory Manual*, Cold Spring Harbor Laboratories, Cold Spring Harbor, NY, 1989, and other like references). For example, a polynucleotide may be identified, as described in more detail below, by screening a microarray of cDNAs for tumor-associated expression (*i*.*e*., expression that is at least two fold greater in a tumor than in normal tissue, as determined using a representative assay provided herein). Such screens may be performed, for example, using the microarray technology of Affymetrix, Inc. (Santa Clara, CA) according to the manufacturer's instructions (and essentially as described by Schena et al., Proc. Natl. Acad. Sci. USA 93:10614-10619, 1996 and Heller et al., Proc. Natl. Acad. Sci. USA 94:2150-2155, 1997). Alternatively, polynucleotides may be amplified from cDNA prepared from cells expressing the proteins described herein, such as tumor cells.

Many template dependent processes are available to amplify a target sequences of interest present in a sample. One of the best known amplification methods is the polymerase chain reaction (PCR™) which is described in detail in U.S. Patent Nos. 4,683,195, 4,683,202 and 4,800,159, each of which is incorporated herein by reference in its entirety. Briefly, in PCR™, two primer sequences are prepared which are complementary to regions on opposite complementary strands of the target sequence.. An excess of deoxynucleoside triphosphates is added to a reaction mixture along with a DNA polymerase (*e.g., Taq* polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction product and the process is repeated. Preferably reverse transcription and PCR™ amplification procedure may be performed in order to quantify the amount of mRNA amplified. Polymerase chain reaction methodologies are well known in the art.

Any of a number of other template dependent processes, many of which are variations of the PCR ™ amplification technique, are readily known and available in the art. Illustratively, some such methods include the ligase chain reaction (referred to as LCR), described, for example, in Eur.. Pat. Appl. Publ. No. 320,308 and U.S. Patent No. 4,883,750; Qbeta Replicase, described in PCT Intl. Pat. Appl. Publ. No. PCT/US87/00880; Strand Displacement Amplification (SDA) and Repair Chain Reaction (RCR). Still other amplification methods are described in Great Britain Pat.. Appl. No. 2 202 328, and in PCT Intl. Pat. Appl. Publ. No.. PCT/US89/01025. Other nucleic acid amplification procedures include transcription-based amplification systems (TAS) (PCT Intl. Pat. Appl. Publ. No.. WO 88/10315), including nucleic acid sequence based amplification (NASBA) and 3SR. Eur. Pat. Appl. Publ. No. 329,822 describes a nucleic acid amplification process involving cyclically synthesizing single-stranded RNA ("ssRNA"), ssDNA, and double-stranded DNA (dsDNA). PCT Intl, Pat. Appl.. Publ. No.. WO 89/06700 describes a nucleic acid sequence amplification scheme based on the hybridization of a promoter/primer sequence to a target single-stranded DNA ("ssDNA") followed by transcription of many RNA copies of the sequence. Other amplification methods such as "RACE" (Frohman, 1990), and "one-sided PCR" (Ohara, 1989) are also well-known to those of skill in the art.

An amplified portion of a polynucleotide of the present invention may be used to isolate a full length gene from a suitable library (*e*.*g*., a tumor cDNA library) using well known techniques. Within such techniques, a library (cDNA or genomic) is screened using one or more polynucleotide probes or primers suitable for amplification.. Preferably, a library is size-selected to include larger molecules Random primed libraries may also be preferred for identifying 5' and upstream regions of genes. Genomic libraries are preferred for obtaining introns and extending 5' sequences.

For hybridization techniques, a partial sequence may be labeled (*e*.*g*., by nick-translation or end-labeling with ³²P) using well known techniques. A bacterial or bacteriophage library is then generally screened by hybridizing filters containing denatured bacterial colonies (or lawns containing phage plaques) with the labeled probe (*see* Sambrook et al., Molecular Cloning.: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones The resulting overlapping sequences can then assembled into a single contiguous sequence. A full length cDNA molecule can be generated by ligating suitable fragments, using well known techniques.

Alternatively, amplification techniques, such as those described above, can be useful for obtaining a full length coding sequence from a partial cDNA sequence. One such amplification technique is inverse PCR (*see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region A variation on this procedure, which employs two primers that initiate extension in opposite directions from the known sequence, is described in WO 96/38591. Another such technique is known as "rapid amplification of cDNA ends" or RACE. This technique involves the use of an internal primer and an eternal primer, which hybridizes to a polyA region or vector sequence, to identify sequences that are 5' and 3' of a known sequence. Additional techniques include capture PCR (Lagerstrom et al., PCR Methods Applic. 1:111-19, 1991) and walking PCR (Parker et al.., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be peifoimed using well known programs (*e*.*g*., NCBI BLAST searches), and such ESIs may be used to generate a contiguous full length sequence. Full length DNA sequences may also be obtained by analysis of genomic fragments.

In other embodiments of the invention, polynucleotide sequences or fiagments thereof which encode polypeptides of the invention, or fusion proteins or functional equivalents thereof, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide.

As will be understood by those of skill in the art, it may be advantageous in some instances to produce polypeptide-encoding nucleotide sequences possessing non-natuially occurring codons. For example, codons preferred by a particular prokaryotic or eukaryotic host can be selected to increase the rate of protein expression or to produce a recombinant RNA transcript having desirable properties, such as a half-life which is longer than that of a transcript generated from the naturally occurring sequence.

Moreover, the polynucleotide sequences of the present invention can be engineered using methods generally known in the art in order to alter polypeptide encoding sequences for a variety of reasons, including but not limited to, alterations which modify the cloning, processing, and/or expression of the gene product. For example, DNA shuffling by random fragmentation and PCR reassembly of gene fragments and synthetic oligonucleotides may be used to engineer the nucleotide sequences. In addition, site-directed mutagenesis may be used to insert new restriction sites, alter glycosylation patterns, change codon preference, produce splice variants, or introduce mutations, and so forth.

In another embodiment of the invention, natural, modified, or recombinant nucleic acid sequences may be ligated to a heterologous sequence to encode a fusion protein. For example, to screen peptide libraries for inhibitors of polypeptide activity, it may be useful to encode a chimeric protein that can be recognized by a commercially available antibody. A fusion protein may also be engineered to contain a cleavage site located between the polypeptide-encoding sequence and the heterologous protein sequence, so that the polypeptide may be cleaved and purified away from the heterologous moiety.

Sequences encoding a desired polypeptide may be synthesized, in whole or in part, using chemical methods well known in the art (see Caruthets, M. H. et al. (1980) Nucl. Acids Res. Symp. Ser. 215-223, Horn, T. et al. (1980) Nucl. Acids Res. Symp. Ser. 225-232).. Alternatively, the protein itself may be produced using chemical methods to synthesize the amino acid sequence of a polypeptide, or a portion thereof. For example, peptide synthesis can be performed using various solid-phase techniques (Roberge, J. Y. et al. (1995) Science 269:202-204) and automated synthesis may be achieved, for example, using the ABI 431A Peptide Synthesizer (Perkin Elmer, Palo Alto, CA).

A newly synthesized peptide may be substantially purified by preparative high performance liquid chromatography (*e*.*g*., Creighton, T. (1983) Proteins, Structure and Molecular Principles, WH Freeman and Co., New York, N.Y.) or other comparable techniques available in the art. The composition of the synthetic peptides may be confirmed by amino acid analysis or sequencing (*e*.*g*., the Edman degradation procedure). Additionally, the amino acid sequence of a polypeptide, or any part thereof, may be altered during direct synthesis and/or combined using chemical methods with sequences from other proteins, or any part thereof, to produce a variant polypeptide.

In order to express a desired polypeptide, the nucleotide sequences encoding the polypeptide, or functional equivalents, may be inserted into appropriate expression vector, *i*.*e*., a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination Such techniques are described, for example, in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York. N.Y.

A variety of expression vector/host systems may be utilized to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (*e*.*g*., baculovirus); plant cell systems transformed with virus expression vectors (*e*.*g*., cauliflower mosaic virus, CaMV; tobacco mosaic virus, TMV) or with bacterial expression vectors (*e*.*g*., Ii or pBR322 plasmids); or animal cell systems.

The "control elements" or "regulatory sequences" present in an expression vector are those non-translated regions of the vector-enhancers, promoters, 5' and 3' untranslated regions--which interact with host cellular proteins to carry out transcription and translation. Such elements may vary in then strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the PBLUESCRIPT phagemid (Stratagene, La Jolla, Calif.) or PSPORT1 plasmid (Gibco BRL, Gaithersburg, MD) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

In bacterial systems, any of a number of expression vectors may be selected depending upon the use intended for the expressed polypeptide. For example, when large quantities are needed, for example for the induction of antibodies, vectors which direct high level expression of fusion proteins that are readily purified may be used.. Such vectors include, but are not limited to, the multifunctional *E*. *coli* cloning and expression vectors such as BLUESCRIPT (Stratagene), in which the sequence encoding the polypeptide of interest may be ligated into the vector in frame with sequences for the ammo-terminal Met and the subsequent 7 residues of beta-galactosidase so that a hybrid protein is produced; pIN vectors (Van Heeke, G. and S. M. Schuster (1989) J. Biol. Chem. 264:5503-5509); and the like.. pGEX Vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-aganose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, Saccharomyces cerevisiae, a number of vectors containing constitutive or inducible promoters such as alpha factor, alcohol oxidase, and PGH may be used.. For reviews, see Ausubel et al. (supra) and Grant et al. (1987) Methods Enzymol. 153:516-544.,

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, N. (1987) EMEO J. 6:307·311. Alternatively, plant promoters such as the small subunit of RUBISCO or heat shock promoters may be used (Coruzzi, G. et al (1984) EMBO J 3:1671-1680; Broglie, R. et al. (1984) Science 224:838-843; and Winter, J. et al.. (1991) Results Probl. Cell Differ. 17:85-105)., These constructs can be introduced into plant cells by direct DNA transformation or pathogen-mediated transfection. Such techniques are described in a number of generally available reviews (see, for example, Hobbs, S. or Murry, L. E. in McGraw Hill Yearbook of Science and Technology (1992) McGraw Hill, New York, N..Y..; pp.. 191-196).

An insect system may also be used to express a polypeptide of interest. For example, in one such system, Autographa californica nuclear polyhedrosis virus (AcNPV) is used as a vector to express foreign genes in Spodoptera frugiperda cells or in Trichoplusia larvae The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, S frugiperda cells or Irichoplusia larvae in which the polypeptide of interest may be expressed (Engelhard, E. K. et al. (1994) Proc. Natl. Acad. Sci. 91 :3224.3227)..

In mammalian host cells, a number of viral-based expression systems are generally available.. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the vital genome may be used to obtain a viable virus which is capable of expressing the polypeptide in infected host cells (Logan, J. and Shenk, T. (1984) Proc. Natl. Acad. Sci. 81:3655-3659). In addition, transcription enhances, such as the Rous sarcoma virus (RSV) enhancer, may be used to increase expression in mammalian host cells.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading fiame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) Results Probl. Cell Differ. 20:125-162).

In addition, a host cell strain may be chosen for its ability to modulate the expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to facilitate correct insertion, folding and/or function. Different host cells such as CHO; COS, HeLa, MDCK, HEK293, and WI38, which have specific cellular machinery and characteristic mechanisms for such post-translational activities, may be chosen to ensure the correct modification and processing of the foreign protein.

For long-term, high-yield production of recombinant proteins, stable expression is generally preferred. For example, cell lines which stably express a polynucleotide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase (Wigler, M. et all. (1977) Cell 11:223-32) and adenine phosphoribosyltransferase (Lowy, I. et al. (1990) Cell 22:817-23) genes which can be employed in tk.sup- or apit.sup.- cells, respectively. Also, antimetabolite, antibiotic or herbicide resistance can be used as the basis for selection; for example, dhfi which confers resistance to methotrexate (Wigler, M. et al.. (1980) Proc. Natl. Acad. Sci. 77:3567-70); npt, which confers resistance to the aminoglycosides, neomycin and G-418 (Colbere-Garapin, F. et al (1981) J. Mol Biol. 150:1-14); and als or pat, which confer resistance to chlorsulfuion and phosphinotricin acetyltransferase, respectively (Murry, *supra*). Additional selectable genes have been described, for example, trpB, which allows cells to utilize indole in place of tryptophan, or hisD, which allows cells to utilize histinol in place of histidine (Hartman, S. C. and R. C. Mulligan (1988) Proc Natl. Acad. Sci. 85:8047-51). The use of visible markers has gained popularity with such markers as anthocyanin.s, beta-glucuronidase and its substrate GUS, and luciferase and its substrate lucifeiin, being widely used riot only to identify transformants, but also to quantify the amount of transient or stable protein expression attributable to a specific vector system (Rhodes, C. A. et al. (1995) Methods Mol. Biol. 55:121-131).

Although the presence/absence of marker gene expression suggests that the gene of interest is also present, its presence and expression may need to be confirmed. For example, if the sequence encoding a polypeptide is inserted within a marker gene sequence, recombinant cells containing sequences can be identified by the absence of marker gene function. Alternatively, a marker gene can be placed in tandem with a polypeptide-encoding sequence under the control of a single promoter. Expression of the marker gene in response to induction or selection usually indicates expression of the tandem gene as well..

Alternatively, host cells that contain and express a desired polynucleotide sequence may be identified by a variety of procedures known to those of skill in the art. These procedures include, but are not limited to, DNA-DNA or DNA. RNA hybridizations and protein bioassay or immunoassay techniques which include, for example, membrane, solution, or chip based technologies for the detection and/or quantification of nucleic acid or protein.

A variety of protocols for detecting and measuring the expression of polynucleotide-encoded products, using either polyclonal or monoclonal antibodies specific for the product are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on a given polypeptide may be preferred for some applications, but a competitive binding assay may also be employed. These and other assays are described, among other places, in Hampton, R. et al. (1990; Serological Methods, a Laboratory Manual, APS Press, St Paul. Minn.) and Maddox, D., E. et al. (1983; J. Exp. Med. 158:1211-1216).

A wide variety of labels and conjugation techniques are known by those skilled in the art and may be used in various nucleic acid and amino acid assays. Means for producing labeled hybridization or PCR probes for detecting sequences related to polynucleotides include oligolabeling, nick translation, end-labeling or PCR amplification using a labeled nucleotide. Alternatively, the sequences, or any portions thereof may be cloned into a vector for the production of an mRNA probe. Such vectors are known in the art, are commercially available, and may be used to synthesize RNA probes in vitro by addition of an appropriate RNA polymerase such as T7, T 3, or SP6 and labeled nucleotides. These procedure may be conducted using a variety of commercially available kits. Suitable reporter molecules or labels, which may be used include radionuclides, enzymes, fluorescent, chemiluminescent, or chromogenic agents as well as substrates, cofactors, inhibitors, magnetic particles, and the like.

Host cells transformed with a polynucleotide sequence of interest may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides of the invention may be designed to contain signal sequences which direct secretion of the encoded polypeptide through a prokaryotic or eukaryotic cell membrane. Other recombinant constructions may be used to join sequences encoding a polypeptide of interest to nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash). The inclusion of cleavable linker sequences such as those specific for Factor XA or enterokinase (Invitrogen. San Diego, Calif.) between the purification domain and the encoded polypeptide may be used to facilitate purification One such expression vector provides for expression of a fusion protein containing a polypeptide of interest and a nucleic acid encoding 6 histidine residues preceding a thioredoxin or an enterokinase cleavage site The histidine residues facilitate purification on IMIAC (immobilized metal ion affinity chromatography) as described in Porath, J. et al. (1992, Prot. Exp. Purif. 3:263-281) while the enterokinase cleavage site provides a means for purifying the desired polypeptide from the fusion protein. A discussion of vectors which contain fusion proteins is provided in Kroll, D. J. et al. (1993; DNA Cell Biol 12:441-453).

In addition to recombinant production methods, polypeptides of the invention, and fragments thereof, may be produced by direct peptide synthesis using solid-phase techniques (Merrifield J. (1963) J. Am Chem. Sac. 85:2149.2154). Protein synthesis may be performed using manual techniques or by automation. Automated synthesis may be achieved, for example, using Applied Biosystems 431A Peptide Synthesizer (Perkin Elmer). Alternatively, various fragments may be chemically synthesized separately and combined using chemical methods to produce the full length molecule.

### Antibody Compositions, Fragments Thereof and Other Binding Agent

According to another aspect, the present invention further provides binding agents, such as antibodies and antigen-binding fragments thereof, that exhibit immunological binding to a tumor polypeptide disclosed herein, or to a portion, variant or derivative thereof. An antibody, or antigen-binding fragment thereof, is said to "specifically bind," "immunogically bind," and/or is "immunologically reactive" to a polypeptide of the invention if it reacts at a detectable level (within, for example, an ELISA assay) with the polypeptide, and does not react detectably with unrelated polypeptides under similar conditions.

Immunological binding, as used in this context, generally refers to the non-covalent interactions of the type which occur between an immunoglobulin molecule and an antigen for which the immunoglobulin is specific.. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (Kₒₙ) and the "off rate constant" (K_{off}) can be determined by calculation of the concentrations and the actual rates of association and dissociation. The ratio of K_{off} /Kₒₙ enables cancellation of all parameters not related to affinity, and is thus equal to the dissociation constant K_{d}. See, generally, Davies et al. (1990) Annual Rev. Biochem. 59:439-473.

An "antigen-binding site," or "binding portion" of an antibody refers to the part of the immunoglobulin molecule that participates in antigen binding. The antigen binding site is formed by amino acid residues of the N-teiminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". Thus the term "FR" refers to amino acid sequences which are naturally found between and adjacent to hypervariable regions in immunoglobulins. In an antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs."

Binding agents may be further capable of differentiating between patients with and without a cancer, such as lung cancer, using the representative assays provided herein. For example, antibodies or other binding agents that bind to a tumor protein will preferably generate a signal indicating the presence of a cancer in at least about 20% of patients with the disease, more preferably at least about 30% of patients. Alternatively, or in addition, the antibody will generate a negative signal indicating the absence of the disease in at least about 90% of individuals without the cancer. To determine whether a binding agent satisfies this requirement, biological samples (*e*.*g*., blood, sera, sputum, urine and/or tumor biopsies) from patients with and without a cancer (as determined using standard clinical tests) may be assayed as described herein for the presence of polypeptides that bind to the binding agent. Preferably, a statistically significant number of samples with and without the disease will be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e*.*g*., Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e*.*g*., mice, rats, rabbits, sheep or goats).. In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur J. Immunol. 6:511-519, 1976, and improvements thereto. Briefly, these methods involve the preparation of immortal cell lines capable of producing antibodies having the desired specificity (*i*.*e*., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAI (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

A number of therapeutically useful molecules are known in the art which comprise antigen-binding sites that are capable of exhibiting immunological binding properties of an antibody molecule. The proteolytic enzyme papain preferentially cleaves IgG molecules to yield several fragments, two of which (the "F(ab)" fragments) each comprise a covalent heterodimer that includes an intact antigen-binding site.. The enzyme pepsin is able to cleave IgG molecules to provide several fragments, including the "F(ab')₂ " fragment which comprises both antigen-binding sites. An "Fv" fragment can be produced by preferential proteolytic cleavage of an IgM, and on rare occasions IgG or IgA immunoglobulin molecule. Fv fragments are, however, more commonly derived using recombinant techniques known in the art. The Fv fragment includes a non-covalent V_{H}::V_{L} heterodimer including an antigen-binding site which retains much of the antigen recognition and binding capabilities of the native antibody molecule. Inbar et al. (1972) Proc. Nat. Acad. Sci.. USA 69:2659-2662; Hochman et al. (1976) Biochem 15:2706·2710; and Ehrlich et al. (1980) Biochem 19:4091-4096.

A single chain Fv ("sFv") polypeptide is a covalently linked V_{H}::V_{L} heterodimer which is expressed from a gene fusion including V_{H}- and V_{L}-encoding genes linked by a peptide-encoding linker. Huston et al. (1988) Proc. Nat. Acad. Sci. USA 85(16):5879-5883. A number of methods have been described to discern chemical structures for converting the naturally aggregated-but chemically separated light and heavy polypeptide chains from an antibody V region into an sFv molecule which will fold into a three dimensional structure substantially similar to the structure of an antigen-binding site.. See, e..g., U.S. Pat. Nos. 5,091,513 and 5,132,405, to Huston et al.; and U.S. Pat. No.. 4,946,778, to Ladner et al.

Each of the above-described molecules includes a heavy chain and a light chain CDR set, respectively interposed between a heavy chain and a light chain FR set which provide support to the CDRS and define the spatial relationship of the CDRs relative to each other.. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (*e*.*g*., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRS. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures--regardless of the precise CDR amino acid sequence. Further, certain FR residues are known to participate in non-covalent interdomain contacts which stabilize the interaction of the antibody heavy and light chains.

A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al.. (1989) Proc. Nat. Acad.. Sci.. USA 86:4220-4224; Shaw et al. (1987) J Immunol. 138:4534-4538; and Brown et al.. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients.

As used herein, the terms "veneered FRs" and "recombinantly veneered FRs" refer to the selective replacement of FR residues from, *e*.*g*., a rodent heavy or light chain V region, with human FR residues in order to provide a xenogeneic molecule comprising an antigen-binding site which retains substantially all of the native FR polypeptide folding structure. Veneering techniques are based on the understanding that the ligand binding characteristics of an antigen-binding site are determined primarily by the structure and relative disposition of the heavy and light chain CDR sets within the antigen-binding surface. Davies et al. (1990) Ann. Rev. Biochem. 59:439-473. Thus, antigen binding specificity can be preserved in a humanized antibody only wherein the CDR structures, their interaction with each other, and their interaction with the rest of the V region domains are carefully maintained. By using veneering techniques, exterior (*e*.*g*., solvent-accessible) FR residues which are readily encountered by the immune system are selectively replaced with human residues to provide a hybrid molecule that comprises either a weakly immunogenic, or substantially non-immunogenic veneered surface.

The process of veneering makes use of the available sequence data for human antibody variable domains compiled by Kabat et al., in Sequences of Proteins of Immunological Interest, 4th ed., (U.S. Dept. of Health and Human Services, U.S. Government Printing Office, 1987), updates to the Kabat database, and other accessible U..S.. and foreign databases (both nucleic acid and protein). Solvent accessibilities of V region amino acids can be deduced from the known three-dimensional structure for human and murine antibody fragments. There are two general steps in veneering a murine antigen-binding site. Initially, the FRs of the variable domains of an antibody molecule of interest are compared with corresponding FR sequences of human variable domains obtained from the above-identified sources. The most homologous human V regions are then compared residue by residue to corresponding murine amino acids. The residues in the murine FR which differ from the human counterpart are replaced by the residues present in the human moiety using recombinant techniques well known in the art. Residue switching is only carried out with moieties which are at least partially exposed (solvent accessible), and care is exercised in the replacement of amino acid residues which may have a significant effect on the tertiary structure of V region domains, such as proline, glycine and charged amino acids.

In this manner, the resultant "veneered" murine antigen-binding sites are thus designed to retain the murine CDR residues, the residues substantially adjacent to the CDRs, the residues identified as buried or mostly buried (solvent inaccessible), the residues believed to participate in non-covalent (*e*.*g*., electrostatic and hydrophobic) contacts between heavy and light chain domains, and the residues from conserved structural regions of the FRs which are believed to influence the "canonical" tertiary structures of the CDR loops. These design criteria are then used to prepare recombinant nucleotide sequences which combine the CDRs of both the heavy and light chain of a murine antigen-binding site into human appearing FRs that can be used to transfect mammalian cells for the expression of recombinant human antibodies which exhibit the antigen specificity of the murine antibody molecule.

In another embodiment of the invention, monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e*.*g*., covalently bonded) to a suitable monoclonal antibody either directly or indirectly (*e*.*g*., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e*.*g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e*.*g*., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e*.*g*., U.S. Patent No. 4,489,710, to Spitler), by irradiation of a photolabile bond (*e*.*g*., U.S. Patent No. 4,625,014, to Senter et al.), by hydrolysis of derivatized amino acid side chains (*e*.*g*., U..S. Patent No.. 4,638,045, to Kohn et al..), by serum complement-mediated hydrolysis (*e*.*g*., U.S. Patent No.. 4,671,958, to Rodwell et al.), and acid-catalyzed hydrolysis (*e*.*g*., U.S. Patent No.. 4,569,789, to Blattler et al.).

It may be desirable to couple more than one agent to an antibody. In one embodiment, multiple molecules of an agent are coupled to one antibody molecule In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers that provide multiple sites for attachment can be used. Alternatively, a carrier can be used.

A carrier may bear the agents in a variety of ways, including covalent bonding either directly or via a linker group. Suitable carriers include proteins such as albumins (*e*.*g*., U.S. Patent No. 4,507,234, to Kato et al.), peptides and polysaccharides such as aminodextran (*e*.*g*., U.S. Patent No. 4,699,784, to Shih et al..).. A carrier may also bear an agent by noncovalent bonding or by encapsulation, such as within a liposome vesicle (*e*.*g*., U.S.. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No.. 4,673,562, to Davison et al. discloses representative chelating compounds and their synthesis.

### T Cell Compositions

The present invention, in another aspect, provides T cells specific for a tumor polypeptide disclosed herein, or for a variant or derivative thereof. Such cells may generally be prepared *in vitro* or *ex vivo*, using standard procedures. For example, T cells may be isolated from bone marrow, peripheral blood, or a fraction of bone marrow or peripheral blood of a patient, using a commercially available cell separation system, such as the Isolex™ System, available from Nexell Therapeutics, Inc. (Irvine, CA; see also U.S. Patent No. 5,240,856; U.S Patent No. 5,215,926; WO 89/06280; WO 91/16116 and WO 92/07243). Alternatively, T cells may be derived from related or unrelated humans, non-human mammals, cell lines or cultures.

T cells may be stimulated with a polypeptide, polynucleotide encoding a polypeptide and/or an antigen presenting cell (APC) that expresses such a polypeptide. Such stimulation is performed under conditions and for a time sufficient to permit the generation of T cells that are specific for the polypeptide of interest. Preferably, a tumor polypeptide or polynucleotide of the invention is present within a delivery vehicle, such as a microsphere, to facilitate the generation of specific T cells.

T cells are considered to be specific for a polypeptide of the present invention if the T cells specifically proliferate, secrete cytokines or kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res 54:1065-1070, 1994. Alternatively, detection of the proliferation of T cells may be accomplished by a variety of known techniques.. For example, T cell proliferation can be detected by measuring an increased rate of DNA synthesis (*e*.*g*., by pulse-labeling cultures of T cells with tritiated thymidine and measuring the amount of tritiated thymidine incorporated into DNA) Contact with a tumor polypeptide (100 ng/ml - 100 µg/ml, preferably 200 ng/ml - 25 µg/ml) for 3 - 7 days will typically result in at least a two fold increase in proliferation of the T cells. Contact as described above for 2-3 hours should result in activation of the T cells, as measured using standard cytokine assays in which a two fold increase in the level of cytokine release (*e*.*g*., TNF or IFN-γ) is indicative of T cell activation (*see* Coligan et al., Current Protocols in Immunology, vol. 1, Wiley Interscience (Greene 1998)). T cells that have been activated in response to a tumor polypeptide, polynucleotide or polypeptide-expressing APC may be CD4⁺ and/or CD8⁺. Tumor polypeptide-specific T cells may be expanded using standard techniques. Within preferred embodiments, the T cells are derived from a patient, a related donor or an unrelated donor, and are administered to the patient following stimulation and expansion.

For therapeutic purposes, CD4⁺ or CD8⁺ T cells that proliferate in response to a tumor polypeptide, polynucleotide or APC can be expanded in number either *in vitro* or *in vivo*. Proliferation of such T cells *in vitro* may be accomplished in a variety of ways. For example, the T cells can be re-exposed to a tumor polypeptide, or a short peptide corresponding to an immunogenic portion of such a polypeptide, with or without the addition of T cell growth factors, such as interleukin-2, and/or stimulator cells that synthesize a tumor polypeptide. Alternatively, one or more T cells that proliferate in the presence of the tumor polypeptide can be expanded in number by cloning. Methods for cloning cells are well known in the art, and include limiting dilution.

### T Cell Receptor Compositions

The T cell receptor (TCR) consists of 2 different, highly variable polypeptide chains, termed the T-cell receptor α and β chains, that are linked by a disulfide bond (Janeway, Travers, Walport. *Immunobiology*. Fourth Ed., 148-159. Elsevier Science Ltd/Garland Publishing.. 1999). The α/β heterodimer complexes with the invariant CD3 chains at the cell membrane. This complex recognizes specific antigenic peptides bound to MHC molecules. The enormous diversity of TCR specificities is generated much like immunoglobulin diversity, through somatic gene rearrangement. The β chain genes contain over 50 variable (V), 2 diversity (D), over 10 joining (J) segments, and 2 constant region segments (C). The α chain genes contain over 70 V segments, and over 60 J segments but no D segments, as well as one C segment. During T cell development in the thymus, the D to J gene rearrangement of the β chain occurs, followed by the V gene segment rearrangement to the DJ. This functional VDJ_{β} exon is transcribed and spliced to join to a C_{β}. For the α chain, a V_{α} gene segment rearranges to a J_{α} gene segment to create the functional exon that is then transcribed and spliced to the C_{α}. Diversity is further increased during the recombination process by the random addition of P and N-nucleotides between the V, D, and J segments of the β chain and between the V and J segments in the α chain (Janeway, Travers, Walport. *Immunobiology*. Fourth Ed., 98 and 150. Elsevier Science Ltd/Garland Publishing. 1999).

The present invention, in another aspect, provides TCRs specific for a polypeptide disclosed herein, or for a variant or derivative thereof. In accordance with the present invention, polynucleotide and amino acid sequences are provided for the V-J or V-D-J junctional regions or parts thereof for the alpha and beta chains of the T-cell receptor which recognize tumor polypeptides described herein. In general, this aspect of the invention relates to T-cell receptors which recognize or bind tumor polypeptides presented in the context of MHC. In a preferred embodiment the tumor antigens recognized by the T-cell receptors comprise a polypeptide of the present invention. For example, cDNA encoding a TCR specific for a tumor peptide can be isolated from I cells specific for a tumor polypeptide using standard molecular biological and recombinant DNA techniques.

This invention further includes the T-cell receptors or analogs thereof having substantially the same function or activity as the T-cell receptors of this invention which recognize or bind tumor polypeptides. Such receptors include, but are not limited to, a fragment of the receptor, or a substitution, addition or deletion mutant of a T-cell receptor provided herein. This invention also encompasses polypeptides or peptides that are substantially homologous to the T-cell receptors provided herein or that retain substantially the same activity. The term "analog" includes any protein or polypeptide having an amino acid residue sequence substantially identical to the T-cell receptors provided herein in which one or more residues, preferably no more than 5 residues, more preferably no more than 25 residues have been conservatively substituted with a functionally similar residue and which displays the functional aspects of the T-cell receptor as described herein.

The present invention further provides for suitable mammalian host cells, for example, non-specific T cells, that are transfected with a polynucleotide encoding TCRs specific for a polypeptide described herein, thereby rendering the host cell specific for the polypeptide. The α and β chains of the TCR may be contained on separate expression vectors or alternatively, on a single expression vector that also contains an internal ribosome entry site (IRES) for cap-independent translation of the gene downstream of the IRES. Said host cells expressing TCRs specific for the polypeptide may be used, for example, for adoptive immunotherapy of lung cancer as discussed further below.

In further aspects of the present invention, cloned TCRs specific for a polypeptide recited herein may be used in a kit for the diagnosis of lung cancer. For example, the nucleic acid sequence or portions thereof, of tumor-specific TCRs can be used as probes or primers for the detection of expression of the rearranged genes encoding the specific TCR in a biological sample. Therefore, the present invention further provides for an assay for detecting messenger RNA or DNA encoding the TCR specific for a polypeptide.

### Pharmaceutical Compositions

In additional embodiments, the present invention concerns formulation of one or more of the polynucleotide, polypeptide, T-cell and/or antibody compositions disclosed herein in pharmaceutically-acceptable carriers for administration to a cell or an animal, either alone, or in combination with one or more other modalities of therapy.

It will be understood that, if desired, a composition as disclosed herein may be administered in combination with other agents as well, such as, *e*.*g*., other proteins or polypeptides or various pharmaceutically-active agents. In fact, there is virtually no limit to other components that may also be included, given that the additional agents do not cause a significant adverse effect upon contact with the target cells or host tissues. The compositions may thus be delivered along with various other agents as required in the particular instance. Such compositions may be purified from host cells or other biological sources, or alternatively may be chemically synthesized as described herein. Likewise, such compositions may further comprise substituted or derivatized RNA or DNA compositions.

Therefore, in another aspect of the present invention, pharmaceutical compositions are provided comprising one or more of the polynucleotide, polypeptide, antibody, and/or T-cell compositions described herein in combination with a physiologically acceptable carrier. In certain preferred embodiments, the pharmaceutical compositions of the invention comprise immunogenic polynucleotide and/or polypeptide compositions of the invention for use in prophylactic and theraputic vaccine applications. Vaccine preparation is generally described in, for example, M.F. Powell and M.J. Newman, eds., "Vaccine Design (the subunit and adjuvant approach)," Plenum Press (NY, 1995). Generally, such compositions will comprise one or more polynucleotide and/or polypeptide compositions of the present invention in combination with one or more immunostimulants.

It will be apparent that any of the pharmaceutical compositions described herein can contain pharmaceutically acceptable salts of the polynucleotides and polypeptides of the invention.. Such salts can be prepared, for example, from pharmaceutically acceptable non-toxic bases, including organic bases (*e*.*g*., salts of primary, secondary and tertiary amines and basic amino acids) and inorganic bases (*e*.*g*., sodium, potassium, lithium, ammonium, calcium and magnesium salts).

In another embodiment, illustrative immunogenic compositions, *e*.*g*., vaccine compositions, of the present invention comprise DNA encoding one or more of the polypeptides as described above, such that the polypeptide is generated *in situ*. As noted above, the polynucleotide may be administered within any of a variety of delivery systems known to those of ordinary skill in the art. Indeed, numerous gene delivery techniques are well known in the art, such as those described by Rolland, Crit. Rev. Therap. Drug Carrier Systems 15:143-198, 1998, and references cited therein.. Appropriate polynucleotide expression systems will, of course, contain the necessary regulatory DNA regulatory sequences for expression in a patient (such as a suitable promoter and terminating signal). Alternatively, bacterial delivery systems may involve the administration of a bacterium (such as *Bacillus-Calmette-Guerrin*) that expresses an immunogenic portion of the polypeptide on its cell surface or secretes such an epitope.

Therefore, in certain embodiments, polynucleotides encoding immunogenic polypeptides described herein are introduced into suitable mammalian host cells for expression using any of a number of known vrial-based systems. In one illustrative embodiment, retroviruses provide a convenient and effective platform for gene delivery systems. A selected nucleotide sequence encoding a polypeptide of the present invention can be inserted into a vector and packaged in retroviral particles using techniques known in the art. The recombinant virus can then be isolated and delivered to a subject. A number of illustrative retroviral systems have been described (*e*.*g*., U.S. Pat. No. 5,219,740; Miller and Rosman (1989) BioTechniques 7:980-990; Miller, A. D.. (1990) Human Gene Therapy 1:5-14; Scarpa et al. (1991) Virology 180:849-852; Burns et al. (1993) Proc. Natl. Acad. Sci. USA 90:8033-8037; and Boris·Lawrie and Temin (1993) Cur. Opin. Genet. Develop. 3:102-109.

In addition, a number of illustrative adenovirus-based systems have also been described. Unlike retroviruses which integrate into the host genome, adenoviruses persist extrachromosomally thus minimizing the risks associated with insertional mutagenesis (Haj-Ahmad and Graham (1986) J. Virol. 57:267-274; Bett et al. (1993) J. Virol. 67:5911-5921; Mittereder et al. (1994) Human Gene Therapy 5:717-729; Seth et al. (1994) J. Virol. 68:933-940; Barr et al.. (1994) Gene Therapy 1:51-58; Berkner, K. L. (1988) BioTechniques 6:616-629; and Rich et al. (1993) Human Gene Therapy 4:461-476)..

Various adeno-associated virus (AAV) vector systems have also been developed for polynucleotide delivery. AAV vectors can be readily constructed using techniques well known in the art. See, *e*.*g*., U.S. Pat.. Nos. 5,173,414 and 5,139,941; International Publication Nos. WO 92/01070 and WO 93/03769; Lebkowski et al. (1988) Molec. Cell. Biol. 8:3988-3996; Vincent et al. (1990) Vaccines 90 (Cold Spring Harbor Laboratory Press); Carter, B.. J. (1992) Current Opinion in Biotechnology 3:533-539; Muzyczka, N. (1992) Current Topics in Microbiol and Immunol. 158:97-129; Kotin, R. M.. (1994) Human Gene Therapy 5:793-801; Shelling and Smith (1994) Gene Therapy 1:165-169; and Zhou et al. (1994) J. Exp. Med. 179:1867-1875.

Additional viral vectors useful for delivering the polynucleotides encoding polypeptides of the present invention by gene transfer include those derived from the pox family of viruses, such as vaccinia virus and avian poxvirus. By way of example, vaccinia virus recombinants expressing the novel molecules can be constructed as follows. The DNA encoding a polypeptide is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the polypeptide of interest into the viral genome.. The resulting TK.sup..(-) recombinant can be selected by culturing the cells in the presence of 5-bromodeoxyuridine and picking viral plaques resistant thereto.

A vaccinia-based infection/transfection system can be conveniently used to provide for inducible, transient expression or coexpression of one or more polypeptides described herein in host cells of an organism. In this particular system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide or polynucleotides of interest, driven by a T7 promoter. The polymerase expressed in the cytoplasm from the vaccinia virus recombinant transcribes the transfected DNA into RNA which is then translated into polypeptide by the host translational machinery. The method provides for high level, transient, cytoplasmic production of large quantities of RNA and its translation products. See, *e*.*g*., Elroy-Stein and Moss, Proc. Natl. Acad. Sci. USA (1990) 87:6743-6747; Fuerst et al.. Proc. Natl. Acad. Sci. USA (1986) 83:8122-8126.

Alternatively, avipoxviruses, such as the fowlpox and canarypox viruses, can also be used to deliver the coding sequences of interest. Recombinant avipox viruses, expressing immunogens from mammalian pathogens, are known to confer protective immunity when administered to non-avian species. The use of an Avipox vector is particularly desirable in human and other mammalian species since members of the Avipox genus can only productively replicate in susceptible avian species and therefore are not infective in mammalian cells.. Methods for producing recombinant Avipoxviruses are known in the art and employ genetic recombination, as described above with respect to the production of vaccinia viruses. See, *e*.*g*., WO 91/12882; WO 89/03429; and WO 92/03545.

Any of a number of alphavirus vectors can also be used for delivery of polynucleotide compositions of the present invention, such as those vectors described in U.S. Patent Nos. 5,843,723; 6,015,686; 6,008,035 and 6,015,694. Certain vectors based on Venezuelan Equine Encephalitis (VEE) can also be used, illustrative examples of which can be found in U.S. Patent Nos.. 5,505,947 and 5,643,576.

Moreover, molecular conjugate vectors, such as the adenovirus chimeric vectors described in Michael et al. J. Biol. Chem. (1993) 268:6866-6869 and Wagner et al.. Proc. Natl. Acad. Sci. USA (1992) 89:6099-6103, can also be used for gene delivery under the invention.

Additional illustrative information on these and other known viral-based delivery systems can be found, for example, in Fisher-Hoch et al., Proc. Natl Acad. Sci. USA 86:317-321, 1989; Flexner et al., Ann N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502, 1993; Guzman et al., Circulation 88:2838-2848, 1993; and Guzman et al., Cir. Res. 73:1202-1207, 1993.

In certain embodiments, a polynucleotide may be integrated into the genome of a target cell. This integration may be in the specific location and orientation *via* homologous recombination (gene replacement) or it may be integrated in a random, non-specific location (gene augmentation). In yet further embodiments, the polynucleotide may be stably maintained in the cell as a separate, episomal segment of DNA. Such polynucleotide segments or "episomes" encode sequences sufficient to permit maintenance and replication independent of or in synchronization with the host cell cycle. The manner in which the expression construct is delivered to a cell and where in the cell the polynucleotide remains is dependent on the type of expression construct employed.

In another embodiment of the invention, a polynucleotide is administered/delivered as "naked" DNA, for example as described in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

In still another embodiment, a composition of the present invention can be delivered via a particle bombardment approach, many of which have been described. In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U..S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide or polypeptide particles, are accelerated to high speed within a helium gas jet generated by a hand held device, propelling the particles into a target tissue of interest.

In a related embodiment, other devices and methods that may be useful for gas driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

According to another embodiment, the pharmaceutical compositions described herein will comprise one or more immunostimulants in addition to the immunogenic polynucleotide, polypeptide, antibody, T-cell and/or APC compositions of this invention. An immunostimulant refers to essentially any substance that enhances or potentiates an immune response (antibody and/or cell-mediated) to an exogenous antigen. One preferred type of immunostimulant comprises an adjuvant. Many adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Certain adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Within certain embodiments of the invention, the adjuvant composition is preferably one that induces an immune response predominantly of the Th1 type. High levels of Th1-type cytokines (*e*.*g*., IFN-γ, INFα, IL-2 and IL-12) tend to favor the induction of cell mediated immune responses to an administered antigen. In contrast, high levels of Th2-type cytokines (*e*.*g*., IL-4, IL-5, IL-6 and IL-10) tend to favor the induction of humoral immune responses. Following application of a vaccine as provided herein, a patient will support an immune response that includes Th1- and Th2-type responses. Within a referred embodiment, in which a response is predominantly Th1-type, the level of Th1-ype cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173, 1989.

Certain preferred adjuvants for eliciting a predominantly Th1-type response include, for example, a combination of monophosphoryl lipid A, preferably 3-de-O-acylated monophosphoryl lipid A, together with an aluminum salt.. MPL^{®} adjuvants are available from Corixa Corporation (Seattle, WA; *see*, for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462.. Immunostimulatory DNA sequences are also described, for example, by Sato et al., *Science 273*:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins. Other preferred formulations include more than one saponin in the adjuvant combinations of the present invention, for example combinations of at least two of the following group comprising QS21, QS7, Quil A, β-escin, or digitonin.

Alternatively the saponin formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol^{R} to increase viscosity, or may be formulated in a dry powder form with a powder excipient such as lactose.

In one preferred embodiment, the adjuvant system includes the combination of a monophosphoryl lipid A and a saponin derivative, such as the combination of QS21 and 3D-MPL^{®} adjuvant, as described in WO 94/00153, or a less reactogenic composition where the QS21 is quenched with cholesterol, as described in WO 96/33739. Other preferred formulations comprise an oil-in-water emulsion and tocopherol. Another particularly preferred adjuvant formulation employing QS21, 3D-MPL^{®} adjuvant and tocopherol in an oil-in-water emulsion is described in WO 95/17210.

Another enhanced adjuvant system involves the combination of a CpG-containing oligonucleotide and a saponin derivative particularly the combination of CpG and QS21 is disclosed in WO 00/09159. Preferably the formulation additionally comprises an oil in water emulsion and tocopherol.

Additional illustrative adjuvants for use in the pharmaceutical compositions of the invention include Montanide ISA 720 (Seppic, France), SAF (Chiron, California, United States), ISCOMS (CSL), MF-59 (Chiron), the SBAS series of adjuvants (*e*.*g*., SBAS-2 or SBAS-4, available from SmithKline Beecham, Rixensart, Belgium), Detox (Enhanzyn^{®}) (Corixa, Hamilton, MT), RC-529 (Corixa, Hamilton, MT) and other aminoalkyl glucosaminide 4-phosphates (AGPs), such as those described in pending U.S. Patent Application Serial Nos. 08/853,826 and 09/074,720, the disclosures of which are incorporated herein by reference in their entireties, and polyoxyethylene ether adjuvants such as those described in WO 99/52549A1.

Other preferred adjuvants include adjuvant molecules of the general formula

(I): HO(CH₂CH₂O)ₙ-A-R,

wherein, n is 1-50, A is a bond or -C(O)-, R is C₁₋₅₀ alkyl or Phenyl C₁₋₅₀ alkyl.

One embodiment of the present invention consists of a vaccine formulation comprising a polyoxyethylene ether of general formula (I), wherein *n* is between 1 and 50, preferably 4-24, most preferably 9; the *R* component is C₁₋₅₀, preferably C₄-C₂₀ alkyl and most preferably C₁₂ alkyl, and *A* is a bond.. The concentration of the polyoxyethylene ethers should be in the range 0.1-20%, preferably from 0.1-10%, and most preferably in the range 0.1-1%. Preferred polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35·lauryl ether, and polyoxyethylene-23-lauryl ether. Polyoxyethylene ethers such as polyoxyethylene lauryl ether are described in the Merck index (12^{th} edition: entry 7717). These adjuvant molecules are described in WO 99/52549.

The polyoxyethylene ether according to the general formula (I) above may, if desired, be combined with another adjuvant. For example, a preferred adjuvant combination is preferably with CpG as described in the pending UK patent application GB 9820956.2.

According to another embodiment of this invention, an immunogenic composition described herein is delivered to a host via antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i*.*e*., matched HLA haplotype). APCs may generally be isolated from any of a variety of biological fluids and organs, including tumor and peritumoral tissues, and may be autologous, allogeneic, syngeneic or xenogeneic cells..

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity (*see* Timmerman and Levy, Ann. Rev. Med. 50:507-529, 1999). In general, dendritic cells may be identified based on their typical shape (stellate *in situ*, with marked cytoplasmic processes (dendrites) visible *in vitro*), their ability to take up, process and present antigens with high efficiency and their ability to activate naive T cell responses. Dendritic cells may, of course, be engineered to express specific cell-surface receptors or ligands that are not commonly found on dendritic cells *in vivo* or *ex vivo*, and such modified dendritic cells are contemplated by the present invention. As an alternative to dendritic cells, secreted vesicles antigen-loaded dendritic cells (called exosomes) may be used within a vaccine (*see* Zitvogel et al., Nature Med. 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e*.*g*., CD54 and CD11) and costimulatory molecules (*e*.*g*., CD40, CD80, CD86 and 4-1BB).

APCs may generally be transfected with a polynucleotide of the invention (or portion or other variant thereof) such that the encoded polypeptide, or an immunogenic portion thereof, is expressed on the cell surface. Such transfection may take place *ex vivo*, and a pharmaceutical composition comprising such transfected cells may then be used for therapeutic purposes, as described herein. Alternatively, a gene delivery vehicle that targets a dendritic or other antigen presenting cell may be administered to a patient, resulting in transfection that occurs *in vivo*. *In vivo* and *ex vivo* transfection of dendritic cells, for example, may generally be performed using any methods known in the art, such as those described in WO 97/24447, or the gene gun approach described by Mahvi et al.., Immunology and cell Biology 75:456-460, 1997. Antigen loading of dendritic cells may be achieved by incubating dendritic cells or progenitor cells with the tumor polypeptide, DNA (naked or within a plasmid vector) or RNA; or with antigen-expressing recombinant bacterium or viruses (*eg*., vaccinia, fowlpox, adenovirus or lentivirus vectors). Prior to loading, the polypeptide may be covalently conjugated to an immunological partner that provides T cell help (*e*.*g*., a carrier molecule). Alternatively, a dendritic cell may be pulsed with a non-conjugated immunological partner, separately or in the presence of the polypeptide.

While any suitable carrier known to those of ordinary skill in the art may be employed in the pharmaceutical compositions of this invention, the type of carrier will typically vary depending on the mode of administration. Compositions of the present invention may be formulated for any appropriate manner of administration, including for example, topical, oral, nasal, mucosal, intravenous, intracranial, intraperitoneal, subcutaneous and intramuscular administration.

Carriers for use within such pharmaceutical compositions are biocompatible, and may also be biodegradable. In certain embodiments, the formulation preferably provides a relatively constant level of active component release.. In other embodiments, however, a more rapid rate of release immediately upon administration may be desired. The formulation of such compositions is well within the level of ordinary skill in the art using known techniques. Illustrative carriers useful in this regard include microparticles of poly(lactide-co-glycolide), polyacrylate, latex, starch, cellulose, dextran and the like. Other illustrative delayed-release carriers include supramolecular biovectors, which comprise a non-liquid hydrophilic core (*e*.*g*., a cross-linked polysaccharide or oligosaccharide) and, optionally, an external layer comprising an amphiphilic compound, such as a phospholipid (*see e*.*g*., U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented..

In another illustrative embodiment, biodegradable microspheres (*e*.*g*., polylactate polyglycolate) are employed as carriers for the compositions of this invention. Suitable biodegradable microspheres are disclosed, for example, in U.S. Patent Nos. 4,897,268; 5,075,109; 5,928,647; 5,811,128; 5,820,883; 5,853,763; 5,814,344, 5,407,609 and 5,942,252 Modified hepatitis B core protein carrier systems such as described in WO/99 40934, and references cited therein, will also be useful for many applications. Another illustrative carrier/delivery system employs a carrier comprising particulate-protein complexes, such as those described in U.S. Patent No. 5,928,647, which are capable of inducing a class I-restricted cytotoxic T lymphocyte responses in a host.

The pharmaceutical compositions of the invention will often further comprise one or more buffers (*e*.*g*., neutral buffered saline or phosphate buffered saline), carbohydrates (*e*.*g*., glucose, mannose, sucrose or dextrans), mannitol, proteins, polypeptides or amino acids such as glycine, antioxidants, bacteriostats, chelating agents such as EDTA or glutathione, adjuvants (*e*.*g*., aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate.

The pharmaceutical compositions described herein may be presented in unit-dose or multi-dose containers, such as sealed ampoules or vials. Such containers are typically sealed in such a way to preserve the sterility and stability of the formulation until use. In general, formulations may be stored as suspensions, solutions or emulsions in oily or aqueous vehicles. Alternatively, a pharmaceutical composition may be stored in a freeze-dried condition requiring only the addition of a sterile liquid carrier immediately prior to use.

The development of suitable dosing and treatment regimens for using the particular compositions described herein in a variety of treatment regimens, including *e*.*g*., oral, parenteral, intravenous, intranasal, and intramuscular administration and formulation, is well known in the art, some of which are briefly discussed below for general purposes of illustration.

In certain applications, the pharmaceutical compositions disclosed herein may be delivered *via* oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet

The active compounds may even be incorporated with excipients and used in the form of ingestible tablets, buccal tables, troches, capsules, elixirs, suspensions, syrups, wafers, and the like (see, for example, Mathiowitz et al., Nature 1997 Mar 27;386(6623):410-4; Hwang et al., Crit Rev Ther Drug Carrier Syst 1998;15(3):243-84; U. S. Patent 5,641,515; U. S. Patent 5,580,579 and U. S. Patent 5,792,451). Tablets, troches, pills, capsules and the like may also contain any of a variety of additional components, for example, a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Of course, any material used in preparing any dosage unit form should be pharmaceutically pure and substantially non-toxic in the amounts employed. In addition, the active compounds may be incorporated into sustained-release preparation and formulations.

Typically, these formulations will contain at least about 0.1% of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions of the present invention may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth..

In certain circumstances it will be desirable to deliver the pharmaceutical compositions disclosed herein parenterally, intravenously, intramuscularly, or even intraperitoneally. Such approaches are well known to the skilled artisan, some of which are further described, for example, in U. S. Patent 5,543,158; U. S. Patent 5,641,515 and U. S. Patent 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms

Illustrative pharmaceutical forms suitable for injectable use include sterile aqueous solutions or dispersions and sterile powders for the extemporaneous preparation of sterile injectable solutions or dispersions (for example, see U. S. Patent 5,466,468).. In all cases the form must be sterile and must be fluid to the extent that easy syringability exists.. It must be stable under the conditions of manufacture and storage and must be preserved against the contaminating action of microorganisms, such as bacteria and fungi The carrier can be a solvent or dispersion medium containing, for example, water, ethanol, polyol (*e.g.,* glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologics standards.

In another embodiment of the invention, the compositions disclosed herein may be formulated in a neutral or salt form. Illustrative pharmaceutically acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like.. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions.. The phrase "pharmaceutically-acceptable" refers to molecular entities and compositions that do not produce an allergic or similar untoward reaction when administered to a human.

In certain embodiments, the pharmaceutical compositions may be delivered by intranasal sprays, inhalation, and/or other aerosol delivery vehicles. Methods for delivering genes, nucleic acids, and peptide compositions directly to the lungs *via* nasal aerosol sprays has been described, *e.g.,* in U. S. Patent 5,756,353 and U. S. Patent 5,804,212. Likewise, the delivery of drugs using intranasal microparticle resins (Takenaga et al., J Controlled Release 1998 Mar 2;52(1-2):81-7) and lysophosphatidyl-glycerol compounds (U.. S. Patent 5,725,871) are also well-known in the pharmaceutical arts. Likewise, illustrative transmucosal drug delivery in the form of a polytetrafluoroetheylene support matrix is described in U. S. Patent 5,780,045.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like.. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol 1998 Jul;16(7):307-21; Takakura, Nippon Rinsho 1998 Mar;56(3):691-5; Chandran et al., Indian J Exp Biol. 1997 Aug;35(8):801-9; Margalit, Crit Rev Ther Drug Carrier Syst. 1995;12(2-3):233-61; U.S. Patent 5,567,434; U.S. Patent 5,552,157; U.S. Patent 5,565,213; U.S. Patent 5,738,868 and U.S.. Patent 5,795,587, each specifically incorporated herein by reference in its entirety).

Liposomes have been used successfully with a number of cell types that are normally difficult to transfect by other procedures, including T cell suspensions, primary hepatocyte cultures and PC 12 cells (Renneisen et al., J Biol Chem. 1990 Sep 25;265(27):16337-42; Muller et al., DNA Cell Biol. 1990 Apr;9(3):221-9). In addition, liposomes are free of the DNA length constraints that are typical of viral-based delivery systems. Liposomes have been used effectively to introduce genes, various drugs, radiotherapeutic agents, enzymes, viruses, transcription factors, allosteric effectors and the like, into a variety of cultured cell lines and animals. Furthermore, he use of liposomes does not appear to be associated with autoimmune responses or unacceptable toxicity after systemic delivery.

In certain embodiments, liposomes are formed from phospholipids that are dispersed in an aqueous medium and spontaneously form multilamellar concentric bilayer vesicles (also termed multilamellar vesicles (MLVs).

Alternatively, in other embodiments, the invention provides for pharmaceutically-acceptable nanocapsule formulations of the compositions of the present invention. Nanocapsules can generally entrap compounds in a stable and reproducible way (see, for example, Quintanar-Guerrero et al., Drug Dev Ind Pharm 1998 Dec;24(12):1113-28) To avoid side effects due to intracellular polymeric overloading, such ultrafine particles (sized around 0.1 µm) may be designed using polymers able to be degraded *in vivo.* Such particles can be made as described, for example, by Couvreur et al., Crit Rev Ther Drug Carrier Syst 1988;5(1):1-20; zur Muhlen et al., Eur J Pharm Biopharm. 1998 Mar;45(2):149-55; Zambaux et al. J Controlled Release. 1998 Jan 2;50(1-3):31-40; and U. S. Patent 5,145,684.

### Cancer Therapeutic Methods

Immunologic approaches to cancer therapy are based on the recognition that cancer cells can often evade the body's defenses against aberrant or foreign cells and molecules, and that these defenses might be therapeutically stimulated to regain the lost ground, *e.g.* pgs.. 623-648 in Klein, Immunology (Wiley-Interscience, New York, 1982). Numerous recent observations that various immune effectors can directly or indirectly inhibit growth of tumors has led to renewed interest in this approach to cancer therapy, *e.g.* Jager, et aL, Oncology 2001;60(1):1-7; Renner, et al., Ann Hematol 2000 Dec;79(12):651-9.

Four-basic cell types whose function has been associated with antitumor cell immunity and the elimination of tumor cells from the body are: i) B-lymphocytes which secrete immunoglobulins into the blood plasma for identifying and labeling the nonself invader cells; ii) monocytes which secrete the complement proteins that are responsible for lysing and processing the immunoglobulin-coated target invader cells; iii) natural killer lymphocytes having two mechanisms for the destruction of tumor cells, antibody-dependent cellular cytotoxicity and natural killing; and iv) T-lymphocytes possessing antigen specific receptors and having the capacity to recognize a tumor cell carrying complementary marker molecules (Schreiber, H., 1989, in Fundamental Immunology (ed). W. E. Paul, pp. 923-955).

Cancer immunotherapy generally focuses on inducing humoral immune responses, cellular immune responses, or both. Moreover, it is well established that induction of CD4⁺ T helper cells is necessary in order to secondarily induce either antibodies or cytotoxic CD8⁺ T cells. Polypeptide antigens that are selective or ideally specific for cancer cells, particularly lung cancer cells, offer a powerful approach for inducing immune responses against lung cancer, and are an important aspect of the present invention.

Therefore, in further aspects of the present invention, the pharmaceutical compositions described herein may be used for the treatment of cancer, particularly for the immunotherapy of lung cancer. Within such methods, the pharmaceutical compositions described herein are administered to a patient, typically a warm-blooded animal, preferably a human. A patient may or may not be afflicted with cancer. Accordingly, the above pharmaceutical compositions may be used to prevent the development of a cancer or to treat a patient afflicted with a cancer. Pharmaceutical compositions and vaccines may be administered either prior to or following surgical removal of primary tumors and/or treatment such as administration of radiotherapy or conventional chemotherapeutic drugs As discussed above, administration of the pharmaceutical compositions may be by any suitable method, including administration by intravenous, intraperitoneal, intramuscular, subcutaneous, intranasal, intradermal, anal, vaginal, topical and oral routes.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides as provided herein)

Within other embodiments, immunotherapy may be passive immunotherapy, in which treatment involves the delivery of agents with established tumor immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic I lymphocytes and CD4⁺ I-helper tumor-infiltrating lymphocytes), killer cells (such as Natural Killer cells and lymphokine-activated killer cells), B cells and antigen-presenting cells (such as dendritic cells and macrophages) expressing a polypeptide provided herein T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Monoclonal antibodies may be labeled with any of a variety of labels for desired selective usages in detection, diagnostic assays or therapeutic applications (as described in U.S. Patent Nos. 6,090,365; 6,015,542; 5,843,398; 5,595,721; and 4,708,930, hereby incorporated by reference in their entirety as if each was incorporated individually). In each case, the binding of the labelled monoclonal antibody to the determinant site of the antigen will signal detection or delivery of a particular therapeutic agent to the antigenic determinant on the non-normal cell.. A further object of this invention is to provide the specific monoclonal antibody suitably labelled for achieving such desired selective usages thereof.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells.. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides or transfected with one or more polynucleotides using standard techniques well known in the art. For example, antigen presenting cells can be transfected with a polynucleotide having a promoter appropriate for increasing expression in a recombinant virus or other expression system Cultured effector cells for use in therapy must be able to grow and distribute widely, and to survive long term *in vivo*. Studies have shown that cultured effector cells can be induced to grow in vivo and to survive long term in substantial numbers by repeated stimulation with antigen supplemented with IL-2 (*see,* for example, Cheever et al., Immunological Reviews 157:177, 1997)..

Alternatively, a vector expressing a polypeptide recited herein may be introduced into antigen presenting cells taken from a patient and clonally propagated ex *vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions described herein, as well as dosage, will vary from individual to individual, and may be readily established using standard techniques. In general, the pharmaceutical compositions and vaccines may be administered by injection (*e.g.,* intracutaneous, intramuscular, intravenous or subcutaneous), intranasally (*e.g.,* by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i.e.,* untreated) level. Such response can be monitored by measuring the and-tumor antibodies in a patient or by vaccine-dependent generation of cytolytic effector cells capable of killing the patient's tumor cells *in vitro*. Such vaccines should also be capable of causing an immune response that leads to an improved clinical outcome (*e.g.,* more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0..1 mL to about 5 mL

In general, an appropriate dosage and treatment regimen provides the active compound(s) in an amount sufficient to provide therapeutic and/or prophylactic benefit Such a response can be monitored by establishing an improved clinical outcome (*e.g.,* more frequent remissions, complete or partial, or longer disease-free survival) in treated patients as compared to non-treated patients. Increases in preexisting immune responses to a tumor protein generally correlate with an improved clinical outcome. Such immune responses may generally be evaluated using standard proliferation, cytotoxicity or cytokine assays, which may be performed using samples obtained from a patient before and after treatment.

### Cancer Detection and Diagnostic Compositions. Methods and Kits

In general, a cancer may be detected in a patient based on the presence of one or more lung tumor proteins and/or polynucleotides encoding such proteins in a biological sample (for example, blood, sera, sputum urine and/or tumor biopsies) obtained from the patient. In other words, such proteins may be used as markers to indicate the presence or absence of a cancer such as lung cancer. In addition, such proteins may be useful for the detection of other cancers. The binding agents provided herein generally permit detection of the level of antigen that binds to the agent in the biological sample..

Polynucleotide primers and probes may be used to detect the level of mRNA encoding a tumor protein, which is also indicative of the presence or absence of a cancer In general, a tumor sequence should be present at a level that is at least two fold, preferably three-fold, and more preferably five-fold or higher in tumor tissue than in normal tissue of the same type from which the tumor arose. Expression levels of a particular tumor sequence in tissue types different from that in which the tumor arose are irrelevant in certain diagnostic embodiments since the presence of tumor cells can be confirmed by observation of predetermined differential expression levels, e.g., 2-fold, 5-fold, etc, in tumor tissue to expression levels in normal tissue of the same type.

Other differential expression patterns can be utilized advantageously for diagnostic purposes. For example, in one aspect of the invention, overexpression of a tumor sequence in tumor tissue and normal tissue of the same type, but not in other normal tissue types, *e.g.* PBMCs, can be exploited diagnostically. In this case, the presence of metastatic tumor cells, for example in a sample taken from the circulation or some other tissue site different from that in which the tumor arose, can be identified and/or confirmed by detecting expression of the tumor sequence in the sample, for example using RT-PCR analysis In many instances, it will be desired to enrich for tumor cells in the sample of interest, e.g., PBMCs, using cell capture or other like techniques.

There are a variety of assay formats known to those of ordinary skill in the art for using a binding agent to detect polypeptide markers in a sample. *See, e.g*.*,* Hallow and Lane, Antibodies: A Laboratory Manual, Cold Spring Harbor Laboratory, 1988.. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin.. Alternatively, a competitive assay may be utilized, in which a polypeptide is labeled with a reporter group and allowed to bind to the immobilized binding agent after incubation of the binding agent with the sample The extent to which components of the sample inhibit the binding of the labeled polypeptide to the binding agent is indicative of the reactivity of the sample with the immobilized binding agent. Suitable polypeptides for use within such assays include full length lung tumor proteins and polypeptide portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day.. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked.. Any suitable blocking agent known to those of ordinary skill in the art, such as bovine serum albumin or Tween 20™ (Sigma Chemical Co.., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i.e.,* incubation time) is a period of time that is sufficient to detect the presence of polypeptide within a sample obtained from an individual with lung cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time.. At room temperature, an incubation time of about 30 minutes is generally sufficient.

Unbound sample may then be removed by washing the solid support with an appropriate buffer, such as PBS containing 0.1% Tween 20™. The second antibody, which contains a reporter group, may then be added to the solid support. Preferred reporter groups include those groups recited above..

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as lung cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates (*i*.*e*., sensitivity) and false positive rates (100%-specificity) that correspond to each possible cut-off value for the diagnostic test result. The cut-off value on the plot that is the closest to the upper left-hand corner (*i*.*e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample.. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use tumor polypeptide to detect antibodies that bind to such polypeptides in a biological sample. The detection of such tumor protein specific antibodies may correlate with the presence of a cancer.

A cancer may also, or alternatively, be detected based on the presence of T cells that specifically react with a tumor protein in a biological sample. Within certain methods, a biological sample comprising CD4⁺ and/or CD8⁺ T cells isolated from a patient is incubated with a tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated I cells.. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with polypeptide (*e*.*g*., 5-25 µg/ml). It may be desirable to incubate another aliquot of a T cell sample in the absence of tumor polypeptide to serve as a control For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a tumor protein in a biological sample For example, at least two oligonucleotide primers may be employed in a polymerase chain reaction (PCR) based assay to amplify a portion of a tumor cDNA derived from a biological sample, wherein at least one of the oligonucleotide primers is specific for (*i*.*e*., hybridizes to) a polynucleotide encoding the tumor protein The amplified cDNA is then separated and detected using techniques well known in the art, such as gel electrophoresis

Similarly, oligonucleotide probes that specifically hybridize to a polynucleotide encoding a tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a tumor protein of the invention that is at least 10 nucleotides, and preferably at least 20 nucleotides, in length. Preferably, oligonucleotide primers and/or probes hybridize to a polynucleotide encoding a polypeptide described herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence as disclosed herein. Techniques for both PCR based assays and hybridization assays are well known in the art (*see*, for example, Mullis et al., Cold Spring Harbor Symp. Quaint. Biol, 51:263, 1987; Erlich ed, PCR Technology, Stockton Press, NY,1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another aspect of the present invention, cell capture technologies may be used in conjunction, with, for example, real-time PCR to provide a more sensitive tool for detection of metastatic cells expressing lung tumor antigens. Detection of lung cancel cells in biological samples, e.g., bone marrow samples, peripheral blood, and small needle aspiration samples is desirable for diagnosis and prognosis in lung cancer patients.

Immunomagnetic beads coated with specific monoclonal antibodies to surface cell markers, or tetrameric antibody complexes, may be used to first enrich or positively select cancer cells in a sample. Various commercially available kits may be used, including Dynabeads® Epithelial Enrich (Dynal Biotech, Oslo, Norway), StemSep™ (StemCell Technologies, Inc., Vancouver, BC), and RosetteSep (StemCell Technologies).. A skilled artisan will recognize that other methodologies and kits may also be used to enrich or positively select desired cell populations. Dynabeads® Epithelial Enrich contains magnetic beads coated with mAbs specific for two glycoprotein membrane antigens expressed on normal and neoplastic epithelial tissues. The coated beads may be added to a sample and the sample then applied to a magnet, thereby capturing the cells bound to the beads.. The unwanted cells are washed away and the magnetically isolated cells eluted from the beads and used in further analyses.

RosetteSep can be used to enrich cells directly from a blood sample and consists of a cocktail of tetrameric antibodies that targets a variety of unwanted cells and crosslinks them to glycophorin A on red blood cells (RBC) present in the sample, forming rosettes. When centrifuged over Ficoll, targeted cells pellet along with the free RBC. The combination of antibodies in the depletion cocktail determines which cells will be removed and consequently which cells will be recovered.. Antibodies that are available include, but are not limited to: CD2, CD3, CD4, CD5, CD8, CD10, CD11b, CD14, CD15, CD16, CD19, CD20, CD24, CD25, CD29, CD33, CD34, CD36, CD38, CD41, CD45, CD45RA, CD45RO, CD56, CD66B, CD66e, HLA-DR, IgE, and TCRαβ.

Additionally, it is contemplated in the present invention that mAbs specific for lung tumor antigens can be generated and used in a similar manner.. For example, mAbs that bind to tumor-specific cell surface antigens may be conjugated to magnetic beads, or formulated in a tetrameric antibody complex, and used to enrich or positively select metastatic lung tumor cells from a sample. Once a sample is enriched or positively selected, cells may be lysed and RNA isolated. RNA may then be subjected to RT-PCR analysis using lung tumor-specific primers in a real-time PCR assay as described herein. One skilled in the art will recognize that enriched or selected populations of cells may be analyzed by other methods (*e.g. in situ* hybridization or flow cytometry).

In another embodiment, the compositions described herein may be used as markers for the progression of cancer. In this embodiment, assays as described above for the diagnosis of a cancer may be performed over time, and the change in the level of reactive polypeptide(s) or polynucleotide(s) evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple tumor protein markers may be assayed within a given sample. It will be apparent that binding agents specific for different proteins provided herein may be combined within a single assay. Further, multiple primers or probes may be used concurrently, The selection of tumor protein markers may be based on routine experiments to determine combinations that results in optimal sensitivity.. In addition, or alternatively, assays for tumor proteins provided herein may be combined with assays for other known tumor antigens..

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a tumor protein. Such antibodies or fragments may be provided attached to a support material, as described above.. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a tumor protein in a biological sample. Such kits generally comprise at least one oligonucleotide probe or primer, as described above, that hybridizes to a polynucleotide encoding a tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a tumor protein.

The following examples are offered by way of illustration and not by way of limitation.

### EXAMPLES

### EXAMPLE 1

### ISOLATION AND CHARACTERIZATION OF cDNA SEQUENCES E`NCODING LUNG TUMOR POLYPEP7IDES

This example illustrates the isolation of cDNA molecules encoding lung tumor-specific polypeptides from lung tumor cDNA libraries.

### A. ISOLATION OF CDNA SEQUENCES FROM A LUNG SQUAMOUS CELL CARCINOMA LIBRARY

A human lung squamous cell carcinoma cDNA expression library was constructed from poly A⁺ RNA from a pool of two patient tissues using a Superscript Plasmid System for cDNA Synthesis and Plasmid Cloning kit (BRL Life Technologies, Gaithersburg, MD) following the manufacturer's protocol. Specifically, lung carcinoma tissues were homogenized with polytron (Kinematica, Switzerland) and total RNA was extracted using Trizol reagent (BRL Life Technologies) as directed by the manufacturer The poly A⁺ RNA was then purified using an oligo dT cellulose column as described in Sambrook et al., Molecular Cloning. A Laboratory Manual, Cold Spring Harbor Laboratories, Cold Spring Harbor, NY, 1989.. First-strand cDNA was synthesized using the NotI/Oligo-dT18 primer. Double-stranded cDNA was synthesized, ligated with BstXI/EcoRI adaptors (Invitrogen, San Diego, CA) and digested with NotI. Following size fractionation with cDNA size fractionation columns (BRL Life Technologies), the cDNA was ligated into the BstXUNotI site of pcDNA3.1 (Invitrogen) and transformed into ElectroMax *E. coli* DH10B cells (BRL Life Technologies) by electroporation.

Using the same procedure, a normal human lung cDNA expression library was prepared from a pool of four tissue specimens: The cDNA libraries were characterized by determining the number of independent colonies, the percentage of clones that carried insert, the average insert size and by sequence analysis.. The lung squamous cell carcinoma library contained 2.7 x 10⁶ independent colonies, with 100% of clones having an insert and the average insert size being 2100 base pairs. The normal lung cDNA library contained 1.4 x 10⁶ independent colonies, with 90% of clones having inserts and the average insert size being 1800 base pairs. For both libraries, sequence analysis showed that the majority of clones had a full length cDNA sequence and were synthesized from mRNA

cDNA library subtraction was performed using the above lung squamous cell carcinoma and normal lung cDNA libraries, as described by Hara *et al*. (*Blood*, *84*:189-199, 1994) with some modifications. Specifically, a lung squamous cell carcinoma-specific subtracted cDNA library was generate as follows. Normal tissue cDNA library (80 µg) was digested with BamHI and XhoI, followed by a filling-in reaction with DNA polymerase Klenow fragment. After phenol-chlorofoim extraction and ethanol precipitation, the DNA was dissolved in 133 µl of H₂O, heat-denatured and mixed with 133 µl (1.33 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (67 µl) was added and the biotinylation reaction was repeated. After extraction with butanol five times, the DNA was ethanol-precipitated and dissolved in 23 µl H₂O to form the driver DNA.

To form the tracer DNA, 10 µg lung squamous cell carcinoma cDNA library was digested with NotI and SpeI, phenol chloroform extracted and passed through Chroma spin-400 columns (Clontech, Palo Alto, CA). Typically, 5 µg of cDNA was recovered after the sizing column. Following ethanol precipitation, the tracer DNA was dissolved in 5 µl H₂O. Tracer DNA was mixed with 15 µl driver DNA and 20 µl of 2 x hybridization buffer (1..5 M NaCl/10 mM EDTA/50 mM HEPES pH 7.5/0.2% sodium dodecyl sulfate), overlaid with mineral oil, and heat-denatured completely. The sample was immediately transferred into a 68°C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68°C for 2 hours (short hybridization [SH]). After removal of biotinylated double-stranded DNA, subtracted cDNA was ligated into NotI/Spel site of chloramphenicol resistant pBCSK⁺ (Stratagene, La Jolla, CA) and transformed into ElectroMax *E*. *coli* DH10B cells by electroporation to generate a lung squamous cell carcinoma specific subtracted cDNA library (herein after referred to as "lung subtraction I").

A second lung squamous cell carcinoma specific subtracted cDNA library (referred to as "lung subtraction II") was generated in a similar way to the lung subtraction library I, except that eight frequently recovered genes from lung subtraction I were included in the driver DNA, and 24,000 independent clones were recovered.

To analyze the subtracted cDNA libraries, plasmid DNA was prepared from 320 independent clones, randomly picked from the subtracted lung squamous cell carcinoma specific libraries. Representative cDNA clones were further characterized by DNA sequencing with a Parkin Elmer/Applied Biosystems Division Automated Sequencer Model 373A and/or Model 377 (Foster City, CA). The cDNA sequences for sixty isolated clones are provided in SEQ ID NO: 1-60. These sequences were compared to known sequences in the gene bank using the EMBL and GenBank databases (release 96). No significant homologies were found to the sequences provided in SEQ ID NO: 2, 3, 19, 38 and 46. The sequences of SEQ ID NO: 1*,* 6*-*8, 10-13, 15, 17, 18, 20-27, 29, 30, 32, 34-37, 39-45, 47-49, 51, 52, 54, 55 and 57-59 were found to show some homology to previously identified expressed sequence tags (ESTs). The sequences of SEQ ID NO: 9, 28, 31 and 33 were found to show some homology to previously identified non-human gene sequences and the sequences of SEQ ID NO: 4, 5, 14, 50, 53, 56 and 60 were found to show some homology to gene sequences previously identified in humans.

The subtraction procedure described above was repeated using the above lung squamous cell carcinoma cDNA library as the tracer DNA, and the above normal lung tissue cDNA library and a cDNA library from normal liver and heart (constructed from a pool of one sample of each tissue as described above), plus twenty other cDNA clones that were frequently recovered in lung subtractions I and II, as the driver DNA (lung subtraction III). The normal liver and heart cDNA library contained 1.76 x 10⁶ independent colonies, with 100% of clones having inserts and the average insert size being 1600 base pairs. Ten additional clones were isolated (SEQ ID NO: 61-70). Comparison of these cDNA sequences with those in the gene bank as described above, revealed no significant homologies to the sequences provided in SEQ ID NO: 62 and 67. The sequences of SEQ ID NO: 61, 63-66, 68 and 69 were found to show some homology to previously isolated ESTs and the sequence provided in SEQ ID NO: 70 was found to show some homology to a previously identified rat gene.

In further studies, the subtraction procedure described above was repeated using the above lung squamous cell carcinoma cDNA library as the tracer DNA, and a cDNA library from a pool of normal lung, kidney, colon, pancreas, brain, resting PBMC, heart, skin and esophagus as the driver DNA, with esophagus cDNAs making up one third of the driver material. Since esophagus is enriches in normal epithelial cells, including differentiated squamous cells, this procedure is likely to enrich genes that are tumor specific lather than tissues specific. The cDNA sequences of 48 clones determined in this subtraction are provided in SEQ ID NO: 177-224. The sequences of SEQ ID NO: 177, 178, 180, 181, 183, 187, 192, 195-197, 208, 211, 212, 215, 216, 218 and 219 showed some homology to previously identified genes. The sequences of SEQ ID NO: 179, 182, 184-186, 188-191, 193, 194, 198-207, 209 210, 213, 214, 217, 220 and 224 showed some homology to previously determined ESTs. The sequence of SEQ ID NO: 221-223 showed no homology to any previously determined sequence.

### B. ISOLATION OF cDNA SEQUENCES FROM A LUNG ADENOCARCINOMA LIBRARY

A human lung adenocarcinoma cDNA expression library was constructed as described above The library contained 3.2 x 10⁶ independent colonies, with 100% of clones having an insert and the average insert size being 1500 base pairs.. Library subtraction was performed as described above using the normal lung and normal liver and heart cDNA expression libraries described above as the driver DNA.. Twenty-six hundred independent clones were recovered,

Initial cDNA sequence analysis from 100 independent clones revealed many ribosomal protein genes. The cDNA sequences for fifteen clones isolated in this subtraction are provided in SEQ ID NO: 71-86. Comparison of these sequences with those in the gene bank as described above revealed no significant homologies to the sequence provided in SEQ ID NO: 84. The sequences of SEQ ID NO: 71, 73, 74, 77, 78 and 80-82 were found to show some homology to previously isolated ESTs, and the sequences of SEQ ID NO: 72, 75, 76, 79, 83 and 85 were found to show some homology to previously identified human genes.

In further studies, a cDNA library (referred to as mets3616A) was constructed from a metastatic lung adenocarcinoma. The determined cDNA sequences of 25 clones sequenced at random from this library are provided in SEQ ID NO: 255-279. The mets3616A cDNA library was subtracted against a cDNA library prepared from a pool of normal lung, liver, pancreas, skin, kidney, brain and resting PBMC. To increase the specificity of the subtraction, the driver was spiked with genes that were determined to be most abundant in the mets3616A cDNA library, such as EF1-alpha, integrin-beta and anticoagulant protein PP4, as well as with cDNAs that were previously found to be differentially expressed in subtracted lung adenocarcinoma cDNA libraries. The determined cDNA sequences of 51 clones isolated from the subtracted library (referred to as mets3616A-S1) are provided in SEQ ID NO: 280-330.

Comparison of the sequences of SEQ ID NO: 255-330 with those in the public databases revealed no significant homologies to the sequences of SEQ ID NO: 255-258, 260, 262-264, 270, 272, 275, 276, 279, 281, 287, 291, 296, 300 and 310. The sequences of SEQ ID NO: 259, 261, 265-269, 271, 273, 274, 277, 278, 282-285, 288-290, 292, 294, 297-299, 301, 303-309, 313, 314, 316, 320-324 and 326-330 showed some homology to previously identified gene sequences, while the sequences of SEQ ID NO: 280, 286, 293, 302, 310, 312, 315,317.319 and 325 showed some homology to previously isolated expressed sequence tags (ESTs).

### EXAMPLE 2

### DETERMINATION OF TISSUE SPECIFICITY OF LUNG TUMOR POLYPEPTIDES

Using gene specific primers, mRNA expression levels for seven representative lung tumor polypeptides described in Example 1 were examined in a variety of normal and tumor tissues using RT-PCR.

Briefly, total RNA was extracted from a variety of normal and tumor tissues using Trizol reagent as described above.. First strand synthesis was carried out using 2 µg of total RNA with Superscript II reverse transcriptase (BRL Life Technologies) at 42°C for one hour. The cDNA was then amplified by PCR with gene-specific primers. To ensure the semi-quantitative nature of the RI-PCR, β-action was used as an internal control for each of the tissues examined. 1 µl of 1:30 dilution of cDNA was employed to enable the linear range amplification of the β-actin template and was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in five different types of tumor tissue (lung squamous cell carcinoma from 3 patients, lung adenocarcinoma, colon tumor from 2 patients, breast tumor and prostate tumor), and thirteen different normal tissues (lung from 4 donors, prostate, brain, kidney, liver, ovary, skeletal muscle, skin, small intestine, stomach, myocardium, retina and testes).. Using a 10-fold amount of cDNA, the antigen LST-S1-90 (SEQ ID NO: 3) was found to be expressed at high levels in lung squamous cell carcinoma and in breast tumor, and at low to undetectable levels in the other tissues examine.

The antigen LST-S2-68 (SEQ ID NO: 15) appears to be specific to lung and breast tumor, however, expression was also detected in normal kidney.. Antigens LST-S1-169 (SEQ ID NO: 6) and LSI-S1-133 (SEQ ID NO: 5) appear to be very abundant in lung tissues (both normal and tumor), with the expression of these two genes being decreased in most of the normal tissues tested.. Both LST-S1-169 and LST-S1-133 were also expressed in breast and colon tumors. Antigens LSI-S1-6 (SEQ ID NO: 7) and LST-S2-I2-5F (SEQ ID NO: 47) did not show tumor or tissue specific expression, with the expression of LST-S1-28 being rare and only detectable in a few tissues. The antigen LST-S3-7 (SEQ ID NO: 63) showed lung and breast tumor specific expression, with its message only being detected in normal testes when the PCR was performed for 30 cycles. Lower level expression was detected in some normal tissues when the cycle number was increased to 35.. Antigen LST-S3-13 (SEQ ID NO: 66) was found to be expressed in 3 out of 4 lung tumors, one breast tumor and both colon tumor samples. Its expression in normal tissues was lower compared to tumor, and was only detected in 1 out of 4 normal lung tissues and in normal tissues from kidney, ovary and retina Expression of antigens LST-S3-4 (SEQ ID NO: 62) and LST-S3-14 (SEQ ID NO: 67) was rare and did not show any tissue or tumor specificity.. Consistent with Northern blot analyses, the RT-PCR results on antigen LAT-S1-A-10A (SEQ ID NO: 78) suggested that its expression is high in lung, colon, stomach and small intestine tissues, including lung and colon tumors, whereas its expression was low or undetectable in other tissues.

A total of 2002 cDNA fragments isolated in lung subtractions I, II and III, described above, were colony PCR amplified and their mRNA expression levels in lung tumor, normal lung, and various other normal and tumor tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Seventeen non-redundant cDNA clones showed over-expression in lung squamous tumors, with expression in normal tissues tested (lung, skin, lymph node, colon, liver, pancreas, breast, heart, bone marrow, large intestine, kidney, stomach, brain, small intestine, bladder and salivary gland) being either undetectable, or 10-fold less compared to lung squamous tumors. The determined cDNA sequences for the clone L513S are provided in SEQ ID NO: 87 and 88; those for L514S are provided in SEQ ID NO: 89 and 90; those for L516S in SEQ ID NO: 91 and 92; that for L517S in SEQ ID NO: 93; that for L519S in SEQ ID NO: 94; those for L520S in SEQ ID NO: 95 and 96; those for L521S in SEQ ID NO: 97 and 98; that for L522S in SEQ ID NO: 99; that for L523S in SEQ ID NO: 100; that for L524S in SEQ ID NO: 101; that for L525S in SEQ ID NO: 102; that for L526S in SEQ ID NO: 103; that for L527S in SEQ ID NO: 104; that for L528S in SEQ ID NO: 105; that for L529S in SEQ ID NO: 106; and those for L530S in SEQ ID NO: 107 and 108. Additionally, the full-length cDNA sequence for L530S is provided in SEQ ID NO: 151, with the corresponding amino acid sequence being provided in SEQ ID NO: 152. L530S shows homology to a splice variant of a p53 tumor suppressor homologue, p63.. The cDNA sequences of 7 known isoforms of p63 are provided in SEQ ID NO: 331-337, with the corresponding amino acid sequences being provided in SEQ ID NO: 338-344, respectively

Due to polymorphisms, the clone L531S appears to have two forms. A first determined full-length cDNA sequence for L531S is provided in SEQ ID NO: 109, with the corresponding amino acid sequence being provided in SEQ ID NO: 110.. A second determined full-length cDNA sequence for L531S is provided in SEQ ID NO: 111, with the corresponding amino acid sequence being provided in SEQ ID NO: 112. The sequence of SEQ ID NO: 111 is identical to that of SEQ ID NO: 109, except that it contains a 27 bp insertions. Similarly, L514S has two alternatively spliced forms; the first variant cDNA is listed as SEQ ID NO: 153, with the corresponding amino acid sequence being provided in SEQ ID NO: 155. The full-length cDNA for the second variant form of L514S is provided in SEQ ID NO: 154, with the corresponding amino acid sequence being provided in SEQ ID NO: 156.

Full length cloning for L524S (SEQ ID NO: 101) yielded two variants (SEQ ID NO: 163 and 164) with the corresponding amino acid sequences of SEQ ID NO: 165 and 166, respectively. Both variants have been shown to encode parathyroid hormone-related peptide.

Attempts to isolate the full-length cDNA for L519S, resulted in the isolation of the extended cDNA sequence provided in SEQ ID NO: 173, which contains a potential open reading frame. The amino acid sequence encoded by the sequence of SEQ ID NO: 173 is provided in SEQ ID NO: 174. Additionally, the full-length cDNA sequence for the clone of SEQ ID NO: 100 (known as L523S), a known gene, is provided in SEQ ID NO: 175, with the corresponding amino acid sequence being provided in SEQ ID NO: 176. In further studies, a full-length cDNA sequence for L523S was isolated from a L523S-positive tumor cDNA library by PCR amplification using gene specific primers designed from the sequence of SEQ ID NO: 175. The determined full-length cDNA sequence is provided in SEQ ID NO: 347 The amino acid sequence encoded by this sequence is provided in SEQ ID NO: 348. This protein sequence differs from the previously published protein sequence at two amino acid positions, namely at positions 158 and 410.

Comparison of the sequences of L514S and L531S (SEQ ID NO: 87 and 88, and 109, respectively) with those in the gene bank, as described above, revealed no significant homologies to known sequences. The sequences of L513S, L516S, L517S, L519S, L520S and L530S (SEQ ID NO: 87 and 88, 91 and 92, 93, 94, 95 and 96, 107 and 108, respectively) were found to show some homology to previously identified ESTs. The sequences of L521S, L522S, L523S, L524S, L525S, L526S, L527S, L528S and L529S (SEQ ID NO: 97 and 98, 99, 99, 101, 102, 103, 104, 105, and 106, respectively) were found to represent known genes. The determined full-length cDNA sequence for L520S is provided in SEQ ID NO: 113, with the corresponding amino acid sequence being provided in SEQ ID NO: 114. Subsequent microarray analysis showed L520S to be overexpressed in breast tumors in addition to lung squamous tumors.

Further analysis demonstrated that L529S (SEQ ID NO: 106 and 115), L525S (SEQ ID NO: 102 and 120) and L527S (SEQ ID NO: 104) are cytoskeletal components and potentially squamous cell specific proteins. L529S is connexin 26, a gap junction protein. It was found to be highly expressed in one lung squamous tumor, referred to as 9688I, and moderately over expressed in two others. However, lower level expression of connexin 26 is also detectable in normal skin, colon, liver and stomach. The over-expression of connexin 26 in some breast tumors has been reported and a mutated form of L529S may result in over-expression in lung tumors. L525S is plakophilin 1, a desmosomal protein found in plaque-bearing adhering junctions of the skin. Expression levels for L525S mRNA was highly elevated in three out of four lung squamous tumors tested, and in normal skin. L527S has been identified as keratin 6 isoform, type II 58 Kd keratin and cytokeratin 13, and shows over-expression in squamous tumors and low expression in normal skin, breast and colon tissues. Keratin and keratin-related genes have been extensively documented as potential markers for lung cancer including CYFRA2.1 (Pastor, A, et al, Eur. Respir. J., 10:603-609, 1997). L513S (SEQ ID NO: 87 and 88) shows moderate over-expression in several tumor tissues tested, and encodes a protein that was first isolated as a pemphigus vulgaris antigen.

L520S (SEQ ID NO: 95 and 96) and L521S (SEQ ID NO: 97 and 98) are highly expressed in lung squamous tumors, with L520S being up-regulated in normal salivary gland and L521S being over-expressed in normal skin. Both belong to a family of small proline rich proteins and represent markers for fully differentiated squamous cells. L521S has been described as a specific marker for lung squamous tumor (Hu, R., et al, Lung Cancer, 20:25-30, 1998). L515S (SEQ ID NO: 162) encodes IGF-β2 and L516S is an aldose reductase homologue. Both are moderately expressed in lung squamous tumors and in normal colon. Notably, L516S (SEQ ID NO: 91 and 92) is up-regulated in metastatic tumors but not primary lung adenocarcinoma, an indication of its potential role in metatasis and a potential prognostic marker L522S (SEQ ID NO: 99) is moderately over-expressed in lung squamous tumors with minimum expression in normal tissues. L522S has been shown to belong to a class IV alcohol dehydrogenase, ADH7, and its expression profile suggests it is a squamous cell specific antigen. L523 S (SEQ ID NO: 100) is moderately over-expressed in lung squamous tumor, human pancreatic cancer cell lines and pancreatic cancer tissues, suggesting this gene may be a shared antigen between pancreatic and lung squamous cell cancer.

L524S (SEQ ID NO: 101) is over-expressed in the majority of squamous tumors tested and is homologous with parathyroid hoimone-related peptide (PTHrP), which is best known to cause humoral hypercalcaemia associated with malignant tumors such as leukemia, prostate and breast cancer. It is also believed that PTHrP is most commonly associated with squamous carcinoma of lung and rarely with lung adenocacinoma (Davidson, L.A, et al, J. Pathol., 178: 398-401, 1996). L528S (SEQ ID NO: 105) is highly over-expressed in two lung squamous tumors with moderate expression in two other squamous tumors, one lung adenocarcinoma and some normal tissues, including skin, lymph nodes, heart, stomach and lung. It encodes the NMB gene that is similar to the precursor of melanocyte specific gene Pmel17, which is reported to be preferentially expressed in low-metastatic potential melanoma cell lines. This suggests that LS28S may be a shared antigen in both melanoma and lung squamous cell carcinoma. L526S (SEQ ID NO: 103) was overexpressed in all lung squamous cell tumor tissues tested and has been shown to share homology with a gene (ATM) in which a mutation causes ataxia telangiectasia, a genetic disorder in humans causing a predisposition to cancer, among other symptoms. ATM encodes a protein that activates a p53 mediated cell-cycle checkpoint through direct binding and phosphorylation of the p53 molecule. Approximately 40% of lung cancers are associated with p53 mutations, and it is speculated that over-expression of ATM is a result of compensation for loss of p53 function, but it is unknown whether over-expression is the cause of result of lung squamous cell carcinoma. Additionally, expression of L526S (ATM) is also detected in a metastatic but not lung adenocarcinoma, suggesting a role in metastasis.

Expression of L523S (SEQ ID NO: 175), was examined by real time RT-PCR as described above. In a first study using a panel of lung squamous tumors, L523S was found to be expressed in 4/7 lung squamous tumors, 2/3 head and neck squamous tumors and 2/2 lung adenocarcinomas, with low level expression being observed in skeletal muscle, soft palate and tonsil. In a second study using a lung adenocarcinoma panel, expression of L523S was observed in 4/9 primary adenocarcinomas, 2/2 lung pleural effusions, 1/1 metastatic lung adenocarcinomas and 2/2 lung squamous tumors, with little expression being observed in normal tissues.

Expression of L523S in lung tumors and various normal tissues was also examined by Northern blot analysis, using standard techniques. In a first study, L523S was found to be expressed in a number of lung adenocarcinomas and squamous cell carcinomas, as well as normal tonsil. No expression was observed in normal lung. In a second study using a normal tissue blot (referred to as HB-12) from Clontech, no expression was observed in brain, skeletal muscle, colon, thymus, spleen, kidney, liver, small intestine, lung or PBMC, although there was strong expression in placenta.

### EXAMPLE 3

### ISOLATION AND CHARACTERIZATION OF LUNG TUMOR POLYPEPTIDES BY PCR-BASED SUBTRACTION

Eight hundred and fifty seven clones from a cDNA subtraction library, containing cDNA from a pool of two human lung squamous tumors subtracted against eight normal human tissue cDNAs including lung, PBMC, brain, heart, kidney, liver, pancreas, and skin, (Clontech, Palo Alto, CA) were derived and submitted to a first round of PCR amplification. This library was subjected to a second round of PCR amplification, following the manufacturer's protocol. The resulting cDNA fragments were subcloned into the P7-Adv vector (Clontech, Palo Alto, CA) and transformed into DH5α *E*. *coli* (Gibco, BRL). DNA was isolated from independent clones and sequenced using a Perkin Elmer/Applied Biosystems Division Automated Sequencer Model 373A.

One hundred and sixty two positive clones were sequenced. Comparison of the DNA sequences of these clones with those in the EMBL and GenBank databases, as described above, revealed no significant homologies to 13 of these clones, hereinafter referred to as Contigs 13, 16, 17, 19, 22, 24, 29, 47, 49, 56-59. The determined cDNA sequences for these clones are provided in SEQ ID NO: 125, 127-129, 131-133, 142, 144, 148-150, and 157, respectively.. Contigs 1, 3-5, 7-10, 12, 11, 15, 20, 31, 33, 38, 39, 41, 43, 44, 45, 48, 50, 53, 54 (SEQ ID NO: 115-124, 126, 130, 134-141, 143, 145-147, respectively) were found to show some degree of homology to previously identified DNA sequences. Contig 57 (SEQ ID NO: 149) was found to represent the clone L519S (SEQ ID NO: 94) disclosed in US. Patent Application No.. 09/123,912, filed July 27, 1998. To the best of the inventors' knowledge, none of these sequences have been previously shown to be differentially over-expressed in lung tumors.

mRNA expression levels for representative clones in lung tumor tissues, normal lung tissues (n=4), resting PBMC, salivary gland, heart, stomach, lymph nodes, skeletal muscle, soft palate, small intestine, large intestine, bronchial, bladder, tonsil, kidney, esophagus, bone marrow, colon, adrenal gland, pancreas, and skin (all derived from human) were determined by RT-PCR as described above. Expression levels using microarray technology, as described above, were examined in one sample of each tissue type unless otherwise indicated..

Contig 3 (SEQ ID NO: 116) was found to be highly expressed in all head and neck squamous cell tumors tested (17/17), and expressed in the majority (8/12) of lung squamous tumors, (high expression in 7/12, moderate in 2/12, and low in 2/12), while showing negative expression for 2/4 normal lung tissues and low expression in the remaining two samples. Contig 3 showed moderate expression in skin and soft palate, and lowered expression levels in resting PBMC, large intestine, salivary gland, tonsil, pancreas, esophagus, and colon. Contig 11 (SEQ ID NO: 124) was found to be expressed in all head and neck squamous cell tumors tested (17/17), with high levels of expression being seen in 14/17 tumors, and moderately levels of expression being seen in 3/17 tumors. Additionally, high expression was seen in 3/12 lung squamous tumors and moderate expression in 4/12 lung squamous tumors. Contig 11 was negative for 3/4 normal lung samples, with the remaining sample having only low expression. Contig 11 showed low to moderate reactivity to salivary gland, soft palate, bladder, tonsil, skin, esophagus, and large intestine. Contig 13 (SEQ ID NO: 125) was found to be expressed in all head and neck squamous cell tumors tested (17/17), with high expression in 12/17, and moderate expression in 5/17. Contig 13 was expressed in 7/12 lung squamous tumors, with high expression in 4/12 and moderate expression in three samples. Analysis of normal lung samples showed negative expression for 2/4 and low to moderate expression in the remaining two samples. Contig 13 showed low to moderate reactivity to resting PBMC, salivary gland, bladder, pancreas, tonsil, skin, esophagus, and large intestine, as well as high expression in soft palate.. Subsequent full-length cloning efforts revealed that contig 13 (also known as L761P) maps to the 3' untranslated region of the hSec10p gene. The full-length sequence for this gene is set forth in SEQ ID NO: 368, and encodes the protein set forth in SEQ ID NO: 369.

Contig 16 (SEQ ID NO: 127) was found to be moderately expressed in several head and neck squamous cell tumors (6/17) and one lung squamous tumor, while showing no expression in any normal lung samples tested. Contig 16 showed low reactivity to resting PBMC, large intestine, skin, salivary gland, and soft palate. Contig 17 (SEQ ID NO: 128) was shown to be expressed in all head and neck squamous cell tumors tested (17/17) (highly expressed in 5/17, and moderately expresses in 12/17). Determination of expression levels in lung squamous tumors showed one tumor sample with high expression and 3/12 with moderate levels. Contig 17 was negative for 2/4 normal lung samples, with the remaining samples having only low expression. Additionally, low level expression was found in esophagus and soft palate. Contig 19 (SEQ ID NO: 129) was found to be expressed in most head and neck squamous cell tumors tested (11/17); with two samples having high expression levels, 6/17 showing moderate expression, and low expression being found in 3/17. Testing in lung squamous tumors revealed only moderate expression in 3/12 samples. Expression levels in 2/4 of normal lung samples were negative, the two other samples having only low expression. Contig 19 showed low expression levels in esophagus, resting PBMC, salivary gland, bladder, soft palate and pancreas.

Contig 22 (SEQ ID NO: 131), was shown to be expressed in most head and neck squamous cell tumors tested (13/17) with high expression in four of these samples, moderate expression in 6/17, and low expression in 3/17. Expression levels in lung squamous tumors were found to be moderate to high for 3/12 tissues tested, with negative expression in two normal lung samples and low expression in two other samples (n=4).. Contig 22 showed low expression in skin, salivary gland and soft palate.. Similarly, Contig 24 (SEQ ID NO: 132) was found to be expressed in most head and neck squamous cell tumors tested (13/17) with high expression in three of these samples, moderate expression in 6/17, and low expression in 4/17. Expression levels in lung squamous tumors were found to be moderate to high for 3/12 tissues tested, with negative expression for three normal lung samples and low expression in one sample (n=4). Contig 24 showed low expression in skin, salivary gland and soft palate.. Contig 29 (SEQ ID NO: 133) was expressed in nearly all head and neck squamous cell tumors tested (16/17): highly expressed in 4/17, moderately expressed in 11/17, with low expression in one sample. Also, it was moderately expresses in 3/12 lung squamous tumors, while being negative for 2/4 normal lung samples Contig 29 showed low to moderate expression in large intestine, skin, salivary gland, pancreas, tonsil, heart and soft palate Contig 47 (SEQ ID NO: 142) was expressed in most head and neck squamous cell tumors tested (12/17): moderate expression in 10/17, and low expression in two samples. In lung squamous tumors, it was highly expressed in one sample and moderately expressed in two others (n=13). Contig 47 was negative for 2/4 normal lung samples, with the remaining two samples having moderate expression. Also, Contig 47 showed moderate expression in large intestine, and pancreas, and low expression in skin, salivary gland, soft palate, stomach, bladder, resting PBMC, and tonsil

Contig 48 (SEQ ID NO: 143) was expressed in all head and neck squamous cell tumors tested (17/17): highly expressed in 8/17 and moderately expressed in 7/17, with low expression in two samples. Expression levels in lung squamous tumors were high to moderate in three samples (n=13). Contig 48 was negative for one out of four normal lung samples, the remaining showing low or moderate expression.. Contig 48 showed moderate expression in soft palate, large intestine, pancreas, and bladder, and low expression in esophagus, salivary gland, resting PBMC, and heart. Contig 49 (SEQ ID NO: 144) was expressed at low to moderate levels in 6/17 head and neck squamous cell tumors tested. Expression levels in lung squamous tumors were moderate in three samples (n=13). Contig 49 was negative for 2/4 normal lung samples, the remaining samples showing low expression. Modeiate expression levels in skin, salivary gland, large intestine, pancreas, bladder and resting PBMC were shown, as well as low expression in soft palate, lymph nodes, and tonsil.. Contig 56 (SEQ ID NO: 148) was expressed in low to moderate levels in 3/17 head and neck squamous cell tumors tested, and in lung squamous tumors, showing low to moderate levels in three out of thirteen samples. Notably, low expression levels were detected in one adenocarcinoma lung tumor sample (n=2). Contig 56 was negative for 3/4 normal lung samples, and showed moderate expression levels in only large intestine, and low expression in salivary gland, soft palate, pancreas, bladder, and resting PBMC. Contig 58, also known as L769P, (SEQ ID NO: 150) was expressed at moderate levels in 11/17 head and neck squamous cell tumors tested and low expression in one additional sample. Expression in lung squamous tumors showed low to moderate levels in three out of thirteen samples. Contig 58 was negative for 3/4 normal lung samples, with one sample having low expression. Moderate expression levels in skin, large intestine, and resting PBMC were demonstrated, as well as low expression in salivary gland, soft palate, pancreas, and bladder. Contig 59 (SEQ ID NO: 157) was expressed in some head, neck, and lung squamous tumors. Low level expression of Contig 59 was also detected in salivary gland and large intestine.

The full-length cDNA sequence for Contig 22, also referred to as L763P, is provided in SEQ ID NO: 158, with the corresponding amino acid sequence being provided in SEQ ID NO: 159. Real-time RI-PCR analysis of L763P revealed that it is highly expressed in 3/4 lung squamous tumors as well as 4/4 head and neck squamous tumors, with low level expression being observed in normal brain, skin, soft pallet and trachea. Subsequent database searches revealed that the sequence of SEQ ID NO: 158 contains a mutation, resulting in a frameshift in the corresponding protein sequence. A second cDNA sequence for L763P is provided in SEQ ID NO: 345, with the corresponding amino acid sequence being provided in SEQ ID NO: 346. The sequences of SEQ ID NO: 159 and 346 are identical with the exception of the C-terminal 33 amino acids of SEQ ID NO: 159.

The full-length cDNA sequence incorporating Contigs 17, 19, and 24, referred to as L762P, is provided in SEQ ID NO: 160, with the corresponding amino acid sequence being provided in SEQ ID NO: 161. Further analysis of L762P has determined it to be a type I membrane protein and two additional variants have been sequenced. Variant 1 (SEQ ID NO: 167, with the corresponding amino acid sequence in SEQ ID NO: 169) is an alternatively spliced form of SEQ ID NO: 160 resulting in deletion of 503 nucleotides, as well as deletion of a short segment of the expressed protein. Variant 2 (SEQ ID NO: 168, with the corresponding amino acid sequence in SEQ ID NO: 170) has a two nucleotide deletion at the 3' coding region in comparison to SEQ ID NO: 160, resulting in a secreted form of the expressed protein. Real-time RI-PCR analysis of L762P revealed that is over-expressed in 3/4 lung squamous tumors and 4/4 head & neck tumors, with low level expression being observed in normal skin, soft pallet and trachea.

An epitope of L762P was identified as having the sequence KPGHWTYTLNNTHHSLQALK (SEQ ID NO: 382), which corresponds to amino acids 571-590 of SEQ ID NO:161.

The full-length cDNA sequence for contig 56 (SEQ ID NO: 148), also referred to as L773P, is provided in SEQ ID NO: 171, with the amino acid sequence in SEQ ID NO: 172. L773P was found to be identical to dihydroxyl dehydrogenase at the 3' portion of the gene, with divergent 5' sequence. As a result, the 69 N-terminal amino acids are unique. The cDNA sequence encoding the 69 N-teiminal amino acids is provided in SEQ ID NO: 349, with the N-terminal amino acid sequence being provided in SEQ ID NO: 350. Real-time PCR revealed that L773P is highly expressed in lung squamous tumor and lung adenocarcinoma, with no detectable expression in normal tissues. Subsequent Northern blot analysis of L773P demonstrated that this transcript is differentially over-expressed in squamous tumors and detected at approximately 1.6 Kb in primary lung tumor tissue and approximately 1.3 Kb in primary head and neck tumor tissue.

Subsequent microarray analysis has shown Contig 58, also referred to as L769S (SEQ ID NO: 150), to be overexpressed in breast tumors in addition to lung squamous tumors.

### EXAMPLE 4

### ISOLATION AND CHARACTERIZATION OF LUNG TUMOR POLYPEPTIDES BYPCR-BASED SUBTRACIION

Seven hundred and sixty clones from a cDNA subtraction library, containing cDNA from a pool of two human lung primary adenocarcinomas subtracted against a pool of nine normal human tissue cDNAs including skin, colon, lung, esophagus, brain, kidney, spleen, pancreas and liver, (Clontech, Palo Alto, CA) were derived and submitted to a first round of PCR amplification. This library (referred to as ALT-1) was subjected to a second round of PCR amplification, following the manufacturer's protocol. The expression levels of these 760 cDNA clones in lung tumor, normal lung, and various other normal and tumor tissues, were examined using microarray technology (Incyte, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. A total of 118 clones, of which 55 were unique, were found to be over-expressed in lung tumor tissue, with expression in normal tissues tested (lung, skin, lymph node, colon, liver, pancreas, breast, heart, bone marrow, large intestine, kidney, stomach, brain, small intestine, bladder and salivary gland) being either undetectable, or at significantly lower levels. One of these clones, having the sequence as provided in SEQ ID NO:420 (clone #19014), shows homology to a previously identified clone, L773P. Clone L773P has the full-length cDNA sequence provided in SEQ ID NO:171 and the amino acid sequence provided in SEQ ID NO:172 The isolation of clone #19014 is also described in co-pending U.S. Patent application 09/285,479, filed April 2,1999.

### EXAMPLE 5

### SYNTHESIS OF POLYPEPIDES

Polypeptides may be synthesized on a Perkin Elmer/Applied Biosystems Division 430A peptide synthesizer using FMOC chemistry with HPTU (O-Benzotriazole-N,N,N',N'-tetiamethyluronium hexafluorophosphate) activation. A Gly-Cys-Gly sequence may be attached to the amino terminus of the peptide to provide a method of conjugation, binding to an immobilized surface, or labeling of the peptide Cleavage of the peptides from the solid support is carried out using the following cleavage mixture: trifluoroacetic acid:ethanedithiol:thioanisole:water:phenol (40:1:2:2:3). After cleaving for 2 hours, the peptides are precipitated in cold methyl-t-butyl-ether. Ihe peptide pellets are then dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure factions, the peptides are characterized using electrospray or other types of mass spectrometry and by amino acid analysis

### EXAMPLE 6

### PREPARATION OF ANTIBODIES AGAINSI LUNG CANCER ANIIGENS

Polyclonal antibodies against the lung cancer antigens L514S, L528S, L531S, L523 and L773P (SEQ ID NO: 155, 225, 112, 176 and 171, respectively) were prepared as follows.

Rabbits were immunized with recombinant protein expressed in and purified from *E. coli* as described below. For the initial immunization, 400 µg of antigen combined with muramyl dipeptide (MDP) was injected subcutaneously (S.C). Animals were boosted S.C. 4 weeks later with 200 µg of antigen mixed with incomplete Freund's Adjuvant (IFA). Subsequent boosts of 100 µg of antigen mixed with IFA were injected S.C. as necessary to induce high antibody titer responses. Serum bleeds from immunized rabbits were tested for antigen-specific reactivity using ELISA assays with purified protein.. Polyclonal antibodies against L514S, L528S, L531S, L523S and L773P were affinity purified from high titer polyclonal sera using purified protein attached to a solid support.

Immunohistochemical analysis using polyclonal antibodies against L514S was performed on a panel of 5 lung tumor samples, 5 normal lung tissue samples and normal colon, kidney, liver, brain and bone marrow. Specifically, tissue samples were fixed in formalin solution for 24 hours and embedded in paraffin before being sliced into 10 micron sections. Tissue sections were permeabilized and incubated with antibody for 1 hr. HRP-labeled anti-mouse followed by incubation with DAB chromogen was used to visualize L514S immunoreactivity. L514S was found to be highly expressed in lung tumor tissue with little or no expression being observed in normal lung, brain or bone marrow Light staining was observed in colon (epithelial crypt cells positive) and kidney (tubules positive). Staining was seen in normal liver but no mRNA has been detected in this tissue making this result suspect.

Using the same procedure, immunohistochemical analysis using polyclonal antibodies against L528S demonstrated staining in lung tumor and normal lung samples, light staining in colon and kidney, and no staining in liver and heat.

Immunohistochemical analysis using polyclonal antibodies against L531S demonstrated staining in lung tumor samples, light membrane staining in most normal lung samples, epithelial staining in colon, tubule staining in kidney, ductal epithelial staining in liver and no staining in heart.

Immunohistochemical analysis using polyclonal antibodies against L523S demonstrated staining in all lung cancer samples tested but no staining in normal lung, kidney, liver, colon, bone marrow or cerebellum

Generation of polyclonal anti-sera against L762P (SEQ ID NO: 169 and 170) was performed as follows. 400 micrograms of lung antigen was combined with 100 micrograms of muramyldipeptide (MDP). An equal volume of Incomplete Freund's Adjuvant (IFA) was added and then mixed until an emulsion was formed. Rabbits were injected subcutaneously (S.C.). After four weeks the animals were injected S.C. with 200 micrograms of antigen mixed with an equal volume of IFA. Every four weeks animals were boosted with 100 micrograms of antigen Seven days following each boost the animal was bled. Sera was generated by incubating the blood at 4°C for 12-24 hours followed by centrifugation.

Characterization of polyclonal antisera was carried out as follows. Ninety six well plates were coated with antigen by incubing with 50 microliters (typically 1 microgram) at 4°C for 20 his. 250 microliters of BSA blocking buffer was added to the wells and incubated at room temperature for 2 hrs Plates were washed 6 times with PBS/0.01% Tween.. Rabbit sera was diluted in PBSand 50 microliters of diluted sera was added to each well and incubated at room temperature for 30 min. Plates were washed as described above before addition of 50 microliters of goat anti-rabbit horse radish peroxidase (HRP) at a 1:10000 dilution and incubation at room temperature for 30 min. Plates were washed as described above and 100µl of IMB Microwell Peroxidase Substrate was added to each well. Following a 15 minute incubation in the dark at room temperature, the colorimetric reaction was stopped with 100µl 1N H₂SO₄ and read immediately at 450 nm. Antisera showed strong reactivity to antigen L762P.

Immunohistochemical analysis using polyclonal antibodies against L762P demonstrated staining in all lung cancer samples tested, some light staining in the bronchiole epithelium of normal lung, tubule staining in kidney, light epithelial staining in colon and no staining in heart or liver.

In order to evaluate L773P protein expression in various tissues, immunohistochemistry (IHC) analysis was performed using an affinity purified L773P polyclonal antibody. Briefly, tissue samples were fixed in formalin solution for 12-24 hrs and embedded in paraffin before being sliced into 8 micron sections. Steam heat induced epitope retrieval (SHIER) in 0.1 M sodiuym citrate buffer (pH 6.0) was used for optimal staining conditions. Sections were incubated with 10% serum/PBS for 5 minutes. Primary antibody was added to each section for 25 minutes at indicated concentrations followed by 25 minute incubation with either anti rabbit or anti-mouse biotinylated antibody. Endogenous peroxidase activitiy was blocked by three 1.5 minute incubations with hydrogen peroxidase. The avidin biotin complex/horse radish peroxidase (ABC/HRP) system was used along with DAB chromogen to visualize L773P expression. Slides were counterstainied with hematoxylin to visualize cell nuclei Using this approach, L77.3P protein was detected in 6/8 lung tumors, 4/6 normal lung samples (very light staining in some cases), 1/1 kidney samples (very light staining), 0/1 heart samples, 1/1 colon samples (very light staining) and 0/1 liver samples.

### EXAMPLE 7

### PEPTIDE PRIMING OF MICE AND PROPAGATION OF CTL LINES

Immunogenic peptides from the lung cancer antigen L762P (SEQ ID NO: 161) for HLA-A2/K^{b}-restricted CD8+ T cells were identified as follows.

The location of HLA-A2 binding peptides within the lung cancer antigen L762P (SEQ ID NO: 161) was predicted using a computer program which predicts peptides sequences likely to being to HLA-A*0201 by fitting to the known peptide binding motif for HLA-A*0201 (Rupert et al. (1993) Cell 74:929; Rammensee et al., (1995) Immunogenetics 41:178-228). A series of 19 synthetic peptides corresponding to a selected subset of the predicted HLA-A*0201 binding peptides was prepared as described above.

Mice expressing the transgene for human HLA A2/K^{b} (provided by Dr L. Sherman, The Scripps Research Institute, La Jolla, CA) were immunized with the synthetic peptides, as described by Theobald et al.., Proc. Natl Acad. Sci. USA 92:11993-11997, 1995, with the following modifications. Mice were immunized with 50µg of L726P peptide and 120µg of an I-A^{b} binding peptide derived from hepatitis B virus protein emulsified in incomplete Freund's adjuvant.. Three weeks later these mice were sacrificed and single cell suspensions prepared. Cells were then resuspended at 7 x 10⁶ cells/ml in complete media (RPMI-1640; Gibco BRL, Gaithersburg, MD) containing 10% FCS, 2mM Glutamine (Gibco BRL), sodium pyruvate (Gibco BRL), non-essential amino acids (Gibco BRL), 2 x 10⁵ M 2-mercaptoethanol, 50U/ml penicillin and streptomycin, and cultured in the presence of irradiated (3000 rads) L762P peptide. (5µg/ml) and 10mg/ml B₂-microglobulin- (3 µg/ml) LPS blasts (A2 transgenic spleens cells cultured in the presence of 7µg/ml dextran sulfate and 25µg/ml LPS for 3 days).. After six days, cells (5 x 10⁵/ml) were restimulated with 2.5 x 10⁶/ml peptide-pulsed irradiated (20,000 rads) EL4A2Kb cells (Sherman et al, Science 258:815-818, 1992) and 5 x 10⁶/ml irradiated (3000 rads) A2/K^{b}-transgenic spleen feeder cells. Cells were cultured in the presence of 10U/ml IL-2. Cells were restimulated on a weekly basis as described, in preparation for cloning the line..

Peptide-specific cell lines were cloned by limiting dilution analysis with irradiated (20,000 rads) L762P peptide-pulsed EL4 A2Kb tumor cells (1 x 10⁴ cells/well) as stimulators and irradiated (3000 rads) A2/K^{b}-transgenic spleen cells as feeders (5 x 10⁵ cells/ well) grown in the presence of 10U/ml IL-2. On day 7, cells were restimulated as before On day 14, clones that were growing were isolated and maintained in culture.

Cell lines specific for the peptides L762P-87 (SEQ ID NO: 226; corresponding to amino acids 87-95 of SEQ ID NO: 161), L762P-145 (SEQ ID NO: 227; corresponding to amino acids 145-153 of SEQ ID NO: 161), L762P-585 (SEQ ID NO: 228; corresponding to amino acids 585-593 of SEQ ID NO: 161), L762P-425 (SEQ ID NO: 229; corresponding to amino acids 425-433 of SEQ ID NO: 161), L762P(10)-424 (SEQ ID NO: 2.30; corresponding to amino acids 424-433 of SEQ ID NO: 161) and L762P(10)-458 (SEQ ID NO: 231; corresponding to amino acids 458-467 of SEQ ID NO: 161) demonstrated significantly higher reactivity (as measured by percent specific lysis) against L762P peptide-pulsed EL4-A2/K^{b} tumor target cells than control peptide-pulsed EL4-A2/K^{b} tumor target cells.

### EXAMPLE 8

### IDENTIFICATION OF CD4 IMMUNOGENIC T CELL EPITOPES DERIVED FROM THE LUNG CANCER ANTIGEN L762P

CD4 T cell lines specific for the antigen L762P (SEQ ID NO: 161) were generated as follows.

A series of 28 overlapping peptides were synthesized that spanned approximately 50% of the L762P sequence. For priming, peptides were combined into pools of 4-5 peptides, pulsed at 20 micrograms/ml into dendritic cells for 24 hours. The dendritic cells were then washed and mixed with positively selected CD4+ T cells in 96 well U-bottomed plates. Forty cultures were generated for each peptide pool.. Cultures were restimulated weekly with fresh dendritic cells loaded with peptide pools.. Following a total of 3 stimulation cycles, cells were rested for an additional week and tested for specificity to antigen presenting cells (APC) pulsed with peptide pools using interferon-gamma ELISA and proliferation assays. For these assays, adherent monocytes loaded with either the relevant peptide pool or an irrelevant peptide were used as APC. T cell lines that appeared to specifically recognize L762P peptide pools both by cytokine release and proliferation were identified for each pool. Emphasis was placed on identifying T cells with proliferative responses. T cell lines that demonstrated either both L762P-specific cytokine secretion and proliferation, or strong proliferation alone were further expanded to be tested for recognition of individual peptides from the pools, as well as for recognition of recombinant L762P.. The source of recombinant L762P was *E. coli,* and the material was partially purified and endotoxin positive. These studies employed 10 micrograms of individual peptides, 10 or 2 micrograms of an irrelevant peptide, and 2 or 0.5 micrograms of either L762P protein or an irrelevant, equally impure, *E. coli* generated recombinant protein. Significant interferon-gamma production and CD4 T cell proliferation was induced by a number of L762P-derived peptides in each pool. The amino acid sequences for these peptides are provided in SEQ ID NO: 232-251. These peptides correspond to amino acids 661-680, 676-696, 526-545, 874-893, 811-830, 871-891, 856-875, 826-845, 795-815, 736-755, 706-725, 706-725, 691-710, 601-620, 571-590, 556-575, 616-635, 646-665, 631-650, 541-560 and 586-605, respectively, of SEQ ID NO: 161.

CD4 T cell lines that demonstrated specificity for individual L762P-derived peptides were further expanded by stimulation with the relevant peptide at 10 miciograms/ml. Two weeks post-stimulation, T cell lines were tested using both proliferation and IFN-gamma ELISA assays for recognition of the specific peptide A number of previously identified T cells continued to demonstrate L762P-peptide specific activity. Each of these lines was further expanded on the relevant peptide and, following two weeks of expansion, tested for specific recognition of the L762P-peptide in titration experiments, as well as for recognition of recombinant *E. coli*-derived L762P protein For these experiments, autologous adherent monocytes were pulsed with either the relevant L762P-derived peptide, an irrelevant mammaglobin-derived peptide, recombinant *E. coli*-derived L762P (approx. 50% pure), or an irrelevant *E. coli*-derived protein. The majority of T cell lines were found to show low affinity for the relevant peptide, since specific proliferation and IFN-gamma ratios dramatically decreased as L762P peptide was diluted.. However, four lines were identified that demonstrated significant activity even at 0..1 micrograms/ml peptide. Each of these lines (referred to as A/D5, D/F5, E/A7 and E/B6) also appeared to specifically proliferate in response to the *E. coli*-derived L762P protein preparation, but not in response to the irrelevant protein preparation. The amino acid sequences of the L762P-derived peptides recognized by these lines are provided in SEQ ID NO: 234, 249, 236 and 245, respectively. No protein specific IFN-gamma was detected for any of the lines. Lines A/D5, F/A7 and E/B6 were cloned on autologous adherent monocytes pulsed with the relevant peptide at 0.1 (A/D5 and E/A7) or 1 (D/F5) microgram/ml. Following growth, clones were tested for specificity for the relevant peptide. Numerous clones specific for the relevant peptide were identified for lines A/D5 and E/A7

### EXAMPLE 9

### PROTEIN EXPRESSION OF LUNG TUMOR-SPECIFIC ANTIGENS

### a) Expression of L514S in E. coli

The lung tumor antigen L514S (SEQ ID NO: 89) was subcloned into the expression vector pE32b at NcoI and NotI sites, and transformed into *E. coli* using standard techniques. The protein was expressed from residues 3-153 of SEQ ID NO: 89.. The expressed amino acid sequence and the corresponding DNA sequence are provided in SEQ ID NO: 252 and 253, respectively.

### b) Expression of L762P

Amino acids 32-944 of the lung tumor antigen L762P (SEQ ID NO: 161), with a 6X His Tag, were subcloned into a modified pET28 expression vector, using kanamycin resistance, and transformed into BL21 CodonPlus using standard techniques. Low to moderate levels of expression were observed. The determined DNA sequence of the L762P expression construct is provided in SEQ ID NO: 254.

### EXAMPLE 10

### IDENTIFICATION OF MHC CLASS II RESTRICTING ALLELE FOR L762P PEPIIDE-SPECIFIC RESPONSES

A panel of HLA mismatched antigen presenting cells (APC) were used to identify the MHC class II restricting allele for the L762P-peptide specific responses of CD4 T cell clones derived from lines that recognized L762P peptide and recombinant protein. Clones from two lines, AD-5 and EA-7, were tested as described below.. The AD-5 derived clones were found to be restricted by the HLA-DRB-1101 allele, and an EA-7 derived clone was found to be restricted by the HLA DRB-0701 or DQB1-0202 allele. Identification of the restriction allele allows targeting of vaccine therapies using the defined peptide to individuals that express the relevant class II allele. Knowing the relevant restricting allele will also enable clinical monitoring for responses to the defined peptide since only individuals that express the relevant allele will be monitored.

CD4 T cell clones derived from line AD-5 and EA-7 were stimulated on autologous APC pulsed with the specific peptide at 10 µg/ml, and tested for recognition of autologous APC (from donor D72) as well as against a panel of APC partially matched with D72 at class II alleles. Table 2 shows the HLA class typing of the APC tested. Adherent monocytes (generated by 2 hour adherence) from four different donors, referred to as D45, D187, D208, and D326, were used as APC in these experiments. Autologous APC were not included in the experiment. Each of the APC were pulsed with the relevant peptide (5a for AD-5 and 3e for 3A-7) or the irrelevant mammoglobin peptide at 10 µg/ml, and cultures were established for 10,000 I cells and about 20,000 APC/well. As shown in Table 3, specific proliferation and cytokine production could be detected only when partially matched donor cells were used as APC. Based on the MHC typing analysis, these results strongly suggest that the restricting allele for the L762-specific response of the AD-5 derived clones is HLA-DRB-1101 and for the EA-7 derived clone the restricting allele is HLA DRB-0701 or DQB1-0202.

**Table 2 - HLA Typing of APC**

| DONOR | DR | DR | DQ | DQ |
|---|---|---|---|---|
| D72 | B1-1101 | B1-0701 | B1-0202 | B1-0301 |
| D45 | -3 | -15 | B1-0201 | B1-0602 |
| D187 | -4 | -15 | 1 | -7 |
| D208 | B1-1101 | B1-0407 | -3 | -3 |
| D326 | B1-0301 | B1-0701 | B1-0202 | B1-0201 |

**Table 3 - L762P Peptide Responses Map to HLA DR Alleles**

| | AD-5 | | | | | | | | | | | | | | | | | | | | EA-7 | |
|---|---|---|---|---|---|---|---|---|---|---|---|---|---|---|---|---|---|---|---|---|---|---|
| | A11 | | B10 | | C10 | | C11 | | E6 | | F1 | | F9 | | G8 | | G9 | | G10 | | G12 | |
| Donor | Prol | γ- | Prol | γ-IFN | Prol | γ-IFN | Prol | γ-IFN | Prol | γ-IFN | Prol | γ-IFN | Prol | γ-IFN | Prol | γ-IFN | Prol | γ- IFN | Prol | γ- IFN | Prol | γ-IFN |
| D72 DR-0701, -1101, DQ-0202, -7 | 46 | | 31 | | 34 | | 24 | | 31 | | 40 | | 55 | | 45 | | 43 | | 91 | | 10 | |
| D45 DR-3,-15, DQ-1, -0201 | 3.2 | 1.7 | 5.5 | 1.2 | 33 | 1 | 1.0 | 1.5 | 1.1 | 1.1 | 1.6 | 1.1 | 1.4 | 1.3 | 0.2 | 1.1 | 1.1 | 1.1 | 1.2 | 1.5 | 0.8 | 1.1 |
| D187 DR-4, -15, DQ-1,-7 | 1.4 | 1.2 | 1.3 | 1 | 1.4 | 1.1 | 1.4 | 1.7 | 1.0 | 1.1 | 1.4 | 1.2 | 1.2 | 1.1 | 0.9 | 1 | 1.0 | 1 | 1.0 | 1.6 | 0.5 | 1 |
| D208 DR-4 -1101, DQ-3 | 138 | 13 | 38 | 5.4 | 18.8 | 10 | 14.6 | 4.6 | 15.3 | 6.1 | 45.9 | 8.6 | 73.3 | 14.1 | 38.0 | 7.7 | 174.3 | 16.1 | 113.6 | 19.6 | 0.8 | 1 |
| D326 DR-3, -0701, DQ-0202 | 0.7 | 4 | 0.3 | 1 | 0.3 | 1.4 | 1.0 | 2 | 0.8 | 1.1 | 0.3 | 1.1 | 0.7 | 1.1 | 0.6 | 1.2 | 0.4 | 1 | 1.2 | 5 | 14.1 | 6.8 |

### EXAMPLE 11

### FUSION PROTEINS OF N-TERMINAL AND C-TERMINAL PORTIONS OF L763P

In another embodiment, a *Mycobacterium tuberculosis*-derived polynucleotide, referred to as Ra12, is linked to at least an immunogenic portion of a polynucleotide of this invention. Ra12 compositions and methods for their use in enhancing expression of heterologous polynucleotide sequences are described in U.S. Patent Application 60/158,585, the disclosure of which is incorporated herein by reference in its entirety.. Briefly, Ra12 refers to a polynucleotide region that is a subsequence of a *Mycobacterium tuberculosis* MTB32A nucleic acid. MTB32A is a serine protease of 32 KD molecular weight encoded by a gene in virulent and avirulent strains of *M. tuberculosis.* The nucleotide sequence and amino acid sequence of MTB32A have been described (for example, U.S. Patent Application 60/158,585; see also, Skeiky et al., Infection and Immun. (1999) 67:3998-4007, incorporated herein by reference). Surprisingly, it was discovered that a 14 KD C-terminal fragment of the MTB32A coding sequence expresses at high levels on its own and remains as a soluble protein throughout the purification process. Moreover, this fragment may enhance the immunogenicity of heterologous antigenic polypeptides with which it is fused. This 14 KD C-terminal fragment of the MTB32A is referred to herein as Ra12 and represents a fragment comprising some or all of amino acid residues 192 to 323 of MTB32A

Recombinant nucleic acids which encode a fusion polypeptide comprising a Ra12 polypeptide and a heterologous lung tumor polypeptide of interest, can be readily constructed by conventional genetic engineering techniques. Recombinant nucleic acids are constructed so that, preferably, a Ra12 polynucleotide sequence is located 5' to a selected heterologous lung tumor polynucleotide sequence. It may also be appropriate to place a Ra12 polynucleotide sequence 3' to a selected heterologous polynucleotide sequence or to insert a heterologous polynucleotide sequence into a site within a Ra12 polynucleotide sequence..

In addition, any suitable polynucleotide that encodes a Ra12 or a portion or other variant thereof can be used in constructing recombinant fusion polynucleotides comprising Ra12 and one or more lung tumor polynucleotides disclosed herein. Preferred Ra12 polynucleotides generally comprise at least about 15 consecutive nucleotides, at least about 30 nucleotides, at least about 60 nucleotides, at least about 100 nucleotides, at least about 200 nucleotides, or at least about 300 nucleotides that encode a portion of a Ra12 polypeptide.

Ra12 polynucleotides may comprise a native sequence (*i*.*e*., an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide or a portion thereof.

Two specific embodiments of fusions between Ra12 and antigens of the present invention are described in this example.

### A. N-Terminal Portion of L763P

A fusion protein of full-length Ra12 and the N-terminal portion of L763P (referred to as L763P-N; amino acid residues 1-130 of SEQ ID NO: 159) was expressed as a single recombinant protein in E. coli. The cDNA for the N-terminal portion was obtained by PCR with a cDNA for the full length L763P and primers L763F3 (5' CGGCGAATTCATGGATTGGGGGACGCTGC; SEQ ID NO: 383) and 1763RV3 (5' CGGCCTCGAGTCACCCCTCTATCCGAACCTTCTGC; SEQ ID NO: 384). The PCR product with expected size was recovered from agarose gel, digested with restriction enzymes EcoRI and XhoI, and cloned into the corresponding sites in the expression vector pCRX1. The sequence for the fusion of full-length of Ra12 and L763P-N was confirmed by DNA sequencing.. The determined cDNA sequence is provided in SEQ ID NO:351, with the corresponding amino acid sequence being provided in SEQ ID NO: 352).

### B. C-Terminal Portion of L763P

A fusion protein of full-length Ra12 and the C-terminal portion of L763P (referred to as L763P-C; amino acid resides 100-262 of SEQ ID NO: 159) was expressed as a single recombinant protein in *E*. *coli.* The cDNA of the C-terminal portion of L763P was obtained by PCR with a cDNA for the full length of L763P and primers L763P4 (5' CGGCGAATTCCACGAACCACTCGCAAGTTCAG; SEQ ID NO: 385) and L763RV4 (5' CGGCTCGAG-TTAGCTTGGGCCTGTGATTGC; SEQ ID NO: 386). The PCR product with expected size was recovered from agarose gel, digested with restriction enzymes EcoRI and XhoI, and cloned into the corresponding sites in the expression vector pCRX1. The sequence for the fusion of full-length Ra12 and L763P-C was confirmed by DNA sequencing.. The determined DNA sequence is provided in SEQ ID NO:353, with the corresponding amino acid sequence being provided in SEQ ID NO: 354.

The recombinant proteins described in this example are useful for the preparation of vaccines, for antibody therapeutics, and for diagnosis of lung tumors.

### EXAMPLE 12

### EXPRESSION IN E. COLI OF L762P HIS TAG FUSION PROTEIN

PCR was performed on the L762P coding region with the following primers:
forward primer starting at amino acid 32.
PDM-278 5'ggagtacagcttcaagacaatggg 3' (SEQ ID NO:355) Tm 57°C.
Reverse primer including natural stop codon after amino acid 920, creating EcoRI site
PDM-280 5'ccatgggaattcattataataattttgttcc 3' (SEQ ID NO:356) IM55°C.

The PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 (DE3) pLys S and BL21 (DE3) CodonPlus RIL expression hosts.

The protein sequence of expressed recombinant L762P is shown in SEQ ID NO:357, and the DNA sequence is shown in SEQ ID NO:358.

### EXAMPLE 13

### EXPRESSION IN E. COLI OF A L773PA HIS TAG FUSION PROTEIN

The L773PA coding region (encoding amino acids 2-71 of SEQ ID NO: 172) was PCR amplified using the following primers:
Forward primer for L773PA starting at amino acid 2:
   PDM-299 5'tggcagcccctcttcttcaagtggc 3' (SEQ ID NO:359) Tm63°C.
   Reverse primer for L773PA creating artificial stop codon after amino acid 70:
      PDM-355 5'cgccagaattcatcaaacaaatctgttagcacc 3' (SEQ ID NO:360) Tm62°C.

The resulting PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and transformed into BL21 (DE3) pLys S and BL21 (DE3) CodonPlus RIL expression hosts.

The protein sequence of expressed recombinant L773PA is shown in SEQ ID NO:361, and the DNA sequence is shown in SEQ ID NO:362.

### EXAMPLE 14

### IDENTIFTCATION OF EPITOPES DERIVED FROM LUNG TUMOR SPECIFIC POLYPEPTIDES

A series of peptides from the L773P amino acid sequence (SEQ ID NO: 172) were synthesized and used in *in vitro* priming experiments to generate peptide-specific CD4 T cells. These peptides were 20-mers that overlapped by 15 amino acids and corresponded to amino acids 1-69 of the L773P protein. This region has been demonstrated to be tumor-specific. Following three *in vitro* stimulations, CD4 T cell lines were identified that produced IFNγ in response to the stimulating peptide but not the control peptide.. Some of these T cell lines demonstrated recognition of recombinant L773P and L773PA (tumor-specific region) proteins.

To perform the experiments, a total of eleven 20-mer peptides (SEQ ID NO: 363, 365 and 387-395) overlapping by 15 amino acids and derived from the N-terminal tumor-specific region of L773P (corresponding to amino acids 1-69 of SEQ ID NO:172) were generated by standard procedures. Dendritic cells were derived from PBMC of a normal donor using GMCSF and IL-4 by standard protocol. Purified CD4 T cells were generated from the same donor as the dendritic cells using MACS beads and negative selection of PBMCs. Dendritic cells were pulsed overnight with the individual 20-mer peptides at a concentration of 10 µg/ml. Pulsed dendritic cells were washed and plated at 1 x 10⁴/well of a 96-well U-bottom plates, and purified CD4 cells were added at 1 x 10⁵ well.. Cultures were supplemented with 10 ng/ml IL-6 and 5 ng/ml IL-12, and incubated at 37°C. Cultures were re-stimulated as above on a weekly basis using as APC dendritic cells generated and pulsed as above, supplemented with 5 ng/ml IL-7 and 10 µg/ml IL-2. Following 3 *in vitro* stimulation cycles, cell lines (each corresponding to one well) were tested for cytokine production in response to the stimulating peptide vs. an irrelevant peptide.

A small number of individual CD4 T cell lines (9/528) demonstrated cytokines release (IFNγ) in response to the stimulating peptide but not to control peptide.. The CD4 I cell lines that demonstrated specific activity were restimulated on the appropriate L773P peptide and reassayed using autologous dendritic cells pulsed with 10 µg/ml of the appropriate L773P peptide, an irrelevant control peptide, recombinant L773P protein (amino acids 2-364, made in *E*. *coli*), recombinant L773PA (amino acids 2-71, made in *E. coli*), or an appropriate control protein (L3E, made in *E*. *coli*). Three of the nine lines tested (1-3C, 1-6G, and 4-12B) recognized the appropriate L773P peptide as well as recombinant L773P and L773PA. Four of the lines tested (4-8A, 4-8E, 4-12D, and 4-12E) recognized the appropriate L773P peptide only. Two of the lines tested (5-6F and 9-3B) demonstrated non-specific activity.

These results demonstrate that the peptide sequences MWQPLFFKWLLSCCPGSSQI (amino acids 1-20 of SEQ ID NO: 172; SEQ ID NO:363) and GSSQIAAAASTQPEDDINTQ (amino acids 16-35 of SEQ ID NO: 172; SEQ ID NO: 365) may represent naturally processed epitopes of L773P, which are capable of stimulating human class II MHC-restricted CD4 I cell responses.

In subsequent studies, the above epitope mapping experiment was repeated using a different donor. Again, some of the resulting I cell lines were found to respond to peptide and recombinant protein. An additional peptide was found to be naturally processed. Specifically, purified CD4 cells were stimulated on a total of eleven 20 mer peptides overlapping by 15 amino acids (SEQ ID NO: 363, 387, 388, 365 and 389-395, respectively). The priming was carried out as described above, except that a peptide concentration of 0..5 ug/mL rather than 10 ug/mL was employed In the initial screen of the cell lines 9 of the 528 lines released at least a three-fold greater level of IFN-gamma with stimulating peptide vs. control peptide. These 9 lines were restimulated on the appropriate peptide and then tested on dendritic cells pulsed with a titration of appropriate peptide (10 µg/mL, 1 µg/mL and 0.1 ug/mL), and 10 µg/mL of a control peptide. Six of the 9 lines recognized recombinant L773P as well as peptide. The six lines referred to as 1-1E, 1-2E, 1-4H, 1-6A, 1-6G and 2-12B recognized L773PA and the appropriate peptide. These results demonstrate that the peptides of SEQ ID NO: 363 and 387 represent naturally processed epitopes of L773P.

Using the procedures described above, CD4+ T cell responses were generated from PBMC of normal donors using dendritic cells pulsed with overlapping 20-mer peptides (SEQ ID NO: 396-419) spanning the L523S polypeptide sequence (SEQ ID NO: 176). A number of CD4+ T cells demonstrated reactivity with the priming peptides as well as with L523S recombinant protein, with the dominant reactivity of these lines being within the peptides 4, 7 and 21 (SEQ ID NO: 399, 402 and 416; corresponding to amino acids 30.39, 60-79 and 200-219, respectively, of SEQ ID NO: 176).

Epitopes within the scope of the invention include epitopes restricted by other class II MHC molecules. In addition, variants of the peptide can be produced wherein one or more amino acids are altered such that there is no effect on the ability of the peptides to bind to MHC molecules, no effect on their ability to elicit T cell responses, and no effect on the ability of the elicited T cells to recognize recombinant protein.

### EXAMPLE 15

### SURFACE EXPRESSION OF L762P AND ANTIBODY EPITOPES THEREOF

Rabbits were immunized with full-length histidine-tagged L762P protein generated in *E*. *coli.* Sera was isolated from rabbits and screened for specific recognition of L762P in ELISA assays. One polyclonal serum, referred to as 2692L, was identified that specifically recognized recombinant L762P protein. The 2692L anti-L762P polyclonal antibodies were purified from the serum by affinity purification using L762P affinity columns. Although L762P is expressed in a subset of primary lung tumor samples, expression appears to be lost in established lung tumor cell lines. Therefore, to characterize surface expression of L762P, a retrovirus construct that expresses L762P was used to transduce primary human fibroblasts as well as 3 lung tumor cell lines (522-23, HTB, and 343T). Transduced lines were selected and expanded to examine L762P surface expression by FACS analysis.. For this analysis, non-transduced and transduced cells were harvested using cell dissociation medium, and incubated with 10-50 microgiams/ml of either affinity purified anti-L762P or irrelevant antisera. Following a 30 minute incubation on ice, cells were washed and incubated with a secondary, FITC conjugated, anti rabbit IgG antibody as above. Cells were washed, resuspended in buffer with Propidium Iodide (PI) and examined by FACS using an Excalibur fluorescence activated cell sorter. For FACS analysis, PI-positive (i.e. dead/permeabilized cells) were excluded. The polyclonal anti-L762P sera specifically recognized and bound to the surface of L762P-transduced cells but not the non-transduced counterparts. These results demonstrate that L762P is localized to the cell surface of both fibroblasts as well as lung tumor cells.

To identify the peptide epitopes recognized by 2692L, an epitope mapping approach was pursued. A series of overlapping 19-21 mers (5 amino acid overlap) was synthesized that spanned the C terminal portion of L762P (amino acids 481-894 of SEQ ID NO: 161). In an initial experiment peptides were tested in pools-Specific reactivity with the L762P antiserum was observed with pools A, B, C, and E. To identify the specific peptides recognized by the antiserum, flat bottom 96 well microtiter plates were coated with individual peptides at 10 microgram/ml for 2 hours at 37 °C. Wells were then aspirated and blocked with phosphate buffered saline containing 5% (w/v) milk for 2 hours at 37 °C, and subsequently washed in PBS containing 0.1% Tween 20 (PBST). Purified rabbit anti-L762P serum 2692L was added at 200 or 20 ng/well to triplicate wells in PBST and incubated overnight at room temperature. This was followed by washing 6 times with PBST and subsequently incubating with HRP-conjugated donkey anti rabbit IgG (H+L)Affinipure F(ab') fragment at 1:2,000 for 60 minutes. Plates were then washed, and incubated in tetramethyl benzidine substrate. Reactions were stopped by the addition of 1N sulfuric acid and plates were read at 450/570 nm using an ELISA plate reader..

The resulting data, presented in Table 4 below, demonstrates that the L762P antisera recognized at least 6 distinct peptide epitopes from the 3' half of L762P.

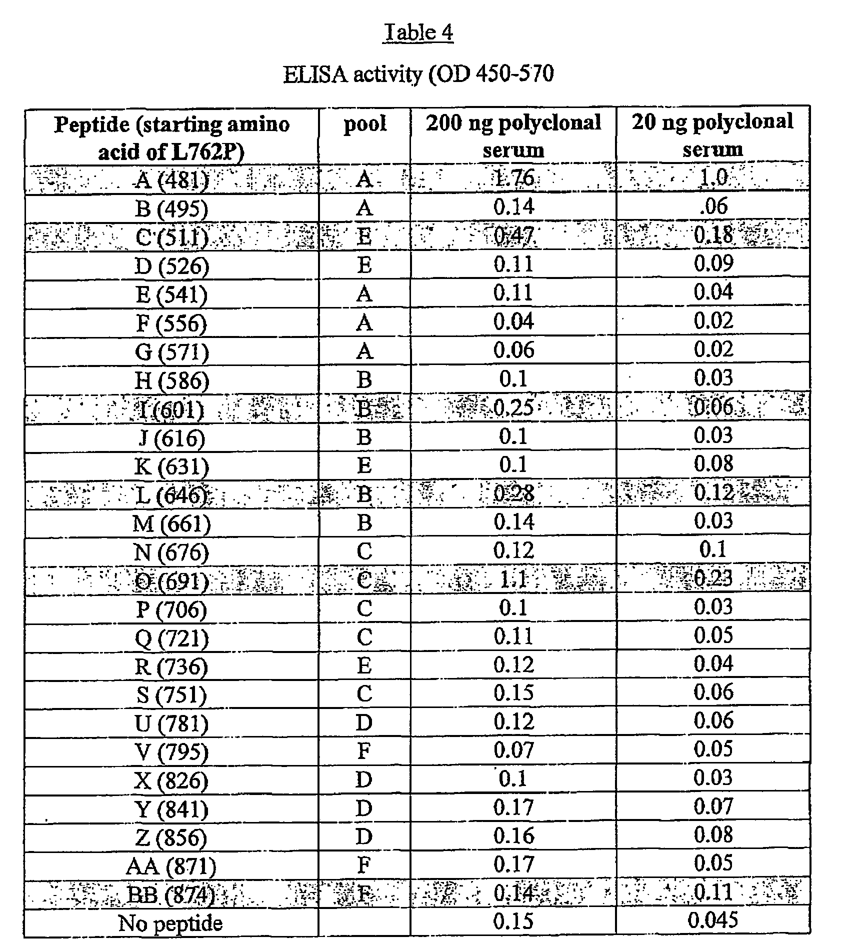

Individual peptides were identified from each of the pools, and additionally a weak reactivity was identified with peptide BB from pool F. The relevant peptide epitopes are summarized in the Table 5 below The amino acid sequences for peptides BB, O, L, I, A and C are provided in SEQ ID NO: 376-381, respectively, with the corresponding cDNA sequences being provided in SEQ ID NO: 373, 370, 372, 374, 371 and 375, respectively.

**Table 5**

| ELISA activity (OD 450-570) | | | | | | |
|---|---|---|---|---|---|---|
| **Peptide** | **Nucleotides of L762P** | **Amino acids of L762P** | **Sequence** | **pool** | **200 ng** | **20 ng** |
| A | 1441-1500 | 481-500 | SRISSGTGDIFQQHIQLEST | A | 1.76 | 1.0 |
| C | 1531-1590 | 511-530 | KNTVTVDNTVGNDTMFLVTW | E | 0.47 | 0.18 |
| I | 1801-1860 | 601-620 | AVPPATVEAFVERDSLHFPH | B | 0.25 | 0.06 |
| L | 1936-1955 | 646-665 | PETGDPVTLRLLDDGAGADV | B | 0.28 | 0.12 |
| O | 2071-2130 | 691-710 | VNHSPSISTPAHSIPGSHAMIL | C | 1.1 | 0.23 |
| BB | 2620-2679 | 874-893 | LQSAVSNIAQAPLFIPPNSD | F | 0.14 | 0.11 |
| None | - | - | - | - | 0.15 | 0.05 |

### EXAMPLE 16

### DETECTION OF ANTIBODIES AGAINST LUNG TUMOR ANTIGENS IN PATIENT SERA

Antibodies specific for the lung tumor antigens L773PA (SEQ ID NO:361), L514S (SEQ ID NO:155 and 156), L523S (SEQ ID NO:176), L762P (SEQ ID NO:161) and L763P (SEQ ID NO:159) were shown to be present in effusion fluid or sera of lung cancel patients but not in normal donors. More specifically, the presence of antibodies against L773PA, L514S, L523S, L762P and L763P in effusion fluid obtained from lung cancer patients and in seta from normal donors was detected by ELISA using recombinant proteins and HRP-conjugated anti-human Ig. Briefly, each protein (100 ng) was coated in 96-well plate at pH 9.5. In parallel, BSA (bovine serum albumin) was also coated as a control protein. The signals ([S], absorbance measured at 405 nm) against BSA ([N]) were determined. The results of these studies are shown in Table 6, wherein - represents [S]/[N] < 2; +/- represents [S]/[N] >2; ++ represents [S]/[N] >3; and +++ represents [S]/[N] >5.

**Table 6**

| Detection of Antibodies Against Lung Tumor Antigens | | | | | |
|---|---|---|---|---|---|
| | L514S | L523S | L762P | L763P | L773PA |
| Effusion fluid | | | | | |
| #1 | +++ | ++ | ++ | - | ++ |
| #2 | - | - | +/- | ++ | +/- |
| #3 | - | - | - | - | +/- |
| #4 | +/- | ++ | +/- | - | +/- |
| #5 | +/- | +++ | +/- | +/- | ++ |
| #7 | - | +/- | - | - | +/- |
| #8 | - | +++ | - | - | ++ |
| #10 | - | ++ | +/- | +/- | - |
| #11 | +/- | ++ | ++ | - | ++ |
| #12 | +++ | +/- | - | +/- | +/- |
| #13 | - | +/- | | - | +/- |
| #14 | - | +++ | +/- | +/- | ++ |
| #15 | +/- | ++ | +/- | - | ++ |
| #17 | - | +/- | - | - | +/- |
| #18 | - | ++ | - | - | - |
| #19 | - | +/- | - | - | +/- |
| #20 | +1- | +/- | +/- | - | +/- |

| Normal sera | | | | | |
|---|---|---|---|---|---|
| #21 | - | +/- | - | - | - |
| #22 | - | - | - | - | - |
| #23 | - | - | - | - | +/- |
| #24 | - | +/- | - | - | - |
| #25 | +/- | +/- | - | - | +/- |

Using Western blot analyses, antibodies against L523S were found to be present in 3 out of 4 samples of effusion fluid from lung cancel patients, with no L523S antibodies being detected in the three samples of normal sera tested..

### EXAMPLE 17

### EXPRESSION IN E. COLI OF A L514S HIS TAG FUSION PROTEIN

PCR was performed on the L514S-13160 coding region with the following primers:
Forward primer PDM-278 5' cacactagtgtccgcgtggcggcctac 3' (SEQ ID NO:421) Tm 67°C.
Reverse primer PDM-280 5' catgagaattcatcacatgcccttgaaggctccc 3' (SEQ ID NO:422) TM 66°C.

The PCR conditions were as follows:
10µl 10X Pfu buffer
1.0µl 10mM dNTPs
2.0µl 10µM each pinner
83µl sterile water
1.5µl Pfu DNA polymerase (Stratagene, La Jolla, CA)
50ηg DNA
96°C for 2 minutes, 96°C for 20 seconds, 66°C for 15 seconds, 72°C for 1 minute with 40 cycles and then 72°C for 4 minutes..

The PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 CodonPlus (Stratagene, La Jolla, CA) cells for expression.

The amino acid sequence of expressed recombinant L514S is shown in SEQ ID NO:423, and the DNA coding region sequence is shown in SEQ ID NO:424.

### EXAMPLE 18

### EXPRESSION IN E. COLI OF A L523S HIS TAG FUSION PROTEIN

PCR was performed on the L523S coding region with the following primers:
Forward primer PDM-414 5' aacaaactgtatatcggaaacctcagcgagaa 3' (SEQ ID NO:425) Tm 62°C..
Reverse primer PDM-415 5' ecatagaattcattacttccgtcttgactgagg 3' (SEQ ID NO:426) TM 62°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50ηg DNA
96°C for 2 minutes, 96°C for 20 seconds, 62°C for 15 seconds, 72°C for 4 minutes with 40 cycles and then 72°C for 4 minutes..

The PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 CodonPlus (Stratagene, La Jolla, CA) cells for expression.

The amino acid sequence of expressed recombinant L523S is shown in SEQ ID NO:427, and the DNA coding region sequence is shown in SEQ ID NO:428.

### EXAMPLE 19

### EXPRESSION IN E. COLI OF A L762PA HIS TAG FUSION PROTEIN

PCR was performed on the L762PA coding region (L762PA is missing the signal sequence, the C-terminal transmembrane domain and the cytoplasmic tail) with the following primers:
Forward primer PDM-278 5'ggagtacagcttcaagacaatggg 3' (SEQ ID NO:355) Tm 57°C.
Reverse primer PDM-279 5'ccatggaattcattatttcaatataagataatctc 3' (SEQ ID NO:429) IM56°C.

The PCR conditions were as follows:
10µl 10X Pfu buffer
10µl 10mM dNTPs
2.0µl 10µM each primer
83µl sterile water
1.5µl Pfu DNA polymerase (Stratagene, La Jolla, CA)
50ηg DNA
96°C for 2 minutes, 96°C for 20 seconds, 55°C for 15 seconds, 72°C for 5 minutes with 40 cycles and then 72°C for 4 minutes.

The PCR product was digested with EcoRI restriction enzyme, gel purified and then cloned into pPDM His, a modified pET28 vector with a His tag in frame, which had been digested with Eco72I and EcoRI restriction enzymes.. The correct construct was confirmed by DNA sequence analysis and then transformed into BL21 pLys S (Novagen, Madison, WI) cells for expression.

The amino acid sequence of expressed recombinant L762PA is shown in SEQ ID NO:430, and the DNA coding region sequence is shown in SEQ ID NO:431.

### EXAMPLE 20

### EXPRESSION IN E. COLI OF A L773P HIS TAG FUSION PROTEIN

PCR was performed on the L773P coding region with the following primers:
Forward primer PDM-299 5' tggcagcccctcttcttcaagtggc 3' (SEQ ID NO:359) Tm 63°C
Reverse primer PDM-300 5' cgcctgctcgagtcattaatattcatcagaaaatgg 3' (SEQ ID NO:432) TM 63°C.

The PCR conditions were as follows:
10µl 10X Pfu buffer
1.0µl 10mM dNTPs
20µl 10µM each primer
83µl sterile water
1.5µl Pfu DNA polymerase (Stratagene, La Jolla, CA)
50ηg DNA
96°C for 2 minutes, 96°C for 20 seconds, 63°C for 15 seconds, 72°C for 2 minutes 15 seconds with 40 cycles and then 72°C for 4 minutes.

Ihe PCR product was digested with EcoRI restriction enzyme, gel purified and then cloned into pPDM His, a modified pET28 vector with a His tag in frame, which had been digested with Eco72I and EcoRI restriction enzymes. The correct construct was confirmed by DNA sequence analysis and then transformed into BL21 pLys S (Novagen, Madison, WI) and BL21 CodonPlus (Stratagene, La Jolla, CA) cells for expression.

The amino acid sequence of expressed recombinant L773P is shown in SEQ ID NO:433, and the DNA coding region sequence is shown in SEQ ID NO:434

### EXAMPLE 21

### CLONING AND SEQUENCING OF A T-CELL RECEPTOR CLONE FOR THE LUNG SPECIFIC ANIIGEN L762P

T cell receptor (TCR) alpha and beta chains from a CD4 T cell clone specific for the lung specific antigen L762P were cloned and sequence. Basically, total mRNA from 2 X 10⁶ cells from CTL clone 4H6 was isolated using Trizol reagent and cDNA was synthesized using Ready-to go kits (Pharmacia). To determine Valpha and Vbeta sequences of this clone, a panel of Valpha and Vbeta subtype specific primers was synthesized and used in RT-PCR reactions with cDNA generated from each of the clones. The RT-PCR reactions demonstrated that each of the clones expressed a common Vbeta sequence that corresponded to the Vbeta8 subfamily and a Valpha sequence that corresponded to the Valpha8 subfamily. To clone the full TCR alpha and beta chains from clone 4H6, primers were designed that spanned the initiator and terminator-coding TCR nucleotides.. The primers were as follows:
forward primer for TCR Valpha8 5' ggatccgccgccaccatgacatccattcgagctgta 3' (SEQ ID NO:435; has a BamHI site inserted);
Kozak reverse primer for TCR Valpha8 (antisense) 5' gtcgactcagctggaccacagccgcag 3' (SEQ ID NO:436; has a SalI site inserted plus the TCR alpha constant sequence);
forward primer for TCR Vbeta8 (sense) 5' ggatccgccgccaccatggactcctggaccttctgct 3' (SEQ ID NO:437; has a BamHI site inserted); and
Kozak reverse primer for TCR Vbeta 5' gtcgactcagaaatcctttctcttgac 3' (SEQ ID NO:438; has a SalI site inserted plus the TCR beta constant sequence).
Standard 35 cycle RT-PCR reactions were established using the cDNA synthesized from the CIL clone and the above primers utilizing the proofreading thermostable polymerase, PWO (Roche). The resultant PCR band, about 850 bp for Valpha and about 950 for Vbeta, was ligated into a PCR blunt vector (Invitrogen) and transformed into *E*. *coli. E. coli* transformed with plasmids having full-length alpha and beta chains were identified. Large scale preparations of the corresponding plasmids were generated, and these plasmids were sequenced. The Valpha sequence (SEQ ID NO:439) was shown by nucleotide sequence alignment to be homologous to Valpha8.1, while the Vbeta sequence (SEQ ID NO:440) was shown by nucleotide sequence alignment to be homologous to Vbeta8.2.

### EXAMPLE 22

### RECOMBINANT EXPRESSION OF FULL LENGTH L762P IN MAMMALIAN CELLS

Full length L762P cDNA was subcloned into the mammalian expression vectors VR1012 and pCEP4 (Invitrogen). Both expression vectors had previously been modified to contain a FLAG epitope tag. These constructs were transfected into HEK293 and CHL-1 cells (ATCC) using Lipofectamine 2000 reagent (Gibco).. Briefly, both the HEK and CHL-1 cells were plated at a density of 100,000 cells/ml in DMEM (Gibco) containing 10% FBS (Hyclone) and grown overnight. The following day, 4µl of Lipofectamine 2000 was added to 100µl of DMEM containing no FBS and incubated for 5 minutes at room temperature. The Lipofectamine/DMEM mixture was then added to 1µg of L762P Flag/pCEP4 or L762P Flag/VR1012 plasmid DNA resuspended in 100µl DMEM and incubated for 15 minutes at room temperature. The Lipofectamine/DNA mix was then added to the HEK293 and CHL-1 cells and incubated for 48-72 hours at 37°C with 7% CO₂. Cells were rinsed with PBS, then collected and pelleted by centrifugation. L672P expression was detected in the transfected HEK293 and CHL.1 cell lysates by Western blot analysis and was detected on the surface of transfected HEK cells by flow cytometry analysis.

For Western blot analysis, whole cell lysates were generated by incubating the cells in Triton-X100 containing lysis buffer for 30 minutes on ice. Lysates were then cleared by centrifugation at 10,000 rpm for 5 minutes at 4°C. Samples were diluted with SDS-PAGE loading buffer containing beta-mercaptoethanol, then boiled for 10 minutes prior to loading the SDS-PAGE gel. The protein was transferred to nitrocellulose and probed using 1 µg/ml purified anti-L762P rabbit polyclonal sera (lot #690/73) or non-diluted anti-L762P mAb 153.20.1 supernatant. Blots were revealed using either goat anti-rabbit Ig coupled to HRP or goat anti-mouse Ig coupled to HRP followed by incubation in ECL substrate.

For flow cytometric analysis, cells were washed further with ice cold staining buffer (PBS+1%BSA +Azide) Next, the cells were incubated for 30 minutes on ice with 10ug/ml of purified anti-L762P polyclonal sera (lot #690/73) or a 1:2 dilution of anti-L762P mAb 153.20.1 supernatant. The cells were washed 3 times with staining buffer and then incubated with a 1:100 dilution of goat anti-rabbit Ig(H+L)-FTTC or goat anti-mouse Ig(H+L)-FITC reagent (Southern Biotechnology) for 30 minutes on ice. After 3 washes, the cells were resuspended in staining buffer containing propidium iodide (PI), a vital stain that allows for the exclusion of permeable cells, and analyzed by flow cytometry.

### EXAMPLE 23

### GENERATION OF POLYCLONAL ANTIBODIES TO LUNG TUMOR ANTIGENS

Three lung antigens, L523S (SEQ ID NO:176), L763P (SEQ ID NO:159) and L763 peptide #2684 (SEQ ID NO:441), were expressed and purified for use in antibody generation.

L523S and L763P were expressed in an *E*. *coli* recombinant expression system and grown overnight in LB Broth with the appropriate antibiotics at 37°C in a shaking incubator. The next morning, 10 ml of the overnight culture was added to 500 ml of 2x YT with the appropriate antibiotics in a 2L-baffled Erlemneyer flask. When the optical density of the culture reached 0.4-0.6 at 560 nanometers, the cells were induced with IPIG (1 mM). Four hours after induction with IPTG, the cells were harvested by centrifugation.

The cells were then washed with phosphate buffered saline and centrifuged again. The supernatant was discarded and the cells were either frozen for future use or immediately processed. Twenty milliliters of lysis buffer was added to the cell pellets and vortexed. To break open the *E*. *coli* cells, this mixture was then run through a french press at a pressure of 16,000 psi.. The cells were then centrifuged again and the supernatant and pellet were checked by SDS-PAGE for the partitioning of the recombinant protein.

For proteins that localized to the cell pellet, the pellet was resuspended in 10 mM Tris pH 8.0, 1% CHAPS and the inclusion body pellet was washed and centrifuged again. This procedure was repeated twice more. The washed inclusion body pellet was solubilized with either 8M urea or 6M guanidine HCl containing 10 mM Tris pH 8.0 plus 10 mM imidazole. The solubilized protein was added to 5 ml of nickel chelate resin (Qiagen) and incubated for 45 minutes to 1 hour at room temperature with continuous agitation.

After incubation, the resin and protein mixture was poured through a disposable column and the flow through was collected The column was then washed with 10-20 column volumes of the solubilization buffer. The antigen was then eluted from the column using 8M urea, 10 mM Tris pH 8.0 and 300 mM imidazole and collected in 3 ml fractions. A SDS-PAGE gel was run to determine which fractions to pool for further purification.

As a final purification step, a strong anion exchange resin, in this case Hi-Prep Q (Biorad), was equilibrated with the appropriate buffer and the pooled fractions from above were loaded onto the column. Each antigen was eluted off the column with an increasing salt gradient. Fractions were collected as the column was run and another SDS-PAGE gel was run to determine which fractions from the column to pool.

The pooled fractions were dialyzed against 10 mM Tris pH 8.0. The release criteria were purity as determined by SDS-PAGE or HPLC, concentration as determined by Lowry assay or Amino Acid Analysis, identity as determined by amino terminal protein sequence, and endotoxin level was determined by the Limulus (LAL) assay. The proteins were then put in vials after filtration through a 0.22-micron filter and the antigens were frozen until needed for immunization.

The L763 peptide #2684 was synthesized and conjugated to KLH and froze until needed for immunization.

The polyclonal antisera were generated using 400 micrograms of each lung antigen combined with 100 micrograms of muramyldipeptide (MDP).. An equal volume of Incomplete Freund's Adjuvant (IFA) was added and then mixed and injected subcutaneously (S.C.) into a rabbit. After four weeks, the rabbit was S.C. boosted with 200 micrograms of antigen mixed with an equal volume of IFA. Thereafter the rabbit was I.V. boosted with 100 micrograms of antigen. The animal was bled seven days following each boost.. The blood was then incubated at 4°C for 12-24 hours followed by centrifugation to generate the sera.

The polyclonal antisera were characterized using 96 well plates coated with antigen and incubated with 50 microliters (typically 1 microgram/microliter) of the polyclonal antisera at 4°C for 20 hours. Basically, 250 microliters of BSA blocking buffer was added to the wells and incubated at room temperature for 2 hours. Plates were washed 6 times with PBS/0.1% Tween. The rabbit sera were diluted in PBS/0.1% Tween/0.1%BSA. 50 microliters of diluted sera was added to each well and incubated at room temperature for 30 minutes. The plates were washed as described above, and then 50 microliters of goat anti-rabbit horseradish peroxidase (HRP) at a 1:10000 dilution was added and incubated at room temperature for 30 minutes.

The plates were washed as described above, and 100 microliters of TMB Microwell Peroxidase Substrate was added to each well Following a 15-minute incubation in the dark at room temperature, the colorimetric reaction was stopped with 100 microliters of IN H₂SO₄ and read immediately at 450 nm. All the polyclonal antibodies showed immunoreactivity to the appropriate antigen Tables 7-9 show the antibody reactivity of rabbit antisera in serial dilution to the three lung antigens, L523S, L763P and L763 peptide #2684. The first column shows the antibody dilutions. The columns "Pre-immune sera" indicate ELISA data for two experiments using pre-immune sera. These results are averaged in the fourth column. The columns "anti-L523S, L763P or #2684" indicate ELISA data for two experiments using sera from rabbits immunized as described in this Example, using the respective antigen, referred to as either L523S, L763P or #2684 in the tables..

**Table 7**

| **Antibody dilution** | **Pre-immune sera (1)** | **Pre-immune sera (2)** | **Average** | **Anti-L523S (1)** | **Anti-L523S (2)** | **Average** |
|---|---|---|---|---|---|---|
| **1:1000** | 0.14 | 0.14 | 0.14 | 2.36 | 2.37 | 2.37 |
| **1:2000** | 0.12 | 0.10 | 0.11 | 2.29 | 2.23 | 2.26 |
| **1:4000** | 0.10 | 0.09 | 0.10 | 2.11 | 2.17 | 2.14 |
| **1:8000** | 0.09 | 0.09 | 0.09 | 1.98 | 2.00 | 1.99 |
| **1:16000** | 0.09 | 0.09 | 0.09 | 1.73 | 1.76 | 1.75 |
| **1:32000** | 0.09 | 0.09 | 0.09 | 1.35 | 1.40 | 1.37 |
| **1:64000** | 0.09 | 0.11 | 0.10 | 0.94 | 0.98 | 0.96 |
| **1:128000** | 0.09 | 0.08 | 0.08 | 0.61 | 0.61 | 0.61 |
| **1:256000** | 0.08 | 0.08 | 0.08 | 0.38 | 0.38 | 0.38 |
| **1:512000** | 0.09 | 0.08 | 0.08 | 0.24 | 0.25 | 0.25 |
| **1:1024000** | 0.08 | 0.08 | 0.08 | 0.17 | 0.17 | 0.17 |
| **1:2048000** | 0.08 | 0.08 | 0.08 | 0.14 | 0.13 | 0.13 |

**Table 8**

| **Antibody dilution** | **Pre-immune sera (1)** | **Pre-immune sera (2)** | **Average** | **Anti-L763P (1)** | **Anti-L763P (2)** | **Average** |
|---|---|---|---|---|---|---|
| **1:1000** | 0.09 | 0.11 | 0.10 | 1.97 | 1.90 | 1.93 |
| **1:2000** | 0.07 | 0.07 | 0.07 | 1.86 | 1.84 | 1.85 |
| **1:4000** | 0.06 | 0.06 | 0.06 | 1.82 | 1.81 | 1.81 |
| **1:8000** | 0.06 | 0.06 | 0.06 | 1.83 | 1.81 | 1.82 |
| **1:16000** | 0.06 | 0.05 | 0.06 | 1.79 | 1.74 | 1.76 |
| **1:32000** | 0.06 | 0.06 | 0.06 | 1.56 | 1.51 | 1.53 |
| **1:64000** | 0.06 | 0.05 | 0.05 | 1.35 | 1.34 | 1.35 |
| **1:128000** | 0.05 | 0.05 | 0.05 | 1.01 | 0.98 | 0.99 |
| **1:256000** | 0.06 | 0.05 | 0.05 | 0.69 | 0.70 | 0.70 |
| **1:512000** | 0.06 | 0.05 | 0.05 | 0.47 | 0.44 | 0.46 |
| **1:1024000** | 0.06 | 0.05 | 0.06 | 0.27 | 0.27 | 0.27 |
| **1:2048000** | 0.05 | 0.05 | 0.05 | 0.16 | 0.15 | 0.16 |

**Table 9**

| **Antibody dilution** | **Pre-immune sera (1)** | **Pre-immune sera (2)** | **Average** | **Anti-#2684 (1)** | **Anti-#2684 (2)** | **Average** |
|---|---|---|---|---|---|---|
| **1:1000** | 0.07 | 0.07 | 0.07 | 2.10 | 2.00 | 2.05 |
| **1:2000** | 0.07 | 0.06 | 0.06 | 1.95 | 1.96 | 1.95 |
| **1:4000** | 0.06 | 0.06 | 0.06 | 1.77 | 1.82 | 1.79 |
| 1**:8000** | 0.06 | 0.06 | 0.06 | 1.79 | 1.81 | 1.80 |
| **1:16000** | 0.06 | 0.06 | 0.06 | 1.54 | 1.50 | 1.52 |
| **1:32000** | 0.06 | 0.06 | 0.06 | 1.27 | 1.20 | 1.24 |
| **1:64000** | 0.06 | 0.06 | 0.06 | 0.85 | 0.82 | 0.83 |
| **0** | 0.06 | 0.06 | 0.06 | 0.06 | 0.06 | 0.06 |

Tables 10-12 show the affinity purification of the respective antibodies to the three lung antigens, L523S, L763P and L763 peptide #2684.

**Table 10**

| **Antibody conc. (µg/ml)** | **Affinity pure (salt peak)** | **Affinity pure (salt peak)** | **Average** | **Affinity pure (acid peak)** | **Affinity pure (acid peak)** | **Average** |
|---|---|---|---|---|---|---|
| **1.0** | 2.38 | 2.35 | 2.36 | 2.25 | 2.31 | 2.28 |
| **0.5** | 2.24 | 2.22 | 2.23 | 2.19 | 2.18 | 2.18 |
| **0.25** | 2.05 | 2.09 | 2.07 | 2.01 | 2.03 | 2.02 |
| **0.13** | 1.70 | 1.81 | 1.75 | 1.74 | 1.74 | 1.74 |
| **0.063** | 1.44 | 1.44 | 1.44 | 1.43 | 1.38 | 1.40 |
| **0.031** | 1.05 | 1.05 | 1.05 | 0.99 | 0.99 | 0.99 |
| **0.016** | 0.68 | 0.67 | 0.68 | 0.65 | 0.64 | 0.64 |
| **0.0078** | 0.43 | 0.42 | 0.42 | 0.39 | 0.39 | 0.39 |
| **0.0039** | 0.27 | 0.26 | 0.27 | 0.24 | 0.26 | 0.25 |
| **0.0020** | 0.18 | 0.20 | 0.19 | 0.19 | 0.18 | 0.19 |
| **0.0010** | 0.13 | 0.14 | 0.13 | 0.13 | 0.14 | 0.13 |
| **0.00** | 0.11 | 0.12 | 0.11 | 0.10 | 0.12 | 0.11 |

**Table 11**

| **Antibody dilution** | **Affinity pure** | **Affinity pure** | **Average** |
|---|---|---|---|
| **1:1000** | 1.64 | 1.77 | 1.70 |
| **1:2000** | 1.59 | 1.76 | 1.68 |
| **1:4000** | 1.48 | 1.62 | 1.55 |
| **1:8000** | 1.35 | 1.43 | 1.39 |
| **1:16000** | 1.09 | 1.19 | 1.14 |
| **1:32000** | 0.81 | 0.89 | 0.85 |
| **1:64000** | 0.55 | 0.58 | 0.56 |
| **1:128000** | 0.31 | 0.35 | 0.33 |
| **1:256000** | 0.18 | 0.20 | 0.19 |
| **1:512000** | 0.11 | 0.12 | 0.11 |
| **1:1024000** | 0.07 | 0.07 | 0.07 |
| **1:2048000** | 0.06 | 0.06 | 0.06 |

**Table 12**

| **Antibody conc. (µg/ml)** | **Affinity pure** | **Affinity pure** | **Average** |
|---|---|---|---|
| **1.0** | 2.00 | 2.02 | 2.01 |
| **0.5** | 2.01 | 1.93 | 1.97 |
| **0.25** | 1.84 | 1.83 | 1.84 |
| **0.13** | 1.80 | 1.83 | 1.81 |
| **0.06** | 1.39 | 1.60 | 1.50 |
| **0.03** | 1.33 | 1.35 | 1.34 |
| **0.02** | 0.94 | 0.93 | 0.94 |
| **0.00** | 0.06 | 0.06 | 0.06 |

### EXAMPLE 24

### FULL-LENGTH CDNA SEQUENCE ENCODING L529S

The isolation of a partial sequence (SEQ ID NO:106) for lung antigen L529S was previously provided in Example 2. This partial sequence was used as a query to identify potential full length cDNA and protein sequences by searching against publicly available databases. The predicted full-length cDNA sequence for the isolated cloned sequence of SEQ ID NO:106 is provided in SEQ ID NO:442. The deduced amino acid sequence of the antigen encoded by SEQ ID NO:442 is provided in SEQ ID NO:443. It was previously disclosed in Example 2 that L529S shows similarity to connexin 26, a gap junction protein.

### EXAMPLE 25

### EXPRESSION IN MEGATERIUM OF A HISTIDINE TAG-FREE L523S FUSION PROTEIN

PCR was performed on the L523S coding region with the following primers:
Forward primer PDM-734 5' caatcaggcatgcacaacaaactgtatatcggaaac 3' (SEQ ID NO:444) Tm 63°C.
Reverse primer PDM-735 5' cgtcaagatcttcattacttccgtcttgac 3' (SEQ ID NO:445) TM 60°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla, CA)
50ηg DNA
96°C for 2 minutes, 96°C for 20 seconds, 62°C for 15 seconds, 72°C for 4 minute with 40 cycles and then 72°C for 4 minutes.

The PCR product was digested with SphI and BglII restriction enzymes, gel purified and then cloned into pMEG·3, which had been digested with SphI and BglII restriction enzymes.. The correct construct was confirmed by DNA sequence analysis and then transformed into Megaterium cells for expression.

The amino acid sequence of expressed recombinant L523S is shown in SEQ ID NO:446, and the DNA coding region sequence is shown in SEQ ID NO:447.

### EXAMPLE 26

### EXPRESSION IN E. COLI OF A HISTIDINE IAG-FREE L523S FUSION FROIEIN

PCR was performed on the L552S coding region with the following primers:
Forward primer PDM-733 5' cgtactagcatatgaacaaactgtatatcggaaac 3' (SEQ ID NO:448) Tm 64°C.
Reverse primer PDM-415 5' ccatagaattcattacttccgtcttgactgagg 3' (SEQ ID NO:426) TM 62°C.

The PCR conditions were as follows:
10µl 10X Pfu buffer
1.0µl 10mM dNTPs
2.0µl 10µM each primer
83µl sterile water
1.5µl Pfu DNA polymerase (Stratagene, La Jolla,'CA)
50ηg DNA
96°C for 2 minutes, 96°C for 20 seconds, 62°C for 15 seconds, 72°C for 4 minute with 40 cycles and then 72°C for 4 minutes.

The PCR product was digested with NdeI and EcoRI restriction enzymes, gel purified and then cloned into pPDM, a modified pET28 vector, which had been digested with NdeI and EcoRI restriction enzymes. The correct construct was confirmed by DNA sequence analysis and then transformed into BLR pLys S and HMS 174 pLys S cells for expression.

The amino acid sequence of expressed recombinant L523S is shown in SEQ ID NO:449, and the DNA coding region sequence is shown in SEQ ID NO:450.

### EXAMPLE 27

### EPITOPE-ANALYSIS OF L514S AND L523S-SPECIFIC ANTIBODIES

Peptides of candidate antigens can be used for the evaluation of antibody responses in both preclinical and clinical studies. These data allow one to further confirm the antibody response against a certain candidate antigen. Protein-based ELISA with and without competitive peptides and peptide-based ELISA can be used to evaluate these antibody responses. Peptide ELISA is especially useful since it can further exclude the false positive of the antibody titer observed in protein-based ELISA as well as to provide the simplest assay system to test antibody responses to candidate antigens. In this example, data was obtained using both L514S- and L523S-peptides that show that individual cancer patients produce L514S- and L5238-specific antibodies. The L514S-specific antibodies recognize primarily the following epitope of L514S:
aa86-110: LGKEVRDAKITPEAFEKLGFPAAKE (SED ID NO:451).

This epitope is the common epitope in humans. A rabbit antibody specific for L514S recognizes two addition epitopes of L514S:
(1) aa21-45: KASDGDYYTLAVPMGDVPMDGISVA (SEQ ID NO:452)
(2) aa121-135: PDRDVNLTHQLNPKVK (SED ID NO:453)

It was further found that the SEQ ID NO:452 is common to both L514S isoforms, L514S-13160 and L514S-13166, whereas the other epitopes, SEQ ID NO:451 and SEQ ID NO:453, are probably specific to the isoform, L514S-13160.

The L523S-specific antibodies recognize primarily the following epitope of L523S:
aa440-460: KIAPAEAPDAKVRNIVIITGP (SEQ ID NO:454).

This epitope is the common epitope in humans. A rabbit antibody specific for L523S recognizes two other epitopes:
(1) aa156-175 PDGAAQQNNNPLQQPRG (SEQ ID NO:455)
(2) aa326-345: RTITVKGNVETCAKAEEEIM (SED ID NO:456)

In further studies, it was determined by peptide based ELISAs that eight additional epitopes of L523S were recognized by L523S-specific antibodies:
(1) aa40-59 AFVDCPDESWALKAIEALS (SEQ ID NO:457)
(2) aa80-99: IRKLQIRMPPHLQWEVLDS (SED ID NO:458)
(3) aa160-179: AQQNPLQQPRGRRGLGQRGS (SEQ ID NO:459)
(4) aa180-199: DVHRKENAGAAEKSTIILST (SED ID NO:460)
(5) aa320-339: LYNPERTITVKGNVEICAKA (SEQ ID NO:461)
(6) aa340-359: EEEWKWWSYENDIASMN (SED ID NO:462)
(7) aa370-389: LNALGLFPPTSGMPPPTSGP (SEQ ID NO:463)
(8) aa380-399: KIAPAEAPDAKVRMVIITGP (SED ID NO:464)

Out of these, six epitopes are common in both lung plural effusion fluid samples and in sera of lung patients.. Of these six, SEQ ID NO:459 and SEQ ID NO:463 have no homology to other L523S-family proteins such as IGF-II mRNA-binding proteins 1 and 2. Accordingly, this indicates that these two peptides can be used as an assay system to determine the antibody response to L523S.

### EXAMPLE 28

### GENERATION OF L523S-SPBCIFIC CTL LINES USING IN VIIRO WHOLE-GENE PRIMING

To determine if L523S is capable of generating a CD8⁺ T cell immune response, CTLs were generated using *in vitro* whole-gene priming methodologies with tumor antigen-vaccinia infected DC (Yee et al, The Journal of Immunology, 157(9):4079-86, 1996), human CIT., lines were derived that specifically recognize autologous fibroblasts transduced with the L552S tumor antigen, as determined by interferon-gamma ELISPOT analysis. Specifically, dendritic cells (DC) were differentiated from Peicoll-purifted monocytes derived from PBMC of normal human donors by plastic adherence and growing for five days in RPMI medium containing 10% human serum, 50 ng/ml human GM-CSF and 30 ng/ml human IL-4., Following the five days of culture, the DC were infected overnight with a recombinant adenovirus that expresses L523S at a multiplicity of infection (M.O.I) of 33, 66 and 100, and matured overnight by the addition of 2 µg/ml CD40 ligand. The virus was then inactivated by UV irradiation. In order to generate a CIL line, autologous PBMC were isolated and CD8+ I cells were emiched for by the negative selection using magnetic beads conjugated to CD4+, CD14+, CD16+, CD19+, CD34+ and CD56+ cells. CD8+ I cells specific for L523S were established in round bottom 96-well plates using 10,000 L523S expressing DCs and 100,000 CD8+ T cells per well in RPMI supplemented with 10% human serum, 10ng/ml of IL-6 and 5ng/ml of IL-12. The cultures were restimulated every 7-10 days using autologous primary fibroblasts retrovirally transduced with L523S, and the costimulatory molecule CD80 in the presence of IL-2. The cells were also stimulated with IFN-gamma to upregulate MHC Class I. The media was supplemented with 10U/ml of IL-2 at the time of stimulation as well as on days 2 and 5 following stimulation. Following three stimulation cycles, ten L523S specific CD8+ I cell lines were identified using interferon-gamma ELISPOT analysis that specifically produce interferon-gamma when stimulated with the L523S tumor antigen-transduced autologous fibroblasts, but not with a control antigen.

One line, 6B1, was cloned using- anti-CD3 and feeder cells. The clones were tested for specificity on L523S-transduced fibroblasts. In addition, using a panel of HLA-mismatched lines transduced with a vector expressing L523S and measuring interferon-gamma production by this CTL line in an ELISPOT assay, it was determined that this clone 6B1.4B8 is restricted by HLA-A0201.

Also using transfected Cos cells, it was shown that clone 6B1.4B8 recognizes Cos cells transfected with pcDNA3 HLA A0201/L523S in an HLA-restricted and antigen specific manner.

An epitope mapping study demonstrated the clone 6B1.4B8 recognizes HLA-A201 LCL loaded with peptide pool 3 (a polypeptide corresponding to amino acid positions 33-59 of L523S.

A peptide pool breakdown study demonstrated that clone 6B1.4B8 recognizes autologous B-LCL loaded with 15-mer peptides from amino acid positions 37-55 of L523S, TGYAFVCPDESWALKAIE (SEQ ID NO:465). A further peptide breakdown study demonstrated that clone 6B1.4B8 recognizes T2 cells loaded with the same 15-mer peptides.

A peptide recognition study demonstrated that clone 6B1.4B8 prefers 12 cells loaded with the peptide FVDCPESWAL (SEQ ID NO:466) which is corresponds to the amino acid sequence at positions 41-51 of L523S and is encoded by the DNA sequence of SEQ ID NO:467.

### EXAMPLE 29

### L523S EXPRESSION IN OIHER HUMAN CANCERS

It was previously disclosed in Example 2 that L523 S is expressed in lung cancers including squamous, adenocarcinoma and small cell carcinoma. To further evaluate the expression profile of this antigen an electronic express profiling was performed. This was done by searching a L523S-specific sequence against a public EST database.. Results of this profiling indicate that L523S may also be present in colon adenocarcinomas, prostate adenocarcinomas, CML, AML, Burkitt's Lymphoma, brain tumors, retinoblastomas, ovarian tumors, tetratocarcinomas, uterus myosarcomas, germ cell tumors as well as pancreatic and cervical tumor cell lines..

### EXAMPLE 30

### IMMUNOHISTOCHEMISTRY ANALYSIS OF L523S

In order to determine which tissues express the lung tumor antigen L523S, immunohistochemistry (IHC) analysis was performed on a diverse range of tissue types. Polyclonal antibodies specific for L523S (SEQ ID NO:176) were generated as described in Example 23. IHC was performed essentially as described in Example 6.. Briefly, tissue samples were fixed in formalin solution for 12-24 hours and embedded in paraffin before being sliced into 8 micron sections. Steam heat induced epitope retrieval (SHIER) in 0.1 sodium citrate buffer (pH 6.0) was used for optimal staining conditions. Sections were incubated with 10% serum in PBS for 5 minutes. The primary L523S antibody was added to each section for 25 minutes followed by a 25 minute incubation with anti-rabbit biotinylated antibody. Endogenous peroxidase activity was blocked by three 1.5 minute incubations with hydrogen peroxidase. The avidin biotin complex/ horse radish peroxidase (ABC/HRP) system was used along with DAB chromogen to visualize antigen expression, Slides were counterstained with hematoxylin to visualize the cell nuclei.

IHC analysis of L523S expression revealed that of the lung cancer tissues tested over 90% of tissue samples demonstrated high over-expression of the lung tumor antigen (10/11 adenocaricomas and 8/9 squamous). Of the normal tissues tested, all were negative for expression of L523S, with the exception of weak staining in normal bronchus, testis, liver, and trachea.

### EXAMPLE 31

### GENERATION AND CHARACTERIZATION OF L762 HUMAN MONOCLONAL ANTIBODTES

Cell supernatants from hybridoma fusions from the Xenomouse strain of transgenic mice were screened for ability to bind to L762P. All results are shown in Table 13. The primary screen was to test monoclonal supernatants for reactivity to L762P by ELISA analysis using recombinant bacterial expressed protein. We next tested the human supernatants for reactivity to surface expressed L762P by whole cell ELISA using fluorimetry analysis. Specific reactivity of the humab supernatants was confirmed by performing FACS analysis on cells transfected with either an irrelevant plasmid or a plasmid expressing L762P" FT/CFI is the relative fold increase in fluorescence intensity (FI) of the anti-L762P humab primary antibody to irrelevant human primary antibody. FI/CFI/A20 is the relative fold increase in fluorescence intensity (FI) of the anti-L762P humab primary antibody to irrelevant human primary antibody over the FI of the anti-L762P mouse monoclonal antibody 153A20.1. FIICFI/R690 is the relative fold increase in fluorescence intensity (FI) of the anti-L762P humab primary antibody to irrelevant human primary antibody over the FI of the anti-L762P rabbit polyclonal antibody- FACS VRL762 is the percentage of cells transfected with plasmid expressing L762P that were positive following staining with indicated monoclonal antibody. FACS VR(-) is the percentage of cells transfected with irrelevant plasmid that were positive following staining with indicated monoclonal antibody. ELISA is the O.D. values of the indicated monoclonal antibody to recombinant L762P protein. The shaded rows in Table 13 indicate those antibodies that will be further cloned and characterized.

**Table 13: Human Monoclonal Antibodies Against L762P**

| **L762PHumAb** | **FI/CFI** | **FI/CFI/A20** | **FI/CFI/R690** | **FACSVRL762** | **FACSVR(-)** | **ELISA** | **L762/VR1013** |
|---|---|---|---|---|---|---|---|
| R-690 | 4.59 | | 1.00 | | | | |
| M-A20 | 2.88 | 1.00 | | | | | |
| **1.176** | 0.51 | 0.18 | 0.11 | | | 0.38 | |
| **1.178** | 1.42 | 0.49 | 031 | | | 0.35 | |
| **1.179** | 0.47 | 0.16 | 0.10 | | | 0.07 | |
| **1.180** | 1.50 | 0.52 | 0.33 | | | 0.26 | |
| **1.182** | 1.45 | 0.50 | 0.32 | | | 026 | |
| **1.183** | 0.75 | 0.26 | 0.16 | | | 0.24 | |
| **1.185** | 0.89 | 0.31 | 0.19 | | | 0.46 | |
| **1.186** | 3.45 | 1.20 | 0.75 | 32.68 | 7.14 | 1.22 | 1.93 |
| **1.187** | 0.36 | 0.13 | 0.08 | | | 0.06 | |
| **1.188** | 0.26 | 0.09 | 0.06 | | | 0.23 | |
| **1.189** | 0.50 | 0.17 | 0.11 | | | 0.44 | |
| **1.190** | 0.53 | 0.18 | 0.12 | | | 0.42 | |
| **1.190** | 3.12 | 1.08 | 0.68 | 41.44 | 17.90 | 0.86 | 1.29 |
| **1.192** | 1.91 | 0.66 | 0.42 | | | 0.12 | |
| **1.193** | 2.87 | 1.00 | 17.82 | | | | 1.06 |
| **1.194** | 1.55 | 0.54 | 0.34 | | | 0.28 | |
| **1.195** | 0.14 | 0.05 | 0.03 | | | 0.37 | |
| **1.196** | 1.97 | 0.68 | 0.43 | | | 0.89 | 1.64 |
| **1.197** | 0.43 | 0.15 | 0.09 | | | 0.08 | |
| **1.198** | 0.54 | 0.19 | 0.12 | | | 0.33 | |
| **1.199** | 0.70 | 0.24 | 0.15 | | | 0.40 | |
| **1.200** | 2.00 | 0.69 | 0.44 | | | 0.38 | 1.56 |
| **1.201** | 1.62 | 0.56 | 0.35 | | | 0.29 | |
| **1.202** | 0.86 | 0.30 | 0.19 | | | 0.36 | |
| **1.203** | 1.56 | 0.27 | 0.18 | | | 0.14 | |
| **1.204** | 3.32 | 0.58 | 0.38 | 24.83 | 6.60 | 0.17 | 1.91 |
| **1.205** | 2.13 | 0.37 | 0.25 | | | 0.09 | |
| **1.206** | 0.45 | 0.08 | 0.05 | | | 0.23 | |
| **1.207** | 0.60 | 0.10 | 0.07 | | | 039 | |
| **1.208** | 0.12 | 0.02 | 0.01 | | | 0.36 | |
| **1.209** | 15.52 | 2.71 | 1.80 | 27.54 | 9.54 | 0.16 | 0.77 |
| **1.210** | 0.92 | 0.16 | 0.11 | | | 0.16 | |
| **1.211** | 2.83 | 0.49 | 0.33 | | | 0.42 | |
| **1.212** | 3.40 | 0.59 | 0.39 | 21.68 | 11.36 | 0.14 | 2.47 |
| **1.213** | 2.32 | 0.40 | 0.27 | | | 0.38 | |
| **1.214** | 0.80 | 0.14 | 0.09 | | | 0.34 | |
| **1.215** | 3.96 | 0.69 | 0.46 | 38.87 | 13.17 | 0.33 | 1.80 |
| **1.216** | 1.26 | 0.22 | 0.15 | | | 0.20 | |
| **1.217** | 1.99 | 0.35 | 0.23 | | | 0.26 | |
| **1.218** | 2.29 | 0.40 | 0.27 | | | 0.10 | |
| **1.219** | 0.15 | 0.03 | 0.02 | | | 0.06 | |
| **1.220** | 0.82 | 0.14 | 0.09 | | | 0.21 | |
| **1.221** | 2.29 | 0.40 | 0.27 | | | 0.12 | |
| **1.222** | 0.57 | 0.10 | 0.07 | | | 0.45 | |
| **1.223** | 0.11 | 0.02 | 0.01 | | | 0.11 | |
| **1.224** | 2.08 | 0.36 | 0.24 | | | 025 | |
| **1.225** | 0.95 | 0.17 | 0.11 | | | 0.22 | |
| **1.226** | -0.32 | -0.06 | -0.04 | | | 0.06 | |
| R-690 | 8.62 | | 1.00 | 72.34 | 39.83 | | |
| M-A20 | 5.73 | 1.00 | | 50.23 | 6.34 | | |
| M-A12 | | | 67.43 | 25.15 | | | |
| M-Irr | | | 7.74 | 735 | | | |
| R-Irr | | | 30.09 | 24.80 | | | |
| H-Irr | | | 25.52 | 39.14 | | | |
| | | | | | | | |
| R-690 | 3.20 | | 1.00 | | | | |
| M-A20 | 2.33 | 1.00 | | | | | |
| **1.250** | 0.15 | 0.06 | 0.05 | | | 0.28 | |
| **1.228** | 0.38 | 0.16 | 0.12 | | | 0.08 | |
| **1.229** | 039 | 0.17 | 0.12 | | | 0.44 | |
| **1.230** | 1.78 | 0.76 | 0.56 | | | 0.13 | 1.35 |
| **1.231** | 0.42 | 0.18 | 0.13 | | | 0.47 | |
| **1.232** | 0.34 | 0.15 | 0.11 | | | 0.25 | |
| **1.233** | 7.07 | 3.04 | 2.21 | 68.84 | 38.60 | 0.43 | 0.75 |
| **1.234** | 2.54 | 1.09 | 0.79 | 33.96 | 10.94 | 0.73 | 1.68 |
| **1.235** | 1.53 | 0.65 | 0.48 | | | 0.19 | 1.45 |
| **1.236** | 0.17 | 0.07 | 0.05 | | | 0.44 | |
| **1.237** | 035 | 0.15 | 0.11 | | | 0.06 | |
| **1.238** | 0.38 | 0.16 | 0.12 | | | 0.06 | |
| **1.239** | 0.40 | 0.17 | 0.13 | | | 0.06 | |
| **1.240** | 2.05 | 0.88 | 0.64 | 28.70 | 7.44 | 0.33 | 1.70 |
| **1.241** | 0.41 | 0.18 | 0.13 | | | 0.41 | |
| **1.242** | 0.52 | 0.23 | 0.16 | | | 0.05 | |
| **1.243** | 2.34 | 1.00 | 0.73 | 30.94 | 28.13 3 | 0.16 | 1.33 |
| **1.244** | 0.94 | 0.40 | 0.29 | | | 0.23 | |
| **1.245** | 0.37 | 0.16 | 0.11 | | | 0.31 | |
| **1.246** | 2.10 | 0.90 | 0.66 | 13.97 | 28.92 | 0.52 | 1.21 |
| **1.247** | 0.33 | 0.14 | 0.10 | | | 037 | |
| **1.248** | 1.80 | 0.77 | 0.56 | | | 0.76 | |
| **1.249** | 2.77 | 1.19 | 0.86 | 28.76 | | 1.15 | 2.38 |
| **1.251** | 0.22 | 0.09 | 0.07 | | | 0.47 | |
| **1.252** | 1.16 | 0.27 | 0.17 | | | 037 | |
| **1.253** | 0.07 | 0.02 | 0.01 | | | 0.43 | |
| **1.254** | 2.05 | 0.48 | 0.30 | | | 0.14 | |
| **1.255** | 0.09 | 0.02 | 0.01 | | | 0.08 | |
| **1.256** | 1.17 | 0.27 | 0.17 | | | 0.13 | |
| **1.257** | 0.42 | 0.10 | 0.06 | | | 0.06 | |
| **1.258** | 0.48 | 0.11 | 0.07 | | | 0.40 | |
| **1.259** | 4.82 | 1.13 | 0.69 | 44.24 | 11.92 | 0.38 | 1.78 |
| **1.260** | 1.80 | 0.42 | 0.26 | | | 0.38 | |
| **2.1** | 2.70 | 0.63 | 0.39 | | | 0.14 | 1.35 |
| **2.3** | 0.06 | 0.01 | 0.01 | | | 0.57 | |
| **2.4** | 3.08 | 0.72 | 0.44 | 31.28 | 11.43 | 0.73 | 1.95 |
| **2.5** | 0.70 | 0.16 | 0.10 | | | 0.45 | |
| **2.6** | 1.26 | 0.29 | 0.18 | | | 0.22 | |
| **2.8** | 0.59 | 0.14 | 0.09 | | | 0.31 | |
| **2.9** | 7.48 | 1.75 | 1.08 | 45.72 | 17.57 | 0.95 | 1.53 |
| **2.10** | 0.35 | 0.08 | 0.05 | | | 0.42 | |
| **2.11** | 2.71 | 0.63 | 0.39 | | | 0.60 | 1.58 |
| **2.12** | 6.04 | 1.41 | 0.87 | 52.50 | 19.59 | | 1.40 |
| **2.13** | 5.50 | 1.28 | 0.79 | 39.78 | 15.24 | | 1.39 |
| **2.14** | 0.68 | 0.16 | 0.10 | | | | |
| **2.15** | 6.51 | 1.52 | 0.94 | 49.90 | 15.36 | | 1.72 |
| **2.16** | 4.58 | 1.07 | 0.66 | 28.62 | 13.02 | | 1.51 |
| **2.17** | 8.10 | 1.89 | 1.17 | 48.76 | 18.24 | | 3.06 |
| R-690 | 6.94 | | 1.00 | | | | |
| M-A20 | 4.28 | 1.00 | | 56.40 | 5.00 | | |
| | | | | | | | |
| R-690 | 4.34 | 1.65 | 1.00 | | | | |
| M-A20 | 2.63 | 1.00 | 0.61 | | | | |
| **2.18** | 2.29 | 0.87 | 0.53 | | | 1.27 | 1.95 |
| **2.20** | 1.85 | 0.70 | 0.43 | | | 0.52 | 2.75 |
| **2.21** | 0.09 | 0.03 | 0.02 | | | 0.40 | |
| **2.22** | 3.26 | 1.24 | 0.75 | 29.4 | 6.2 | 1.45 | 1.8 |
| **2.23** | 0.31 | 0.12 | 0.07 | | 0.12 | | |
| **2.24** | 1.21 | 0.46 | 0.28 | | | 0.65 | |
| **2.25** | 3.47 | 1.32 | 0.80 | 32.5 | 7.1 | 1.35 | 1.46 |
| **2.26** | 4.42 | 1.68 | 1.02 | 35.9 | 5.5 | 0.77 | 1.55 |
| **2.27** | 1.42 | 0.54 | 033 | | | 0.22 | |
| **2.28** | 3.00 | 1.14 | 0.69 | 28.6 | 5.4 | 1.21 | 1.26 |
| **2.29** | 1.41 | 0.53 | 0.32 | | | 0.58 | |
| **2.30** | 0.42 | 0.16 | 0.10 | | | 0.43 | |
| **2.31** | 0.09 | 0.03 | 0.02 | | | 0.07 | |
| **2.34** | 1.94 | 0.74 | 0.45 | | | 1.17 | 1.23 |
| **2.38** | 1.14 | 0.43 | 0.26 | | | 0.09 | |
| **2.39** | 2.50 | 0.95 | 0.57 | 28.2 | | 0.78 | 1.14 |
| **2.40** | 2.02 | 0.77 | 0.46 | | | 0.47 | 0.99 |
| **2.41** | 1.16 | 0.44 | 0.27 | | | 0.08 | |
| **2.42** | 0.41 | 0.16 | 0.09 | | | 0.24 | |
| **2.46** | 2.46 | 0.93 | 0.57 | 16.1 | 4.6 | 1.07 | 1.3 |
| **2.47** | 1.83 | 0.69 | 0.42 | | | 0.31 | 1.54 |
| **2.48** | 2.50 | 0.95 | 0.58 | | | 1.36 | 1.76 |
| **2.49** | 0.50 | 0.19 | 0.12 | | | 0.74 | |
| **2.50** | 2.93 | 1.11 | 0.68 | 15.8 | 4.7 | 0.52 | 1.54 |
| **2.51** | 0.13 | 0.10 | 0.07 | | | 0.30 | |
| **2.52** | 1.11 | 0.79 | 0.56 | 22.1 | 5 | 1.14 | 1.93 |
| **2.52** | 1.87 | 1.34 | 0.94 | 29.8 | 7.8 | 0.58 | 2.84 |
| **2.54** | 1.85 | 1.32 | 0.92 | 15.9 | 8.5 | 0.12 | 2.56 |
| **2.55** | 0.83 | 0.60 | 0.42 | | | 0.32 | |
| **2.58** | 0.46 | 0.33 | 0.23 | | | 0.15 | |
| **2.60** | 0.99 | 0.71 | 0.50 | | | 035 | |
| **2.61** | 2.16 | 1.54 | 1.08 | 30.7 | | 1.34 | 2.88 |
| **2.62** | 0.36 | 0.26 | 0.18 | | | 0.58 | |
| **2.63** | 0.37 | 0.26 | 0.18 | | | 0.41 | |
| **2.64** | 1.60 | 1.14 | 0.80 | 25.7 | 6.1 | 1.39 | 2.85 |
| **2.65** | 0.63 | 0.45 | 0.31 | | | 0.16 | |
| **2.66** | 0.08 | 0.06 | 0.04 | | | 0.06 | |
| **2.67** | 1.34 | 0.96 | 0.67 | 23.3 | | 1.32 | 1.34 |
| **2.68** | 0.66 | 0.47 | 0.33 | | | 0.38 | |
| **2.69** | 2.79 | 1.99 | 1.39 | 463 | 9.7 | 1.47 | 1.68 |
| **2.73** | 1.47 | 1.05 | 0.73 | 28.5 | 7.2 | 1.04 | 1.85 |
| **2.74** | 1.99 | 1.43 | 1.00 | 39.5 | 19.1 | 1.22 | 1.69 |
| **2.75** | 1.46 | 1.04 | 0.73 | 25.6 | 7.5 | 0.68 | 1.55 |
| **2.76** | 1.61 | 1.15 | 0.81 | 27.7 | 7.7 | 0.98 | 1.79 |
| **2.77** | 1.59 | 1.13 | 0.79 | 27.7 | 4.9 | 1.11 | 1.53 |
| **2.78** | 1.55 | 1.11 | 0.77 | 13.9 | 8 | 1.51 | 2.64 |
| **2.79** | 0.33 | 0.24 | 0.16 | 10 | 5.4 | 0.43 | |
| **2.80** | 1.47 | 1.05 | 0.73 | 15.9 | 8.8 | 0.46 | 0.95 |
| R-690 | 2.00 | 1.43 | 1.00 | | | | |
| M-A20 | 1.40 | 1.00 | | 56.4 | 5 | | |
| | | | | | | | |
| R-690 | 3.76 | 3.44 | 1.00 | | | | |
| M-A20 | 1.09 | 1.00 | | | | | |
| **2.81** | 0.25 | 0.23 | 0.07 | | | 0.17 | |
| **2.82** | 0.44 | 0.40 | 0.12 | | | 0.49 | |
| **2.83** | 0.63 | 0.58 | 0.17 | | | 0.80 | |
| **2.84** | 0.13 | 0.12 | 0.04 | | | 0.55 | |
| **2.85** | 0.62 | 0.57 | 0.16 | | | 0.19 | |
| **2.86** | 0.87 | 0.79 | 0.23 | | | 0.16 | |
| **2.87** | 0.84 | 0.77 | 0.22 | | | 0.22 | |
| **2.89** | 5.88 | 5.37 | 1.56 | 45.9 | 37.9 | 0.07 | 0.73 |
| **2.90** | 0.23 | 0.21 | 0.06 | | | 0.60 | |
| **2.91** | -0.37 | -0.34 | -0.10 | | | 0.43 | |
| **2.92** | 0.59 | 0.54 | 0.16 | | | 0.14 | |
| **2.93** | 0.28 | 0.26 | 0.08 | | | 0.44 | |
| **2.94** | 0.32 | 0.29 | 0.08 | | | 0.46 | |
| **2.95** | 0.39 | 0.36 | 0.10 | | | 0.51 | |
| **2.96** | 0.36 | 0.33 | 0.10 | | | 0.26 | |
| **2.97** | 1.26 | 1.15 | 0.33 | 36.8 | 14.1 | 1.01 | 0.89 |
| **2.98** | 0.92 | 0.84 | 0.24 | | | 0.84 | |
| **2.99** | 1.38 | 1.26 | 0.37 | 91.2 | 81.8 | 0.29 | |
| **2.100** | 0.94 | 0.86 | 0.25 | | | 1.40 | |
| **2.102** | 0.77 | 0.70 | 0.21 | | | 0.17 | |
| **2.104** | 1.37 | 1.25 | 0.36 | 10.2 | 7.4 | 0.14 | |
| **2.105** | 0.63 | 0.58 | 0.17 | | | 1.04 | |
| **2.106** | 0.79 | 0.72 | 0.21 | | | 0.84 | |
| **2.107** | 0.81 | 0.74 | 0.22 | | | 0.06 | |
| **2.109** | 0.66 | 1.24 | 0.32 | 19.2 | 6.1 | 0.45 | 0.89 |
| **2.110** | 1.58 | 3.00 | 0.77 | 36.4 | 14.2 | 0.89 | 1.31 |
| **2.112** | 0.80 | 1.52 | 0.39 | 28.8 | 6.4 | 1.16 | 1.35 |
| **2.113** | 0.57 | 1.07 | 0.27 | 31.4 | 10.7 | 0.66 | 1.17 |
| **2.114** | 0.52 | 0.99 | 0.25 | | | 0.32 | |
| **2.115** | 1.02 | 1.94 | 0.50 | 19.9 | 10.7 | 0.63 | 11.3 |
| **2.116** | 0.52 | 0.98 | 0.25 | | | 0.86 | |
| **2.118** | 0.19 | 0.36 | 0.09 | 0.06 | | | |
| **2.119** | 0.78 | 1.48 | 0.38 | 20.4 | 5.3 | 1.22 | 1.16 |
| **2.120** | 0.76 | 14.4 | 0.37 | 21.8 | 6 | 1.29 | 0.97 |
| **2.121** | 1.24 | 2.36 | 0.60 | 28.7 | 10.7 | 0.30 | 1.17 |
| **2.122** | 1.20 | 2.29 | 0.58 | 3.13 | 8.3 | 1.13 | 1.14 |
| **2.123** | 0.67 | 1.27 | 0.33 | 17.7 | 6.8 | 0.74 | 1.27 |
| R-690 | 2.06 | 3.91 | 1.00 | | | | |
| M-A20 | 0.53 | 1.00 | | 56.4 | 5 | | |
| | | | | | | | |
| R-690 | 3.51 | | 1.00 | | | | |
| M-A20 | 2.91 | 1.00 | | | | | |
| **1.1** | 1.05 | 0.36 | 0.30 | | | 0.16 | |
| **1.2** | -0.42 | -0.14 | -0.12 | | | 0.40 | |
| **13** | 1.04 | 0.36 | 0.30 | | | 1.31 | |
| **1.4** | 0.77 | 0.26 | 0.22 | | | 0.43 | |
| **1.5** | 0.19 | 0.06 | 0.05 | | | 0.13 | |
| **1.6** | 1.07 | 0.37 | 0.30 | | | 0.42 | |
| **1.7** | 0.09 | 0.03 | 0.03 | | | 0.33 | 0.80 |
| **1.8** | 2.93 | 1.01 | 0.83 | 54.70 | 45.60 | 0.59 | |
| **1.9** | 1.17 | 0.40 | 0.33 | | | 0.93 | |
| **1.10** | -0.04 | -0.02 | -0.01 | | | 0.08 | |
| **1.11** | -0.30 | -0.10 | -0.09 | | | 0.16 | |
| **1.12** | 0.11 | 0.04 | 0.03 | | | 0.25 | |
| **1.13** | 1.60 | 0.55 | 0.46 | | | 0.08 | |
| **1.14** | 0.69 | 0.24 | 0.20 | | | 0.13 | |
| **1.15** | 0.30 | 0.10 | 0.09 | | | 0.08 | |
| **1.16** | 1.44 | 0.49 | 0.41 | | | 0.08 | |
| **1.17** | -0.31 | -0.10 | -0.09 | | | 0.36 | |
| **1.18** | 0.05 | 0.02 | 0.01 | | | 0.17 | |
| **1.19** | -034 | -0.12 | -0.10 | | | 0.29 | |
| **1.20** | 0.84 | 0.29 | 0.24 | | | 0.45 | |
| **1.21** | -0.20 | -0.07 | -0.06 | | | 0.28 | |
| **1.22** | 0.14 | 0.05 | 0.04 | | | 0.06 | |
| **1.23** | 0.14 | 0.05 | 0.04 | | | 0.08 | |
| **1.24** | 1.02 | 0.35 | 0.29 | | | 0.16 | |
| **1.25** | 0.27 | 0.28 | 0.16 | | | 0.20 | |
| **1.26** | 1.06 | 1.09 | 0.62 | | | 0.31 | |
| **1.27** | 1.07 | 1.10 | 0.63 | | | 0.96 | |
| **1.28** | 2.14 | 2.21 | 1.26 | 3.60 | ND | 0.06 | 0.73 |
| **1.29** | 1.11 | 1.15 | 0.65 | | | 0.44 | 1.64 |
| **1.30** | 0.79 | 0.81 | 0.46 | | | 0.19 | |
| **1.31** | 1.42 | 1.46 | 0.84 | | | 0.23 | 1.27 |
| **1.32** | 1.37 | 1.42 | 0.81 | | | 0.11 | 1.91 |
| **1.33** | 0.29 | 0.30 | 0.17 | | | 0.18 | |
| **1.34** | 1.59 | 1.64 | 0.94 | 37.53 | 8.98 | 1.31 | 2.61 |
| **1.35** | 0.37 | 0.38 | 0.21 | | | 0.32 | |
| **1.36** | 0.70 | 0.72 | 0.41 | | | 0.17 | |
| **1.37** | 1.21 | 1.24 | 0.71 | | | 0.69 | |
| **1.38** | 0.63 | 0.65 | 0.37 | | | 0.38 | |
| **1.39** | 0.87 | 0.90 | 0.51 | | | 0.07 | |
| **1.40** | 0.71 | 0.73 | 0.42 | | | 0.26 | |
| **1.41** | 1.36 | 1.40 | 0.80 | 43.82 | 13.65 | 0.37 | 2.03 |
| **1.42** | 0.64 | 0.66 | 0.38 | | | 1.10 | |
| **1.43** | 0.46 | 0.47 | 0.27 | | | 0.09 | |
| **1.44** | 0.52 | 0.54 | 0.31 | | | 0.28 | |
| **1.45** | 0.74 | 0.76 | 0.44 | | | 0.15 | |
| **1.46** | 0.81 | 0.83 | 0.48 | | | 0.07 | |
| **1.47** | 0.46 | 0.47 | 0.27 | | | 0.24 | |
| **1.48** | 0.62 | 0.63 | 0.36 | | | 0.27 | |
| R-690 | 1.70 | | 1.00 | | | | |
| M-A20 | 0.97 | 1.00 | | | | | |
| | | | | | | | |
| R-690 | 1.84 | | 1.00 | | | | |
| M-A20 | 2.82 | 1.00 | | | | | |
| **1.49** | 0.76 | 0.27 | 0.41 | | | 0.14 | |
| **1.50** | -0.22 | -0.08 | -0.12 | | | 0.36 | |
| **1.51** | -0.35 | -0.12 | -0.19 | | | 0.45 | |
| **1.52** | 1.84 | 0.65 | 1.00 | 45.74 | 9.90 | 1.40 | 2.44 |
| **1.53** | 1.77 | 0.63 | 0.96 | 42.79 | 24.70 | 0.89 | |
| **1.54** | 1.08 | 0.38 | 0.59 | | | 0.80 | |
| **1.55** | 0.81 | 0.29 | 0.44 | | | 0.35 | |
| **1.56** | 1.26 | 0.45 | 0.69 | | | 0.30 | |
| **1.57** | 3.26 | 1.16 | 1.77 | 22.20 | ND | 1.31 | 2.69 |
| **1.58** | 0.81 | 0.29 | 0.44 | | | 0.80 | |
| **1.59** | 2.22 | 0.79 | 1.21 | 24.50 | ND | 1.28 | 2.40 |
| **1.60** | 0.55 | 0.19 | 0.30 | | | 0.23 | |
| **1.61** | 0.13 | 0.04 | 0.07 | | | 0.06 | |
| **1.62** | 0.75 | 0.27 | 0.41 | 24.89 | 10.25 | 0.25 | |
| **1.63** | 0.99 | 0.35 | 0.54 | | | 0.12 | |
| **1.64** | 3.60 | 1.28 | 1.96 | | | 0.06 | 0.88 |
| **1.65** | 0.32 | 0.11 | 0.18 | | | 0.29 | |
| **1.66** | 0.01 | 0.00 | 0.00 | | | 0.30 | |
| **1.67** | 2.00 | 0.71 | 1.09 | 9.30 | ND | 0.38 | |
| **1.68** | 0.86 | 0.30 | 0.47 | | | 0.21 | |
| **1.69** | 3.31 | 1.17 | 1.80 | 8.50 | ND | 0.22 | 2.39 |
| **1.70** | 3.66 | 1.30 | 1.99 | 24.96 | 12.00 | 0.84 | 2.08 |
| **1.71** | 2.01 | 0.71 | 1.09 | | | 0.21 | |
| **1.72** | 6.49 | 2.30 | 3.53 | 6.50 | ND | 0.21 | 1.89 |
| **1.73** | 19.95 | 0.28 | 0.21 | 3.20 | ND | 0.31 | |
| **1.74** | 19.33 | 0.27 | 0.21 | 5.50 | ND | 0.20 | |
| **1.75** | 22.25 | 0.31 | 0.24 | | | 0.10 | |
| **1.76** | 11.42 | 0.16 | 0.12 | | | 0.37 | |
| **1.77** | -15.90 | -0.23 | -0.17 | | | 0.08 | |
| **1.78** | -4.60 | -0.07 | -0.05 | | | 0.26 | |
| **1.79** | 18.78 | 0.27 | 0.20 | | | 0.25 | |
| **1.80** | 35.51 | 0.50 | 0.38 | 9.00 | ND | 0.71 | |
| **1.81** | -4.15 | -0.06 | -0.04 | | | 0.33 | |
| **1.82** | -37.51 | -0.53 | -0.40 | | | 0.17 | |
| **1.83** | 7.11 | 0.10 | 0.08 | | | 0.08 | |
| **1.84** | -21.33 | -0.30 | -0.23 | | | 0.06 | |
| **1.85** | -3.61 | -0.05 | -0.04 | | | 0.13 | |
| **1.86** | -19.68 | -0.28 | -0.21 | | | 0.06 | |
| **1.87** | -339 | -0.05 | -0.04 | | | 030 | |
| **1.88** | 55.61 | 0.79 | 0.59 | 5.50 | ND | 0.10 | 1.25 |
| **1.89** | -6.73 | -0.10 | -0.07 | | | 0.17 | |
| **1.90** | 11.18 | 0.16 | 0.12 | | | 0.10 | |
| **1.91** | -31.50 | -0.45 | -0.33 | | | 0.13 | |
| **1.92** | -7.56 | -0.11 | -0.08 | | | 0.13 | |
| **1.93** | -12.37 | -0.18 | -0.13 | | | 0.11 | |
| **1.94** | 49.60 | 0.70 | 0.53 | 14.10 | ND | 1.39 | 2.33 |
| **1.95** | 10.68 | 0.15 | 0.11 | | | 0.16 | |
| **1.96** | 144.63 | 2.05 | | 63.24 | 74.75 | 0.75 | 0.80 |
| R-690 | 94.09 | 1.33 | 1.00 | | | | |
| M-A20 | 70.64 | 1.00 | | | | | |
| | | | | | | | |
| R-690 | 7.59 | | 1.00 | | | | |
| M-A20 | 5.33 | 1.00 | | | | | |
| **1.97** | 1.47 | 0.28 | 0.19 | | | 0.37 | |
| **1.98** | 3.69 | 0.69 | 0.49 | 38.67 | 16.57 | 0.43 | 1.69 |
| **1.99** | 4.32 | 0.83 | 0.57 | 38.31 | 18.76 | 0.40 | 1.48 |
| **1.100** | 0.22 | 0.04 | 0.03 | | | 032 | |
| **1.101** | 2.06 | 0.39 | 0.27 | | | 0.49 | |
| **1.102** | 0.23 | 0.04 | 0.03 | | | 0.12 | |
| **1.103** | 0.33 | 0.06 | 0.04 | | | 0.28 | |
| **1.104** | 0.45 | 0.08 | 0.06 | | | 0.08 | |
| **1.105** | 4.19 | 0.79 | 0.55 | 37.19 | 12.41 | 0.25 | 2.18 |
| **1.106** | 4.22 | 0.79 | 0.56 | 46.24 | 30.59 | 1.21 | 1.58 |
| **1.107** | 0.15 | 0.03 | 0.02 | | | 0.06 | |
| **1.108** | 0.08 | 0.01 | 0.01 | | | 0.31 | |
| **1.109** | 2.70 | 0.51 | 0.36 | 6.5 | 6 | 0.07 | |
| **1.110** | 1.02 | 0.19 | 0.13 | | | 035 | |
| **1.111** | 2.55 | 0.48 | 0.34 | | | 0.10 | |
| **1.112** | 3.58 | 0.67 | 0.47 | 18.6 | 4.2 | 1.25 | 1.74 |
| **1.113** | 037 | 0.07 | 0.05 | | | 0.35 | |
| **1.114** | -0.06 | -0.01 | -0.01 | | | 0.27 | |
| **1.115** | 0.55 | 0.10 | 0.07 | | | 0.13 | |
| **1.116** | 2.24 | 0.42 | 0.30 | | | 0.44 | |
| **1.117** | 0.56 | 0.10 | 0.07 | | | 0.27 | |
| **1.118** | 0.77 | 0.14 | 0.10 | | | 0.43 | |
| **1.119** | 0.78 | 0.15 | 0.10 | | | 0.41 | |
| **1.120** | 0.73 | 0.14 | 0.10 | | | 0.58 | |
| **1.121** | 0.21 | 0.05 | 0.03 | | | 0.40 | |
| **1.122** | 0.11 | 0.03 | 0.02 | | | 0.29 | |
| **1.123** | 0.41 | 0.11 | 0.07 | | | 0.07 | |
| **1.124** | 3.66 | 0.95 | 0.61 | 41.27 | 34.83 | 0.28 | 1.85 |
| **1.125** | 2.67 | 0.69 | 0.44 | | | 0.27 | 1.55 |
| **1.126** | 2.36 | 0.61 | 039 | | | 0.86 | 1.71 |
| **1.127** | 0.70 | 0.18 | 0.12 | | | 0.11 | |
| **1.128** | 2.99 | 0.77 | 0.50 | | | 0.13 | 1.45 |
| **1.129** | 0.33 | 0.09 | 0.06 | | | 0.39 | |
| **1.130** | 0.40 | 0.10 | 0.07 | | | 0.18 | |
| **1.131** | 1.45 | 0.38 | 0.24 | | | 0.52 | |
| **1.132** | 0.33 | 0.08 | 0.05 | | | 0.25 | |
| **1.133** | 0.17 | 0.04 | 0.03 | | | 0.24 | |
| **1.134** | 0.86 | 0.22 | 0.14 | | | 0.15 | |
| **1.135** | 1.75 | 0.45 | 0.29 | | | 0.30 | |
| **1.136** | 1.35 | 0.35 | 0.23 | | | 0.07 | |
| **1.137** | 2.30 | 0.59 | 0.38 | | | 0.83 | 1.30 |
| **1.138** | 0.83 | 0.21 | 0.14 | | | 0.60 | |
| **1.139** | 1.57 | 0.41 | 0.26 | | | 0.55 | |
| **1.140** | 1.40 | 0.36 | 0.23 | | | 1.28 | |
| **1.142** | -0.10 | -0.03 | -0.02 | | | 0.26 | |
| **1.143** | 1.46 | 0.38 | 0.24 | | | 0.16 | |
| **1.144** | 2.41 | 0.62 | 0.40 | | | 0.76 | |
| R-690 | 6.00 | | 1.00 | | | | |
| M-A20 | 3.86 | 1.00 | | 56.4 | 5 | | |
| | | | | | | | |
| R-690 | 2.58 | 3.22 | 1.00 | | | | |
| M-A20 | 0.80 | 1.00 | | | | | |
| **1.145** | 0.23 | 0.29 | 0.09 | | | 0.18 | |
| **1.146** | -0.12 | -0.15 | -0.05 | | | 0.41 | |
| **1.147** | 0.14 | 0.18 | 0.06 | | | 0.31 | |
| **1.148** | 0.09 | 0.11 | 0.03 | | | 0.43 | |
| **1.149** | 0.39 | 0.49 | 0.15 | | | 0.37 | |
| **1.150** | 2.23 | 2.79 | 0.87 | 17.3 | 5.4 | 0.70 | 1.46 |
| **1.151** | 0.13 | 0.16 | 0.05 | | | 0.29 | |
| **1.152** | 0.55 | 0.69 | 0.21 | | | 033 | |
| **1.154** | -0.20 | -0.25 | -0.08 | | | 0.41 | |
| **1.155** | 0.16 | 0.19 | 0.06 | | | 0.23 | |
| **1.156** | 0.06 | 0.07 | 0.02 | | | 0.31 | |
| **1.158** | 0.54 | 0.67 | 0.21 | | | 0.58 | |
| **1.159** | 0.78 | 0.98 | 0.30 | | | 0.09 | |
| **1.160** | 0.23 | 0.29 | 0.09 | | | 0.08 | |
| **1.162** | 0.63 | 0.78 | 0.24 | | | 0.11 | |
| **1.163** | 0.20 | 0.25 | 0.08 | | | 0.10 | |
| **1.164** | 0.22 | 0.27 | 0.08 | | | 0.09 | |
| **1.166** | 1.41 | 1.76 | 0.55 | 22.9 | 5.3 | 0.52 | 2.41 |
| **1.167** | 0.32 | 0.40 | 0.12 | | | 0.08 | |
| **1.168** | 0.88 | 1.10 | 0.34 | 15.9 | 5.1 | 0.48 | 1.90 |
| **1.170** | 0.22 | 0.42 | 0.11 | | | 0.21 | |
| **1.171** | 0.40 | 0.76 | 0.19 | | | 038 | |
| **1.172** | 0.09 | 0.17 | 0.04 | | | 0.12 | |
| **1.174** | 0.23 | 0.43 | 0.11 | | | 0.15 | |
| **1.175** | 0.14 | 0.26 | 0.07 | | | 0.20 | |
| R-690 | 2.06 | 3.91 | 1.00 | | | | |
| M-A20 | 0.53 | 1.00 | | 56.4 | 5 | | |
| for 1.170 to 1.175 | | | | | | | |
| FI-fluorescence intensity of primary antibody | | | | | | | |
| CFI-fluorescence intensity of human irrelevant primary antibody. | | | | | | | |
| A20-mouse anti-L762P monoclonal antibody | | | | | | | |
| R690-rabbit anti-L762P affinity purified polyclonal antibody | | | | | | | |
| FACS VRL762-percent positive cells from transient transfection of VR1013/L762 expression plasmid | | | | | | | |
| FACS VR(-)-percent positive cells from transient transfection of empty VR1013 expression plasmid | | | | | | | |

### EXAMPLE 32

### EPITOPE MAPPING AND PURIFICATION OF HL523S-SPECIFIC ANTIBODIES

This Example describes the purification of L523S antibodies that can distinguish between human and mouse L523S homologs and will likely distinguish between hL523S and hL523S-family members such as hIMF-1 and hIMP-2.

L523S (full-length cDNA and amino acid sequence set forth in SEQ ID NO:347 and 348, respectively) is one of a family of proteins that includes hIMP-1 and hIMP-2. The members of this family of proteins have a high degree of similarity one to the other and are also highly similar between species. Thus, generating antibodies that specifically recognize human L523S (hL523S) and not other members of the protein family in humans or the mouse homologs, has been problematic. However, in order to evaluate preclinical and clinical L523S DNA/Adenoviral vaccines by detecting the protein expression of L523S, human L523S-specific antibodies are critical.

Polyclonal antibodies specific for hI,523S were generated as described in Example 23. These antibodies were used to map epitopes. The epitope analysis showed 2 particular peptides of hL523S that were recognized, peptide 16/17 and peptide 32.

The amino acid sequences of both hL523S and mouse L523S (mL523S) peptide 16/17 and peptide 32 were then compared. Peptide 32/33 is identical between hL523S and mL523S. However, as the alignment below indicates, peptide 16/17 has 5 amino acid differences between the human and mouse homologs (underlined).

hL523S (16/17) (SEQ ID NO:468):
IPDEMAAQQNPLQQPRGRRGLGQR

mL523S (16/17) (SEQ ID NO:469):
IPDETAAQQNPSPQLRGRRGPGQR

Moreover, peptide-based ELISAs showed that peptide 17 is specifically recognized by lung cancer patient sera #197, and a homology search of peptide 17 between human IMP (hIMP) family members shows that there is little similarity in this region between family members. The hL523S peptide 17 (and 16/17) has less than 50% similarity to hL523S family members such as hIMP-1 and hIMP-2.

Based upon the epitope mapping of L523S-specific antibodies and the data from the homology search, hL523S or mL523S peptide 16/17-conjugated ligands were then used to purify human or mouse L523S-specific antibodies from rabbit polyclonal antibodies generated against hL523S protein as described in Example 23. The data from the antibodies purified by affinity chromatography using ligands conjugated with either hL523S-peptide 16/17 or mL523S-peptide 16/17 suggested that the affinity of antibodies specific to hL523S-peptide 16/17 is much higher than that of antibodies to mL523S-peptide 16/17 since they bind more strongly to hL523S-peptide 16/17 than to mL523S-peptide 16/17. The difference in affinity between the purified antibodies to human and mouse L523S-peptide 16/17 was confirmed by peptide-based ELISA.. The antibodies purified by hL523S-peptide 16/17 selectively bind to human L523S-peptide 16/17 but bind much less or not at all to mL523S-peptide 16/17.

In order to further characterize the original polyclonal antibodies and antibodies purified by hL523S-peptide 16/17, immunoblot analysis was conducted using both human lung adenocarcinoma line as a source of hL523S protein and mouse whole body embryo (day 17 gestation) as the source of mL523S protein. This analysis showed that polyclonal antibodies specific for hL523S recognize hL523S protein expressed in the tumor cell line as well as mL523S protein expressed in whole body embryos of day 17 gestation. However, the addition of hL523S peptide 32/33 blocks binding of antibodies to human and mouse L523S proteins. Thus, the crossreactivity of the polyclonal antibodies to mL523S protein is due to the existence of antibodies specific to hL523S peptide 32/33. In marked contrast, the purified antibodies specific to hL523S peptide 16/17 do not bind mL523S protein expressed in mice embryos but do recognize hL523S protein expressed in human lung adenocarcinoma cells. These data confirm the ELISA data using hL523S-peptide 16/17 and mL523S-peptide 16/17 described above.

The amino acid sequence of hL523S peptide 16/17 used to purify the antibodies is about 60-70% similar to that of the mL523S-peptide 16/17 which is not recognized by hL523S-specific antibodies by Western blot analysis and peptide-based ELISA. The hL523S peptide 16/17 has less than 50% similarity to hL523S family members such as HMP-1 and hIMP-2. Iaken together, these data suggest that it is highly probable that the antibodies purified by hL523S peptide 16/17 described herein will also distinguish hL523S protein from the other hL523S family members.

In summary, antibodies purified with the hL523S peptide 16/17 do not recognize the mouse L523 S homolog. The amino acid sequence of peptide 16/17 between hL523S family members is less similar than between human and mouse L523S. Thus, the hL523S-specific antibodies described above can be used to distinguish between human and mouse L523S and between members of the hL523S family of proteins and can therefore be used for the accurate detection of hL523S protein expression in animals and humans.

### EXAMPLE 33

### IN VIVO IMMUNOGENECIIY OF LUNG TUMOR ANIIGEN L523

This example describes two *in vivo* immunogenicity studies to evaluate the vaccination of mice with either an adenovirus containing L523 or with L523 naked DNA followed by a second immunization with an adenovirus containing L523.

The first study involved the immunization of two strains of mice with L523 adenovirus. The C57B16 strain of mice is homozygous for HLA-type H-2^{b}, while strain B6D2(F1) is heterozygous for the HLA-type, H-2^{b/d}. Table 14 describes the initial immunization strategy employed..

**Table 14: Immunization with L523 Adenovirus alone: Experimental Design**

| **Group** | **Immunization** | **Strain (4/group)** |
|---|---|---|
| 1 | 10⁸ PFU Ad L523 A | C57BL6 |
| 2 | 10⁷ PFU Ad hrGFP A | C57BL6 |
| 3 | 10⁸ PFU Ad L523 A | B6D2(F1) |
| 4 | 10⁷ PFU Ad hrGFP A | B6D2(F1) |
| 5 | Naïve | C57BL6 |
| 6 | Naïve | B6D2(F1) |

| | | |
|---|---|---|
| PFU=plaque forming unit; GFP=green fluorescent protein; Ad=adenovirus.. | | |

Mice were immunized intradermally with either 10⁸ PFU of L523-adenovirus or 10⁷ PFU of an irrelevant adenovirus (hrGFP). Three weeks following immunization, IgG1 and IgG2a antibody responses to L523 were examined in all groups of mice. Briefly, recombinant full length L523 (rL523) was coated onto ELISA plates and serum, at multiple dilutions, was added to the wells.. Following a 60-minute incubation, the serum was washed from the wells and a secondary antibody, either specific for an IgG1 or IgG2a was added to the plates.. Both antibodies were directly conjugated to horseradish peroxide (HRP). The levels of L523 antibodies, either IgG1 or IgG2a, were measured in all groups. In the C57BL6 mice, little to no L523-specific antibodies were detected following immunization. However, in the B6D2(F1) strain of mice immunized with L523 adenovirus, both IgG1 and IgG2a L523-specific antibodies were detected at serum dilution as low as 1/1000.

In addition to detecting L523-specific antibodies in the serum, interferon-gamma (IFN-γ) responses were assayed from immune spleen cells following *in vitro* stimulation with rL523 protein. Briefly, spleen cells were harvested from all mice groups and cultured for 3 days in 96-well plates. Culture conditions included, media alone, 1 or 10µg/ml of rL523 protein, or 5µg/ml of concanavalin A (Con A). After 3 days, the supernatants were harvested and assayed for IFN-γ levels in the supernatants.

Immunization with L523-adenovirus, but not an irrelevant adenovirus, elicited a strong IFN-γ response from the spleen cells which were stimulated with rL523. In general, responses were stronger in the B6D2(F1) mouse strain, as evidenced by both a higher level of IFN-γ production, as well as the fact that stimulation with a lower antigen concentration (1µg/ml) elicited an equally strong response as seen with the higher antigen concentration (10µg/ml).

Finally, T cell proliferation responses were assayed from immune spleen cells by stimulation *in vitro* with rL523 protein. Briefly, spleen cells were cultured for 4 days in 96-well plates with, media alone, 1 or 10µg/ml of rL523 protein, or Con A. The cultures were then pulsed with 3H-thymidine for the final 8 hours of culture. Results are represented as the stimulation index (SI) in the presence of antigen relative to stimulation with media alone. Results were consistent with those obtained in the IFN-γ assay. Immunization with L523-adenovirus, but not an irrelevant adenovirus, elicited a proliferation response in spleen cells stimulated with rL523. A strong SI (average of >20) was observed in spleen cells harvested from the B6D2(F1) mouse strain, with similar levels of proliferation, observed at both protein concentrations.. Little or no T cell proliferation was observed in the C57BL6 mouse strain.

A second study involved the immunization of two strains of mice initially with L523 naked DNA followed by a second immunization with L523 adenovirus two weeks later. The mice were harvested 3 weeks after the boost. Table 15 describes the immunization regimen of the second study.

**Table 15: Immunization with L523 DNA followed by a second immunization with L523-Adenovizus: Experimental Design**

| **Group** | **Immunization** | **Strain (4/group)** |
|---|---|---|
| 1 | L523 DNA +10⁸ PFU Ad L523 A | C57BL6 |
| 2 | 10⁸ PFU Ad L523 A | C57BL6 |
| 3 | relevant DNA + 10⁷ PFU Ad hrGFP A | C57BL6 |
| 4 | 10⁷ PFU Ad hrGFP A | C57BL6 |
| 5 | Naive | C57BL6 |
| 6 | L523 DNA +10⁸ PFU Ad L523 A | B6D2(F1) |
| 7 | 10⁸ PFU Ad L523 A | B6D2(F1) |
| 8 | Irrelevant DNA + 10⁷ PFU Ad hrGFP A | B6D2(F1) |
| 9 | 10⁷ PFU Ad hrGFP A | B6D2(F1) |
| 10 | Naive | B6D2(F1) |

| | | |
|---|---|---|
| PFU=plaque forming unit; GFP=green fluorescent protein; Ad=adenovirus. | | |

As described in the first study, strong IgG1 and IgG2a antibody responses were observed in B6D2(F1) mice following immunization with L523-adenovirus. Immunizing with L523 DNA appealed to increase the overall L523-specific antibody response compared to responses achieved with immunization with L523-adenovirus alone. C57BL6 mice elicited little or no L523-specific antibody responses following immunization with L523-adenovirus, but were some slightly positive responses were detected in mice immunized with L523 DNA followed by a second immunization with L523-adenovirus.

IFN-γ responses were assayed from immune spleen cells by stimulation *in vitro* with rL523 protein. These results confirm those observed in the initial study demonstrating the immunogenecity of L523 in animals. The results also suggest that initially immunizing the animals with L523 DNA, prior to immunization with L523 adeonvirus, does not significantly increase the CD4 response. As with the initial study, responses appear to be stronger in the B6D2(F1) strain of mice than the C57BL6 strain

As with the initial study, T cell proliferation responses were assayed from immune spleen cells by stimulation *in vitro* with rL523 protein. The results from using two rounds of immunization are consistent with those obtained from the first study. Immunization with L523 DNA prior to a second round of immunization with L523-adenovirus did not significantly increase the proliferation responses generated in the mice. As with the first study, responses were stronger in the B6D2(F1) mouse strain than in the C57BL6 strain.

The difference in HLA types between the two strains of mice could explain variations in the extent of the immune responses detected. As described above, the C57BL6 strain is homozygous for H-2^{b}, while the B6D2(F1) is heterozygous for H-2^{b/d}. The increased diversity of the B6D2(F1) strains HLA type allows for a greater number of epitopes derived from the L523 protein to be presented. In this strain, epitopes specific for both H-2^{b} and H-2^{d} can be presented, while only H-2^{b} epitopes can be presented by the C57BL6 strain.

From the foregoing it will be appreciated that, although specific embodiments of the invention have been described herein for purposes of illustration, various modifications may be made without deviating from the spirit and scope of the invention. Accordingly, the invention is not limited except as by the appended claims. The present invention will now be described by the following clauses,
1. A method for inducing an immune response in an animal, comprising:
   a) providing a composition comprising a polynucleotide encoding at least an immunogenic portion of a lung carcinoma polynucleotide wherein the polynucleotide has at least 90%identity with SEQ ID NO:347;
   b) administering said polynucleotide; and
   c) thereby inducing an immune response in an animal.
2. The method of clause 1, wherein said composition further comprises a component selected from the group consisting of a physiologically acceptable carrier or an adjuvant.
3. A method according to clause 1, wherein the lung carcinoma polynucleotide is delivered by a viral based delivery system.
4. A method according to clause 3, wherein the viral based delivery system is an adenovirus.
5. The method of clause 1, wherein the immune response induced is a CD4+ T helper response..
6. The method of clause 1, wherein the immune response induced is a CD8+ cytotoxic T lymphocyte responses.
7. The method of clause 1, wherein the immune response induced is both a CD4+ T helper and CD8+ cytotoxic T cell immune response.
8. An isolated polynucleotide comprising a sequence selected from the group consisting of:
   (a) sequences provided in SEQ ID NO:351, 353, 358, 362, 364, 366, 368,370-375,420,424,428,431,434,442,447,450 and 467;
   (b) complements of the sequences provided in SEQ ID NO:351, 353, 358, 362, 364, 366, 368, 370-375, 420, 424, 428, 431, 434, 442, 447, 450 and 467;
   (c) sequences consisting of at least 10 contiguous residues of a sequence provided in SEQ ID NO:351, 353, 358, 362, 364, 366, 368, 370-375, 420, 424,428,431,434,442,447,450 and 467;
   (d) sequences that hybridize to a sequence provided in SEQ ID NO:351, 353, 358, 362, 364, 366, 368, 370-375, 420, 424, 428, 431, 434, 442, 447, 450 and 467, under highly stringent conditions;
   (e) sequences haying at least 75% identity to a sequence of SEQ ID NO:351, 353, 358, 362, 364, 366, 368, 370-375,420, 424,428,431,434,442,447,450 and 46 7;
   (f) sequences having at least 90% identity to a sequence of SEQ ID NO:351, 353, 358, 362, 364, 366, 368, 370-375, 420, 424, 428, 431, 434, 442, 447, 450 and 467; and
   (g) degenerate variants of a sequence provided in SEQ ID NO:351, 353,358,362,364, 366, 368, 370-375, 420, 424, 428, 431, 434,442,447,450 and 467.
9. An isolated polypeptide comprising an amino acid sequence selected from the group consisting of:
   (a) sequences having at least 90% identity to a polypeptide having an amino acid sequence of any one of the sequences provided in SEQ II7 NO:352, 354, 357, 361, 363, 365, 367, 369, 376-382, 387-419, 423, 427, 430, 4.33, 441, 443, 446, 449,451-466 and 468-469;
   (b) sequences encoded by a polynucleotide of clause 8;
   (c) sequences having at least 70% identity to a sequence encoded by a polynucleotide of clause 8; and
   (d) sequences having at least 90% identity to a sequence encoded by a polynucleotide of clause 8..
10. An expression vector comprising a polynucleotide of clause 8 operably linked to an expression control sequence.
11. A host cell transformed or transfected with an expression vector according to clause 10.
12. An isolated antibody, or antigen-binding fragment thereof, that specifically binds to a polypeptide ofclause9.
13. A method for detecting the presence of a cancer in a patient, comprising the steps of:
   (a) obtaining a biological sample from the patient;
   (b) contacting the biological sample with a binding agent that binds to a polypeptide of clause 9;
   (c) detecting in the sample an amount of polypeptide that binds to the binding agent; and
   (d) comparing the amount of polypeptide to a predetermined out-off value and therefrom determining the presence of a cancer in the patient.
14. A fusion protein comprising at least one polypeptide according to clause 9.
15. A fusion protein according to clause 14, wherein the fusion protein is selected from the group consisting sequences provided in SEQ ID NO:352, 354,423,427, 430 and 433.
16. An oligonucleotide that hybridizes to a sequence recited in SEQ ID NO:351, 353, 358, 362, 364, 366, 368, 370-375, 420, 424, 428, 431, 434, 442, 447, 450 and 467 under highly stringent conditions.
17. A method for stimulating and/or expanding T cells specific for a tumor protein, comprising contacting T cells with at least one component selected from the group consisting of:
   (a) polypeptides according to clause 9;
   (b) polynucleotides according to clause 8; and
   (c) antigen-presenting cells that express a polynucleotide according to clause 8,
   under conditions and for a time sufficient to permit the stimulation auld/or expansion of T cells.
18. An isolated T cell population, comprising T cells prepared according to the method of clause 17.
19. A composition comprising a first component selected fiom the group consisting of physiologically acceptable carriers and immunostimulants, and a second component selected from the group consisting of:
   (a) polypeptides according to clause 9;
   (b) polynucleotides according to clause 8;
   (c) antibodies according to clause 12;
   (d) fusion proteins according to clause 14;
   (e) T cell populations according to clause 18; and
   (f) antigen presenting cells that express a polypeptide according to clause 9.
20. A method for stimulating an immune response in a patient, comprising administering to the patient a composition of clause 19.
21. A method for the treatment of a lung cancer in a patient, comprising administering to the patient a composition of clause 19.
22. A method for determining the presence of a cancer in a patient, comprising the steps of:
   (a) obtaining a biological sample from the patient;
   (b) contacting the biological sample with an oligonucleotide according to clause 9;
   (c) detecting in the sample an amount of a polynucleotide that hybridizes to the oligonucleotide; and
   (d) compare the amount of polynucleotide that hybridizes to the oligonucleotide to a predetermined cut-off value, and therefrom determining the presence of the cancer in the patient.
23. A diagnostic kit comprising at least one oligonucleotide according to clause 16.
24. A diagnostic kit comprising at least one antibody according to clause 12 and a detection reagent wherein the detection reagent comprises a reporter group.
25. A method for the treatment of lung cancer in a patient, comprising the steps of:
   (a) incubating CD4+ and/or CD8+ T cells isolated from a patient with at least one component selected from the group consisting of: (i) polypeptides according to clause 9; (ii) polynucleotides according to clause 8; and (iii) antigen presenting cells that express a polypeptide of clause 9, such that T cell proliferate;
   (b) administering to the patient an effective amount of the proliferated T cells,
   and thereby inhibiting the development of a cancer in the patient.

## Claims

1. The use of the polypeptide of SEQ ID NO:176 or SEQ ID NO:348, or an immunogenic portion thereof, or a composition comprising the polypeptide of SEQ ID NO:176 or SEQ ID NO:348, or an immunogenic portion thereof, for the manufacture of a medicament for the treatment of lung cancer.

2. The use of a fusion protein comprising the polypeptide of SEQ ID NO:176 or SEQ ID NO:348, or an immunogenic portion thereof, or a composition comprising the fusion protein for the manufacture of a medicament for the treatment of lung cancer.

3. The use of the polypeptide of SEQ ID NO:176 or SEQ ID NO:348, or an immunogenic portion thereof, or a composition comprising the polypeptide of SEQ ID NO:176 or SEQ ID NO:348, or an immunogenic portion thereof, for the manufacture of a medicament for inducing an immune response in a lung cancer patient.

4. The use of a fusion protein comprising the polypeptide of SEQ ID NO:176 or SEQ ID NO:348, or an immunogenic portion thereof, or a composition comprising the fusion protein for the manufacture of a medicament for inducing an immune response in a lung cancer patient.

5. The use of the polynucleotide of SEQ ID No. 175 or SEQ ID NO:347 or a composition comprising the polynucleotide of SEQ ID NO:175 or SEQ ID NO:347 for the manufacture of a medicament for the treatment of lung cancer.

6. The use of the polynucleotide of SEQ ID No. 175 or SEQ ID NO:347 or a composition comprising the polynucleotide of SEQ ID NO:175 or SEQ ID NO:347 for the manufacture of a medicament for inducing an immune response in a lung cancer patient.

7. The use of any one of claims 1-6, wherein the composition comprises an immunostimulant.

8. The use of claim 7 wherein the immunostimulant induces an immune response predominantly of the Th1 type.

9. The use of claim 7 wherein the immunostimulant is selected from the group consisting of monophosphoryl lipid A, 3d-MPL, RC-529, AGP's, Seppic Montanide ISA 720, a cytokine, a microsphere, AS-2, QS21, saponin based adjuvants, and immune stimulating complex (iscom) based adjuvants.
